# EUROPÄISCHE PATENTSCHRIFT

(11) **EP 2 027 096 B1**
(45) Veröffentlichungstag und Bekanntmachung des Hinweises auf die Patenterteilung: **23.05.2012**
(21) Anmeldenummer: 07725514.9
(22) Anmeldetag: 23.05.2007
(51) Int. Cl.: C07D 233/70, C07D 249/12, C07D 401/12, C07D 403/12, C07D 409/04, C07D 409/14, A61K 31/4166, A61K 31/4178, A61K 31/4196, A61P 9/00

(54) **SUBSTITUIERTE ARYLIMIDAZ0L0NE UND -TRIAZ0L0NE ALS INHIBITOREN DER VASOPRESSIN-REZEPTOREN**
SUBSTITUTED ARYLIMIDAZ0L0NE AND TRIAZ0L0NE AS INHIBITORS OF VASOPRESSIN RECEPTORS
IMIDAZOLONES ET TRIAZOLONES D'ARYLE SUBSTITUÉS COMME INHIBITEURS DES RÉCEPTEURS DE VASOPRESSINE

(30) Priorität: 23.05.2006 DE 102006024024
(43) Veröffentlichungstag der Anmeldung: 25.02.2009
(73) Patentinhaber: Bayer Pharma Aktiengesellschaft, 13353 Berlin (DE)
(72) Erfinder: MEIER, Heinrich, 42115 Wuppertal (DE); BENDER, Eckhard, 40764 Langenfeld (BE); BRÜGGEMEIER, Ulf, 42799 Leichlingen (DE); FLAMME, Ingo, 51580 Reichshof (DE); KARTHAUS, Dagmar, 42697 Solingen (DE); KOLKHOF, Peter, 42113 Wuppertal (DE); MEIBOM, Daniel, 51373 Leverkusen (DE); SCHNEIDER, Dirk, 42115 Wuppertal (DE); VOEHRINGER, Verena, 42113 Wuppertal (DE); FÜRSTNER, Chantal, 45478 Mülheim/Ruhr (DE); KELDENICH, Jörg, 42113 Wuppertal (DE); LANG, Dieter, 42553 Velbert (DE); POOK, Elisabeth, 42109 Wuppertal (DE); SCHMECK, Carsten, 45472 Mülheim (DE)
(86) Internationale Anmeldenummer: PCT/EP2007/004615
(87) Internationale Veröffentlichungsnummer: WO 2007/134862

(56) Entgegenhaltungen:
- WO-A-2005/105779

## Beschreibung

Die vorliegende Anmeldung betrifft neue, substituierte 4-Arylimidazol-2-one und 5-Aryl-1,2,4-triazolone, Verfahren zu ihrer Herstellung, ihre Verwendung allein oder in Kombinationen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention von kardiovaskulären Erkrankungen.

Der Flüssigkeitsgehalt des menschlichen Körpers unterliegt verschiedenen physiologischen Kontrollmechanismen, die auf seine Konstanterhaltung abzielen (Volumenhomöostase). Dabei werden sowohl die Volumenfüllung des Blutgefäßsystems als auch die Osmolarität des Blutplasmas kontinuierlich von entsprechenden Sensoren (Barorezeptoren und Osmorezeptoren) registriert. Die Informationen, die diese Sensoren an die zuständigen Zentren des Hirns liefern, regulieren das Trinkverhalten und steuern vermittels humoraler und nervaler Signale die Flüssigkeitsausscheidung über die Nieren. Eine zentrale Bedeutung kommt hierbei dem Peptidhormon Vasopressin zu [Schrier R.W., Abraham, W.T., New Engl. J. Med. 341, 577-585 (1999)].

Vasopressin wird in spezialisierten, endokrinen Neuronen im *Nucleus supraopticus* und *N. paraventricularis* in der Wand des dritten Ventrikels (Hypothalamus) produziert und von dort entlang ihrer Nervenfortsätze in den Hypophysenhinterlappen (Neurohypophyse) verbracht. Dort wird das Hormon je nach Stimulus in die Blutbahn abgegeben. Ein Volumenverlust, z.B. infolge akuter Blutung, starkem Schwitzen, langem Dursten oder Diarrhöe, ist ein Stimulus für die verstärkte Ausschüttung des Hormons. Umgekehrt wird die Sekretion von Vasopressin durch eine Erhöhung des Intravasalvolumens, z.B. infolge gesteigerter Flüssigkeitszufuhr, gehemmt.

Vasopressin entfaltet seine Wirkung hauptsächlich über Bindung an drei Rezeptoren, die als V1a-, Vlb- und V2-Rezeptoren klassifiziert werden und zur Familie der G-Protein-gekoppelten Rezeptoren gehören. V1a-Rezeptoren sind vorwiegend auf den Zellen der glatten Gefäßmuskulatur lokalisiert. Ihre Aktivierung ruft eine Vasokonstriktion hervor, wodurch der periphere Widerstand und Blutdruck ansteigen. Daneben sind V1a-Rezeptoren auch in der Leber nachweisbar. V1b-Rezeptoren (auch V3-Rezeptoren genannt) sind im zentralen Nervensystem nachweisbar. Zusammen mit dem Corticotropin-Releasing-Hormon (CRH) reguliert Vasopressin über den V1b-Rezeptor die basale und stressinduzierte Sekretion des Adrenocorticotropen Hormons (ACTH). V2-Rezeptoren finden sich im distalen Tubulusepithel und dem Epithel der Sammelrohre der Niere. Ihre Aktivierung macht diese Epithelien für Wasser durchlässig. Diesem Phänomen liegt der Einbau von Aquaporinen (speziellen Wasserkanälen) in die luminale Membran der Epithelzellen zugrunde.

Welche Bedeutung Vasopressin für die Rückresorption von Wasser aus dem Harn in der Niere hat, wird durch das Krankheitsbild des Diabetes insipidus deutlich, das durch einen Mangel des Hormons - z.B. infolge einer Hypophysenschädigung - hervorgerufen wird. Patienten, die an diesem Krankheitsbild leiden, scheiden bis zu 20 Liter Harn pro 24 Stunden aus, sofern ihnen das Hormon nicht substituiert wird. Dieses Volumen entspricht in etwa 10% des Primärharns. Wegen seiner großen Bedeutung für die Rückresorption von Wasser aus dem Harn wird Vasopressin auch synonym als Antidiuretisches Hormon (ADH) bezeichnet. Folgerichtig führt eine pharmakologische Hemmung der Vasopressin/ADH-Wirkung am V2-Rezeptor zu einer vermehrten Urinausscheidung. Im Gegensatz zu der Wirkung anderer Diuretika (Thiazide und Schleifendiuretika) jedoch bewirken V2-Rezeptor-Antagonisten eine vermehrte Wasserausscheidung, ohne die Ausscheidung von Elektrolyten maßgeblich zu steigern. Das bedeutet, dass durch V2-antagonistische Pharmaka die Volumenhomöostase wiederhergestellt werden kann, ohne dabei in die Elektrolyt-Homöostase einzugreifen. Daher erscheinen V2-antagonistisch wirksame Pharmaka besonders geeignet für die Behandlung aller krankhaften Zustände, die mit einer Überfrachtung des Organismus mit Wasser einhergehen, ohne dass parallel die Elektrolyte adäquat erhöht sind. Eine bedeutende Elektrolyt-Abnormalität ist klinisch-chemisch als Hyponatriämie (Natriumkonzentration < 135 mmol/L) messbar; sie stellt die wichtigste Elektrolyt-Abnormalität bei Krankenhauspatienten dar mit einer Häufigkeit von ca. 5% bzw. 250.000 Fällen pro Jahr allein in den USA. Bei einem Absinken der Plasma-Natriumkonzentration unter 115 mmol/L drohen komatöse Zustände und Tod.

Entsprechend der zugrunde liegenden Ursache unterscheidet man die hypovolämische, euvolämische und hypervolämische Hyponatriämie. Klinisch bedeutsam sind die Formen der Hypervolämie mit Ödembildung. Typische Beispiele hierfür sind das Syndrom der inadäquaten ADH/Vasopressin-Sekretion (SIAD) (z.B. nach Schädel-Hirn-Trauma oder als Paraneoplasie bei Carcinomen) und die hypervolämische Hyponatriämie bei Leberzirrhose, verschiedenen Nierenerkrankungen und Herzinsuffizienz [De Luca L. et al., Am. J. Cardiol. 96 (suppl.), 19L-23L (2005)]. Gerade Patienten mit Herzinsuffizienz weisen trotz ihrer relativen Hyponatriämie und Hypervolämie häufig erhöhte Vasopressin-Spiegel auf, was als die Folge einer generell gestörten neurohumoralen Regulation bei der Herzinsuffizienz angesehen wird [Francis G.S. et al., Circulation 82, 1724-1729 (1990)].

Die gestörte neurohumorale Regulation manifestiert sich im Wesentlichen in einer Erhöhung des Sympathicotonus und einer inadäquaten Aktivierung des Renin-Angiotensin-Aldosteron-Systems. Während die Hemmung dieser Komponenten durch Beta-Rezeptoren-Blocker einerseits und durch ACE-Inhibitoren bzw. Angiotensin-Rezeptor-Blocker andererseits heute fester Bestandteil der pharmakologischen Behandlung der Herzinsuffizienz ist, ist die inadäquate Steigerung der Vasopressin-Sekretion in der fortgeschrittenen Herzinsuffizienz derzeit noch nicht ausreichend therapierbar. Neben der durch V2-Rezeptoren vermittelten Retention von Wasser und den damit verbundenen ungünstigen hämodynamischen Folgen im Sinne einer Nachlasterhöhung werden auch durch V1a-vermittelte Vasokonstriktion die Entleerung des linken Ventrikels, der Druck in den Pulmonalgefäßen und die Herzleistung negativ beeinflusst. Darüber hinaus wird aufgrund tierexperimenteller Daten dem Vasopressin auch eine direkte Hypertrophie-fördernde Wirkung am Herzmuskel beigemessen. Im Unterschied zur renalen Wirkung der Volumenexpansion, die über Aktivierung von V2-Rezeptoren vermittelt ist, wird die direkte Wirkung am Herzmuskel durch Aktivierung von V1a-Rezeptoren ausgelöst.

Aus diesen Gründen erscheinen Substanzen, die die Wirkung des Vasopressins am V2- und/oder am V1a-Rezeptor hemmen, zur Behandlung der Herzinsuffizienz geeignet. Vor allem Verbindungen mit kombinierter Aktivität an beiden Vasopressin-Rezeptoren (V1a und V2) sollten sowohl wünschenswerte renale als auch hämodynamische Effekte bewirken und somit ein besonders ideales Profil für die Behandlung von Patienten mit Herzinsuffizienz bieten. Die Bereitstellung derartig kombinierter Vasopressin-Antagonisten erscheint auch insofern sinnvoll, als ein allein über V2-Rezeptor-Blockade vermittelter Volumenentzug die Erregung von Osmorezeptoren und in der Folge eine weitere kompensatorische Steigerung der Vasopressin-Ausschüttung nach sich ziehen kann. Hierdurch könnten - bei Fehlen einer gleichzeitig den V1a-Rezeptor blockierenden Komponente - die schädlichen Wirkungen des Vasopressins, wie z.B. Vasokonstriktion und Herzmuskelhypertrophie, noch verstärkt werden [Saghi P. et al., Europ. Heart J. 26, 538-543 (2005)].

Über die Synthese bestimmter Imidazolinonacetamid-Derivate und ihre antibakterielle Wirkung wird in J.J. Bronson et al., Bioorg. Med. Chem. Lett. 13, 873-875 (2003) berichtet. Imidazolinonalkansäuren mit pharmakologischer Aktivität werden in EP 051 829-A1 beschrieben. In WO 99/54315 werden substituierte Triazolone mit neuroprotektiver Wirkung offenbart, und in WO 2006/117657 werden Triazolon-Derivate als anti-inflammatorische Agentien beschrieben. Ferner werden in EP 503 548-A1 und EP 587 134-A2 cyclische Harnstoff-Derivate und ihre Verwendung zur Behandlung von Thrombosen beansprucht. Substituierte Imidazol- und Triazolthione als Ionenkanal-Modulatoren werden in WO 2005/086836, WO 2005/086892 und WO 2005/097112 offenbart. Triazolthion-Derivate werden außerdem in WO 02/066447 als Sphingomyelinase-Inhibitoren beschrieben. **In** WO 05/105779 **werden 3-Heterocyclyl-4-Phenyl-substituierte Triazolderivate als Vasopressin-Inhibitoren beschrieben.**

Gegenstand der vorliegenden Erfindung sind Verbindungen der allgemeinen Formel (I) 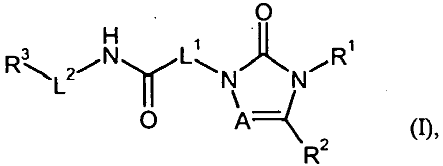 in welcher
A für N oder C-R⁴ steht, worin
   R⁴ Wasserstoff oder (C₁-C₄)-Alkyl bedeutet,
R¹ für (C₁-C₆)-Alkyl, (C₂-C₆)-Alkenyl oder (C₂-C₆)-Alkinyl steht, welche jeweils ein- bis dreifach, gleich oder verschieden, mit Resten ausgewählt aus der Reihe Halogen, Cyano, Oxo, Trifluormethyl, (C₃-C₇)-Cycloalkyl, Phenyl, -OR¹⁰, -NR¹¹R¹², -C(=O)-OR¹³ und -C(=O)-NR¹⁴R¹⁵ substituiert sein können, worin
   (i) (C₃-C₇)-Cycloalkyl bis zu zweifach, gleich oder verschieden, mit (C₁-C₄)-Alkyl, Oxo, Hydroxy, (C₁-C₄)-Alkoxy und/oder Amino substituiert sein kann,
   (ii) Phenyl bis zu dreifach, gleich oder verschieden, mit Resten ausgewählt aus der Reihe Halogen, Cyano, Nitro, (C₁-C₄)-Alkyl, Trifluormethyl, Hydroxy, Hydroxymethyl, (C₁-C₄)-Alkoxy, Trifluormethoxy, (C₁-C₄)-Alkoxymethyl, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl substituiert sein kann,
   (*iii*) R¹⁰, R¹¹, R¹², R¹³, R¹⁴ und R¹⁵ unabhängig voneinander bei jedem einzelnen Auftreten Wasserstoff, (C₁-C₆)-Alkyl oder (C₃-C₇)-Cycloalkyl bedeuten, wobei
      (C₁-C₆)-Alkyl seinerseits bis zu zweifach, gleich oder verschieden, mit Amino, Hydroxy, (C₁-C₄)-Alkoxy, Hydroxycarbonyl und/oder (C₁-C₄)-Alkoxycarbonyl substituiert sein kann
      und
      (C₃-C₇)-Cycloalkyl seinerseits bis zu zweifach, gleich oder verschieden, mit (C₁-C₄)-. Alkyl, Oxo, Hydroxy, (C₁-C₄)-Alkoxy und/oder Amino substituiert sein kann,
      und/oder
   (*iv*) R¹¹ und R¹² sowie R¹⁴ und R¹⁵ jeweils paarweise zusammen mit dem Stickstoffatom, an das sie gebunden sind, einen 4- bis 7-gliedrigen Heterocyclus bilden, welcher ein weiteres Heteroatom aus der Reihe N, O und S enthalten und bis zu zweifach, gleich oder verschieden, mit (C₁-C₄)-Alkyl, Oxo, Hydroxy, (C₁-C₄)-Alkoxy und/oder Amino substituiert sein kann,
   oder
R¹ für (C₃-C₇)-Cycloalkyl steht, welches bis zu zweifach, gleich oder verschieden, mit (C₁-C₄)-Alkyl, (C₁-C₄)-Alkoxy, Hydroxy, Amino und/oder Oxo substituiert sein kann,
R² für Phenyl, Naphthyl, Thienyl, Benzothienyl, Furyl oder Benzofuryl steht, welche jeweils ein- bis dreifach, gleich oder verschieden, mit Resten ausgewählt aus der Reihe Halogen, Cyano, Nitro, (C₁-C₄)-Alkyl, Trifluormethyl, Hydroxy, (C₁-C₄)-Alkoxy, Trifluormethoxy und Phenyl substituiert sein können,
   wobei der letztgenannte Phenyl-Rest seinerseits bis zu zweifach, gleich oder verschieden, mit Resten ausgewählt aus der Reihe Halogen, Cyano, Nitro, (C₁-C₄)-Alkyl, Trifluormethyl, Hydroxy, (C₁-C₄)-Alkoxy, Trifluormethoxy, Hydroxy-(C₁-C₄)-alkyl und (C₁-C₄)-Alkylthio substituiert sein kann,
L¹ für eine Gruppe der Formel -(CR^{5A}R^{5B})ₘ- steht, worin
   m die Zahl 1, 2 oder 3 bedeutet
      und
   R^{5A} und R^{5B} unabhängig voneinander Wasserstoff oder (C₁-C₄)-Alkyl bedeuten
      oder
      zwei an demselben Kohlenstoffatom gebundene Reste R^{5A} und R^{5B} miteinander verknüpft sind und zusammen eine -(CH₂)ₙ-Brücke bilden, worin
      n die Zahl 2, 3, 4 oder 5 bedeutet,
   oder im Falle, dass m für die Zahl 2 oder 3 steht,
   zwei an benachbarten (1,2- oder 2,3-) oder nicht benachbarten (1,3-) Kohlenstoffatomen gebundene Reste R^{5A} und/oder R^{5B} miteinander verknüpft sind und zusammen eine -(CH₂)ₚ-Brücke bilden, worin
   p die Zahl 1, 2, 3 oder 4 bedeutet,
      wobei im Falle, dass die Gruppe -CR^{5A}R^{5B}- mehrfach auftritt, die einzelnen Bedeutungen von R^{5A} und R^{5B} jeweils gleich oder verschieden sein können,
   oder
L¹ für eine Gruppe der Formel 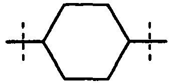 steht,
L² für eine Gruppe der Formel *-CR^{6A}R^{6B}-(CR^{7A}R^{7B})_{q}- oder *-CR⁶AR^{6B}-CR^{7A}R^{7B}-O- steht, worin
   * die Verknüpfungsstelle mit dem N-Atom der Amid-Gruppierung bedeutet,
   q die Zahl 0, 1 oder 2 bedeutet,
   R^{6A} Wasserstoff oder (C₁-C₄)-Alkyl bedeutet,
   R^{6B} Wasserstoff, (C₁-C₄)-Alkyl, Trifluormethyl, (C₃-C₆)-Cycloalkyl oder Phenyl, das bis zu zweifach, gleich oder verschieden, mit Halogen, (C₁-C₄)-Alkyl und/oder Trifluormethyl substituiert sein kann, oder einen Rest der Formel -C(=O)-OR¹⁶ oder -C(=O)-NR¹⁷R¹⁸ bedeutet, worin
      R¹⁶, R¹⁷ und R¹⁸ unabhängig voneinander Wasserstoff, (C₁-C₄)-Alkyl oder (C₃-C₆)-Cycloalkyl darstellen
         oder
      R¹⁷ und R¹⁸ zusammen mit dem Stickstoffatom, an das sie gebunden sind, einen 4-bis 6-gliedrigen Heterocyclus bilden, welcher ein weiteres Heteroatom aus der Reihe N, O und S enthalten und bis zu zweifach, gleich oder verschieden, mit (C₁-C₄)-Alkyl, Hydroxy und/oder (C₁-C₄)-Alkoxy substituiert sein kann,
      oder
   R^{6A} und R^{6B} miteinander verknüpft sind und zusammen eine -(CH₂)ᵣ-Brücke bilden, worin
      r die Zahl 2, 3, 4 oder 5 bedeutet
         und eine CH₂-Gruppe der Brücke gegen -O-, -S- oder >N-R¹⁹, worin
         R¹⁹ Wasserstoff oder (C₁-C₄)-Alkyl darstellt,
         ausgetauscht sein kann,
   R^{7A} Wasserstoff, Fluor, (C₁-C₄)-Alkyl oder (C₁-C₄)-Alkoxy bedeutet,
   R^{7B} Wasserstoff, Fluor, (C₁-C₄)-Alkyl, Hydroxy-(C₁-C₄)-alkyl oder einen Rest der Formel -OR²⁰, -NR²¹R²², -C(=O)-OR²³ oder -C(=O)-NR²⁴R²⁵ bedeutet, worin
      R²⁰, R²¹, R²², R²³, R²⁴ und R²⁵ unabhängig voneinander Wasserstoff, (C₁-C₄)-Alkyl oder (C₃-C₆)-Cycloalkyl darstellen
         oder
      R²¹ und R²² sowie R¹⁴ und R²⁵ jeweils paarweise zusammen mit dem Stickstoffatom, an das sie gebunden sind, einen 4- bis 6-gliedrigen Heterocyclus bilden, welcher ein weiteres Heteroatom aus der Reihe N, O und S enthalten und bis zu zweifach, gleich oder verschieden, mit (C₁-C₄)-Alkyl, Hydroxy und/oder (C₁-C₄)-Alkoxy substituiert sein kann,
      oder
   R^{7A} und R^{7B} zusammen eine Oxo-Gruppe bilden
      oder
   R^{7A} und R^{7B} miteinander verknüpft sind und zusammen eine -(CH₂)ₛ-Brücke bilden, worin
      s die Zahl 2, 3, 4 oder 5 bedeutet
      und eine CH₂-Gruppe der Brücke gegen -O-, -S- oder >N-R²⁶, worin
      R²⁶ Wasserstoff oder (C₁-C₄)-Alkyl darstellt,
      ausgetauscht sein kann,
      wobei im Falle, dass die Gruppe -CR^{7A}R^{7B}- mehrfach auftritt, die einzelnen Bedeutungen von R^{7A} und R^{7B} jeweils gleich oder verschieden sein können,
      oder
L² für eine Gruppe der Formel 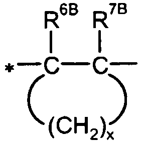 steht, worin
   * die Verknüpfungsstelle mit dem N-Atom der Amid-Gruppierung bedeutet,
   x die Zahl 1, 2, 3 oder 4 bedeutet,
      wobei eine CH₂-Gruppe des Rings gegen -O-, -S- oder >N-R²⁷, worin
      R²⁷ Wasserstoff oder (C₁-C₄)-Alkyl darstellt,
      ausgetauscht sein kann,
      und
      R^{6B} und R^{7B} jeweils die oben angegebenen Bedeutungen haben,
R³ für Phenyl, Naphthyl oder 5- bis 10-gliedriges Heteroaryl mit bis zu drei Heteroatomen aus der Reihe N, O und/oder S steht, welche jeweils ein- bis dreifach, gleich oder verschieden, mit Resten ausgewählt aus der Reihe Halogen, Cyano, Nitro, (C₁-C₄)-Alkyl, Trifluormethyl, Hydroxy, (C₁-C₄)-Alkoxy, Trifluormethoxy, (C₁-C₄)-Alkylthio, (C₁-C₄)-Alkylsulfinyl, (C₁-C₄)-Alkylsulfonyl, Di-(C₁-C₄)-alkylamino und Phenyl substituiert sein können,
   wobei der letztgenannte Phenyl-Rest seinerseits bis zu zweifach, gleich oder verschieden, mit Halogen, Cyano, Nitro, (C₁-C₄)-Alkyl, (C₁-C₄)-Alkoxy, Trifluormethyl und/oder Trifluormethoxy substituiert sein kann,
   oder die Gruppierung
L²-R³ zusammen eine Gruppe der Formel 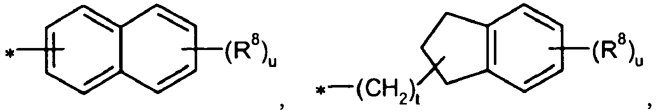 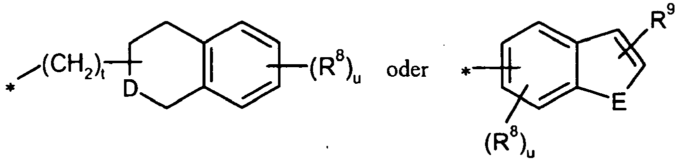 bildet, worin
   * die Verknüpfungsstelle mit dem N-Atom der Amid-Gruppierung bedeutet,
   D CH₂ oder O bedeutet,
   E NH, N-CH₃, O oder S bedeutet,
   t die Zahl 0 oder 1 bedeutet,
   R⁸ einen Substituenten ausgewählt aus der Reihe Halogen, Cyano, Nitro, (C₁-C₄)-Alkyl, (C₁-C₄)-Alkoxy, Trifluormethyl und Trifluormethoxy bedeutet,
   u die Zahl 0, 1 oder 2 bedeutet,
      wobei im Falle, dass der Substituent R⁸ mehrfach auftritt, seine Bedeutungen gleich oder verschieden sein können,
      und
   R⁹ Wasserstoff oder (C₁-C₄)-Alkyl bedeutet,
   sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die vorliegende Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Trisethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:

(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, *iso*-Butyl, *sec*.-Butyl, *tert*.-Butyl, 1-Ethylpropyl, n-Pentyl und n-Hexyl.

Hydroxy-(C₁-C₄)-alkyl steht im Rahmen der Erfindung für einen geradkettigen oder verzweigten Alkylrest mit 1 bis 4 Kohlenstoffatomen, der in der Kette oder endständig eine Hydroxy-Gruppe als Substituenten trägt. Beispielhaft und vorzugsweise seien genannt: Hydroxymethyl, 1-Hydroxyethyl, 2-Hydroxyethyl, 1-Hydroxy-1-methylethyl, 1,1-Dimethyl-2-hydroxyethyl, 1-Hydroxypropyl, 2-Hydroxypropyl, 3-Hydroxypropyl, 1-Hydroxy-2-methylpropyl, 2-Hydroxy-1-methylpropyl, 2-Hydroxy-2-methylpropyl, 1-Hydroxybutyl, 2-Hydroxybutyl, 3- Hydroxybutyl und 4-Hydroxybutyl.

Eine Oxo-Gruppe steht im Rahmen der Erfindung für ein Sauerstoffatom, das über eine Doppelbindung an ein Kohlenstoffatom gebunden ist.

(C₂-C₆)-Alkenyl und (C₂-C₄)-Alkenyl stehen im Rahmen der Erfindung für einen geradkettigen oder verzweigten Alkenylrest mit 2 bis 6 bzw. 2 bis 4 Kohlenstoffatomen und einer Doppelbindung. Bevorzugt ist ein geradkettiger oder verzweigter Alkenylrest mit 2 bis 4 Kohlenstoffatomen. Beispielhaft und vorzugsweise seien genannt: Vinyl, Allyl, n-Prop-1-en-1-yl, Isopropenyl, 2-Methyl-2-propen-1-yl, n-But-1-en-1-yl und n-But-2-en-1-yl.

(C₁-C₆)-Alkinyl und (C₂-C₄)-Alkinyl stehen im Rahmen der Erfindung für einen geradkettigen oder verzweigten Alkinylrest mit 2 bis 6 bzw. 2 bis 4 Kohlenstoffatomen und einer Dreifachbindung. Bevorzugt ist ein geradkettiger oder verzweigter Alkinylrest mit 2 bis 4 Kohlenstoffatomen. Beispielhaft und vorzugsweise seien genannt: Ethinyl, n-Prop-1-in-1-yl, n-Prop-2-in-1-yl, n-But-2-in-1-yl und n-But₋3-in-1-yl.

(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy, n-Butoxy, tert.-Butoxy, n-Pentoxy und n-Hexoxy.

(C₁-C₄)-Alkoxycarbonyl steht im Rahmen der Erfindung für einen geradkettigen oder verzweigten Alkoxyrest mit 1 bis 4 Kohlenstoffatomen, der über eine Carbonylgruppe verknüpft ist. Beispielhaft und vorzugsweise seien genannt: Methoxycarbonyl, Ethoxycarbonyl, *n*-Propoxycarbonyl, Isopropoxycarbonyl, *n*-Butoxycarbonyl und *tert*.-Butoxycarbonyl.

Mono-(C₁-C₄)-alkylamino steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, n-Propylamino, Isopropylamino, n-Butylamino und *tert*.-Butylamino.

Di-(C₁-C₄)-alkylamino steht im Rahmen der Erfmdung für eine Amino-Gruppe mit zwei gleichen oder verschiedenen geradkettigen oder verzweigten Alkylsubstituenten, die jeweils 1 bis 4 Kohlenstoffatome aufweisen. Beispielhaft und vorzugsweise seien genannt: *N*,*N*-Dimethylamino, *N,N-*Diethylamino, *N*-Ethyl-*N*-methylamino, *N*-Methyl-*N*-n-propylamino, *N*-Isopropyl-*N*-n-propylamino, *N*,*N*-Diisopropylamino, *N*-n-Butyl-*N*-methylamino und *N*-*tert*.-Butyl-*N*-methylamino.

Mono- bzw. Di-(C₁-C₄)₋alkylaminocarbonyl steht im Rahmen der Erfindung für eine Amino-Gruppe, die über eine Carbonylgruppe verknüpft ist und die einen geradkettigen oder verzweigten bzw. zwei gleiche oder verschiedene geradkettige oder verzweigte Alkylsubstituenten mit jeweils 1 bis 4 Kohlenstoffatomen aufweist. Beispielhaft und vorzugsweise seien genannt: Methylaminocarbonyl, Ethylaminocarbonyl, *n*-Propylaminocarbonyl, Isopropylaminocarbonyl, *n*-Butylaminocarbonyl, *tert*.-Butylaminocarbonyl, *N*,*N*-Dimethylaminocarbonyl, *N,N-*Diethylaminocarbonyl, *N-*Ethyl-*N*-methylaminocarbonyl, *N*-Methyl-*N*-n-propylaminocarbonyl, *N*-*n*-Butyl-*N*-methylaminocarbonyl und *N*-*tert*.-Butyl-*N*-methylaminocarbonyl.

(C₁-C₄-Alkylthio steht im Rahmen der Erfindung für eine Thio-Gruppe mit einem geradkettigen oder verzweigten Alkylsubstituenten, der 1 bis 4 Kohlenstoffatome aufweist. Beispielhaft und vorzugsweise seien genannt: Methylthio, Ethylthio, n-Propylthio, Isopropylthio, n-Butylthio und *tert.-*Butylthio.

(C₁-C₄)-Alkylsulfinyl steht im Rahmen der Erfindung für einen geradkettigen oder verzweigten Alkylsulfinyl-Rest mit 1 bis 4 Kohlenstoffatomen. Beispielhaft und vorzugsweise seien genannt: Methylsulfinyl, Ethylsulfinyl, n-Propylsulfinyl, Isopropylsulfinyl, n-Butylsulfinyl und *tert*.-Butylsulfinyl.

(C₁-C₄)-Alkylsulfonyl steht im Rahmen der Erfindung für einen geradkettigen oder verzweigten Alkylsulfonyl-Rest mit 1 bis 4 Kohlenstoffatomen. Beispielhaft und vorzugsweise seien genannt: Methylsulfonyl, Ethylsulfonyl, n-Propylsulfonyl, Isopropylsulfonyl, n-Butylsulfonyl und *tert.-*Butylsulfonyl.

(C₃-C₇)₋Cycloalkyl und (C₃-C₆)-Cycloalkyl stehen im Rahmen der Erfindung für eine monocyclische, gesättigte Cycloalkylgruppe mit 3 bis 7 bzw. 3 bis 6 Kohlenstoffatomen. Bevorzugt ist ein Cycloalkylrest mit 3 bis 6 Kohlenstoffatomen. Beispielhaft und vorzugsweise seien genannt: Cyclopropyl, Cyclobutyl, Cyclopentyl, Cyclohexyl und Cycloheptyl.

Ein 4- bis 7-gliedriger bzw. ein 4- bis 6-gliedriger Heterocyclus steht im Rahmen der Erfindung für einen monocyclischen, gesättigten Heterocyclus mit insgesamt 4 bis 7 bzw. 4 bis 6 Ringatomen, der ein Ring-Stickstoffatom enthält, über dieses verknüpft ist und ein weiteres Ring-Heteroatom aus der Reihe N, O und S enthalten kann. Beispielhaft seien genannt: Azetidinyl, Pyrrolidinyl, Pyrazolidinyl, Piperidinyl, Piperazinyl, Morpholinyl, Thiomorpholinyl, Hexahydroazepinyl und Hexahydro-1,4-diazepinyl. Bevorzugt ist ein 4- bis 6-gliedriger Heterocyclus, der neben dem Ring-Stickstoffatom ein weiteres Ring-Heteroatom aus der Reihe N und O enthalten kann. Besonders bevorzugt sind Pyrrolidinyl, Piperidinyl, Piperazinyl und Morpholinyl.

5- bis 10-gliedrige Heteroaryl steht im Rahmen der Erfindung für einen mono- oder gegebenenfalls bicyclischen aromatischen Heterocyclus (Heteroaromaten) mit insgesamt 5 bis 10 Ringatomen, der bis zu drei Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl, Triazinyl, Benzofuranyl, Benzothienyl, Benzimidazolyl, Benzoxazolyl, Benzothiazolyl, Benzotriazolyl, Indolyl, Indazolyl, Chinolinyl, Isochinolinyl, Naphthyridinyl, Chinazolinyl, Chinoxalinyl, Phthalazinyl, Pyrazolo[3,4-b]pyridinyl. Bevorzugt sind mono- oder gegebenenfalls bicyclische 5- bis 10-gliedrige Heteroaryl-Reste mit bis zu zwei Heteroatomen aus der Reihe N, O und/oder S. Besonders bevorzugt sind monocyclische 5- oder 6-gliedrige Heteroaryl-Reste mit bis zu zwei Heteroatomen aus der Reihe N, O und/oder S wie beispielsweise Furyl, Thienyl, Thiazolyl, Oxazolyl, Isothiazolyl, Isoxazolyl, Pyrazolyl, Imidazolyl, Pyridyl, Pyrimidinyl, Pyridazinyl, Pyrazinyl.

Halogen schließt im Rahmen der Erfindung Fluor, Chlor, Brom und Iod ein. Bevorzugt sind Fluor oder Chlor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt im Rahmen der vorliegenden Erfindung sind Verbindungen der Formel (I), in welcher
A für N oder C-R⁴ steht, worin
   R⁴ Wasserstoff oder (C₁-C₄)-Alkyl bedeutet,
R¹ für (C₁-C₆)-Alkyl, das mit Hydroxy, (C₁-C₆)-Alkoxy, (C₃-C₇)-Cycloalkyl oder Phenyl substituiert sein kann, oder für (C₃-C₇)-Cycloalkyl steht,
   wobei die genannten Cycloalkyl-Reste ihrerseits bis zu zweifach, gleich oder verschieden, mit (C₁-C₄)-Alkyl, (C₁-C₄)-Alkoxy, Hydroxy, Amino und/oder Oxo
   und
   der Phenyl-Rest bis zu dreifach, gleich oder verschieden, mit Halogen, Cyano, Nitro, (C₁-C₄)-Alkyl, (C₁-C₄)-Alkoxy, Trifluormethyl und/oder Trifluormethoxy
   substituiert sein können,
R² für Phenyl, Naphthyl, Thienyl, Benzothienyl, Furyl oder Benzofuryl steht, welche jeweils bis zu dreifach, gleich oder verschieden, mit Halogen, Cyano, Nitro, (C₁-C₄)-Alkyl, (C₁-C₄)-Alkoxy, Trifluormethyl, Trifluormethoxy und/oder Phenyl substituiert sein können,
   wobei der letztgenannte Phenyl-Rest seinerseits bis zu zweifach, gleich oder verschieden, mit Halogen, Cyano, Nitro, (C₁-C₄)-Alkyl, (C₁-C₄)-Alkoxy, Trifluormethyl und/oder Trifluormethoxy substituiert sein kann,
L¹ für eine Gruppe der Formel -(CR^{5A}R^{5B})ₘ- steht, worin
   m die Zahl 1, 2 oder 3 bedeutet
      und
   R^{5A} und R^{5B} unabhängig voneinander Wasserstoff oder (C₁-C₄)-Alkyl bedeuten
      oder
      zwei an demselben Kohlenstoffatom gebundene Reste R^{5A} und R^{5B} miteinander verknüpft sind und zusammen eine -(CH₂)ₙ-Brücke bilden, worin
      n die Zahl 2, 3, 4 oder 5 bedeutet,
      oder im Falle, dass m für die Zahl 2 oder 3 steht,
      zwei an benachbarten (1,2- oder 2,3-) oder nicht benachbarten (1,3-) Kohlenstoffatomen gebundene Reste R^{5A} und/oder R^{5B} miteinander verknüpft sind und zusammen eine -(CH₂)ₚ-Brücke bilden, worin
      p die Zahl 1, 2, 3 oder 4 bedeutet,
         wobei im Falle, dass die Gruppe -CR^{5A}R^{5B}- mehrfach auftritt, die einzelnen Bedeutungen von R^{5A} und R^{5B} jeweils gleich oder verschieden sein können,
      oder
L¹ für eine Gruppe der Formel 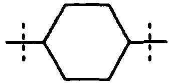 steht,
L² für eine Gruppe der Formel *-CR^{6A}R^{6B}-(CR^{7A}R^{7B})_{q}- oder *-CR^{6A}R^{6B}-CR^{7A}R^{7B}-O- steht, worin
   * die Verknüpfungsstelle mit dem N-Atom der Amid-Gruppierung bedeutet,
   q die Zahl 0, 1 oder 2 bedeutet,
   R^{6A} Wasserstoff oder (C₁-C₄)-Alkyl bedeutet,
   R^{6B} Wasserstoff, (C₁-C₄)-Alkyl, Trifluormethyl, (C₃-C₆)-Cycloalkyl oder Phenyl, das bis zu zweifach, gleich oder verschieden, mit Halogen, (C₁-C₄)-Alkyl und/oder Trifluormethyl substituiert sein kann, bedeutet
      oder
   R^{6A} und R^{6B} miteinander verknüpft sind und zusammen eine -(CH₂)ᵣ-Brücke bilden, worin
      r die Zahl 2, 3, 4 oder 5 bedeutet,
      und
   R^{7A} und R^{7B} unabhängig voneinander Wasserstoff oder (C₁-C₄)-Alkyl bedeuten,
      wobei im Falle, dass die Gruppe -CR^{7A}R^{7B}- mehrfach auftritt, die einzelnen Bedeutungen von R^{7A} und R^{7B} jeweils gleich oder verschieden sein können,
R³ für Phenyl, Naphthyl oder 5- bis 10-gliedriges Heteroaryl mit bis zu drei Heteroatomen aus der Reihe N, O und/oder S steht, welche jeweils bis zu dreifach, gleich oder verschieden, mit Halogen, Cyano, Nitro, (C₁-C₄)-Alkyl, (C₁-C₄)-Alkoxy, Trifluormethyl, Trifluormethoxy, Di-(C₁-C₄)-alkylamino und/oder Phenyl substituiert sein können,
   wobei der letztgenannte Phenyl-Rest seinerseits bis zu zweifach, gleich oder verschieden, mit Halogen, Cyano, Nitro, (C₁-C₄)-Alkyl, (C₁-C₄)-Alkoxy, Trifluormethyl und/oder Trifluormethoxy substituiert sein kann,
   oder die Gruppierung
L²-R³ zusammen eine Gruppe der Formel 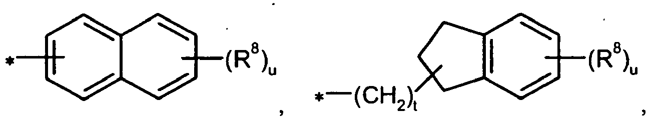 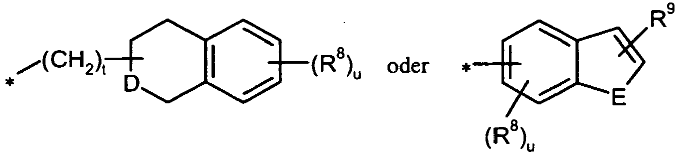 bildet, worin
   * die Verknüpfungsstelle mit dem N-Atom der Amid-Gruppierung bedeutet,
   D CH₂ oder O bedeutet,
   E NH, N-CH₃, O oder S bedeutet,
   t die Zahl 0 oder 1 bedeutet,
   R⁸ einen Substituenten ausgewählt aus der Reihe Halogen, Cyano, Nitro, (C₁-C₄)-Alkyl, (C₁-C₄)-Alkoxy, Trifluormethyl und Trifluormethoxy bedeutet,
   u die Zahl 0, 1 oder 2 bedeutet,
      wobei im Falle, dass der Substituent R⁸ mehrfach auftritt, seine Bedeutungen gleich oder verschieden sein können,
   und
   R⁹ Wasserstoff oder (C₁-C₄)-Alkyl bedeutet,
   sowie ihre Salze, Solvate und Solvate der Salze.
   Gleichfalls bevorzugt im Rahmen der vorliegenden Erfindung sind Verbindungen der Formel (I), in welcher
A für N oder C-R⁴ steht, worin
   R⁴ Wasserstoff oder (C₁-C₄)-Alkyl bedeutet,
R¹ für (C₁-C₆)-Alkyl steht, welches ein- bis dreifach, gleich oder verschieden, mit Resten ausgewählt aus der Reihe Fluor, Chlor, Cyano, Oxo, Trifluormethyl, (C₃-C₆)-Cycloalkyl, Phenyl, -OR¹⁰, -NR¹¹R¹², -C(=O)-OR¹³ und -C(=O)-NR¹⁴R¹⁵ substituiert sein kann, worin
   (i) (C₃-C₆)-Cycloalkyl bis zu zweifach, gleich oder verschieden, mit (C₁-C₄)-Alkyl, Oxo, Hydroxy, (C₁-C₄)-Alkoxy und/oder Amino substituiert sein kann,
   (ii) Phenyl bis zu dreifach, gleich oder verschieden, mit Resten ausgewählt aus der Reihe Fluor, Chlor, Cyano, (C₁-C₄)-Alkyl, Trifluormethyl, Hydroxy, Hydroxymethyl, (C₁-C₄)-Alkoxy, Trifluormethoxy, (C₁-C₄)-Alkoxymethyl, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl substituiert sein kann,
   (iii) R¹⁰, R¹¹, R¹², R¹³, R¹⁴ und R¹⁵ unabhängig voneinander bei jedem einzelnen ¹Auftreten Wasserstoff, (C₁-C₄)-Alkyl oder (C₃-C₆)-Cycloalkyl bedeuten, wobei
      (C₁-C₄)-Alkyl seinerseits bis zu zweifach, gleich oder verschieden, mit Amino, Hydroxy, (C₁-C₄)-Alkoxy, Hydroxycarbonyl und/oder (C₁-C₄)-Alkoxycarbonyl substituiert sein kann
      und
      (C₃-C₆)-Cycloalkyl seinerseits bis zu zweifach, gleich oder verschieden, mit (C₁-C₄)-Alkyl, Oxo, Hydroxy, (C₁-C₄)-Alkoxy und/oder Amino substituiert sein kann,
      und/oder
   (iv) R¹¹ und R¹² sowie R¹⁴ und R¹⁵ jeweils paarweise zusammen mit dem Stickstoffatom, an das sie gebunden sind, einen 4- bis 6-gliedrigen Heterocyclus bilden, welcher ein weiteres Heteroatom aus der Reihe N und O enthalten und bis zu zweifach, gleich oder verschieden, mit (C₁-C₄)-Alkyl, Oxo, Hydroxy, (C₁-C₄)-Alkoxy und/oder Amino substituiert sein kann,
   oder
R¹ für (C₂-C₆)-Alkenyl, welches mit Hydroxy, (C₁-C₄)-Alkoxy, Hydroxycarbonyl oder (C₁-C₄)-Alkoxycarbonyl substituiert sein kann,
   oder
   für (C₃-C₆)-Cycloalkyl, welches bis zu zweifach, gleich oder verschieden, mit (C₁-C₄)-Alkyl, (C₁-C₄)-Alkoxy, Hydroxy, Amino und/oder Oxo substituiert sein kann,
   steht,
R² für Phenyl oder Thienyl steht, welche ein- bis dreifach, gleich oder verschieden, mit Resten ausgewählt aus der Reihe Fluor, Chlor, Cyano, (C₁-C₄)-Alkyl, Trifluormethyl, Hydroxy, (C₁-C₄)-Alkoxy, Trifluormethoxy und Phenyl substituiert sein können,
   wobei der letztgenannte Phenyl-Rest seinerseits bis zu zweifach, gleich oder verschieden, mit Resten ausgewählt aus der Reihe Fluor, Chlor, Cyano, (C₁-C₄)-Alkyl, Trifluormethyl, (C₁-C₄)-Alkoxy und Trifluormethoxy substituiert sein kann,
L¹ für eine Gruppe der Formel -CR^{5A}R^{5B}- steht, worin
   - R^{5A} und R^{5B} unabhängig voneinander Wasserstoff oder (C₁-C₄)-Alkyl bedeuten,
L² für eine Gruppe der Formel *-CR^{6A}R^{6B}-(CR^{7A}R^{7B})_{q}- steht, worin
   * die Verknüpfungsstelle mit dem N-Atom der Amid-Gruppierung bedeutet,
   q die Zahl 0 oder 1 bedeutet,
   R^{6A} Wasserstoff oder (C₁-C₄)-Alkyl bedeutet,
   R^{6B} Wasserstoff, (C₁-C₄)-Alkyl, Trifluormethyl, (C₃-C₆)-Cycloalkyl oder Phenyl, das bis zu zweifach, gleich oder verschieden, mit Fluor, Chlor, (C₁-C₄)-Alkyl und/oder Trifluormethyl substituiert sein kann, oder einen Rest der Formel -C(=O)-OR¹⁶ oder -C(=O)-NR¹⁷R¹⁸ bedeutet, worin
      R¹⁶, R¹⁷ und R¹⁸ unabhängig voneinander Wasserstoff, (C₁-C₄)-Alkyl oder (C₃-C₆)-Cycloalkyl darstellen
         oder
      R¹⁷ und R¹⁸ zusammen mit dem Stickstoffatom, an das sie gebunden sind, einen 4-bis 6-gliedrigen Heterocyclus bilden, welcher ein weiteres Heteroatom aus der Reihe N und O enthalten und bis zu zweifach, gleich oder verschieden, mit (C₁-C₄)-Alkyl, Hydroxy und/oder (C₁-C₄)-Alkoxy substituiert sein kann,
      oder
   R^{6A} und R^{6B} miteinander verknüpft sind und zusammen eine -(CH₂)ᵣ-Brücke bilden, worin
      r die Zahl 2, 3, 4 oder 5 bedeutet
      und eine CH₂-Gruppe der Brücke gegen -O- ausgetauscht sein kann,
   R^{7A} Wasserstoff, Fluor oder (C₁-C₄)-Alkyl bedeutet,
   R^{7B} Wasserstoff, Fluor, (C₁-C₄)-Alkyl, Hydroxy-(C₁-C₄)-alkyl oder einen Rest der Formel-OR²⁰, -NR²¹R²², -C(=O)-OR²³ oder -C(-O)-NR²⁴R²⁵ bedeutet, worin
      R²⁰, R²¹, R²², R²³, R²⁴ und R²⁵ unabhängig voneinander Wasserstoff, (C₁-C₄)-Alkyl oder (C₃-C₆)-Cycloalkyl darstellen
         oder
      R²¹ und R²² sowie R²⁴ und R²⁵ jeweils paarweise zusammen mit dem Stickstoffatom, an das sie gebunden sind, einen 4- bis 6-gliedrigen Heterocyclus bilden, welcher ein weiteres Heteroatom aus der Reihe N und O enthalten und bis zu zweifach, gleich oder verschieden, mit (C₁-C₄)-Alkyl, Hydroxy und/oder (C₁-C₄)-Alkoxy substituiert sein kann,
      oder
   R^{7A} und R^{7B} zusammen eine Oxo-Gruppe bilden
      oder
   R^{7A} und R^{7B} miteinander verknüpft sind und zusammen eine -(CH₂)ₛ-Brücke bilden, worin
      s die Zahl 2, 3, 4 oder 5 bedeutet
      und eine CH₂-Gruppe der Brücke gegen -O- ausgetauscht sein kann,
   oder
L² für eine Gruppe der Formel 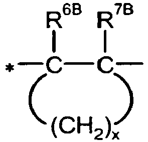 steht, worin
   * die Verknüpfungsstelle mit dem N-Atom der Amid-Gruppierung bedeutet,
   x die Zahl 1, 2, 3 oder 4 bedeutet,
      wobei eine CH₂-Gruppe des Rings gegen -O- ausgetauscht sein kann,
      und
   R^{6B} und R^{7B} jeweils die oben angegebenen Bedeutungen haben,
   und
R³ für Phenyl, Naphthyl oder 5- bis 10-gliedriges Heteroaryl mit bis zu zwei Heteroatomen aus der Reihe N, O und/oder S steht, welche jeweils ein- bis dreifach, gleich oder verschieden, mit Resten ausgewählt aus der Reihe Fluor, Chlor, Cyano, (C₁-C₄)-Alkyl, Trifluormethyl, Hydroxy, (C₁-C₄)-Alkoxy, Trifluormethoxy, (C₁-C₄)-Alkylthio, (C₁-C₄)-Alkylsulfonyl, Di-(C₁-C₄)-alkylamino und Phenyl substituiert sein können,
   wobei der letztgenannte Phenyl-Rest seinerseits bis zu zweifach, gleich oder verschieden, mit Fluor, Chlor, Cyano, (C₁-C₄)-Alkyl, (C₁-C₄)-Alkoxy, Trifluormethyl und/oder Trifluormethoxy substituiert sein kann,
   sowie ihre Salze, Solvate und Solvate der Salze.

Besonders bevorzugt im Rahmen der vorliegenden Erfindung sind Verbindungen der Formel (I), in welcher
A für N oder C-R⁴ steht, worin
   R⁴ Wasserstoff oder (C₁-C₄)-Alkyl bedeutet,
R¹ für (C₁-C₆)-Alkyl, das mit Hydroxy, (C₁-C₄)-Alkoxy, (C₃-C₆)-Cycloalkyl oder Phenyl substituiert sein kann, oder für (C₃-C₆)-Cycloalkyl steht, wobei die genannten Cycloalkyl-Reste ihrerseits bis zu zweifach, gleich oder verschieden, mit (C₁-C₄)-Alkyl, (C₁-C₄)-Alkoxy, Hydroxy und/oder Amino
   und
   der Phenyl-Rest bis zu zweifach, gleich oder verschieden, mit Halogen, Cyano, Nitro, (C₁-C₄)-Alkyl, (C₁-C₄)-Alkoxy, Trifluormethyl und/oder Trifluormethoxy
   substituiert sein können,
R² für Phenyl, Naphthyl, Thienyl oder Benzothienyl steht, welche jeweils bis zu dreifach, gleich oder verschieden, mit Halogen, Cyano, Nitro, (C₁-C₄)-Alkyl, (C₁-C₄)-Alkoxy, Trifluormethyl, Trifluormethoxy und/oder Phenyl substituiert sein können,
   wobei der letztgenannte Phenyl-Rest seinerseits bis zu zweifach, gleich oder verschieden, mit Halogen, Cyano, Nitro, (C₁-C₄)-Alkyl, (C₁-C₄)-Alkoxy, Trifluormethyl und/oder Trifluormethoxy substituiert sein kann,
L¹ für eine Gruppe der Formel -(CR^{5A}R^{5B})ₘ- steht, worin
   m die Zahl 1, 2 oder 3 bedeutet
      und
   R^{5A} und R^{5B} unabhängig voneinander Wasserstoff oder Methyl bedeuten
      oder
      zwei an demselben Kohlenstoffatom gebundene Reste R^{5A} und R^{5B} miteinander verknüpft sind und zusammen eine -(CH₂)ₙ-Brücke bilden, worin
      n die Zahl 2, 3, 4 oder 5 bedeutet,
         oder im Falle, dass m für die Zahl 2 oder 3 steht,
         zwei an benachbarten (1,2- oder 2,3-) oder nicht benachbarten (1,3-) Kohlenstoffatomen gebundene Reste R^{5A} und/oder R^{5B} miteinander verknüpft sind und zusammen eine -(CH₂)ₚ-Brücke bilden, worin
      p die Zahl 1, 2, 3 oder 4 bedeutet,
      wobei im Falle, dass die Gruppe -CR^{5A}R^{5B}- mehrfach auftritt, die einzelnen Bedeutungen von R^{5A} und R^{5B} jeweils gleich oder verschieden sein können,
   oder
L¹ für eine Gruppe der Formel 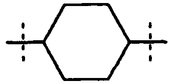 steht,
L² für eine Gruppe der Formel *-CR^{6A}R^{6B}-(CH₂)_{q}- steht, worin
   * die Verknüpfungsstelle mit dem N-Atom der Amid-Gruppierung bedeutet,
   q die Zahl 0 oder 1 bedeutet,
   R^{6A} Wasserstoff oder Methyl bedeutet,
   R^{6B} Wasserstoff, (C₁-C₄)-Alkyl, Trifluormethyl oder (C₃-C₆)-Cycloalkyl bedeutet oder
   R^{6A} und R^{6B} miteinander verknüpft sind und zusammen eine -(CH₂)ᵣ-Brücke bilden, worin
      r die Zahl 2, 3, 4 oder 5 bedeutet,
      und
R³ für Phenyl, Naphthyl oder 5- bis 10-gliedriges Heteroaryl mit bis zu zwei Heteroatomen aus der Reihe N, O und/oder S steht, welche jeweils bis zu dreifach, gleich oder verschieden, mit Halogen, Cyano, Nitro, (C₁-C₄)-Alkyl, (C₁-C₄)-Alkoxy, Trifluormethyl, Trifluormethoxy und/oder Phenyl substituiert sein können,
   wobei der letztgenannte Phenyl-Rest seinerseits bis zu zweifach, gleich oder verschieden, mit Halogen, Cyano, Nitro, (C₁-C₄)-Alkyl, (C₁-C₄)-Alkoxy, Trifluormethyl und/oder Trifluormethoxy substituiert sein kann,
   sowie ihre Salze, Solvate und Solvate der Salze.

Gleichfalls besonders bevorzugt im Rahmen der vorliegenden Erfindung sind Verbindungen der Formel (I), in welcher
A für N oder C-R⁴ steht; worin
   R⁴ Wasserstoff oder Methyl bedeutet,
R¹ für (C₁-C₆)-Alkyl steht, welches ein- bis zweifach, gleich oder verschieden, mit Resten ausgewählt aus der Reihe Fluor, Oxo, Trifluormethyl, (C₃-C₆)-Cycloalkyl, Phenyl, -OR¹⁰, -C(=O)-OR¹³ und -C(=O)-NR¹⁴R¹⁵ substituiert sein kann, worin
   (i) (C₃-C₆)-Cycloalkyl bis zu zweifach, gleich oder verschieden, mit (C₁-C₄)-Alkyl, Hydroxy und/oder (C₁-C₄)-Alkoxy substituiert sein kann,
   (ii) Phenyl bis zu zweifach, gleich oder verschieden, mit Resten ausgewählt aus der Reihe Fluor, Chlor, Cyano, (C₁-C₄)-Alkyl, Trifluormethyl, Hydroxymethyl, (C₁-C₄)-Alkoxy, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl substituiert sein kann,
      und
   (*iii*) R¹⁰, R¹³, R¹⁴ und R¹⁵ unabhängig voneinander bei jedem einzelnen Auftreten Wasserstoff, (C₁-C₄)-Alkyl oder (C₃-C₆)-Cycloalkyl bedeuten, wobei
      (C₁-C₄)-Alkyl seinerseits bis zu zweifach, gleich oder verschieden, mit Hydroxy, (C₁-C₄)-Alkoxy, Hydroxycarbonyl und/oder (C₁-C₄)-Alkoxycarbonyl substituiert sein kann
      und
      (C₃-C₆)-Cycloalkyl seinerseits bis zu zweifach, gleich oder verschieden, mit (C₁-C₄)-Alkyl, Hydroxy und/oder (C₁-C₄)-Alkoxy substituiert sein kann,
   oder
R¹ für (C₂-C₆)-Alkenyl, welches mit Hydroxycarbonyl oder (C₁-C₄)-Alkoxycarbonyl substituiert sein kann,
   oder
   für (C₃-C₆)-Cycloalkyl, welches bis zu zweifach, gleich oder verschieden, mit (C₁-C₄)-Alkyl, (C₁-C₄)-Alkoxy und/oder Hydroxy substituiert sein kann,
   steht,
R² für Phenyl oder Thienyl steht, welche ein- bis zweifach, gleich oder verschieden, mit Resten ausgewählt aus der Reihe Fluor, Chlor, (C₁-C₄)-Alkyl, Trifluormethyl, (C₁-C₄)-Alkoxy und Trifluormethoxy substituiert sein können,
L¹ für eine Gruppe der Formel -CR^{5A}R^{5B}- steht, worin
   R^{5A} und R^{5B} unabhängig voneinander Wasserstoff oder Methyl bedeuten,
L² für eine Gruppe der Formel *-CR^{6A}R^{6B}-(CR^{7A}R^{7B})_{q}- steht, worin
   * die Verknüpfungsstelle mit dem N-Atom der Amid-Gruppierung bedeutet,
   q die Zahl 0 oder 1 bedeutet,
   R^{6A} Wasserstoff oder Methyl bedeutet,
   R^{6B} Wasserstoff, Methyl, Trifluormethyl oder einen Rest der Formel -C(=O)-OR¹⁶ oder -C(=O)-NR¹⁷R¹⁸ bedeutet, worin
      R¹⁶, R¹⁷ und R¹⁸ unabhängig voneinander Wasserstoff, (C₁-C₄)-Alkyl oder (C₃-C₆)-Cycloalkyl darstellen
         oder
      R¹⁷ und R¹⁸ zusammen mit dem Stickstoffatom, an das sie gebunden sind, einen 4-bis 6-gliedrigen Heterocyclus bilden, welcher ein O-Atom als weiteres Heteroatom enthalten kann,
      oder
   R^{6A} und R^{6B} miteinander verknüpft sind und zusammen eine -(CH₂)ᵣ-Brücke bilden, worin
      r die Zahl 2, 3, 4 oder 5 bedeutet
      und eine CH₂-Gruppe der Brücke gegen -O- ausgetauscht sein kann,
   R^{7A} Wasserstoff, Fluor oder Methyl bedeutet,
   R^{7B} Wasserstoff, Fluor, Methyl oder einen Rest der Formel -C(=O)-OR²³ oder -C(=O)-NR²⁴R²⁵ bedeutet, worin
      R²³, R²⁴ und R²⁵ unabhängig voneinander Wasserstoff, (C₁-C₄)-Alkyl oder (C₃-C₆)-Cycloalkyl darstellen
         oder
      R²⁴ und R²⁵ zusammen mit dem Stickstoffatom, an das sie gebunden sind, einen 4-bis 6-gliedrigen Heterocyclus bilden, welcher ein O-Atom als weiteres Heteroatom enthalten kann,
      oder
   R^{7A} und R^{7B} zusammen eine Oxo-Gruppe bilden
      oder
   R^{7A} und R^{7B} miteinander verknüpft sind und zusammen eine -(CH₂)ₛ-Brücke bilden, worin
      s die Zahl 2, 3, 4 oder 5 bedeutet
      und eine CH₂-Gruppe der Brücke gegen -O- ausgetauscht sein kann,
   oder
L² für eine Gruppe der Formel 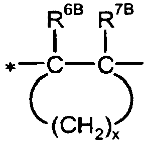 steht, worin
   * die Verknüpfungsstelle mit dem N-Atom der Amid-Gruppierung bedeutet,
   x die Zahl 1, 2, 3 oder 4 bedeutet,
      wobei eine CH₂-Gruppe des Rings gegen -O- ausgetauscht sein kann,
      und
      R^{6B} und R^{7B} jeweils die oben angegebenen Bedeutungen haben,
   und
R³ für Phenyl, Naphthyl, Pyridyl, Chinolinyl oder Isochinolinyl steht, welche jeweils ein- bis zweifach, gleich oder verschieden, mit Resten ausgewählt aus der Reihe Fluor, Chlor, Cyano, (C₁-C₄)-Alkyl, Trifluormethyl, (C₁-C₄)-Alkoxy, Trifluormethoxy, (C₁-C₄)-Alkylthio und Phenyl substituiert sein können,
   wobei der letztgenannte Phenyl-Rest seinerseits bis zu zweifach, gleich oder verschieden, mit Fluor, Chlor, Cyano, (C₁-C₄)-Alkyl, (C₁-C₄)-Alkoxy, Trifluormethyl und/oder Trifluormethoxy substituiert sein kann,
sowie ihre Salze, Solvate und Solvate der Salze.

Insbesondere bevorzugt im Rahmen der vorliegenden Erfindung sind Verbindungen der Formel (I), in welcher
A für N oder CH steht,
R¹ für (C₁-C₆)-Alkyl steht, welches ein- bis zweifach, gleich oder verschieden, mit Resten ausgewählt aus der Reihe Fluor, Oxo, Hydroxy, (C₁-C₄)-Alkoxy, Trifluormethyl, (C₃-C₆)-Cycloalkyl und Phenyl substituiert sein kann,
   wobei Phenyl seinerseits bis zu zweifach, gleich oder verschieden, mit Resten ausgewählt aus der Reihe Fluor, Chlor, Cyano, (C₁-C₄)-Alkyl, Trifluormethyl, Hydroxymethyl, (C₁-C₄)-Alkoxy, Trifluormethoxy, Hydroxycarbonyl, Aminocarbonyl und Di-(C₁-C₄)-alkylaminocarbonyl substituiert sein kann,
   oder
R¹ für (C₂-C₄)-Alkenyl oder (C₃-C₆)-Cycloalkyl steht,
R² für Phenyl oder Thienyl steht, welche ein- bis zweifach, gleich oder verschieden, mit Resten ausgewählt aus der Reihe Fluor, Chlor, Brom, (C₁-C₄)-Alkyl und (C₁-C₄)-Alkoxy substituiert sein können,
L¹ für -CH₂-, -CH(CH₃)- oder -CH₂CH₂- steht,
L² für eine Gruppe der Formel *-CR^{6A}R^{6B}-(CR^{7A}R^{7B})_{q}- steht, worin
   * die Verknüpfungsstelle mit dem N-Atom der Amid-Gruppierung bedeutet,
   q die Zahl 0 oder 1 bedeutet,
   R^{6A} Wasserstoff oder Methyl bedeutet,
   R^{6B} Wasserstoff, Methyl, Trifluormethyl oder einen Rest der Formel -C(=O)-NR¹⁷R¹⁸ bedeutet, worin
   R¹⁷ und R¹⁸ unabhängig voneinander Wasserstoff, (C₁-C₄)-Alkyl oder (C₃-C₆)-Cycloalkyl darstellen
      oder
   R¹⁷ und R¹⁸ zusammen mit dem Stickstoffatom, an das sie gebunden sind, einen 4-bis 6-gliedrigen Heterocyclus bilden, welcher ein O-Atom als weiteres Heteroatom enthalten kann,
   oder
R^{6A} und R^{6B} miteinander verknüpft sind und zusammen eine -(CH₂)ᵣ-Brücke bilden, worin r die Zahl 2, 3, 4 oder 5 bedeutet
   und eine CH₂-Gruppe der Brücke gegen -O- ausgetauscht sein kann,
R^{7A} Wasserstoff, Fluor oder Methyl bedeutet,
R^{7B} Wasserstoff, Fluor, Methyl oder einen Rest der Formel -C(=O)-OR²³ oder -C(=O)-NR²⁴R²⁵ bedeutet, worin
   R²³, R²⁴ und R²⁵ unabhängig voneinander Wasserstoff oder (C₁-C₄)-Alkyl darstellen
      oder
   R²⁴ und R²⁵ zusammen mit dem Stickstoffatom, an das sie gebunden sind, einen 4-bis 6-gliedrigen Heterocyclus bilden, welcher ein O-Atom als weiteres Heteroatom enthalten kann,
   oder
R^{7A} und R^{7B} zusammen eine Oxo-Gruppe bilden
   oder
R^{7A} und R^{7B} miteinander verknüpft sind und zusammen eine -(CH₂)ₛ-Brücke bilden, worin
   s die Zahl 2, 3, 4 oder 5 bedeutet
      und eine CH₂-Gruppe der Brücke gegen -O- ausgetauscht sein kann,
   oder
L² für eine Gruppe der Formel 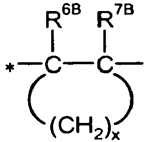 steht, worin
   * die Verknüpfungsstelle mit dem N-Atom der Amid-Gruppierung bedeutet,
   x die Zahl 1, 2, 3 oder 4 bedeutet
      und
   R^{6B} und R^{7B} jeweils die oben angegebenen Bedeutungen haben,
   und
R³ für Phenyl oder Pyridyl, welche ein- bis zweifach, gleich oder verschieden, mit Resten ausgewählt aus der Reihe Fluor, Chlor, (C₁-C₄)-Alkyl, Trifluormethyl, (C₁-C₄)-Alkoxy und Trifluormethoxy substituiert sein können, oder für Naphthyl steht,
   sowie ihre Salze, Solvate und Solvate der Salze.

Ganz besonders bevorzugt im Rahmen der vorliegenden Erfindung sind Verbindungen der Formel (I), in welcher
A für N oder CH steht,
R¹ für (C₁-C₆)-Alkyl steht, welches ein- bis zweifach, gleich oder verschieden, mit Resten ausgewählt aus der Reihe Fluor, Oxo, Hydroxy, Methoxy, Ethoxy, Trifluormethyl, Cyclopropyl und Phenyl substituiert sein kann,
   wobei Phenyl seinerseits bis zu zweifach, gleich oder verschieden, mit Resten ausgewählt aus der Reihe Fluor, Chlor, Cyano, Methyl, Hydroxymethyl, Methoxy, Hydroxycarbonyl, Aminocarbonyl und Dimethylaminocarbonyl substituiert sein kann,
   oder
R¹ für Vinyl, Allyl oder Cyclopropyl steht,
R² für Phenyl oder Thienyl steht, welche ein- bis zweifach, gleich oder verschieden, mit Resten ausgewählt aus der Reihe Fluor, Chlor, Methyl und Methoxy substituiert sein können,
L¹ für -CH₂- steht,
L² für eine Gruppe der Formel *-CR^{6A}R^{6B}-(CR^{7A}R^{7B})_{q}- steht, worin
   * die Verknüpfungsstelle mit dem N-Atom der Amid-Gruppierung bedeutet,
   q die Zahl 0 oder 1 bedeutet,
   R^{6A} Wasserstoff oder Methyl bedeutet,
   R^{6B} Wasserstoff, Methyl, Trifluormethyl oder einen Rest der Formel -C(=O)-NR¹⁷R¹⁸ bedeutet, worin
      R¹⁷ und R¹⁸ unabhängig voneinander Wasserstoff, Methyl, Ethyl oder Cyclopropyl darstellen
         oder
      R¹⁷ und R¹⁸ zusammen mit dem Stickstoffatom, an das sie gebunden sind, einen Azetidin-, Pyrrolidin-, Piperidin- oder Morpholin-Ring bilden,
      oder
   R^{6A} und R^{6B} miteinander verknüpft sind und zusammen mit dem Kohlenstoffatom, an das sie gebunden sind, eine Gruppe der Formel 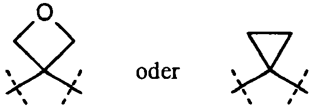 bilden,
   R^{7A} Wasserstoff, Fluor oder Methyl bedeutet,
   R^{7B} Wasserstoff, Fluor, Methyl oder einen Rest der Formel -C(=O)-OR²³ oder -C(=O)-NR²⁴R²⁵ bedeutet, worin
      R²³, R²⁴ und R²⁵ unabhängig voneinander Wasserstoff, Methyl oder Ethyl darstellen
         oder
      R²⁴ und R²⁵ zusammen mit dem Stickstoffatom, an das sie gebunden sind, einen Azetidin-, Pyrrolidin-, Piperidin- oder Morpholin-Ring bilden,
      oder
   R^{7A} und R^{7B} zusammen eine Oxo-Gruppe bilden
      oder
   R^{7A} und R^{7B} miteinander verknüpft sind und zusammen eine -(CH₂)ₛ-Brücke bilden, worin
      s die Zahl 2, 3, 4 oder 5 bedeutet
         und eine CH₂-Gruppe der Brücke gegen -O- ausgetauscht sein kann,
      oder
L² für eine Gruppe der Formel 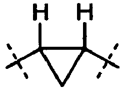 steht,
   und
R³ für Phenyl, das ein- oder zweifach, gleich oder verschieden, mit Fluor, Chlor, Trifluormethyl und/oder Trifluormethoxy substituiert sein kann, oder für 1-Naphthyl steht,
   sowie ihre Salze, Solvate und Solvate der Salze.

Ganz besonders bevorzugt im Rahmen der vorliegenden Erfindung sind auch Verbindungen der Formel (I), in welcher
A für N oder CH steht,
R¹ für (C₁-C₄)-Alkyl, 2-Methoxyethyl, Cyclopropyl, Cyclohexylmethyl, Benzyl oder 1-Phenethyl steht,
   wobei der Phenylring in den genannten Benzyl- und 1-Phenethyl-Resten mit Fluor, Chlor, Methyl, Trifluormethyl, Methoxy oder Trifluormethoxy substituiert sein kann,
R² für Phenyl oder Thienyl, welche jeweils ein- oder zweifach, gleich oder verschieden, mit Fluor, Chlor, Brom, Methyl und/oder Methoxy substituiert sind, steht,
L¹ für -CH₂-, -CH₂CH₂- oder -CH(CH₃)- steht,
L² für -CH₂-, -CH(CH₃)- oder -C(CH₃)₂- steht
   und
R³ für Phenyl, das ein- oder zweifach, gleich oder verschieden, mit Fluor, Chlor, Trifluormethyl und/oder Trifluormethoxy substituiert ist, oder für 1-Naphthyl steht,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man
[A] eine Verbindung der Formel (II) 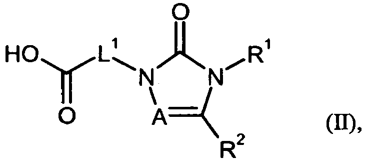 in welcher A, L¹, R¹ und R² jeweils die oben angegebenen Bedeutungen haben,
   in einem inerten Lösungsmittel unter Aktivierung der Carbonsäure-Funktion mit einer Verbindung der Formel (III)

   R³-L²-NH₂ (III),

   in welcher L² und R³ die oben angegebenen Bedeutungen haben,
   kuppelt
   oder
[B] eine Verbindung der Formel (IV) 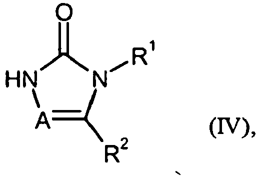 in welcher A, R¹ und R² jeweils die oben angegebenen Bedeutungen haben,
   in einem inerten Lösungsmittel in Gegenwart einer Base mit einer Verbindung der Formel (V) 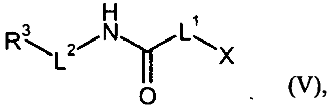 in welcher L¹, L² und R³ jeweils die oben angegebenen Bedeutungen haben
   und
   X für eine Abgangsgruppe, wie beispielsweise Halogen, Mesylat oder Tosylat, steht,
   umsetzt
und die resultierenden Verbindungen der Formel (I) gegebenenfalls mit den entsprechenden (i) Lösungsmitteln und/oder (ii) Basen oder Säuren in ihre Solvate, Salze und/oder Solvate der Salze überführt.

Inerte Lösungsmittel für den Verfahrensschritt (II) + (III) → (I) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Ethylacetat, Acetonitril, Pyridin, Dimethylsulfoxid, *N,N*-Dimethylformamid, *N,N*'-Dimethylpropylenharnstoff (DMPU) oder *N*-Methylpyrrolidon (NMP). Ebenso ist es möglich, Gemische der genannten Lösungsmittel zu verwenden. Bevorzugt sind Dichlormethan, Tetrahydrofuran, Dimethylformamid, Dimethylsulfoxid oder Gemische dieser Lösungsmittel.

Als Kondensationsmittel für die Amidbildung im Verfahrensschritt (II) + (III) → (I) eignen sich beispielsweise Carbodiimide wie *N,N*'-Diethyl-, *N,N*'-Dipropyl-, *N,N*'-Diisopropyl-, *N,N*'-Dicyclohexylcarbodiimid (DCC) oder *N*-(3-Dimethylaminoisopropyl)-*N'*-ethylcarbodiimid-Hydrochlorid (EDC), Phosgen-Derivate wie *N,N*'-Carbonyldiimidazol (CDI), 1,2-Oxazoliumverbindungen wie 2-Ethyl-5-phenyl-1,2-oxazolium-3-sulfat oder 2-*tert*.-Butyl-5-methyl-isoxazolium-perchlorat, Acylaminoverbindungen wie 2-Ethoxy-1-ethoxycarbonyl-1,2-dihydrochinolin, oder Isobutylchlorformiat, Propanphosphonsäureanhydrid, Cyanophosphonsäurediethylester, Bis-(2-oxo-3-oxazoli-dinyl)-phosphorylchlorid, Benzotriazol-1-yloxy-tris(dimethylamino)phosphonium-hexafluorophosphat, Benzotriazol-1-yloxy-tris(pyrrolidino)phosphonium-hexafluorophosphat (PyBOP), *O-*(Benzotriazol-1-yl)-*N,N,N',N*'-tetramethyluronium-tetrafluoroborat (TBTU), *O*-(Benzotriazol-1-yl)-*N,N,N',N*'-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N,N,N',N*'-tetramethyl-uronium-hexafluorophosphat (HATU) oder *O*-(1*H*-6-Chlorbenzotriazol-1-yl)-1,1,3,3-tetramethyl-uronium-tetrafluoroborat (TCTU), gegebenenfalls in Kombination mit weiteren Hilfsstoffen wie 1-Hydroxybenzotriazol (HOBT) oder *N*-Hydroxysuccinimid (HOSu), sowie als Basen Alkalicarbonate, z.B. Natrium- oder Kaliumcarbonat oder -hydrogencarbonat, oder organische Basen wie Trialkylamine, z.B. Triethylamin, *N*-Methylmorpholin, N-Methylpiperidin oder *N,N*-Diisopropylethylamin. Bevorzugt wird EDC in Kombination mit HOBt oder TBTU in Verbindung mit *N,N*-Diisopropylethylamin verwendet.

Die Kondensation (II) + (III) → (I) wird im Allgemeinen in einem Temperaturbereich von -20°C bis +60°C, bevorzugt bei 0°C bis +40°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (IV) + (V) → (I) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Aceton, Methylethylketon, Ethylacetat, Acetonitril, *N,N*-Dimethylformamid, Dimethylsulfoxid, *N,N*'-Dimethylpropylenharnstoff (DMPU), *N*-Methylpyrrolidon (NMP) oder Pyridin. Ebenso ist es möglich, Gemische der genannten Lösungsmittel einzusetzen. Bevorzugt wird Acetonitril, Aceton oder Dimethylformamid verwendet.

Als Basen für den Verfahrensschritt (IV) + (V) → (I) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium-oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)amid oder Lithiumdiisopropylamid, oder organische Amine wie Triethylamin, *N*-Methylmorpholin, *N-*Methylpiperidin, *N,N*-Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Kalium- oder Cäsiumcarbonat verwendet.

Die Base wird hierbei in einer Menge von 1 bis 5 Mol, bevorzugt in einer Menge von 1 bis 2.5 Mol, bezogen auf 1 Mol der Verbindung der Formel (IV), eingesetzt. Die Reaktion erfolgt im Allgemeinen in einem Temperaturbereich von 0°C bis +100°C, bevorzugt bei +20°C bis +80°C. Die Umsetzung kann bei normalem, erhöhtem oder bei erniedrigtem Druck durchgeführt werden (z.B. von 0.5 bis 5 bar). Im Allgemeinen arbeitet man bei Normaldruck.

Die Herstellung der erfindungsgemäßen Verbindungen kann durch das folgende Syntheseschema veranschaulicht werden:

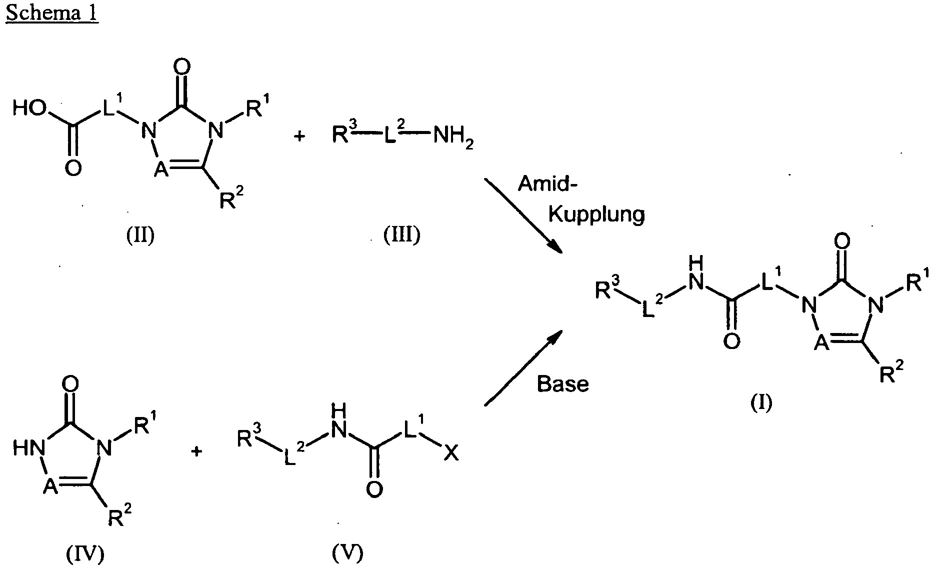

Die Verbindungen der Formel (II), in welcher A für N steht, können durch baseninduzierte Alkylierung von 5-Aryl-2,4-dihydro-3*H*-1,2,4-triazol-3-onen der Formel (IVa) zu den *N*²-substituierten Verbindungen (VIIa) und nachfolgende Ester-Hydrolyse erhalten werden (siehe Schema 2):

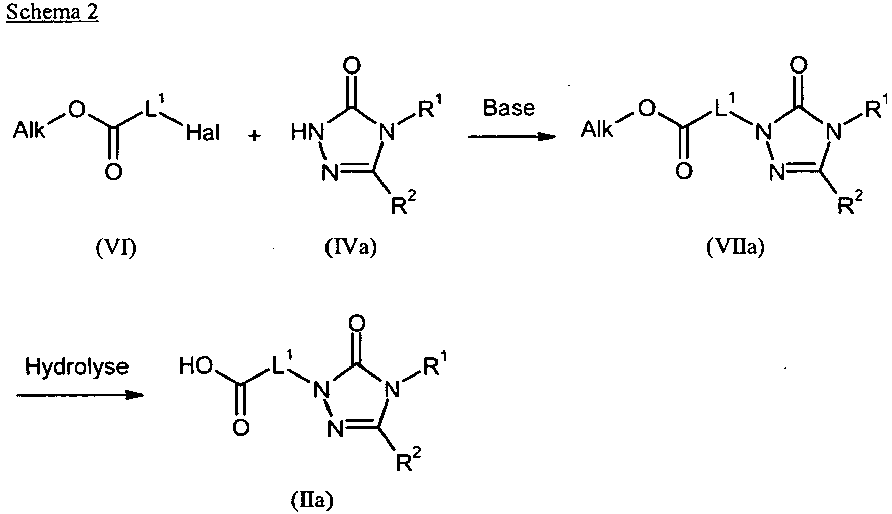

[Alk = Alkyl, Hal = Halogen].

Die Verbindungen der Formel (VIIa) können alternativ auch aus literaturbekannten N-(Alkoxy-carbonyl)arylthioamiden der Formel (IX) [siehe z.B. M. Arnswald, W.P. Neumann, J. Org. Chem. 58 (25), 7022-7028 (1993); E.P. Papadopoulos, J. Org. Chem. 41 (6), 962-965 (1976)] durch Reaktion mit Hydrazino-Estem der Formel (VIII) und nachfolgende Alkylierung an N-4 des Triazolons (Xa) hergestellt werden (Schema 3):

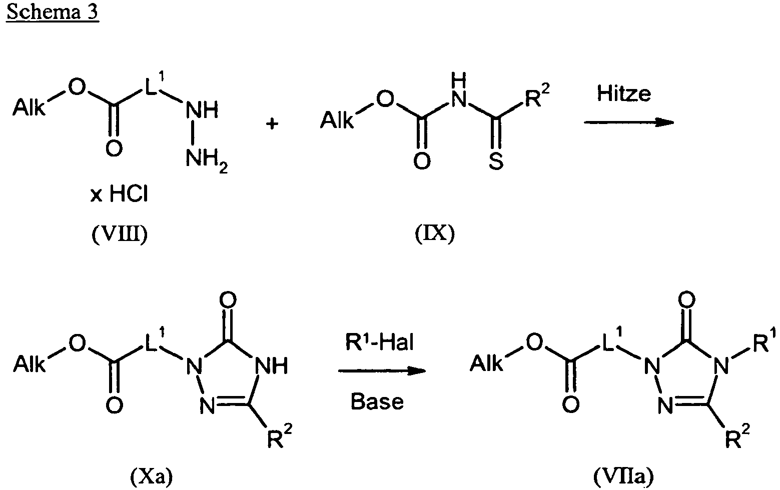

Die Verbindungen der Formel (II), in welcher A für C-R⁴ steht, sind durch Umsetzung von α-Aminoketonen der Formel (XI) mit Isocyanaten der Formel (XII) und nachfolgende Ester-Hydrolyse zugänglich (Schema 4). Die Verbindungen der Formel (XI) ihrerseits lassen sich in literaturbekannter Weise aus α-Bromketonen der Formel (XIV) und Amino-Estern der Formel (XV) synthetisieren (Schema 5):

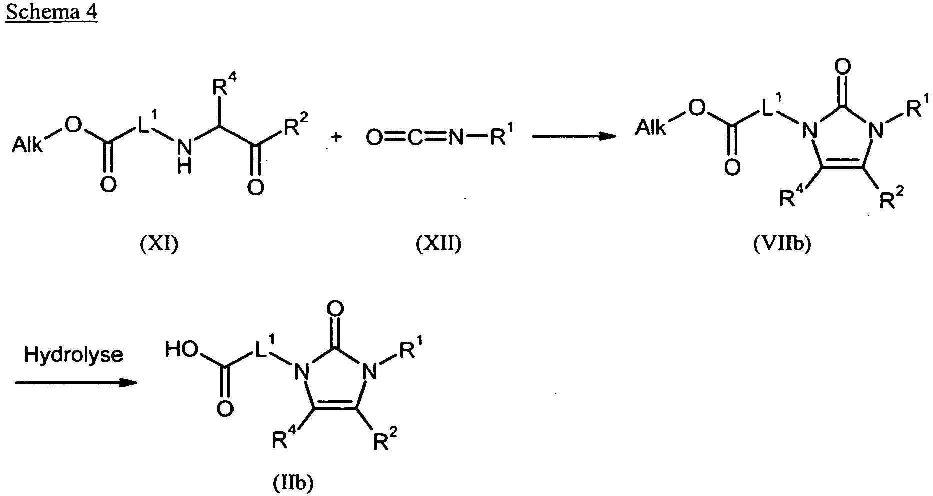

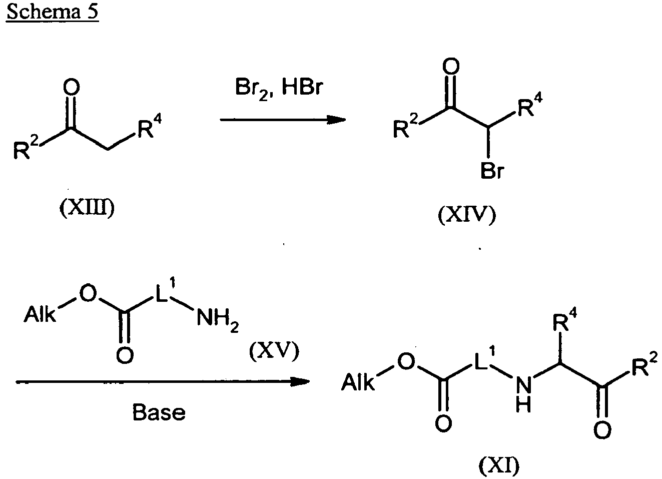

Die Verbindungen der Formel (IV), in welcher A für N steht, können ausgehend von Carbonsäurehydraziden der Formel (XVI) durch Umsetzung mit Isocyanaten der Formel (XII) oder Nitrophenylcarbamaten der Formel (XVII) und nachfolgende baseninduzierte Cyclisierung der intermediären Hydrazincarboxamide (XVIII) hergestellt werden (Schema 6):

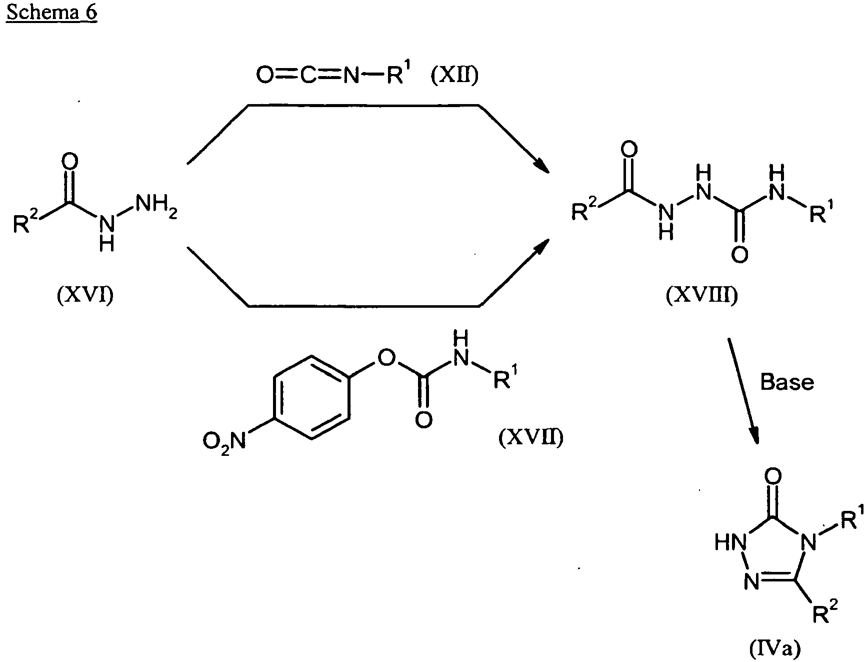

Die Verbindungen der Formel (V) sind beispielsweise durch Acylierung von Aminen der Formel (III) mit Carbonsäurechloriden der Formel (XIX) zugänglich (Schema 7):

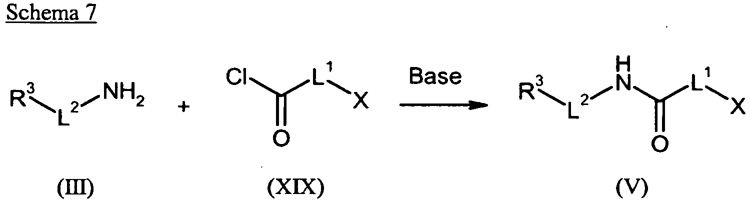

In einer besonderen Verfahrensvariante können Verbindungen der Formel (VIIa) oder (VIIb) gegebenenfalls auch dadurch hergestellt werden, dass in den in Schema 2, 3, 4 bzw. 6 beschriebenen Verfahren anstelle von R¹ zunächst eine temporäre Schutzgruppe (PG), wie beispielsweise Allyl oder 4-Methoxybenzyl, eingesetzt wird; nach deren Abspaltung zu Verbindungen der Formel (X) lassen sich Verbindungen der Formel (VII) durch entsprechende N-Alkylierung erhalten (Schema 8):

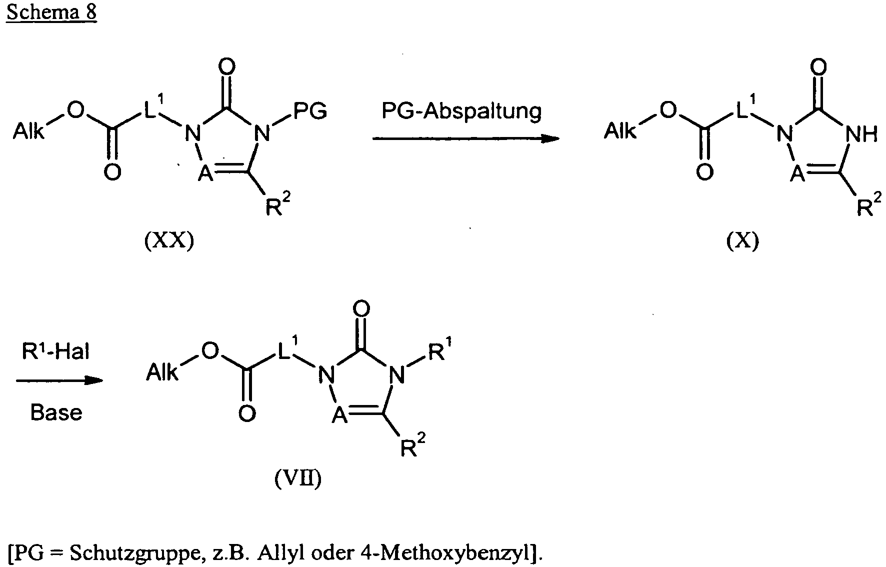

Analoge Umwandlungen PG → R¹ können gegebenenfalls auch später auf der Stufe der Verbindungen der Formel (IIa), (IIb) oder (I) erfolgen (Schema 9):

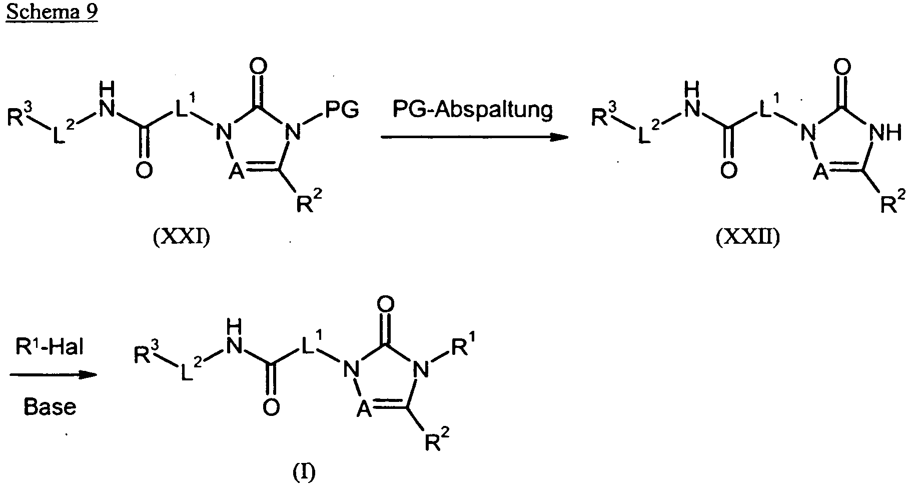

Die Einführung und Abspaltung der Schutzgruppe PG erfolgt hierbei nach literaturüblichen Methoden [siehe z.B. T.W. Greene und P.G.M. Wuts, Protective Groups in Organic Synthesis, Wiley, New York, 1999]. So wird die Allyl-Gruppe vorzugsweise mit Hilfe von Ameisensäure in Gegenwart des Tetrakis(triphenylphosphin)palladium(0)-Katalysators und einer Amin-Base wie Triethylamin entfernt. Die Abspaltung der p-Methoxybenzyl-Schutzgruppe erfolgt bevorzugt mit Hilfe starker Säuren, wie beispielsweise Trifluoressigsäure, oder auf oxidativem Wege, z.B. durch Behandlung mit 2,3-Dichlor-5,6-dicyano-1,4-benzochinon (DDQ) oder Ammoniumcer(IV)nitrat.

Die nachfolgende N-Alkylierung (X) → (VII) bzw. (XXII) → (I) wird analog zum zuvor beschriebenen Verfahrensschritt (IV) + (V) → (I) durchgeführt. Als inerte Lösungsmittel kommen vorzugsweise Aceton, Acetonitril, Dimethylformamid, Dimethylsulfoxid, Toluol, Tetrahydrofuran, Glykoldimethylether oder Gemische hiervon in Betracht. Als Base wird bevorzugt Natriumhydrid oder Kalium- oder Cäsiumcarbonat verwendet. Gegebenenfalls können diese Alkylierungen vorteilhaft unter Zusatz von Katalysatoren wie beispielsweise Lithiumbromid, Natriumiodid, Tetra-n-butylammoniumbromid oder Benzyltriethylammoniumchlorid durchgeführt werden. Die Umsetzungen erfolgen im Allgemeinen in einem Temperaturbereich von 0°C bis +150°C, bevorzugt bei +20°C bis +80°C.

Die Verbindungen der Formeln (III), (VI), (VIII), (IX), (XII), (XIII), (XV), (XVI), (XVII) und (XIX) sind vielfältig kommerziell verfügbar, literaturbekannt oder nach allgemein bekannten Verfahren zugänglich.

Die erfindungsgemäßen Verbindungen besitzen wertvolle pharmakologische Eigenschaften und können zur Vorbeugung und/oder Behandlung von verschiedenen Erkrankungen und krankheitsbedingten Zuständen bei Menschen und Tieren verwendet werden.

Bei den erfindungsgemäßen Verbindungen handelt es sich um potente selektive V1a-, selektive V2- und insbesondere um duale V1a/V2-Rezeptor-Antagonisten, die die Vasopressin-Aktivität *in vitro* und *in vivo* inhibieren. Darüber hinaus wirken die erfindungsgemäßen Verbindungen auch als Antagonisten am verwandten Oxytozin-Rezeptor.

Die erfindungsgemäßen Verbindungen sind insbesondere für die Prophylaxe und/oder Behandlung von kardiovaskulären Erkrankungen geeignet. In diesem Zusammenhang seien als Zielindikationen beispielhaft und vorzugsweise genannt: akute und chronische Herzinsuffizienz, arterielle Hypertonie, koronare Herzerkrankung, stabile und instabile Angina pectoris, myokardiale Ischämie, Myokardinfarkt, Schock, Arteriosklerose, atriale und ventrikuläre Arrhythmien, transitorische und ischämische Attacken, Hirnschlag, entzündliche kardiovaskuläre Erkrankungen, periphere und kardiale Gefäßerkrankungen, periphere Durchblutungsstörungen, arterielle pulmonale Hypertonie, Spasmen der Koronararterien und peripherer Arterien, Thrombosen, thromboembolische Erkrankungen, Ödembildung wie zum Beispiel pulmonales Ödem, Hirnödem, renales Ödem oder Herzinsuffizienz-bedingtes Ödem, sowie Restenosen wie nach Thrombolysetherapien, percutan-transluminalen Angioplastien (PTA), transluminalen Koronarangioplastien (PTCA), Herztransplantationen und Bypass-Operationen.

Im Sinne der vorliegenden Erfindung umfasst der Begriff Herzinsuffizienz auch spezifischere oder verwandte Krankheitsformen wie Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Weiterhin eignen sich die erfindungsgemäßen Verbindungen zur Verwendung als Diuretikum für die Behandlung von Ödemen und bei Elektrolytstörungen, insbesondere bei der hypervolämischen und euvolämischen Hyponaträmie.

Die erfindungsgemäßen Verbindungen sind weiter geeignet für die Prophylaxe und/oder Behandlung der polyzystischen Nierenkrankheit (PCKD) und des Syndroms der inadäquaten ADH-Sekretion (SIADH).

Außerdem können die erfindungsgemäßen Verbindungen für die Prophylaxe und/oder Behandlung der Leberzirrhose, des Aszites, von Diabetes mellitus und diabetischen Folgeerkrankungen, wie z.B. Neuropathie und Nephropathie, von akutem und chronischem Nierenversagen sowie von chronischer Niereninsuffizienz verwendet werden.

Ferner eignen sich die erfindungsgemäßen Verbindungen für die Prophylaxe und/oder Behandlung zentralnervöser Störungen wie Angstzuständen und Depressionen, von Glaukom sowie von Krebs, insbesondere von Lungentumoren.

Darüber hinaus können die erfindungsgemäßen Verbindungen für die Prophylaxe und/oder Behandlung von entzündlichen Erkrankungen, asthmatischen Erkrankungen, chronisch-obstruktiven Atemwegserkrankungen (COPD), Schmerzzuständen, Prostatahypertrophien, Inkontinenz, Blasenentzündung, hyperaktiver Blase, Erkrankungen der Nebenniere wie zum Beispiel Phäochromozytom und Nebennierenapoplexie, Erkrankungen des Darms wie zum Beispiel Morbus Crohn und Diarrhoe, oder von Menstruationsstörungen wie zum Beispiel Dysmenorrhöen eingesetzt werden.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, die mindestens eine der erfindungsgemäßen Verbindungen sowie einen oder mehrere weitere Wirkstoffe enthalten, insbesondere zur Behandlung und/oder Prophylaxe der zuvor genannten Erkrankungen. Als hierfür geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Diuretika, insbesondere Schleifendiuretika sowie Thiazide und Thiazid-ähnliche Diuretika;
- positiv-inotrop wirksame Verbindungen, wie beispielsweise Herzglycoside (Digoxin), betaadrenerge und dopaminerge Agonisten wie Isoproterenol, Adrenalin, Noradrenalin, Dopamin und Dobutamin;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil, sowie PDE 3-Inhibitoren wie Amrinone und Milrinone;
- natriuretische Peptide, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Calcium-Sensitizer, wie beispielhaft und vorzugsweise Levosimendan;
- NO- und Häm-unabhängige Aktivatoren der Guanylatcyclase, wie insbesondere die in WO 01/19355, WO 01/19776, WO 01/19778, WO 01/19780, WO 02/070462 und WO 02/070510 beschriebenen Verbindungen;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- Inhibitoren der humanen neutrophilen Elastase (HNE), wie beispielsweise Sivelestat oder DX-890 (Reltran);
- die Signaltransduktionskaskade inhibierende Verbindungen, wie beispielsweise Tyrosinkinase-Inhibitoren, insbesondere Sorafenib, Imatinib, Gefitinib und Erlotinib;
- den Energiestoffwechsel des Herzens beeinflussende Verbindungen, wie beispielhaft und vorzugsweise Etomoxir, Dichloracetat, Ranolazine oder Trimetazidine;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Vasopeptidase-Inhibitoren, Inhibitoren der neutralen Endopeptidase, Endothelin-Antagonisten, Renin-Inhibitoren, alpha-Rezeptoren-Blocker, beta-Rezeptoren-Blocker, Mineralocorticoid-Rezeptor-Antagonisten und Rho-Kinase-Inhibitoren; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Vasopeptidase-Inhibitoren, Inhibitoren der neutralen Endopeptidase, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Vasopeptidase-Inhibitor bzw. Inhibitor der neutralen Endopeptidase (NEP), wie beispielhaft und vorzugsweise Omapatrilat oder AVE-7688, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton, Eplerenon, Canrenon oder Kalium-Canrenoat, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095 oder BA-1049, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705, BAY 60-5521, BAY 78-7499 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfmdungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 10 mg/kg, vorzugsweise etwa 0.01 bis 1 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- Alk: Alkyl
- Boc: *tert*.-Butoxycarbonyl
- CI: chemische Ionisation (bei MS)
- DCI: direkte chemische Ionisation (bei MS)
- DME: 1,2-Dimethoxyethan
- DMF: Dimethylformamid
- DMSO: Dimethylsulfoxid
- d. Th.: der Theorie (bei Ausbeute)
- EDC: *N'*-(3-Dimethylaminopropyl)-*N*-ethylcarbodimid (-Hydrochlorid)
- EE: Essigsäureethylester
- eq.: Äquivalent(e)
- ESI: Elektrospray-Ionisation (bei MS)
- FMOC: 9-Fluorenylmethoxycarbonyl
- GC/MS: Gaschromatographie-gekoppelte Massenspektrometrie
- ges.: gesättigt
- h: Stunde(n)
- Hal: Halogen
- HOBt: 1-Hydroxy-1*H* benzotriazol-Hydrat
- HPLC: Hochdruck-, Hochleistungsflüssigchromatographie
- konz.: konzentriert
- LC/MS: Flüssigchromatographie-gekoppelte Massenspektrometrie
- LDA: Lithiumdiisopropylamid
- LiHMDS: Lithiumhexamethyldisilazan
- min: Minute(n)
- MS: Massenspektrometrie
- NMR: Kernresonanzspektrometrie
- PG: Schutzgruppe
- rac: racemisch / Racemat
- R_{f}: Retentionsfaktor (bei Dünnschichtchromatographie auf Kieselgel)
- RT: Raumtemperatur
- Rₜ: Retentionszeit (bei HPLC)
- TBTU: *O*-(Benzotriazol-1-yl)-*N*,*N*,*N*',*N*'-tetramethyluronium-Tetrafluoroborat
- THF: Tetrahydrofuran
- TMOF: Trimethylorthoformiat
- UV: Ultraviolett-Spektrometrie
- v/v: Volumen zu Volumen-Verhältnis (einer Lösung)

### LC/MS-, HPLC- und GC/MS-Methoden:

**Methode 1 (HPLC):** Instrument: HP 1100 mit DAD-Detektion; Säule: Kromasil 100 RP-18, 60 mm x 2.1 mm, 3.5 µm; Eluent A: 5 ml HClO₄ (70%-ig) / Liter Wasser, Eluent B: Acetonitril; Gradient: 0 min 2% B → 0.5 min 2% B → 4.5 min 90% B → 9 min 90% B → 9.2 min 2% B → 10 min 2% B; Fluss: 0.75 ml/min; Säulentemperatur: 30°C; UV-Detektion: 210 nm.
**Methode 2 (HPLC):** Instrument: HP 1100 mit DAD-Detektion; Säule: Kromasil 100 RP-18, 60 mm x 2.1 mm, 3.5 µm; Eluent A: 5 ml HClO₄ (70%-ig) / Liter Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.
**Methode 3 (LC/MS):** Instrument: Micromass Platform LCZ mit HPLC Agilent Serie 1100; Säule: Thermo Hypersil GOLD 3µ 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.
**Methode 4 (LC/MS):** Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.
**Methode 5 (LC/MS):** Instrument: Micromass Quattro LCZ mit HPLC Agilent Serie 1100; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.
**Methode 6 (LC/MS):** Instrument MS: Waters ZQ 2000; Instrument HPLC: Agilent 1100, 2-Säulen-Schaltung; Autosampler: HTC PAL; Säule: YMC-ODS-AQ, 50 mm x 4.6 mm, 3.0 µm; Eluent A: Wasser + 0.1% Ameisensäure, Eluent B: Acetonitril + 0.1 % Ameisensäure; Gradient: 0.0 min 100% A → 0.2 min 95% A → 1.8 min 25% A → 1.9 min 10% A → 2.0 min 5% A → 3.2 min 5% A → 3.21 min 100% A → 3.35 min 100% A; Ofen: 40°C; Fluss: 3.0 ml/min; UV-Detektion: 210 nm.
**Methode 7 (LC/MS):** Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.
**Methode 8 (LC/MS):** Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.
**Methode 9 (präparative HPLC):** Instrument: Abimed Gilson Pump 305/306, Manometric Module 806; Säule: Grom-Sil 120 ODS-4HE 10 µm, 250 mm x 30 mm; Eluent: A = Wasser, B = Acetonitril; Gradient: 0.0 min 30% B, 3 min 30% B, 31 min 95% B, 44 min 95% B, 44.01 min 30% B, 45 min 30% B; Fluss: 50 ml/min; Säulentemperatur: RT; UV-Detektion: 210 nm.
**Methode 10 (präparative HPLC):** Instrument: Abimed Gilson Pump 305/306, Manometric Module 806; Säule: Grom-Sil 120 ODS-4HE 10 µm, 250 mm x 20 mm; Eluent: A = Wasser, B = Acetonitril; Gradient: 0.0 min 10% B, 5 min 10% B, 30 min 95% B, 34 min 95% B, 34.01 min 10% B, 38 min 10% B; Fluss: 25 ml/min; Säulentemperatur: RT; UV-Detektion: 210 nm.
**Methode 11 (präparative HPLC):** Instrument: Abimed Gilson Pump 305/306, Manometric Module 806; Säule: Grom-Sil 120 ODS-4HE 10 µm, 250 mm x 20 mm; Eluent: A = Wasser, B = Acetonitril; Gradient: 0.0 min 10% B, 3 min 10% B, 30 min 95% B, 42 min 95% B, 42.01 min 10% B, 45 min 10% B; Fluss: 50 ml/min; Säulentemperatur: RT; UV-Detektion: 210 nm.
**Methode 12 (präparative HPLC):** Instrument: Abimed Gilson Pump 305/306, Manometric Module 806; Säule: Grom-Sil 120 ODS-4HE 10 µm, 250 mm x 40 mm; Eluent: A = Wasser, B = Acetonitril; Gradient: 0.0 min 10% B, 3 min 10% B, 27 min 98% B, 34 min 98% B, 38 min 10% B; Fluss: 50 ml/min; Säulentemperatur: RT; UV-Detektion: 214 nm.
**Methode 13 (präparative HPLC):** Instrument: Abimed Gilson Pump 305/306, Manometric Module 806; Säule: Macherey-Nagel VP 50/21 Nucleosil 100-5 C18 Nautilus 5 µm; Eluent: A = Acetonitril, B = Wasser + 0.1 % Ameisensäure; Gradient: 0.0 min 10% A, 2.00 min 10% A, 6.00 min 90% A, 7.00 min 90% A, 7.10 min 10% A, 8 min 10% A; Laufzeit: ca. 10 min pro Trennung; Fluss: 25 ml/min; Säulentemperatur: RT; UV-Detektion: 220 nm.
**Methode 14 (chirale präparative HPLC):** Chirale Kieselgel-Phase basierend auf dem Selektor Poly(*N*-methacryloyl-L-leucin-*tert*.-butylamid); Säule: 680 mm x 40 mm; Elutionsmittel: iso-Hexan/Essigsäureethylester 1:1 (v/v); Fluss: 50 ml/min; Temperatur: 24°C; UV-Detektion: 260 nm. Analytische Säule: 250 mm x 4.6 mm; gleiches Elutionsmittel; Fluss: 2 ml/min.
**Methode 15 (chirale präparative HPLC):** Chirale Kieselgel-Phase basierend auf dem Selektor Poly(*N*-methacryloyl-L-leucin-dicyclopropylmethylamid); Säule: 250 mm x 30 mm; Elutionsmittel: *iso*-Hexan/Essigsäureethylester 3:7 (v/v); Fluss: 25 ml/min; Temperatur: 24°C; UV-Detektion: 260 nm. Analytische Säule: 250 mm x 4.6 mm; gleiches Elutionsmittel; Fluss: 2 ml/min.
**Methode 16 (chirale präparative HPLC):** Chirale Kieselgel-Phase basierend auf dem Selektor Poly(N-methacryloyl-D-valin-3-pentylamid); Säule: 250 mm x 20 mm; Elutionsmittel: *iso*-Hexan/ Essigsäureethylester 1:3 (v/v); Fluss: 25 ml/min; Temperatur: 24°C; UV-Detektion: 260 nm. Analytische Säule: 250 mm x 4.6 mm; gleiches Elutionsmittel; Fluss: 2 ml/min.
**Methode 17 (LC/MS):** Instrument: Micromass Quattro LCZ mit HPLC Agilent Serie 1100; Säule: Phenomenex Onyx Monolithic C18, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.
**Methode 18 (LC/MS):** Instrument: Micromass Quattro LCZ mit HPLC Agilent Serie 1100; Säule: Phenomenex Gemini 3µ, 30 mm x 3.00 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.
**Methode 19 (LC/MS):** Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.
**Methode 20 (präparative HPLC):** Säule: Grom-Sil 120 ODS-4HE, 10 µm, 250 mm x 30 mm; Eluent A: 0.1% Ameisensäure in Wasser, Eluent B: Acetonitril; Fluss: 50 ml/min; Programm: 0-3 min 10% B, 3-27 min Gradient bis 95% B; 27-34 min 95% B; 34.01-38 min 10% B.
**Methode 21 (GC/MS):** Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).
**Methode 22 (LC/MS):** Gerätetyp MS: Micromass ZQ; Gerätetyp HPLC: Waters Alliance 2795; Säule: Phenomenex Synergi 2.5 p MAX-RP 100A Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.
**Methode 23 (LC/MS):** Instrument: Micromass Quattro LCZ mit HPLC Agilent Serie 1100; Säule: Phenomenex Synergi 2.5 µ MAX-RP 100A Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 3.0 min 5% A → 4.0 min 5% A → 4.1 min 90% A; Fluss: 2 ml/min; Ofen: 50°C; UV-Detektion: 208-400 nm.
**Methode 24 (LC/MS):** Instrument MS: Micromass TOF (LCT); Instrument HPLC: Waters 2690; Autosampler: Waters 2700; Säule: YMC-ODS-AQ, 50 mm x 4.6 mm, 3.0 µm; Eluent A: Wasser + 0.1 % Ameisensäure, Eluent B: Acetonitril + 0.1 % Ameisensäure; Gradient: 0.0 min 100% A → 0.2 min 95% A → 1.8 min 25% A → 1.9 min 10% A → 2.0 min 5% A → 3.2 min 5% A → 3.21 min 100% A → 3.35 min 100% A; Ofen: 40°C; Fluss: 3.0 ml/min; UV-Detektion: 210 nm.

### Ausgangsverbindungen und Intermediate:

### Beispiel 1A

### 2-[3-(Trifluormethyl)phenyl]propan-2-amin

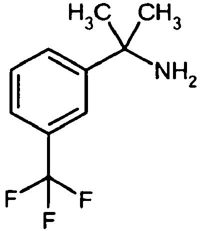

14.0 g (56.8 mmol) wasserfreies Cer(III)chlorid werden unter Argonatmosphäre in 60 ml Tetrahydrofuran 2 Stunden bei Raumtemperatur gerührt. Nach Abkühlen auf -50°C tropft man bei dieser Temperatur 35.5 ml einer 1.6 M Lösung von Methyllithium in Diethylether langsam zu und lässt 30 min bei -50°C nachrühren. Sodann tropft man bei -50°C 3.24 g (18.9 mmol) 3-Trifluormethylbenzonitril, gelöst in 30 ml Tetrahydrofuran, zu, lässt langsam auf Raumtemperatur aufwärmen und über Nacht nachrühren. Zur Aufarbeitung werden 20 ml einer 25%-igen wässrigen Ammoniak-Lösung zugegeben, das Gemisch über Kieselgur filtriert und das Eluat im Vakuum eingeengt. Der Rückstand wird in Essigsäureethylester aufgenommen und zweimal mit 1 N Salzsäure extrahiert. Die vereinigten wässrigen Phasen werden mit 1 N Natronlauge auf pH 12 eingestellt und zweimal mit Essigsäureethylester extrahiert. Nach Trocknen der vereinigten organischen Phasen über Magnesiumsulfat und Entfernen des Lösungsmittels im Vakuum verbleiben 3.41 g (98% d. Th.) der Zielverbindung.
LC/MS [Methode 3]: Rₜ = 2.44 min
¹H-NMR (400 MHz, DMSO-d₆): δ = 1.39 (s, 6H), 2.01 (br. s, 2H), 7.48 - 7.56 (m, 2H), 7.78 - 7.85 (m, 1H), 7.90 (s, 1H).

### Beispiel 2A

### 2-(4-Chlorbenzoyl)-N-cyclopropylhydrazincarboxamid

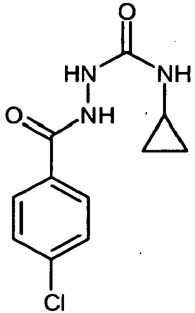

Unter einer Argonatmosphäre werden 4.00 g (23.4 mmol) 4-Chlorbenzoesäurehydrazid in 50 ml Tetrahydrofuran vorgelegt. Bei 50°C werden 1.95 g (23.4 mmol) Cyclopropylisocyanat, gelöst in 50 ml Tetrahydrofuran, zugetropft und das Gemisch über Nacht bei 50°C weiter gerührt. Man engt das Lösungsmittel im Vakuum ein, gibt zum Rückstand Diethylether und isoliert und reinigt den sich bildenden Feststoff durch Filtration und Nachwaschen mit Diethylether. Man erhält so 5.92 g (ca. 100% d. Th.) der Zielverbindung.
HPLC [Methode 2]: Rₜ = 3.69 min
MS [CIpos]: m/z = 371 (M+NH₄)⁺, 354 (M+H)⁺
¹H-NMR (400 MHz, DMSO-d₆): δ = 0.33 - 0.46 (m, 2H), 0.51 - 0.65 (m, 2H), 2.44 - 2.52 (m, 1H), 6.69 (s, 1H), 7.55, 7.57 (AA'-Teil eines AA'BB'-Systems, 2H), 7.88 (s, 1H), 7.88, 7.90 (BB'-Teil eines AA'BB'-Systems, 2H), 10.16 (s, 1 H).

In analoger Weise werden die folgenden Verbindungen erhalten:

| **Beispiel Nr.** | **Struktur** | **LC/MS Rₜ [Methode]** | **¹H-NMR (400 MHz, DMSO-d₆)** |
|---|---|---|---|
| **3A** | 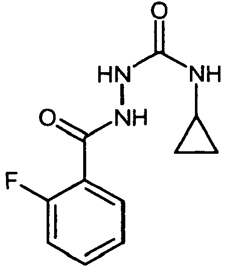 | Rₜ = 0.86 min [7] | δ = 0.33 - 0.46 (m, 2H), 0.52 - 0.66 (m, 2H), 2.45 -.52 (m, 1 H), 6.57 (s, 1 H), 7.26 - 7.34 (m, 2H), 7.51 -7.59 (m, 1 H), 7.62 - 7.69 (m, 1 H), 7.98 (s, 1H), 9.86 (s, 1H). |
| **4A** | 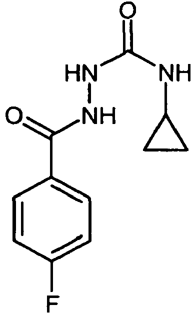 | Rₜ = 1.00 mm [7] | δ = 0.33 - 0.46 (m, 2H), 0.51 - 0.65 (m, 2H), 2.44 -2.52 (m, 1 H), 6.67 (d, 1H), 7.32 (t, 2H), 7.86 (s, 1H), 7.95 (dd, 1 H), 10.10 (s, 1 H). |
| **5A** | 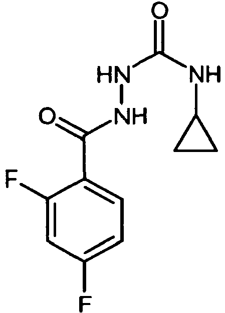 | Rₜ = 1.00 min [7] | |
| **6A** | 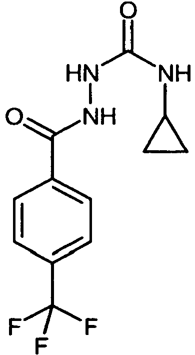 | Rₜ = 1.48 min [7] | |
| **7A** | 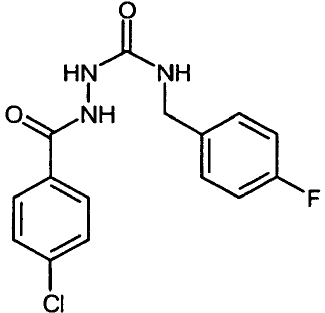 | Rₜ = 1.78 min [7] | δ = 4.22 (d, 2H), 7.09 -7.18 (m, 2H), 7.27 - 7.34 (m, 2H), 7.56, 7.58 (AA'-Teil eines AA'BB'-Systems, 2H), 7.91, 7.93 (BB'-Teil eines AA'BB'-Systems, 2H), 8.03 (s, 1H), 10.26 (s, 1H). |

### Beispiel 8A

### 2-(3-Chlorbenzoyl)-N-cyclopropylhydrazincarboxamid

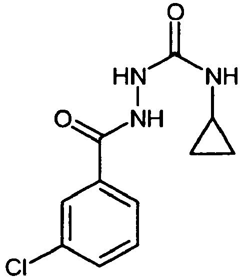

Unter einer Argonatmosphäre werden 430 mg (2.52 mmol) 3-Chlorbenzoesäurehydrazid in 6 ml Tetrahydrofuran vorgelegt. Man tropft 209 mg (2.52 mmol) Cyclopropylisocyanat, gelöst in 2 ml Tetrahydrofuran zu und lässt über Nacht bei Raumtemperatur weiter rühren. Man engt ein und reinigt durch Verrühren des Rückstandes mit Diethylether, Filtration, Nachwaschen mit Diethylether und Trocknen im Vakuum. Man erhält so 514 mg (80% d. Th.) der Zielverbindung.
LC/MS [Methode 5]: Rₜ = 1.41 min
¹H-NMR (400 MHz, DMSO-d₆): δ = 0.34 - 0.46 (m, 2H), 0.51 - 0.65 (m, 2H), 2.44 - 2.56 (m, 1 H), 6.71 (s, 1 H), 7.52 (t, 1 H), 7.64 (dd, 1H), 7.83 (d, 1 H), 7.91 (d, 1 H), 7.92 (s, 1 H), 10.19 (s, 1 H).

In analoger Weise wird die folgende Verbindung erhalten:

| **Beispiel Nr.** | **Struktur** | **LC/MS Rₜ [Methode]** | **¹H-NMR (400 MHz, DMSO-d₆)** |
|---|---|---|---|
| **9A** | 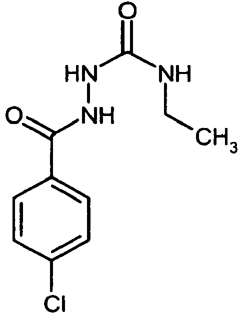 | Rₜ = 1.44 min [8] | δ = 1.00 (t, 3H), 3.05 (dq, 2H), 6.50 (br. t, 1H), 7.55, 7.57 (AA'-Teil eines AA'BB'-Systems, 2H), 7.83 (s, 1 H), 7.89, 7.91 (BB'-Teil eines AA'BB'-Systems, 2H), 10.17 (s, 1 H). |

### Beispiel 10A

### 2-(2-Chlorbenzoyl)-N-cyclopropylhydrazincarboxamid

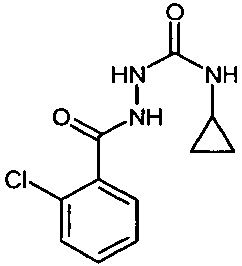

Unter einer Argonatmosphäre werden 10.0 g (58.6 mmol) 2-Chlorbenzoesäurehydrazid in 50 ml Tetrahydrofuran vorgelegt. Bei 50°C werden 4.87 g (58.6 mmol) Cyclopropylisocyanat, gelöst in 50 ml Tetrahydrofuran, zugetropft und das Gemisch über Nacht bei 50°C weiter gerührt. Der erhaltene Niederschlag wird nach Abkühlen auf Raumtemperatur abfiltriert und mit Diethylether nachgewaschen. Man erhält so 13.9 g (93% d. Th.) der Zielverbindung.
LC/MS [Methode 7]: Rₜ = 1.01 min
¹H-NMR (400 MHz, DMSO-d₆): δ = 0.33 - 0.45 (m, 2H), 0.53 - 0.67 (m, 2H), 2.45 - 2.53 (m, 1H), 6.47 (d, 1H), 7.39 - 7.55 (m, 4H), 8.00 (s, 1H), 9.95 (s, 1H).

In analoger Weise werden die folgenden Verbindungen erhalten:

| **Beispiel Nr.** | **Struktur** | **LC/MS Rₜ [Methode]** | **¹H-NMR (400 MHz, DMSO-d₆)** |
|---|---|---|---|
| **11A** | 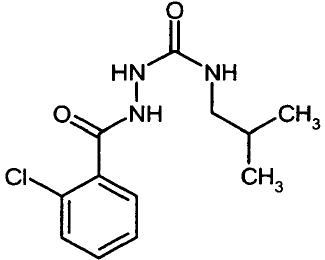 | Rₜ = 1.41 min [7] | δ = 0.85 (d, 6H), 1.60 -1.75 (m, 1 H), 2.89 (t, 2H), 6.27 (t, 1H), 7.39 - 7.57 (m, 4H), 7.96 (s, 1H), 10.00 (s, 1H). |
| **12A** | 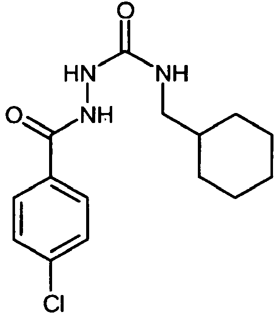 | Rₜ = 1.93min [7] | δ = 0.77 - 0.92 (m, 2H), 1.04 - 1.23 (m, 3H), 1.31 -1.43 (m, 1H), 1.56- 1.71 (m, 5H), 2.87 (t, 2H), 6.49 (br. t, 1H), 7.55, 7.57 (AA'-Teil eines AA'BB'-Systems, 2H), 7.78 (s, 1 H), 7.89, 7.91 (BB'-Teil eines AABB'-Systems, 2H), 10.16 (s, 1H). |
| **13A** | 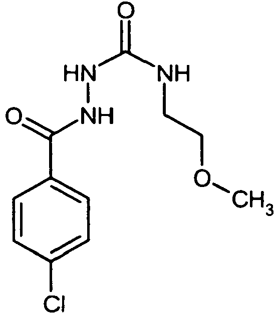 | Rₜ = 1.18 min [7] | δ = 3.18 (dt, 2H), 3.25 (s, 3H), 3.29 - 3.35 (m, 2H), 6.53 (br. t, 1H), 7.56, 7.58 (AA'-Teil eines AA'BB'-Systems, 2H), 7.89, 7.91 (BB'-Teil eines AA'BB'-Systems, 2H), 7.94 (s, 1 H), 10.21 (s, 1 H). |

### Beispiel 14A

### N-Cyclopropyl-2-(2-methoxybenzoyl)-hydrazincarboxamid

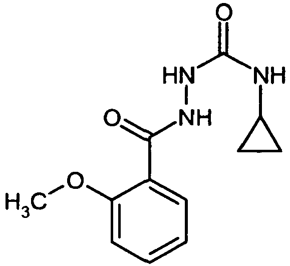

Zu 500 mg (3.01 mmol) 2-Methoxybenzoesäurehydrazid, gelöst in 7 ml THF, werden 250 mg (3.01 mmol) Cyclopropylisocyanat, gelöst in 3 ml THF, getropft und das Gemisch über Nacht bei Raumtemperatur gerührt. Der entstandene Niederschlag wird abfiltriert, mit Diethylether gewaschen und im Vakuum getrocknet. Man erhält so 709 mg (94% d. Th.) der Zielverbindung.
LC/MS [Methode 5]: Rₜ = 1.27 min
¹H-NMR (400 MHz, DMSO-d₆): δ = 0.32 - 0.45 (m, 2H), 0.53 - 0.66 (m, 2H), 2.45 - 2.53 (m, 1 H), 3.88 (s, 3H), 7.05 (t, 1 H), 7.15 (d, 1H), 7.50 (ddd, 1 H), 6.49 (br. s, 1 H), 7.72 (dd, 1 H), 7.99 (d, 1H), 9.62 (d, 1 H).

### Analog wird erhalten:

### Beispiel 15A

### N-Ethyl-2-(4-methoxybenzoyl)-hydrazincarboxamid

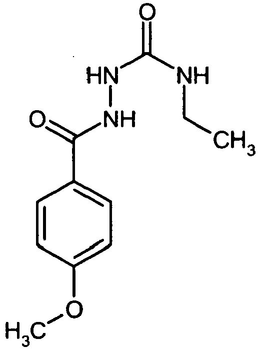

LC/MS [Methode 5]: Rₜ = 1.14 min
¹H-NMR (400 MHz, DMSO-d₆): δ = 1.00 (t, 3H), 3.05 (dq, 2H), 3.81 (s, 3H), 6.43 (br. s, 1H), 7.00, 7.02 (AA'-Teil eines AA'BB'-Systems, 2H), 7.73 (s, 1H), 7.86, 7.88 (BB'-Teil eines AA'BB'-Systems, 2H), 9.94 (s, 1H).

### Beispiel 16A

### 2-(2-Chlorbenzoyl)-N-(4-methoxyphenylmethyl)-hydrazincarboxamid

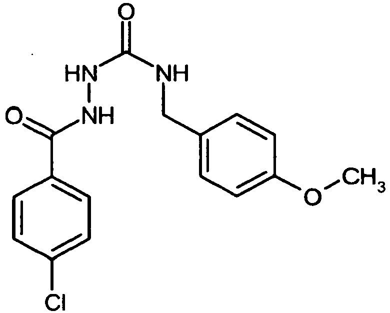

2.50 g (14.7 mmol) 4-Chlorbenzoesäurehydrazid werden bei Raumtemperatur in 30 ml Tetrahydrofuran vorgelegt. Zu dieser Suspension werden unter Rühren 2.50 g (15.3 mmol) 4-Methoxyphenylmethylisocyanat, gelöst in 6 ml Tetrahydrofuran, zügig zugetropft. Man lässt das Gemisch 6 h bei Raumtemperatur weiter rühren und dann etwa 65 h lang stehen. Unter Rühren werden dann 50 ml Diethylether eingetragen, das Reaktionsgefäß in einem Eis/Wasser-Bad abgekühlt, der Niederschlag abfiltriert, mit kaltem Diethylether nachgewaschen und im Vakuum getrocknet. Man erhält so 4.80 g (98% d. Th.) der Zielverbindung.
LC/MS [Methode 5]: Rₜ = 1.87 min
¹H-NMR (400 MHz, DMSO-d₆): δ = 3.72 (s, 3H), 4.17 (d, 2H), 6.85, 6.87 (AA'-Teil eines AA'BB'-Systems, 2H), 7.03 (br. t, 1H), 7.18, 7.20 (BB'-Teil eines AA'BB'-Systems, 2H), 7.56, 7.58 (AA'-Teil eines AA'BB'-Systems, 2H), 7.90, 7.93 (BB'-Teil eines AA'BB'-Systems, 2H), 7.96 (s, 1H), 10.24 (s, 1H).

### Beispiel 17A

### 2-(4-Chlorbenzoyl)-N-(3-fluorphenylmethyl)-hydrazincarboxamid

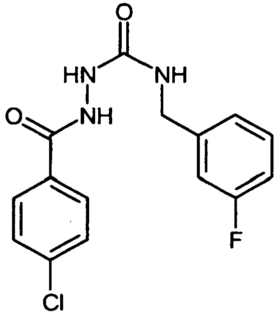

553 mg (3.24 mmol) 4-Chlorbenzoesäurehydrazid werden bei Raumtemperatur in 10 ml Tetrahydrofuran vorgelegt. Zu dieser Suspension werden unter Rühren 500 mg (3.31 mmol) 3-Fluorphenylmethylisocyanat, gelöst in 5 ml Tetrahydrofuran, zügig zugetropft. Man lässt das Gemisch über Nacht bei Raumtemperatur weiter rühren. Man versetzt das Reaktionsgemisch mit 50 ml Diethylether, gewinnt den Niederschlag durch Filtration, wäscht mit Diethylether nach und trocknet im Vakuum. Man erhält so 965 mg (92% d. Th.) der Zielverbindung.
LC/MS [Methode 8]: Rₜ = 2.00 min
¹H-NMR (400 MHz, DMSO-d₆): δ = 4.26 (d, 2H), 6.98 - 7.12 (m, 3H), 7.20 (br. s, 1H), 7.34 (q, 1H), 7.56, 7.58 (AA'-Teil eines AA'BB'-Systems, 2H), 7.91, 7.94 (BB'-Teil eines AA'BB'-Systems, 2H), 8.08 (s, 1H), 10.28 (s, 1H).

In analoger Weise zu den drei vorgenannten Beispielen erhält man:

| **Beispiel Nr.** | **Struktur** | **LC/MS Rₜ [Methode]** | **¹H-NMR (400 MHz, DMSO-d₆)** |
|---|---|---|---|
| **18A** | 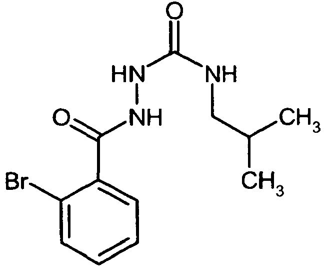 | Rₜ = 1.46 min [7] | δ = 0.85 (d, 6H), 1.60 -1.75 (m, 1H), 2.89 (t, 2H), 6.25 (t, 1H), 7.40 (td, 1H), 7.46 (td, 1H), 7.51 (dd, 1H), 7.68 (d, 1H), 7.96 (s, 1H), 10.00 (s, 1H). |
| **19A** | 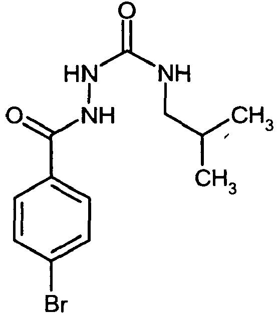 | Rₜ = 1.63 min [7] | δ = 0.83 (d, 6H), 1.60 -1.75 (m, 1H), 2.85 (t, 2H), 6.53 (t, 1H), 7.69, 7.71 (AA'-Teil eines AA'BB'-Systems, 2H), 7.80 (br. d, 1H), 7.82, 7.84 (BB'-Teil eines AA'BB'-Systems, 2H), 10.20 (br. d, 1H). |
| **20A** | 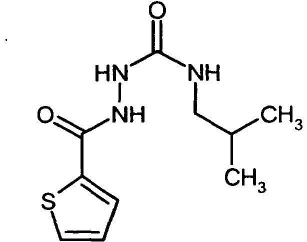 | Rₜ = 1.39 min [5] | δ = 0.83 (d, 6H), 1.60 -1.74 (m, 1H), 2.85 (t, 2H), 6.49 (br. t, 1H), 7.17 (t, 1H), 7.77 - 7.85 (m, 3H), 10.13 (s, 1H). |
| **21A** | 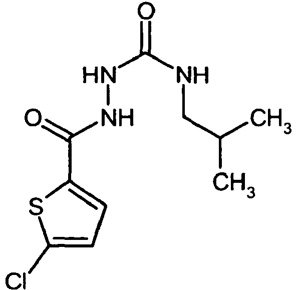 | Rₜ = 1.99 min [8] | δ = 0.82 (d, 6H), 1.59 -1.74 (m, 1H), 2.84 (t, 2H), 6.53 (br. t, 1H), 7.22 (d, 1H), 7.70 (d, 1H), 7.85 (s, 1H), 10.23 (s, 1H). |
| **22A** | 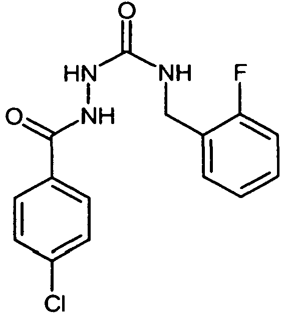 | Rₜ = 1.97 min [8] | δ=4.28 (d, 2H), 7.03 -7.19 (m, 3H), 7.24 - 7.32 (m, 1H), 7.37 (t, 1H), 7.56, 7.58 (AA'-Teil eines AA'BB'-Systems, 2H), 7.91, 7.93 (BB'-Teil eines AA'BB'-Systems, 2H), 8.08 (s, 1H), 10.29 (s, 1H). |
| **23A** | 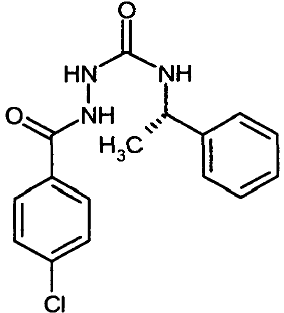 | Rₜ = 1.97 min [5] | δ = 1.36 (d, 3H), 4.81 (dq, 1H), 6.93 (d, 1H), 7.18 -7.25 (m, 1H), 7.28 - 7.35 (m, 4H), 7.55, 7.58 (AA'-Teil eines AA'BB'-Systems, 2H), 7.88 (br. s, 1H), 7.89, 7.91 (BB'-Teil eines AA'BB'-Systems, 2H), 10.22 (s, 1H). |
| **24A** | 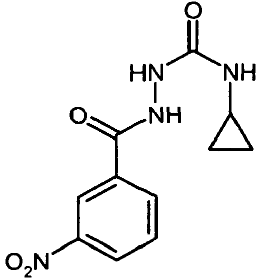 | Rₜ = 1.01 min [7] | |
| **25A** | 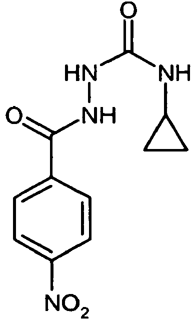 | Rₜ = 1.50 min [7] | |
| **26A** | 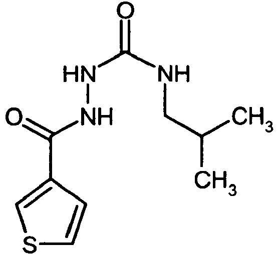 | Rₜ = 1.21 min [7] | |
| **27A** | 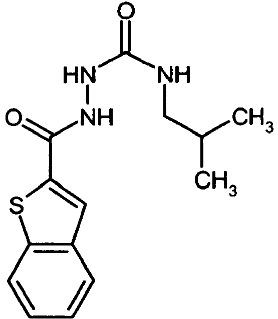 | Rₜ= 2.10 min [8] | |
| **28A** | 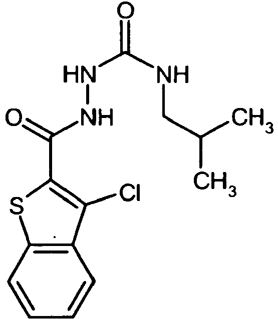 | Rₜ = 1.78 min [5] | |
| **29A** | 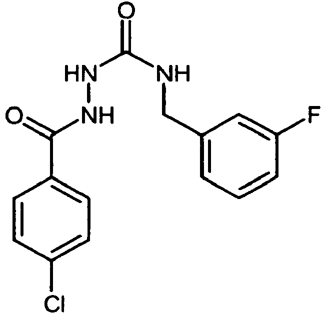 | Rₜ = 2.00 min [8] | |
| **30A** | 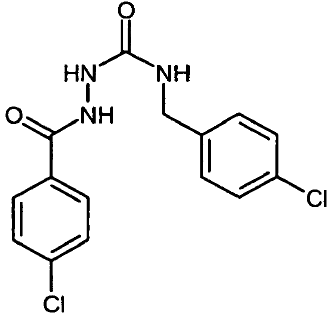 | Rₜ = 2.06 min [5] | |
| **31A** | 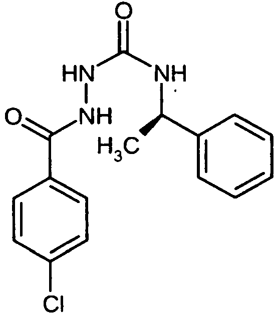 | Rₜ = 1.97 min [5] | |

### Beispiel 32A

### 2-(3-Brombenzoyl)-N-isobutylhydrazincarboxamid

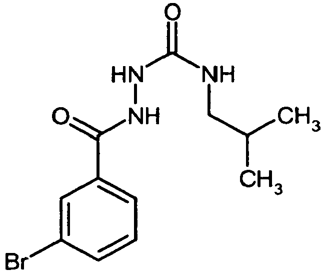

5.00 g (23.3 mmol) 3-Brombenzoesäurehydrazid werden bei Raumtemperatur in 50 ml Tetrahydrofuran vorgelegt. Zu dieser Suspension werden unter Rühren 2.70 g (27.2 mmol) Isobutylisocyanat, gelöst in 10 ml Tetrahydrofuran, zügig zugetropft. Man lässt das Gemisch zunächst bei Raumtemperatur weiter rühren und dann über Nacht stehen. Der nach Zugabe von 100 ml Diethylether entstehende Niederschlag wird abfiltriert und mit Diethylether nachgewaschen. Man erhält so eine erste Menge von 1.42 g (19% d. Th.) der Zielverbindung. Die Mutterlauge wird eingeengt, der Rückstand erneut in Diethylether aufgeschlämmt und filtriert. Nach Waschen mit Diethylether und Trocknen im Vakuum verbleiben weitere 5.62 g (77% d. Th.) der Zielverbindung.
LC/MS [Methode 5]: Rₜ = 1.78 min
¹H-NMR (400 MHz, DMSO-d₆): δ = 0.83 (d, 6H), 2.85 (t, 2H), 6.56 (br. t, 1H), 7.46 (t, 1H), 7.77 (d, 1H), 7.82 (s, 1H), 7.88 (d, 1H), 8.07 (s, 1H), 10.22 (s, 1H).

### Beispiel 33A

### 2-(2-Phenylbenzoyl)-N-isobutylhydrazincarboxamid

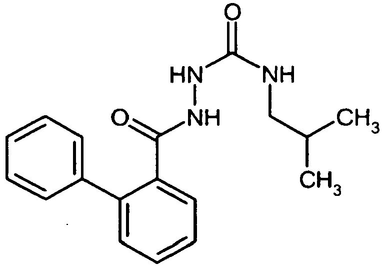

1.00 g (4.71 mmol) 2-Phenylbenzoesäurehydrazid werden bei Raumtemperatur in 10 ml Tetrahydrofuran vorgelegt. Unter Rühren werden 0.51 g (5.15 mmol) Isobutylisocyanat, gelöst in 2 ml Tetrahydrofuran, zügig zugetropft. Man lässt das Gemisch zunächst bei Raumtemperatur weiter rühren und dann über Nacht stehen. Zugabe eines gleichen Volumens an Diethylether und von 10 ml Cyclohexan führt zu einer Abscheidung einer geringen Menge eines Feststoffs, der abfiltriert und verworfen wird. Einengen des Filtrats ergibt 1.53 g (ca. 100% d. Th.) eines leicht THFfeuchten Produkts, das als solches weiter verwendet wird.
LC/MS [Methode 8]: Rₜ = 2.1 min.

### Beispiel 34A

### 2-(4-Chlorbenzoyl)-N-methylhydrazincarboxamid

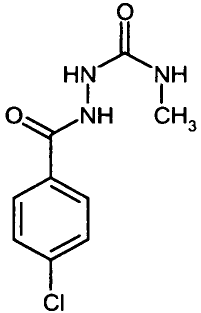

Zu 500 mg (2.93 mmol) 4-Chlorbenzoesäurehydrazid in 15 ml Dichlormethan werden nacheinander 575 mg (2.93 mmol) 4-Nitrophenyl-methylcarbamat sowie 417 mg (3.22 mmol) *N,N*-Diisopropylethylamin gegeben und das Gemisch bei Raumtemperatur über Nacht gerührt. Man engt ein, reinigt den Rückstand durch präparative HPLC [Methode 9] und erhält so 410 mg (61% d. Th.) der Zielverbindung.
LC/MS [Methode 5]: Rₜ = 1.24 min
¹H-NMR (400 MHz, DMSO-d₆): δ = 2.57 (d, 3H), 6.45 (br. d, 1H), 7.56, 7.58 (AA'-Teil eines AA'BB'-Systems, 2H), 7.89 (br. s, 1H), 7.89, 7.91 (BB'-Teil eines AA'BB'-Systems, 2H), 10.19 (s, 1H).

### Beispiel 35A

### N-Methyl-2-(4-methylbenzoyl)-hydrazincarboxamid

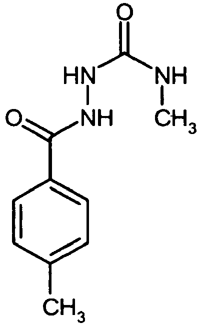

Zu 500 mg (3.33 mmol) 4-Methylbenzoesäurehydrazid in 15 ml Dichlormethan werden 653 mg (3.33 mmol) 4-Nitrophenyl-methylcarbamat und 473 mg (3.22 mmol) *N,N*-Diisopropylethylamin gegeben und das Gemisch bei Raumtemperatur über Nacht gerührt. Der gebildete Niederschlag wird durch Filtration gewonnen, mit Diethylether gewaschen und im Vakuum getrocknet. Man erhält so 602 mg (87% d. Th.) der Zielverbindung.
LC/MS [Methode 7]: Rₜ = 0.94 min.

### Beispiel 36A

### 5-(4-Chlorphenyl)-4-cyclopropyl-2,4-dihydro-3H-1,2,4-triazol-3-on

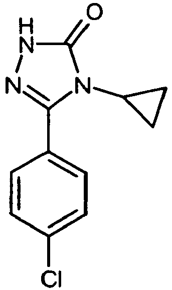

14.65 g (57.7 mmol) 2-(4-Chlorbenzoyl)-*N*-cyclopropylhydrazincarboxamid aus Beispiel 2A werden in 60 ml 2 N Natronlauge über Nacht zum Rückfluss erhitzt. Nach Abkühlen wird mit 2 N Salzsäure auf pH 1 angesäuert und das Gemisch mit Essigsäureethylester extrahiert. Die organische Phase wird über Natriumsulfat getrocknet, filtriert und eingeengt. Der Rückstand wird mit Dichlormethan verrührt, der entstehende Niederschlag abfiltriert, mit Dichlormethan nachgewaschen und im Vakuum getrocknet. Man erhält so 10.9 g (69% d. Th.) der Zielverbindung.
LC/MS [Methode 7]: Rₜ = 1.57 min
¹H-NMR (400 MHz, DMSO-d₆): δ = 0.50 - 0.62 (m, 2H), 0.79 - 0.93 (m, 2H), 3.10 (dddd, 1H), 7.57, 7.59 (AA'-Teil eines AA'BB'-Systems, 2H), 7.79, 7.81 (BB'-Teil eines AA'BB'-Systems, 2H), 11.85 (s, 1H).

### Beispiel 37A

### 4-Cyclopropyl-5-(2-fluorphenyl)-2,4-dihydro-3H-1,2,4-triazol-3-on

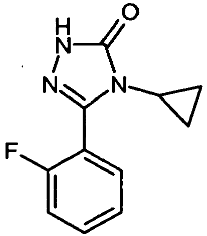

0.89 g (3.75 mmol) 2-(2-Fluorbenzoyl)-*N*-cyclopropylhydrazincarboxamid aus Beispiel 3A werden in 3.75 ml 2 N Natronlauge etwa 45 h lang zum Rückfluss erhitzt. Zur Vervollständigung der Umsetzung gibt man weitere 5 ml 6 N Natronlauge nach und erhitzt nochmals für 6 h zum Rückfluss. Nach Abkühlen wird mit 1 N Salzsäure unter Rühren angesäuert und das Reaktionsgemisch dreimal mit Essigsäureethylester extrahiert. Die vereinigten organischen Phasen werden über Magnesiumsulfat getrocknet und eingeengt. Man erhält so 0.74 g (73% d. Th.) der Zielverbindung, welche ohne weitere Reinigung weiter umgesetzt wird.
LC/MS [Methode 4]: Rₜ = 1.66 min.

### Beispiel 38A

### 4-Cyclopropyl-5-(2,4-difluorphenyl)-2,4-dihydro-3H-1,2,4-triazol-3-on

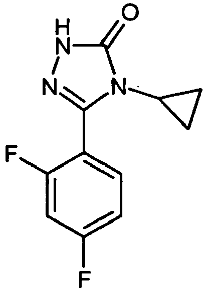

1.00 g (3.92 mmol) *N*-Cyclopropyl-2-(2,4-difluorbenzoyl)-hydrazincarboxamid aus Beispiel 5A werden in 4 ml 4 N Natronlauge 28 h lang zum Rückfluss erhitzt. Nach Abkühlen wird mit 2 N Salzsäure auf etwa pH 2 angesäuert, mit Wasser verdünnt und viermal mit Essigsäureethylester extrahiert. Die vereinigten organischen Phasen werden über Magnesiumsulfat getrocknet, filtriert, eingeengt und der Rückstand im Vakuum getrocknet. Man erhält so 0.415 g (31% d. Th.) der Zielverbindung, welche als solche weiter umgesetzt wird.
LC/MS [Methode 7]: Rₜ = 1.38 min.

### Beispiel 39A

### 5-(2-Chlorphenyl)-4-cyclopropyl-2,4-dihydro-3H-1,2,4-triazol-3-on

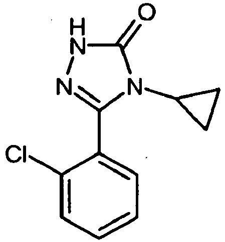

7.00 g (27.6 mmol) 2-(2-Chlorbenzoyl)-*N*-cyclopropylhydrazincarboxamid aus Beispiel 10A werden in 30 ml 3 N Natronlauge für etwa 60 h zum Rückfluss erhitzt (Umsatzkontrolle durch LC/MS-Analytik). Nach Abkühlen wird mit 1 N Salzsäure angesäuert und das Gemisch dreimal mit Essigsäureethylester extrahiert. Die vereinigten organischen Phasen werden zweimal mit gesättigter Natriumhydrogencarbonat-Lösung gewaschen, über Magnesiumsulfat getrocknet, filtriert und eingeengt. Man erhält so 3.32 g (48% d. Th.) der Zielverbindung.
LC/MS [Methode 7]: Rₜ = 1.38 min
¹H-NMR (400 MHz, DMSO-d₆): δ = 0.49 - 0.56 (m, 2H), 0.60 - 0.67 (m, 2H), 2.80 (dddd, 1H), 7.46 - 7.67 (m, 4H), 11.86 (br. s, 1H).

In analoger Weise werden erhalten:

| **Beispiel Nr.** | **Struktur** | **LC/MS Rₜ [Methode]** | **¹H-NMR (400 MHz, DMSO-d₆)** |
|---|---|---|---|
| **40A** | 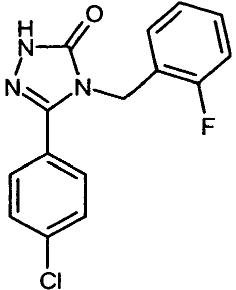 | Rₜ = 1.97 min [7] | δ = 4.97 (s, 2H), 7.02 (t, 1H), 7.10 (t, 1H), 7.13 (t, 1H), 7.29 (q, 1H), 7.53 (Zentrum eines AA'BB'-Systems, 4H), 12.15 (s, 1H). |
| **41A** | 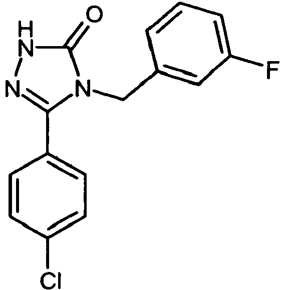 | Rₜ = 1.98 min [7] | δ = 4.94 (s, 2H), 6.90 (t, 1H), 7.08 (td, 1H), 7.35 (dt, 1H), 7.53 (Zentrum eines AA'BB'-Systems, 4H), 12.19 (s, 1H). |
| **42A** | 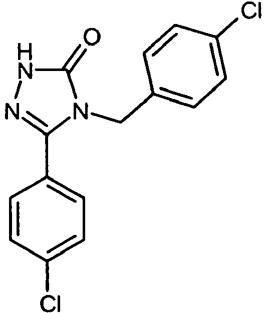 | Rₜ = 2.33 min [8] | |
| **43A** | 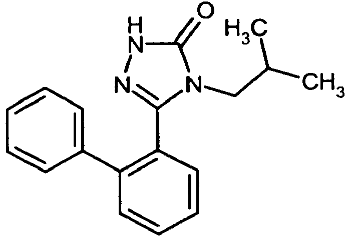 | Rₜ = 2.17 min [5] | |
| **44A** | 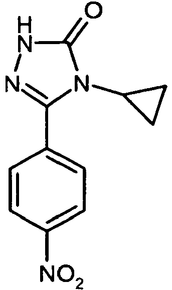 | Rₜ = 2.01 min [8] | |

In analoger Weise werden nach zusätzlicher Reinigung durch präparative HPLC [Methode 9] erhalten:

| **Beispiel Nr.** | **Struktur** | **LC/MS Rₜ [Methode]** | **¹H-NMR (400 MHz, DMSO-d₆)** |
|---|---|---|---|
| **45A** | 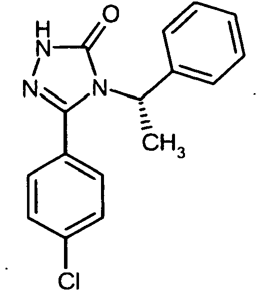 | Rₜ = 2.21 min [5] | δ = 1.76 (d, 3H), 5.19 (q, 1H), 7.18 - 7.36 (m, 5H), 7.36, 7.38 (AA'-Teil eines AA'BB'-Systems, 2H), 7.53, 7.55 (BB'-Teil eines AA'BB'-Systems, 2H), 12.00 (s, 1H). |
| **46A** | 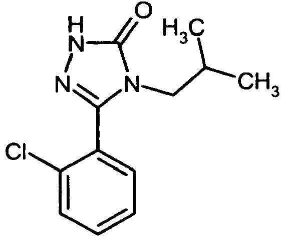 | Rₜ = 1.70 min [7] | δ = 0.66 (d, 6H), 1.57 -1.73 (m, 1H), 3.26 (d, 2H), 7.48 - 7.55 (m, 1H), 7.57 -7.64 (m, 2H), 7.64 - 7.69 (m, 1H), 12.00 (s, 1H). |
| **47A** | 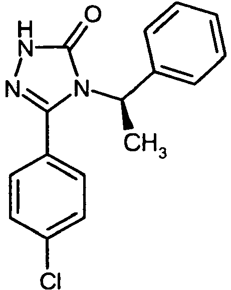 | Rₜ = 2.21 min [5] | |
| **48A** | 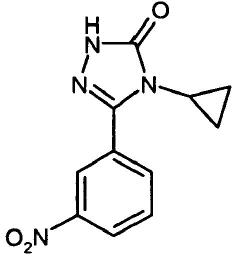 | Rₜ = 1.60 min [8] | |

### Beispiel 49A

### 5-(4-Chlorphenyl)-4-(2-methoxyethyl)-2,4-dihydro-3H-1,2,4-triazol-3-on

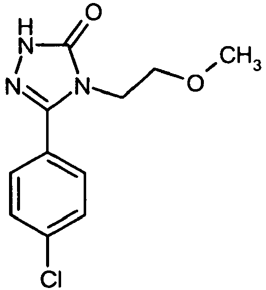

1.28 g (4.71 mmol) 2-(4-Chlorbenzoyl)-*N*-(2-methoxyethyl)-hydrazincarboxamid aus Beispiel 13A werden in 10 ml 3 N Natronlauge über Nacht zum Rückfluss erhitzt. Nach Abkühlen wird unter Eiskühlung mit 1 N Salzsäure auf etwa pH 2.5 angesäuert und der gebildete Niederschlag abfiltriert und im Vakuum getrocknet. Man erhält so 1.09 g (92% d. Th.) der Zielverbindung, die ohne weitere Reinigung umgesetzt wird.
LC/MS [Methode 7]: Rₜ = 1.53 mim
¹H-NMR (400 MHz, DMSO-d₆): δ = 3.11 (s, 3H), 3.45 (t, 2H), 3.83 (t, 2H), 7.58, 7.60 (AA'-Teil eines AA'BB'-Systems, 2H), 7.70, 7.72 (BB'-Teil eines AA'BB'-Systems, 2H), 11.98 (s, 1H).

### Analog erhält man:

| **Beispiel Nr.** | **Struktur** | **LC/MS Rₜ [Methode]** | **¹H-NMR (400 MHz, DMSO-d₆)** |
|---|---|---|---|
| **50A** | 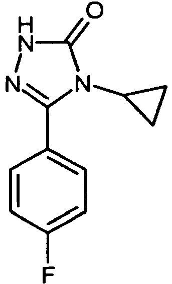 | Rₜ = 1.61 min [8] | δ = 0.48 - 0.62 (m, 2H), 0.77 - 0.91 (m, 2H), 3.08 (dddd, 1H), 7.35 (t, 2H), 7.81 (dd, 2H), 11.77 (s, 1H). |
| **51A** | 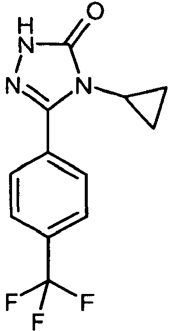 | Rₜ = 1.94 min [8] | δ = 0.51 - 0.64 (m, 2H), 0.80 - 0.95 (m, 2H), 3.15 (dddd, 1H), 7.87, 7.89 (AA'-Teil eines AA'BB'-Systems, 2H), 8.01, 8.03 (BB'-Teil eines AA'BB'-Systems, 2H), 11.99 (s, 1H). |
| **52A** | 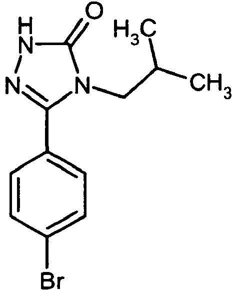 | Rₜ = 2.27 min [4] | δ = 0.68 (d, 6H), 1.58 -1.73 (m, 1H), 3.55 (d, 2H), 7.60, 7.62 (AA'-Teil eines AA'BB'-Systems, 2H), 7.72, 7.74 (BB'-Teil eines AA'BB'-Systems, 2H), 11.96 (s, 1H). |
| **53A** | 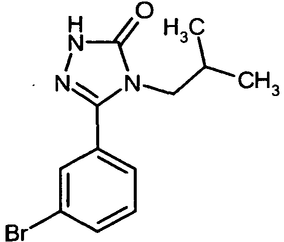 | Rₜ = 1.88 min [7] | δ = 0.69 (d, 6H), 1.57 -1.73 (m, 1H), 3.55 (d, 2H), 7.49 (t, 1H), 7.67 (br. d, 1H), 7.74 (br. d, 1H), 7.85 (t, 1H), 11.98 (br. s, 1H). |
| **54A** | 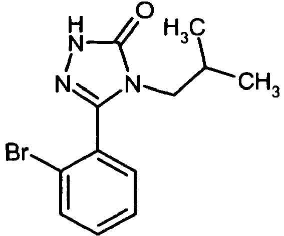 | Rₜ = 1.75 min [7] | δ = 0.68 (d, 6H), 1.57 -1.73 (m, 1H), 3.24 (d, 2H), 7.48 - 7.61 (m, 3H), 7.77 -7.85 (m, 1H), 11.90 (s, 1H). |
| **55A** | 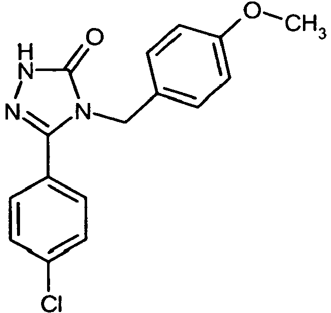 | Rₜ = 2.10 min [5] | δ = 3.69 (s, 3H), 4.86 (s, 2H), 6.83, 6.85 (AA'-Teil eines AA'BB'-Systems, 2H), 6.96, 6.99 (BB'-Teil eines AA'BB'-Systems, 2H), 7.54 (Zentrum eines AA'BB'-Systems, 4H), 12.11 (br. s, 1H). |
| **56A** | 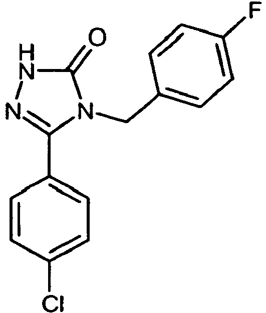 | Rₜ = 2.22 min [8] | δ = 4.91 (s, 2H), 7.07 -7.16 (m, 4H), 7.53 (s, 4H), 12.16 (br. s, 1H). |
| **57A** | 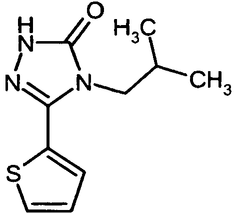 | Rₜ = 1.56 min [7] | δ = 0.80 (d, 6H), 1.79 -1.94 (m, 1H), 3.64 (d, 2H), 7.21 (dd, 1H), 7.55 (d, 1H), 7.75 (d, 1H), 11.96 (s, 1H). |
| **58A** | 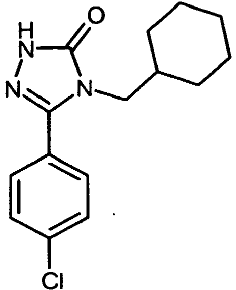 | Rₜ = 2.18 min [7] | |

| **Beispiel Nr.** | **Struktur** | **LC/MS Rₜ [Methode]** | **¹H-RMR (400 MHz, DMSO-d₆)** |
|---|---|---|---|
| **59A** | 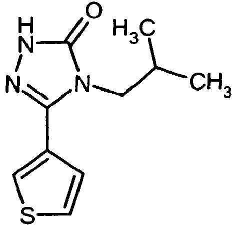 | Rₜ = 1.54 min [7] | |
| **60A** | 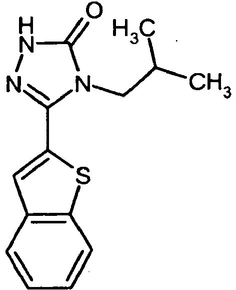 | Rₜ = 2.14 min [5] | |

In analoger Weise werden nach zusätzlicher Reinigung durch präparative HPLC [Methode 12] erhalten:

| **Beispiel Nr.** | **Struktur** | **LC/MS Rₜ [Methode]** |
|---|---|---|
| **61A** | 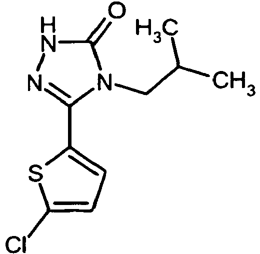 | Rₜ = 1.88 min [7] |
| **62A** | 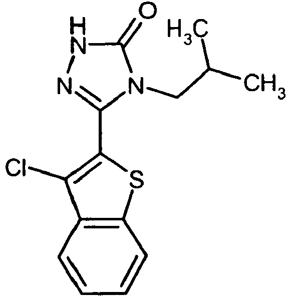 | Rₜ = 2.45 min [8] |

### Beispiel 63A

### 4-Cyclopropyl-5-(2-methoxyphenyl)-2,4-dihydro-3H-1,2,4-triazol-3-on

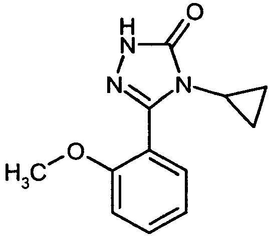

700 mg (2.81 mmol) *N*-Cyclopropyl-2-(2-methoxybenzoyl)-hydrazincarboxamid aus Beispiel 14A werden in 10 ml 3 N Natronlauge über Nacht zum Rückfluss erhitzt. Nach Abkühlen wird mit verdünnter Salzsäure auf pH 5-6 angesäuert, das Gemisch eingeengt und der Rückstand durch präparative HPLC [Methode 12] gereinigt. Man erhält so 240 mg (37% d. Th.) der Zielverbindung.
LC/MS [Methode 5]: Rₜ = 1.49 min
¹H-NMR (400 MHz, DMSO-d₆): δ = 0.37 - 0.50 (m, 2H), 0.53 - 0.67 (m, 2H), 2.76 (dddd, 1H), 3.84 (s, 3H), 7.05 (t, 1H), 7.17 (t, 1H), 7.34 (dd, 1H), 7.53 (ddd, 1H), ca. 11.5 - 12 (breit, 1H).

### Beispiel 64A

### 5-(4-Chlorphenyl)-4-methyl-2,4-dihydro-3H-1,2,4-triazol-3-on

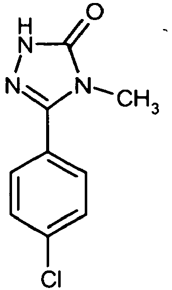

400 mg (1.78 mmol) 2-(4-Chlorbenzoyl)-*N*-methylhydrazincarboxamid aus Beispiel 34A werden in 7 ml 3 N Natronlauge über Nacht zum Rückfluss erhitzt. Nach Abkühlen wird mit wässriger Zitronensäure-Lösung auf pH ca. 11 eingestellt und der gebildete Niederschlag abfiltriert, mit Wasser gewaschen und im Vakuum getrocknet. Man erhält so 350 mg (95% d. Th.) der Zielverbindung.
LC/MS [Methode 7]: Rₜ = 1.39 min
¹H-NMR (400 MHz, DMSO-d₆): δ = 3.24 (s, 3H), 7.59, 7.61 (AA'-Teil eines AA'BB'-Systems, 2H), 7.71, 7.73 (BB'-Teil eines AA'BB'-Systems, 2H), 11.96 (s, 1H).

### Analog erhält man:

| **Beispiel Nr.** | **Struktur** | **LC/MS Rₜ [Methode]** | **¹H-NMR (400 MHz, DMSO-d₆)** |
|---|---|---|---|
| **65A** | 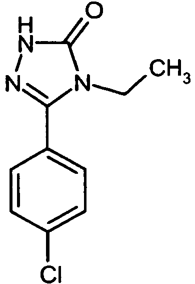 | Rₜ = 1.55 min [7] | δ = 1.08 (t, 3H), 3.70 (q, 2H), 7.60, 7.62 (AA'-Teil eines AABB'-Systems, 2H), 7.66, 7.68 (BB'-Teil eines AA'BB'-Systems, 2H), 11.95 (s, 1H). |
| **66A** | 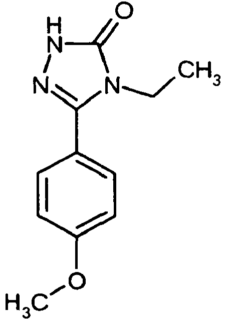 | Rₜ = 1.53 min [8] | |
| **67A** | 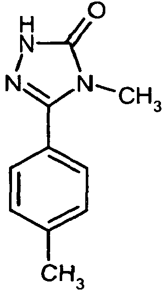 | Rₜ = 1.30 min [7] | |

### Beispiel 68A

### [3-(4-Chlorphenyl)-4-cyclopropyl-5-oxo-4,5-dihydro-1H-1,2,4-triazol-1-yl]essigsäureethylester

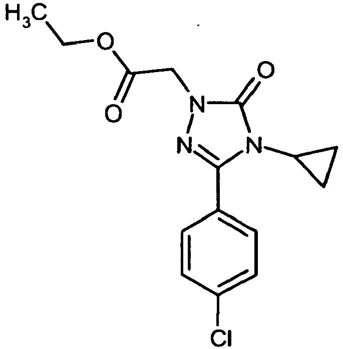

Zu 500 mg (2.12 mmol) 5-(4-Chlorphenyl)-4-cyclopropyl-2,4-dihydro-3*H*-1,2,4-triazol-3-on aus Beispiel 36A und 260 mg (2.12 mmol) Chloressigsäureethylester in 10 ml Acetonitril werden 586 mg (4.24 mmol) Kaliumcarbonat gegeben und das Gemisch unter Rühren für 2 Stunden zum Rückfluss erhitzt. Man engt danach ein, extrahiert den in Wasser aufgenommenen Rückstand mit Dichlormethan, trocknet die organische Phase über Natriumsulfat und engt erneut ein. Nach Reinigung durch Flash-Chromatographie über Kieselgel (Eluent: zuerst Dichlormethan, dann Dichlormethan/Methanol 100:1) erhält man 448 mg (66% d. Th.) der Zielverbindung.
MS [CIpos]: m/z = 339 (M+NH₄)⁺, 322 (M+H)⁺
¹H-NMR (400 MHz, DMSO-d₆): δ = 0.50 - 0.64 (m, 2H), 0.83 - 0.97 (m, 2H), 1.21 (t, 3H), 3.21 (dddd, 1H), 4.15 (q, 2H), 4.62 (s, 2H), 7.59, 7.61 (AA'-Teil eines AA'BB'-Systems, 2H), 7.81, 7.83 (BB'-Teil eines AA'BB'-Systems, 2H).

### Beispiel 69A

### [3-(4-Chlorphenyl)-4-(2-methoxyethyl)-5-oxo-4,5-dihydro-1H-1,2,4-triazol-1-yl]essigsäureethylester

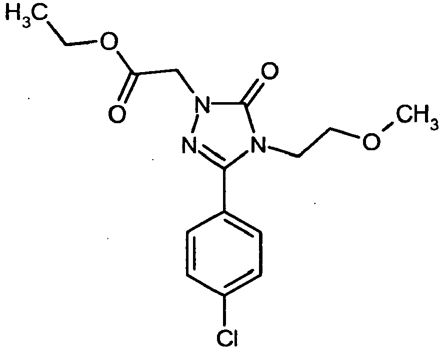

Zu 1.09 g (4.30 mmol) 5-(4-Chlorphenyl)-4-(2-methoxyethyl)-2,4-dihydro-3*H*-1,2,4-triazol-3-on aus Beispiel 49A und 1.19 g (8.59 mmol) Kaliumcarbonat in 20 ml Acetonitril werden 527 mg (4.30 mmol) Chloressigsäureethylester gegeben und das Gemisch unter Rühren für 3 Stunden zum Rückfluss erhitzt. Nach Reinigung des erhaltenen Rohprodukts durch präparative HPLC [Methode 9] erhält man 810 mg (42% d. Th.) der Zielverbindung.
LC/MS [Methode 5]: Rₜ = 2.16 min
¹H-NMR (400 MHz, DMSO-d₆): δ = 1.21 (t, 3H), 3.11 (s, 3H), 3.47 (t, 2H), 3.89 (t, 2H), 4.16 (q, 2H), 4.67 (s, 2H), 7.60, 7.63 (AA'-Teil eines AA'BB'-Systems, 2H), 7.72, 7.74 (BB'-Teil eines AA'BB'-Systems, 2H).

In analoger Weise werden nach Reinigung durch präparative HPLC [Methode 11] erhaltene:

| **Beispiel Nr.** | **Struktur** | **LC/MS oder HPLC, MS Rₜ [Methode]** | **¹H-NMR (400 MHz, DMSO-d₆)** |
|---|---|---|---|
| **70A** | 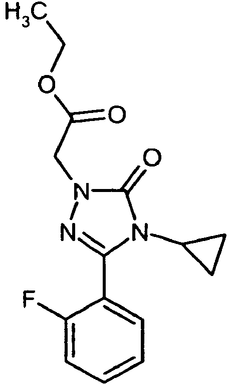 | Rₜ = 1.74 min [7] | δ = 0.45 - 0.68 (m, 2H), 0.68 - 0.82 (m, 2H), 1.21 (t, 3H), 2.92 - 3.00 (m, 1H), 4.15 (q, 2H), 4.63 (s, 2H), 7.35 - 7.47 (m, 2H), 7.59 - 7.69 (m, 2H). |
| **71A** | 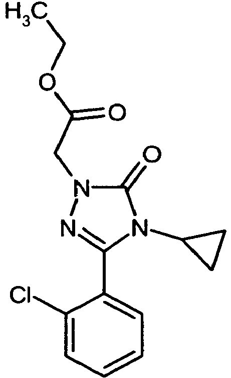 | Rₜ = 1.84 min [7] | δ = 0.48 - 0.61 (m, 2H), 0.61 - 0.75 (m, 2H), 1.21 (t, 3H), 2.90 (dddd, 1H), 4.15 (q, 2H), 4.62 (s, 2H), 7.49 - 7.55 (m, 1H), 7.58 -7.70 (m, 3H). |
| **72A** | 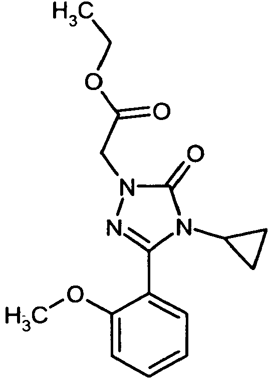 | HPLC [2]: Rₜ = 4.04 min; MS [ESIpos]: m/z = 318 (M+H)⁺ | δ = 0.38 - 0.52 (m, 2H), 0.58 - 0.72 (m, 2H), 1.21 (t, 3H), 2.86 (dddd, 1H), 3.86 (s, 3H), 4.15 (q, 2H), 4.58 (s, 2H), 7.06 (t, 1H), 7.19 (d, 1H), 7.33 (dd, 1H), 7.56 (ddd, 1H). |
| **73A** | 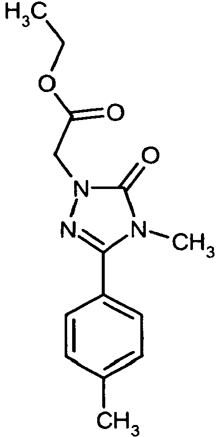 | HPLC [1] : Rₜ = 4.11 min; MS [CIpos]: m/z = 293 (M+NH₄)⁺, 276 (M+H)⁺ | δ = 1.21 (t, 3H), 2.38 (s, 3H), 3.30 (s, 3H), 4.16 (q, 2H), 4.65 (s, 2H), 7.35, 7.37 (AA'-Teil eines AA'BB'-Systems, 2H), 7.58, 7.60 (BB'-Teil eines AA'BB'-Systems, 2H). |
| **74A** | 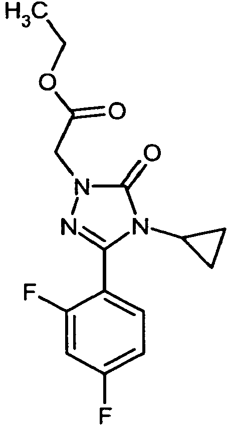 | Rₜ = 1.83 min [7] | |

In analoger Weise werden nach Reinigung durch präparative HPLC [Methode 12] erhalten:

| **Beispiel Nr.** | **Struktur** | **LC/MS Rₜ [Methode]** |
|---|---|---|
| **75A** | 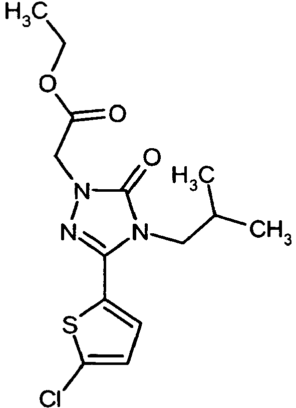 | Rₜ = 2.36 min [7] |
| **76A** | 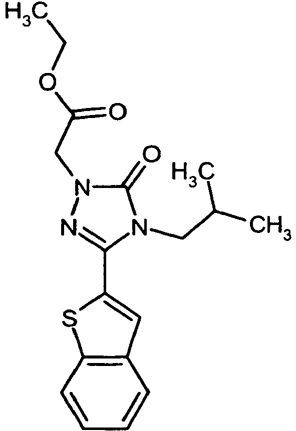 | Rₜ = 2.43 min [7] |
| **77A** | 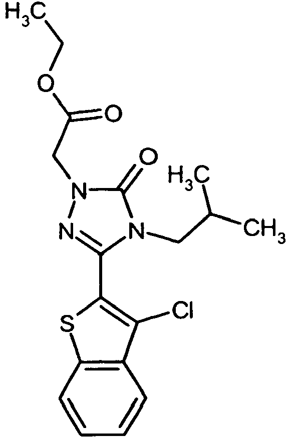 | Rₜ = 2.56 min [7] |

### Beispiel 78A

### [3-(4-Chlorphenyl)-4-(4-methoxyphenylmethyl)-5-oxo-4,5-dihydro-1H-1,2,4-triazol-1-yl]essigsäureethylester

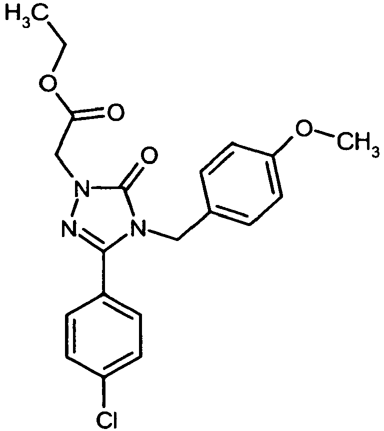

Zu 2.90 g (9.18 mmol) 5-(4-Chlorphenyl)-4-(4-methoxyphenylmethyl)-2,4-dihydro-3*H*-1,2,4-triazol-3-on aus Beispiel 55A und 1.13 g (9.18 mmol) Chloressigsäureethylester in 60 ml Acetonitril werden 2.54 g (18.4 mmol) Kaliumcarbonat gegeben und das Gemisch unter Rühren über Nacht zum Rückfluss erhitzt. Man engt danach ein, verteilt den Rückstand zwischen Essigsäureethylester und Wasser und extrahiert die wässrige Phase noch dreimal mit Essigsäureethylester. Durch Einengen der vereinigten und über Magnesiumsulfat getrockneten organischen Phasen erhält man 3.58 g (97% d. Th.) der Zielverbindung.
LC/MS [Methode 8]: Rₜ = 2.54 min
¹H-NMR (400 MHz, DMSO-d₆): δ = 1.22 (t, 3H), 3.70 (s, 3H), 4.18 (q, 2H), 4.73 (s, 2H), 4.94 (s, 2H), 6.83, 6.85 (AA'-Teil eines AA'BB'-Systems, 2H), 6.97, 6.99 (BB'-Teil eines AA'BB'-Systems, 2H), 7.55 (Zentrum eines AA'BB'-Systems, 4H).

### Analog werden erhalten:

| **Beispiel Nr.** | **Struktur** | **LC/MS Rₜ [Methode]** | **¹H-NMR (400 MHz, DMSO-d₆)** |
|---|---|---|---|
| **79A** | 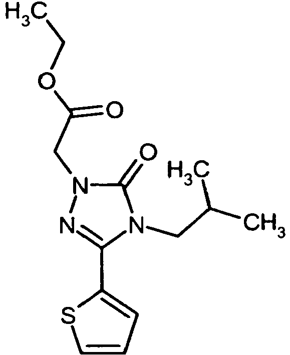 | Rₜ = 3.51 min [3] | δ = 0.81 (d, 6H), 1.20 (t, 3H), 1.80 - 1.95 (m, 1H), 3.71 (d, 2H), 4.15 (q, 2H), 4.67 (s, 2H), 7.24 (dd, 1H), 7.62 (d, 1H), 7.80 (d, 1H). |
| **80A** | 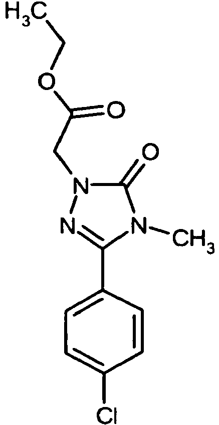 | Rₜ = 1.84 min [7] | |
| **81A** | 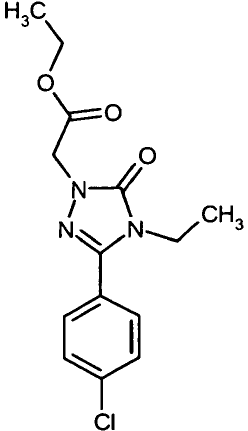 | Rₜ = 1.98 min [7] | |
| **82A** | 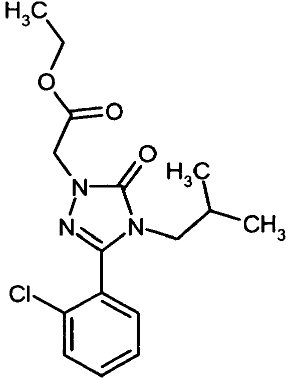 | Rₜ = 2.13 min [7] | |
| **83A** | 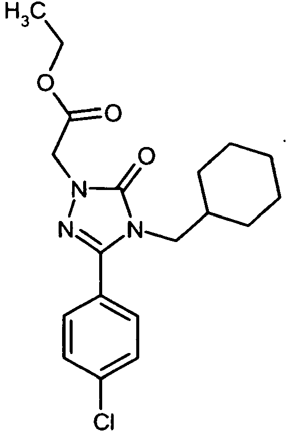 | Rₜ = 2.80 min [5] | |

Analog wird nach zusätzlicher Reinigung durch präparative HPLC [Methode 12] erhalten:

### Beispiel 84A

### [4-Isobutyl-5-oxo-3-(3-thienyl)-4,5-dihydro-1H-1,2,4-triazol-1-yl]essigsäuremethylester

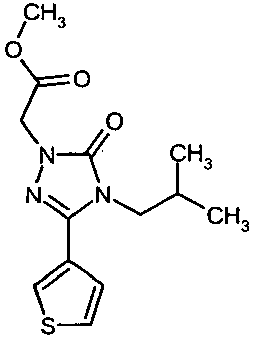

LC/MS [Methode 8]: Rₜ = 2.06 min.

### Beispiel 85A

### rac-2-[3-(4-Chlorphenyl)-4-cyclopropyl-5-oxo-4,5-dihydro-1H-1,2,4-triazol-1-yl]propionsäureethylester

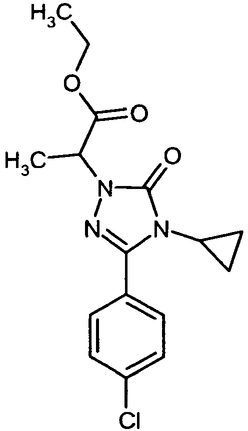

Zu 500 mg (2.12 mmol) 5-(4-Chlorphenyl)-4-cyclopropyl-2,4-dihydro-3*H*-1,2,4-triazol-3-on aus Beispiel 36A in 5 ml Acetonitril werden 384 mg (2.12 mmol) 2-Brompropionsäureethylester und 1.38 g (4.24 mmol) Cäsiumcarbonat gegeben und das Reaktionsgemisch über Nacht auf 85°C erhitzt. Man engt danach im Vakuum ein, verteilt den Rückstand zwischen Wasser und Dichlormethan, trocknet die abgetrennte organische Phase über Natriumsulfat und engt wieder ein. Nach Reinigung des Rückstands durch Flash-Chromatographie über Kieselgel (Eluent: zuerst Dichlormethan, dann Dichlormethan/Methanol 200:1) erhält man 729 mg (97% d. Th.) der Zielverbindung.
HPLC [Methode 2]: Rₜ = 4.47 min
MS [CIpos]: m/z = 353 (M+NH₄)⁺, 336 (M+H)⁺
¹H-NMR (400 MHz, DMSO-d₆): δ = 0.49 - 0.65 (m, 2H), 0.84 - 0.96 (m, 2H), 1.16 (t, 3H), 1.56 (d, 3H), 3.21 (dddd, 1H), 4.12 (q, 2H), 4.94 (q, 1H), 7.59, 7.61 (AA'-Teil eines AA'BB'-Systems, 2H), 7.81, 7.83 (BB'-Teil eines AA'BB'-Systems, 2H).

Analog erhält man nach zusätzlicher Reinigung durch präparative HPLC:

### Beispiel 86A

### 2-[3-(4-Chlorphenyl)-4-cyclopropyl-5-oxo-4,5-dihydro-1H-1,2,4-triazol-1-yl]-2-methylpropion-säureethylester

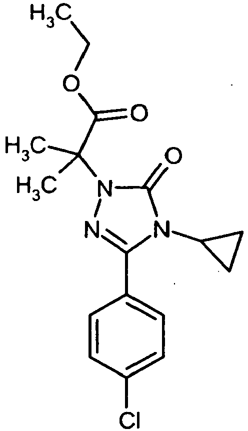

HPLC [Methode 2]: Rₜ = 4.75 min
MS [CIpos]: m/z = 367 (M+NH₄)⁺, 350 (M+H)⁺.
¹H-NMR (400 MHz, DMSO-d₆): δ = 0.48 - 0.61 (m, 2H), 0.82 - 0.96 (m, 2H), 1.15 (t, 3H), 1.64 (s, 6H), 3.17 (dddd, 1H), 4.12 (q, 2H), 7.59, 7.61 (AA'-Teil eines AA'BB'-Systems, 2H), 7.81, 7.84 (BB'-Teil eines AA'BB'-Systems, 2H).

### Beispiel 87A

### 3-[3-(4-Chlorphenyl)-4-cyclopropyl-5-oxo-4,5-dihydro-1H-1,2,4-triazol-1-yl]propionsäureethylester

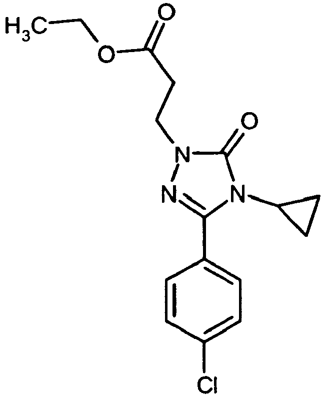

Zu 500 mg (2.12 mmol) 5-(4-Chlorphenyl)-4-cyclopropyl-2,4-dihydro-3*H*-1,2,4-triazol-3-on aus Beispiel 36A in 5 ml Acetonitril werden 290 mg (2.12 mmol) 3-Chlorpropionsäureethylester und 586 mg (4.24 mmol) Kaliumcarbonat gegeben und das Reaktionsgemisch über Nacht auf 85°C erhitzt. Man setzt 1.38 g (4.24 mmol) Cäsiumcarbonat und eine Spatelspitze Kaliumiodid hinzu und rührt für weitere 4 h bei 85°C. Man engt danach im Vakuum ein, verteilt den Rückstand zwischen Wasser und Dichlormethan, trocknet die abgetrennte organische Phase über Natriumsulfat und engt wieder ein. Nach Reinigung des Rückstands durch Flash-Chromatographie über Kieselgel (Eluent: zuerst Dichlormethan, dann Dichlormethan/Methanol 100:1) erhält man 580 mg (80% d. Th.) der Zielverbindung.
HPLC [Methode 2]: Rₜ = 4.18 min
MS [ESIpos]: m/z = 336 (M+H)⁺
¹H-NMR (400 MHz, DMSO-d₆): δ = 0.50 - 0.63 (m, 2H), 0.81 - 0.95 (m, 2H), 1.15 (t, 3H), 3.15 (dddd, 1H), 2.72 (t, 2H), 3.96 (t, 2H), 4.05 (q, 2H), 7.58, 7.60 (AA'-Teil eines AA'BB'-Systems, 2H), 7.78, 7.80 (BB'-Teil eines AA'BB'-Systems, 2H).

### Beispiel 88A

### [3-(4-Chlorphenyl)-4-cyclopropyl-5-oxo-4,5-dihydro-1H-1,2,4-triazol-1-yl]essigsäure

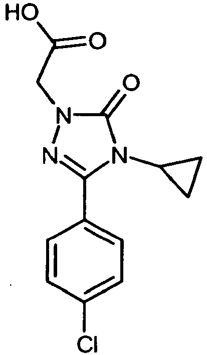

### Methode A:

4.84 g (15.0 mmol) [3-(4-Chlorphenyl)-4-cyclopropyl-5-oxo-4,5-dihydro-1*H*-1,2,4-triazol-1-yl]-essigsäureethylester aus Beispiel 69A werden in 12 ml Methanol vorgelegt und mit 4 ml 20%-iger Kalilauge 2 Stunden bei Raumtemperatur gerührt. Man engt ein und stellt mit 2 N Salzsäure auf etwa pH 1 ein. Der ausgefallene Feststoff wird abfiltriert, mit Wasser und Dichlormethan gewaschen und anschliessend im Vakuum getrocknet. Man erhält so 4.06 g (95% d. Th.) der Zielverbindung.
MS [ESIpos]: m/z = 294 (M+H)⁺; [ESIneg]: m/z = 292 (M-H)⁻
¹H-NMR (400 MHz, DMSO-d₆): δ = 0.50 - 0.64 (m, 2H), 0.82 - 0.97 (m, 2H), 3.20 (dddd, 1H), 4.47 (s, 2H), 7.58, 7.61 (AA'-Teil eines AA'BB'-Systems, 2H), 7.81, 7.83 (BB'-Teil eines AA'BB'-Systems, 2H).

### Methode B:

Zu 700 mg (2.97 mmol) 5-(4-Chlorphenyl)-4-cyclopropyl-2,4-dihydro-3*H*-1,2,4-triazol-3-on aus Beispiel 49A und 821 mg (5.94 mmol) Kaliumcarbonat in 14.6 ml Acetonitril werden 364 mg (2.97 mmol) Chloressigsäureethylester gegeben und das Gemisch unter Rühren für 3 Stunden zum Rückfluss erhitzt. Man engt danach ein, nimmt in 10 ml Methanol auf, fügt 1 ml 20%-ige Kalilauge hinzu und rührt 4 h bei Raumtemperatur. Zur Aufarbeitung wird das Reaktionsgemisch mit Wasser verdünnt, mit 2 N Salzsäure auf pH 3 angesäuert und dann fünfmal mit Essigsäureethylester extrahiert. Die vereinigten organischen Phasen werden über Magnesiumsulfat getrocknet und eingeengt. So erhält man 684 mg (79% d. Th.) der Zielverbindung.

Analog zu Beispiel 88A / Methode A werden erhalten:

| **Beispiel Nr.** | **Struktur** | **LC/MS Rₜ [Methode]** | **¹H-NMR (400 MHz, DMSO-d₆)** |
|---|---|---|---|
| **89A** | 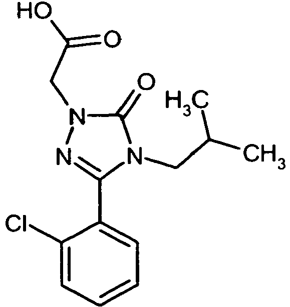 | Rₜ = 2.01 min [4] | δ = 0.68 (d, 6H), 1.59 -1.74 (m, 1H), 4.55 (s, 2H), 7.50 - 7.56 (m, 1H), 7.57 -7.71 (m, 3H), 13.09 (br. s, 1H). |
| **90A** | 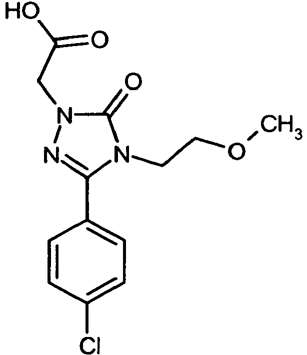 | Rₜ = 1.52 min [7] | δ = 3.11 (s, 3H), 3.47 (t, 2H), 3.89 (t, 2H), 4.54 (s, 2H), 7.60, 7.62 (AA'-Teil eines AA'BB'-Systems, 2H), 7.72, 7.75 (BB'-Teil eines AA'BB'-Systems, 2H), 13.13 (br. s, 1H). |
| **91A** | 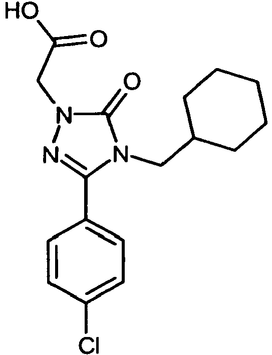 | Rₜ =2.16 mm [7] | δ = 0.67 - 0.80 (m, 2H), 0.93 - 1.10 (m, 3H), 1.35 -1.63 (m, 6H), 3.64 (d, 2H), 4.55 (s, 2H), 7.60, 7.63 (AA'-Teil eines AA'BB'-Systems, 2H), 7.69, 7.71 (BB'-Teil eines AA'BB'-Systems, 2H), 13.11 (br. s, 1H). |
| **92A** | 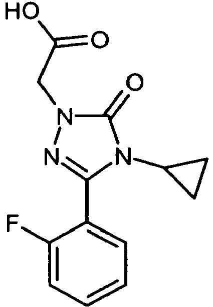 | Rₜ = 1.44 min [5] | |
| **93A** | 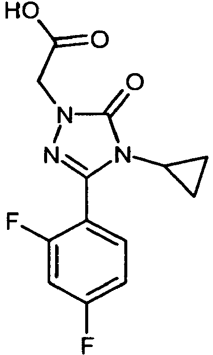 | Rₜ = 1.64 min [4] | |
| **94A** | 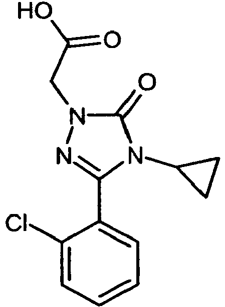 | Rₜ = 1.65 min [4] | |

### Beispiel 95A

### [4-Cyclopropyl-3-(2-methoxyphenyl)-5-oxo-4,5-dihydro-1H-1,2,4-triazol-1-yl]essigsäure

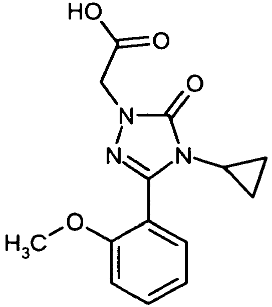

140 mg (0.44 mmol) [4-Cyclopropyl-3-(2-methoxyphenyl)-5-oxo-4,5-dihydro-1*H*-1,2,4-triazol-1-yl]-essigsäureethylester aus Beispiel 72A werden in 0.3 ml Methanol vorgelegt und mit 0.12 ml 20%-iger Kalilauge über Nacht bei Raumtemperatur gerührt. Man engt danach ein und stellt mit 1 N Salzsäure auf etwa pH 1 ein. Der ausgefallene Feststoff wird abfiltriert, mit Diethylether gewaschen und anschliessend im Vakuum getrocknet. Man erhält so 81 mg (63% d. Th.) der Zielverbindung.
HPLC [Methode 1]: Rₜ = 3.61 min
MS [ESIpos]: m/z = 290 (M+H)⁺; [ESIneg]: m/z = 288 (M-H)⁻
¹H-NMR (400 MHz, DMSO-d₆): δ = 0.38 - 0.51 (m, 2H), 0.57 - 0.72 (m, 2H), 2.86 (dddd, 1H), 3.86 (s, 3H), 4.46 (s, 2H), 7.06 (t, 1H), 7.19 (d, 1H), 7.33 (d, 1H), 7.55 (t, 1H), 13.07 (br. s, 1H).

### Beispiel 96A

### [3-(4-Chlorphenyl)-4-(4-methoxyphenylmethyl)-5-oxo-4,5-dihydro-1H-1,2,4-triazol-1-yl]essigsäure

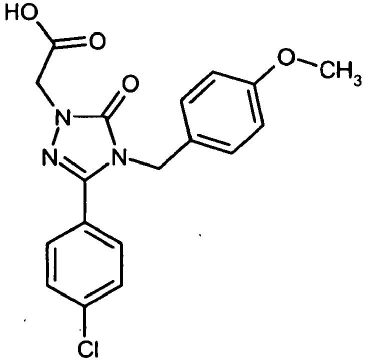

3.58 g (8.91 mmol) [3-(4-Chlorphenyl)-4-(4-methoxyphenylmethyl)-5-oxo-4,5-dihydro-1*H*-1,2,4-triazol-1-yl]-essigsäureethylester aus Beispiel 78A werden in 40 ml Methanol vorgelegt und mit 4 ml 20%-iger Kalilauge über Nacht bei Raumtemperatur gerührt. Man stellt mit 1 N Salzsäure auf pH 6 ein und reinigt durch präparative HPLC [Methode 12]. So erhält man 2.71 g (81% d. Th.) der Zielverbindung.
LC/MS [Methode 5]: Rₜ = 1.94 min
¹H-NMR (400 MHz, DMSO-d₆): δ = 3.70 (s, 3H), 4.60 (s, 2H), 4.93 (s, 2H), 6.83, 6.85 (AA'-Teil eines AA'BB'-Systems, 2H), 6.98, 7.00 (BB'-Teil eines AA'BB'-Systems, 2H), 7.56 (Zentrum eines AA'BB'-Systems, 4H), 13.19 (br. s, 1H).

### Analog werden erhalten:

| **Beispiel Nr.** | **Struktur** | **LC/MS Rₜ [Methode]** | **¹H-NMR (400 MHz, DMSO-d₆)** |
|---|---|---|---|
| **97A** | 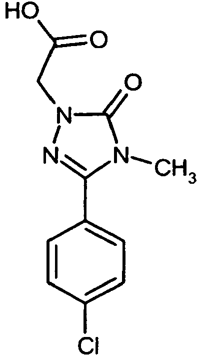 | Rₜ = 1.56 min [5] | δ = 3.29 (s, 3H), 3.91 (s, 2H), 7.58, 7.60 (AA'-Teil eines AA'BB'-Systems, 2H), 7.71, 7.73 (BB'-Teil eines AA'BB'-Systems, 2H). |
| **98A** | 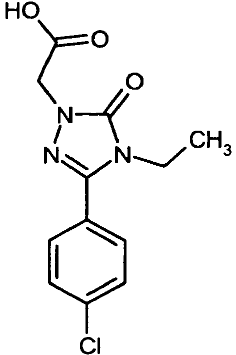 | Rₜ = 1.69 min [5] | δ = 1.11 (t, 3H), 3.75 (q, 2H), 3.92 (s, 2H), 7.59, 7.61 (AA'-Teil eines AA'BB'-Systems, 2H), 7.65, 7.67 (BB'-Teil eines AA'BB'-Systems, 2H). |
| **99A** | 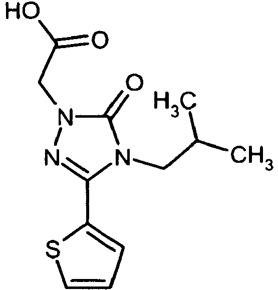 | Rₜ = 1.55 min [7] | δ=0.81 (d, 6H), 1.76 -1.93 (m, 1H), 3.66 (d, 2H), 3.91 (s, 2H), 7.20 (dd, 1H), 7.52 (dd, 1H), 7.72 (dd, 1H). |
| **100A** | 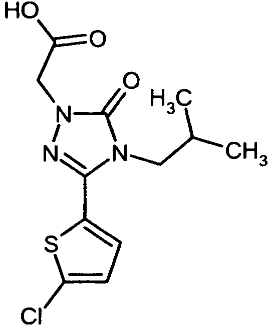 | Rₜ = 2.32 min [8] | |
| **101A** | 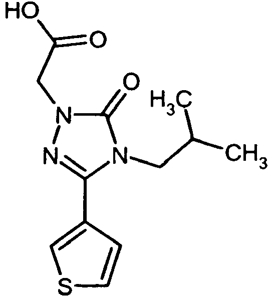 | Rₜ = 1.72 min [5] | |

| **Beispiel Nr.** | **Struktur** | **LC/MS Rₜ [Methode]** | **¹N-NMR (400 MHz, DMSO-d₆)** |
|---|---|---|---|
| **102A** | 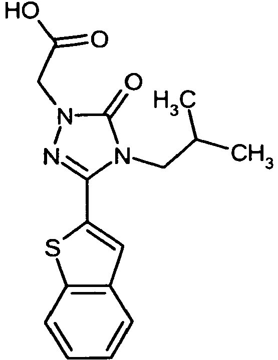 | Rₜ = 2.14 min [5] | |
| **103A** | 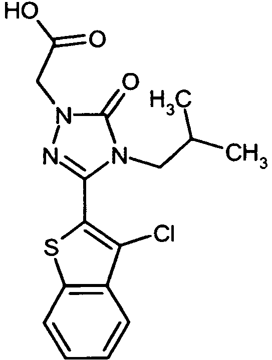 | Rₜ = 2.58 min [8] | |

### Analog zu Beispiel 88A / Methode B wird erhalten:

### Beispiel 104A

### [3-(3-Chlorphenyl)-4-cyclopropyl-5-oxo-4,5-dihydro-1H-1,2,4-triazol-1-yl]essigsäure

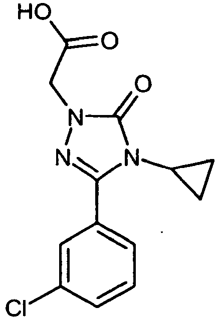

LC/MS [Methode 8]: Rₜ = 1.92 min
¹H-NMR (400 MHz, DMSO-d₆): δ = 0.52 - 0.66 (m, 2H), 0.82 - 0.97 (m, 2H), 3.25 (dddd, 1H), 7.56 (t, 1H), 7.62 (br. d, 1H), 7.77 (d, 1H), 7.83 (br. s, 1H), 13.17 (br. s, 1H).

### Beispiet 105A

### rac-2-[3-(4-Chlorphenyl)-4-cyclopropyl-5-oxo-4,5-dihydro-1H-1,2,4-triazol-1-yl]propionsäure

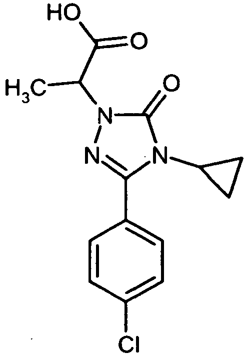

630 mg (1.88 mmol) *rac*-2-[3-(4-Chlorphenyl)-4-cyclopropyl-5-oxo-4,5-dihydro-1*H*-1,2,4-triazol-1-yl]-propionsäureethylester aus Beispiel 85A werden in 8 ml Methanol vorgelegt, mit 4 ml 20%-iger Kalilauge versetzt und 2 Stunden bei Raumtemperatur gerührt. Man entfernt das Methanol im Vakuum, säuert den wässrigen Rückstand mit 2 N Salzsäure an, extrahiert mit Dichlormethan, trocknet die organische Phase über Natriumsulfat und engt im Vakuum ein. So erhält man 463 mg (80% d. Th.) der Zielverbindung.
HPLC [Methode 2]: Rₜ = 3.96 min
MS [ESIpos]: m/z = 307 (M+H)^{+ 1}H-NMR (400 MHz, DMSO-d₆): δ = 0.47 - 0.68 (m, 2H), 0.82 - 0.97 (m, 2H), 1.54 (d, 3H), 3.20 (dddd, 1H), 4.83 (q, 1H), 7.58, 7.60 (AA'-Teil eines AA'BB'-Systems, 2H), 7.81, 7.83 (BB'-Teil eines AA'BB'-Systems, 2H), 13.02 (s, 1H).

### Analog erhält man:

### Beispiel 106A

### 2-[3-(4-Chlorphenyl)-4-cyclopropyl-5-oxo-4,5-dihydro-1H-1,2,4-triazol-1-yl]-2-methylpropionsäure

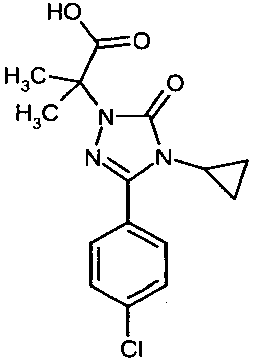

HPLC [Methode 2]: Rₜ = 4.17 min
MS [ESIpos]: m/z = 322 (M+H)⁺
¹H-NMR (400 MHz, DMSO-d₆): δ = 0.48 - 0.61 (m, 2H), 0.81 - 0.95 (m, 2H), 1.64 (s, 6H), 3.17 (dddd, 1H), 7.58, 7.60 (AA'-Teil eines AA'BB'-Systems, 2H), 7.80, 7.83 (BB'-Teil eines AA'BB'-Systems, 2H), 12.88 (s, 1H).

### Beispiel 107A

### 3-[3-(4-Chlorphenyl)-4-cyclopropyl-5-oxo-4,5-dihydro-1H-1,2,4-triazol-1-yl]propionsäure

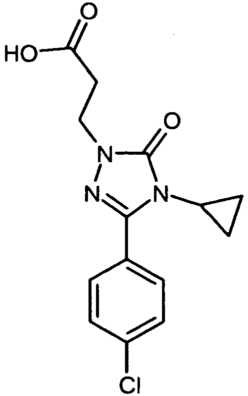

560 mg (1.67 mmol) 3-[3-(4-Chlorphenyl)-4-cyclopropyl-5-oxo-4,5-dihydro-1*H*-1,2,4-triazol-1-yl]-propionsäureethylester aus Beispiel 87A werden in 1.5 ml Methanol vorgelegt, mit 0.5 ml 20%-iger Kalilauge versetzt und 2 h bei Raumtemperatur gerührt. Man entfernt das Methanol im Vakuum, säuert den wässrigen Rückstand mit 2 N Salzsäure auf pH 1 an und isoliert den entstandenen Niederschlag durch Filtration. So erhält man 439 mg (73% d. Th.) der Zielverbindung.
HPLC [Methode 2]: Rₜ = 3.81 min
MS [ESIpos]: m/z = 308 (M+H)⁺
¹H-NMR (400 MHz, DMSO-d₆): δ = 0.50 - 0.63 (m, 2H), 0.80 - 0.97 (m, 2H), 2.66 (t, 2H), 3.15 (dddd, 1H), 3.92 (t, 2H), 7.58, 7.60 (AA'-Teil eines AA'BB'-Systems, 2H), 7.78, 7.80 (BB'-Teil eines AA'BB'-Systems, 2H), 12.36 (s, 1H).

### Beispiel 108A

### [3-(4-Methoxyphenyl)-5-oxo-4,5-dihydro-1H-1,2,4-triazol-1-yl]essigsäureethylester

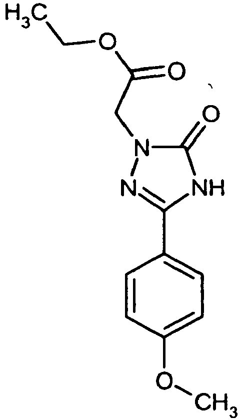

Zu 479 mg (2.00 mmol) *N*-(Ethoxycarbonyl)-4-methoxyphenylcarbonsäurethioamid [E.P. Papadopoulos, J. Org. Chem. 41 (6), 962-965 (1976)] in 10 ml Ethanol werden 618 mg (4.00 mmol) Hydrazinoessigsäureethylester-Hydrochlorid gegeben und das Gemisch für sechs Stunden zum Rückfluss erhitzt. Nach Abkühlen wird die resultierende Suspension mit Diethylether verrührt und der Niederschlag durch Filtration isoliert. Verrühren dieses Rohproduktes mit Wasser, erneutes Filtrieren und Trocknen im Vakuum ergibt 167 mg (30% d. Th.) der Zielverbindung.
LC/MS [Methode 7]: Rₜ = 1.54 min
¹H-NMR (400 MHz, DMSO-d₆): δ = 1.21 (t, 3H), 3.81 (s, 3H), 4.16 (q, 2H), 4.57 (s, 2H), 7.04, 7.06 (AA'-Teil eines AA'BB'-Systems, 2H), 7.72, 7.74 (BB'-Teil eines AA'BB'-Systems, 2H), 12.20 (s, 1H).

### Beispiele 109A

### 4-Ethyl-[3-(4-methoxyphenyl)-5-oxo-4,5-dihydro-1H-1,2,4-triazol-1-yl]essigsäureethylester

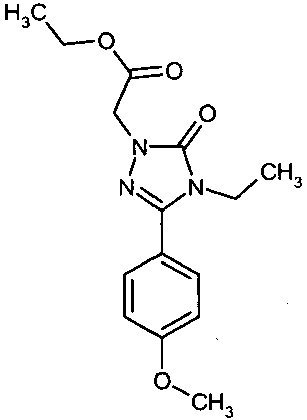

### Methode A:

6.1 mg (0.15 mmol) Natriumhydrid (60% in Mineralöl) werden in 0.5 ml Dimethylformamid vorgelegt und mit 40 mg (0.14 mmol) [3-(4-Methoxyphenyl)-5-oxo-4,5-dihydro-1*H*-1,2,4-thazol-1-yl]-essigsäureethylester aus Beispiel 108A in 2 ml Dimethylformamid versetzt. Man lässt 10 min bei Raumtemperatur rühren, gibt dann 22 mg (0.012 ml, 0.14 mmol) Iodethan hinzu und lässt über Nacht bei Raumtemperatur weiter rühren. Zur Aufarbeitung wird die Reaktionsmischung mit 2 ml Wasser versetzt, mit 1 N Salzsäure auf pH 2 gestellt und durch präparative HPLC aufgereinigt. Man erhält so 4.1 mg (9% d. Th.) der Zielverbindung.
LC/MS [Methode 4]: Rₜ = 2.20 min
¹H-NMR (400 MHz, DMSO-d₆): δ = 1.10 (t, 3H), 1.21 (t, 3H), 3.74 (q, 2H), 3.83 (s, 3H), 4.15 (q, 2H), 4.63 (s, 2H), 7.09, 7.11 (AA'-Teil eines AA'BB'-Systems, 2H), 7.72, 7.74 (BB'-Teil eines AA'BB'-Systems, 2H).

Daneben isoliert man 3.8 mg (9% d. Th.) [5-Ethoxy-3-(4-methoxyphenyl)-1*H*-1,2,4-triazol-1-yl]-essigsäureethylester:
LC/MS [Methode 4]: Rₜ = 2.59 min
¹H-NMR (400 MHz, DMSO-d₆): δ = 1.21 (t, 3H), 1.36 (t, 3H), 3.79 (s, 3H), 4.17 (q, 2H), 4.49 (q, 2H), 4.87 (s, 2H), 6.98, 7.01 (AA'-Teil eines AA'BB'-Systems, 2H), 7.82, 7.85 (BB'-Teil eines AA'BB'-Systems, 2H).

### Methode B:

Zu 200 mg (0.912 mmol) 4-Ethyl-5-(4-methoxyphenyl)-2,4-dihydro-3*H*-1,2,4-triazol-3-on aus Beispiel 66A und 252 mg (1.82 mmol) Kaliumcarbonat in 1.8 ml Acetonitril werden 112 mg (0.912 mmol) Chloressigsäureethylester gegeben und das Gemisch unter Rühren für 3 Stunden zum Rückfluss erhitzt. Nach Reinigung des erhaltenen Rohproduktes durch präparative HPLC [Methode 9] erhält man 212 mg (76% d. Th.) der Zielverbindung.

### Beispiel 110A

### 4-Ethyl-[3-(4-methoxyphenyl)-5-oxo-4,5-dihydro-1H-1,2,4-triazol-1-yl]essigsäure

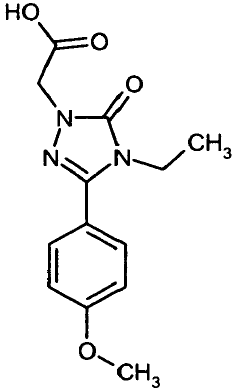

205 mg (0.67 mmol) 4-Ethyl-[3-(4-methoxyphenyl)-5-oxo-4,5-dihydro-1*H*-1,2,4-triazol-1-yl]-essigsäureethylester aus Beispiel 109A werden in 0.46 ml Methanol gelöst, mit 0.18 ml 20%-iger Kalilauge versetzt und über Nacht gerührt. Anschließend bringt man mit 1 N Salzsäure auf einen pH von 1, engt ein und trocknet den Rückstand im Vakuum. Man erhält so 207 mg der Zielverbindung als Rohprodukt, das als solches weiter umgesetzt wird.
LC/MS [Methode 4]: Rₜ = 1.65 min.
Analog wird erhalten:

### Beispiel 111A

### 4-Methyl-[3-(4-methylphenyl)-5-oxo-4,5-dihydro-1H-1,2,4-triazol-1-yl]essigsäure

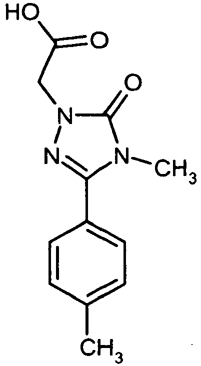

LC/MS [Methode 4]: Rₜ = 1.65 min.

### Beispiel 112A

### 2-Brom-1-(4-chlorphenyl)propan-1-on

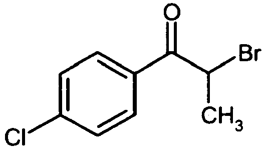

3000 mg (17.791 mmol) 1-(4-Chlorphenyl)propan-1-on werden in 15 ml Dichlormethan vorgelegt und mit einem Tropfen Bromwasserstoffsäure versetzt. Das Gemisch wird bei 35°C gerührt, dann werden 2843 mg (17.791 mmol) Brom tropfenweise so zugegeben, dass sich die Reaktionslösung nach jeder Zugabe wieder entfärbt. Drei Stunden nach Beginn der Reaktion engt man den Ansatz bis zur Trockene ein. Es werden 4490 mg (96% d. Th.) der Zielverbindung erhalten.
HPLC [Methode 2]: Rₜ = 4.92 min
¹H-NMR (400 MHz, DMSO-d₆): δ = 1.78 (d, 3H), 5.82 (q, 1H), 7.64 (d, 2H), 8.05 (d, 2H).

### Beispiel 113A

### N-[2-(4-Chlorphenyl)-2-oxoethyl]-N'-cyclopropylharnstoff

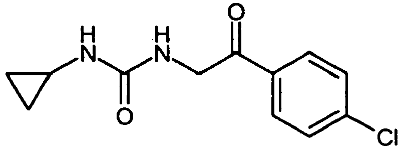

1000 mg (4.853 mmol) 2-Amino-1-(4-chlorphenyl)ethanon-Hydrochlorid werden in 20 ml Dichlormethan vorgelegt, auf 0°C gekühlt und tropfenweise mit einer Lösung von 363 mg (4.367 mmol) Cyclopropylisocyanat in 2 ml Dichlormethan versetzt. Es wird 10 min bei 0°C nachgerührt und dann eine Lösung von 627 mg (4.853 mmol) *N,N*-Diisopropylethylamin in 4 ml Dichlormethan zugetropft. Nach zweistündigem Rühren bei Raumtemperatur engt man das Reaktionsgemisch ein und reinigt das Rohprodukt durch Flash-Chromatographie an Kieselgel (Eluent: Dichlormethan/Methanol 100:1). Es werden 1000 mg (73% d. Th.) der Zielverbindung erhalten.
MS [CIpos]: m/z = 270 (M+NH₄)⁺
HPLC [Methode 2]: Rₜ = 3.99 min.

### Analog werden hergestellt:

| **Beispiel Nr.** | **Struktur** | **LC/MS oder HPLC, MS Rₜ [Methode]** | **¹H-NMR (400 MHz, DMSO-d₆)** |
|---|---|---|---|
| **114A** | 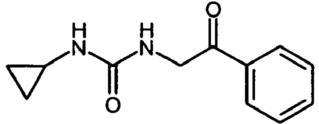 | Rₜ = 1.64 min [4] | δ = 0.36 (m, 2H), 0.59 (m, 2H), 2.43 (m, 1H), 4.56 (d, 2H), 6.18 (br. t, 1H), 6.49 (br. d, 1H), 7.54 (t, 2H), 7.66 (t, 1H), 7.97 (d, 2H). |
| **115A** | 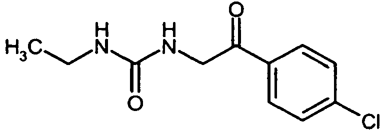 | MS [EIpos]: m/z = 241 (M+H)⁺; HPLC [2]: Rₜ = 3.69 min | δ = 1.00 (t, 3H), 3.02 (dq, 2H), 4.53 (d, 2H), 6.15 (br. t, 1H), 6.22 (br. t, 1H), 7.61 (d, 2H), 7.98 (d, 2H). |
| **116A** | 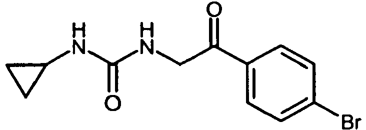 | Rₜ = 1.94 min [4] | δ = 0.36 (m, 2H), 0.59 (m, 2H), 2.95 (m, 1H), 4.52 (d, 2H), 6.20 (br. t, 1 H), 6.49 (br. d, 1H), 7.75 (d, 2H), 7.91 (d, 2H). |

### Beispiel 117A

### N-[2-(4-Chlorphenyl)-1-methyl-2-oxoethyl]-glycinethylester

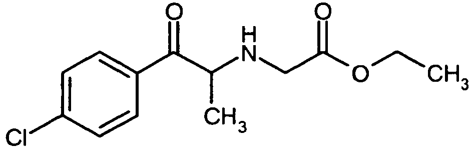

200 mg (0.808 mmol) 2-Brom-1-(4-chlorphenyl)propan-1-on aus Beispiel 112A werden in 1 ml Acetonitril gelöst und mit 226 mg (1.616 mmol) Glycinethylester-Hydrochlorid sowie 209 mg (1.616 mmol) *N,N*-Diisopropylethylamin versetzt. Nach Rühren über Nacht bei Raumtemperatur engt man das Reaktionsgemisch ein und verteilt den Rückstand zwischen Wasser und Dichlormethan. Die organische Phase wird abgetrennt, über Natriumsulfat getrocknet und eingeengt. Die Reinigung des Rohprodukts erfolgt mittels Flash-Chromatographie an Kieselgel (Eluent: zunächst Dichlormethan, dann Dichlormethan/Methanol 200:1). Es werden so 91 mg (42% d. Th.) der Zielverbindung erhalten.
MS [CIpos]: m/z = 270 (M+H)⁺
HPLC [Methode 2]: Rₜ = 3.77 min
¹H-NMR (400 MHz, DMSO-d₆): δ = 1.15 (t, 3H), 1.17 (d, 3H), 3.33 (s, 2H), 4.05 (q, 2H), 4.40 (q, 1H), 7.61 (d, 2H), 8.01 (d, 2H).

### Beispiele 118A

### 5-(4-Chlorphenyl)-1-cyclopropyl-1,3-dihydro-2H-imidazol-2-on

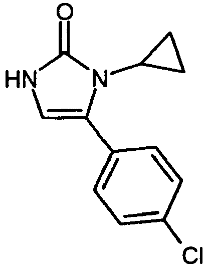

1525 mg (6.035 mmol) *N*-[2-(4-Chlorphenyl)-2-oxoethyl]-*N*'-cyclopropylhamstoff aus Beispiel 113A werden in 25 ml konzentrierter Salzsäure suspendiert, mit 25 ml Methanol versetzt und eine Stunde lang bei Raumtemperatur gerührt. Das Reaktionsgemisch wird bis zur Trockene eingeengt und der Rückstand durch Flash-Chromatographie an Kieselgel (Eluent: Dichlormethan/Methanol 100:1, dann 50:1) gereinigt. Man erhält 1300 mg (90% d. Th.) der Zielverbindung.
MS [CIpos]: m/z = 252 (M+NH₄)⁺
HPLC [Methode 2]: Rₜ = 3.92 min
¹H-NMR (400 MHz, DMSO-d₆): δ = 0.46 (m, 2H), 0.78 (m, 2H), 2.95 (tt, 1H), 6.62 (d, 1H), 7.44 (d, 2H), 7.54 (d, 2H), 10.17 (s, 1H).

### Analog werden hergestellt:

| **Beispiel Nr.** | **Struktur** | **LC/MS oder HPLC, MS Rₜ [Methode]** | **¹H-NMR (400 MHz, DMSO-d₆)** |
|---|---|---|---|
| **119A** | 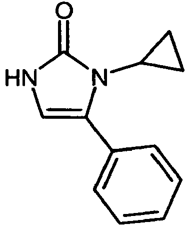 | MS [CIpos]: m/z = 218 (M+NH₄)⁺ | δ = 0.46 (m, 2H), 0.76 (m, 2H), 2.95 (tt, 1H), 6.53 (d, 1H), 7.29 (t, 1H), 7.38 (t, 2H), 7.51 (d, 2H), 10.09 (s, 1H). |
| **120A** | 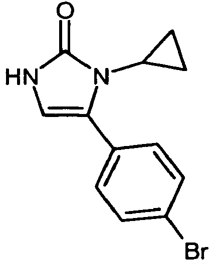 | MS [CIpos]: m/z = 296 und 298 (M+NH₄)⁺; HPLC [2]: Rₜ = 4.08 min | δ = 0.46 (m, 2H), 0.79 (m, 2H), 2.95 (tt, 1H), 6.63 (d, 1H), 7.47 (d, 2H), 7.57 (d, 2H), 10.17 (s, 1H). |

### Beispiel 121A

### 5-(4-Chlorphenyl)-1-ethyl-1,3-dihydro-2H-imidazol-2-on

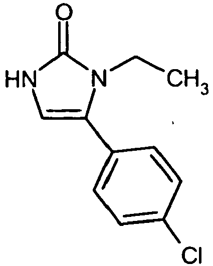

990 mg (4.113 mmol) *N*-[2-(4-Chlorphenyl)-2-oxoethyl]-*N*'-ethylharnstoff aus Beispiel 115A werden in 16 ml konzentrierter Salzsäure suspendiert, mit 16 ml Methanol versetzt und eine Stunde bei Raumtemperatur gerührt. Das Reaktionsgemisch wird bis zur Trockene eingeengt, der Rückstand mit Dichlormethan extrahiert, die organische Phase über Natriumsulfat getrocknet und wieder eingeengt. Man reinigt das Rohprodukt durch Flash-Chromatographie an Kieselgel (Eluent: Dichlormethan/Methanol 100:1, dann 50:1) und erhält so 701 mg (77% d. Th.) der Zielverbindung.
MS [ESIpos]: m/z = 223 (M+H)⁺
HPLC [Methode 2]: Rₜ = 3.94 min
¹H-NMR (400 MHz, DMSO-d₆): δ = 0.99 (t, 3H), 3.66 (q, 2H), 6.60 (d, 1H), 7.43 (d, 2H), 7.48 (d, 2H), 10.28 (s, 1H).

### Bespiel 122A

### [4-(4-Chlorphenyl)-3-cyclopropyl-2-oxo-2,3-dihydro-1H-imidazol-1-yl]-essigsäureethylester

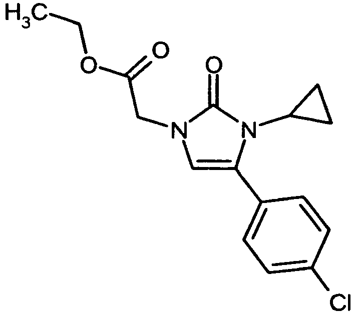

740 mg (3.15 mmol) 5-(4-Chlorphenyl)-1-cyclopropyl-1,3-dihydro-2*H*-imidazol-2-on aus Beispiel 118A werden in 15 ml Acetonitril gelöst und mit 386 mg (3.15 mmol) Chloressigsäureethylester sowie 872 mg (6.31 mmol) Kaliumcarbonat versetzt. Das Gemisch wird über Nacht unter Rückfluss gerührt. Man engt danach ein, verteilt den Rückstand zwischen Dichlormethan und Wasser, trennt die organische Phase ab, trocknet diese über Natriumsulfat und engt erneut ein. Das Rohprodukt wird durch Flash-Chromatographie an Kieselgel (Eluent: zunächst Dichlormethan, dann Dichlormethan/Methanol 200:1 → 100:1) gereinigt und ergibt so 602 mg (57% d. Th.) der Zielverbindung.
MS [ESIpos]: m/z = 321 (M+H)⁺
HPLC [Methode 2]: Rₜ = 4.33 min
¹H-NMR (400 MHz, DMSO-d₆): δ = 0.47 (m, 2H), 0.81 (m, 2H), 1.21 (t, 3H), 3.04 (tt, 1H), 4.14 (q, 2H), 4.40 (s, 2H), 6.77 (s, 1H), 7.47 (d, 2H), 7.55 (d, 2H).

### Beispiel 123A

### (3-Cyclopropyl-2-oxo-4-phenyl-2,3-dihydro-1H-imidazol-1-yl)-essigsäureethylester

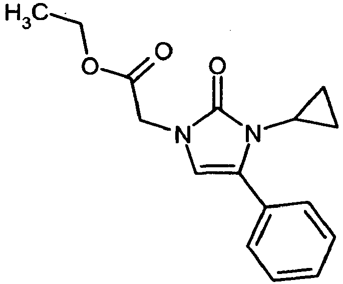

270 mg (1.35 mmol) 1-Cyclopropyl-5-phenyl-1,3-dihydro-2*H*-imidazol-2-on aus Beispiel 119A werden in 5 ml Acetonitril gelöst und mit 165 mg (1.35 mmol) Chloressigsäureethylester sowie 373 mg (2.70 mmol) Kaliumcarbonat versetzt. Das Gemisch wird 4 h unter Rückfluss gerührt, worauf weitere 165 mg (1.35 mmol) Chloressigsäureethylester zugegeben werden. Nach Rühren unter Rückfluss über Nacht engt man das Reaktionsgemisch ein, verteilt den Rückstand zwischen Dichlormethan und Wasser, trennt die organische Phase ab, trocknet diese über Natriumsulfat und engt erneut ein. Das Rohprodukt wird durch Flash-Chromatographie an Kieselgel (Eluent: Dichlormethan/Methanol zunächst 200:1, dann 100: 1) gereinigt und ergibt so 353 mg (91 % d. Th.) der Zielverbindung.
MS [ESIpos]: m/z = 287 (M+H)⁺
¹H-NMR (400 MHz, DMSO-d₆): δ = 0.47 (m, 2H), 0.78 (m, 2H), 1.21 (t, 3H), 3.04 (tt, 1H), 4.14 (q, 2H), 4.40 (s, 2H), 6.70 (s, 1H), 7.32 (t, 2H), 7.42 (t, 2H), 7.51 (d, 2H).

### Beispiel 124A

### [4-(4-Bromphenyl)-3-cyclopropyl-2-oxo-2,3-dihydro-1H-imidazol-1-yl]-essigsäureethylester

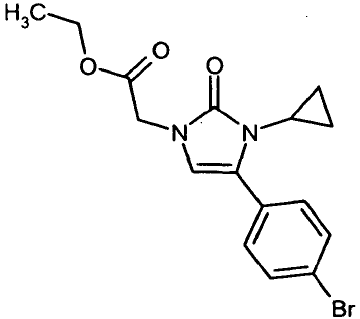

392 mg (1.40 mmol) 5-(4-Bromphenyl)-1-cyclopropyl-1,3-dihydro-2*H*-imidazol-2-on aus Beispiel 120A werden in 7.7 ml Acetonitril gelöst, mit 172 mg (1.40 mmol) Chloressigsäureethylester sowie 388 mg (2.81 mmol) Kaliumcarbonat versetzt und zwei Stunden zum Rückfluss erhitzt. Man filtriert nach dem Abkühlen, engt das Filtrat ein, verteilt den Rückstand zwischen Essigsäureethylester und Wasser, trennt die organische Phase ab, trocknet über Natriumsulfat und engt erneut ein. Man erhält so 502 mg (98% d. Th.) der Zielverbindung, welche ohne weitere Reinigung umgesetzt wird.
LC/MS [Methode 7]: Rₜ = 2.07 min
¹H-NMR (400 MHz, DMSO-d₆): δ = 0.40 - 0.53 (m, 2H), 0.74 - 0.88 (m, 2H), 1.21 (t, 3H), 3.04 (dddd, 1H), 4.14 (q, 2H), 4.40 (s, 2H), 6.78 (s, 1H), 7.47, 7.49 (AA'-Teil eines AA'BB'-Systems, 2H), 7.59, 7.61 (BB'-Teil eines AA'BB'-Systems, 2H).

### Beispiel 125A

### 2-[4-(4-Chlorphenyl)-3-cyclopropyl-2-oxo-2,3-dihydro-1H-imidazol-1-yl]-propionsäureethylester

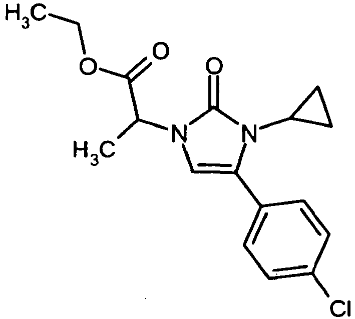

500 mg (2.13 mmol) 5-(4-Chlorphenyl)-1-cyclopropyl-1,3-dihydro-2*H*-imidazol-2-on aus Beispiel 118A werden in 5 ml Acetonitril gelöst und mit 386 mg (2.13 mmol) 2-Brompropionsäureethylester sowie 1388 mg (4.26 mmol) Caesiumcarbonat versetzt. Das Gemisch wird über Nacht unter Rückfluss gerührt. Man engt danach ein, verteilt den Rückstand zwischen Dichlormethan und Wasser, trennt die organische Phase ab, trocknet diese über Natriumsulfat und engt erneut ein. Das Rohprodukt wird durch Flash-Chromatographie an Kieselgel (Eluent: zunächst Dichlormethan, dann Dichlormethan/Methanol 200:1 → 100:1) gereinigt und ergibt so 338 mg (45% d. Th.) der Zielverbindung.
MS [ESIpos]: m/z = 335 (M+H)⁺
HPLC [Methode 2]: Rₜ = 4.50 min
¹H-NMR (400 MHz, DMSO-d₆): δ = 0.43 (m, 1H), 0.51 (m, 1H), 0.81 (m, 2H), 1.18 (t, 3H), 2.94 (d, 3H), 3.04 (tt, 1H), 4.12 (q, 2H), 4.74 (q, 1H), 6.92 (s, 1H), 7.47 (d, 2H), 7.58 (d, 2H).

### Beispiele 126A

### [4-(4-Chlorphenyl)-3-cyclopropyl-5-methyl-2-oxo-2,3-dihydro-1H-imidazol-1-yl]-essigsäureethylester

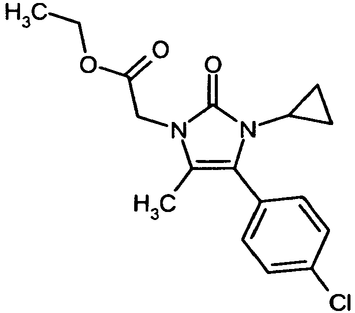

355 mg (1.32 mmol) *N*-[2-(4-Chlorphenyl)-1-methyl-2-oxoethyl]-glycinethylester aus Beispiel 117A werden in 5 ml Tetrahydrofuran vorgelegt, mit 109 mg (1.32 mmol) Cyclopropylisocyanat versetzt und über Nacht bei Raumtemperatur gerührt. Das Reaktionsgemisch wird eingeengt und der Rückstand durch Flash-Chromatographie an Kieselgel (Eluent: zunächst Dichlormethan, dann Dichlormethan/Methanol 200:1 → 100:1) gereinigt. Man erhält so 425 mg (91% d. Th.) der Zielverbindung.
MS [ESIpos]: m/z = 335 (M+H)⁺
HPLC [Methode 1]: Rₜ = 4.49 min
¹H-NMR (400 MHz, DMSO-d₆): δ = 0.14 (m, 1H), 0.25 (m, 1H), 0.39 (m, 1H), 0.78 (m, 1H), 1.21 (t, 3H), 2.23 (tt, 1H), 2.50 (s, 3H), 3.32 (s, 2H), 4.11 (q, 2H), 7.45 (d, 2H), 7.51 (d, 2H).

### Beispiel 127A

### [4-(4-Chlorphenyl)-3-ethyl-2-oxo-2,3-dihydro-1H-imidazol-1-yl]-essigsäure

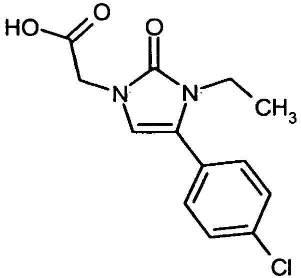

685 mg (3.076 mmol) 5-(4-Chlorphenyl)-1-ethyl-1,3-dlhydro-2*H*-imidazol-2-on aus Beispiel 119A werden in 10 ml Acetonitril vorgelegt, mit 377 mg (3.076 mmol) Chloressigsäureethylester und 850 mg (6.152 mmol) Kaliumcarbonat versetzt und über Nacht unter Rückfluss gerührt. Man engt das Reaktionsgemisch ein, verteilt den Rückstand zwischen Dichlormethan und Wasser, trennt die organische Phase ab, trocknet diese über Natriumsulfat und engt erneut ein. Das Rohprodukt wird durch Flash-Chromatographie an Kieselgel (Eluent: zunächst Dichlormethan, dann Dichlormethan/Methanol 100:1 → 50:1) gereinigt und ergibt so 226 mg (26% d. Th.) der Zielverbindung.
MS [ESIpos]: m/z = 281 (M+H)⁺
HPLC [Methode 2]: Rₜ = 3.89 min
¹H-NMR (400 MHz, DMSO-d₆): δ = 1.02 (t, 3H), 3.69 (q, 2H), 4.13 (s, 2H), 6.70 (s, 1H), 7.41 (d, 2H), 7.49 (d, 2H), 10.30 (br. s, 1H).

### Beispiel 128A

### (3-Cyciopropyl-2-oxo-4-phenyl-2,3-dihydro-1H-imidazol-1-yl)-essigsäure

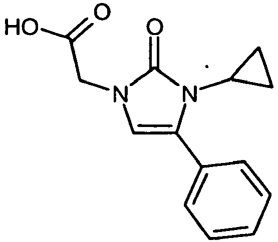

350 mg (1.222 mmol) (3-Cyclopropyl-2-oxo-4-phenyl-2,3-dihydro-1*H*-imidazol-1-yl)-essigsäureethylester aus Beispiel 123A werden in 2 ml Methanol vorgelegt, mit 0.5 ml 20%-iger Kalilauge versetzt und zwei Stunden bei Raumtemperatur gerührt. Man zieht das Methanol am Rotationsverdampfer ab, säuert den Rückstand mit 2 N Salzsäure an und extrahiert mit Dichlormethan. Die organische Phase wird abgetrennt, über Natriumsulfat getrocknet und eingeengt. Zur Reinigung erfolgt eine Flash-Chromatographie an Kieselgel (Eluent: Dichlormethan/Methanol zunächst 50:1, dann 25:1). Es werden so 166 mg (53% d. Th.) der Zielverbindung erhalten.
MS [ESIpos]: m/z = 259 (M+H)⁺
HPLC [Methode 2]: Rₜ = 3.84 min
¹H-NMR (400 MHz, DMSO-d₆): δ = 0.47 (m, 2H), 0.78 (m, 2H), 3.04 (tt, 1H), 4.30 (s, 2H), 6.69 (s, 1H), 7.32 (t, 1H), 7.41 (t, 2H), 7.51 (d, 2H), 12.96 (br. s, 1H).

### Beispiel 129A

### [4-(4-Bromphenyl)-3-cyclopropyl-2-oxo-2,3-dihydro-1H-imidazol-1-yl]-essigsäure

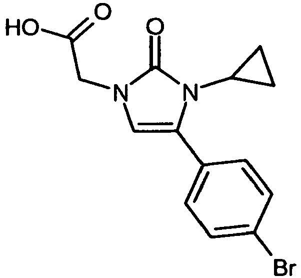

502 mg (1.28 mmol) [4-(4-Bromphenyl)-3-cyclopropyl-2-oxo-2,3-dihydro-1*H*-imidazol-1-yl]-essigsäureethylester aus Beispiel 124A werden in 0.94 ml Methanol vorgelegt, mit 0.34 ml 20%-iger Kalilauge versetzt und über Nacht bei Raumtemperatur gerührt. Man stellt mit 1 N Salzsäure auf pH 3, gewinnt den entstehenden Niederschlag durch Filtration, wäscht das Produkt mit Wasser und trocknet es im Vakuum. Man erhält so 369 mg (80% d. Th.) der Zielverbindung, welche ohne weitere Reinigung umgesetzt wird.
LC/MS [Methode 7]: Rₜ = 1.71 min
¹H-NMR (400 MHz, DMSO-d₆): δ = 0.40 - 0.53 (m, 2H), 0.74 - 0.88 (m, 2H), 3.03 (dddd, 1H), 4.30 (s, 2H), 6.78 (s, 1H), 7.46, 7.49 (AA'-Teil eines AA'BB'-Systems, 2H), 7.59, 7.61 (BB'-Teil eines AA'BB'-Systems, 2H), 12.98 (br. s, 1H).

### Analog werden hergestellt:

| **Beispiel Nr.** | **Struktur** | **LC/MS oder HPLC, MS Rₜ [Methode]** | **¹H-NMR (400 MHz, DMSO-d₆)** |
|---|---|---|---|
| **130A** | 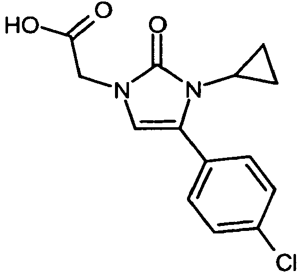 | Rₜ = 2.42 min [4] | δ = 0.39 - 0.53 (m, 2H), 0.73 - 0.88 (m, 2H), 3.03 (dddd, 1H), 4.30 (s, 2H), 6.77 (s, 1H), 7.45, 7.48 (AA'-Teil eines AA'BB'-Systems, 2H), 7.53, 7.55 (BB'-Teil eines AA'BB'-Systems, 2H), 12.99 (br. s, 1H). |
| **131A** | 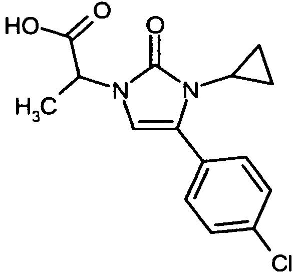 | MS [ESIpos]: m/z = 307 (M+H)⁺; HPLC [2]: Rₜ = 4.02 min | δ = 0.42 (m, 1H), 0.52 (m, 1H), 0.81 (m, 2H), 1.51 (d, 3H), 3.04 (tt, 1H), 4.66 (q, 1H), 6.91 (s, 1H), 7.46 (d, 2H), 7.57 (d, 2H), 12.97 (br. s, 1H). |
| **132A** | 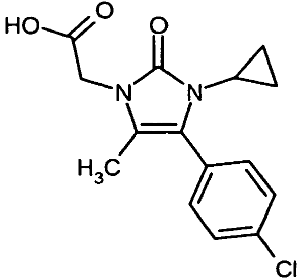 | MS [ESIpos]: m/z = 307 (M+H)⁺; HPLC [2]: R, = 4.02 min | δ = 0.40 (m, 2H), 0.67 (m, 2H), 1.96 (s, 3H), 2.89 (tt, 1H), 4.34 (s, 2H), 7.44 (d, 2H), 7.49 (d, 2H), 13.01 (br. s, 1H). |

### Beispiel 133A

### 2-Chlor-N-{1-methyl-1-[3-(trifluormethyl)phenyl]ethyl}acetamid

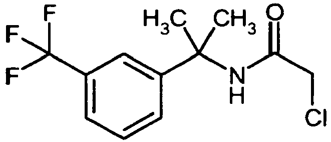

2.5 g (12.3 mmol) der Verbindung aus Beispiel 1A und 1.70 g (12.3 mmol) Kaliumcarbonat werden in 30 ml Dichlormethan vorgelegt und bei RT langsam mit einer Lösung von 1.46 g (12.9 mmol) Chloracetylchlorid in 5 ml Dichlormethan versetzt. Die Mischung wird 3 h bei RT gerührt und dann mit 150 ml Wasser und langsam mit 30 ml 1 N Salzsäure versetzt. Es wird dreimal mit Dichlormethan extrahiert. Die vereinigten organischen Phasen werden über Magnesiumsulfat getrocknet und am Rotationsverdampfer vom Lösungsmittel befreit. Man erhält 2.65 g (77% d. Th.) der Titelverbindung.
LC/MS [Methode 17]: Rₜ = 3.37 min; m/z = 280 (M+H)⁺
¹H-NMR (400 MHz, DMSO-d₆): δ = 1.59 (s, 6H), 4.05 (s, 2H), 7.50-7.67 (m, 4H), 8.60 (s, 1H).

### Beispiel 134A

### 2-Chlor-N-[2-(trifluormethyl)benzyl]acetamid

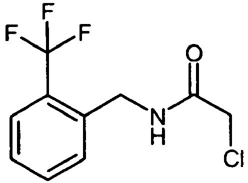

Analog zu Beispiel 133A erhält man aus 2.5 g (14.3 mmol) 2-Trifluorbenzylamin und 1.69 g (15.0 mmol) Chloracetylchlorid 2.43 g (68% d. Th.) der Titelverbindung.
LC/MS [Methode 18]: Rₜ = 2.00 min; m/z = 252 (M+H)⁺
¹H-NMR (400 MHz, DMSO-d₆): δ = 4.18 (s, 2H), 4.49 (d, J = 6 Hz, 2H), 7.47-7.52 (m, 2H), 7.68 (t, J = 7.5 Hz, 1H), 7.73 (d, J = 7.8 Hz, 1H), 8.81 (br. t, 1H).

### Beispiele 135A

### 5-Brom-4-(2-fluorbenzyl)-2,4-dihydro-3H-1,2,4-triazol-3-on

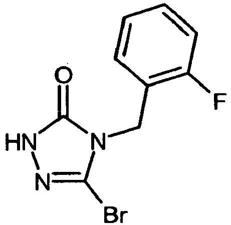

### Stufe a): Herstellung von N-(2-Fluorbenzyl)-2-formylhydrazincarboxamid

Unter Argon werden 1.99 g (33 mmol) Formylhydrazin in 80 ml THF vorgelegt. Die Lösung wird auf 50°C erwärmt, tropfenweise mit einer Lösung von 5.00 g (33 mmol) 2-Fluorbenzylisocyanat in 50 ml THF versetzt und die resultierende Mischung 30 min bei 50°C weitergerührt. Danach wird sie am Rotationsverdampfer vom Lösungsmittel befreit. Der Rückstand wird mit Diethylether verrührt, das Präzipitat abgesaugt, mit Diethylether nachgewaschen und der weiße Feststoff im Hochvakuum getrocknet. Man erhält 5.73 g (82% d. Th.) des Zielprodukts.
LC/MS [Methode 7]: Rₜ = 0.88 min; m/z = 212 (M+H)⁺.

### Stufe b): Herstellung von 4-(2-Fluorbenzyl)-2,4-dihydro-3H-1,2,4-triazol-3-on

Das Produkt aus Stufe a (5.73 g, 27.1 mmol) wird in 60 ml 3 M Natronlauge 5 h unter Rückfluss verrührt. Die Mischung wird dann in einem Eisbad abgekühlt und langsam mit 1 N Salzsäure auf pH 2 angesäuert. Der ausgefallene Feststoff wird abgesaugt, mit Wasser nachgewaschen und im Hochvakuum getrocknet. Man erhält 3.38 g (64% d. Th.) des Zielprodukts.
LC/MS [Methode 17]: Rₜ = 1.35 min; m/z = 194 (M+H)⁺.

### Stufe c): Herstellung von 5-Brom-4-(2-fluorbenzyl)-2,4-dihydro-3H-1,2,4-triazol-3-on

Das Produkt aus Stufe b (3.35 g, 17.3 mmol) wird zusammen mit Natriumhydroxid (970 mg, 24.2 mmol) in 37 ml Wasser vorgelegt. Bei RT wird unter Rühren Brom (893 µl, 17.3 mmol) zugetropft. Während der Zugabe fällt ein hellbrauner Feststoff aus. Es wird über Nacht bei RT nachgerührt. Der ausgefallene Feststoff wird abgesaugt, mit etwas Wasser nachgewaschen und dann im Hochvakuum getrocknet. Man erhält 4.25 g des Zielprodukts in ausreichender Reinheit (ca. 83% laut LC/MS).
LC/MS [Methode 8]: Rₜ = 1.81 min; m/z = 272 (M+H)⁺
¹H-NMR (400 MHz, DMSO-d₆): δ = 4.84 (s, 2H), 7.14-7.28 (m, 3H), 7.35-7.42 (m, 1H), 12.22 (s, 1H).

### Beispiel 136A

### 5-Brom-4-(2-methoxyethyl)-2,4-dihydro-3H-1,2,4-triazol-3-on

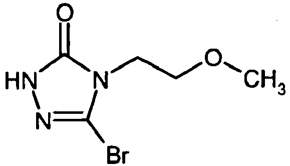

Die Titelverbindung wird analog zur synthetischen Sequenz, die für Beispiel 135A beschrieben ist, ausgehend von 2-Methoxyethylisocyanat hergestellt.
LC/MS [Methode 3]: Rₜ = 2.12 min; m/z = 222 (M+H)⁺
¹H-NMR (400 MHz, DMSO-d₆): δ = 3.24 (s, 3H), 3.51 (d, J = 5.5 Hz, 2H), 3.72 (d, J = 5.5 Hz, 2H), 12.10 (s, 1H).

### Beispiel 137A

### 5-Brom-4-(3-fluorbenzyl)-2,4-dihydro-3H-1,2,4-triazol-3-on

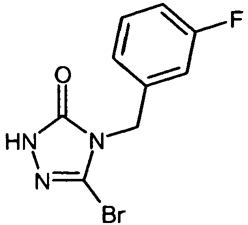

Die Titelverbindung wird analog zur synthetischen Sequenz, die für Beispiel 135A beschrieben ist, ausgehend von 3-Fluorbenzylisocyanat hergestellt.
LC/MS [Methode 8]: Rₜ = 1.79 min; m/z = 272 (M+H)⁺
¹H-NMR (400 MHz, DMSO-d₆): δ = 4.81 (s, 2H), 7.04-7.12 (m, 2H), 7.15 (dt, 1H), 7.43 (q, 1H), 12.3 (s, 1H).

### Beispiel 138A

### [3-Brom-4-(2-fluorbenzyl)-5-oxo-4,5-dihydro-1H-1,2,4-triazol-1-yl]-essigsäuremethylester

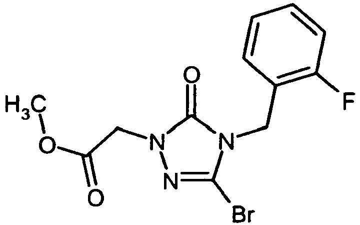

300 mg (1.1 mmol) der Verbindung aus Beispiel 135A werden zusammen mit 150 mg (1.38 mmol) Chloressigsäuremethylester und 168 mg (1.21 mmol) Kaliumcarbonat in 10 ml Acetonitril unter Rückfluss 2 h verrührt. Nach dem Abkühlen wird die Mischung mit Ethylacetat verdünnt und mit 1 N Salzsäure versetzt. Die organische Phase wird abgetrennt, mit gesättigter Kochsalz-Lösung gewaschen, über Natriumsulfat getrocknet und am Rotationsverdämpfer von den flüchtigen Komponenten befreit. Der Rückstand wird im Hochvakuum getrocknet. Das so erhaltene Produkt (360 mg, Reinheit ca. 73% laut LC/MS) wird ohne weitere Reinigung in der nächsten Synthesestufe eingesetzt.
LC/MS [Methode 17]: Rₜ = 2.83 min; m/z = 344 (M+H)⁺
¹H-NMR (400 MHz, DMSO-d₆): δ = 3.70 (s, 3H), 4.59 (s, 2H), 4.92 (s, 2H), 7.15-7.30 (m, 3H), 7.35-7.43 (m, 1H).

### Beispiel 139A

### [3-Brom-4-(2-fluorbenzyl)-5-oxo-4,5-dihydro-1H-1,2,4-triazol-l-yl]-essigsäure

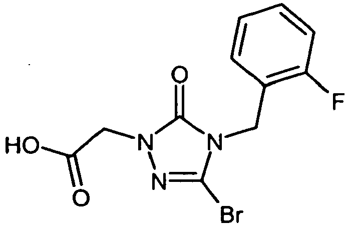

Die Verbindung aus Beispiel 138A (360 mg) wird in 10 ml Methanol gelöst und mit 4.2 ml einer 1 M wässrigen Lithiumhydroxid-Lösung versetzt. Die Mischung wird über Nacht bei RT gerührt und dann am Rotationsverdampfer vom Methanol befreit. Der Rückstand wird mit 200 ml Wasser verdünnt und langsam mit 1 N Salzsäure auf pH 2 angesäuert. Die wässrige Phase wird dreimal mit Ethylacetat extrahiert, die vereinigten organischen Phasen über Natriumsulfat getrocknet und das Lösungsmittel am Rotationsverdampfer entfernt. Der Rückstand wird in wenig DMSO gelöst und durch präparative HPLC (Methode 20) gereinigt. Man erhält 246 mg (0.75 mmol) der Titelverbindung.
LC/MS [Methode 19]: Rₜ = 2.00 min; m/z = 330 (M+H)⁺
¹H-NMR (400 MHz, DMSO-d₆): δ = 4.53 (s, 2H), 4.91 (s, 2H), 7.15-7.30 (m, 3H), 7.35-7.45 (m, 1H), 13.10 (br. s, 1H).

### Beispiel 140A

### 2-[3-Brom-4-(2-fluorbenzyl)-5-oxo-4,5-dihydro-1H-1,2,4-triazol-1-yl]-N-{2-[(2-trifluormethyl)-phenyl]ethyl}-acetamid

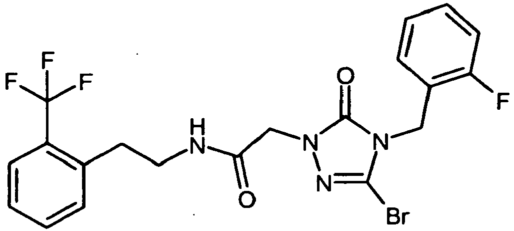

246 mg (0.64 mmol) der Verbindung aus Beispiel 139A und 121 mg HOBt (0.90 mmol) werden in 5 ml DMF vorgelegt und mit 172 mg EDC (0.90 mmol) versetzt. Nach 20 min Rühren bei RT wird 2-(2-Trifluormethylphenyl)ethylamin (139 mg, 0.74 mmol) hinzugegeben und die Mischung 1 h bei RT weitergerührt. Anschließend wird die Reaktionsmischung direkt über präparative HPLC (Methode 20) aufgetrennt. Man isoliert 321 mg (99% d. Th.) der Titelverbindung.
LC/MS [Methode 17]: Rₜ = 3.60 min; m/z = 503 (M+H)⁺
¹H-NMR (400 MHz, DMSO-d₆): δ = 2.90 (t, 2H), 3.29-3.37 (m, 2H), 4.36 (s, 2H), 4.90 (s, 2H), 7.19-7.29 (m, 3H), 7.37-7.50 (m, 3H), 7.62 (t, 1H), 7.69 (d, 1H), 8.33 (t, 1H).

### Beispiel 141A

### 2-[3-Brom-4-(2-fluorbenzyl)-5-oxo-4,5-dihydro-1H-1,2,4-triazol-1-yl]-N-{1-methyl-1-[(3-trifluormethyl)phenyl]ethyl}-acetamid

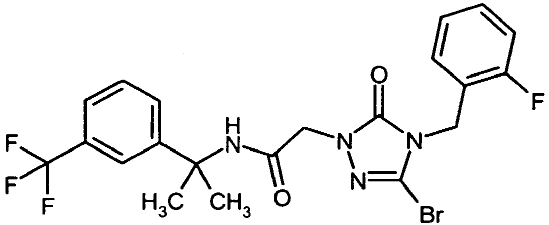

703 mg (2.15 mmol) der Verbindung aus Beispiel 135A werden zusammen mit 600 mg (2.15 mmol) 2-Chlor-*N*-{1-methyl-1-[3-(trifluormethyl)phenyl]ethyl}-acetamid (Beispiel 133A) und 593 mg (4.29 mmol) Kaliumcarbonat in 15 ml Acetonitril über Nacht unter Rückfluss gerührt. Nach dem Abkühlen wird die Mischung filtriert und das Filtrat direkt über präparative HPLC (Methode 20) gereinigt. Man erhält 780 mg (71% d. Th.) der Titelverbindung.
LC/MS [Methode 17] : Rₜ = 3.73 min; m/z = 515 (M+H)⁺
¹H-NMR (400 MHz, DMSO-d₆): δ = 1.59 (s, 6H), 4.43 (s, 2H), 4.86 (s, 2H), 7.10-7.26 (m, 3H), 7.34-7.41 (m, 1H), 7.50-7.58 (m, 2H), 7.61 (s, 1H), 7.65 (br. d, J ~ 6.8 Hz, 1H), 8.54 (s, 1H).

### Beispiel 142A

### 2-[3-Brom-4-(2-fluorbenzyl)-5-oxo-4,5-dihydro-1H-1,2,4-triazol-1-yl]-N-[(2-trifluormethyl)-benzyl]-acetamid

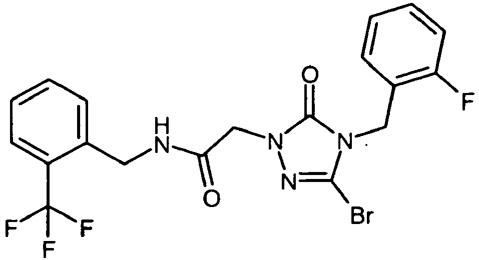

312 mg (0.95 mmol) der Verbindung aus Beispiel 135A werden zusammen mit 240 mg (0.95 mmol) 2-Chlor-*N*-[2-(trifluormethyl)benzyl]acetamid (Beispiel 134A) und 264 mg (1.21 mmol) Kaliumcarbonat in 6 ml Acetonitril über Nacht unter Rückfluss gerührt. Nach dem Abkühlen wird die Mischung mit Wasser versetzt und dreimal mit Dichlormethan extrahiert. Die vereinigten organischen Phasen werden über Magnesiumsulfat getrocknet und am Rotationsverdampfer von den flüchtigen Komponenten befreit. Der Rückstand wird über präparative HPLC (Methode 20) gereinigt. Man erhält 385 mg (79% d. Th.) der Titelverbindung.
LC/MS [Methode 8]: Rₜ = 2.46 min; m/z = 487 (M+H)⁺
¹H-NMR (400 MHz, DMSO-d₆): δ = 4.48 (d, J = 5.6 Hz, 2H), 4.52 (s, 2H), 4.90 (s, 2H), 7.18-7.29 (m, 3H), 7.36-7.43 (m, 1H), 7.49 (t, J = 7.6 Hz, 1H), 7.54 (d, J = 7.6 Hz, 1H), 7.67 (t, J = 7.6 Hz, 1H), 7.73 (d, J = 7.6 Hz, 1H), 8.74 (t, J = 5.8 Hz, 1H).

### Beispiel 143A

### 2-[3-Brom-4-(2-methoxyethyl)-5-oxo-4,5-dihydro-1H-,2,4-triazol-1-yl]-N-{1-methyl-1-[(3-trifluormethyl)phenyl]ethyl}-acetamid

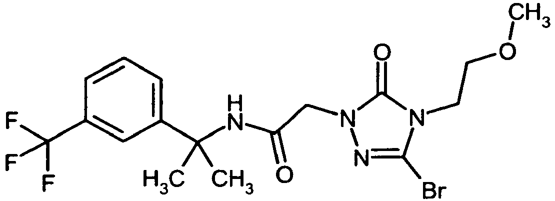

0.82 g der Verbindung aus Beispiel 136A (91% Reinheit, 3.58 mmol) werden zusammen mit 1.0 g (3.58 mmol) 2-Chlor-*N*-{1-methyl-1-[3-(trifluormethyl)phenyl]ethyl}-acetamid (Beispiel 133A) und 0.99 g (7.15 mmol) Kaliumcarbonat in 25 ml Acetonitril über Nacht unter Rückfluss gerührt. Nach dem Abkühlen wird die Mischung mit Wasser versetzt und dreimal mit Dichlormethan extrahiert. Die vereinigten organischen Phasen werden über Natriumsulfat getrocknet und am Rotationsverdampfer von den flüchtigen Komponenten befreit. Der Rückstand wird im Hochvakuum getrocknet. Man erhält 1.47 g (88% d. Th.) der Titelverbindung.
LC/MS [Methode 17]: Rₜ = 3.24 min; m/z = 465 (M+H)⁺
¹H-NMR (400 MHz, DMSO-d₆): δ = 1.58 (s, 6H), 3.22 (s, 3H), 3.50 (t, 2H), 3.74 (t, 2H), 4.39 (s, 2H), 7.50-7.57 (m, 2H), 7.60 (s, 1H), 7.65 (br. d, 1H), 8.55 (s, 1H).

### Beispiel 144A

### 2-(3-Brom-4-cyclopropyl-5-oxo-4,5-dihydro-1H-1,2,4-triazol-1-yl)-N-{1-methyl-1-[(3-trifluormethyl)phenyl]ethyl}-acetamid

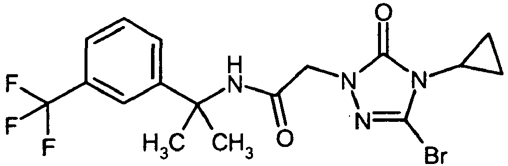

438 mg (2.15 mmol) 5-Brom-4-cydopropyl-2,4-dihydro-3*H*-1,2,4-triazol-3-on (zur Herstellung siehe EP 0 425 948-A2, Beispiel II-3) werden zusammen mit 600 mg (2.15 mmol) 2-Chlor-*N*-{1-methyl-1-[3-(trifluormethyl)phenyl]ethyl}-acetamid (Beispiel 133A) und 593 mg (4.29 mmol) Kaliumcarbonat in 15 ml Acetonitril über Nacht unter Rückfluss gerührt. Nach dem Abkühlen wird die Mischung mit Wasser versetzt und dreimal mit Ethylacetat extrahiert. Die vereinigten organischen Phasen werden über Magnesiumsulfat getrocknet und am Rotationsverdampfer von den flüchtigen Komponenten befreit. Der Rückstand wird im Hochvakuum getrocknet. Man erhält 860 mg (90% d. Th.) der Titelverbindung als leicht braunen Feststoff.
LC/MS [Methode 17]: Rₜ = 3.35 min; m/z = 447 (M+H)⁺
¹H-NMR (400 MHz, DMSO-d₆): δ = 0.86-0.92 (m, 2H), 0.92-1.01 (m, 2H), 1.58 (s, 6H), 2.80 (m, 1H), 4.33 (s, 2H), 7.50-7.57 (m, 2H), 7.59 (s, 1H), 7.65 (m, 1H), 8.50 (s, 1H).

### Beispiel 145A

### 2-(3-Brom-4-cyclopropyl-5-oxo-4,5-dihydro-1H-1,2,4-triazol-1-yl)-N-[(2-trifluormethyl)benzyl]-acetamid

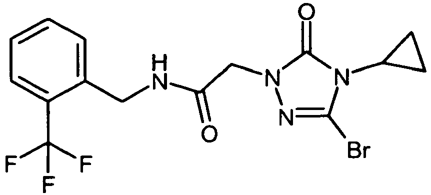

Nach dem gleichen Verfahren wie unter Beispiel 144A beschrieben werden ausgehend von 487 mg (2.38 mmol) 5-Brom-4-cyclopropyl-2,4-dihydro-3*H*-1,2,4-triazol-3-on (zur Herstellung siehe EP 0 425 948-A2, Beispiel II-3) und 600 mg (2.38 mmol) 2-Chlor-*N*-[2-(trifluormethyl)benzyl]-acetamid (Beispiel 134A) 900 mg (90% d. Th.) der Titelverbindung als leicht brauner Feststoff erhalten.
LC/MS [Methode 17]: Rₜ = 3.02 min; m/z = 419 (M+H)⁺
¹H-NMR (400 MHz, DMSO-d₆): δ = 0.90-1.03 (m, 4H), 2.85 (m, 1H), 4.41 (s, 2H), 4.48 (d, 2H), 7.45-7.54 (m, 2H), 7.69 (t, 1H), 7.72 (d, 1H), 8.54 (t, 1H).

### Beispiel 146A

### [3-Brom-4-(3-fluorbenzyl)-5-oxo-4,5-dihydro-1H-1,2,4-triazol-1-yl]-essigsäuremethylester

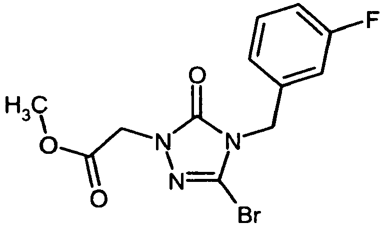

1.50 g (4.69 mmol) der Verbindung aus Beispiel 137A werden zusammen mit 508 mg (4.69 mmol) Chloressigsäuremethylester und 1.30 g (9.4 mmol) Kaliumcarbonat in 33 ml Acetonitril 4 h unter Rückfluss gerührt. Nach dem Abkühlen wird die Mischung mit 1 N Salzsäure neutralisiert und mit Ethylacetat verdünnt. Die organische Phase wird abgetrennt, mit gesättigter Kochsalz-Lösung gewaschen, über Natriumsulfat getrocknet und am Rotationsverdampfer von den flüchtigen Komponenten befreit. Der Rückstand wird in 20 ml Dichlormethan gelöst und auf 3 g Diatomeenerde aufgezogen. Nach Entfernen des Lösungsmittels am Rotationsverdampfer wird der Feststoff auf eine Kieselgel-Chromatographie-Säule übertragen und das Produkt durch Elution mit Cyclohexan/ Essigsäureethylester (Gradient 5:1 → 1:1) gereinigt. Man erhält 1.36 g (84% d. Th.) der Titelverbindung.
LC/MS [Methode 8]: Rₜ = 2.07 min; m/z = 344 (M+H)⁺
¹H-NMR (400 MHz, DMSO-d₆): δ = 3.70 (s, 3H), 4.69 (s, 2H), 4.90 (s, 2H), 7.08 (br. d, 2H), 7.17 (br. t, 1H), 7.45 (q, 1H).

### Beispiel 147A

### [3-(4-Chlor-2-methoxyphenyl)-4-(3-fluorbenzyl)-5-oxo-4,5-dihydro-1H-1,2,4-triazol-1-yl]-essigsäuremethylester

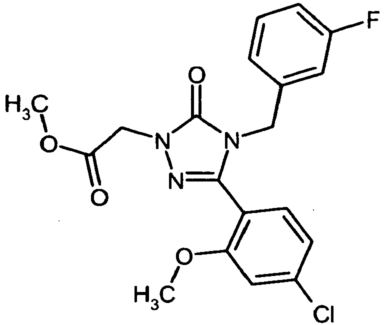

Unter Argon werden 250 mg (0.73 mmol) der Verbindung aus Beispiel 146A und 190 mg (1.07 mmol) 4-Chlor-2-methoxyphenylboronsäure in 7 ml entgastem DMF gelöst. Eine zuvor entgaste Lösung von Natriumcarbonat (2 N in Wasser, 1.09 ml, 2.18 mmol) sowie 42 mg Tetrakis(triphenylphosphin)palladium (0.036 mmol) werden hinzugefügt. Die resultierende Mischung wird erwärmt und 8 h bei 90°C gerührt. Nach Abkühlen auf RT wird mit 10%-iger Salzsäure angesäuert und die Mischung filtriert. Das Filtrat wird über präparative HPLC (Methode 20) aufgereinigt. Es werden 159 mg (54% d. Th.) der Titelverbindung sowie 54 mg (18% d. Th.) der durch Verseifung entstandenen korrespondierenden Säure (siehe auch Beispiel 148A) erhalten.
LC/MS [Methode 17]: Rₜ = 3.54 min; m/z = 406 (M+H)⁺
¹H-NMR (400 MHz, DMSO-d₆): δ = 3.69 (s, 3H), 3.72 (s, 3H), 4.72 (s, 4H), 6.78 (br. d, 1H), 6.82 (d, 1H), 7.05 (dt, 1H), 7.07 (dd, 1H), 7.23 (d, 1H), 7.25 (d, 1H), 7.31 (dt, 1H).

### Beispiel 148A

### [3-(4-Chlor-2-methoxyphenyl)-4-(3-fluorbenzyl)-5-oxo-4,5-dihydro-1H-1,2,4-triazol-1-yl]-essigsäure

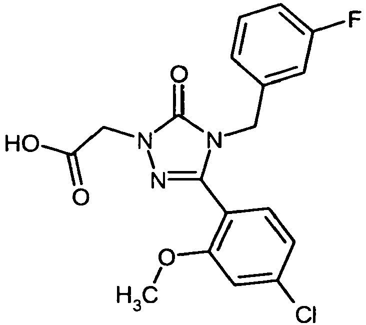

159 mg (0.39 mmol) der Verbindung aus Beispiel 147A in 4 ml Methanol werden mit 1.57 ml einer 1 N Lösung von Lithiumhydroxid in Wasser (1.57 mmol) versetzt und die resultierende Mischung über Nacht bei RT gerührt. Das Methanol wird am Rotationsverdampfer entfernt, der Rückstand mit Wasser verdünnt und die resultierende wässrige Phase zweimal mit Dichlormethan extrahiert. Diese organischen Phasen werden verworfen. Die wässrige Phase wird dann mit 1 N Salzsäure angesäuert und dreimal mit Dichlormethan extrahiert. Die Extrakte werden vereinigt, über Magnesiumsulfat getrocknet, am Rotationsverdampfer vom Lösungsmittel befreit und der Rückstand im Hochvakuum getrocknet. Man erhält 144 mg (94% d. Th.) der Titelverbindung.
LC/MS [Methode 17]: Rₜ = 3.20 min; m/z = 392 (M+H)⁺
¹H-NMR (400 MHz, DMSO-d₆): δ = 3.69 (s, 3H), 4.57 (s, 2H), 4.71 (s, 2H), 6.76-6.84 (m, 2H), 7.05 (dt, 1H), 7.07 (dd, 1H), 7.23 (d, 1H), 7.24 (d, 1H), 7.29 (dt, 1H), 13.2 (br. s, 1H).

### Beispiel 149A

### [4-(3-Fluorbenzyl)-3-(2-hydroxyphenyl)-5-oxo-4,5-dihydro-1H-1,2,4-triazol-1-yl]-essigsäuremethylester

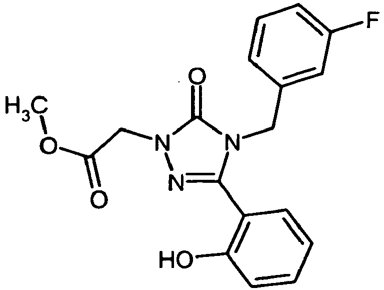

Nach der für Beispiel 147A beschriebenen Methode erhält man ausgehend von 250 mg (0.73 mmol) der Verbindung aus Beispiel 146A und 138 mg (1.02 mmol) 2-Hydroxyphenylboronsäure 22 mg (8% d. Th.) der Titelverbindung sowie 222 mg (85% d. Th.) der durch Verseifung entstandenen korrespondierenden Säure (siehe auch Beispiel 150A).
LC/MS [Methode 17]: Rₜ = 2.94 min; m/z = 358 (M+H)⁺
¹H-NMR (400 MHz, DMSO-d₆): δ = 3.72 (s, 3H), 4.70 (s, 2H), 4.82 (s, 2H), 6.73 (br. d, 1H), 6.79 (d, 1H), 6.83 (t, 1H), 6.98 (d, 1H), 7.03 (dt, 1H), 7.12 (dd, 1H), 7.28 (dt, 1H), 7.35 (ddd, 1H), 10.04 (s, 1H).

### Beispiele 150A

### [4-(3-Fluorbenzyl)-3-(2-hydroxyphenyl)-5-oxo-4,5-dihydro-1H-1,2,4-triazol-1-yl]-essigsäure

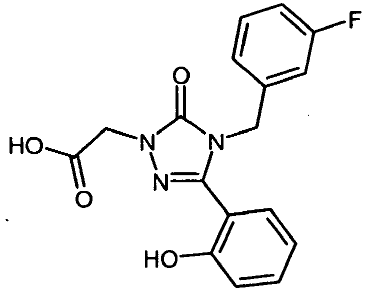

Die Titelverbindung wird entweder direkt als Nebenkomponente bei der Synthese des Beispiels 149A erhalten oder kann durch Verseifung des Methylesters aus Beispiel 149A nach der gleichen Methode, wie unter Beispiel 148A beschrieben, hergestellt werden.
LC/MS [Methode 17]: Rₜ = 3.20 min; m/z = 392 (M+H)⁺
¹H-NMR (400 MHz, DMSO-d₆): δ = 4.55 (s, 2H), 4.82 (s, 2H), 6.75 (br. d, 1H), 6.79 (d, 1H), 6.83 (t, 1H), 6.98 (d, 1H), 7.02 (dt, 1H), 7.12 (dd, 1H), 7.26 (dt, 1H), 7.35 (ddd, 1H), 10.04 (s, 1H), 13.2 (br. s, 1H).

### Beispiel 151A

### N-(2-Fluorbenzyl)-2-(5-chlorthiophen-2-carbonyl)-hydrazincarboxamid

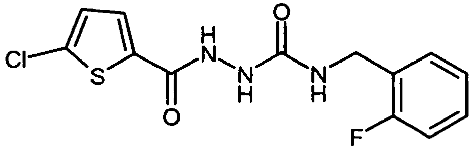

3.53 g (20 mmol) 5-Chlor-2-thiophencarbonsäurehydrazid werden unter Erwärmung in 100 ml THF gelöst und die Lösung anschließend wieder auf RT abgekühlt. 3.08 g (20.4 mmol) 2-Fluorbenzylisocyanat werden zügig hinzugetropft, wobei sich eine dicke Suspension bildet. Diese wird noch über Nacht bei RT gerührt und dann mit 100 ml Diethylether verdünnt. Der Niederschlag wird durch Filtration isoliert, mit Diethylether gewaschen und im Hochvakuum getrocknet. Man erhält 6.3 g eines Feststoffs (96% d. Th.), welcher ohne zusätzliche Reinigung weiter umgesetzt wird.
MS [DCI/NH₃]: m/z = 328 (M+H)⁺, 345 (M+NH₄)⁺
¹H-NMR (400 MHz, DMSO-d₆): δ = 4.27 (d, 2H), 7.12 (br. s, 1H), 7.12-7.19 (m, 2H), 7.23 (d, 1H), 7.25-7.31 (m, 1H), 7.35 (t, 1H), 7.70 (d, 1H), 8.12 (s, 1H).

### Beispiel 152A

### 5-(5-Chlorthiophen-2-yl)-4-(2-fluorbenzyl)-2,4-dihydro-3H-1,2,4-triazol-3-on

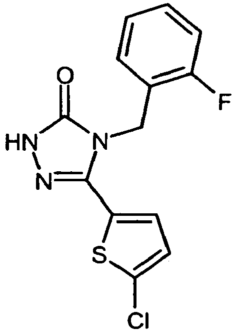

6.3 g (19.2 mmol) der Verbindung aus Beispiel 151A werden in 38.4 ml 4 N Natronlauge über Nacht unter Rückfluss erhitzt. Nach dem Abkühlen auf RT wird die Mischung mit Wasser verdünnt und durch Zugabe von 1 N Salzsäure auf pH 10 gestellt. Das Produkt wird mit Essigsäureethylester mehrmals extrahiert. Die vereinigten organischen Phasen werden mit Wasser bis zu einem neutralen pH-Wert gewaschen, dann mit gesättigter Kochsalz-Lösung gewaschen und über Natriumsulfat getrocknet. Nach Filtration wird das Lösungsmittel am Rotationsverdampfer entfernt. Es werden 4.07 g (68% d. Th.) der Titelverbindung erhalten.
MS [DCI/NH₃]: m/z = 310 (M+H)⁺, 327 (M+NH₄)⁺
¹H-NMR (400 MHz, DMSO-d₆): δ = 5.08 (s, 2H), 7.03 (t, 1H), 7.13-7.26 (m, 4H), 7.31-7.38 (m, 1H), 12.25 (br. s, 1H).

### Beispiel 153A

### [3-(5-Chlorthiophen-2-yl)-4-(2-fluorbenzyl)-5-oxo-4,5-dihydro-1H-1,2,4-triazol-1-yl]-essigsäuremethylester

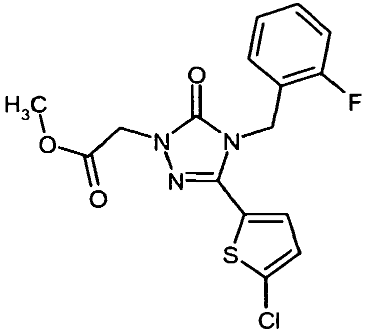

2.39 g (7.71 mmol) der Verbindung aus Beispiel 152A werden in 79 ml Acetonitril zusammen mit 1.05 g Chloressigsäuremethylester (9.6 mmol) und 1.17 g Kaliumcarbonat (8.5 mmol) über Nacht unter Rückfluss erhitzt. Nach dem Abkühlen auf RT wird die Mischung mit Essigsäureethylester verdünnt. Die erhaltene organische Phase wird mit 1 N Salzsäure, dann mit ges. Kochsalz-Lösung gewaschen, über Natriumsulfat getrocknet und am Rotationsverdampfer vom Lösungsmittel befreit. Das Produkt wird im Hochvakuum getrocknet. Man erhält 2.9 g (92% d. Th.) der Titelverbindung.
LC/MS [Methode 7]: Rₜ = 2.30 min; m/z = 382 (M+H)⁺
¹H-NMR (400 MHz, DMSO-d₆): δ = 3.71 (s, 3H), 4.75 (s, 2H), 5.15 (s, 2H), 7.02 (t, 1H), 7.13-7.28 (m, 4H), 7.32-7.39 (m, 1H).

### Beispiel 154A

### [3-(5-Chlorthiophen-2-yl)-4-(2-fluorbenzyl)-5-oxo-4,5-dihydro-1H-1,2,4-triazol-1-yl]-essigsäure

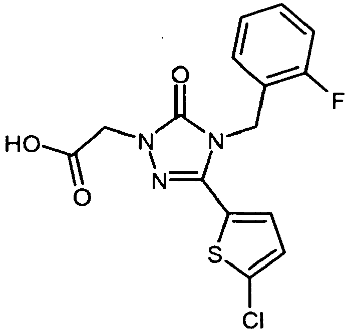

2.90 g (7.60 mmol) des Esters aus Beispiel 153A werden in 77 ml Methanol gelöst und mit einer 1 N Lösung von Lithiumhydroxid in Wasser (30.3 ml, 30.4 mmol) versetzt. Die Mischung wird über Nacht bei RT gerührt, dann am Rotationsverdampfer vom Methanol befreit und mit 200 ml Wasser verdünnt. Nach Zugabe von 1 N Salzsäure bis zu einem pH-Wert von 2 fällt das Produkt als weißer Feststoff aus, der durch Filtration und Trocknen im Hochvakuum isoliert wird. Man erhält 2.45 g (88% d. Th.) der Titelverbindung.
LC/MS [Methode 7]: Rₜ = 1.93 min; m/z = 368 (M+H)⁺
¹H-NMR (400 MHz, DMSO-d₆): δ = 4.60 (s, 2H), 5.13 (s, 2H), 7.03 (t, 1H), 7.12-7.28 (m, 4H), 7.32-7.38 (m, 1H), 13.23 (br. s, 1H).

### Beispiel 155A

### [3-(4-Chlorphenyl)-4-(2-fluorbenzyl)-5-oxo-4,5-dihydro-1H-1,2,4-triazol-1-yl]-essigsäuremethylester

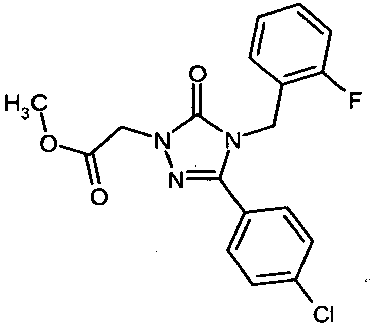

Die Titelverbindung wird nach dem gleichen Verfahren, wie für Beispiel 153A beschrieben, ausgehend von der Verbindung aus Beispiel 40A und Chloressigsäuremethylester erhalten.
LC/MS [Methode 8]: Rₜ = 2.45 min; m/z = 375 (M+H)⁺
¹H-NMR (400 MHz, DMSO-d₆): δ = 3.71 (s, 3H), 4.75 (s, 2H), 5.05 (s, 2H), 7.03 (t, 1H), 7.11 (t, 1H), 7.16 (d, 1H), 7.50-7.60 (m, 4H).

### Beispiele 156A

### [3-(4-Chlorphenyl)-4-(2-fluorbenzyl)-5-oxo-4,5-dihydro-1H-1,2,4-triazol-1-yl]-essigsäure

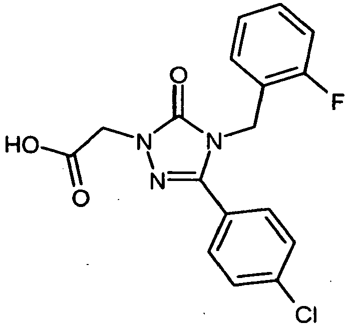

Analog zu Beispiel 154A wird die Titelverbindung durch Verseifung des Esters aus Beispiel 155A hergestellt. Die Reinheit ist laut LC/MS und NMR ca. 86%.
LC/MS [Methode 17]: Rₜ = 3.17 min; m/z = 361 (M+H)⁺
¹H-NMR (400 MHz, DMSO-d₆): δ = 4.60 (s, 2H), 5.04 (s, 2H), 7.03 (t, 1H), 7.06-7.17 (m, 2H), 7.27-7.32 (m, 1H), 7.52-7.58 (m, 4H), 13.18 (br. s, 1H).

### Beispiel 157A

### [5-Chlor-2-(trifluormethyl)phenyl]methylamin-Hydrochlorid

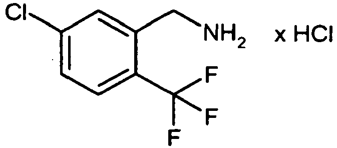

29.2 ml Boran-THF-Komplex (29.2 mmol) werden unter Argon zu einer eisgekühlten Lösung von 5-Chlor-2-(trifluormethyl)benzonitril (1.50 g, 7.3 mmol) in 45 ml wasserfreiem THF langsam zugetropft. Nach Ende der Zugabe wird die Mischung für 1 h unter Rückfluss erhitzt, dann über Nacht unter Abkühlen auf RT gerührt. Unter Eiskühlung werden danach 30 ml 1 N Salzsäure zugetropft. Das THF wird am Rotationsverdampfer entfernt. Der ausgefallene Feststoff wird abfiltriert und verworfen. Das Filtrat wird mit Wasser verdünnt und zweimal mit Dichlormethan extrahiert. Diese organischen Phasen werden ebenso verworfen. Die saure wässrige Phase wird mit 1 N Natronlauge auf pH 14 gestellt und dreimal mit Dichlormethan extrahiert. Die vereinigten organischen Phasen werden über Natriumsulfat getrocknet und am Rotationsverdampfer vom Lösungsmittel befreit. Der Rückstand wird in 20 ml Diethylether aufgenommen und mit 3 ml einer 4 N Lösung von Chlorwasserstoff in Dioxan versetzt, wobei das Produkt ausfällt. Das Lösungsmittel wird am Rotationsverdampfer, dann im Hochvakuum vollständig entfernt. Es werden 1.89 g (99% d. Th.) der Titelverbindung isoliert.
LC/MS [Methode 17]: Rₜ = 1.02 min; m/z = 210 (M+H)⁺
¹H-NMR (400 MHz, DMSO-d₆): δ = 4.17 (s, 2H), 7.85 (d, 1H), 7.89 (s, 1H), 7.92 (d, 1H), 8.70 (br. s, 3H).

Analog zu Beispiel 157A werden aus den entsprechenden Nitrilen durch Boran-Reduktion die folgenden Amine (als Hydrochlorid) erhalten:

| **Beispiel Nr.** | **Struktur** | **Analytische Daten** |
|---|---|---|
| **158A** | 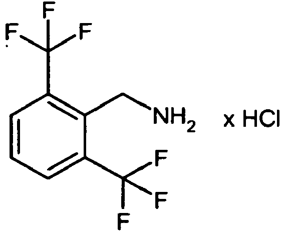 | LC/MS [Methode 17]: Rₜ = 0.69 min; m/z = 210 (M+H)⁺ |
| | | ¹H-NMR (400 MHz, DMSO-d₆): δ = 4.24 (s, 2H), 7.92 (t, 1H), 8.20 (d, 2H), 8.51 (br. s, 3H). |
| **159A** | 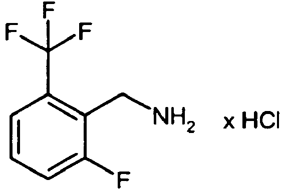 | LC/MS [Methode 3]: Rₜ = 1.77 min; m/z = 194 (M+H)⁺ |
| | | ¹H-NMR (400 MHz, DMSO-d₆): δ = 4.16 (s, 2H), 7.67-7.82 (m, 3H), 8.51 (br. s, 3H). |
| **160A** | 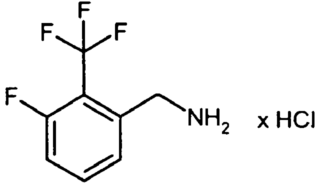 | LC/MS [Methode 3]: Rₜ = 1.89 min; m/z = 194 (M+H)⁺ |
| | | ¹H-NMR (400 MHz, DMSO-d₆): δ = 4.21 (s, 2H), 7.51-7.58 (m, 2H), 7.80-7.86 (m, 1H), 8.51 (br. s, 3H). |
| **161A** | 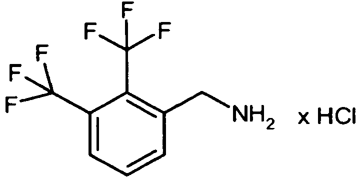 | HPLC [Methode 2]: Rₜ = 3.60 min; MS (DCI/NH₃): m/z = 244 (M+H)⁺, 261 (M+NH₄)⁺ |
| | | ¹H-NMR (400 MHz, DMSO-d₆): δ = 4.24 (s, 2H), 7.92 (t, 1H), 8.20 (d, 2H), 8.51 (br. s, 3H). |

### Beispiel 162A

### 1-[2-(Trifluormethyl)benzyl]cyclopropanamin-Hydrochlorid

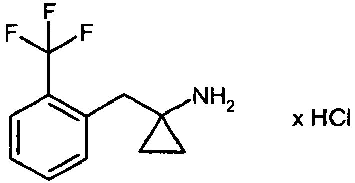

Unter Argon werden 4.68 ml (14.0 mmol) einer 3 M Lösung von Ethylmagnesiumbromid in Diethylether langsam bei RT zu einer Lösung von 1.3 g 2-(Trifluormethyl)phenylacetonitril (7.2 mmol) und 2.20 g (7.7 mmol) Titan(IV)isopropylat in 50 ml Diethylether getropft. Es wird 1 h bei RT nachgerührt, dann mit 1.78 ml Bortrifluorid-Diethylether Komplex versetzt und 30 min bei RT weiter gerührt. Zur Aufarbeitung werden 50 ml 2 M Natronlauge hinzugegeben und die Mischung dreimal mit Diethylether extrahiert. Die vereinigten organischen Phasen werden zweimal mit je 70 ml 1 N Salzsäure extrahiert. Die vereinigten wässrigen Phasen werden dann mit 2 N Natronlauge auf pH 14 gestellt und dreimal mit Dichlormethan extrahiert. Diese vereinigten organischen Phasen werden über Natriumsulfat getrocknet und am Rotationsverdampfer bis zu einem Volumen von ca. 30 ml eingeengt. Es wird mit 4 ml einer 4 N Lösung von Chlorwasserstoff in Dioxan versetzt. Der ausgefallene Feststoff wird abgesaugt und im Hochvakuum getrocknet. Man erhält 670 mg (38% d. Th.) der Titelverbindung.
LC/MS [Methode 17]: Rₜ = 1.02 min; m/z = 210 (M+H)⁺
¹H-NMR (400 MHz, DMSO-d₆): δ = 0.64 (m, 2H), 1.01 (m, 2H), 3.27 (s, 2H), 7.50 (t, 1H), 7.61 (d, 2H), 7.68 (t, 1H), 7.72 (d, 1H), 8.50 (br. s, 3H).

### Beispiel 163A

### 2-Methyl-2-[3-(trifluormethyl)phenyl]-propannitril

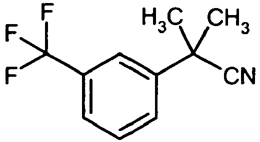

500 mg (2.7 mmol) 3-(Trifluormethyl)phenylacetonitril in 6 ml wasserfreiem Diethylether werden unter Argon unter leichter Kühlung mit 316 mg (8.1 mmol) Natriumamid versetzt. Anschließend wird die Mischung auf 0°C gekühlt und mit 1.53 g (10.8 mmol) Iodmethan versetzt. Nach 30 min wird das Eisbad entfernt und die Reaktionsmischung weiter über Nacht bei RT gerührt. Danach werden 2 ml einer ges. Ammoniumchlorid-Lösung zugesetzt. Die Mischung wird mit Diethylether verdünnt und zweimal mit Wasser gewaschen. Die organische Phase wird über Magnesiumsulfat getrocknet und am Rotationsverdampfer vom Lösungsmittel befreit. Der Rückstand (rötliche Flüssigkeit) entspricht der reinen Titelverbindung (545 mg, 95% d. Th.).
¹H-NMR (400 MHz, DMSO-d₆): δ = 1.76 (s, 6H), 7.70 (t, 1H), 7.75 (d, 1H), 7.82 (s, 1H), 7.88 (d, 1H).

### Beispiel 164A

### 2-Methyl-2-[3-(trifluormethyl)phenyl]propanamin-Hydrochlorid

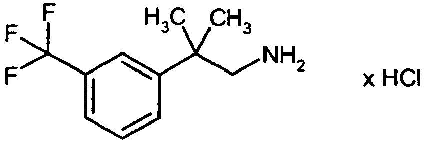

Die Titelverbindung wird analog zu Beispiel 157A durch Boran-Reduktion des Nitrils aus Beispiel 163A erhalten.
LC/MS [Methode 3]: Rₜ = 2.59 min; m/z = 218 (M+H)⁺
¹H-NMR (400 MHz, DMSO-d₆): δ = 1.49 (s, 6H), 3.11 (s, 2H), 7.59-7.68 (m, 2H), 7.71 (s, 1H), 7.76 (d, 1H), 7.78 (br. s, 3H).

### Beispiel 165A

### 2-Methyl-2-[2-(trifluormethyl)phenyl]-propannitril

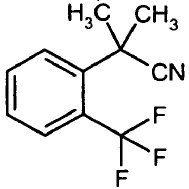

Eine Lösung von 2.0 g (10.8 mmol) 2-(Trifluormethyl)phenylacetonitril und 6.13 g (43 mmol) Iodmethan in 16 ml DMSO wird langsam mit 3.16 ml 50%-iger Natronlauge versetzt, so dass die Reaktionstemperatur zwischen 40°C und 45°C gehalten wird. Nach vollständiger Zugabe wird die Mischung bei RT über Nacht gerührt. Es wird mit Wasser verdünnt und dreimal mit Dichlormethan extrahiert. Die vereinigten organischen Phasen werden mit Wasser gewaschen, über Natriumsulfat getrocknet und am Rotationsverdampfer vom Lösungsmittel befreit. Der Rückstand entspricht der Titelverbindung (2.30 g, 100% d. Th.).
¹H-NMR (400 MHz, DMSO-d₆): δ = 1.80 (s, 6H), 7.63 (t, 1H), 7.72-7.82 (m, 2H), 7.85 (d, 1H).

### Beispiel 166A

### 2-Methyl-2-[2-(trifluormethyl)phenyl]propanamin-Hydrochlorid

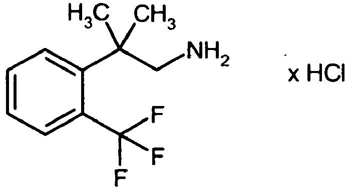

Die Titelverbindung wird analog zu Beispiel 157A durch Boran-Reduktion des Nitrils aus Beispiel 165A erhalten.
LC/MS [Methode 8]: Rₜ = 1.10 min; m/z = 218 (M+H)⁺
¹H-NMR (400 MHz, DMSO-d₆): δ = 1.50 (s, 6H), 3.15 (s, 2H), 7.53 (t, 1H), 7.65-7.74 (m, 2H), 7.85 (d, 1H), 7.90 (br. s, 3H).

### Beispiel 167A

### 2-[3-(Trifluormethyl)phenyl]propannitril

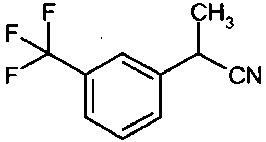

1.0 g (5.4 mmol) 3-(Trifluormethyl)phenylacetonitril und 767 mg Iodmethan (5.4 mmol) werden in 5 ml Toluol bei 80°C vorgelegt und langsam mit einer 50%-igen Suspension von Natriumamid in Toluol versetzt. Anschließend wird die Mischung noch 1 h bei 80°C gerührt, danach auf RT gekühlt und mit Wasser, dann Dichlormethan versetzt. Die organische Phase wird abgetrennt, mit Wasser gewaschen, über Magnesiumsulfat getrocknet und am Rotationsverdampfer vom Lösungsmittel befreit. Der Rückstand (1.7 g, 84% d. Th.) enthält die Titelverbindung mit einer Reinheit laut GC/MS von ca. 53% und wird ohne Reinigung für die nächste Reaktion eingesetzt.
GC/MS [Methode 21]: Rₜ = 3.42 min; m/z = 199 (M)⁺.

### Beispiel 168A

### 2-[3-(Trifluormethyl)phenyl]propanamin-Hydrochlorid

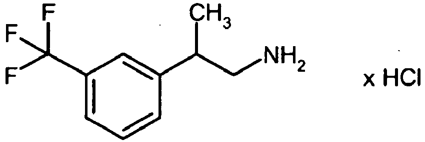

Die Titelverbindung wird analog zu Beispiel 157A durch Boran-Reduktion des Nitrils aus Beispiel 167A (Rohprodukt) erhalten (Ausbeute 31% d. Th.).
LC/MS [Methode 3]: Rₜ = 2.52 min; m/z = 204 (M+H)⁺
¹H-NMR (400 MHz, DMSO-d₆): δ = 1.29 (d, 3H), 3.00-3.11 (m, 2H), 3.13-3.25 (m, 1H), 7.55-7.69 (m, 4H), 7.98 (br. s, 3H).

### Beispiel 169A

### 2-Amino-1-[2-(trifluormethyl)phenyl]ethanon-Hydrochlorid

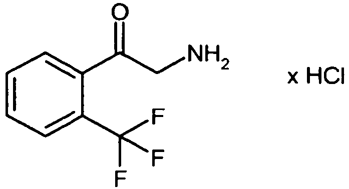

Eine Lösung von 1.0 g (3.75 mmol) 2-Brom-1-[(2-trifluormethyl)phenyl]ethanon in 4 ml Acetonitril wird bei RT mit 413 mg (4.34 mmol) Natriumdiformylamid versetzt und 2.5 h gerührt. Danach wird die Suspension auf 70°C erhitzt und heiss filtriert. Der Feststoff wird mit 2 ml heissem Acetonitril gewaschen. Die kombinierten Filtrate werden am Rotationsverdampfer vom Lösungsmittel befreit. Der dunkle ölige Rückstand entspricht nach LC/MS [Methode 8; Rₜ = 2.00 min; m/z = 260 (M+H)⁺] der Diformyl-Zwischenstufe. Dieser Rückstand wird mit 10 ml einer 5%-igen ethanolischen Chlorwasserstoff-Lösung versetzt und zwei Tage bei RT gerührt. Die flüchtigen Komponenten werden am Rotationsverdampfer entfernt. Der erhaltene gelbe Feststoff wird in 20 ml Diethylether 10 min unter Rückfluss verrührt und danach die Suspension auf RT gekühlt. Der weisse Feststoff wird abgesaugt, mit Diethylether gewaschen und im Hochvakuum getrocknet. Man erhält 570 mg (64% d. Th.) der Titelverbindung.
HPLC [Methode 2]: Rₜ = 3.19 min;
MS (DCI/NH₃): m/z = 204 (M+H)⁺, 221 (M+NH₄)⁺
¹H-NMR (400 MHz, DMSO-d₆): δ = 4.53 (s, 2H), 7.80-7.90 (m, 2H), 7.95 (d, 1H), 8.03 (d, 1H), 8.49 (br. s, 3H).

### Beispiele 170A

### [2-Chlor-3-(trifluormethyl)phenyl]-methylammonium-Trifluoracetat

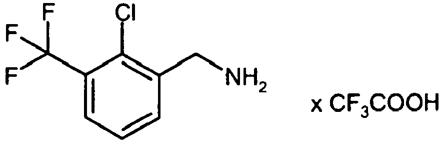

1.75 g Rink-Amid^{®} (0.96 mmol) wird aktiviert (FMOC-Abspaltung) durch Verrühren mit 10 ml einer 25%-igen Lösung von Piperidin in DMF für 30 min, dann Filtration des Harzes und erneute Behandlung mit 10 ml einer 25%-igen Lösung von Piperidin in DMF für 30 min. Das Polymer wird abgesaugt, nacheinander je dreimal mit DMF, Methanol und Dichlormethan gewaschen und dann in 10 ml Orthoameisensäuretrimethylester (TMOF) quellen gelassen. 400 mg (1.9 mmol) 2-Chlor-3-(trifluormethyl)benzaldehyd werden hinzugegeben und die Suspension 5 h gerührt. Dann wird das Polymer abfiltriert, nacheinander je dreimal mit DMF, Methanol und Dichlormethan gewaschen und am Rotationsverdampfer getrocknet. Erneut lässt man das Polymer in 10 ml TMOF vorquellen, dann gibt man 987 mg (3.84 mmol) Tetrabutylammoniumborhydrid und langsam 878 µl (15 mmol) Essigsäure hinzu. Die Suspension wird über Nacht bei RT gerührt. Das Polymer wird abgesaugt und nacheinander je fünfmal mit DMF, Methanol und Dichlormethan gewaschen. Anschließend wird es mit 20 ml Trifluoressigsäure /Dichlormethan (1:1) verrührt. Nach 1 h wird das Polymer abgesaugt und mit Dichlormethan gewaschen. Das Filtrat wird am Rotationsverdampfer von den flüchtigen Komponenten befreit und der Rückstand kurz im Hochvakuum getrocknet. Man erhält 170 mg (27% d. Th.) der Titelverbindung in annehmbarer Reinheit (>50%), welche in dieser Form weiter eingesetzt wird.
LC/MS [Methode 3]: Rₜ = 2.30 min; m/z = 210 (M+H)⁺
¹H-NMR (400 MHz, DMSO-d₆): δ = 4.25 (q, 2H), 7.69 (t, 1H), 7.88 (d, 1H), 7.85 (d, 1H), 8.32 (br. s, 3H).

### Beispiel 171A

### [2-Methyl-3-(trifluormethyl)phenyl]-methylammonium-Trifluoracetat

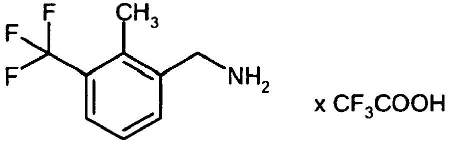

Die Titelverbindung wird aus 2-Methyl-3-(trifluormethyl)benzaldehyd nach dem gleichen Verfahren, wie für Beispiel 170A beschrieben, hergestellt (Ausbeute: 49% d. Th.).
LC/MS [Methode 3]: Rₜ = 2.36 min; m/z = 190 (M+H)⁺
¹H-NMR (400 MHz, DMSO-d₆): δ = 2.44 (s, 3H), 4.17 (q, 2H), 7.49 (t, 1H), 7.68 (d, 1H), 7.72 (d, 1H), 8.22 (br. s, 3H).

### Beispiel 172A

### [2-(Trifluormethyl)phenyl]-glycinmethylester-Hydrochlorid

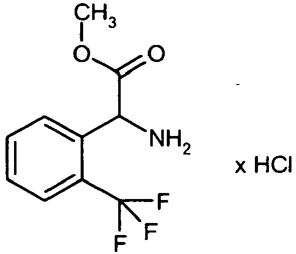

300 mg (1.37 mmol) DL-[2-(Trifluormethyl)phenyl]-glycin werden in 9 ml Methanol vorgelegt und bei RT langsam mit 130 µl Thionylchlorid versetzt. Die Lösung wird über Nacht unter Rückfluss erhitzt, dann auf RT abgekühlt und am Rotationsverdampfer von den flüchtigen Komponenten befreit. Da der Rückstand laut LC/MS noch ca. 44% Edukt enthält, wird er nochmals unter den oben beschriebenen Bedingungen umgesetzt. Hiernach ist die Umsetzung vollständig. Man erhält 371 mg (93% d. Th.) der Titelverbindung.
LC/MS [Methode 3]: Rₜ = 2.14 min; m/z = 233 (M+H)⁺
¹H-NMR (400 MHz, DMSO-d₆): δ = 3.72 (s, 3H), 5.27 (s, 1H), 7.66-7.78 (m, 2H), 7.84 (t, 1H), 7.89 (d, 1H), 9.21 (br. s, 3H).

### Beispiel 173A

### N-tert.-Butoxycarbonyl-[3-(trifluormethyl)phenyl]-glycin

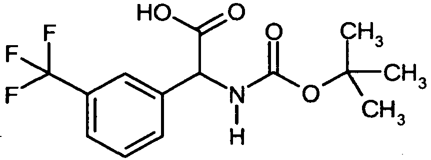

226 mg (1.03 mmol) DL-[3-(Trifluormethyl)phenyl]-glycin werden in 10 ml einer wässrigen 5%-igen Natriumhydrogencarbonat-Lösung gelöst und mit 4 ml Dioxan versetzt. 261 µl (1.13 mmol) Di-tert.-butyldicarbonat werden zugegeben und die Mischung über Nacht bei RT gerührt. Zur Aufarbeitung wird die Lösung vorsichtig mit 1 N Salzsäure bis pH 2 angesäuert. Das ausgefallene Produkt wird durch Zugabe von Acetonitril wieder gelöst und durch präparative HPLC (Methode 20) gereinigt. Man erhält 135 mg (41% d. Th.) der Titelverbindung.
LC/MS [Methode 8]: Rₜ = 2.53 min; m/z = 319 (M+H)⁺
¹H-NMR (400 MHz, DMSO-d₆): δ = 1.39 (s, 9H), 5.28 (d, 1H), 7.59 (t, 1H), 7.68 (d, 1H), 7.72 (d, 1H), 7.79 (s, 1H), 7.80 (d, 1H), 12.99 (br. s, 1H).

### Beispiel 174A

### tert.-Butyl {2-(dimethylamino)-2-oxo-1-[3-(trifluormethyl)phenyl]ethyl}carbamat

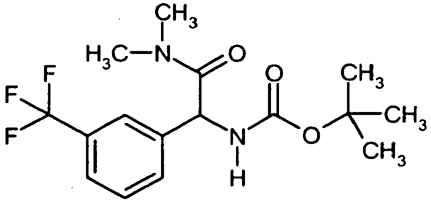

125 mg (392 µmol) der Verbindung aus Beispiel 173A und 95 mg HOBt (705 µmol) werden in 8 ml DMF vorgelegt und bei RT mit 135 mg (705 µmol) EDC versetzt. Nach 20 min wird eine 2 M Lösung von Dimethylamin in THF (294 µl, 587 µmol) hinzugefügt und die Mischung über Nacht bei RT gerührt. Nach Zugabe von 1 ml 1 N Salzsäure wird die Mischung direkt durch präparative HPLC (Methode 20) aufgetrennt. Man erhält 90 mg (64% d. Th.) der Titelverbindung.
LC/MS [Methode 17]: Rₜ = 3.55 min; m/z = 347 (M+H)⁺.

### Beispiel 175A

### 2-Dimethylamino-2-oxo-1-[3-(trifluormethyl)phenyl]ethan-Hydrochlorid

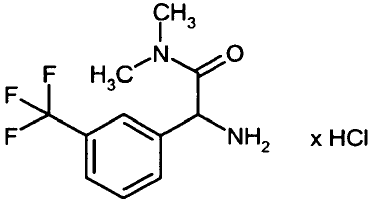

130 mg (375 µmol) der Verbindung aus Beispiel 174A werden in 2 ml einer 4 M Lösung von Chlorwasserstoff in Dioxan bei RT verrührt. Nach vollständiger Umsetzung werden die flüchtigen Komponenten am Rotationsverdampfer entfernt und der Rückstand im Hochvakuum getrocknet. Man erhält 105 mg (99% d. Th.) der Titelverbindung.
LC/MS [Methode 3]: Rₜ = 2.42 min; m/z = 247 (M+H)⁺
¹H-NMR (400 MHz, DMSO-d₆): δ = 5.70 (s, 1H), 7.72 (t, 1H), 7.80 (d, 1H), 7.83 (d, 1H), 7.95 (s, 1H), 8.75 (br. s, 3H).

Alternativ kann die Abspaltung der *tert*.-Butoxycarbonyl-Schutzgruppe durch Behandlung der Verbindung aus Beispiel 174A mit überschüssiger Trifluoressigsäure in Dichlormethan erfolgen. Nach Entfernung der flüchtigen Komponenten am Rotationsverdampfer erhält man das Produkt als Trifluoracetat-Salz.

### Beispiel 176A

### N-tert.-Butoxycarbonyl-[2-(trifluormethyl)phenyl]-glycin

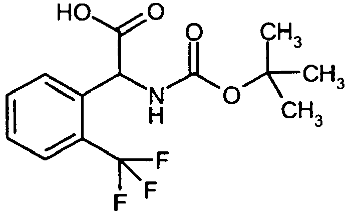

1.0 g (4.56 mmol) DL-[2-(Trifluormethyl)phenyl]-glycin werden in 30 ml einer wässrigen 5%-igen Natriumhydrogencarbonat-Lösung gelöst und mit 4 ml Dioxan versetzt. Es werden 1.15 ml (5.02 mmol) Di-*tert*.-butyldicarbonat zugegeben und die Mischung über Nacht bei RT gerührt. Zur Aufarbeitung wird die Reaktionsmischung in 100 ml 1 N Salzsäure gegossen und dreimal mit Dichlormethan extrahiert. Die vereinigten organischen Phasen werden über Natriumsulfat getrocknet und am Rotationsverdampfer von den flüchtigen Komponenten befreit. Der Rückstand wird im Hochvakuum getrocknet. Man erhält 1.26 g (86% d. Th.) der Titelverbindung.
MS [DCI/NH₃]: m/z = 337 (M+NH₄)⁺
¹H-NMR (400 MHz, DMSO-d₆): δ = 1.35 (s, 9H), 5.49 (d, 1H), 7.55 (t, 1H), 7.61 (d, 1H), 7.69 (t, 1H), 7.72 (d, 1H), 7.82 (d, 1 H), 12.99 (br. s, 1H).

Nach der gleichen Reaktionssequenz Amidbildung / Boc-Abspaltung, wie sie bei den Beispielen 174A und 175A beschrieben ist, werden aus Beispiel 173A bzw. Beispiel 176A und den entsprechenden Aminen die folgenden Verbindungen hergestellt (Methylamin wird hierbei als Lösung in Ethanol eingesetzt; Ammoniak wird als 33%-ige-Lösung in Wasser eingesetzt):

| **Beispiel Nr.** | **Struktur** | **Edukt** | **Analytische Daten** |
|---|---|---|---|
| **177A** | 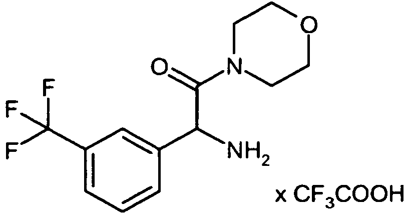 | 173A | LC/MS [Methode 17]: Rₜ = 1.12 min; m/z = 289 (M+H)⁺. |
| **178A** | 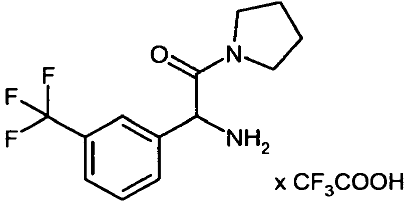 | 173A | LC/MS [Methode 17]: Rₜ = 1.44 min; m/z = 273 (M+H)⁺. |
| **179A** | 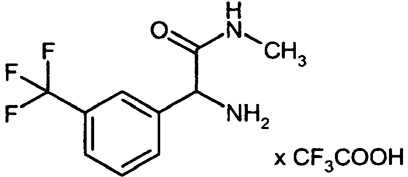 | 173A | LC/MS [Methode 17]: Rₜ = 0.81 min; m/z = 233 (M+H)⁺. |
| **180A** | 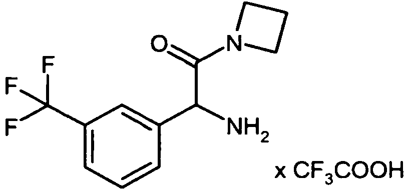 | 173A | LC/MS [Methode 17]: Rₜ = 1.19 min; m/z = 259 (M+H)⁺. |
| **181A** | 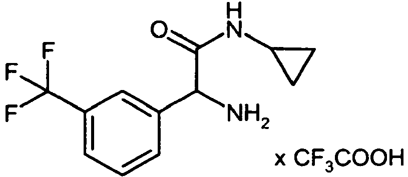 | 173A | LC/MS [Methode 17]: Rₜ = 1.25 min; m/z = 259 (M+H)⁺. |
| **182A** | 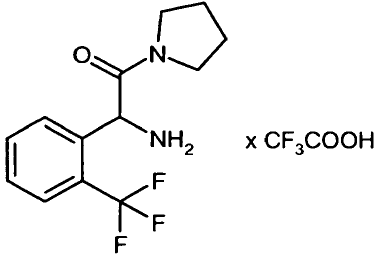 | 176A | LC/MS [Methode 3]: Rₜ = 2.34 min; m/z = 273 (M+H)⁺. |
| **183A** | 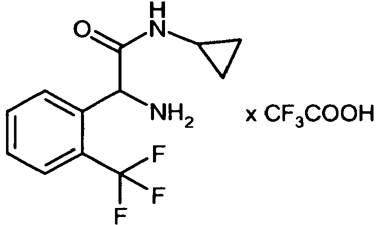 | 176A | LC/MS [Methode 3]: Rₜ = 2.14 min; m/z = 259 (M+H)⁺. |
| **184A** | 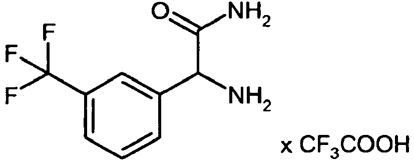 | 173A | LC/MS [Methode 3]: Rₜ = 1.65 min; m/z = 219 (M+H)⁺. |
| **185A** | 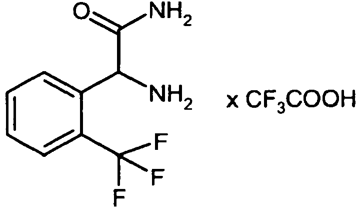 | 176A | LC/MS [Methode 3]: Rₜ = 0.95 min; m/z = 219 (M+H)⁺. |
| **186A** | 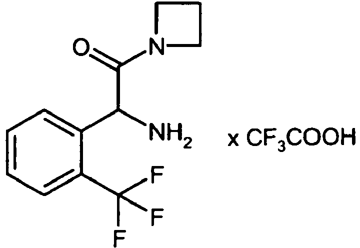 | 176A | LC/MS [Methode 3]: Rₜ = 2.20 min; m/z = 259 (M+H)⁺. |

### Beispiel 187A

### 2-(2,3-Dichlorbenzyl)-2-methylpropannitril

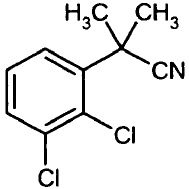

Aus 1.00 g 2,3-Dichlorphenylacetonitril (5.37 mmol) erhält man nach der unter Beispiel 165A beschriebenen Methode 1.10 g der Titelverbindung (96% d. Th.).
GC/MS [Methode 21]: Rₜ = 5.56 min; m/z = 213 (M)⁺
¹H-NMR (400 MHz, DMSO-d₆): δ = 1.81 (s, 6H), 7.46 (t, 1H), 7.55 (dd, 1H), 7.72 (dd, 1H).

### Beispiel 188A

### 2-(2,6-Dichlorbenzyl)-2-methylpropannitril

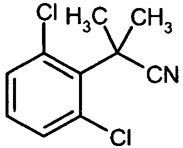

Aus 500 mg 2,6-Dichlorphenylacetonitril (2.69 mmol) erhält man nach der unter Beispiel 165A beschriebenen Methode 262 mg der Titelverbindung (46% d. Th.) sowie 135 mg (25% d. Th.) des monomethylierten Derivats (siehe unter Beispiel 189A).
GC/MS [Methode 21]: Rₜ = 5.71 min; m/z = 213 (M)⁺
¹H-NMR (400 MHz, DMSO-d₆): δ = 2.01 (s, 6H), 7.49 (t, 1H), 7.54 (d, 2H).

### Beispiel 189A

### 2-(2,3-Dichlorbenzyl)propannitril

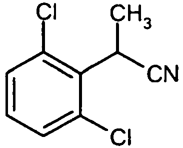

Die Titelverbindung wird als Nebenprodukt bei der Herstellung von Beispiel 188A erhalten.
GC/MS [Methode 21]: Rₜ = 5.28 min; m/z = 199 (M)⁺
¹H-NMR (400 MHz, DMSO-d₆): δ = 1.60 (d, 3H), 4.97 (q, 1H), 7.42 (t, 1H), 7.56 (d, 2H).

### Beispiel 190A

### 2-[2,3-Dichlorphenyl]-2-methylpropanamin-Hydrochlorid

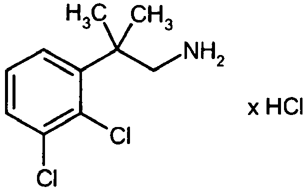

Die Titelverbindung wird analog zu Beispiel 157A durch Boran-Reduktion des Nitrils aus Beispiel 187A erhalten.
LC/MS [Methode 17]: Rₜ = 1.71 min; m/z = 218 (M+H)⁺
¹H-NMR (400 MHz, DMSO-d₆): δ = 1.51 (s, 6H), 3.40 (s, 2H), 7.38 (t, 1H), 7.42 (d, 1H), 7.63 (d, 1H), 7.80 (br. s, 3H).

### Beispiel 191A

### 2-[2,6-Dichlorphenyl]-2-methylpropanamin-Hydrochlorid

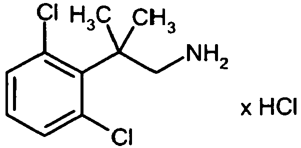

Die Titelverbindung wird analog zu Beispiel 157A durch Boran-Reduktion des Nitrils aus Beispiel 188A erhalten.
LC/MS [Methode 3]: Rₜ = 2.48 min; m/z = 218 (M+H)⁺
¹H-NMR (400 MHz, DMSO-d₆): δ = 1.72 (s, 6H), 3.50 (s, 2H), 7.29 (t, 1H), 7.47 (d, 2H), 8.00 (br. s, 3H).

### Beispiel 192A

### 2-(2,6-Dichlorphenyl)-propanamin-Hydrochlorid

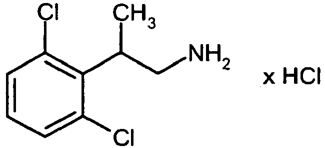

Die Titelverbindung wird analog zu Beispiel 157A durch Boran-Reduktion des Nitrils aus Beispiel 189A erhalten.
LC/MS [Methode 3]: Rₜ = 2.39 min; m/z = 204 (M+H)⁺
¹H-NMR (400 MHz, DMSO-d₆): δ = 1.40 (d, 3H), 3.25 (dd, 1H), 3.35 (dd, 1H), 3.93 (m, 1H), 7.32 (t, 1H), 7.45 (d, 1H), 7.51 (d, 1H), 8.07 (br. s, 3H).

### Beispiel 193A

### 3-{[(Benzyloxy)carbonyl]amino}-2-[2-(trifluormethyl)phenyl]propansäureethylester

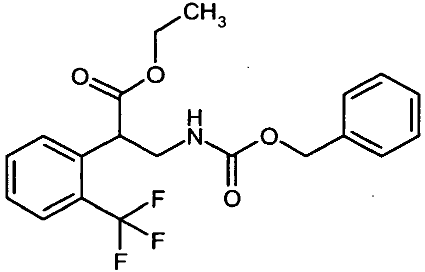

Eine LDA-Lösung wird unter Argon durch langsames Zutropfen einer n-Butyllithium-Lösung (1.6 M in Hexan, 2.6 ml, 4.13 mmol) zu einer Lösung von 591 µl Diisopropylamin (4.21 mmol) in 3 ml wasserfreiem THF bei -15°C und 10 min Rühren bei 0°C hergestellt. Diese LDA-Lösung wird auf -70°C gekühlt und langsam mit einer Lösung von 500 mg 2-(Trifluormethyl)phenylessigsäureethylester (2.15 mmol) und *N*-Methoxymethyl-benzylcarbamat (350 mg, 1.79 mmol) in 3 ml THF versetzt. Nach 15 min bei -70°C werden 1.17 ml (3.95 mmol) Titan(IV)isopropylat zugegeben. Die Mischung wird noch 1 h bei dieser Temperatur nachgerührt, dann über Nacht bei -60°C. Anschlie-βend wird innerhalb einer Stunde auf 0°C erwärmen gelassen und noch eine weitere Stunde bei dieser Temperatur gerührt. Die Mischung wird mit 20 ml 1 N Salzsäure versetzt und dreimal mit Dichlormethan extrahiert. Die vereinigten organischen Phasen werden über Magnesiumsulfat getrocknet und am Rotationsverdampfer von den flüchtigen Komponenten befreit. Der Rückstand wird im Hochvakuum getrocknet. Man erhält 450 mg (49% d. Th., Reinheit ca. 92%) der Titelverbindung.
MS [DCl/NH₃]: m/z = 413 (M+NH₄)⁺
¹H-NMR (400 MHz, DMSO-d₆): δ = 1.00 (t, 3H), 3.35 (m, 1H), 3.69 (m, 1H), 3.99-4.11 (m, 2H), 4.23 (t, 1H), 4.99 (s, 2H), 7.26-7.38 (m, 5H), 7.48-7.56 (m, 2H), 7.62-7.75 (m, 3H).

### Beispiel 194A

### 3-Amino-2-[2-(trifluormethyl)phenyl]propansäureethylester-Hydrochlorid

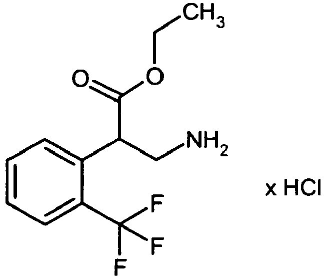

450 mg (1.05 mmol) der Verbindung aus Beispiel 193A werden in 10 ml Ethanol gelöst und unter Wasserstoff (Normaldruck) mit 50 mg Palladium (10% auf Kohle) als Katalysator hydriert. Eine Reaktionskontrolle nach 18 h zeigt vollständige Umsetzung. Der Katalysator wird abfiltriert und das Filtrat am Rotationsverdampfer von den flüchtigen Komponenten befreit. Der Rückstand wird in 10 ml Diethylether aufgenommen und mit 0.4 ml einer 4 M Lösung von Chlorwasserstoff in Dioxan versetzt. Der ausgefallene Feststoff wird abgesaugt und im Hochvakuum getrocknet. Es werden 262 mg (84% d. Th.) der Titelverbindung erhalten.
LC/MS [Methode 3]: Rₜ = 2.52 min; m/z = 262 (M+H)⁺
¹H-NMR (400 MHz, DMSO-d₆): δ = 1.11 (t, 3H), 3.06 (m, 1H), 3.51 (m, 1H), 4.07-4.18 (m, 2H), 4.31 (m, 1H), 7.53 (d, 1H), 7.59 (t, 1H), 7.22 (d, 1H), 7.80 (d, 1H), 8.18 (br. s, 3H).

### Beispiel 195A

### 3-{[(Benzyloxy)carbonyl]amino}-2-[3-(trifluormethyl)phenyl]propansäureethylester

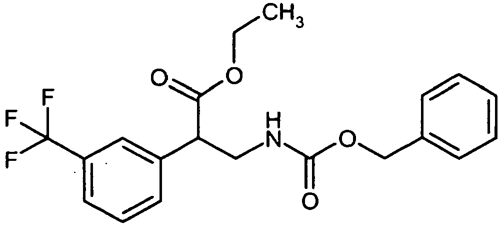

Nach dem gleichen Verfahren wie für Beispiel 193A beschrieben, werden aus 700 mg (3.02 mmol) 3-(Trifluormethyl)phenylessigsäureethylester 334 mg (28% d. Th.) der Titelverbindung erhalten.
LC/MS [Methode 8]: Rₜ = 2.83 min; m/z = 396 (M+H)⁺
¹H-NMR (400 MHz, DMSO-d₆): δ = 1.12 (t, 3H), 3.40 (m, 1H), 3.60 (m, 1H), 4.00 (t, 1H), 4.03-4.15 (m, 2H), 4.99 (s, 2H), 7.22-7.38 (m, 5H), 7.46 (t, 1H), 7.52-7.70 (m, 4H).

### Beispiel 196A

### 3-Amino-2-[3-(trifluormethyl)phenyl]propansäureethylester-Hydrochlorid

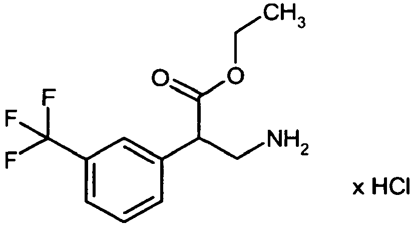

Analog zur Herstellung von Beispiel 194A erhält man aus 315 mg der Verbindung aus Beispiel 195A 220 mg der Titelverbindung (Reinheit ca. 87%, 81 % d. Th.). Sie wird ohne weitere Reinigung eingesetzt.
LC/MS [Methode 8]: Rₜ = 1.27 min; m/z = 262 (M+H)⁺
¹H-NMR (400 MHz, DMSO-d₆): δ = 1.12 (t, 3H), 3.20 (m, 1H), 3.51 (m, 1H), 4.12 (q, 2H), 4.22 (t, 1H), 7.07-7.25 (m, 4H), 8.11 (br. s, 3H).

### Beispiel 197A

### 1-(2,3-Dichlorbenzyl)cyclopropanamin-Hydrochlorid

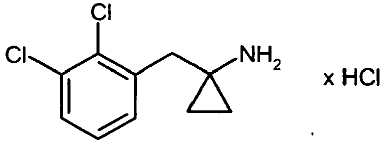

Aus 1.00 g 2,3-Dichlorphenylacetonitril (5.37 mmol) erhält man nach der unter Beispiel 162A beschriebenen Methode 723 mg der Titelverbindung (53% d. Th.).
LC/MS [Methode 3]: Rₜ = 2.48 min; m/z = 216 (M+H)⁺
¹H-NMR (400 MHz, DMSO-d₆): δ = 0.71 (m, 2H), 0.98 (m, 2H), 3.23 (s, 2H), 7.38 (t, 1H), 7.46 (d, 1H), 7.60 (d, 1H), 8.41 (br. s, 3H).

### Beispiel 198A

### 1-(2,6-Dichlorbenzyl)cyclopropanamin-Hydrochlorid

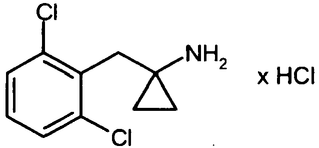

Aus 1.30 g 2,6-Dichlorphenylacetonitril (6.99 mmol) erhält man nach der unter Beispiel 162A beschriebenen Methode 1.24 g der Titelverbindung (62% d. Th.).
LC/MS [Methode 8]: Rₜ = 0.94 min; m/z = 216 (M+H)⁺
¹H-NMR (400 MHz, DMSO-d₆): δ = 0.35 (m, 2H), 0.91 (m, 2H), 3.52 (s, 2H), 7.36 (t, 1H), 7.50 (d, 2H), 8.59 (br. s, 3H).

### Beispiel 199A

### 1-[2,3-Bis(trifluormethyl)phenyl]ethaniminiumchlorid

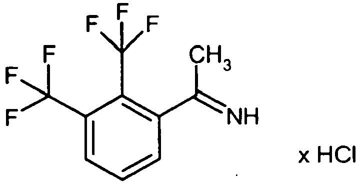

Unter Argon wird eine Lösung von 200 mg (0.84 mmol) 2,3-Bis(trifluormethyl)benzonitril in 2.5 ml Toluol auf Rückfluss erhitzt und mit 3.59 ml Methylmagnesiumbromid (1.4 M Lösung in Toluol/THF 3:1; 5 mmol) versetzt. Es wird 3 h bei Rückflusstemperatur weiter gerührt, dann auf RT abgekühlt. Danach werden 10 ml einer ges. Natriumcarbonat-Lösung zugetropft. Die Reaktionsmischung wird mit Wasser verdünnt und zweimal mit Ethylacetat extrahiert. Die vereinigten organischen Phasen werden zweimal mit 1 N Salzsäure extrahiert. Die vereinigten wässrigen Phasen werden mit 2 N Natronlauge auf pH 12 gestellt und zweimal mit Dichlormethan extrahiert. Diese organischen Phasen werden vereinigt, über Natriumsulfat getrocknet und filtriert. Das Filtrat wird mit 1 ml einer 4 N Lösung von Chlorwasserstoff in Dioxan versetzt und dann am Rotationsverdampfer vom Lösungsmittel befreit. Es werden 172 mg (67% d. Th.) der Titelverbindung erhalten.
MS [DCI/NH₃]: m/z = 256 (M+H)⁺, 273 (M+NH₄)⁺
¹H-NMR (400 MHz, DMSO-d₆): δ = 2.82 (s, 3H), 8.11 (d, 1H), 8.17 (t, 1H), 8.31 (d, 1H), 13.35 (br. s, 2H).

### Beispiel 200A

### 1-[2,3-Bis(trifluormethyl)phenyl]ethanamin-Hydrochlorid

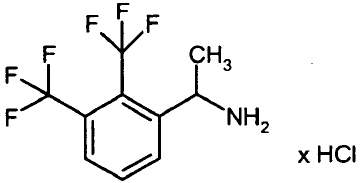

170 mg (0.58 mmol) der Verbindung aus Beispiel 199A werden in 4 ml Methanol gelöst und bei RT nacheinander mit 147 mg (2.33 mmol) Natriumcyanoborhydrid und 334 µl Essigsäure versetzt. Die Mischung wird über Nacht bei RT gerührt, dann mit Wasser verdünnt und zweimal mit Dichlormethan ausgeschüttelt. Die saure wässrige Phase wird mit 2 N Natronlauge auf pH 14 gestellt und dreimal mit Dichlormethan extrahiert. Die vereinigten organischen Phasen werden über Natriumsulfat getrocknet und filtriert. Das Filtrat wird mit 1 ml einer 4 N Lösung von Chlorwasserstoff in Dioxan versetzt und dann am Rotationsverdampfer vom Lösungsmittel befreit. Der Rückstand wird im Hochvakuum getrocknet und entspricht der Titelverbindung (160 mg, 93% d. Th.).
MS [DCI/NH₃]: m/z = 258 (M+H)⁺, 275 (M+NH₄)⁺
¹H-NMR (400 MHz, DMSO-d₆): δ = 1.60 (d, 3H), 4.65 (q, 1H), 8.03-8.11 (m, 2H), 8.39 (d, 1H), 8.78 (br. s, 3H).

### Beispiel 201 A

### [3-(Trifluormethyl)phenyl]-p-toluolsulfonylhydrazon

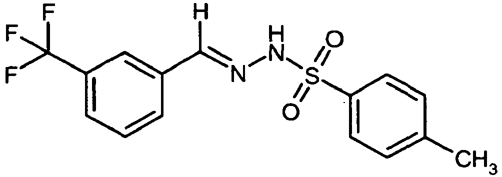

Eine Lösung von 2.35 g (12.6 mmol) p-Toluolsulfonylhydrazin in 5 ml Methanol wird bei RT langsam mit 2.00 g 3-(Trifluormethyl)benzaldehyd versetzt. Es wird über Nacht bei RT gerührt und dann das Lösungsmittel am Rotationsverdampfer entfernt. Der Rückstand wird in 40 ml Cyclohexan/Dichlormethan (5:1) aufgenommen und über Nacht gerührt. Der ausgefallene Feststoff wird abgesaugt, mit wenig Cyclohexan/Dichlormethan (5:1) gewaschen und im Hochvakuum getrocknet. Man erhält so 1.27 g der Titelverbindung. Da die Mutterlauge noch viel Produkt enthält, wird sie am Rotationsverdampfer bis auf ein Volumen von ca. 10 ml eingeengt. Der ausgefallene Feststoff wird erneut abgesaugt, mit wenig Cyclohexan/Essigsäureethylester (5:1) gewaschen und im Hochvakuum getrocknet. Man erhält weitere 2.02 g der Titelverbindung (Ausbeute insgesamt 84% d. Th.).
LC/MS [Methode 19]: Rₜ = 3.62 min; m/z = 343 (M+H)⁺
¹H-NMR (400 MHz, DMSO-d₆): δ = 3.36 (s, 3H), 7.40 (d, 2H), 7.63 (t, 1H), 7.72-7.79 (m, 3H), 7.85-7.90 (m, 2H), 8.00 (s, 1H), 11.70 (s, 1H).

### Beispiel 202A

### cis-N-{2-[3-(Trifluormethyl)phenyl]cyclopropyl}phthalimid

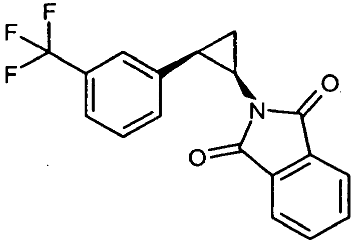

1.00 g (2.92 mmol) der Verbindung aus Beispiel 201A werden in 14 ml THF bei -78°C unter Argon langsam mit 4.38 ml LiHMDS (1 M Lösung in THF, 4.38 mmol) versetzt. Nach 15 min bei dieser Temperatur lässt man die Reaktionsmischung auf RT erwärmen. Das THF wird am Rotationsverdampfer entfernt. Zum verbleibenden Lithium-Salz werden 67 mg Benzyltriethylammoniumchlorid (0.29 mmol), 13 mg Rhodiumacetat-Dimer (29 µmol), 2.02 g *N*-Vinylphthalimid (11.68 mmol) sowie anschließend 14 ml Dioxan gegeben. Die Reaktionsmischung wird über Nacht bei RT gerührt, dann auf Wasser gegossen und dreimal mit Dichlormethan extrahiert. Die vereinigten organischen Phasen werden über Natriumsulfat getrocknet und das Lösungsmittel am Rotationsverdampfer entfernt. Der Rückstand wird über präparative HPLC (nach Methode 20, jedoch mit Acetonitril/0.3% Salzsäure statt Acetonitril/Ameisensäure) gereinigt. Die produkthaltigen Fraktionen werden am Rotationsverdampfer von den flüchtigen Komponenten befreit und der Rückstand im Hochvakuum getrocknet. Man erhält 346 mg der Titelverbindung (Reinheit ca. 73%, 26% d. Th.).
¹H-NMR (400 MHz, DMSO-d₆): δ = 1.30 (t, 3H), 1.68 (m, 1H), 2.01 (m, 1H), 2.70 (q, 1H), 3.16 (m, 1H), 7.27 (s, 1H), 7.32-7.41 (m, 3H), 7.69-7.80 (m, 4H).

### Beispiel 203A

### cis-2-[3-(Trifluormethyl)phenyl]cyclopropylamin-Hydrochlorid

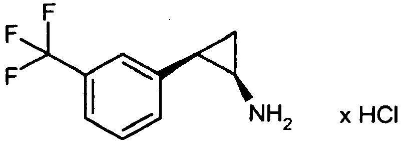

346 mg (0.76 mmol) der Verbindung aus Beispiel 202A werden in 3 ml Ethanol mit 185 µl (3.8 mmol) Hydrazinhydrat bei 40°C für 3 h verrührt und dann am Rotationsverdampfer von allen flüchtigen Komponenten befreit. Der Rückstand wird mit 3-4 ml DMSO versetzt, filtriert und das Filtrat durch präparative HPLC (Methode 20) aufgereinigt. Die produkthaltigen Fraktionen werden mit 3 ml 1 N Salzsäure versetzt und am Rotationsverdampfer von den flüchtigen Komponenten befreit. Der Rückstand wird im Hochvakuum getrocknet und entspricht der Titelverbindung (66 mg, 36% d. Th.).
LC/MS [Methode 23]: Rₜ = 0.65 min; m/z = 202 (M+H)⁺
¹H-NMR (400 MHz, DMSO-d₆): δ = 1.31-1.37 (m, 2H), 2.49 (m, 1H), 2.89 (m, 1H), 7.56-7.73 (m, 4H), 8.14 (br. s, 3H).

### Beispiel 204A

### Difluor-[3-(trifluormethyl)phenyl]-acetonitril

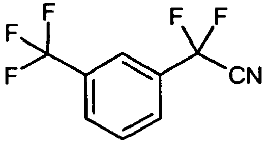

Eine Lösung von 500 mg [3-(Trifluormethyl)phenyl]-acetonitril (2.70 mmol) in 12 ml wasserfreiem THF wird bei -78°C tropfenweise mit 3.50 ml einer tert.-Butyllithium-Lösung (1.7 M in Pentan, 5.94 mmol) versetzt. Die braune Reaktionsmischung wird 1 h bei -78°C gerührt, dann wird eine Lösung von 2.04 g (6.5 mmol) N-Fluorbenzolsulfonsäureimid in 12 ml THF zugegeben. Es wird noch 2 h bei -78°C gerührt und dann die Reaktion durch Zugabe von 0.1 M Salzsäure gestoppt. Nach Erwärmung auf RT wird die Mischung zweimal mit Dichlormethan extrahiert. Die vereinigten organischen Phasen werden mit verdünnter Natriumbicarbonat-Lösung, dann mit ges. Kochsalz-Lösung gewaschen, anschließend über Natriumsulfat getrocknet und am Rotationsverdampfer vom Lösungsmittel befreit. Der Rückstand wird durch präparative HPLC (Methode 20) gereinigt. Die produkthaltigen Fraktionen werden am Rotationsverdampfer vom Acetonitril befreit und die verbleibende wässrige Phase mit Dichlormethan extrahiert. Die organischen Phasen werden über Magnesiumsulfat getrocknet und das Lösungsmittel am Rotationsverdampfer entfernt. Man erhält 85 mg der Titelverbindung in ca. 81% Reinheit (12% d. Th.).
GC/MS [Methode 21]: Rₜ = 1.49 min; m/z = 221 (M)⁺
¹H-NMR (400 MHz, DMSO-d₆): δ = 7.35-7.43 (m, 2H), 7.63-7.70 (m, 2H).

### Beispiel 205A

### 2,2-Difluor-2-[3-(trifluormethyl)phenyl]ethanamin-Hydrochlorid

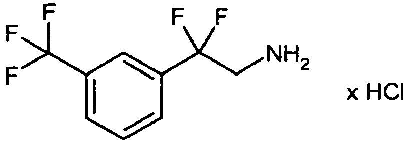

Die Boran-Reduktion der Verbindung aus Beispiel 204A nach der unter Beispiel 157A beschriebenen Methode ergibt 73 mg (68% d. Th.) der Titelverbindung.
LC/MS [Methode 23]: Rₜ = 0.69 min; m/z = 226 (M+H)⁺
¹H-NMR (400 MHz, DMSO-d₆): δ = 3.37 (t, 2H), 7.81 (t, 1H), 7.94-8.03 (m, 3H), 8.71 (br. s, 3H).

### Beispiel 206A

### 2-(4-Chlorbenzoyl)-N-allylhydrazincarboxamid

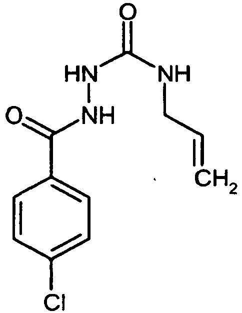

Unter einer Argonatmosphäre werden 19.00 g (111.4 mmol) 4-Chlorbenzoesäurehydrazid in 150 ml THF vorgelegt. Bei 50°C werden 9.44 g (111.6 mmol) Allylisocyanat, gelöst in 110 ml THF, zugetropft und das Gemisch über Nacht bei 50°C weiter gerührt. Man engt danach das Lösungsmittel im Vakuum ein, gibt zum Rückstand Diethylether und isoliert und reinigt den sich bildenden Feststoff durch Filtration und Nachwaschen mit Diethylether. Man erhält so 26.80 g (95% d. Th.) der Zielverbindung.
LC/MS [Methode 18]: Rₜ = 1.51 min; MS [ESIpos]: m/z = 254 (M+H)⁺
¹H-NMR (400 MHz, DMSO-d₆): δ = 3.60-3.70 (m, 2H), 5.01 (d, 1H), 5.15 (d, 1H), 5.80 (m, 1H), 6.70 (s, 1H), 7.56 (d, 2H), 7.90 (d, 2H), 7.92 (s, 1H), 10.21 (s, 1H).

Auf analoge Weise werden die folgenden Verbindungen hergestellt:

| **Beispiel Nr.** | **Struktur** | **LC/MS oder HPLC, MS R, [Methode]** |
|---|---|---|
| **207A** | 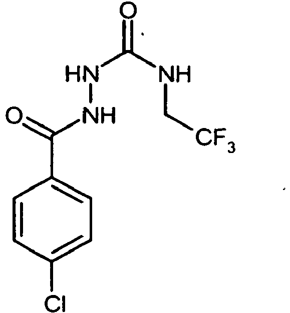 | LC/MS: Rₜ = 1.66 min [8] |
| | | [ESIpos]: m/z = 296 (M+H)⁺ |
| **208A** | 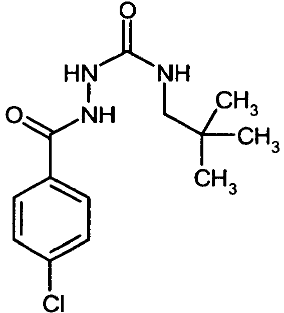 | LC/MS: Rₜ = 2.75 min [17] |
| | | [ESIpos]: m/z = 284 (M+H)⁺ |
| **209A** | 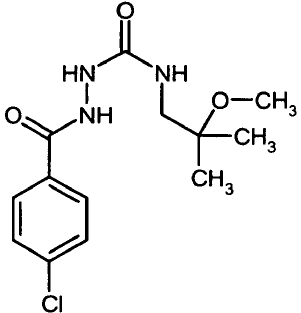 | LC/MS: Rₜ = 2.07 min [17] |
| | | [ESIpos]: m/z = 300 (M+H)⁺ |

### Beispiel 210A

### 5-(4-Chlorphenyl)-4-allyl-2,4-dihydro-3H-1,2,4-triazol-3-on

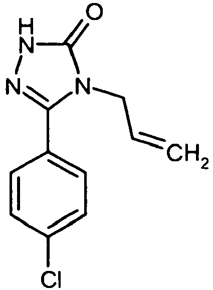

26.80 g (105.6 mmol) 2-(4-Chlorbenzoyl)-*N*-allylhydrazincarboxamid aus Beispiel 206A werden in 211 ml 3 N Natronlauge über Nacht unter Rückfluss erhitzt. Nach dem Abkühlen wird mit 6 N Salzsäure auf pH 10 eingestellt, wobei das Produkt nahezu vollständig ausfällt. Der Niederschlag wird abgesaugt, mit viel Wasser gewaschen und anschließend mit Methanol verrührt. Es bleibt ein unlöslicher weißer Niederschlag zurück, von dem abfiltriert wird. Das Filtrat wird im Vakuum eingeengt und der verbleibende Rückstand wird im Hochvakuum getrocknet. Man erhält so 21.5 g (86% d. Th.) der Zielverbindung.
LC/MS [Methode 18]: Rₜ = 1.79 min; MS [ESIpos]: m/z = 236 (M+H)⁺
¹H-NMR (400 MHz, DMSO-d₆): δ = 4.30-4.35 (m, 2H), 4.90 (d, 1H), 5.12 (d, 1H), 5.85 (m, 1H), 7.57 (d, 2H), 7.63 (d, 2H), 12.06 (s, 1H).

Auf analoge Weise werden die folgenden Verbindungen hergestellt:

| **Beispiel Nr.** | **Struktur** | **Edukt** | **LC/MS oder HPLC, MS Rₜ [Methode]** |
|---|---|---|---|
| **211A** | 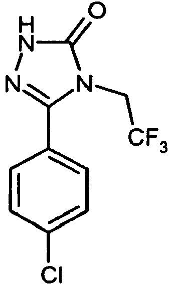 | 207A | LC/MS: Rₜ = 2.66 min [19] |
| | | | [ESIpos]: m/z = 278 (M+H)⁺ |
| **212A** | 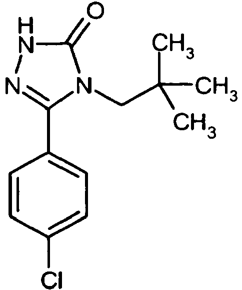 | 208A | LC/MS: Rₜ = 2.98 min [19] |
| | | | [ESIpos]: m/z = 266 (M+H)⁺ |
| **213A** | 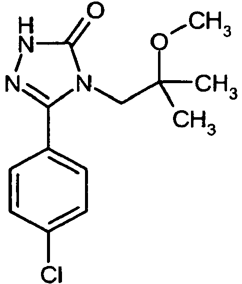 | 209A | LC/MS: Rₜ = 1.91 min [8] |
| | | | [ESIpos]: m/z = 282 (M+H)⁺ |

### Beispiel 214A

### [3-(4-Chlorphenyl)-4-allyl-5-oxo-4,5-dihydro-1H-1,2,4-triazol-1-yl]-essigsäuremethylester

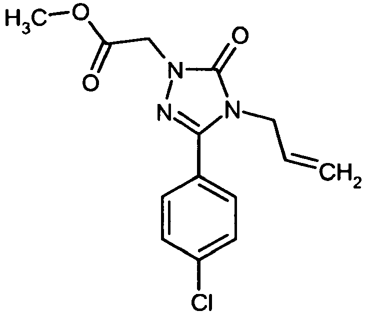

Zu 21.50 g (91.2 mmol) 5-(4-Chlorphenyl)-4-allyl-2,4-dihydro-3*H*-1,2,4-triazol-3-on aus Beispiel 210A und 11.88 g (109.5 mmol) Chloressigsäuremethylester in 350 ml Acetonitril werden 13.87 g (100.35 mmol) Kaliumcarbonat gegeben und das Gemisch unter Rühren für 5 h unter Rückfluss erhitzt. Man engt danach ein und wäscht den in Essigsäureethylester aufgenommenen Rückstand mit 1 N Salzsäure und anschließend mit gesättigter Natiumchlorid-Lösung. Die organische Phase wird über Natriumsulfat getrocknet. Nach Filtration wird das Filtrat im Vakuum eingeengt. Nach Reinigung durch Flash-Chromatographie an Kieselgel (Eluent: Cyclohexan/Essigsäureethylester 2:1) erhält man 24.00 g (85% d. Th.) der Zielverbindung.
¹H-NMR (400 MHz, DMSO-d₆): δ = 3.70 (s, 3H), 4.36-4.43 (m, 2H), 4.72 (s, 2H), 4.93 (d, 1H), 5.15 (d, 1H), 5.86 (m, 1H), 7.60 (d, 2H), 7.66 (d, 2H).

Auf analoge Weise werden die folgenden Verbindungen hergestellt:

| **Beispiel Nr.** | **Struktur** | **Edukt** | **LC/MS oder HPLC, MS Rₜ [Methode]** |
|---|---|---|---|
| **215A** | 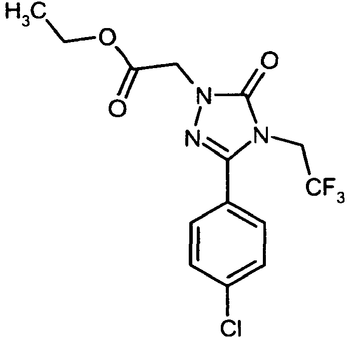 | 211A | LC/MS: Rₜ = 2.39 min [8] |
| | | | [ESIpos]: m/z = 364 (M+H)⁺ |
| **216A** | 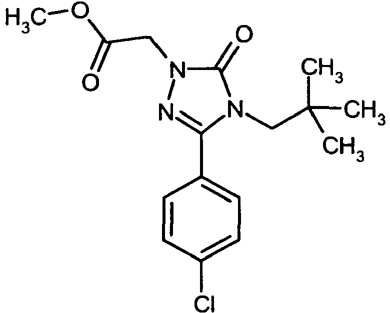 | 212A | LC/MS: Rₜ = 3.36 min [19] |
| | | | [ESIpos]: m/z = 338 (M+H)⁺ |
| **217A** | 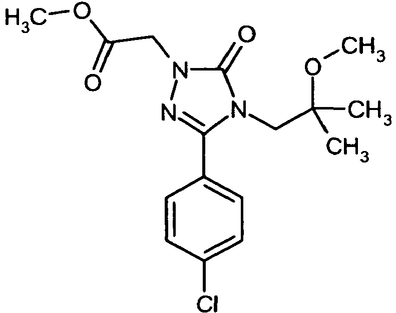 | 213A | LC/MS: Rₜ = 2.19 min [8] |
| | | | [ESIpos]: m/z = 354 (M+H)⁺ |

### Beispiel 218A

### [3-(4-Chlorphenyl)-4-allyl-5-oxo-4,5-dihydro-1H-1,2,4-triazol-l-yl]-essigsäure

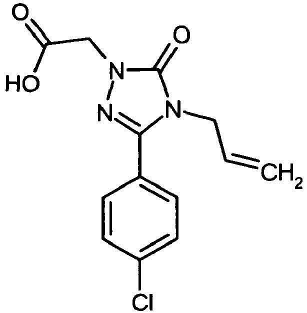

4.88 g (15.9 mmol) [3-(4-Chlorphenyl)-4-allyl-5-oxo-4,5-dihydro-1*H*-1,2,4-triazol-1-yl]-essigsäureethylester aus Beispiel 214A werden in 48 ml Methanol vorgelegt und mit 5 ml 20%-iger Kalilauge 2 h bei Raumtemperatur gerührt. Die Lösung wird auf ca. die Hälfte eingeengt, anschließend wird mit Wasser verdünnt und mit Essigsäureethylester extrahiert. Die wässrige Phase wird mit ca. 2 ml konz. Salzsäure angesäuert und zweimal mit je 100 ml Essigsäureethylester extrahiert. Die letzten organischen Extrakte werden vereinigt, über Natriumsulfat getrocknet, filtriert und im Vakuum eingeengt. Nach dem Trocknen im Hochvakuum erhält man so 4.20 g (90% d. Th.) der Zielverbindung.
LC/MS [Methode 8]: Rₜ = 2.93 min; MS [ESIpos]: m/z = 294 (M+H)^{+ 1}H-NMR (400 MHz, DMSO-d₆): δ = 4.36-4.43 (m, 2H), 4.59 (s, 2H), 4.93 (d, 1H), 5.15 (d, 1H), 5.87 (m, 1H), 7.60 (d, 2H), 7.67 (d, 2H), 13.17 (s, 1H).

Auf analoge Weise werden die folgenden Verbindungen hergestellt:

| **Beispiel Nr.** | **Struktur** | **Edukt** | **LC/MS oder HPLC, MS Rₜ [Methode]** |
|---|---|---|---|
| **219A** | 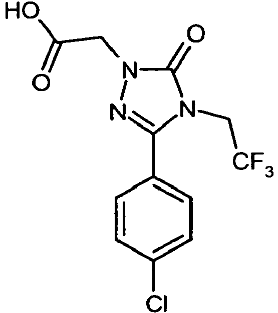 | 215A | LC/MS: Rₜ = 2.69 min [19] |
| | | | [ESIpos]: m/z = 336 (M+H)⁺ |
| **220A** | 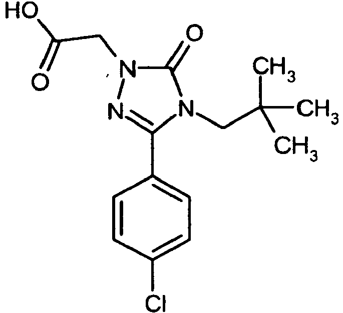 | 216A | LC/MS: Rₜ = 2.98 min [19] |
| | | | [ESIpos]: m/z = 324 (M+H)⁺ |
| **221A** | 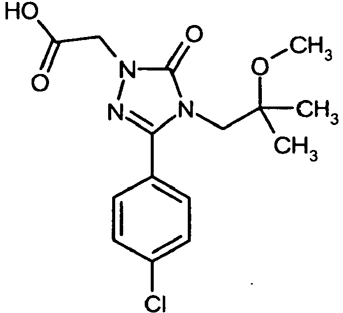 | 217A | LC/MS: Rₜ = 2.69 min [17] |
| | | | [ESIpos]: m/z = 340 (M+H)⁺ |

### Beispiel 222A

### [3-(4-Chlorphenyl)-5-oxo-4,5-dihydro-1H-1,2,4-triazol-1-yl]-essigsäuremethylester

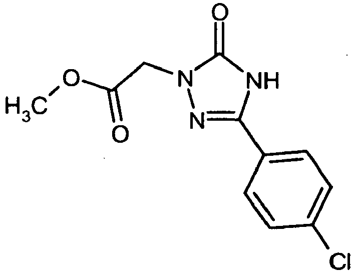

Unter Argonatmosphäre werden 200 mg (0.65 mmol) der Verbindung aus Beispiel 214A, 49 µl Ameisensäure (1.3 mmol), 226 µl Triethylamin (1.63 mmol) und 38 mg Tetrakis(triphenylphosphin)palladium(0) (32 µmol) in 2 ml entgastem Dioxan gelöst und über Nacht bei Rückflusstemperatur gerührt. Um den ausgefallenen Feststoff zu lösen, wird die Reaktionsmischung nach Abkühlung auf RT mit 20 ml Methanol verdünnt. Der Palladium-Katalysator wird abfiltriert und das Filtrat am Rotationsverdampfer von den flüchtigen Komponenten befreit. Der Rückstand wird mit 5 ml Acetonitril verrührt und dann abgesaugt. Der Feststoff wird mit Acetonitril gewaschen und im Hochvakuum getrocknet. Er entspricht der Titelverbindung mit einer Reinheit von ca. 76% (130 mg, 57% d. Th.) und wird ohne weitere Reinigung in die nächste Reaktion eingesetzt.
LC/MS [Methode 19]: Rₜ = 2.34 min; m/z = 268 (M+H)⁺
¹H-NMR (400 MHz, DMSO-d₆): δ = 3.70 (s, 3H), 4.63 (s, 2H), 7.59 (d, 2H), 7.80 (d, 2H).

### Beispiel 223A

### {4-[4-(tert.-Butoxy)-4-oxo-n-butyl]-3-(4-chlorphenyl)-5-oxo-4,5-dihydro-1H-1,2,4-triazol-1-yl}-essigsäuremethylester

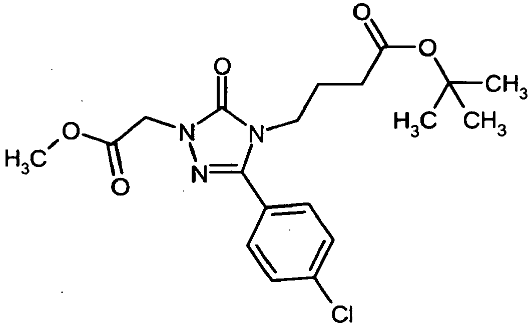

Unter Argonatmosphäre wird eine Lösung von 130 mg (0.38 mmol) der Verbindung aus Beispiel 222A in 1 ml DMF und 2 ml DME bei 0°C mit 505 µl einer LiHMDS-Lösung (1M in THF, 505 µmol) versetzt. Man entfernt das Kühlbad und lässt 15 min bei RT rühren, bevor man 113 mg (505 µmol) 4-Brombutansäure-tert.-butylester zugibt. Die Mischung wird über Nacht bei 70°C gerührt. Nach dem Abkühlen werden 0.5 ml 1 N Salzsäure zugegeben. Die Reaktionsmischung wird dann direkt durch präparative HPLC (Methode 20) aufgetrennt. Man erhält 48 mg (30% d. Th.) der Titelverbindung.
LC/MS [Methode 17]: Rₜ = 3.67 min; m/z = 410 (M+H)⁺
¹H-NMR (400 MHz, DMSO-d₆): δ = 1.31 (s, 9H), 1.69 (quin, 2H), 2.12 (t, 2H), 3.70 (s, 3H), 3.79 (t, 2H), 4.69 (s, 2H), 7.52 (d, 2H), 7.70 (d, 2H).

### Beispiel 224A

### {4-[4-(tert.-Butoxy)-4-oxo-n-butyl]-3-(4-chlorphenyl)-5-oxo-4,5-dihydro-1H-1,2,4-triazol-1-yl}-essigsäure

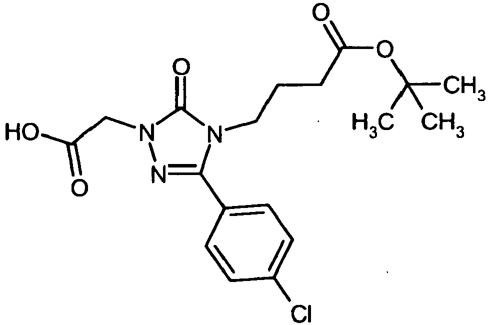

Eine Lösung von 48 mg (117 µmol) der Verbindung aus Beispiel 223A in 2 ml Methanol wird mit einer 1 N Lithiumhydroxid-Lösung in Wasser (470 µl, 470 µmol) versetzt. Nach 1 h bei RT entfernt man das Methanol am Rotationsverdampfer. Der Rückstand wird in DMSO gelöst und durch präparative HPLC gereinigt. Man erhält 41 mg (88% d. Th.) der Titelverbindung.
LC/MS [Methode 8]: Rₜ = 2.48 min; m/z = 396 (M+H)⁺
¹H-NMR (400 MHz, DMSO-d₆): δ = 1.33 (s, 9H), 1.68 (quin, 2H), 2.14 (t, 2H), 3.77 (t, 2H), 4.54 (s, 2H), 7.61 (d, 2H), 7.70 (d, 2H), 13.14 (br. s, 1H).

### Beispiel 225A

### [3-(4-Chlorphenyl)-4-(2-oxoethyl)-5-oxo-4,5-dihydro-1H-1,2,4-triazol-1-yl]-essigsäuremethylester

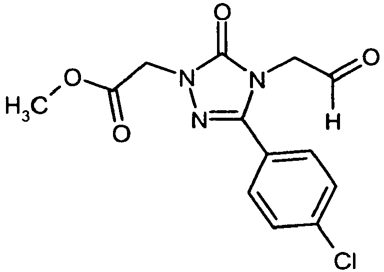

1.00 g (3.25 mmol) der Verbindung aus Beispiel 214A und 217 mg OsEnCat 40 (mikroverkapseltes Osmiumtetroxid, 0.3 mmol/g, 65 µmol) werden in 20 ml Dioxan und 9 ml Wasser vorgelegt und bei RT langsam mit 2.09 g (9.8 mmol) Natriumperiodat versetzt. Unter starkem Rühren lässt man solange reagieren (1-4 Tage), bis die HPLC-Kontrolle der Reaktionsmischung adäquaten Umsatz zeigt. Zur Aufarbeitung wird der Osmium-Katalysator durch Filtration entfernt, mit Dioxan nachgewaschen und das gesamte Filtrat am Rotationsverdampfer von den organischen Lösungsmitteln befreit. Der wässrige Rückstand wird mit mehr Wasser verdünnt und dreimal mit Dichlormethan extrahiert. Die vereinigten organischen Phasen werden über Natriumsulfat getrocknet und das Lösungsmittel am Rotationsverdampfer entfernt. Der ölige Rückstand wird im Hochvakuum getrocknet. Man erhält 948 mg (Reinheit ca. 84%, 79% d. Th.) der Titelverbindung.
LC/MS [Methode 17]: Rₜ = 1.89 min; m/z = 310 (M+H)⁺
¹H-NMR (400 MHz, CDCl₃): δ = 3.79 (s, 3H), 4.61 (s, 2H), 4.67 (s, 2H), 7.37-7.59 (m, 4H), 9.62 (s, 1H).

### Beispiel 226A

### [3-(4-Chlorphenyl)-5-oxo-4-(3,3,3-trifluor-2-hydroxypropyl)-4,5-dihydro-1H-1,2,4-triazol-1-yl]-essigsäuremethylester (Racemat)

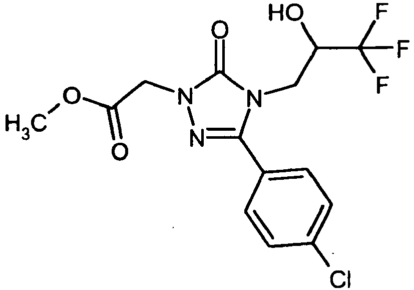

Zu einer Lösung von 948 mg (2.57 mmol) der Verbindung aus Beispiel 225A in 17 ml THF werden bei 0°C nacheinander 6.69 ml einer 0.5 M Lösung von (Trifluormethyl)trimethylsilan in THF (3.34 mmol) sowie 39 µl einer 1 M Lösung von Tetra-n-butylammoniumfluorid in THF (39 µmol) gegeben. Man lässt die Temperatur auf RT steigen und rührt noch 1 h nach. Zur Aufarbeitung wird die Reaktionsmischung mit 8 ml 1 N Salzsäure versetzt. Man lässt 1 h bei RT rühren, bevor man das THF am Rotationsverdampfer entfernt. Der wässrige Rückstand wird mit Essigsäureethylester extrahiert. Die organische Phase wird zweimal mit Wasser und einmal mit ges. Kochsalz-Lösung gewaschen, über Magnesiumsulfat getrocknet und am Rotationsverdampfer vom Lösungsmittel befreit. Der Rückstand wird über Kieselgel-Chromatographie (Laufmittel: Dichlormethan/Methanol 100:1 → 100:2) gereinigt. Man erhält 630 mg (65% d. Th.) der Titelverbindung.
LC/MS [Methode 8]: Rₜ = 2.23 min; m/z = 380 (M+H)⁺
¹H-NMR (400 MHz, DMSO-d₆): δ = 3.70 (s, 3H), 3.84 (dd, 1H), 4.00 (dd, 1H), 4.25 (m, 1H), 4.71 (s, 2H), 6.91 (d, 1H), 7.63 (d, 2H), 7.76 (d, 2H).

Das Racemat aus Beispiel 226A kann durch HPLC an chiraler Phase in die Enantiomere getrennt werden [Säule: Chirale Kieselgel-Phase basierend auf dem Selektor Poly(*N*-methacryloyl-L-isoleucin-3-pentylamid, 430 mm x 40 mm; Eluent: Stufengradient iso-Hexan/Ethylacetat 1:1 → Ethylacetat → iso-Hexan/Ethylacetat 1:1; Fluss: 80 ml/min; Temperatur: 24°C; UV-Detektion: 260 nm]. Man erhält auf diese Weise aus 615 mg racemischer Verbindung 265 mg des zuerst eluierenden Enantiomers 1 (Beispiel 227A) sowie 271 mg des später eluierenden Enantiomers 2 (Beispiel 228A).

### Beispiel 227A

### [3-(4-Chlorphenyl)-5-oxo-4-(3,3,3-trifluor-2-hydroxypropyl)-4,5-dihydro-1H-1,2,4-tr-iazol-1-yl]-essigsäuremethylester (Enantiomer 1)

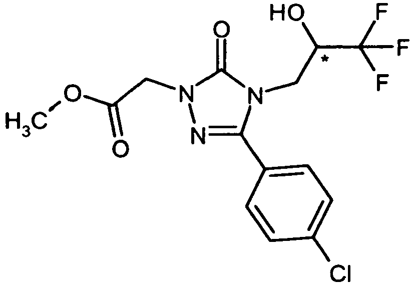

Zuerst eluierendes Enantiomer aus der Racemat-Trennung von Beispiel 226A.
Rₜ = 3.21 min [Säule: Chirale Kieselgel-Phase basierend auf dem Selektor Poly(*N*-methacryloyl-L-isoleucin-3-pentylamid, 250 mm x 4.6 mm; Eluent: iso-Hexan/Ethylacetat 1:1; Fluss: 1 ml/min; UV-Detektion: 260 nm].

### Beispiel 228A

### [3-(4-Chlorphenyl)-5-oxo-4-(3,3,3-trifluor-2-hydroxypropyl)-4,5-dihydro-1H-1,2,4-triazol-1-yl]-essigsäuremethylester (Enantiomer 2)

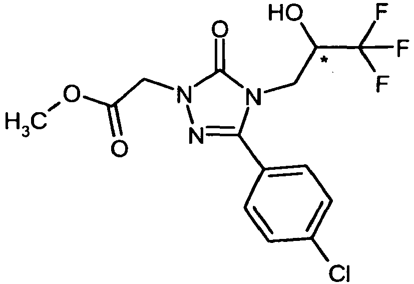

Zuletzt eluierendes Enantiomer aus der Racemat-Trennung von Beispiel 226A.
Rₜ = 4.48 min [Säule: Chirale Kieselgel-Phase basierend auf dem Selektor Poly(*N*-methacryloyl-L-isoleucin-3-pentylamid, 250 mm x 4.6 mm; Eluent: iso-Hexan/Ethylacetat 1:1; Fluss: 1 ml/min; UV-Detektion: 260 nm].

### Beispiel 229A

### [3-(4-Chlorphenyl)-5-oxo-4-(3,3,3-trifluor-2-hydroxypropyl)-4,5-dihydro-1H-1,2,4-triazol-1-yl]-essigsäure (Enantiomer 1)

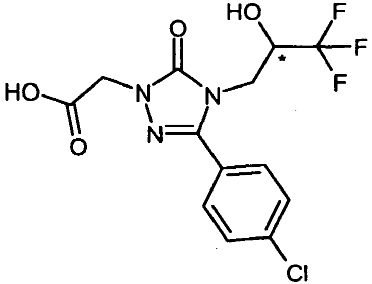

Der enantiomerenreine Ester aus Beispiel 227A (265 mg, 0.70 mmol) wird in 14 ml Methanol gelöst und mit 2.8 ml einer 1 M Lösung von Lithiumhydroxid in Wasser versetzt. Die Mischung wird 1 h bei RT gerührt und dann am Rotationsverdampfer vom Methanol befreit. Der Rückstand wird mit 200 ml Wasser verdünnt und einmal mit Dichlormethan extrahiert. Diese organische Phase wird verworfen. Die wässrige Phase wird langsam mit 1 N Salzsäure auf pH 2 angesäuert. Das Produkt wird dreimal mit Dichlormethan extrahiert, die vereinigten organischen Phasen werden über Natriumsulfat getrocknet und das Lösungsmittel am Rotationsverdämpfer entfernt. Der Rückstand wird im Hochvakuum getrocknet. Man erhält so 142 mg (56% d. Th.) der Titelverbindung. Da die wässrige Phase noch weiteres Produkt enthält, wird sie am Rotationsverdampfer bis zur Trockene eingeengt, der Rückstand in wenig DMSO gelöst und durch präparative HPLC (Methode 20) gereinigt. Man erhält weitere 71 mg (28% d. Th.) der reinen Titelverbindung.
[α]_{D}²⁰ = +3.4° (Methanol, c = 0.37 g/100 ml)
LC/MS [Methode 17]: Rₜ = 2.83 min; m/z = 366 (M+H)⁺
¹H-NMR (400 MHz, DMSO-d₆): δ = 3.84 (dd, 1H), 4.00 (dd, 1H), 4.25 (m, 1H), 4.58 (s, 2H), 6.91 (d, 1H), 7.63 (d, 2H), 7.78 (d, 2H), 13.20 (br. s, 1H).

### Beispiel 230A

### [3-(4-Chlorphenyl)-5-oxo-4-(3,3,3-trifluor-2-hydroxypropyl)-4,5-dihydro-1H-1,2,4-triazol-1-yl]-essigsäure (Enantiomer 2)

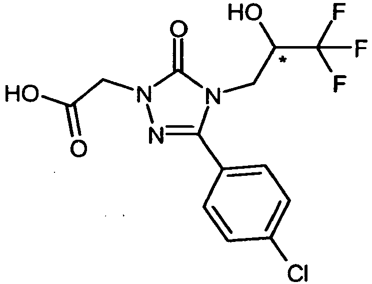

Analog zu Beispiel 229A werden aus 271 mg des enantiomerenreinen Esters aus Beispiel 228A 210 mg (80% d. Th.) der Titelverbindung erhalten.
[α]_{D}²⁰ = 4.6° (Methanol, c = 0.44 g/100 ml)
LC/MS [Methode 17]: Rₜ = 2.83 min; m/z = 366 (M+H)⁺
¹H-NMR (400 MHz, DMSO-d₆): δ = 3.84 (dd, 1H), 4.00 (dd, 1H), 4.25 (m, 1H), 4.58 (s, 2H), 6.91 (d, 1H), 7.63 (d, 2H), 7.78 (d, 2H), 13.20 (br. s, 1H).

### Beispiel 231A

### {[2-(4-Chlorphenyl)-2-oxoethyl]amino} essigsäuremethylester

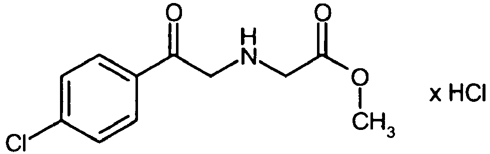

Eine Lösung von Glycinmethylester-Hydrochlorid (5.00 g, 39.8 mmol) in 80 ml Methylisobutylketon wird mit 12.6 g Natriumcarbonat versetzt und die Mischung über Nacht bei RT gerührt. Anschließend wird zu dieser Suspension eine Lösung von 2-Brom-1-(4-chlorphenyl)ethanon (8.37 g, 35.8 mmol) in 40 ml Methylisobutylketon getropft. Die Mischung wird noch 1 h bei RT gerührt, dann der Feststoff abgesaugt und mit 55 ml Methylisobutylketon gewaschen. Das Filtrat wird mit 12 ml 6 N Salzsäure angesäuert, dann mit 6.4 ml Isopropanol versetzt. Der ausgefallene Feststoff wird abgesaugt, mit wenig Methylisobutylketon gewaschen, dann in 240 ml Acetonitril verrührt und abermals abgesaugt. Es werden so 3.41 g (34% d. Th.) der reinen Titelverbindung erhalten.
LC/MS [Methode 3]: Rₜ = 2.20 min; m/z = 242 (M+H)⁺.

### Beispiel 232A

### [4-(4-Chlorphenyl)-2-oxo-2,3-dihydro-1H-imidazol-1-yl]-essigsäuremethylester

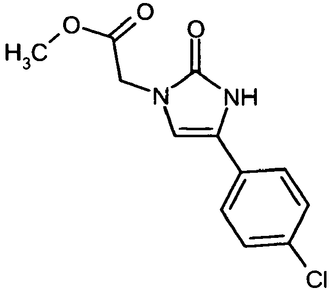

Zu einer Lösung von 995 mg (12.27 mmol) Kaliumisocyanat in 11 ml Methanol/Wasser (7:3) wird eine Lösung von 3.41 g (12.27 mmol) der Verbindung aus Beispiel 231A in 14 ml Methanol/ Wasser (7:3) getropft. Man rührt 1 h bei RT. Die dicke Suspension wird mit 7.4 ml Wasser und 39 ml Methanol verdünnt, um das Rühren zu erleichtern, dann 1 h auf Rückfluss erhitzt und anschließend über Nacht bei RT stehen gelassen. Nach Abkühlung auf 0°C wird der Niederschlag abfiltriert, mit eiskaltem Wasser gewaschen und über Nacht im Trockenschrank bei 60°C getrocknet. Man erhält 2.84 g (76% d. Th.) der Titelverbindung.
LC/MS [Methode 4]: Rₜ = 2.03 min; m/z = 267 (M+H)⁺.

### Beispiel 233A

### [4-(4-Chlorphenyl)-2-oxo-2,3-dihydro-1H-imidazol-1-yl]-essigsäure

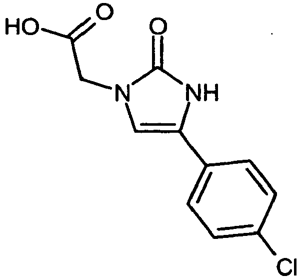

770 mg (2.89 mmol) [4-(4-Chlorphenyl)-2-oxo-2,3-dihydro-1*H*-imidazol-1-yl]-essigsäuremethylester aus Beispiel 232A werden in 20 ml Methanol vorgelegt, mit 5.8 ml 1 M wässriger Lithiumhydroxid-Lösung versetzt und 18 h bei Raumtemperatur gerührt. Man zieht danach das Methanol am Rotationsverdampfer ab und säuert den Rückstand mit 1 N Salzsäure an. Der ausgefallene Niederschlag wird abgesaugt, mit Wasser gewaschen und im Hochvakuum getrocknet. Es werden so 690 mg (94% d. Th.) der Zielverbindung erhalten.
LC/MS [Methode 17]: Rₜ = 2.19 min; MS [ESIpos]: m/z = 253 (M+H)^{+ 1}H-NMR (400 MHz, DMSO-d₆): δ = 4.30 (s, 2H), 7.08 (s, 1H), 7.41 (d, 2H), 7.51 (d, 2H), 10.08 (s, 1H), 13.01 (s, 1H).

### Beispiel 234A

### 2-[4-(4-Chlorphenyl)-2-oxo-2,3-dihydro-1H-imidazol-1-yl]-N-{1-methyl-1-[3-(trifluormethyl)-phenyl]ethyl}acetamid

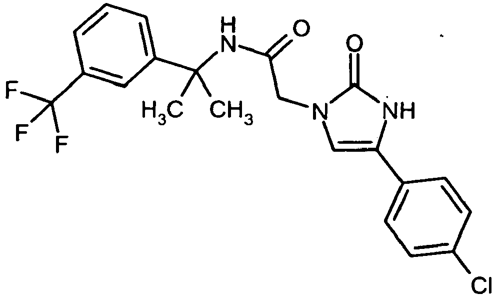

318 mg (1.26 mmol) [4-(4-Chlorphenyl)-2-oxo-2,3-dihydro-1*H*-imidazol-1-yl]-essigsäure aus Beispiel 233A werden in 10 ml DMF vorgelegt und mit 221 mg (1.64 mmol) HOBt sowie 314 mg (1.64 mmol) EDC-Hydrochlorid versetzt. Nach 10 min Rühren werden 332 mg (1.64 mmol) 1-Methyl-1-[(3-trifluormethyl)phenyl]ethylamin aus Beispiel 1A zugegeben und das Gemisch über Nacht bei Raumtemperatur gerührt. Zur Aufarbeitung wird das Reaktionsgemisch mit 100 ml Wasser verrührt. Anschließend wird der ausgefallene Niederschlag abgesaugt, mit Wasser gewaschen und im Hochvakuum getrocknet. Weitere Reinigung erfolgt durch präparative HPLC [Methode 10]. Man erhält so 209 mg (38% d. Th.) der Zielverbindung.
LC/MS [Methode 17]: Rₜ = 3.57 min; MS [ESIpos]: m/z = 438 (M+H)⁺
¹H-NMR (400 MHz, DMSO-d₆): δ = 1.60 (s, 6H), 4.26 (s, 2H), 6.96 (s, 1H), 7.32-7.69 (m, 8H), 8.71 (s, 2H), 10.71 (s, 1H).

### Beispiel 235A

### 1-[2-(5-Chlor-2-thienyl)-2-oxoethyl]-3-(2-fluorbenzyl)-harnstoff

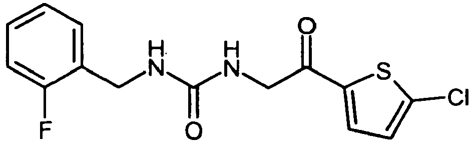

850 mg (4.007 mmol) 2-Amino-1-(4-chlor-2-thienyl)ethanon-Hydrochlorid werden in 26 ml Dichlormethan vorgelegt, auf 0°C gekühlt und tropfenweise mit einer Lösung von 606 mg (4.007 mmol) 2-Fluorbenzylisocyanat in 2 ml Dichlormethan versetzt. Es wird 10 min bei 0°C nachgerührt und dann eine Lösung von 518 mg (4.007 mmol) *N,N*-Diisopropylethylamin in 4 ml Dichlormethan zugetropft. Nach zweistündigem Rühren bei Raumtemperatur engt man das Reaktionsgemisch ein und setzt das Rohprodukt (1300 mg, 99% d. Th.) ohne Reinigung weiter um.
LC/MS [Methode 8]: Rₜ = 2.11 min; MS [ESIpos]: m/z = 327 (M+H)⁺
¹H-NMR (400 MHz, DMSO-d₆): δ = 1.60 (s, 6H), 4.26 (s, 2H), 6.96 (s, 1H), 7.32-7.69 (m, 8H), 8.71 (s, 2H), 10.71 (s, 1H).

### Beispiel 236A

### 5-(5-Chlor-2-thienyl)-1-(2-fluorbenzyl)-1,3-dihydro-2H-imidazol-2-on

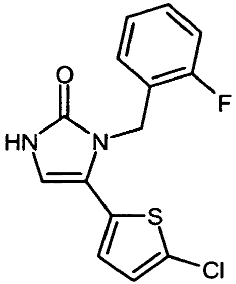

1300 mg (ca. 4.0 mmol) 1-[2-(5-Chlor-2-thienyl)-2-oxoethyl]-3-(2-fluorbenzyl)-harnstoff (Beispiel 235A) werden in 15 ml konzentrierter Salzsäure suspendiert, mit 15 ml Methanol verdünnt und 2 h bei RT gerührt. Die Suspension wird filtriert, das Filtrat im Vakuum eingeengt und der Rückstand durch präparative HPLC [Methode 10] gereinigt. Man erhält so 220 mg (18% d. Th.) der Zielverbindung.
LC/MS [Methode 7]: Rₜ = 2.10 min; MS [ESIpos]: m/z = 309 (M+H)⁺
¹H-NMR (400 MHz, DMSO-d₆): δ = 4.90 (s, 2H), 6.78-7.38 (m, 7H), 10.61 (s, 1H).

### Beispiel 237A

### [4-(5-Chlor-2-thienyl)-3-(2-fluorbenzyl)-2-oxo-2,3-dihydro-1H-imidazol-1-yl]-essigsäureethylester

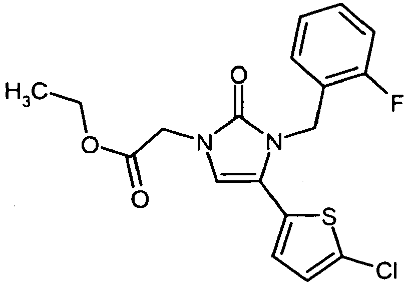

650 mg (2.15 mmol) 5-(5-Chlor-2-thienyl)-1-(2-fluorbenzyl)-1,3-dihydro-2*H*-imidazol-2-on aus Beispiel 236A, 516 mg (4.21 mmol) Chloressigsäureethylester und 582 mg (4.21 mmol) Kaliumcarbonat werden in 12 ml Acetonitril 7 h lang bei 80°C gerührt. Die Reaktionslösung wird mit Essigsäureethylester verdünnt und dreimal mit gesättigter Natriumchlorid-Lösung gewaschen. Die organische Phase wird über Natriumsulfat getrocknet. Nach Filtration vom Trockenmittel wird das Filtrat im Vakuum eingeengt. Nach Reinigung durch Flash-Chromatographie über Kieselgel (Eluent: Cyclohexan/Essigsäureethylester zunächst 5:1, dann 1:1) erhält man 610 mg (74% d. Th.) der Zielverbindung.
LC/MS [Methode 17]: Rₜ = 3.74 min; MS [ESIpos]: m/z = 395 (M+H)⁺
¹H-NMR (400 MHz, DMSO-d₆): δ = 1.22 (t, 3H), 4.18 (q, 2H), 4.51 (s, 2H), 4.97 (s, 2H), 6.86-6.94 (m, 2H), 6.98 (s, 1H), 7.05 (d, 1H), 7.10-7.22 (m, 2H), 7.27-7.36 (m, 1H).

### Beispiel 238A

### [4-(5-Chlor-2-thienyl)-3-(2-fluorbenzyl)-2-oxo-2,3-dihydro-1H-imidazol-1-yl]-essigsäure

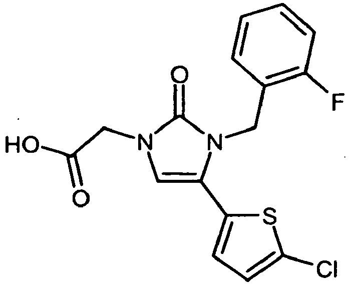

Analog der Vorschrift von Beispiel 129A werden aus 165 mg (0.418 mmol) der Verbindung aus Beispiel 237A 150 mg (99% d. Th.) der Zielverbindung erhalten.
LC/MS [Methode 7]: Rₜ = 2.01 min; MS [ESIpos]: m/z = 367 (M+H)^{+ 1}H-NMR (400 MHz, DMSO-d₆): δ = 4.40 (s, 2H), 4.97 (s, 2H), 6.82-6.95 (m, 2H), 6.98 (s, 1H), 7.04-7.22 (m, 3H), 7.25-7.36 (m, 1H).

### Beispiel 239A

### [4-(4-Chlorphenyl)-3-(cyclopropylmethyl)-2-oxo-2,3-dihydro-1H-imidazol-1-yl]-essigsäuremethylester

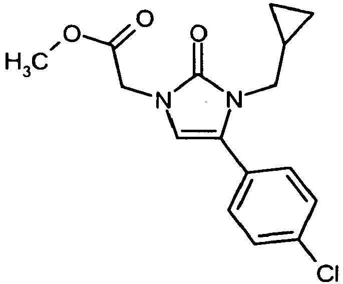

300 mg (1.13 mmol) der Verbindung aus Beispiel 232A werden zusammen mit 1.10 g (3.38 mmol) Cäsiumcarbonat in 12 ml Aceton vorgelegt und mit 456 mg (3.38 mmol) Brommethylcyclopropan versetzt. Man rührt 2 h bei 50°C. Das Reaktionsgemisch wird danach mit je 10 ml Essigsäureethylester und Wasser verdünnt und mit 1 N Salzsäure angesäuert. Die Phasen werden getrennt und die wässrige Phase wird noch einmal mit 10 ml Essigsäureethylester extrahiert. Die vereinigten organischen Phasen werden über Natriumsulfat getrocknet, filtriert und im Vakuum eingeengt. Weitere Reinigung erfolgt durch präparative HPLC [Methode 10]. Man erhält so 60 mg (17% d. Th.) der Zielverbindung.
LC/MS [Methode 8]: Rₜ = 2.28 min; MS [ESIpos]: m/z = 321 (M+H)⁺
¹H-NMR (400 MHz, DMSO-d₆): δ = 0.05 (m, 2H), 0.39 (m, 2H), 0.76 (m, 1H), 3.60 (d, 2H), 3.70 (s, 3H), 4.46 (s, 2H), 6.73 (s, 1H), 7.40-7.55 (m, 4H).

### Beispiel 240A

### [4-(4-Chlorphenyl)-3-(cyclopropylmethyl)-2-oxo-2,3-dihydro-1H-imidazol-1-yl]-essigsäure

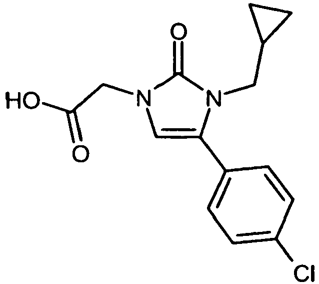

Analog der Vorschrift von Beispiel 129A werden ausgehend von 87 mg (0.271 mmol) [4-(4-Chlorphenyl)-3-(cyclopropylmethyl)-2-oxo-2,3-dihydro-1*H*-imidazol-1-yl]-essigsäuremethylester aus Beispiel 239A 84 mg (100% d. Th.) der Zielverbindung erhalten.
LC/MS [Methode 17]: Rₜ = 2.86 min; MS [ESIpos]: m/z = 307 (M+H)⁺
¹H-NMR (500 MHz, DMSO-d₆): δ = 0.05 (m, 2H), 0.28 (m, 2H), 0.65 (m, 1H), 3.60 (d, 2H), 4.33 (s, 2H), 6.73 (s, 1H), 7.40-7.55 (m, 4H), 13.03 (br. s, 1H).

### Beispiel 241A

### 2-[3-(4-Chlorphenyl)-5-oxo-4,5-dihydro-1H-1,2,4-triazol-1-yl]-N-{1-methyl-1-[3-(trifluormethyl)-phenyl]ethyl}-essigsäureamid

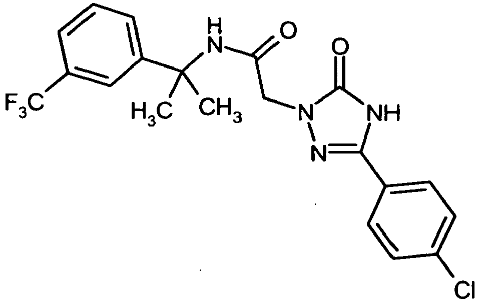

2.00 g (4.12 mmol) 2-[3-(4-Chlorphenyl)-4-allyl-5-oxo-4,5-dihydro-1*H*-1,2,4-triazol-1-yl]-*N*-{1-methyl-1-[3-(trifluormethyl)phenyl]ethyl}-essigsäureamid aus Beispiel 371 werden in 20 ml entgastem Dioxan gelöst und unter Argon mit 97 mg Tetrakis(triphenylphosphin)palladium(0) (0.084 mmol), 1.46 ml (10.44 mmol) Triethylamin sowie 0.32 ml (8.35 mmol) Ameisensäure versetzt und zwei Stunden bei 85°C gerührt. Man lässt die Suspension danach auf Raumtemperatur abkühlen, saugt die ausgefallenen Kristalle ab und wäscht mit Isopropanol nach. Die Mutterlauge wird im Vakuum eingeengt und mit Isopropanol versetzt, wobei weitere Kristalle ausfallen, die ebenfalls abgesaugt und mit Isopropanol gewaschen werden. Man erhält so zusammen 1.56 g (85% d. Th.) der Zielverbindung.
LC/MS [Methode 8]: Rₜ = 2.47 min; MS [ESIpos]: m/z = 439 (M+H)⁺
¹H-NMR (400 MHz, DMSO-d₆): δ = 1.60 (s, 6H), 4.61 (s, 2H), 7.50-7.70 (m, 6H), 7.78 (d, 2H), 8.55 (s, 1H), 12.27 (s, 1H).

Auf analoge Weise werden die folgenden Verbindungen erhalten:

| **Beispiel Nr.** | **Struktur** | **LC/MS oder HPLC, MS Rₜ [Methode]** |
|---|---|---|
| **242A** | 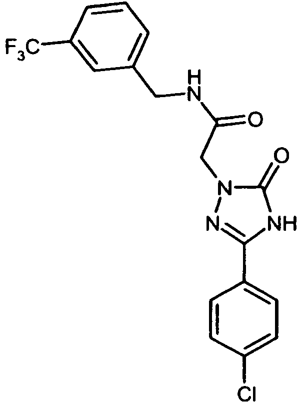 | LC/MS: Rₜ = 2.09 min [7] |
| | | [ESIpos]: m/z = 411 (M+H)⁺ |
| **243A** | 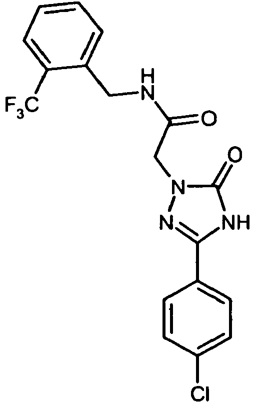 | LC/MS: Rₜ = 2.06 min [7] |
| | | [ESIpos]: m/z = 411 (M+H)⁺ |
| **244A** | 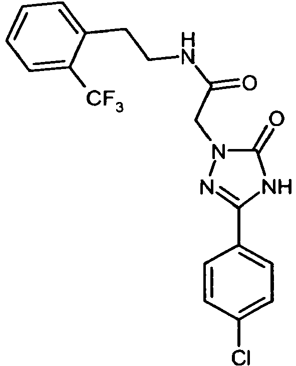 | LC/MS: Rₜ = 2.37 min [8] |
| | | [ESIpos]: m/z = 425 (M+H)⁺ |
| **245A** | 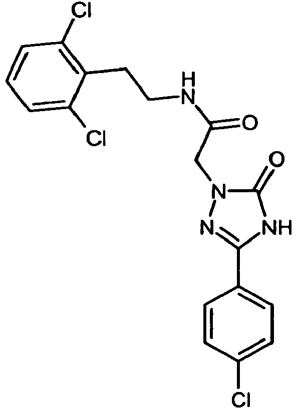 | LC/MS: Rₜ = 2.37 min [8] |
| | | [ESIpos]: m/z = 424/426 (M+H)⁺ |

### Beispiel 246A

### 2-[(3-Chlor-4-methyl-2-thienyl)carbonyl]-N-isobutylhydrazincarboxamid

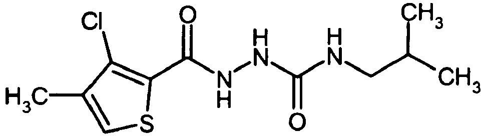

1.00 g (5.25 mmol) 3-Chlor-4-methylthien-2-ylcarbonsäurehydrazid werden bei Raumtemperatur in 10 ml THF vorgelegt. Unter Rühren werden 520 mg (5.25 mmol) Isobutylisocyanat, gelöst in 2 ml THF, zügig zugetropft. Man lässt das Gemisch über Nacht bei Raumtemperatur weiter rühren. Zur Aufarbeitung versetzt man das Reaktionsgemisch mit 10 ml Diethylether, kühlt im Wasser/EisBad auf etwa 0°C, gewinnt den gebildeten Niederschlag durch Filtration, wäscht mit Diethylether nach und trocknet im Vakuum. Man erhält so 1.29 g (85% d. Th.) der Zielverbindung.
LC/MS [Methode 7]: Rₜ = 1.63 min
¹H-NMR (400 MHz, DMSO-d₆): δ = 0.84 (d, 6H), 1.59-1.73 (m, 1H), 2.18 (s, 3H), 2.86 (t, 2H), 6.37 (t, 1H), 7.60 (s, 1H), 7.92 (s, 1H), 9.70 (s, 1H).

### Beispiel 247A

### 5-(3-Chlor-4-methyl-2-thienyl)-4-isobutyl-2,4-dihydro-3H-1,2,4-triazol-3-on

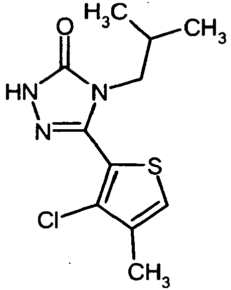

Eine Suspension von 1.28 g (4.42 mmol) 2-[(3-Chlor-4-methyl-2-thienyl)carbonyl]-N-isobutyl-hydrazincarboxamid aus Beispiel 246A in 12 ml 3 N Natronlauge wird zunächst über Nacht unter Rückfluss erhitzt. Nach dem Abkühlen wird filtriert. Das Filtrat enthält verunreinigtes Produkt, während der abfiltrierte Feststoff hauptsächlich dem Edukt entspricht. Dieser Feststoff wird in ca. 15 ml 3 N ethanolischer Natronlauge wieder aufgenommen und erneut über Nacht unter Rückfluss erhitzt. Nach Neutralisation mit 1 N Salzsäure und Einengen wird der Rückstand gemeinsam mit dem eingeengten Filtrat aus der wässrigen Umsetzung durch präparative HPLC [Methode 12] aufgereinigt. Nach Einengen und Trocknen der Produktfraktionen erhält man so 562 mg (47% d. Th.) der Zielverbindung.
LC/MS [Methode 8]: Rₜ = 2.13 min.

### Beispiel 248A

### 2-[3-(3-Chlor-4-methyl-2-thienyl)-4-isobutyl-5-oxo-4,5-dihydro-1H-1,2,4-triazol-1-yl]-essigsäureethylester

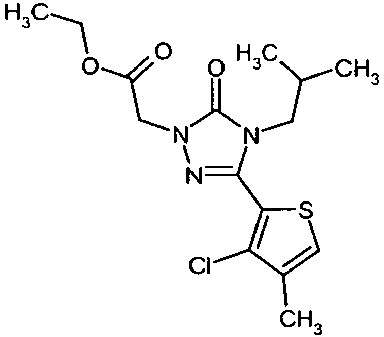

Zu einer Suspension von 560 mg (2.06 mmol) 5-(3-Chlor-4-methyl-2-thienyl)-4-isobutyl-2,4-dihydro-3H-1,2,4-triazol-3-on aus Beispiel 247A und 253 mg (2.06 mmol) Chloressigsäureethylester in 10 ml Acetonitril gibt man 570 mg (4.12 mmol) Kaliumcarbonat und erhitzt für 4 h unter Rückfluss. Zur Aufarbeitung wird eingeengt, der Rückstand mit Wasser aufgenommen und dreimal mit Essigsäureethylester extrahiert. Die vereinigten organischen Phasen werden eingeengt, und das als Rückstand verbleibende Rohprodukt wird durch präparative HPLC [Methode 12] gereinigt. Man erhält so 705 mg (96% d. Th.) der Zielverbindung.
LC/MS [Methode 5]: Rₜ = 2.49 min.

### Beispiel 249A

### 2-[3-(3-Chlor-4-methyl-2-thienyl)-4-isobutyl-5-oxo-4,5-dihydro-1H-1,2,4-triazol-1-yl]-essigsäure

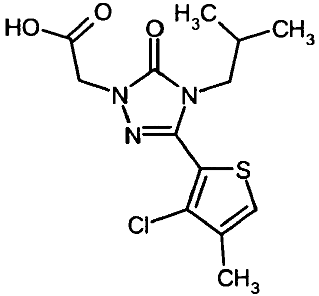

700 mg (1.96 mmol) 2-[3-(3-Chlor-4-methyl-2-thienyl)-4-isobutyl-5-oxo-4,5-dihydro-1*H*-1,2,4-triazol-1-yl]-essigsäureethylester aus Beispiel 248A werden in 10 ml Methanol vorgelegt und mit 1 ml 20%-iger Kalilauge versetzt. Man rührt über Nacht bei Raumtemperatur, stellt das Reaktionsgemisch dann mit 1 N Salzsäure auf pH 6 und reinigt direkt über präparative HPLC [Methode 12]. Man erhält so 555 mg (86% d. Th.) der Zielverbindung.
LC/MS [Methode 8]: Rₜ = 2.17 min;
¹H-NMR (400 MHz, DMSO-d₆): δ = 0.72 (d, 6H), 1.71-1.86 (m, 1H), 2.23 (s, 3H), 3.45 (d, 2H), 4.56 (s, 2H), 7.72 (s, 1H), 13.15 (br. s, 1H).

### Beispiel 250A

### 2-[3-(4-Chlorphenyl)-5-oxo-4,5-dihydro-1H-1,2,4-triazol-1-yl]-N-[3-trifluormethyl)phenylmethyl]-essigsäureamid

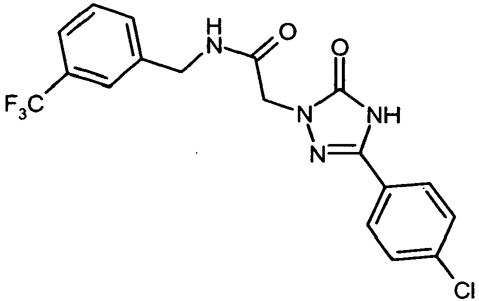

Zu 780 mg (1.47 mmol) 2-[3-(4-Chlorphenyl)-4-(4-methoxyphenylmethyl)-5-oxo-4,5-dihydro-1*H* 1,2,4-triazol-1-yl]-*N*-[3-(trifluormethyl)phenylmethyl]-essigsäureamid aus Beispiel 153 in 10 ml Trifluoressigsäure gibt man 31.8 mg (0.294 mmol) Anisol und rührt für 72 h unter Rückfluss. Zur Aufarbeitung wird das Reaktionsgemisch nach Abkühlen auf Wasser gegeben und die Mischung mit Essigsäureethylester extrahiert. Die organische Phase wird eingeengt, der Rückstand in Methanol aufgenommen und über präparative HPLC [Methode 12] gereinigt. Man erhält auf diese Weise 360 mg (60% d. Th.) der Zielverbindung.
LC/MS [Methode 5]: Rₜ = 2.22 min;
¹H-NMR (400 MHz, DMSO-d₆): δ = 4.40 (d, 2H), 4.45 (s, 2H), 7.54-7.65 (m, 6H), 7.79, 7.81 (BB'-Teil eines AABB'-Systems, 2H), 8.70 (t, 1H), 12.35 (s, 1H).

### Beispiel 251A

### 3-(Nitromethyl)-3-[3-(trifluormethyl)phenyl]oxetan

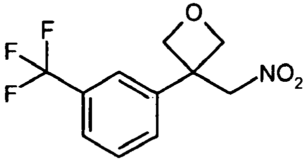

Eine Lösung von 103 mg 1-Brom-3-(trifluormethyl)benzol (0.46 mmol) in 4 ml wasserfreiem THF wird bei -78°C langsam mit einer n-Butyllithium-Lösung (1.6 M in Hexan, 312 µl, 0.50 mmol) versetzt. Nach 15 min Rühren bei -78°C wird eine Lösung von 50 mg (0.43 mmol) 3-Nitromethylenoxetan [Herstellung: G. Wuitschik et al., Angew. Chem. Int. Ed. 45 (46), 7736-7739 (2006)] in 2 ml THF zugegeben. Die Mischung wird über Nacht bei -78°C gerührt und dann die Reaktion durch Zugabe von 5 ml gesättigter Ammoniumchlorid-Lösung bei -78°C gestoppt. Nach Aufwärmen auf RT wird die Mischung mit Wasser verdünnt und dreimal mit Dichlormethan extrahiert. Die vereinigten organischen Phasen werden über Natriumsulfat getrocknet und am Rotationsverdampfer von den flüchtigen Komponenten befreit. Der ölige Rückstand wird kurz im Hochvakuum getrocknet. Man erhält 53 mg (33% d. Th) der Titelverbindung in ca. 70% Reinheit.
GC/MS [Methode 21]: Rₜ = 5.44 min; m/z = 201 [M-CH₂NO₂]⁺
¹H-NMR (400 MHz, CDCl₃): δ = 4.95 (d, 2H), 5.07 (s, 2H), 5.09 (d, 2H), 7.31 (d, 1H), 7.36 (s, 1H), 7.53 (t, 1H), 7.60 (d, 1H).

### Beispiel 252A

### 1-{3-[3-(Trifluormethyl)phenyl]oxetan-3-yl}-methanamin-Hydrochlorid

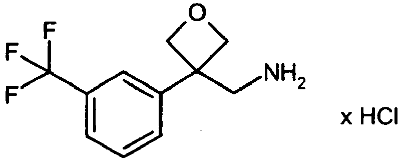

50 mg der Verbindung aus Beispiel 251A (0.134 mmol) werden in 2 ml Ethanol in Gegenwart von 15 mg (0.11 mmol) Palladiumhydroxid (20%-ig auf Kohle) unter 1 atm Wasserstoff bei RT über Nacht hydriert. Der Katalysator wird danach abfiltriert, und das Filtrat wird mit Wasser verdünnt, mit 1 N Salzsäure auf pH 1 gestellt und zweimal mit Dichlormethan gewaschen. Die wässrige Phase wird mit 2 N Natronlauge auf pH 13 gestellt und dreimal mit Dichlormethan extrahiert. Die letzteren organischen Phasen werden vereinigt, über Natriumsulfat getrocknet und filtriert. Das Filtrat wird mit 200 µl einer 4 N Lösung von Chlorwasserstoff in Dioxan versetzt und am Rotationsverdampfer eingeengt. Der Rückstand wird im Hochvakuum getrocknet. Man erhält 20 mg der Titelverbindung, die als Rohprodukt (Reinheit ca. 60%) weiter eingesetzt wird.
LC/MS [Methode 22]: Rₜ = 0.47 min; m/z = 231 [M+H]⁺.

### Ausführungsbeispiele:

### Beispiel 1

### 2-[3-(4-Chlorphenyl)-4-cyclopropyl-5-oxo-4,5-dihydro-1H-1,2,4-triazol-1-yl]-N-[3-(trifluormethyl)phenylmethyl]-essigsäureamid

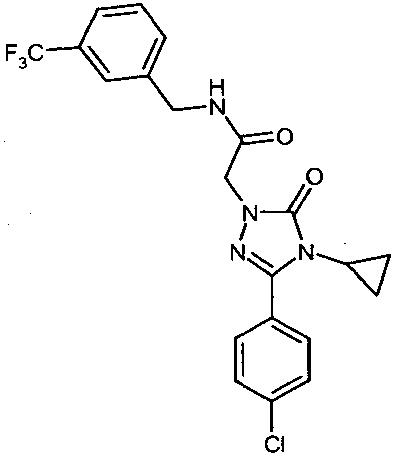

50.0 mg (0.170 mmol) [3-(4-Chlorphenyl)-4-cyclopropyl-5-oxo-4,5-dihydro-1*H*-1,2,4-triazol-1-yl]-essigsäure aus Beispiel 88A und 32.8 mg (0.187 mmol) 3-Trifluormethylbenzylamin werden in 2 ml Dimethylformamid vorgelegt und mit 27.6 mg (0.204 mmol) HOBt versetzt. Nach 10 min Rühren werden 42.4 mg (0.221 mmol) EDC-Hydrochlorid zugegeben und das Gemisch über Nacht bei Raumtemperatur gerührt. Zur Aufarbeitung wird das Reaktionsgemisch zwischen Dichlormethan und Wasser verteilt, die organische Phase abgetrennt, über Natriumsulfat getrocknet und eingeengt. Der Rückstand wird durch Flash-Chromatographie an Kieselgel (Eluent: Dichlormethan/Methanol zunächst 200:1, dann 100:1) gereinigt und ergibt so 76 mg (99% d. Th.) der Zielverbindung.
MS [ESIpos]: m/z = 451 (M+H)⁺
HPLC [Methode 1]: Rₜ = 4.74 min
¹H-NMR (400 MHz, DMSO-d₆): δ = 0.59 (m, 2H), 0.90 (m, 2H), 3.18 (tt, 1H), 4.40 (d, 2H), 4.44 (s, 2H), 7.53-7.66 (m, 6H), 7.80 (d, 2H), 8.67 (t, 1H).

Auf analoge Weise werden die folgenden Verbindungen hergestellt:

| **Beispiel Nr.** | **Struktur** | **LC/MS oder HPLC, MS Rₜ [Methode]** | **¹H-NMR (400 MHz, DMSO-d₆)** |
|---|---|---|---|
| **2** | 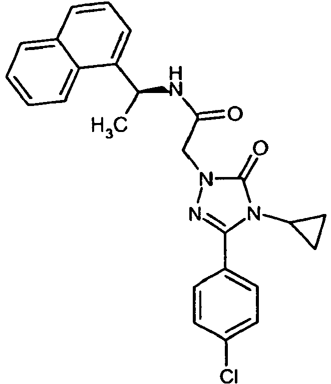 | MS [ESIpos]: m/z = 447 (M+H)⁺; Rₜ = 4.73 min [2] | δ = 0.56 (m, 2H), 0.89 (m, 2H), 1.51 (d, 3H), 3.16 (tt, 1 H), 4.42 (s, 2H), 5.71 (dq, 1H), 7.46-7.56 (m, 4H), 7.58 (d, 2H), 7.78 (d, 2H), 7.83 (d, 1H), 7.94 (d, 1H), 8.09 (d, 1H), 8.75 (d, 1H). |
| **3** | 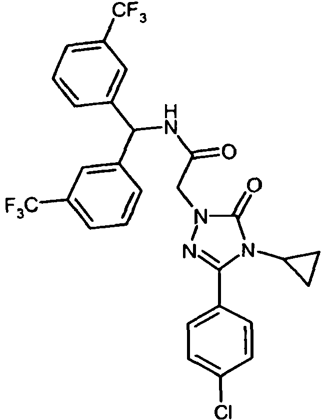 | MS [ESIpos]: m/z = 595 (M+H)⁺; Rₜ = 5.16 min [1] | δ = 0.57 (m, 2H), 0.89 (m, 2H), 3.17 (tt, 1H), 4.46 (s, 2H), 6.38 (d, 1H), 7.56-7.69 (m, 8H), 7.73 (br. s, 2H), 7.78 (d, 2H), 9.28 (d, 1H). |
| **4** | 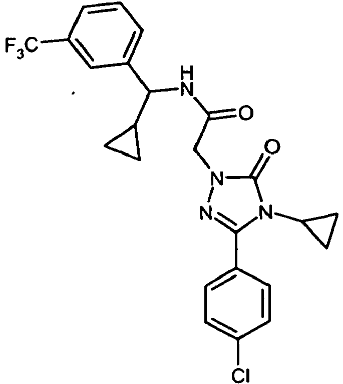 | MS [ESIpos]: m/z = 491 (M+H)⁺; Rₜ = 4.86 min [1] | δ = 0.32-0.60 (m, 6H), 0.89 (m, 2H), 1.17 (m, 1 H), 3.17 (tt, 1H), 4.30 (m, 1H), 4.45 (s, 2H), 7.54-7.73 (m, 6H), 7.79 (d, 2H), 8.84 (d, 1H). |

| **Beispiel Nr.** | **Struktur** | **LC/MS oder HPLC, MS R, [Methode]** | **¹H-NMR (400 MHz, DMSO-d₆)** |
|---|---|---|---|
| **5** | 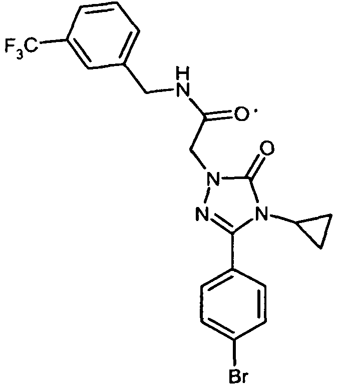 | MS [ESIpos]: m/z =495/497 (M+H)⁺; Rₜ = 4.69 min [1] | δ = 0.60 (m, 2H), 0.90 (m, 2H), 3.17 (tt, 1H), 4.41 (d, 2H), 4.45 (s, 2H), 7.53-7.64 (m, 4H), 7.73 (br. s, 4H), 8.66 (t, 1H). |
| **6** | 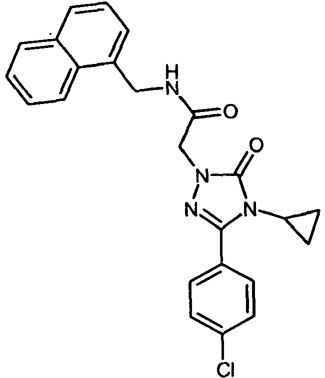 | MS [ESIpos]: m/z = 433 (M+H)⁺; Rₜ = 4.60 min [2] | δ = 0.60 (m, 2H), 0.91 (m, 2H), 3.18 (tt, 1H), 4.45 (s, 2H), 4.78 (d, 2H), 7.44-7.50 (m, 2H), 7.52-7.58 (m, 2H), 7.61 (d, 2H), 7.82 (d, 2H), 7.86 (m, 1H), 7.96 (m, 1H), 8.07 (m, 1H), 8.63 (t, 1H). |
| **7** | 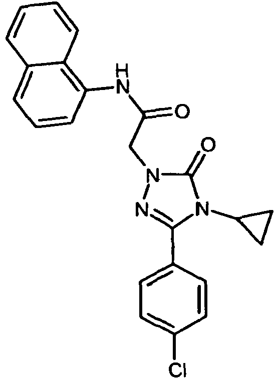 | MS [ESIpos]: m/z = 419 (M+H)⁺; Rₜ = 4.52 min [2] | δ = 0.62 (m, 2H), 0.92 (m, 2H), 3.21 (tt, 1H), 4.78 (s, 2H), 7.50 (t, 1H), 7.56 (t, 2H), 7.62 (d, 2H), 7.67 (d, 1H), 7.80 (d, 1H), 7.86 (d, 2H), 7.95 (t, 1H), 8.13 (m, 1H), 10.14 (s, 1H). |
| **8** | 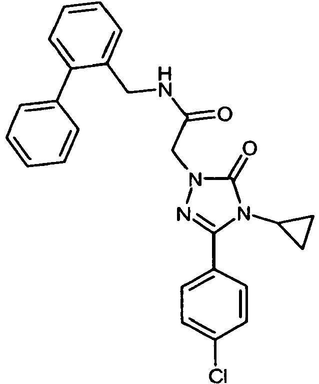 | MS (CIpos): m/z = 476 (M+NH₄)⁺, 459 (M+H)⁺; Rₜ = 4.79 min [2] | δ = 0.58 (m, 2H), 0.90 (m, 2H), 3.17 (tt, 1 H), 4.22 (d, 2H), 4.40 (s, 2H), 7.22 (m, 1H), 7.31-7.48 (m, 8H), 7.60 (d, 2H), 7.81 (d, 2H), 8.49 (t, 1H). |
| **9** | 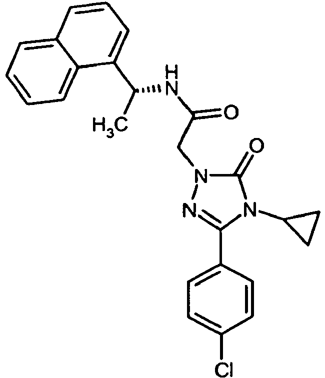 | MS [ESIpos]: m/z = 447 (M+H)⁺; Rₜ = 4.52 min [1] | δ = 0.57 (m, 2H), 0.89 (m, 2H), 1.52 (d, 3H), 3.16 (tt, 1H), 4.43 (s, 2H), 5.71 (dq, 1H), 7.46-7.61 (m, 6H), 7.78 (d, 2H), 7.84 (d, 1H), 7.94 (d, 1H), 8.09 (br. d, 1H), 8.75 (d, 1H). |
| **10** | 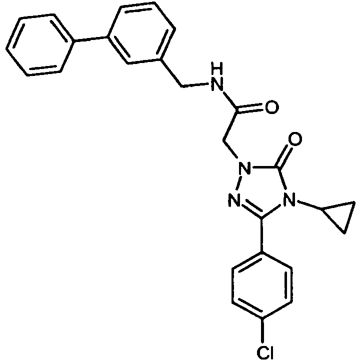 | MS [ESIpos]: m/z = 459 (M+H)⁺ | δ = 0.60 (m, 2H), 0.90 (m, 2H), 3.17 (tt, 1H), 4.39 (d, 2H), 4.45 (s, 2H), 7.27 (br. d, 1H), 7.33-7.47 (m, 4H), 7.52-7.60 (m, 4H), 7.65 (d, 2H), 7.80 (d, 2H), 8.61 (t, 1H). |
| **11** | 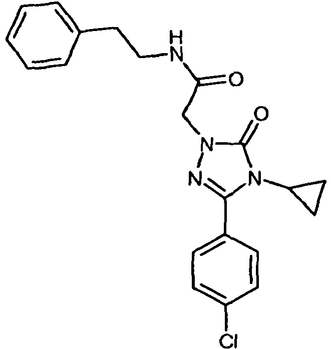 | Rₜ = 2.28 min [5] | δ = 0.59 (m, 2H), 0.91 (m, 2H), 2.72 (t, 2H), 3.18 (tt, 1H), 4.33 (s, 2H), 7.17-7.31 (m, 5H), 7.60 (d, 2H), 7.81 (d, 2H), 8.14 (t, 1H). |
| **12** | 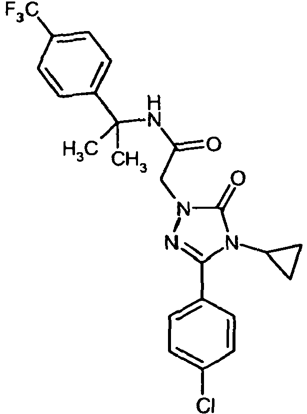 | MS [ESIpos]: m/z = 479 (M+H)⁺; Rₜ = 4.85 min [1] | δ = 0.55 (m, 2H), 0.88 (m, 2H), 1.57 (s, 6H), 3.15 (tt, 1H), 4.43 (s, 2H), 7.53-7.64 (m, 6H), 7.78 (d, 2H), 8.55 (s, 1H). |
| **13** | 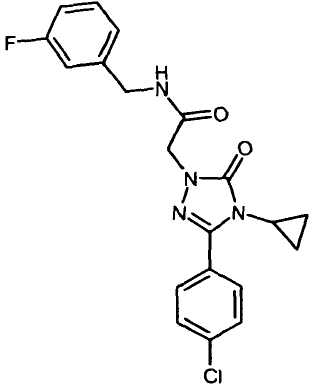 | MS [ESIpos]: m/z = 401 (M+H)⁺ | δ = 0.60 (m, 2H), 0.90 (m, 2H), 3.18 (tt, 1H), 4.33 (d, 2H), 4.45 (s, 2H), 7.03-7.14 (m, 3H), 7.36 (dd, 1H), 7.60 (d, 2H), 7.82 (d, 2H), 8.61 (t, 1H). |

| **Beispiel Nr.** | **Struktur** | **LC/MS oder HPLC, MS Rₜ [Methode]** | **¹H-NMR (400 MHz, DMSO-d₆)** |
|---|---|---|---|
| **14** | 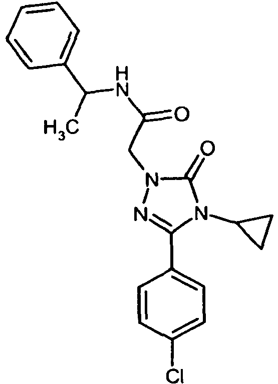 | MS [ESIpos]: m/z = 397 (M+H)⁺ | δ = 0.57 (m, 2H), 0.89 (m, 2H), 1.37 (d, 3H), 3.17 (tt, 1H), 4.41 (s, 2H), 4.91 (dq, 1H), 7.20-7.35 (m, 5H), 7.59 (d, 2H), 7.80 (d, 2H), 8.58 (d, 1H). |
| **15** | 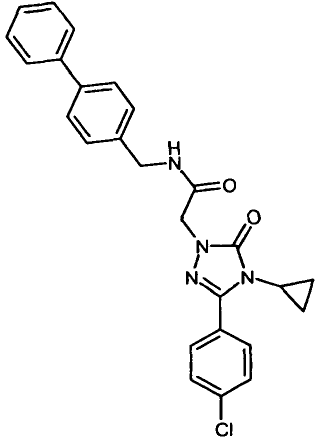 | MS [CIpos]: m/z = 476 (M+NH₄)⁺, 459 (M+H)⁺; Rₜ = 4.79 min [2] | δ = 0.60 (m, 2H), 0.91 (m, 2H), 3.18 (tt, 1H), 4.35 (d, 2H), 4.44 (s, 2H), 7.33-7.49 (m, 5H), 7.58-7.68 (m, 6H), 7.82 (d, 2H), 8.60 (t, 1H). |
| **16** | 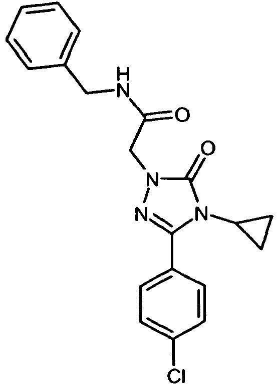 | Rₜ = 2.20 min [5] | δ = 0.59 (m, 2H), 0.90 (m, 2H), 3.18 (tt, 1H), 4.31 (d, 2H), 4.43 (s, 2H), 7.21-7.36 (m, 5H), 7.60 (d, 2H), 7.81 (d, 2H), 8.56 (t, 1H). |
| **17** | 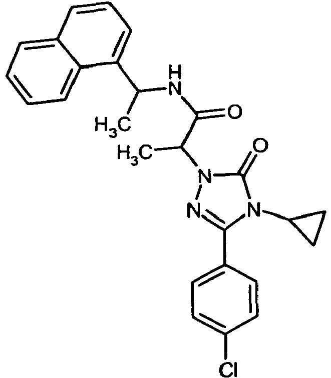 | MS [CIpos]: m/z = 478 (M+NH₄)⁺, 461 (M+H)⁺; Rₜ = 4.88 min [2] | |
| **18** | 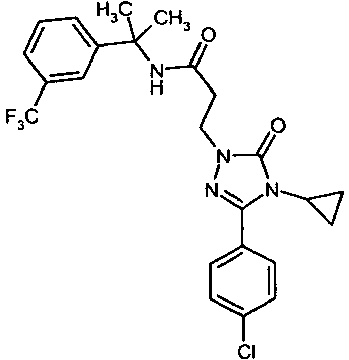 | MS [ESIpos]: m/z = 493 (M+H)⁺; Rₜ = 4.80 min [1] | δ = 0.56 (m, 2H), 0.89 (m, 2H), 1.52 (s, 6H), 2.59 (t, 2H), 3.15 (tt, 1H), 3.87 (t, 2H), 7.43 (t, 1H), 7.49-7.64 (m, 5H), 7.81 (d, 2H), 8.32 (t, 1H). |
| **19** | 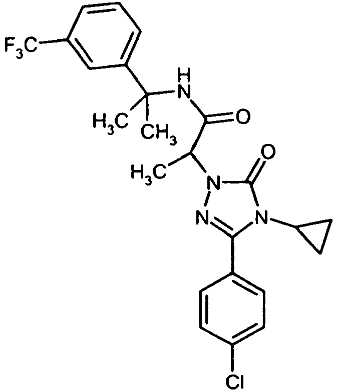 | MS [ESIpos]: m/z = 493 (M+H)⁺; Rₜ = 4.96 min [1] | δ = 0.56 (m, 2H), 0.89 (m, 2H), 1.53 (d, 3H), 1.56 (s, 3H), 1.58 (s, 3H), 3.15 (tt, 1H), 4.81 (q, 1H), 7.47-7.60 (m, 5H), 7.64 (br. d, 1H), 7.78 (d, 2H), 8.43 (s, 1H). |
| **20** | 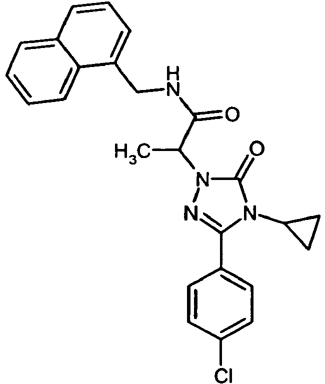 | MS [CIpos]: m/z = 464 (M+NH₄)⁺, 447 (M+H)⁺; Rₜ = 4.77 min [2] | δ = 0.58 (m, 2H), 0.89 (m, 2H), 1.55 (d, 3H), 3.16 (tt, 1H), 4.73 (dd, 1H), 4.79 (dd, 1H), 4.82 (q, 1H), 7.42-7.48 (m, 2H), 7.50-7.56 (m, 2H), 7.60 (d, 2H), 7.80 (d, 2H), 7.84 (m, 1H), 7.94 (m, 1H), 8.04 (m, 1H), 8.44 (t, 1H). |
| **21** | 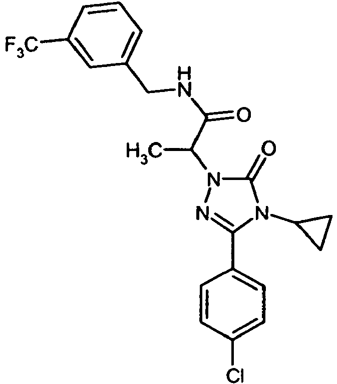 | MS [CIpos]: m/z = 482 (M+NH₄)⁺, 465 (M+H)⁺; Rₜ = 4.78 min [2] | δ = 0.61 (m, 2H), 0.90 (m, 2H), 1.53 (d, 3H), 3.16 (tt, 1H), 4.34 (dd, 1H), 4.44 (dd, 1H), 4.80 (q, 1H), 7.51-7.63 (m, 6H), 7.80 (d, 2H), 8.48 (s, 1H). |
| **22** | 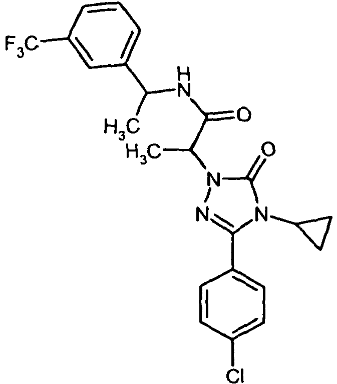 | MS [ES1pos]: m/z = 479 (M+H)⁺; Rₜ = 4.87 min [1] | |
| **23** | 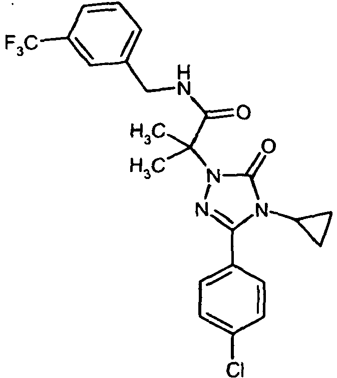 | MS [ESIpos]: -m/z = 479 (M+H)⁺; Rₜ = 4.89 min [2] | δ = 0.56 (m, 2H), 0.87 (m, 2H), 1.64 (s, 6H), 3.13 (tt, 1 H), 4.37 (d, 2H), 7.50-7.67 (m, 6H), 7.82 (d, 2H), 8.31 (s, 1H). |
| **24** | 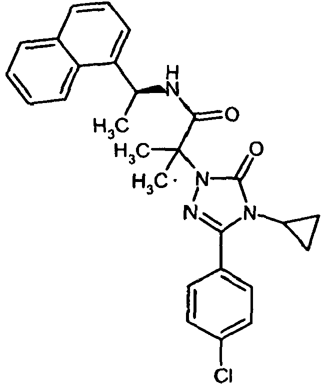 | MS [ESIpos]: m/z = 475 (M+H)⁺; Rₜ = 4.99 min [2] | δ = 0.55 (m, 2H), 0.89 (m, 2H), 1.49 (d, 3H), 1.62 (d, 6H), 3.13 (tt, 1H), 5.71 (dq, 1H), 7.42-7.61 (m, 6H), 7.80 (m, 3H), 7.93 (m, 1H), 8.08 (br. d, 8.17 (d, 1H). |
| **25** | 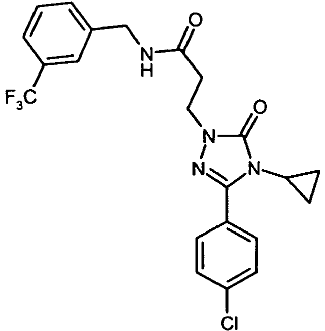 | MS [ESIpos]: m/z = 465 (M+H)⁺; Rₜ = 4.55 min [1] | δ=0.56 (m, 2H), 0.88 (m, 2H), 2.62 (t, 2H), 3.13 (tt, 1H), 3.95 (t, 2H), 4.35 (d, 2H), 7.43-7.61 (m, 6H), 7.77 (d, 2H), 8.57 (t, 1H). |
| **26** | 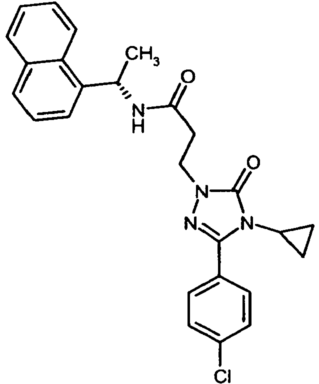 | MS [ESIpos]: m/z = 461 (M+H)⁺; Rₜ = 4.63 min [1] | δ = 0.54 (m, 2H), 0.87 (m, 2H), 1.45 (d, 3H), 2.60 (t, 2H), 3.12 (tt, 1H), 3.92 (m, 2H), 5.69 (dq, 1H), 7.39 (t, 1H), 7.47-7.55 (m, 3H), 7.58 (d, 2H), 7.75 (d, 2H), 7.80 (d, 1H), 7.93 (m, 1H), 8.07 (m, 1H), 8.56 (d, 1H). |

### Beispiel 27

### 2-[3-(4-Chlorphenyl)-4-cyclopropyl-5-oxo-4,5-dihydro-1H-1,2,4-triazol-1-yl]-N-{1-[3-(trifluormethyl)phenyl]ethyl}essigsäureamid

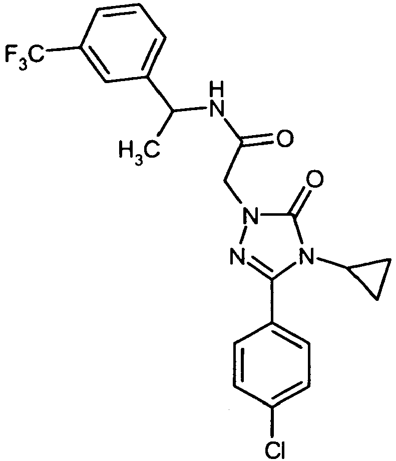

40.0 mg (0.136 mmol) [3-(4-Chlorphenyl)-4-cyclopropyl-5-oxo-4,5-dihydro-1*H*-1,2,4-triazol-1-yl]-essigsäure aus Beispiel 88A, 28.3 mg (0.150 mmol) 1-[(3-Trifluormethyl)phenyl]ethylamin und 22.1 mg (0.163 mmol) HOBt werden in 1 ml Dimethylformamid vorgelegt und mit 33.9 mg (0.177 mmol) EDC-Hydrochlorid versetzt. Das Gemisch wird über Nacht bei Raumtemperatur gerührt und dann zur Aufarbeitung mit 15 ml Wasser versetzt. Der entstandene Niederschlag wird durch Filtrieren isoliert und anschliessend durch präparative HPLC [Methode 9] gereinigt. Man erhält so 20 mg (32% d. Th.) der Zielverbindung.
LC/MS [Methode 7]: Rₜ = 2.34 min
¹H-NMR (400 MHz, DMSO-d₆): δ = 0.50 - 0.64 (m, 2H), 0.82 - 0.96 (m, 2H), 1.39.(d, 3H), 3.17 (dddd, 1H), 4.42 (s, 2H), 5.00 (dq, 1H), 7.52 - 7.69 (m, 4H), 7.57, 7.60 (AA'-Teil eines AA'BB'-Systems, 2H), 7.78, 7.80 (BB'-Teil eines AA'BB'-Systems, 2H), 8.71 (d, 1H).

Auf analoge Weise werden die folgenden Verbindungen hergestellt:

| **Beispiel Nr.** | **Struktur** | **LC/MS Rₜ [Methode]** | **¹H-NMR (400 MHz, DMSO-d₆)** |
|---|---|---|---|
| **28** | 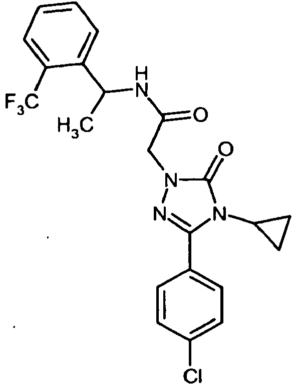 | Rₜ = 2.48 min [5] | δ = 0.49 - 0.62 (m, 2H), 0.81 - 0.96 (m, 2H), 1.36 (d, 3H), 3.16 (dddd, 1H), 4.41 (Zentrum eines AB-Systems, 2H), 5.20 (dq, 1H), 7.45 (t, 1H), 7.57, 7.59 (AA'-Teil eines AABB'-Systems, 2H), 7.64 - 7.73 (m, 3H), 7.77, 7.79 (BB'-Teil eines AA'BB'-Systems, 2H), 8.83 (d, 1H). |
| **29** | 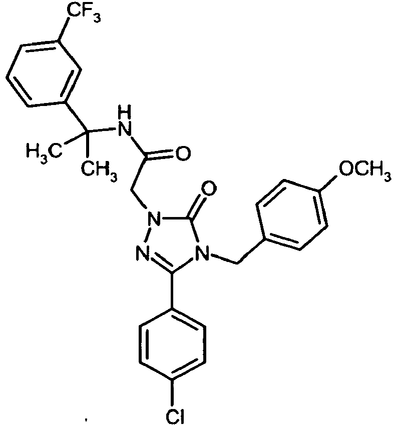 | Rₜ = 2.68 min [7] | δ = 1.60 (s, 6H), 3.68 (s, 3H), 4.52 (s, 2H), 4.89 (s, 2H), 6.80, 6.82 (AA'-Teil eines AABB'-Systems, 2H), 6.96, 6.98 (BB'-Teil eines AABB'-Systems, 2H), 7.49 - 7.57 (m, 6H), 7.63 (s, 1H), 7.68 (d, 1H), 8.59 (s, 1H). |
| **30** | 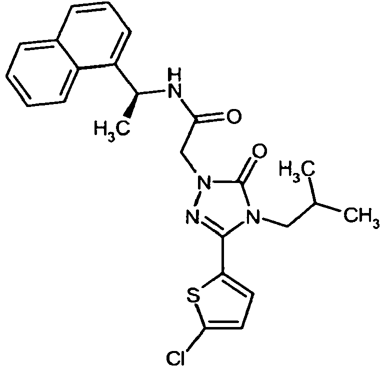 | Rₜ = 2.62 min [7] | δ = 0.81 (d, 6H), 1.51 (d, 3H), 1.87 (m, 1H), 3.67 (d, 2H), 4.47 (s, 2H), 5.70 (dq, 1H), 7.26 (d, 1H), 7.45 -7.60 (m, 5H), 7.84 (d, 1H), 7.91 - 7.97 (m, 1H), 8.09 (d, 1H), 8.80 (d, 1H). |
| **31** | 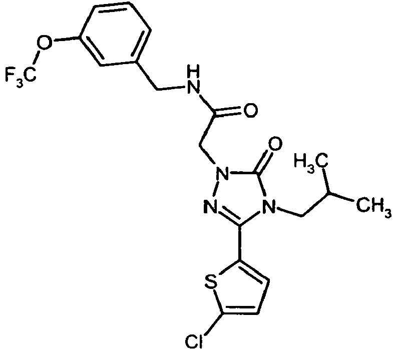 | Rₜ = 2.58 min [7] | δ = 0.83 (d, 6H), 1.90 (m, 1H), 3.69 (d, 2H), 4.36 (d, 2H), 4.49 (s, 2H), 7.21 -7.34 (m, 4H), 7.43 - 7.50 (m, 2H), 8.70 (t, 1H). |
| **32** | 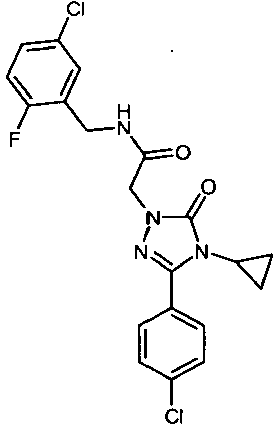 | Rₜ = 2.36 min [5] | δ = 0.53 - 0.67 (m, 2H), 0.83 - 0.97 (m, 2H), 3.18 (dddd, 1H), 4.33 (d, 2H), 4.46 (s, 2H), 7.21 - 7.28 (m, 1H), 7.34 - 7.40 (m, 2H), 7.59, 7.61 (AA'-Teil eines AA'BB'-Systems, 2H), 7.81, 7.83 (BB'-Teil eines AA'BB'-Systems, 2H), 8.62 (t, 1H). |
| **33** | 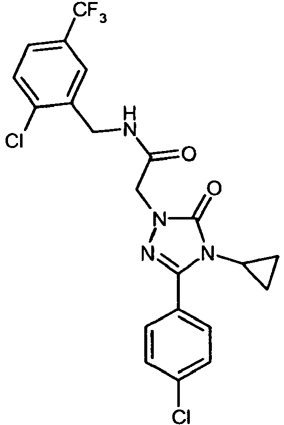 | Rₜ = 2.53 min [5] | δ = 0.56 - 0.63 (m, 2H), 0.83 - 0.97 (m, 2H), 3.18 (dddd, 1H), 4.44 (d, 2H), 4.49 (s, 2H), 7.58, 7.60 (AA'-Teil eines AA'BB'-Systems, 2H), 7.65 - 7.73 (m, 3H), 7.80, 7.82 (BB'-Teil eines AA'BB'-Systems, 2H), 8.71 (t, 1H). |
| **34** | 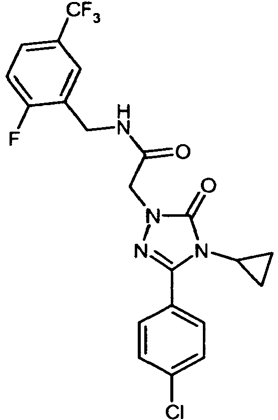 | Rₜ = 2.44 min [5] | δ = 0.53 - 0.66 (m, 2H), 0.83 - 0.97 (m, 2H), 3.17 (dddd, 1H), 4.41 (d, 2H), 4.45 (s, 2H), 7.44 (t, 1H), 7.58, 7.60 (AA'-Teil eines AA'BB'-Systems, 2H), 7.68 - 7.75 (m, 2H), 7.79, 7.81 (BB'-Teil eines AA'BB'-Systems, 2H), 8.67 (t, 1H). |
| **35** | 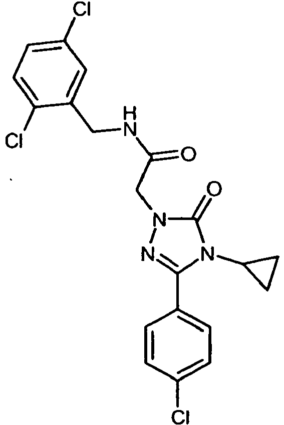 | Rₜ = 2.46 min [5] | δ = 0.54 - 0.67 (m, 2H), 0.84 - 0.98 (m, 2H), 3.18 (dddd, 1H), 4.36 (d, 2H), 4.49 (s, 2H), 7.35 - 7.43 (m, 2H), 7.49 (d, 1H), 7.59, 7.61 (AA'-Teil eines AA'BB'-Systems, 2H), 7.82, 7.84 (BB'-Teil eines AA'BB'-Systems, 2H), 8.65 (t, 1H). |
| **36** | 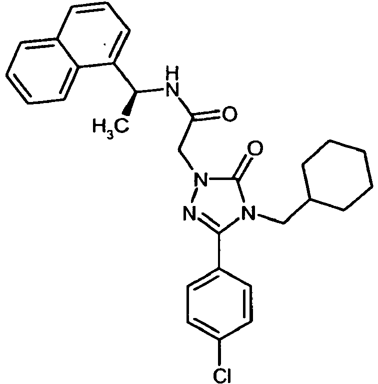 | Rₜ = 2.99 min [5] | δ = 0.66 - 0.79 (m, 2H), 0.92 - 1.09 (m, 3H), 1.34 -1.45 (m, 3H), 1.46 - 1.57 (m, 3H), 1.51 (d, 3H), 3.62 (d, 2H), 4.47 (s, 2H), 5.70 (dq, 1H), 7.46 - 7.67 (m, 8H), 7.84 (d, 1H), 7.92 -7.97 (m, 1H), 8.07 - 8.12 (m, 1H), 8.78 (d, 1H). |
| **37** | 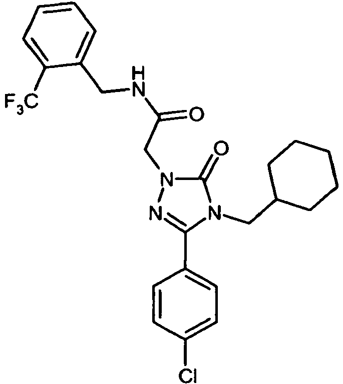 | Rₜ = 2.91 min [5] | δ = 0.68 - 0.82 (m, 2H), 0.94 - 1.10 (m, 3H), 1.36 -1.62 (m, 6H), 3.63 (d, 2H), 4.49 (d, 2H), 4.54 (s, 2H), 7.48 (t, 1H), 7.56 (d, 1H), 7.60 - 7.74 (m, 6H), 8.70 (t, 1H). |
| **38** | 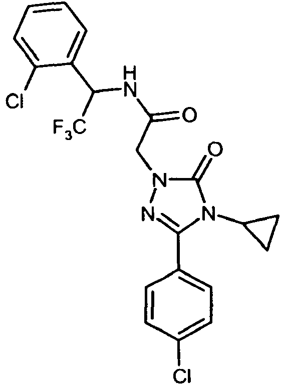 | Rₜ = 2.68 min [4] | δ = 0.49 - 0.64 (m, 2H), 0.81 - 0.96 (m, 2H), 3.18 (dddd, 1H), 4.57 (Zentrum eines AB-Systems, 2H), 6.17 - 6.29 (m, 1H), 7.44 -7.54 (m, 2H), 7.54 - 7.62 (m, 1H), 7.57, 7.59 (AA'-Teil eines AA'BB'-Systems, 2H), 7.74 (d, 1H), 7.77, 7.80 (BB'-Teil eines AA'BB'-Systems, 2H), 9.67 (d, 1H). |
| **39** | 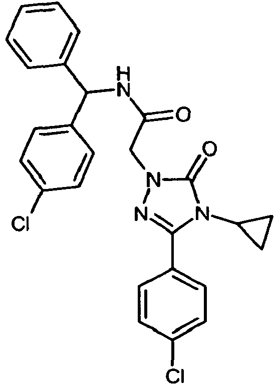 | Rₜ = 2.83 min [4] | δ = 0.49 - 0.64 (m, 2H), 0.82 - 0.97 (m, 2H), 3.17 (dddd, 1H), 4.52 (s, 2H), 6.11 (d, 1H), 7.23 - 7.43 (m, 9H), 7.58, 7.60 (AA'-Teil eines AA'BB'-Systems, 2H), 7.78, 7.81 (BB'-Teil eines AA'BB'-Systems, 2H), 9.09 (d, 1H). |
| **40** | 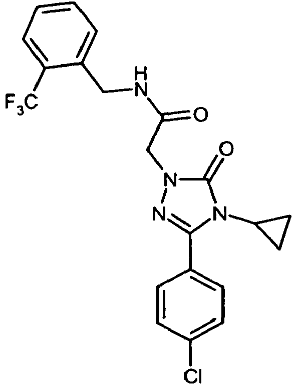 | Rₜ = 2.55 min [4] | δ = 0.52 - 0.67 (m, 2H), 0.83 - 0.98 (m, 2H), 3.18 (dddd, 1H), 4.49 (br. s, 4H), 7.48 (t, 1H), 7.56 (d, 1H), 7.60, 7.62 (AA'-Teil eines AA'BB'-Systems, 2H), 7.66 (t, 1H), 7.72 (d, 1H), 7.81, 7.83 (BB'-Teil eines AA'BB'-Systems, 2H), 8.66 (t, 1H). |
| **41** | 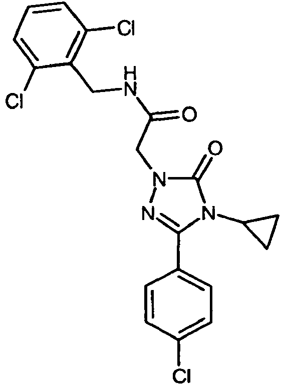 | Rₜ = 2.51 min [4] | δ = 0.50 - 0.65 (m, 2H), 0.82 - 0.97 (m, 2H), 3.18 (dddd, 1H), 4.37 (s, 2H), 4.53 (d, 2H), 7.38 (t, 1H), 7.50 (d, 2H), 7.58, 7.60 (AA'-Teil eines AA'BB'-Systems, 2H), 7.80, 7.82 (BB'-Teil eines AA'BB'-Systems, 2H), 8.39 (t, 1H). |
| **42** | 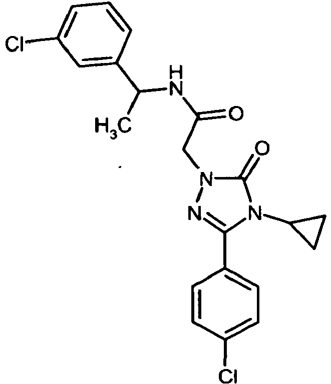 | Rₜ = 2.42 min [5] | δ = 0.50 - 0.64 (m, 2H), 0.82 - 0.97 (m, 2H), 1.37 (d, 3H), 3.17 (dddd, 1H), 4.42 (Zentrum eines AB-Systems, 2H), 4.91 (dq, 1H), 7.26 - 7.40 (m, 4H), 7.58, 7.60 (AA'-Teil eines AA'BB'-Systems, 2H), 7.79, 7.81 (BB'-Teil eines AA'BB'-Systems, 2H), 8.63 (t, 1H). |

Weiter werden auf analoge Weise erhalten:

| **Beispiel Nr.** | **Struktur** | **LC/MS Rₜ [Methode]** |
|---|---|---|
| **43** | 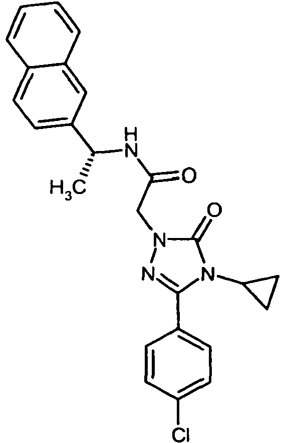 | Rₜ = 2.67 min [4] |
| **44** | 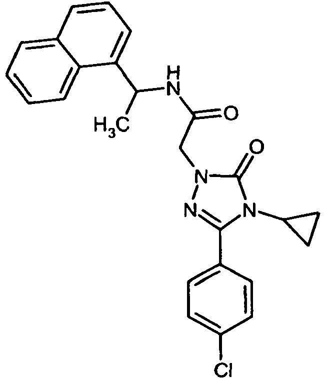 | Rₜ = 2.33 min [7] |
| **45** | 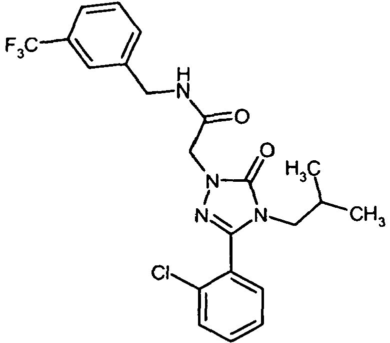 | Rₜ = 2.37 min [7] |
| **46** | 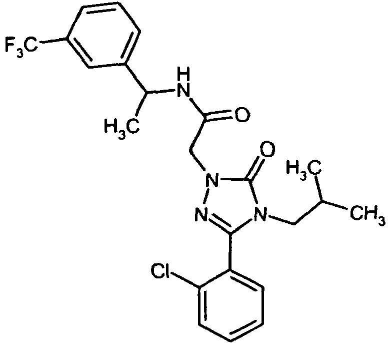 | Rₜ = 2.45 min [7] |
| **47** | 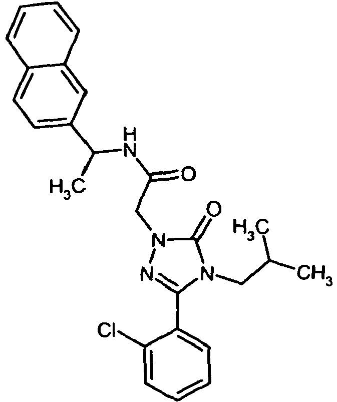 | Rₜ = 2.45 min [7] |
| **48** | 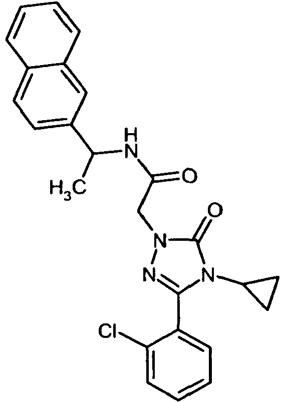 | Rₜ = 2.22 min [7] |
| **49** | 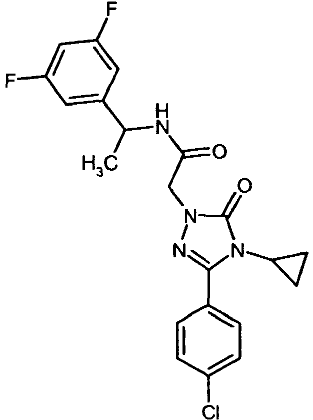 | Rₜ = 2.20 min [7] |
| **50** | 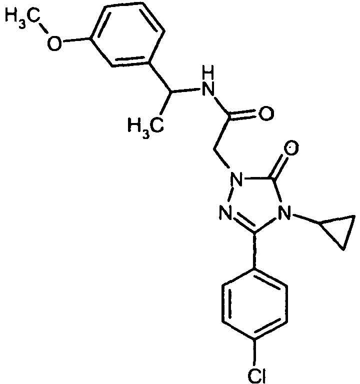 | Rₜ = 2.42 min [4] |
| **51** | 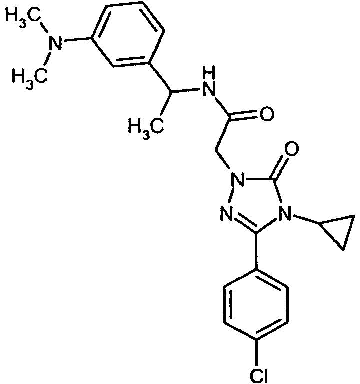 | Rₜ = 1.76 min [7] |
| **52** | 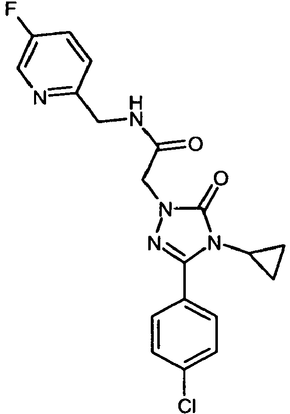 | Rₜ = 1.75 min [7] |
| **53** | 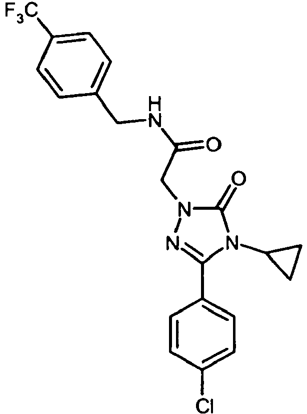 | Rₜ = 2.62 min [4] |
| **54** | 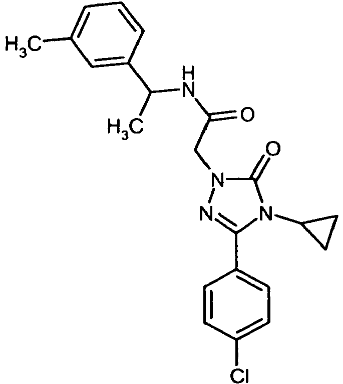 | Rₜ = 2.22 min [7] |
| **55** | 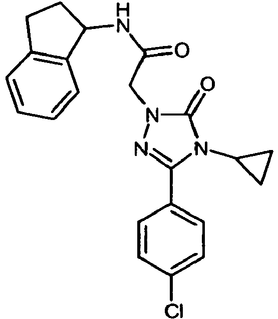 | Rₜ = 2.42 min [4] |
| **56** | 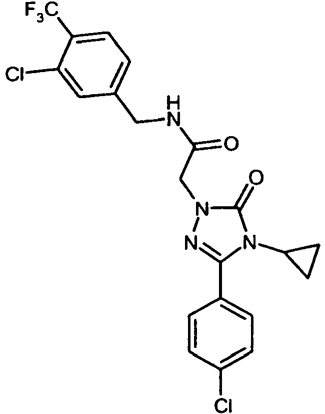 | Rₜ = 2.64 min [4] |
| **57** | 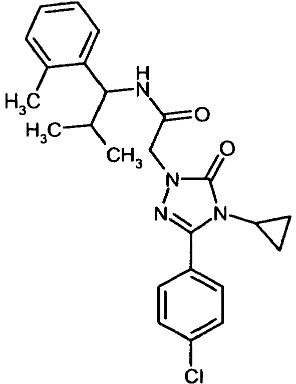 | Rₜ = 2.70 min [4] |
| **58** | 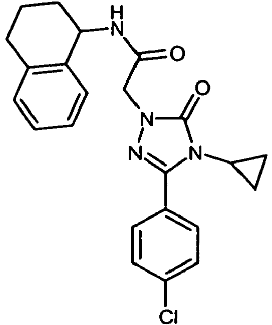 | Rₜ = 2.51 min [4] |
| **59** | 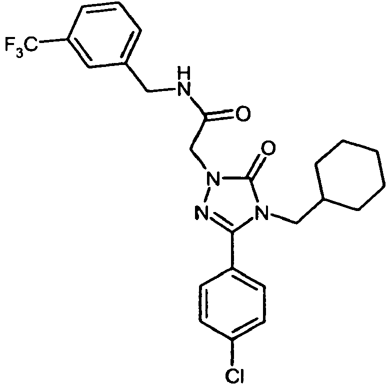 | Rₜ = 2.91 min [5] |
| **60** | 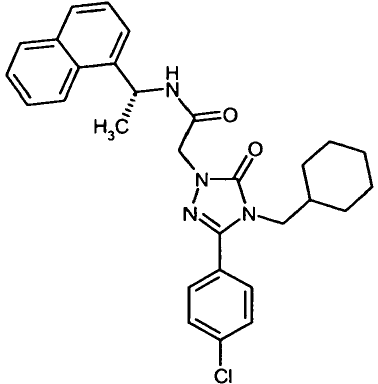 | Rₜ = 2.99 min [5] |
| **61** | 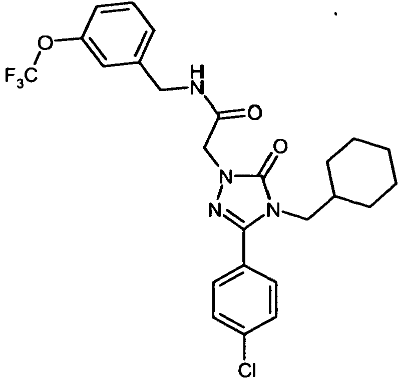 | Rₜ = 2.96 min [5] |
| **62** | 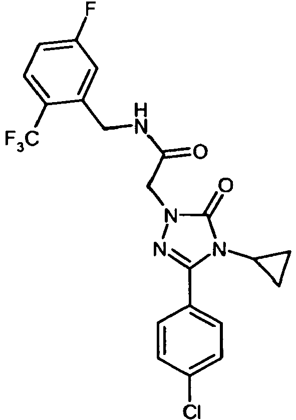 | Rₜ = 2.46 min [5] |
| **63** | 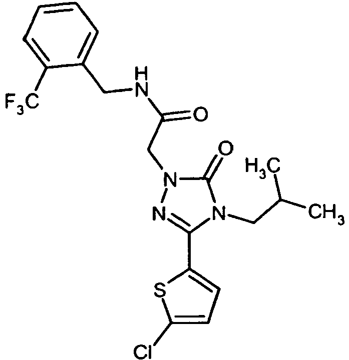 | Rₜ = 2.68 min [5] |
| **64** | 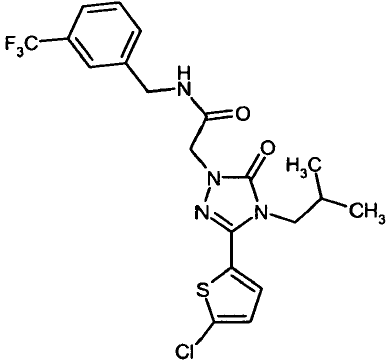 | Rₜ = 2.53 min [7] |
| **65** | 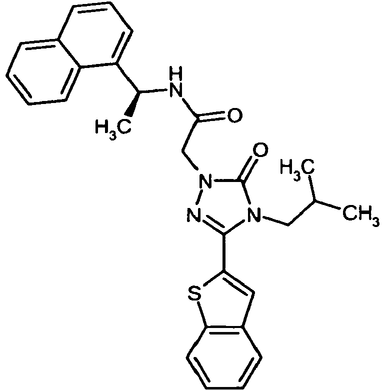 | Rₜ = 2.67 min [7] |
| **66** | 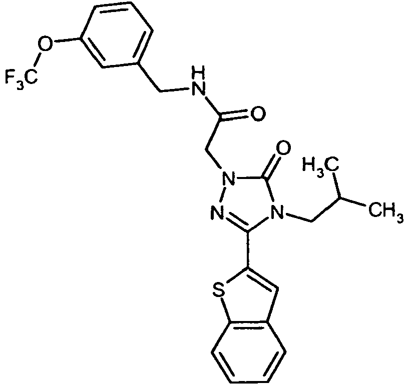 | Rₜ = 2.64 min [7] |
| **67** | 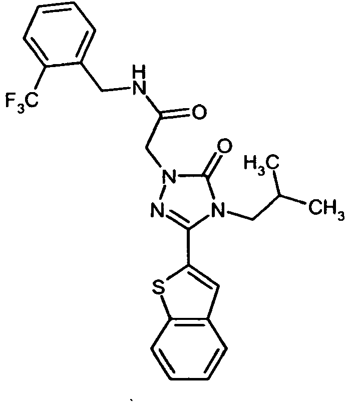 | Rₜ = 2.59 min [7] |
| **68** | 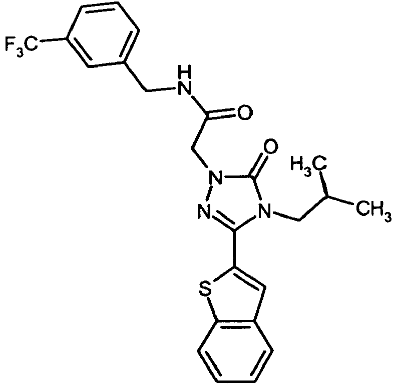 | Rₜ =2.58 min [7] |
| **69** | 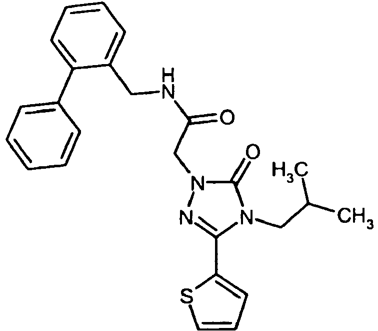 | Rₜ = 2.42 min [7] |
| **70** | 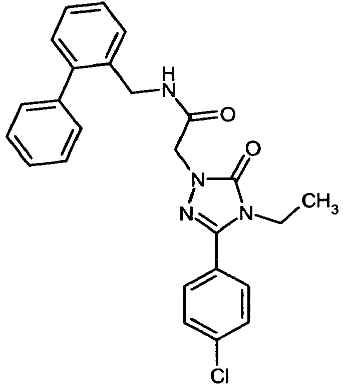 | Rₜ = 2.56 min [5] |
| **71** | 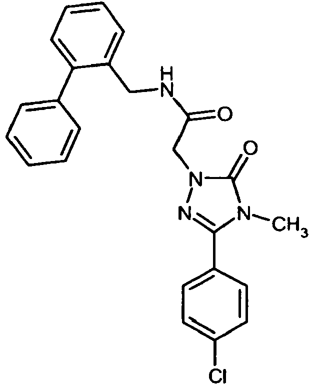 | Rₜ = 2.46 min [5] |
| **72** | 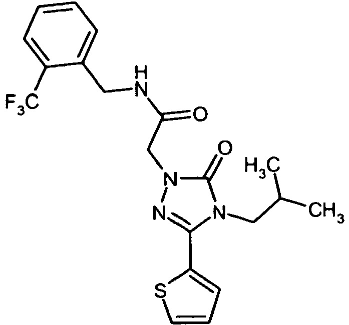 | Rₜ = 2.27 min [7] |
| **73** | 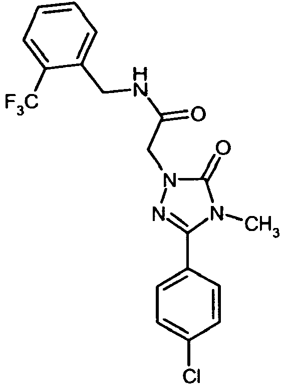 | Rₜ = 2.27 min [7] |
| **74** | 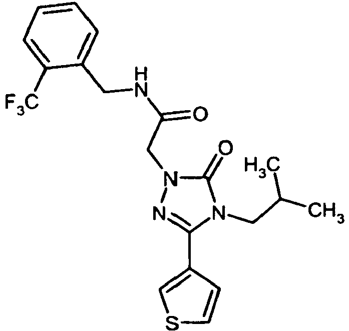 | Rₜ = 2.45 min [8] |

### Beispiel 75

### 2-[3-(4-Chlorphenyl)-4-cyclopropyl-5-oxo-4,5-dihydro-1H-1,2,4-triazol-1-yl]-N-{1-methyl-1-[3-(trifluormethyl)phenyl]ethyl}essigsäureamid

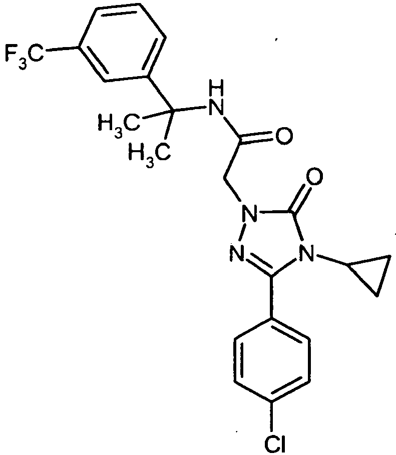

70.0 mg (0.238 mmol) [3-(4-Chlorphenyl)-4-cyclopropyl-5-oxo-4,5-dihydro-1*H*-1,2,4-triazol-1-yl]-essigsäure aus Beispiel 88A und 53.3 mg (0.262 mmol) 1-Methyl-1-[(3-trifluormethyl)phenyl]-ethylamin aus Beispiel 1A werden in 2 ml Dimethylformamid vorgelegt und mit 38.6 mg (0.286 mmol) HOBt versetzt. Nach 10 min Rühren werden 59.4 mg (0.310 mmol) EDC-Hydrochlorid zugegeben und das Gemisch über Nacht bei Raumtemperatur gerührt. Zur Aufarbeitung wird das Reaktionsgemisch zwischen Dichlormethan und Wasser verteilt, die organische Phase abgetrennt, über Natriumsulfat getrocknet und eingeengt. Der Rückstand wird durch Flash-Chromatographie an Kieselgel (Eluent: Dichlormethan/Methanol zunächst 200:1, dann 100:1) gereinigt. Man erhält so 97 mg (85% d. Th.) der Zielverbindung.
HPLC [Methode 1]: Rₜ = 4.79 min
MS [ESIpos]: m/z = 479 (M+H)⁺; [ESIneg]: m/z = 477 (M-H)-¹H-NMR (400 MHz, DMSO-d₆): δ = 0.48 - 0.61 (m, 2H), 0.81 - 0.95 (m, 2H), 1.59 (s, 6H), 3.15 (dddd, 1H), 4.42 (s, 2H), 7.48 - 7.69 (m, 4H), 7.57, 7.59 (AA'-Teil eines AA'BB'-Systems, 2H), 7.77, 7.79 (BB'-Teil eines AA'BB'-Systems, 2H), 8.55 (s, 1H).

### Beispiel 76

### 2-[3-(4-Chlorphenyl)-4-cyclopropyl-5-oxo-4,5-dihydro-1H-1,2,4-triazol-1-yl]-N-[1-(3,5-dichlorphenyl)-1-methylethyl]essigsäureamid

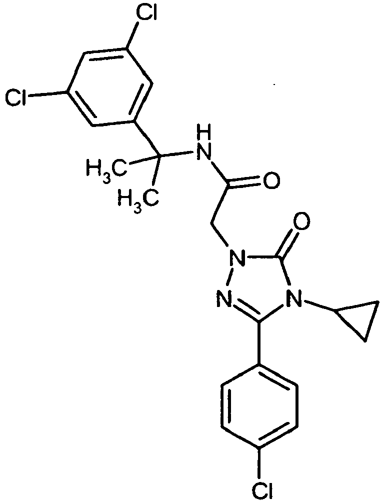

70.0 mg (0.238 mmol) [3-(4-Chlorphenyl)-4-cyclopropyl-5-oxo-4,5-dihydro-1*H*-1,2,4-triazol-1-yl]-essigsäure aus Beispiel 88A und 48.6 mg (0.238 mmol) 2-(3,5-Dichlorphenyl)propan-2-amin werden in 2 ml Dimethylformamid vorgelegt und mit 38.6 mg (0.286 mmol) HOBt versetzt. Nach 10 min Rühren werden 59.4 mg (0.310 mmol) EDC-Hydrochlorid zugegeben und das Gemisch über Nacht bei Raumtemperatur gerührt. Der Ansatz wird ohne weitere Aufarbeitung direkt mittels präparativer HPLC [Methode 10] gereinigt. Man erhält so 70 mg (61 % d. Th.) der Zielverbindung.
MS [ESIpos]: m/z = 479 (M+H)⁺
HPLC [Methode 1]: Rₜ = 4.99 min
¹H-NMR (400 MHz, DMSO-d₆): δ = 0.57 (m, 2H), 0.89 (m, 2H), 1.54 (s, 6H), 3.16 (tt, 1H), 4.42 (s, 2H), 7.33 (d, 2H), 7.39 (t, 1H), 7.58 (d, 2H), 7.80 (d, 2H), 8.53 (s, 1H).

### Beispiel 77

### 2-[3-(4-Chlorphenyl)-4-cyclopropyl-5-oxo-4,5-dihydro-1H-1,2,4-triazol-1-yl]-N-(1-methyl-1-phenylethyl)essigsäureamid

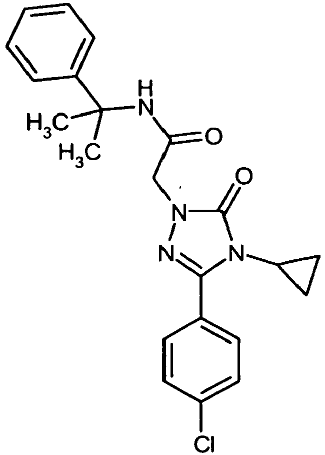

70.0 mg (0.238 mmol) [3-(4-Chlorphenyl)-4-cyclopropyl-5-oxo-4,5-dihydro-1*H*-1,2,4-triazol-1-yl]-essigsäure aus Beispiel 88A und 32.2 mg (0.238 mmol) 2-Phenylpropan-2-amin werden in 2 ml Dimethylformamid vorgelegt und mit 38.7 mg (0.286 mmol) HOBt versetzt. Nach 10 min Rühren werden 59.4 mg (0.310 mmol) EDC-Hydrochlorid zugegeben und das Gemisch über Nacht bei Raumtemperatur gerührt. Danach werden erneut 32.2 mg (0.238 mmol) 2-Phenylpropan-2-amin, 38.7 mg (0.286 mmol) HOBt sowie 59.4 mg (0.310 mmol) EDC-Hydrochlorid zur Reaktionsmischung gegeben und für weitere zwei Stunden bei Raumtemperatur gerührt. Der Ansatz wird ohne weitere Aufarbeitung direkt mittels präparativer HPLC [Methode 10] gereinigt. Man erhält so 44 mg (45% d. Th.) der Zielverbindung.
MS [ESIpos]: m/z = 411 (M+H)⁺
HPLC [Methode 1]: Rₜ = 4.99 min
¹H-NMR (400 MHz, DMSO-d₆): δ = 0.55 (m, 2H), 0.89 (m, 2H), 1.56 (s, 6H), 3.15 (tt, 1H), 4.41 (s, 2H), 7.17 (t, 1H), 7.27 (t, 2H), 7.35 (d, 2H), 7.59 (d, 2H), 7.79 (d, 2H), 8.34 (s, 1H).

### Beispiel 78

### N-[1-(3-Chlorphenyl)cyclobutyl]-2-[3-(4-chlorphenyl)-4-cyclopropyl-5-oxo-4,5-dihydro-1H-1,2,4-triazol-1-yl]essigsäureamid

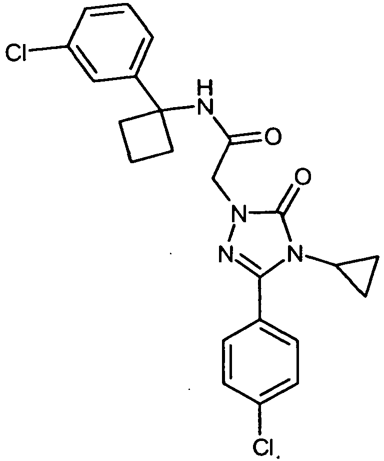

57.2 mg (0.262 mmol) 1-(3-Chlorphenyl)cyclobutanamin-Hydrochlorid werden in 2 ml Dimethylformamid vorgelegt und mit 26.5 mg (0.262 mmol) Triethylamin versetzt. Nach 10 min Rühren werden 70.0 mg (0.238 mmol) [3-(4-Chlorphenyl)-4-cyclopropyl-5-oxo-4,5-dihydro-1*H*-1,2,4-triazol-1-yl]-essigsäure aus Beispiel 88A und 38.7 mg (0.286 mmol) HOBt zugegeben. Nach weiteren 10 min Rühren wird mit 59.4 mg (0.310 mmol) EDC-Hydrochlorid versetzt und über Nacht bei Raumtemperatur gerührt. Zur Aufarbeitung wird das Reaktionsgemsich zwischen Dichlormethan und Wasser verteilt, die organische Phase abgetrennt, über Natriumsulfat getrocknet und eingeengt. Der Rückstand wird durch Flash-Chromatographie an Kieselgel (Eluent: Dichlormethan/ Methanol zunächst 200:1, dann 100:1) gereinigt und ergibt so 66 mg (61% d. Th.) der Zielverbindung.
MS [ESIpos]: m/z = 457 (M+H)⁺
HPLC [Methode 2]: Rₜ = 4.82 min
¹H-NMR (400 MHz, DMSO-d₆): δ = 0.57 (m, 2H), 0.89 (m, 2H), 1.83 (m, 1H), 2.02 (m, 1H), 2.44 (t, 4H), 3.16 (tt, 1H), 4.39 (s, 2H), 7.25 (br. d, 1H), 7.31-7.41 (m, 3H), 7.58 (d, 2H), 7.79 (d, 2H), 8.83 (s, 1H).

### Beispiel 79

### N-[1-(3-Chlorphenyl)cyclohexyl]-2-[3-(4-chlorphenyl)-4-cyclopropyl-5-oxo-4,5-dihydro-1H-1,2,4-triazol-1-yl]essigsäureamid

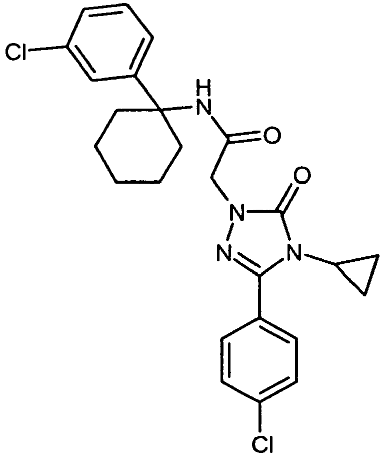

70.0 mg (0.238 mmol) [3-(4-Chlorphenyl)-4-cyclopropyl-5-oxo-4,5-dihydro-1*H*-1,2,4-triazol-1-yl]-essigsäure aus Beispiel 88A und 50.0 mg (0.238 mmol) 1-(3-Chlorphenyl)cyclohexanamin werden in 2 ml Dimethylformamid vorgelegt und mit 38.7 mg (0.286 mmol) HOBt versetzt. Nach 10 min Rühren werden 59.4 mg (0.310 mmol) EDC-Hydrochlorid zugegeben und das Gemisch über Nacht bei Raumtemperatur gerührt. Danach werden erneut 50.0 mg (0.238 mmol) 1-(3-Chlorphenyl)-cyclohexanamin, 38.7 mg (0.286 mmol) HOBt und 59.4 mg (0.310 mmol) EDC-Hydrochlorid zur Reaktionsmischung gegeben und zunächst für zwei Stunden bei Raumtemperatur, dann über Nacht bei 60°C gerührt. Abschließendes zweistündiges Rühren bei 80°C sowie direkte Reinigung des Ansatzes ohne weitere Aufarbeitung mittels präparativer HPLC [Methode 10] liefern 6 mg (5% d. Th.) der Zielverbindung.
LC/MS [Methode 7]: Rₜ = 2.64 min
¹H-NMR (400 MHz, DMSO-d₆): δ = 0.56 (m, 2H), 0.89 (m, 2H), 1.49-1.69 (m, 8H), 2.25 (m, 2H), 3.16 (tt, 1H), 4.49 (s, 2H), 7.22 (br. d, 1H), 7.27-7.38 (m, 3H), 7.59 (d, 2H), 7.79 (d, 2H), 8.11 (s, 1H).

### Beispiel 80

### 2-[3-(4-Chlorphenyl)-4-cyclopropyl-5-oxo-4,5-dihydro-1H-1,2,4-triazol-1-yl]-N-{1-[3-(trifluormethyl)phenyl]ethyl} essigsäureamid (Enantiomer A)

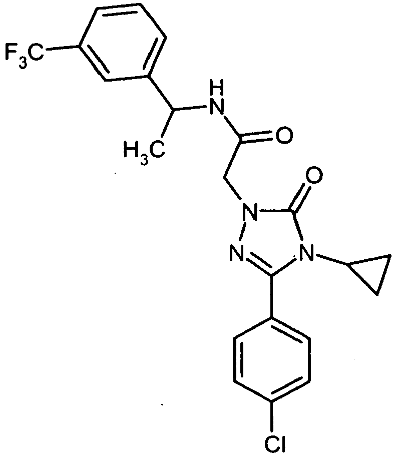

2000.0 mg (6.809 mmol) [3-(4-Chlorphenyl)-4-cyclopropyl-5-oxo-4,5-dihydro-1*H*-1,2,4-triazol-1-yl]-essigsäure aus Beispiel 88A und 1417.0 mg (7.490 mmol) 1-[3-(Trifluormethyl)phenyl]ethanamin werden in 10 ml Dimethylformamid vorgelegt und mit 1104.0 mg (8.171 mmol) HOBt versetzt. Nach 10 min Rühren werden 1697.0 mg (8.852 mmol) EDC-Hydrochlorid zugegeben und das Gemisch über Nacht bei Raumtemperatur gerührt. Zur Aufarbeitung wird das Reaktionsgemisch zwischen Dichlormethan und Wasser verteilt, die organische Phase abgetrennt, über Natriumsulfat getrocknet und eingeengt. Der Rückstand wird durch Flash-Chromatographie an Kieselgel (Eluent: Dichlormethan/Methanol zunächst 200:1, dann 100:1) gereinigt. Eine darauffolgende Enantiomerentrennung mittels präparativer HPLC an chiraler Phase [Methode 14] ergibt 1460 mg (46% d. Th.) der enantiomerenreinen Zielverbindung (siehe auch Beispiel 81).
MS [ESIpos]: m/z = 465 (M+H)⁺
HPLC [Methode 2]: Rₜ = 4.74 min
¹H-NMR (400 MHz, DMSO-d₆): δ = 0.57 (m, 2H), 0.89 (m, 2H), 1.39 (d, 3H), 3.17 (tt, 1H), 4.42 (s, 2H), 5.00 (dq, 1H), 7.52-7.68 (m, 6H), 7.79 (d, 2H), 8.69 (d, 1H).
chirale HPLC [Methode 14]: Rₜ = 2.02 min.

### Beispiel 81

### 2-[3-(4-Chlorphenyl)-4-cyclopropyl-5-oxo-4,5-dihydro-1H-1,2,4-triazol-1-yl]-N-{1-[3-(trifluormethyl)phenyl]ethyl}essigsäureamid (Enantiomer B)

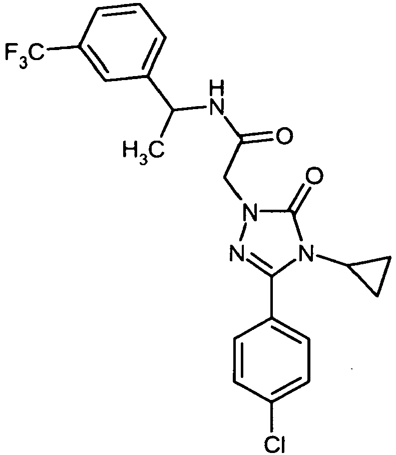

2000.0 mg (6.809 mmol) [3-(4-Chlorphenyl)-4-yclopropyl-5-oxo-4,5-dihydro-1*H*-,2,4-triazol-1-yl]-essigsäure aus Beispiel 88A und 1417.0 mg (7.490 mmol) 1-[3-(Trifluormethyl)phenyl]ethan-amin werden in 10 ml Dimethylformamid vorgelegt und mit 1104.0 mg (8.171 mmol) HOBt versetzt. Nach 10 min Rühren werden 1697.0 mg (8.852 mmol) EDC-Hydrochlorid zugegeben und das Gemisch über Nacht bei Raumtemperatur gerührt. Zur Aufarbeitung wird das Reaktionsgemisch zwischen Dichlormethan und Wasser verteilt, die organische Phase abgetrennt, über Natriumsulfat getrocknet und eingeengt. Der Rückstand wird durch Flash-Chromatographie an Kieselgel (Eluent: Dichlormethan/Methanol zunächst 200:1, dann 100:1) gereinigt. Eine darauffolgende Enantiomerentrennung mittels präparativer HPLC an chiraler Phase [Methode 14] ergibt 1260 mg (40% d. Th.) der enantiomerenreinen Zielverbindung (siehe auch Beispiel 80).
MS [ESIpos]: m/z = 465 (M+H)⁺
HPLC [Methode 2]: Rₜ = 4.74 min
¹H-MR (400 MHz, DMSO-d₆): δ= 0.58 (m, 2H), 0.89 (m, 2H), 1.40 (d, 3H), 3.17 (tt, 1H), 4.43 (s, 2H), 5.01 (dq, 1H), 7.52-7.68 (m, 6H), 7.79 (d, 2H), 8.70 (d, 1H).
chirale HPLC [Methode 14]: Rₜ = 2.71 min.

### Beispiel 82

### N-(5-Brom-2-fluorphenylmethyl)-2-[3-(4-chlorphenyl)-4-yclopropyl-5-oxo-4,5-dihydro-1H-1,2,4-triazol-1-yl]essigsäureamid

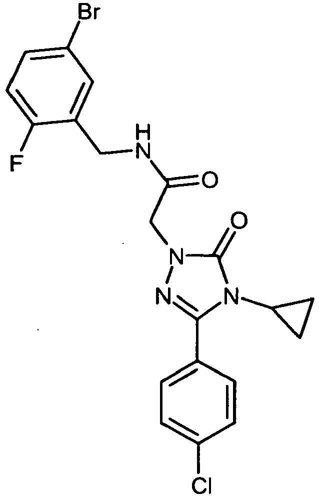

40.0 mg (0.144 mmol) [3-(4-Chlorphenyl)-4-cyclopropyl-5-oxo-4,5-dihydro-1*H*-1,2,4-triazol-1-yl]-essigsäure aus Beispiel 93A, 36.0 mg (0.150 mmol) 5-Brom-2-fluorbenzylamin-Hydrochlorid, 22.1 mg (0.163 mmol) HOBt sowie 17.6 mg (0.136 mmol) *N*,*N*-Diisopropylethylamin werden in 1.5 ml Dimethylformamid vorgelegt und mit 33.9 mg (0.177 mmol) EDC-Hydrochlorid versetzt. Man rührt über Nacht bei Raumtemperatur, verdünnt dann mit 15 ml Wasser und extrahiert mit Essigsäureethylester. Nach Einengen der organischen Phase wird das Rohprodukt durch präparative HPLC [Methode 13] gereinigt. Man erhält so 21 mg (32% d. Th.) der Zielverbindung.
LC/MS [Methode 5]: Rₜ = 2.39 min
¹H-NMR (400 MHz, DMSO-d₆): δ = 0.53 - 0.67 (m, 2H), 0.83 - 0.98 (m, 2H), 3.18 (dddd, 1H), 4.34 (d, 2H), 4.45 (s, 2H), 7.15 - 7.23 (m, 1H), 7.47 - 7.53 (m, 2H), 7.59, 7.61 (AA'-Teil eines AA'BB'-Systems, 2H), 7.82, 7.84 (BB'-Teil eines AA'BB'-Systems, 2H), 8.62 (t, 1H).

### Beispiel 83

### 2-[3-(4-Chlorphenyl)-4-(2-methoxyethyl)-5-oxo-4,5-dihydro-1H-1,2,4-triazol-1-yl]-N-[3-(trifluormethyl)phenylmethyl]essigsäureamid

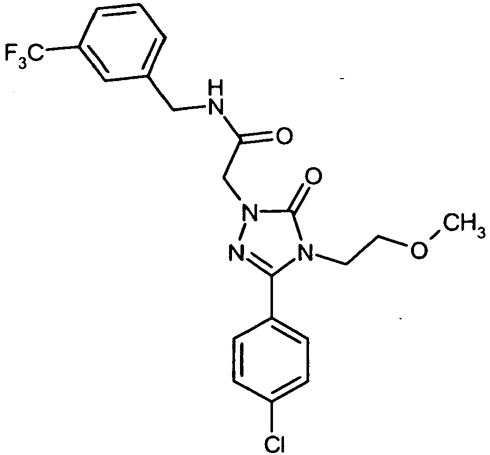

50.0 mg (0.160 mmol) 2-[3-(4-Chlorphenyl)-4-(2-methoxyethyl)-5-oxo-4,5-dihydro-1*H*-1,2,4-triazol-1-yl]-essigsäure aus Beispiel 90A, 30.9 mg (0.176 mmol) 3-Trifluormethylbenzylamin und 40.0 mg (0.209 mmol) HOBt werden in 2 ml Dimethylformamid vorgelegt und mit 26.0 mg (0.192 mmol) EDC-Hydrochlorid versetzt. Man rührt über Nacht bei Raumtemperatur, verrührt dann mit 15 ml Wasser und gewinnt den entstehenden Niederschlag durch Filtration. Das Rohprodukt wird mit Wasser gewaschen und im Vakuum getrocknet. Man erhält so 66.9 mg (89% d. Th.) der Zielverbindung.
LC/MS [Methode 7]: Rₜ = 2.24 min
¹H-NMR (400 MHz, DMSO-d₆): δ = 3.13 (s, 3H), 3.50 (t, 2H), 3.88 (t, 2H), 4.41 (d, 2H), 4.50 (s, 2H), 7.54 - 7.65 (m, 6H), 7.70 - 7.75 (m, 2H), 8.71 (t, 1H).

Auf analoge Weise werden die folgenden Verbindungen erhalten:

| **Beispiel Nr.** | **Struktur** | **LC/MS Rₜ [Methode]** | **¹H-NMR (400 MHz, DMSO-d₆)** |
|---|---|---|---|
| **84** | 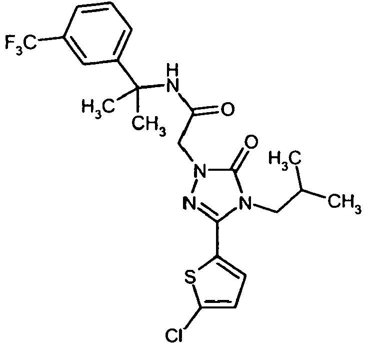 | Rₜ = 2.71 min [7] | δ = 0.80 (d, 6H), 1.58 (s, 6H), 1.79 - 1.91 (m, 1H), 3.65 (d, 2H), 4.46 (s, 2H), 7.25 (d, 1H), 7.45 (d, 1H), 7.48 - 7.56 (m, 2H), 7.60 (s, 1H), 7.66 (d, 1H), 8.56 (s, 1H). |
| **85** | 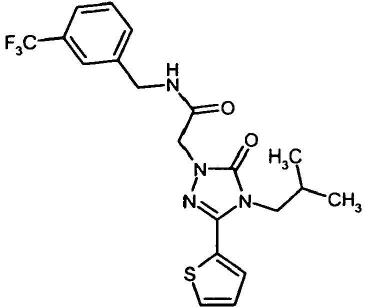 | Rₜ = 2.43 min [5] | δ = 0.83 (d, 6H), 1.83 -1.95 (m, 1H), 3.70 (d, 2H), 4.41 (d, 2H), 4.50 (s, 2H), 7.23 (dd, 1H), 7.54 - 7.64 (m, 5H), 7.79 (dd, 1H), 8.72 (t, 1H). |
| **86** | 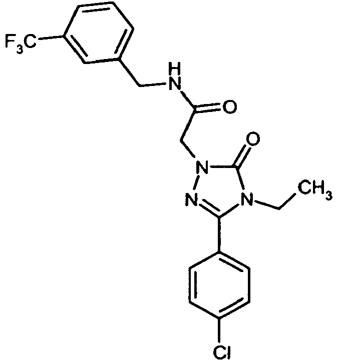 | Rₜ = 2.41 min [5] | δ = 1.13 (t, 3H), 3.76 (q, 2H), 4.41 (d, 2H), 4.49 (s, 2H), 7.54 - 7.70 (m, 8H), 8.70 (t, 1H). |
| **87** | 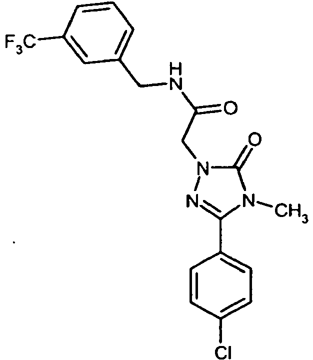 | Rₜ = 2.31 min [5] | δ = 3.30 (s, 3H), 4.41 (d, 2H), 4.50 (s, 2H), 7.54 -7.65 (m, 6H), 7.70 - 7.76 (m, 2H), 8.68 (t, 1H). |
| **88** | 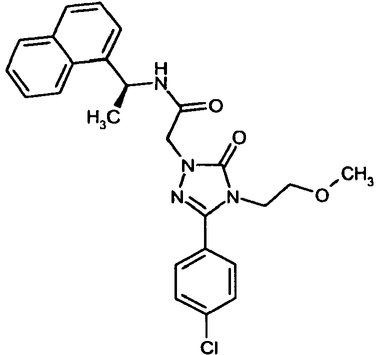 | Rₜ = 2.30 min [7] | δ = 1.52 (d, 3H), 3.11 (s, 3H), 3.47 (t, 2H), 3.86 (t, 2H), 4.47 (Zentrum eines AB-Systems, 2H), 5.71 (dq, 1H), 7.47 - 7.63 (m, 6H), 7.67 - 7.72 (m, 2H), 7.84 (d, 1H), 7.91 - 7.97 (m, 1H), 8.10 (d, 1H), 8.78 (d, 1H). |
| **89** | 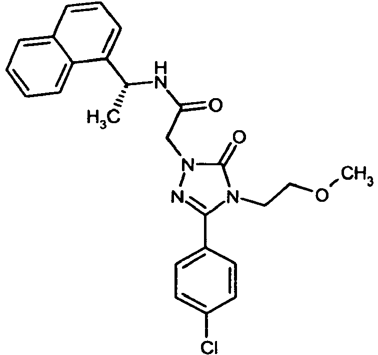 | Rₜ = 2.30 min [7] | δ = 1.52 (d,3H),3.11 (s, 3H), 3.47 (t, 2H), 3.86 (t, 2H), 4.47 (Zentrum eines AB-Systems, 2H), 5.71 (dq, 1H), 7.47 - 7.63 (m, 6H), 7.67 - 7.72 (m, 2H), 7.84 (d, 1H), 7.91 - 7.97 (m, 1 H), 8.10 (d, 1H), 8.78 (d, 1H). |
| **90** | 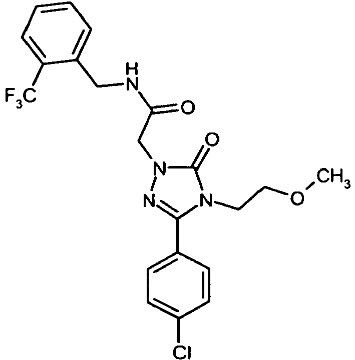 | Rₜ = 2.23 min [7] | δ = 3.13 (s, 3H), 3.50 (t, 2H), 3.88 (t, 2H), 4.49 (d, 2H), 4.54 (s, 2H), 7.48 (t, 1H), 7.55 - 7.77 (m, 7H), 8.71 (t, 1H). |
| **91** | 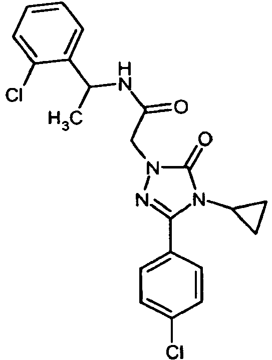 | Rₜ = 2.44 min [5] | δ = 0.49 - 0.63 (m, 2H), 0.81 - 0.97 (m, 2H), 1.34 (d, 3H), 3.16 (dddd, 1H), 4.43 (s, 2H), 5.20 (dq, 1H), 7.26 (dt, 1H), 7.33 (dt, 1H), 7.40 dd, 1H), 7.47 (dd, 1H), 7.58, 7.60 (AA'-Teil eines AA'BB'-Systems, 2H), 7.78, 7.80 (BB'-Teil eines AA'BB'-Systems, 2H), 8.77 (d, 1H). |
| **92** | 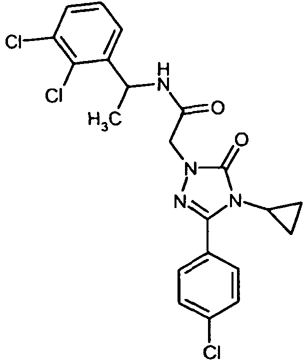 | Rₜ = 2.57 min [5] | δ = 0.49 - 0.62 (m, 2H), 0.81 - 0.96 (m, 2H), 1.35 (d, 3H), 3.16 (dddd, 1H), 4.43 (s, 2H), 5.20 (dq, 1H), 7.36 (t, 1H), 7.44 (dd, 1H), 7.53 (dd, 1H), 7.58, 7.60 (AA'-Teil eines AA'BB'-Systems, 2H), 7.77, 7.80 (BB'-Teil eines AA'BB'-Systems, 2H), 8.85 (d, 1H). |
| **93** | 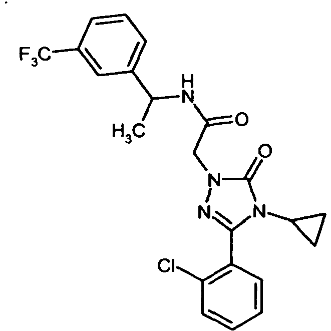 | Rₜ = 2.23 min [7] | δ = 0.45 - 0.59 (m, 2H), 0.59 - 0.74 (m, 2H), 1.40 (d, 3H), 2.87 (dddd, 1H), 4.43 (s, 2H), 5.01 (dq, 1H), 7.37 - 7.69 (m, 8H), 8.69 (d, 1H). |
| **94** | 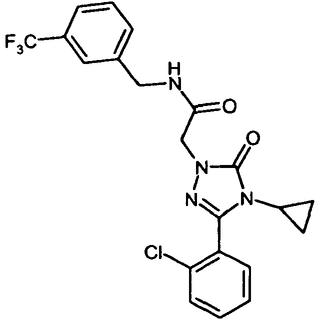 | Rₜ = 2.15 min [7] | δ = 0.51 - 0.58 (m, 2H), 0.64 - 0.71 (m, 2H), 2.89 (dddd, 1H), 4.42 (d, 2H), 4.46 (s, 2H), 7.48 - 7.69 (m, 8H), 8.65 (t, 1H). |
| **95** | 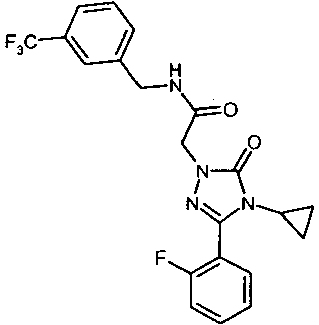 | Rₜ = 2.08 min [7] | δ = 0.44 - 0.59 (m, 2H), 0.67 - 0.81 (m, 2H); 2.90 -2.98 (m, 1H), 4.41 (d, 2H), 4.46 (s, 2H), 7.37 (t, 1H), 7.43 (dd, 1 H), 7.53 - 7.68 (m, 6H), 8.69 (t, 1H). |
| **96** | 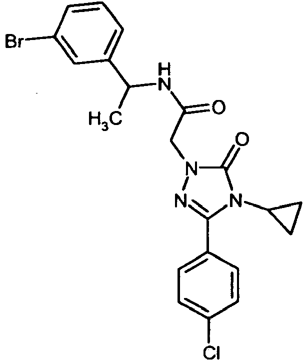 | Rₜ = 2.48 min [5] | δ = 0.51 - 0.64 (m, 2H), 0.82 - 0.97 (m, 2H), 1.36 (d, 3H), 3.17 (dddd, 1H), 4.41 (Zentrum eines AB-Systems, 2H), 4.90 (dq, 1H), 7.25 - 7.35 (m, 2H), 7.43 (dt, 1H), 7.51 (t, 1H), 7.58, 7.60 (AA'-Teil eines AA'BB'-Systems, 2H), 7.79, 7.81 (BB'-Teil eines AA'BB'-Systems, 2H), 8.62 (d, 1H). |

Weiter werden auf analoge Weise erhalten:

| **Beispiel Nr.** | **Struktur** | **LC/MS Rₜ [Methode]** |
|---|---|---|
| **97** | 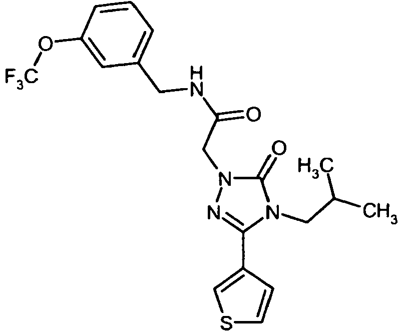 | Rₜ = 2.49 min [5] |
| **98** | 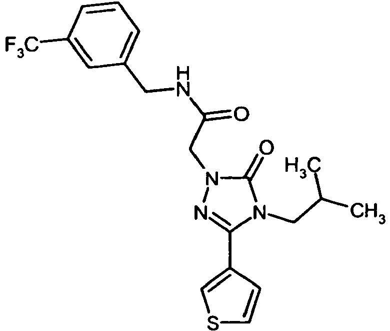 | Rₜ = 2.43 min [5] |
| **99** | 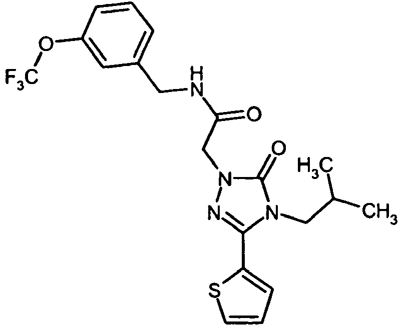 | Rₜ = 2.48 min [5] |
| **100** | 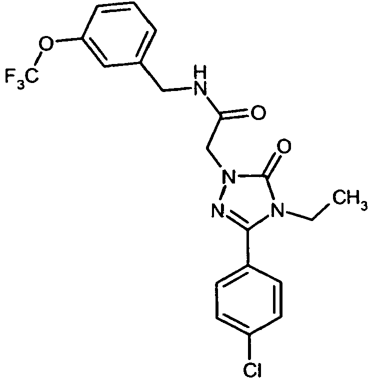 | Rₜ = 2.46 min [5] |
| **101** | 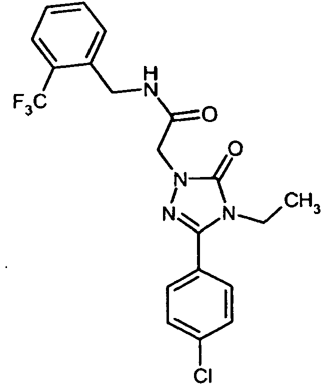 | Rₜ = 2.40 min [5] |
| **102** | 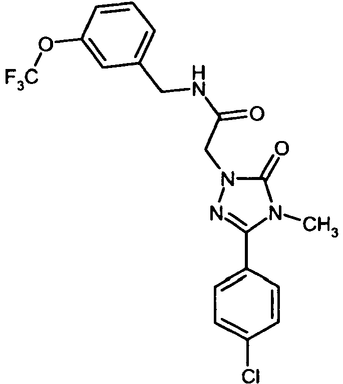 | Rₜ = 2.22 min [7] |
| **103** | 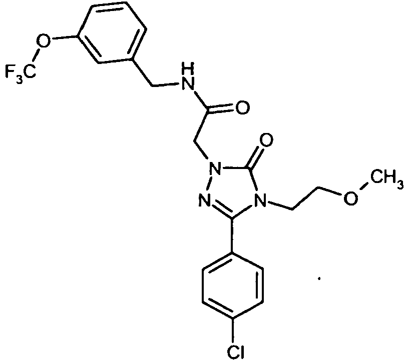 | Rₜ = 2.29 min [7] |
| **104** | 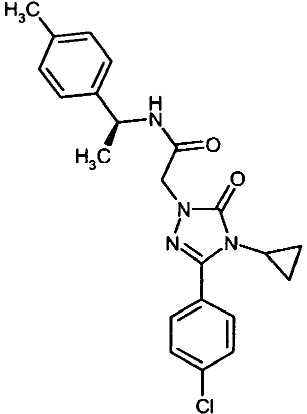 | Rₜ = 2.42 min [5] |
| **105** | 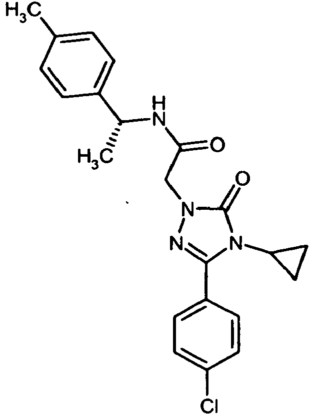 | Rₜ = 2.42 min [5] |
| **106** | 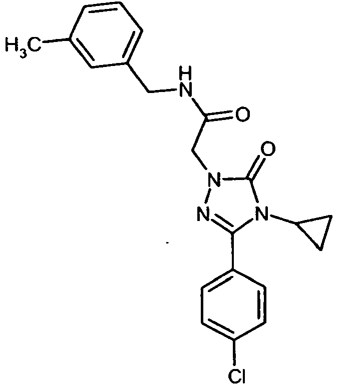 | Rₜ = 2.40 min [4] |
| **107** | 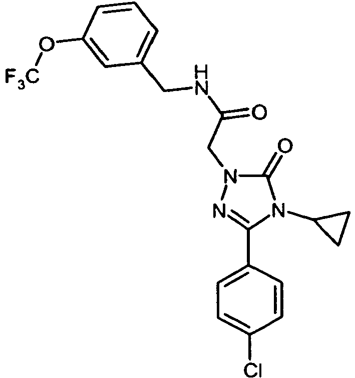 | Rₜ = 2.57 min [4] |
| **108** | 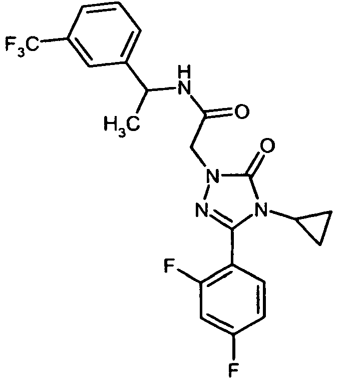 | Rₜ = 2.22 min [7] |
| **109** | 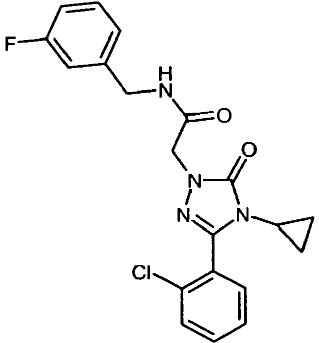 | Rₜ = 1.94 min [7] |
| **110** | 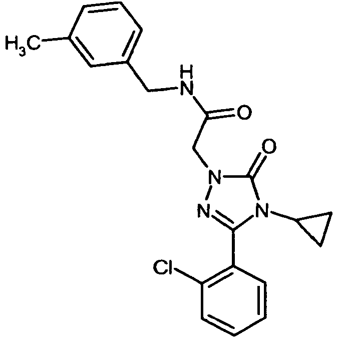 | Rₜ = 2.02 min [7] |
| **111** | 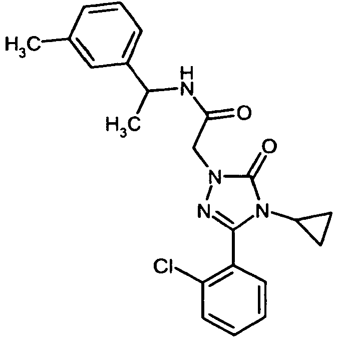 | Rₜ = 2.10 min [7] |
| **112** | 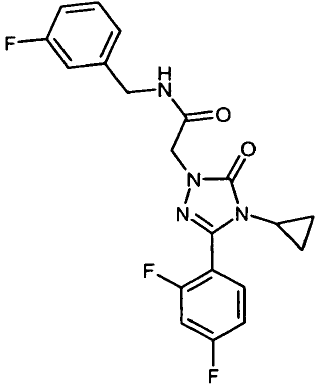 | Rₜ = 1.93 min [7] |
| **113** | 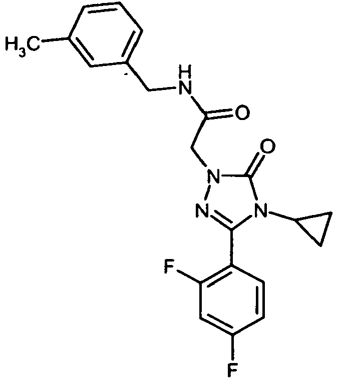 | Rₜ = 2.01min [7] |
| **114** | 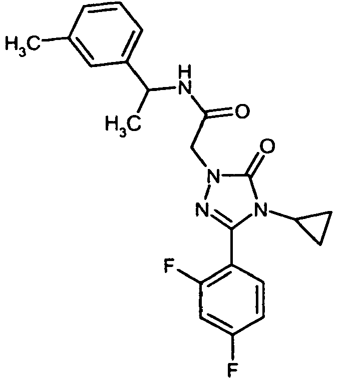 | Rₜ = 2.09 min [7] |
| **115** | 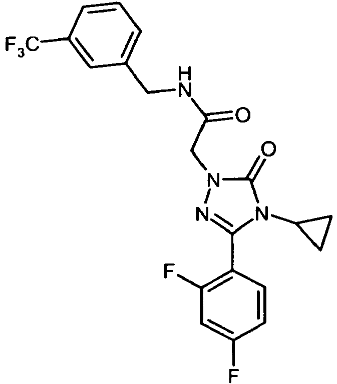 | Rₜ =2.13min [7] |
| **116** | 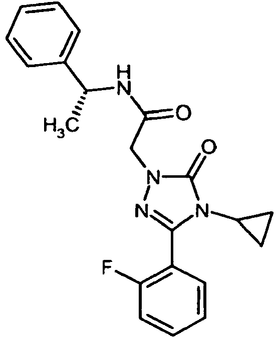 | Rₜ = 1.88 min [7] |
| **117** | 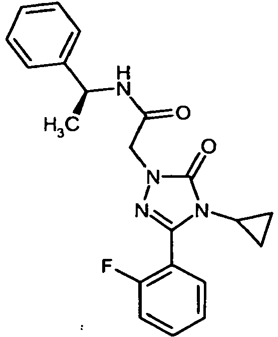 | Rₜ = 1.89 min [7] |
| **118** | 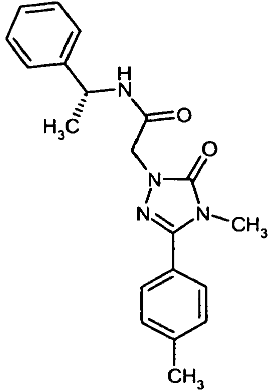 | Rₜ = 2.13 min [5] |
| **119** | 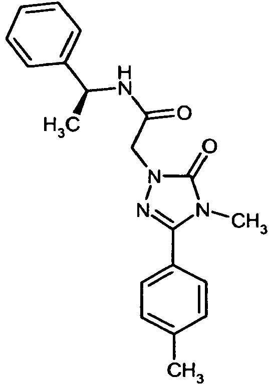 | Rₜ = 2.12 min [5] |
| **120** | 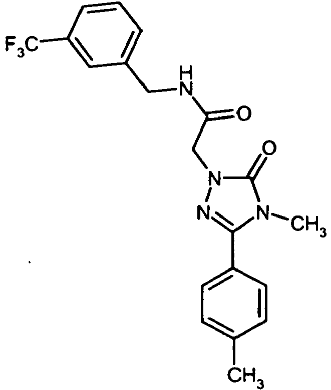 | Rₜ = 2.30 min [5] |
| **121** | 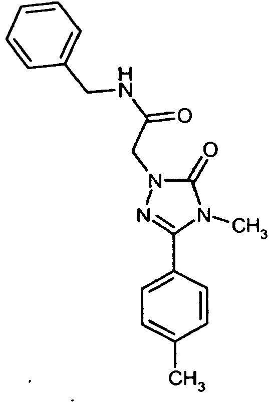 | Rₜ = 2.03 min [5] |
| **122** | 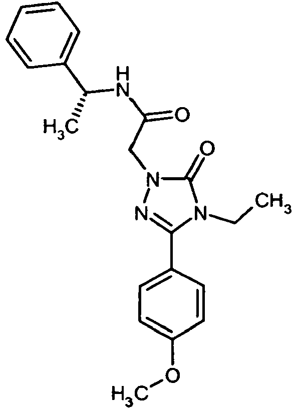 | Rₜ = 2.09 min [5] |
| **123** | 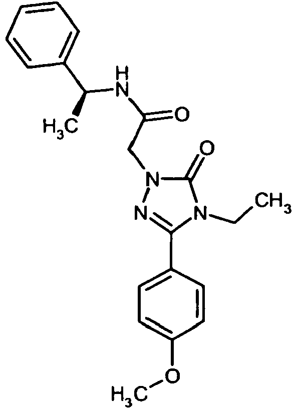 | Rₜ = 2.09 min [5] |
| **124** | 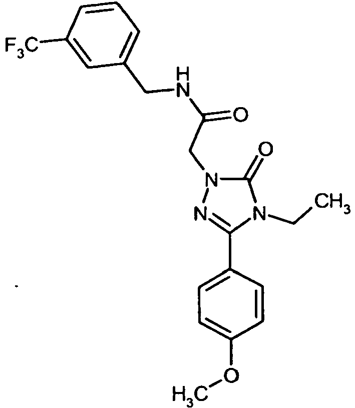 | Rₜ = 2.28 min [5] |
| **125** | 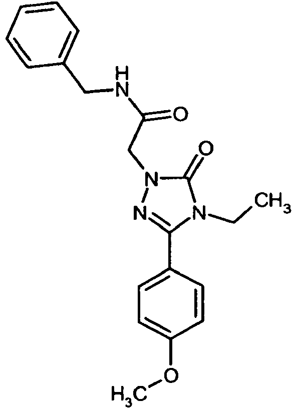 | Rₜ = 2.01 min [5] |

### Beispiel 126

### 2-[4-Cyclopropyl-3-(2-fluorphenyl)-5-oxo-4,5-dihydro-1H-1,2,4-triazol-1-yl]-N-(2-phenylethyl)-essigsäureamid

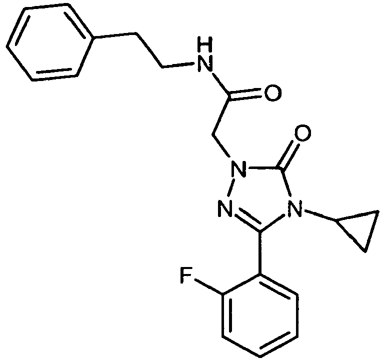

40.0 mg (0.144 mmol) [4-Cyclopropyl-3-(2-fluorphenyl)-2,4-dihydro-3*H*-1,2,4-triazol-3-yl]-essigsäure aus Beispiel 92A, 19.2 mg (0.159 mmol) 2-Phenylethylamin und 23.4 mg (0.173 mmol) HOBt werden in 2 ml Dimethylformamid vorgelegt und mit 36.0 mg (0.188 mmol) EDC-Hydrochlorid versetzt. Man rührt über Nacht bei Raumtemperatur, verdünnt dann mit 10 ml Wasser und extrahiert mit Essigsäureethylester. Nach Einengen der organischen Phase wird das Rohprodukt durch präparative HPLC [Methode 9] gereinigt. Man erhält so 32.0 mg (58% d. Th.) der Zielverbindung.
LC/MS [Methode 4]: Rₜ = 2.13 min
¹H-NMR (400 MHz, DMSO-d₆): δ = 0.45 - 0.58 (m, 2H), 0.68 - 0.82 (m, 2H), 2.72 (t, 2H), 2.90 - 2.98 (m, 1H), 3.29 (t, 2H), 4.34 (s, 2H), 7.17 - 7.24 (m, 3H), 7.24 - 7.31 (m, 2H), 7.35 - 7.47 (m, 2H), 7.57 - 7.69 (m, 2H), 8.17 (t, 1H).

Auf analoge Weise werden erhalten:

| **Beispiel Nr.** | **Struktur** | **LC/MS Rₜ [Methode]** |
|---|---|---|
| **127** | 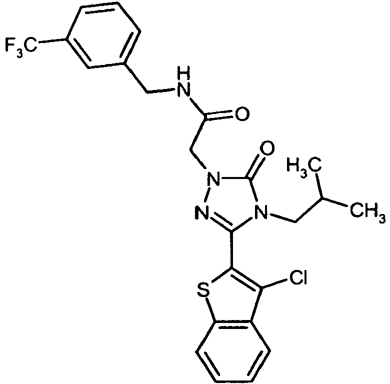 | Rₜ = 2.87 min [8] |
| **128** | 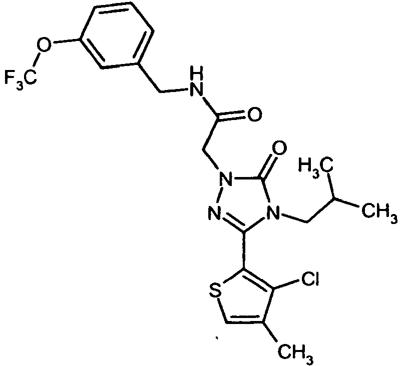 | Rₜ = 2.73 min [8] |
| **129** | 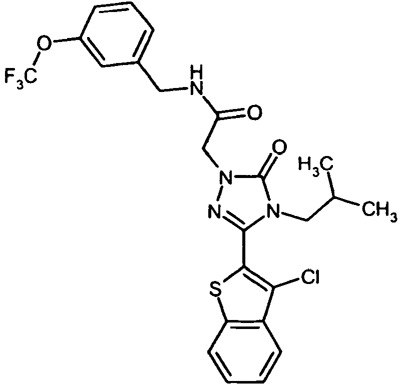 | Rₜ = 2.92mm [8] |
| **130** | 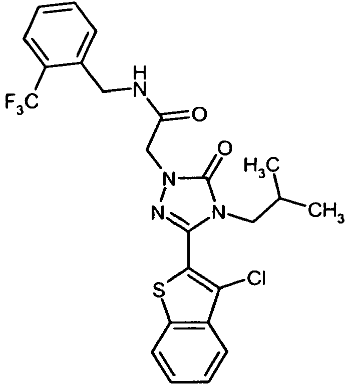 | Rₜ = 2.88 min [8] |
| **131** | 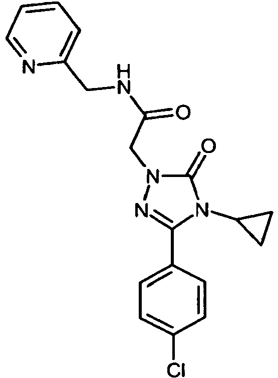 | Rₜ = 1.63 min [5] |
| **132** | 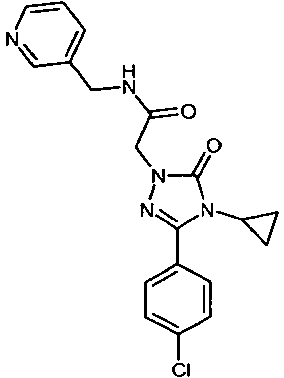 | Rₜ = 1.45 min [5] |
| **133** | 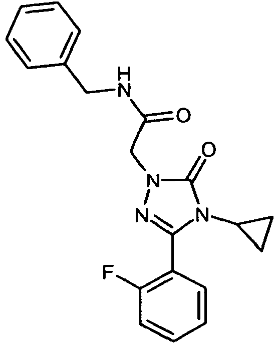 | Rₜ = 2.06 min [4] |
| **134** | 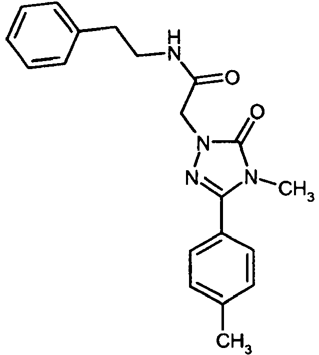 | Rₜ = 2.15 min [4] |
| **135** | 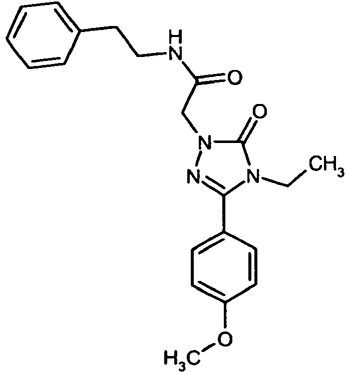 | Rₜ = 1.88 min [7] |

### Beispiel 136

### 2-[4-Cyclopropyl-3-(2-methoxyphenyl)-5-oxo-4,5-dihydro-1H-1,2,4-triazol-1-yl]-N-[3-(trifluormethyl)phenylmethyl]essigsäureamid

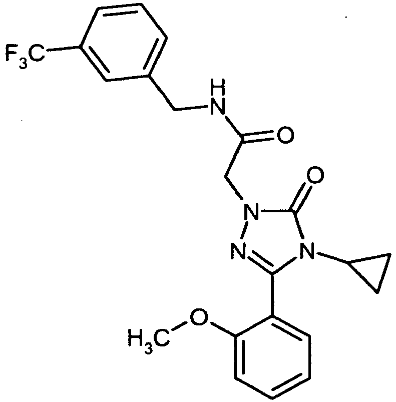

### Methode a):

76 mg (0.26 mmol) [4-Cyclopropyl-3-(2-methoxyphenyl)-5-oxo-4,5-dihydro-1*H*-1,2,4-triazol-1-yl]-essigsäure aus Beispiel 95A und 45 mg (0.28 mmol) Carbonyldiimidazol werden in 1 ml 1,2-Dichlorethan gerührt, bis eine anfängliche Gasentwicklung beendet ist. Man gibt eine Lösung von 51 mg (0.29 mmol) 3-Trifluormethylbenzylamin in 0.55 ml 1,2-Dichlorethan hinzu und rührt über Nacht bei 70°C. Zur Aufarbeitung wird das Lösungsmittel im Vakuum entfernt und der Rückstand durch präparative HPLC [Methode 11] gereinigt. Man erhält so 46 mg (39% d. Th.) der Zielverbindung.

### Methode b):

150 mg (0.52 mmol) [4-Cyclopropyl-3-(2-methoxyphenyl)-5-oxo-4,5-dihydro-1*H*-1,2,4-triazol-1-yl]-essigsäure aus Beispiel 95A, 100 mg (0.57 mmol) 3-Trifluormethylbenzylamin sowie 84 mg (0.62 mmol) HOBt werden in 3.2 ml Dimethylformamid vorgelegt und mit 129 mg (0.67 mmol) EDC-Hydrochlorid versetzt. Man rührt über Nacht bei Raumtemperatur und reinigt anschließend direkt durch präparative HPLC [Methode 11]. Man erhält so 222 mg (96% d. Th.) der Zielverbindung.
LC/MS [Methode 7]: Rₜ = 2.05 min
¹H-NMR (400 MHz, DMSO-d₆): δ = 0.41 - 0.55 (m, 2H), 0.58 - 0.72 (m, 2H), 2.86 (dddd, 1H), 3.86 (s, 3H), 4.42 (d, 2H), 4.43 (s, 2H), 7.06 (t, 1H), 7.19 (d, 1H), 7.33 (dd, 1H), 7.52 - 7.65 (m, 5H), 8.65 (t, 1H).

### Beispiel 137

### 2-[3-(3-Chlorphenyl)-4-cyclopropyl-5-oxo-4,5-dihydro-1H-1,2,4-triazol-1-yl]-N-[3-(trifluormethyl)phenylmethyl]essigsäureamid

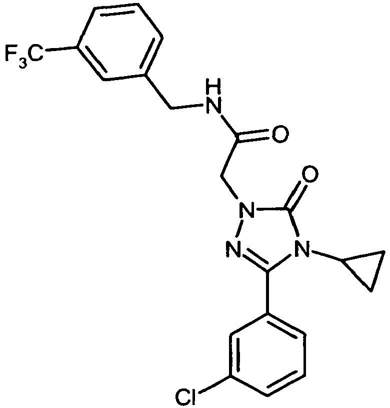

30.0 mg (0.102 mmol) [3-(3-Chlorphenyl)-4-cyclopropyl-5-oxo-4,5-dihydro-1*H*-1,2,4-triazol-1-yl]-essigsäure aus Beispiel 104A, 19.7 mg (0.112 mmol) 3-Trifluormethylbenzylamin und 16.6 mg (0.123 mmol) HOBt werden in 0.75 ml Dimethylformamid vorgelegt und mit 25.5 mg (0.133 mmol) EDC-Hydrochlorid versetzt. Man rührt über Nacht bei Raumtemperatur und reinigt die Reaktionslösung direkt durch präparative HPLC [Methode 9]. Man erhält so 20 mg (43% d. Th.) der Zielverbindung.
LC/MS [Methode 7]: Rₜ = 2.27 min
¹H-NMR (400 MHz, DMSO-d₆): δ = 0.53 - 0.68 (m, 2H), 0.83 - 0.98 (m, 2H), 3.22 (dddd, 1H), 4.41 (d, 2H), 4.46 (s, 2H), 7.53 - 7.64 (m, 6H), 7.74 - 7.83 (m, 2H), 8.66 (t, 1H).

Analog werden erhalten:

| **Beispiel Nr.** | **Struktur** | **LC/MS Rₜ [Methode]** |
|---|---|---|
| **138** | 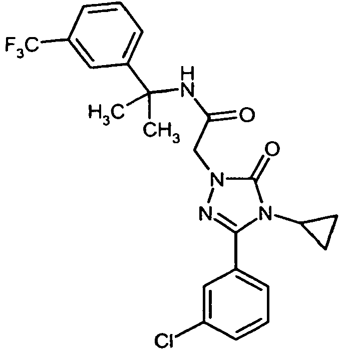 | Rₜ = 2.42 min [7] |
| **139** | 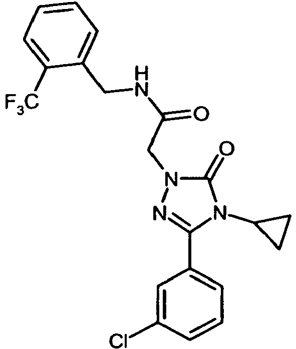 | Rₜ = 2.25 min [7] |
| **140** | 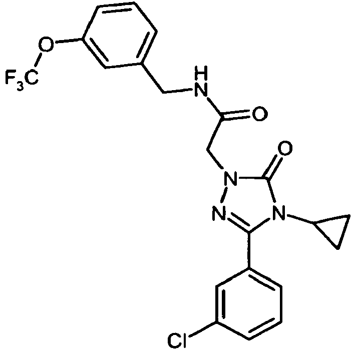 | Rₜ = 2.32 min [7] |

### Beispiel 141

### 2-[3-(4-Chlorphenyl)-4-(3-fluorphenylmethyl)-5-oxo-4,5-dihydro-1H-1,2,4-triazol-1-yl]-N-[3-(trifluormethyl)phenylmethyl]essigsäureamid

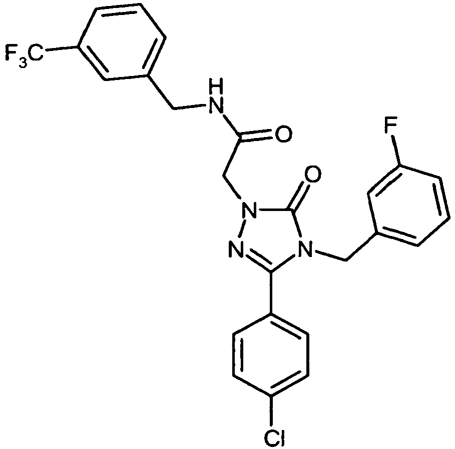

200 mg (0.66 mmol) 5-(4-Chlorphenyl)-4-(3-fluorphenylmethyl)-2,4-dihydro-3*H*-1,2,4-triazol-3-on aus Beispiel 41A, 193 mg (0.69 mmol) 2-Chlor-*N*-[3-(trifluormethyl)phenylmethyl]essigsäureamid [herstellbar gemäß EP 0 163 607, Beispiel 28] und 182 mg (1.32 mmol) Kaliumcarbonat werden in 2.5 ml Acetonitril suspendiert und über Nacht zum Rückfluss erhitzt. Man verdünnt nach dem Abkühlen mit Wasser, reinigt über präparative HPLC [Methode 9] und erhält so 219 mg (64% d. Th.) der Zielverbindung.
LC/MS [Methode 7]: Rₜ = 2.57 min
¹H-NMR (400 MHz, DMSO-d₆): δ = 4.44 (d, 2H), 4.58 (s, 2H), 5.01 (s, 2H), 6.93 - 7.01 (m, 2H), 7.09 (td, 1H), 7.36 (dt, 1H), 7.49 - 7.67 (m, 8H), 8.76 (t, 1H).

### Analog werden erhalten:

| **Beispiel Nr.** | **Struktur** | **LC/MS Rₜ [Methode]** | **¹H-NMR (400 MHz, DMSO-d₆)** |
|---|---|---|---|
| **142** | 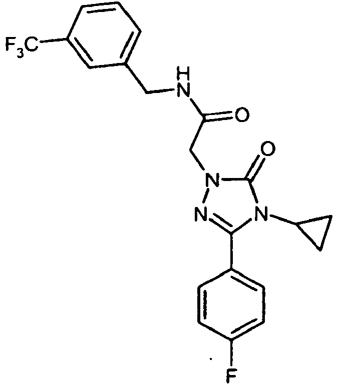 | Rₜ = 2.16 min [7] | δ = 0.52 - 0.66 (m, 2H), 0.81 - 0.96 (m, 2H), 3.17 (dddd, 1H), 4.41 (d, 2H), 4.44 (s, 2H), 7.37 (t, 2H), 7.52 - 7.66 (m, 4H), 7.79 -7.88 (m, 2H), 8.65 (t, 1H). |
| **143** | 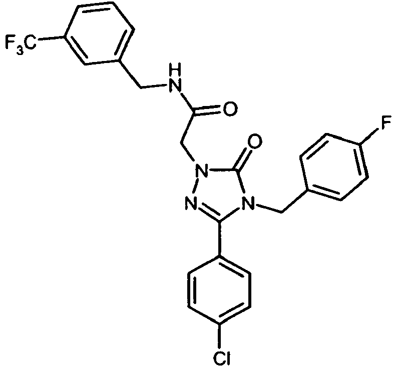 | Rₜ = 2.58 min [7] | δ = 4.43 (d, 2H), 4.57 (s, 2H), 4.98 (s, 2H), 7.09 -7.20 (m, 4H), 7.49 - 7.67 (m, 8H), 8.75 (t, 1H). |
| **144** | 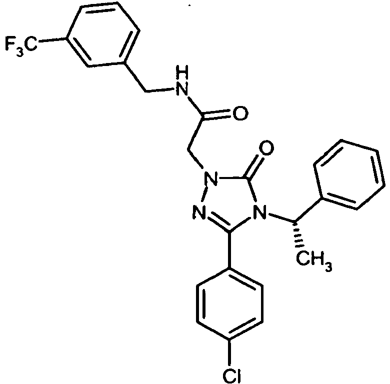 | Rₜ = 2.82 min [8] | δ = 1.78 (d, 3H), 4.43 (d, 2H), 4.50 (s, 2H), 5.26 (q, 1H), 7.24 - 7.39 (m, 7H), 7.51 - 7.66 (m, 6H), 8.73 (t, 1H). |
| **145** | 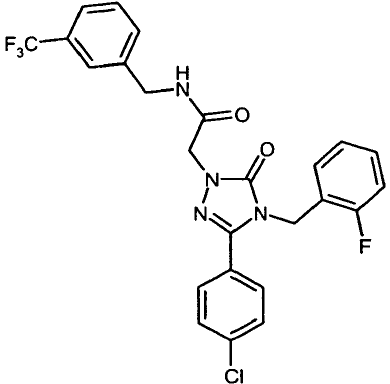 | Rₜ = 2.55 min [7] | δ = 4.43 (d, 2H), 4.56 (s, 2H), 5.03 (s, 2H), 7.06 -7.19 (m, 3H), 7.26 - 7.35 (m, 1H), 7.51 - 7.66 (m, 8H), 8.75 (t, 1H). |

Auf analoge Weise werden weiter erhalten:

| **Beispiel Nr.** | **Struktur** | **LC/MS Rₜ [Methode]** |
|---|---|---|
| **146** | 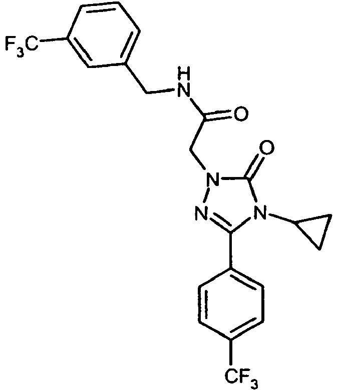 | Rₜ = 2.53 min [5] |
| **147** | 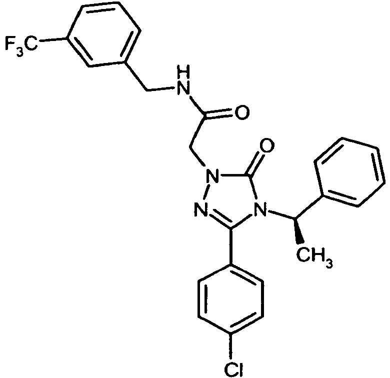 | Rₜ = 2.65 min [7] |
| **148** | 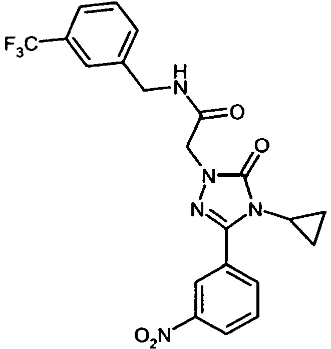 | Rₜ = 2.27 min [5] |
| **149** | 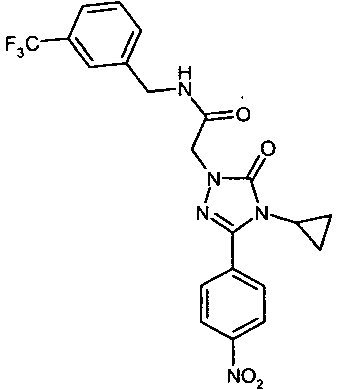 | Rₜ = 2.29 min [5] |
| **150** | 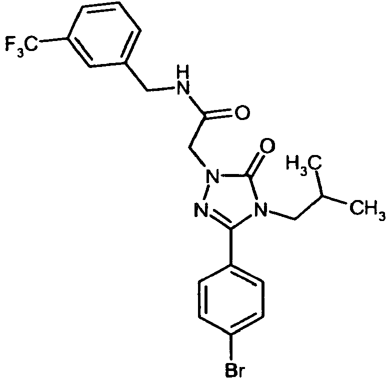 | Rₜ = 2.69 min [5] |
| **151** | 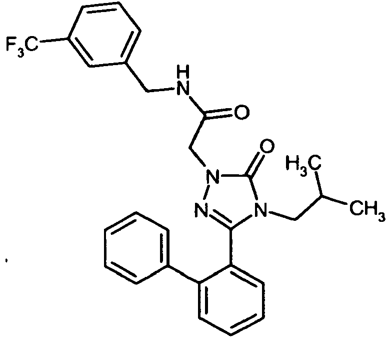 | Rₜ = 2.77 min [5] |
| **152** | 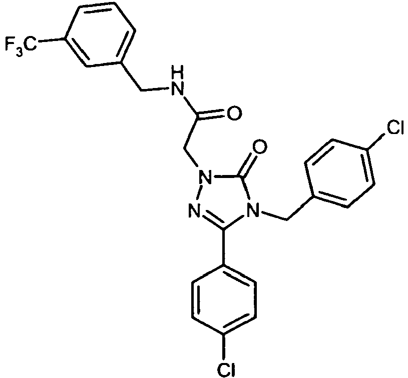 | Rₜ = 2.86 min [8] |

### Beispiel 153

### 2-[3-(4-Chlorphenyl)-4-(4-methoxyphenylmethyl)-5-oxo-4,5-dihydro-1H-1,2,4-triazol-1-yl]-N-[3-(trifluormethyl)phenylmethyl]essigsäureamid

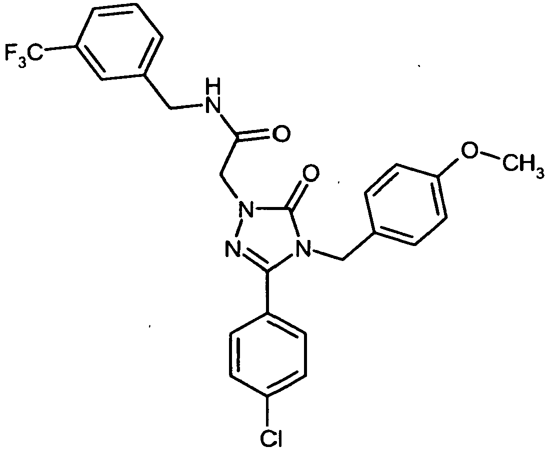

1.00 g (3.17 mmol) 5-(4-Chlorphenyl)-4-(4-methoxyphenylmethyl)-2,4-dihydro-3*H*-1,2,4-triazol-3-on aus Beispiel 55A, 0.80 g (3.17 mmol) 2-Chlor-*N*-[3-(trifluormethyl)phenylmethyl]essigsäureamid und 0.88 g (6.33 mmol) Kaliumcarbonat werden in 20 ml Acetonitril suspendiert und für 8 h zum Rückfluss erhitzt. Das Gemisch wird danach im Vakuum eingeengt, der Rückstand in Wasser aufgenommen und dreimal mit Essigsäureethylester extrahiert. Die vereinigten organischen Phasen werden eingeengt und der Rückstand durch präparative HPLC gereinigt [Methode 12]. Man erhält so 1.07 g (64% d. Th.) der Zielverbindung.
LC/MS [Methode 5]: Rₜ = 2.67 min
¹H-NMR (400 MHz, DMSO-d₆): δ = 3.70 (s, 3H), 4.43 (d, 2H), 4.6 (s, 2H), 4.92 (s, 2H), 6.83, 6.86 (AA'-Teil eines AA'BB'-Systems, 2H), 7.02, 7.04 (BB'-Teil eines AA'BB'-Systems, 2H), 7.51 - 7.66 (m, 8H), 8.75 (t, 1H).

### Analog werden erhalten:

| **Beispiel Nr.** | **Struktur** | **LC/MS Rₜ [Methode]** | **¹H-NMR (400 MHz, DMSO-d₆)** |
|---|---|---|---|
| **154** | 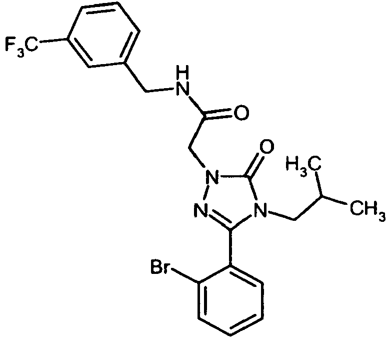 | Rₜ = 2.42 min [7] | δ = 0.69 (d, 2H), 1.59 -1.75 (m, 1H), 3.30 (d, 2H), 4.42 (d, 2H), 4.51 (s, 2H), 7.50 -7.65 (m, 7H), 7.83 (br. d, 1H), 8.69 (t, 1H). |
| **155** | 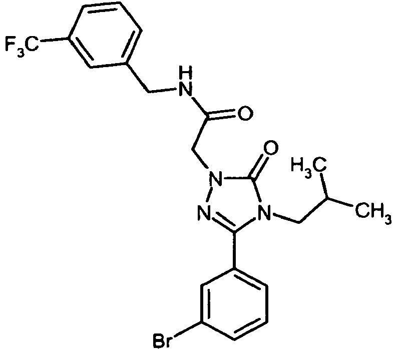 | Rₜ = 2.80 min [8] | δ = 0.71 (d, 6H), 1.62 -1.76 (m, 1H), 3.62 (d, 2H), 4.42 (d, 2H), 4.51 (s, 2H), 7.51 (t, 1H), 7.52 - 7.65 (m, 4H), 7.69 (d, 1H), 7.77 (d, 1H), 7.83 (br. s, 1H), 8.69 (t, 1H). |

Eine Bibliothek weiterer Ausführungsbeispiele wird parallelsynthetisch wie folgt hergestellt:

0.12 mmol des entsprechenden Amins und 0.10 mmol der entsprechenden Triazolylessigsäure werden in 0.6 ml Dimethylsulfoxid gelöst, mit 25.8 mg (0.2 mmol) *N*,*N*-Diisopropylethylamin und 41.7 mg (0.130 mmol) TBTU versetzt und über Nacht bei Raumtemperatur geschüttelt. Die Reaktionslösung wird filtriert und das Filtrat mittels präparativer LC/MS [Methode 6] aufgereinigt. Auf diese Weise werden erhalten:

| **Beispiel Nr.** | **Struktur** | **LC/MS [Methode 6]** |
|---|---|---|
| **156** | 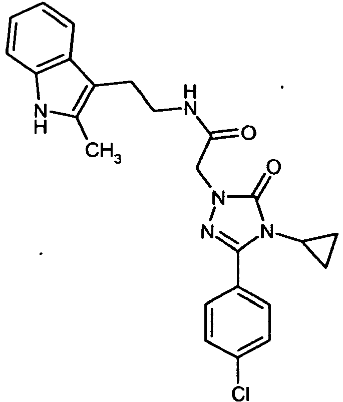 | Rₜ = 2.01 min; MS [ESIpos]: m/z = 450 (M+H)⁺ |
| **157** | 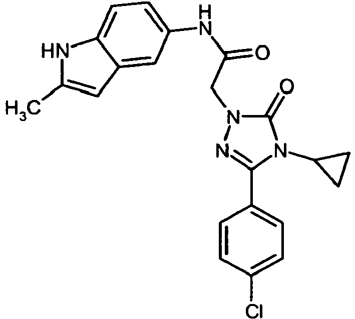 | Rₜ = 1.93 min; MS [ESIpos]: m/z = 422 (M+H)⁺ |
| **158** | 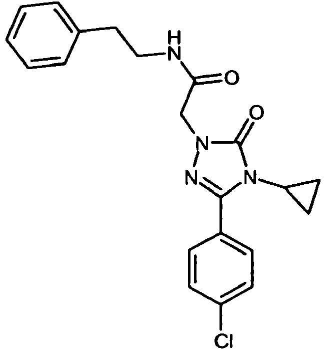 | Rₜ = 2.00 min; MS [ESIpos]: m/z = 397 (M+H)⁺ |
| **159** | 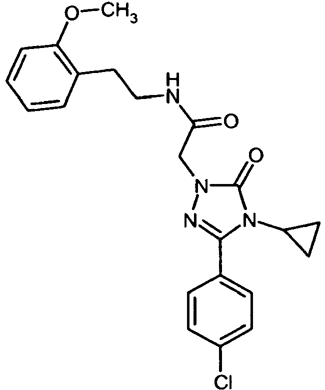 | Rₜ = 2.02 min; MS [ESIpos]: m/z = 427 (M+H)⁺ |
| **160** | 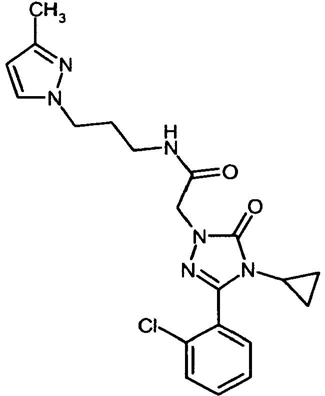 | Rₜ = 1.73 min; MS [ESIpos]: m/z = 415 (M+H)⁺ |
| **161** | 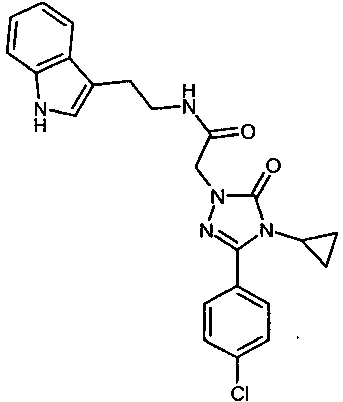 | Rₜ = 1.97 min; MS [ESIpos]: m/z = 436 (M+H)⁺ |
| **162** | 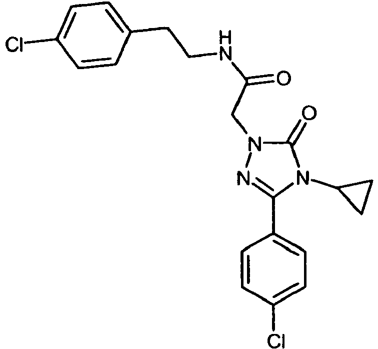 | Rₜ = 2.11 min; MS [ESIpos]: m/z = 431 (M+H)⁺ |
| **163** | 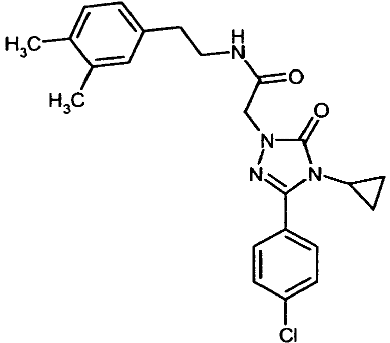 | Rₜ = 2.14 min; MS [ESIpos]: m/z = 425 (M+H)⁺ |
| **164** | 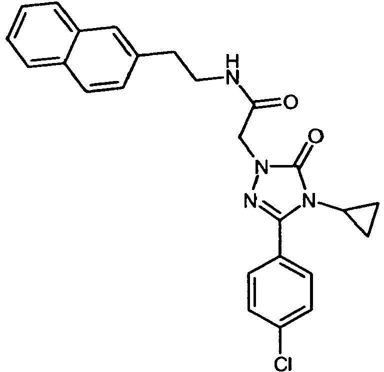 | Rₜ = 2.15 min; MS [ESIpos]: m/z = 447 (M+H)⁺ |
| **165** | 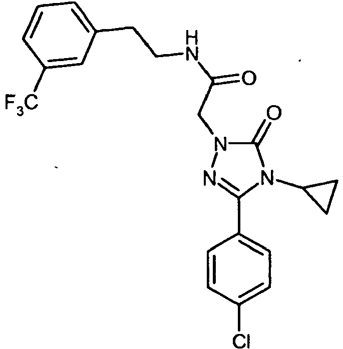 | Rₜ = 2.12 min; MS [ESIpos]: m/z = 465 (M+H)⁺ |
| **166** | 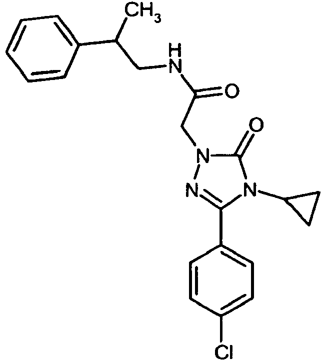 | Rₜ = 2.06 min; MS [ESIpos]: m/z = 411 (M+H)⁺ |
| **167** | 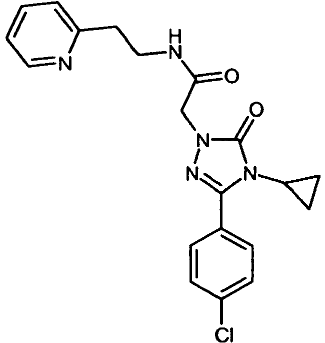 | Rₜ = 1.35 min; MS [ESIpos]: m/z = 398 (M+H)⁺ |
| **168** | 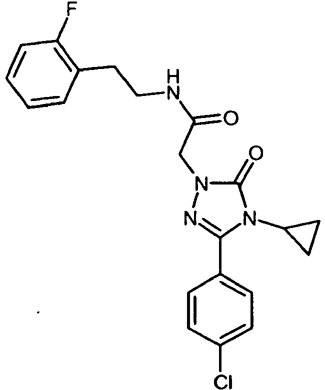 | Rₜ = 2.02 min; MS [ESIpos]: m/z = 415 (M+H)⁺ |
| **169** | 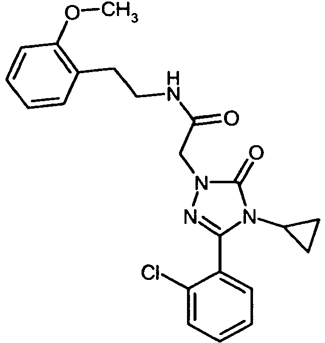 | Rₜ = 1.97 min; MS [ESIpos]: m/z = 427 (M+H)⁺ |
| **170** | 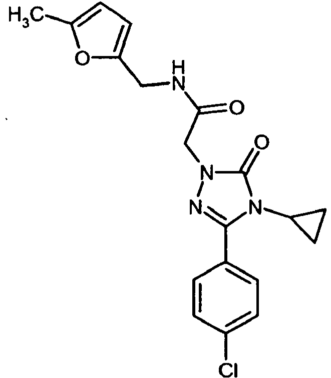 | Rₜ = 1.93 min; MS [ESIpos]: m/z = 387 (M+H)⁺ |
| **171** | 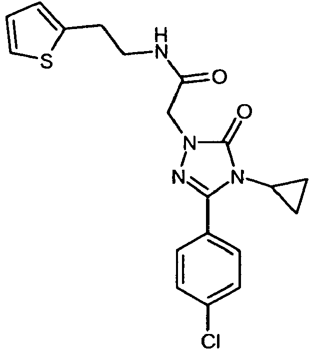 | Rₜ = 1.97 min; MS [ESIpos]: m/z = 403 (M+H)⁺ |
| **172** | 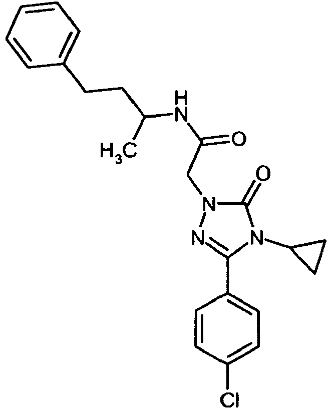 | Rₜ = 2.11 min; MS [ESIpos]: m/z = 425 (M+H)⁺ |
| **173** | 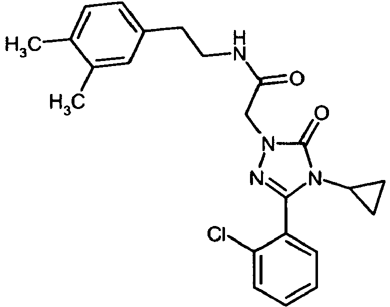 | Rₜ = 2.08 min; MS [ESIpos]: m/z = 425 (M+H)⁺ |
| **174** | 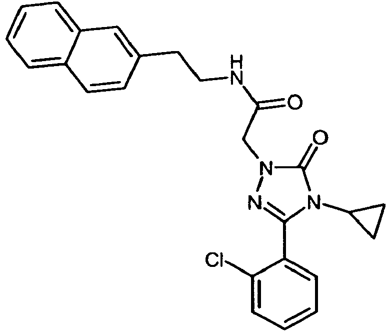 | Rₜ = 2.09 min; MS [ESIpos]: m/z = 447 (M+H)⁺ |
| **175** | 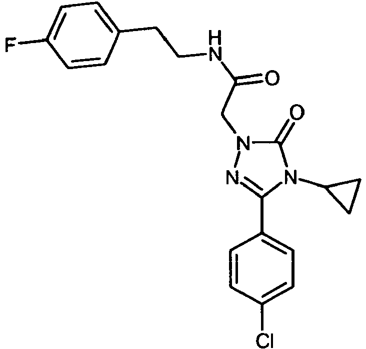 | Rₜ = 2.01 min; MS [ESIpos]: m/z = 415 (M+H)⁺ |
| **176** | 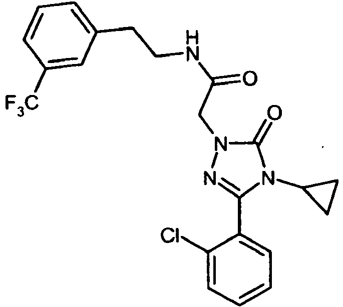 | Rₜ = 2.07 min; MS [ESIpos]: m/z = 465 (M+H)⁺ |
| **177** | 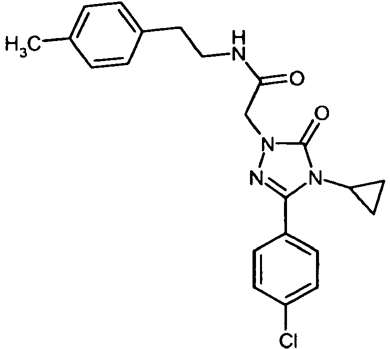 | Rₜ = 2.08 min; MS [ESIpos]: m/z = 411 (M+H)⁺ |
| **178** | 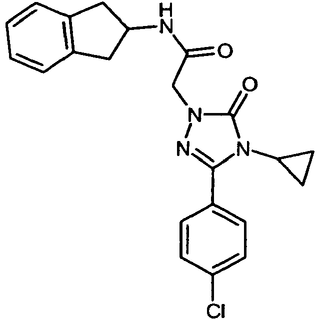 | Rₜ = 2.03 min; MS [ESIpos]: m/z = 409 (M+H)⁺ |
| **179** | 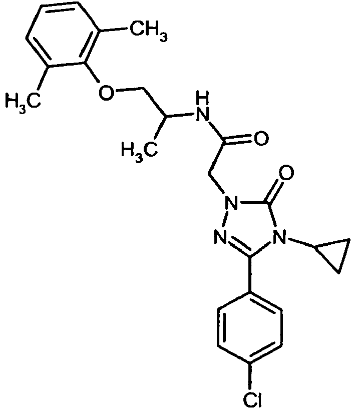 | Rₜ = 2.18 min; MS [ESIpos]: m/z = 455 (M+H)⁺ |
| **180** | 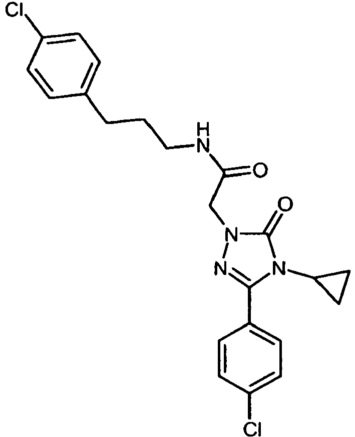 | Rₜ = 2.14 min; MS [ESIpos]: m/z = 445 (M+H)⁺ |
| **181** | 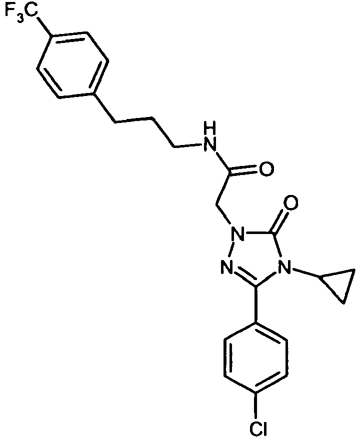 | Rₜ = 2.19 min; MS [ESIpos]: m/z = 479 (M+H)⁺ |
| **182** | 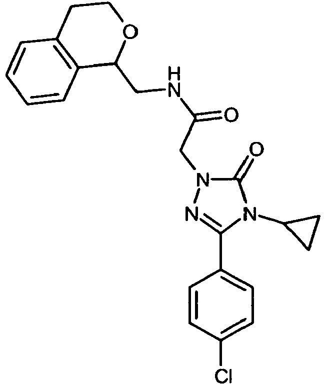 | Rₜ = 2.00 min; MS [ESIpos]: m/z = 439 (M+H)⁺ |
| **183** | 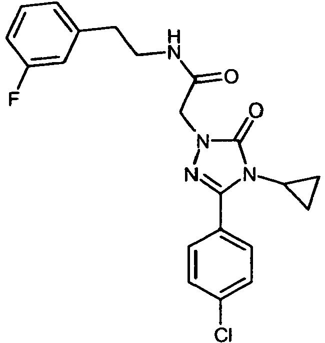 | Rₜ = 2.03 min; MS [ESIpos]: m/z = 415 (M+H)⁺ |
| **184** | 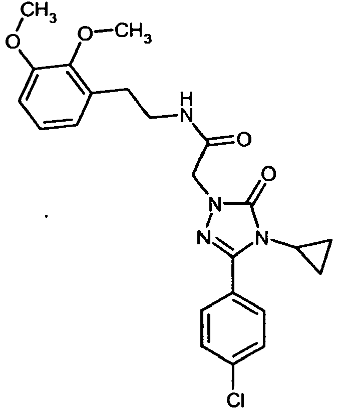 | Rₜ = 2.02 min; MS [ESIpos]: m/z = 457 (M+H)⁺ |
| **185** | 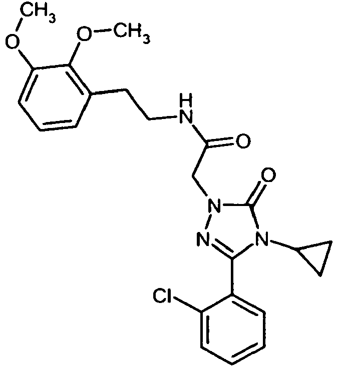 | Rₜ = 1.94 min; MS [ESIpos]: m/z = 457 (M+H)⁺ |

### Beispiel 186

### 2-[3-(4-Chlorphenyl)-4-cyclopropyl-5-oxo4,5-dihydro-1H-1,2,4-triazol-1-yl]-N-[(1S)-1-(1-naphthyl)ethyl]propionsäureamid (Diastereomer A)

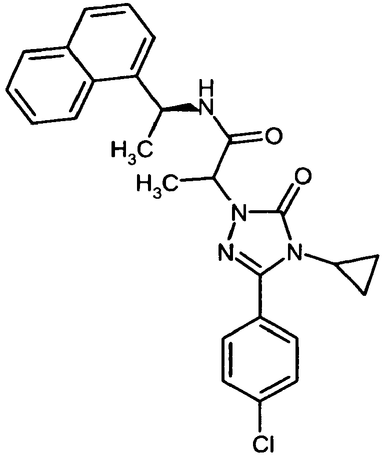

100.0 mg (0.325 mmol) 2-[3-(4-Chlorphenyl)-4-cyclopropyl-5-oxo-4,5-dihydro-1*H*-1,2,4-triazol-1-yl]-propionsäure aus Beispiel 105A werden in 2 ml DMF vorgelegt und mit 61.2 mg (0.357 mmol) (1*S*)-1-(1-Naphthyl)ethanamin, 52.7 mg (0.390 mmol) HOBt sowie 81.0 mg (0.422 mmol) EDC-Hydrochlorid versetzt. Nach Rühren des Gemisches über Nacht bei Raumtemperatur wird zwischen Dichlormethan und Wasser verteilt, die organische Phase abgetrennt, über Natriumsulfat getrocknet und eingeengt. Der Rückstand wird durch Flash-Chromatographie an Kieselgel (Eluent: zunächst Dichlormethan, dann Dichlormethan/Methanol 100:1) gereinigt und dann mittels präparativer HPLC an chiraler Phase [Methode 15] weiter aufgetrennt. Man erhält so 52 mg (35% d. Th.) der diastereomerenreinen Zielverbindung (siehe auch Beispiel 187).
MS [ESIpos]: m/z = 461 (M+H)⁺
HPLC [Methode 2]: Rₜ = 4.84 min
chirale HPLC [Methode 15]: Rₜ = 2.49 min
¹H-NMR (400 MHz, DMSO-d₆): δ = 0.52 (m, 2H), 0.87 (m, 2H), 1.50 (d, 3H), 1.53 (d, 3H), 3.13 (tt, 1H), 4.80 (q, 1H), 5.68 (dq, 1H), 7.43 (t, 1H), 7.48-7.61 (m, 5H), 7.74 (d, 2H), 7.82 (d, 1H), 7.93 (m, 1H), 8.05 (m, 1H), 8.54 (d, 1H).

### Beispiel 187

### 2-[3-(4-Chlorphenyl)-4-cyclopropyl-5-oxo-4,5-dihydro-1H-1,2,4-triazol-1-yl]-N-[(1S)-1-(1-naphthyl)ethyl]propionsäureamid (Diastereomer B)

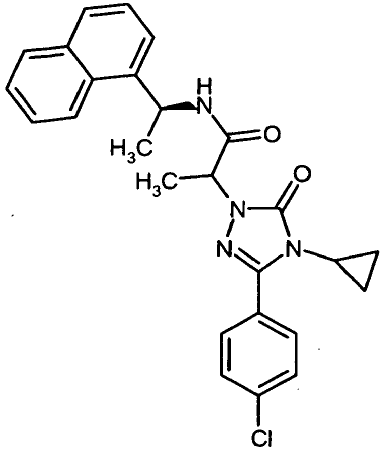

100.0 mg (0.325 mmol) 2-[3-(4-Chlorphenyl)-4-cyclopropyl-5-oxo-4,5-dihydro-1*H*-1,2,4-triazol-1-yl]-propionsäure aus Beispiel 105A werden in 2 ml DMF vorgelegt und mit 61.2 mg (0.357 mmol) (1*S*)-1-(1-Naphthyl)ethanamin, 52.7 mg (0.390 mmol) HOBt sowie 81.0 mg (0.422 mmol) EDC-Hydrochlorid versetzt. Nach Rühren des Gemisches über Nacht bei Raumtemperatur wird zwischen Dichlormethan und Wasser verteilt, die organische Phase abgetrennt, über Natriumsulfat getrocknet und eingeengt. Der Rückstand wird durch Flash-Chromatographie an Kieselgel (Eluent: zunächst Dichlormethan, dann Dichlonnethan/Methanol 100:1) gereinigt und dann mittels präparativer HPLC an chiraler Phase [Methode 15] weiter aufgetrennt. Man erhält so 51 mg (34% d. Th.) der diastereomerenreinen Zielverbindung (siehe auch Beispiel 186).
MS [ESIpos]: m/z = 461 (M+H)⁺
HPLC [Methode 2]: Rₜ = 4.84 min
chirale HPLC [Methode 15]: Rₜ = 5.03 min
¹H-NMR (400 MHz, DMSO-d₆): δ = 0.57 (m, 2H), 0.89 (m, 2H), 1.51 (br. d, 6H), 3.16 (tt, 1H), 4.78 (q, 1H), 5.67 (dq, 1H), 7.46-7.61 (m, 6H), 7.78 (d, 2H), 7.83 (d, 1H), 7.94 (br. d, 1H), 8.06 (br. d, 1H), 8.60 (d, 1H).

### Beispiel 188

### 2-[3-(4-Chlorphenyl)-4-cyclopropyl-5-oxo-4,5-dihydro-1H-1,2,4-triazol-1-yl]-N-[(1R)-1-(1-naphthyl)ethyl]propionsäureamid (Diastereomer A)

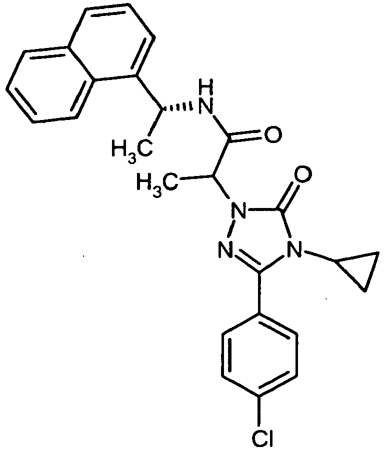

100.0 mg (0.325 mmol) 2-[3-(4-Chlorphenyl)-4-cyclopropyl-5-oxo-4,5-dihydro-1*H*-1,2,4-triazol-1-yl]-propionsäure aus Beispiel 105A werden in 2 ml DMF vorgelegt und mit 61.2 mg (0.357 mmol) (1R)-1-(1-Naphthyl)ethanamin, 52.7 mg (0.390 mmol) HOBt sowie 81.0 mg (0.422 mmol) EDC-Hydrochlorid versetzt. Nach Rühren des Gemisches über Nacht bei Raumtemperatur wird zwischen Dichlormethan und Wasser verteilt, die organische Phase abgetrennt, über Natriumsulfat getrocknet und eingeengt. Der Rückstand wird durch Flash-Chromatographie an Kieselgel (Eluent: zunächst Dichlormethan, dann Dichlormethan/Methanol 100:1) gereinigt und dann mittels präparativer HPLC an chiraler Phase [Methode 16] weiter aufgetrennt. Man erhält so 44 mg (29% d. Th.) der diastereomerenreinen Zielverbindung (siehe auch Beispiel 189).
MS [ESIpos]: m/z = 461 (M+H)⁺
HPLC [Methode 2]: Rₜ = 4.84 min
chirale HPLC [Methode 16]: Rₜ = 2.43 min
¹H-NMR (400 MHz, DMSO-d₆): δ = 0.52 (m, 2H), 0.87 (m, 2H), 1.50 (d, 3H), 1.53 (d, 3H), 3.13 (tt, 1H), 4.80 (q, 1H), 5.68 (dq, 1H), 7.43 (t, 1H), 7.47-7.61 (m, 5H), 7.74 (d, 2H), 7.82 (d, 1H), 7.93 (m, 1H), 8.05 (m, 1H), 8.53 (d, 1H).

### Beispiel 189

### 2-[3-(4-Chlorphenyl)-4-cyclopropyl-5-oxo-4,5-dihydro-1H-1,2,4-triazol-1-yl]-N-[(1R)-1-(1-naphthyl)ethyl]propionsäureamid (Diastereomer B)

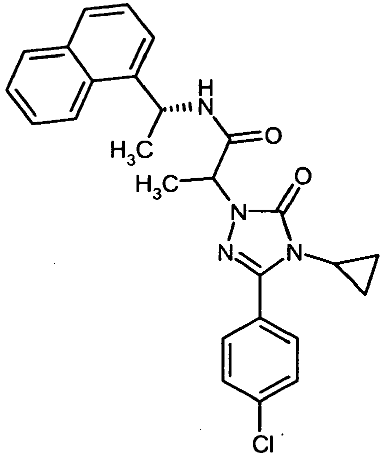

100.0 mg (0.325 mmol) 2-[3-(4-Chlorphenyl)-4-cyclopropyl-5-oxo-4,5-dihydro-1*H*-1,2,4-triazol-1-yl]-propionsäure aus Beispiel 105A werden in 2 ml DMF vorgelegt und mit 61.2 mg (0.357 mmol) (1R)-1-(1-Naphthyl)ethanamin, 52.7 mg (0.390 mmol) HOBt sowie 81.0 mg (0.422 mmol) EDC-Hydrochlorid versetzt. Nach Rühren des Gemisches über Nacht bei Raumtemperatur wird zwischen Dichlormethan und Wasser verteilt, die organische Phase abgetrennt, über Natriumsulfat getrocknet und eingeengt. Der Rückstand wird durch Flash-Chromatographie an Kieselgel (Eluent: zunächst Dichlormethan, dann Dichlormethan/Methanol 100:1) gereinigt und dann mittels präparativer HPLC an chiraler Phase [Methode 16] weiter aufgetrennt. Man erhält so 58 mg (39% d. Th.) der diastereomerenreinen Zielverbindung (siehe auch Beispiel 188).
MS [ESIpos]: m/z = 461 (M+H)⁺
HPLC [Methode 2]: Rₜ = 4.84 min
chirale HPLC [Methode 16]: Rₜ = 6.14 min
¹H-NMR (400 MHz, DMSO-d₆): δ = 0.57 (m, 2H), 0.89 (m, 2H), 1.51 (br. d, 6H), 3.16 (tt, 1H), 4.77 (q, 1H), 5.67 (dq, 1H), 7.46-7.61 (m, 6H), 7.78 (d, 2H), 7.83 (br. d, 1H), 7.94 (br. d, 1H), 8.06 (br. d, 1H), 8.60 (d, 1H).

### Beispiel 190

### rac-2-(3-Cyclopropyl-2-oxo-4-phenyl-2,3-dihydro-1H-imidazol-1-yl)-N-[1-(2-naphthyl)ethyl]-essigsäureamid

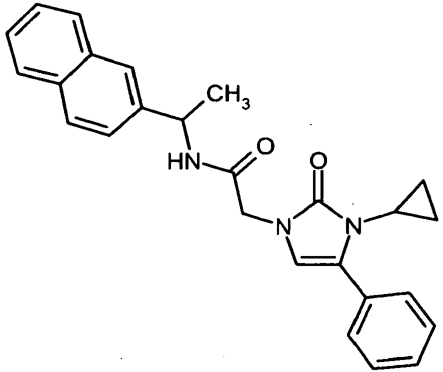

40 mg (0.155 mmol) (3-Cyclopropyl-2-oxo-4-phenyl-2,3-dihydro-1*H-*imidazol-1-yl)-essigsäure aus Beispiel 128A, 29.2 mg (0.17 mmol) 1-(2-Naphthyl)ethylamin, 38.6 mg (0.20 mmol) EDC-Hydrochlorid sowie 25.1 mg (0.19 mmol) HOBt werden in 1.5 ml trockenem DMF gelöst und über Nacht bei RT gerührt. Das Rohprodukt wird durch präparative HPLC [Methode 10, mit 0.01 M Salzsäure-Zusatz im Wasser] gereinigt. Man erhält die Zielverbindung mit einer Ausbeute von 41 mg (63% d. Th.).
HPLC [Methode 2]: Rₜ = 4.44 min
MS [ESIpos]: m/z = 412 (M+H)⁺
¹H-NMR (400 MHz, DMSO-d₆): δ = 0.46 (m, 2H), 0.78 (m, 2H), 1.47 (d, 3H), 3.00 (m, 1H), 4.28 (s, 2H), 5.08 (m, 1H), 6.65 (s, 1H), 7.25-7.54 (m, 8H), 7.77-7.93 (m, 4H), 8.70 (d, 1H).

### Beispiel 191

### rac-2-[4-(4-Bromphenyl)-3-cyclopropyl-2-oxo-2,3-dihydro-1H-imidazol-1-yl]-N-{1-[3-(trifluormethyl)phenyl]ethyl}essigsäureamid.

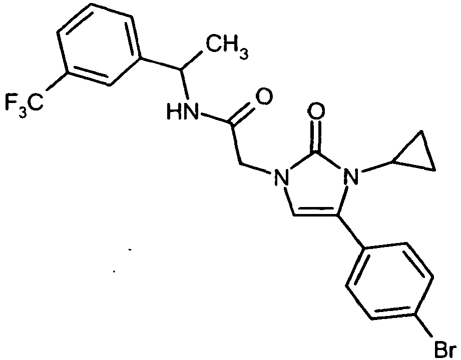

40.0 mg (0.119 mmol) 4-(4-Bromphenyl)-3-cyclopropyl-2-oxo-2,3-dihydro-1*H*-imidazol-1-yl]-essigsäure aus Beispiel 129A, 24.7 mg (0.130 mmol) 1-[3-(Trifluormethyl)phenyl]ethylamin und 19.2 mg (0.142 mmol) HOBt werden in 1.5 ml Dimethylformamid vorgelegt und mit 29.6 mg (0.154 mmol) EDC-Hydrochlorid versetzt. Man rührt über Nacht bei Raumtemperatur, verrührt dann mit 15 ml Wasser, isoliert den entstandenen Niederschlag und reinigt dieses Rohprodukt durch präparative HPLC [Methode 13]. Man erhält so 23 mg (38% d. Th.) der Zielverbindung.
LC/MS [Methode 7]: Rₜ = 2.43 min
¹H-NMR (400 MHz, DMSO-d₆): δ = 0.39 - 0.53 (m, 2H), 0.73 - 0.88 (m, 2H), 1.39 (d, 3H), 3.00 (dddd, 1H), 4.25 (Zentrum eines AB-Systems, 2H), 4.99 (dq, 1H), 6.72 (s, 1H), 7.44, 7.46 (AA'-Teil eines AABB'-Systems, 2H), 7.52 - 7.68 (m, 4H), 7.57, 7.59 (BB'-Teil eines AABB'-Systems, 2H), 8.71 (d, 1H).

### Analog werden erhalten:

| **Beispiel Nr.** | **Struktur** | **LC/MS Rₜ [Methode]** | **¹H-NMR (400 MHz, DMSO-d₆)** |
|---|---|---|---|
| **192** | 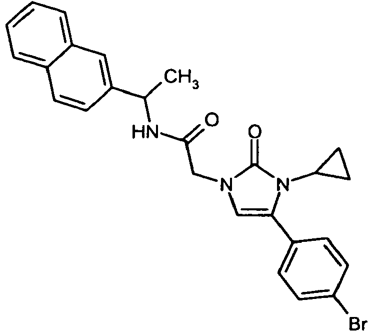 | Rₜ = 2.42 min [7] | δ = 0.42 - 0.50 (m, 2H), 0.77 - 0.84 (m, 2H), 1.47 (d, 3H), 3.00 (dddd, 1H), 4.27 (s, 2H), 5.07 (dq, 1H), 6.73 (s, 1H), 7.42 - 7.60 (m, 7H), 7.81 (br. s, 1H), 7.83 - 7.91 (m, 3H), 8.70 (d, 1H). |
| **193** | 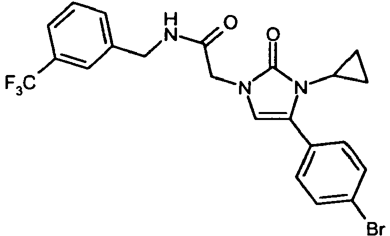 | Rₜ = 2.34 min [7] | δ = 0.45 - 0.52 (m, 2H), 0.78 - 0.85 (m, 2H), 3.02 (dddd, 1 H), 4.27 (s, 2H), 4.39 (d, 2H), 6.78 (s, 1H), 7.46, 7.48 (AA'-Teil eines AA'BB'-Systems, 2H), 7.54 - 7.64 (m, 4H), 7.58, 7.60 (BB'-Teil eines AA'BB'-Systems, 2H), 8.66 (t, 1H). |

### Beispiel 194

### 2-[4-(4-Chlorphenyl)-3-cyclopropyl-2-oxo-2,3-dihydro-1H-imidazol-1-yl]-N-[3-(trifluormethyl)-phenylmethyl]essigsäureamid

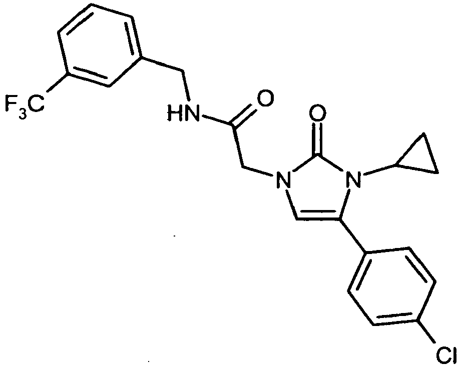

50.0 mg (0.171 mmol) 4-(4-Chlorphenyl)-3-cyclopropyl-2-oxo-2,3-dihydro-1*H*-imidazol-1-yl]-essigsäure aus Beispiel 130A, 32.9 mg (0.188 mmol) 3-Trifluormethylbenzylamin und 27.7 mg (0.205 mmol) HOBt werden in 1.5 ml Dimethylformamid vorgelegt und mit 42.6 mg (0.222 mmol) EDC-Hydrochlorid versetzt. Man rührt über Nacht bei Raumtemperatur, verrührt dann mit 19 ml Wasser und gewinnt den entstehenden Niederschlag durch Filtration. Das Rohprodukt wird mit Wasser gewaschen und im Vakuum getrocknet. Man erhält so 65 mg (85% d. Th.) der Zielverbindung.
LC/MS [Methode 4]: Rₜ = 2.59 min
¹H-NMR (400 MHz, DMSO-d₆): δ = 0.42 - 0.55 (m, 2H), 0.73 - 0.88 (m, 2H), 3.02 (dddd, 1H), 4.27 (s, 2H), 4.39 (d, 2H), 6.76 (s, 1H), 7.43 - 7.66 (m, 8H), 8.66 (t, 1H).

### Beispiel 195

### rac-2-[4-(4-Chlorphenyl)-3-cyclopropyl-2-oxo-2,3-dihydro-1H-imidazol-1-yl]-N-{1-[3-(trifluormethyl)phenyl]ethyl}essigsäureamid

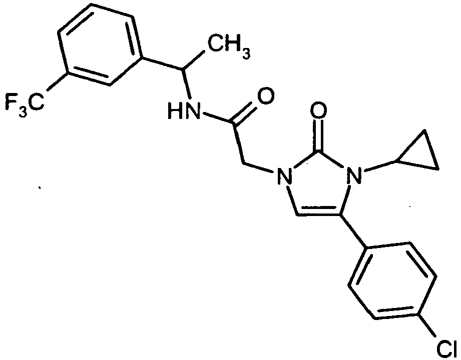

50.0 mg (0.171 mmol) 4-(4-Chlorphenyl)-3-cyciopropyl-2-oxo-2,3-dihydro-1*H*-imidazol-1-yl]-essigsäure aus Beispiel 130A, 35.5 mg (0.188 mmol) 1-[3-(Trifluormethyl)phenyl]ethylamin und 27.7 mg (0.205 mmol) HOBt werden in 1.5 ml Dimethylformamid vorgelegt und mit 42.6 mg (0.222 mmol) EDC-Hydrochlorid versetzt. Man rührt über Nacht bei Raumtemperatur, verrührt dann mit 19 ml Wasser und gewinnt den entstehenden Niederschlag durch Filtration. Das Rohprodukt wird mit Wasser gewaschen, im Vakuum getrocknet und durch präparative HPLC [Methode 9] gereinigt. Man erhält so 14 mg (18% d. Th.) der Zielverbindung.
LC/MS [Methode 7]: Rₜ = 2.38 min
¹H-NMR (400 MHz, DMSO-d₆): δ = 0.39 - 0.53 (m, 2H), 0.73 - 0.87 (m, 2H), 1.39 (d, 3H), 3.00 (dddd, 1H), 4.25 (Zentrum eines AB-Systems, 2H), 4.50 (dq, 1H), 6.72 (s, 1H), 7.44, 7.46 (AA'-Teil eines AA'BB'-Systems, 2H), 7.51, 7.53 (BB'-Teil eines AA'BB'-Systems, 2H), 7.54 - 7.69 (m, 4H), 8.71 (d, 1H).

### Analog wird erhalten:

### Beispiel 196

### rac-2-[4-(4-Chlorphenyl)-3-cyclopropyl-2-oxo-2,3-dihydro-1H-imidazol-1-yl]-N-[1-(2-naphthyl)-ethyl]essigsäureamid

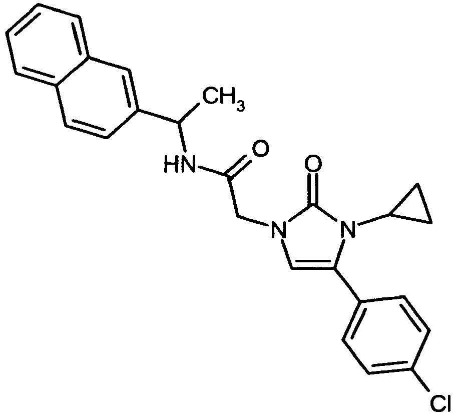

LC/MS [Methode 7]: Rₜ = 2.38 min
¹H-NMR (400 MHz, DMSO-d₆): δ = 0.39 - 0.53 (m, 2H), 0.73 - 0.88 (m, 2H), 1.47 (d, 3H), 3.01 (dddd, 1H), 4.27 (s, 2H), 5.07 (dq, 1H), 6.72 (s, 1H), 7.41 - 7.54 (m, 7H), 7.81 (s, 1H), 7.84 - 7.91 (m, 3H), 8.70 (d, 1H).

### Beispiel 197

### 2-[4-(4-Chlorphenyl)-3-cyclopropyl-2-oxo-2,3-dihydro-1H-imidazol-1-yl]-N-[(1S)-1-(1-naphthyl)-ethyl]essigsäureamid

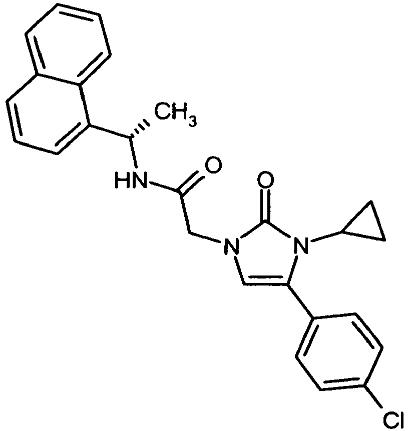

150 mg (0.512 mmol) [4-(4-Chlorphenyl)-3-cyclopropyl-2-oxo-2,3-dihydro-1*H*-imidazol-1-yl]-essigsäure aus Beispiel 130A, 96.5 mg (0.564 mmol) (1R)-1-(1-Naphthyl)ethylamin, 128 mg (0.67 mmol) EDC-Hydrochlorid und 83.1 mg (0.62 mmol) HOBt werden in 2 ml trockenem DMF gelöst und über Nacht bei Raumtemperatur gerührt. Das Rohprodukt wird durch Flash-Chromatographie an Kieselgel (Eluent: zunächst Dichlormethan, dann Dichlormethan/Methanol 100:1) aufgereinigt. Man erhält die Zielverbindung mit einer Ausbeute von 172 mg (75% d. Th.).
HPLC [Methode 2]: Rₜ = 4.78 min
MS [ESIpos]: m/z = 446 (M+H)⁺
¹H-NMR (400 MHz, DMSO-d₆): δ = 0.45 (m, 2H), 0.79 (m, 2H), 1.51 (d, 3H), 3.00 (m, 1H), 4.25 (d, 2H), 5.70 (m, 1H), 6.70 (s, 1H), 7.40-7.61 (m, 8H), 7.84 (d, 1H), 7.95 (d, 1H), 8.10 (d, 1H), 8.76 (d, 1H).

### Analog der Beispiele 190 und 197 werden erhalten:

| **Beispiel Nr.** | **Struktur** | **LC/MS oder HPLC, MS Rₜ [Methode]** | **¹H-NMR (400 MHz, DMSO-d₆)** |
|---|---|---|---|
| **198** | 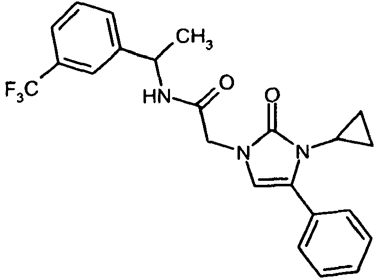 | MS [ESIpos]: m/z = 430 (M+H)⁺; [ESIneg]: m/z = 428 (M-H)⁻ Rₜ = 4.44 min [2] | δ = 0.46 (m, 2H), 0.77 (m, 2H), 1.40 (d, 3H), 3.00 (m, 1H), 4.25 (s, 2H), 5.00 (m, 1H), 6.63 (s, 1H), 7.26-7.73 (m, 9H), 8.70 (d, 1H). |
| **199** | 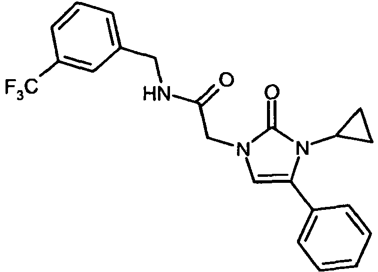 | MS [ESIpos]: m/z = 416 (M+H)⁺; [ESIneg]: m/z = 414 (M-H)⁻ Rₜ = 4.38 min [2] | δ = 0.49 (m, 2H), 0.78 (m, 2H), 3.02 (m, 1H), 4.28 (s, 2H), 4.40 (d, 2H), 6.69 (s, 1H), 7.26-7.65 (m, 9H), 8.66 (t, 1H). |
| **200** | 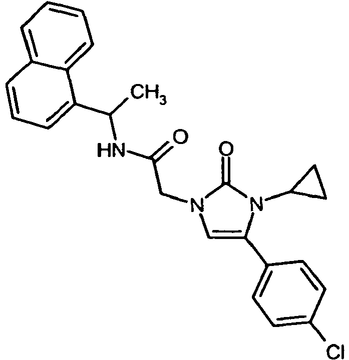 | MS [ESIpos]: m/z = 446 (M+H)⁺; [ESIneg]: m/z = 444 (M-H)⁻ Rₜ = 4.78 min [2] | δ = 0.45 (m, 2H), 0.79 (m, 2H), 1.51 (d, 3H), 3.00 (m, 1H), 4.26 (d, 2H), 5.70 (m, 1H), 6.69 (s, 1H), 7.40-7.60 (m, 8H), 7.84 (d, 1H), 7.94 (d, 1H), 8.10 (d, 1H), 8.75 (d, 1H). |
| **201** | 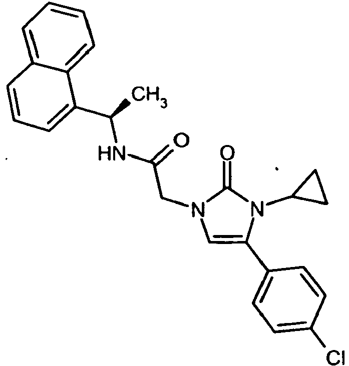 | MS [ESIpos]: m/z = 446 (M+H)⁺; [ESIneg]: m/z = 444 (M-H)⁻ Rₜ = 4.77 min [2] | δ = 0.45 (m, 2H), 0.79 (m, 2H), 1.52 (d, 3H), 3.00 (m, 1H), 4.26 (d, 2H), 5.70 (m, 1H), 6.70 (s, 1H), 7.40-7.60 (m, 8H), 7.84 (d, 1H), 7.94 (d, 1H), 8.11 (d, 1H), 8.76 (d, 1H). |
| **202** | 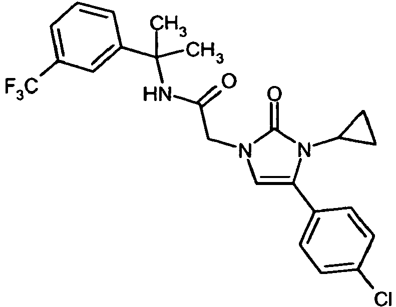 | MS [ESIpos]: m/z = 478 (M+H)⁺; [ESIneg] : m/z = 476 (M-H)⁻ Rₜ = 4.88 min [2] | δ = 0.44 (m, 2H), 0.79 (m, 2H), 1.58 (s, 6H), 2.99 (m, 1H), 4.25 (s, 2H), 6.65 (s, 1H), 7.41-7.56 (m, 6H), 7.60 (s, 1H), 7.66 (d, 1H), 8.54 (s, 1 H). |
| **203** | 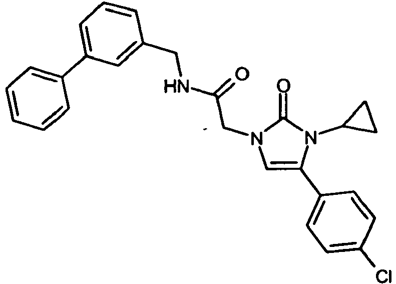 | MS [CI]: m/z = 458 (M+H)⁺ Rₜ = 4.84 min [2] | δ = 0.49 (m, 2H), 0.81 (m, 2H), 3.02 (m, 1H), 4.28 (s, 2H), 4.38 (d, 2H), 6.77 (s, 1H), 7.23-7.70 (m, 13H), 8.63 (t, 1H). |
| **204** | 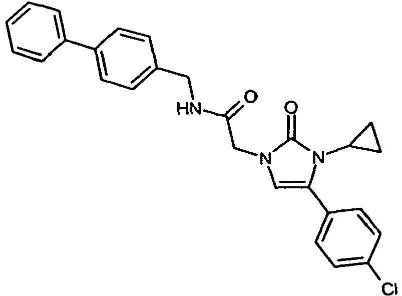 | MS [CI]: m/z = 458 (M+H)⁺ Rₜ = 4.84 min [2] | δ = 0.49 (m, 2H), 0.82 (m, 2H), 3.03 (m, 1H), 4.28 (s, 2H), 4.34 (d, 2H), 6.78 (s, 1H), 7.33-7.68 (m, 13H), 8.61 (t, 1H). |
| **205** | 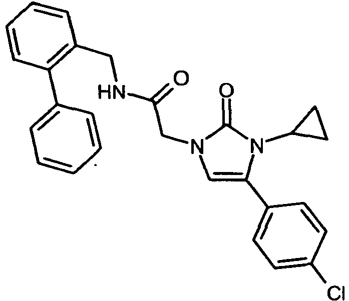 | Rₜ = 2.44 min [7] | δ = 0.47 (m, 2H), 0.81 (m, 2H), 3.01 (m, 1H), 4.17-4.27 (m, 4H), 6.72 (s, 1H), 7.22 (d, 1H), 7.31-7.57 (m, 12H), 8.50 (t, 1H). |
| **206** | 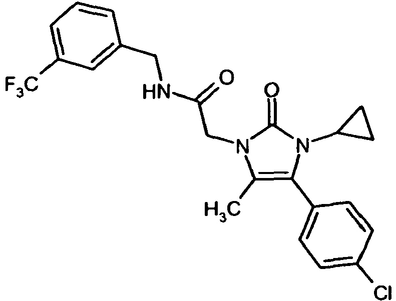 | MS [CI]: m/z = 464 (M+H)⁺ Rₜ = 4.78 min [2] | δ = 0.41 (m, 2H), 0.67 (m, 2H), 1.94 (s, 3H), 2.87 (m, 1H), 4.32 (s, 2H), 4.40 (d, 2H), 7.39-7.65 (m, 8H), 8.72 (t, 1H). |
| **207** | 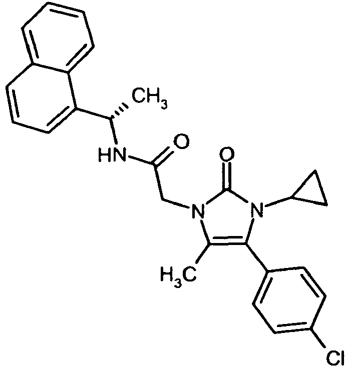 | MS [ESIpos]: m/z = 460 (M+H)⁺; [ESIneg]: m/z = 458 (M-H)⁻ Rₜ = 4.87 min [2] | δ = 0.37 (m, 2H), 0.66 (m, 2H), 1.52 (d, 3H), 1.91 (s, 3H), 2.86 (m, 1H), 4.29 (q, 2H), 5.71 (m, 1H), 7.37-7.60 (m, 8H), 7.84 (d, 1H), 7.95 (d, 1H), 8.09 (d, 1H), 8.78 (d, 1H). |
| **208** | 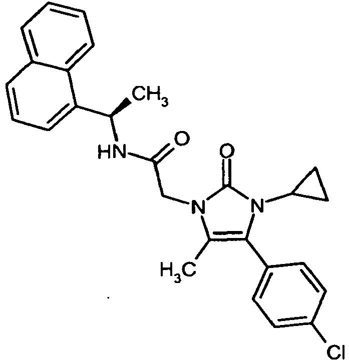 | MS [ESIpos]: m/z = 460 (M+H)⁺; [ESIneg]: m/z = 458 (M-H)⁻ Rₜ = 4.87 min [2] | δ = 0.37 (m, 2H), 0.66 (m, 2H), 1.52 (d, 3H), 1.90 (s, 3H), 2.86 (m, 1H), 4.29 (q, 2H), 5.71 (m, 1H), 7.37-7.60 (m, 8H), 7.84 (d, 1H), 7.95 (d, 1H), 8.09 (d, 1H), 8.78 (d, 1H). |
| **209** | 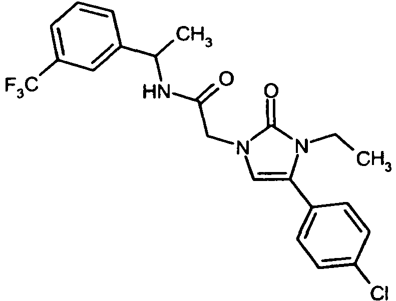 | MS [ESIpos]: m/z = 452 (M+H)⁺; [ESIneg]: m/z =450 (M-H)⁻ Rₜ = 4.78 min [2] | δ = 1.00 (s, 3H), 1.40 (d, 3H), 3.68 (q, 2H), 4.29 (s, 2H), 5.00 (m, 1H), 6.68 (s, 1H), 7.37-7.70 (m, 8H), 8.72 (d, 1H). |
| **210** | 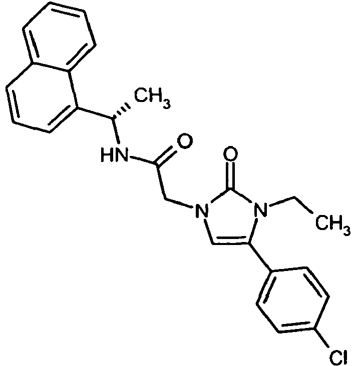 | MS [ESIpos]: m/z = 434 (M+H)⁺; [ESIneg]: m/z = 432 (M-H)⁻ Rₜ = 4.78 min [2] | δ = 1.00 (t, 3H), 1.52 (d, 3H), 3.67 (q, 2H), 4.30 (d, 2H), 5.71 (m, 1H), 6.67 (s, 1H), 7.37-7.60 (m, 8H), 7.84 (d, 1H), 7.95 (d, 1H), 8.10 (d, 1H), 8.78 (d, 1H). |
| **211** | 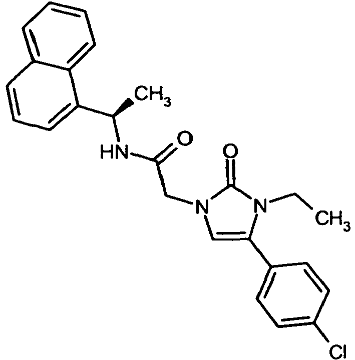 | MS [ESIpos]: m/z = 434 (M+H)⁺; [ESIneg]: m/z = 432 (M-H)⁻ Rₜ = 4.78 min [2] | δ = 1.00 (t, 3H), 1.52 (d, 3H), 3.67 (q, 2H), 4.30 (d, 2H), 5.71 (m, 1H), 6.67 (s, 1H), 7.36-7.61 (m, 8H), 7.84 (d, 1H), 7.94 (d, 1H), 8.10 (d, 1H), 8.78 (d, 1H). |
| **212** | 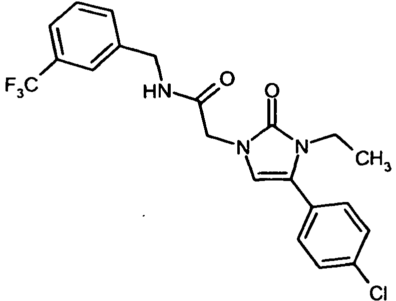 | MS [ESIpos]: m/z = 438 (M+H)⁺; [ESIneg]: m/z = 436 (M-H)⁻ Rₜ = 4.69 min [2] | δ = 1.02 (t, 3H), 3.69 (q, 2H), 4.32 (s, 2H), 4.40 (d, 2H), 6.74 (s, 1H), 7.39-7.66 (m, 8H), 8.69 (t, 1H). |

### Beispiel 213

### 2-[4-(4-Chlorphenyl)-3-cyclopropyl-2-oxo-2,3-dihydro-1H-imidazol-1-yl]-N-[3-(trifluormethyl)-phenylmethyl]propionsäureamid

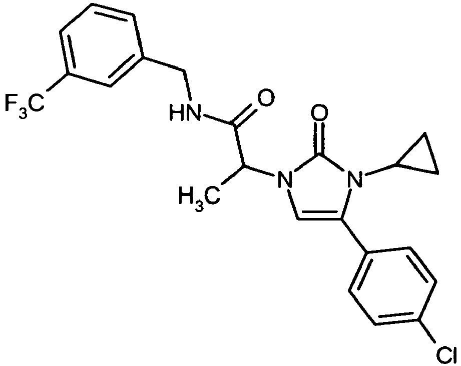

50 mg (0.163 mmol) 2-(4-[4-Chlorphenyl]-3-cyclopropyl-2-oxo-2,3-dihydro-1*H*-imidazol-1-yl)-propionsäure aus Beispiel 131A, 31.4 mg (0.179 mmol) 3-Trifluormethylbenzylamin, 40.6 mg (0.212 mmol) EDC-Hydrochlorid sowie 26.4 mg (0.196 mmol) HOBt werden in 1 ml trockenem Dimethylformamid gelöst und über Nacht bei Raumtemperatur gerührt. Der Ansatz wird zwischen Wasser und Dichlormethan verteilt, die abgetrennte organische Phase über Natriumsulfat getrocknet und eingeengt. Das verbleibende Rohprodukt wird durch Flash-Chromatographie an Kieselgel (Laufmittel: zunächst Dichlormethan, dann Dichlormethan/Methanol 100:1 → 50:1) gereinigt. Man erhält so 42 mg (56% d. Th.) der Zielverbindung.
MS [ESIpos]: m/z = 464 (M+H)⁺; [ESIneg]: m/z = 462 (M-H)⁻
¹H-NMR (400 MHz, DMSO-d₆): δ = 0.42 - 0.54 (m, 2H), 0.75 - 0.87 (m, 2H), 1.47 (d, 3H), 3.02 (dddd, 1H), 4.37 (d, 2H), 4.74 (q, 1H), 6.91 (s, 1H), 7.44, 7.47 (AA'-Teil eines AA'BB'-Systems, 2H), 7.53 - 7.63 (m, 4H), 7.55, 7.57 (BB'-Teil eines AABB'-Systems, 2H), 8.75 (t, 1H).

### Analog werden erhalten:

| **Beispiel Nr.** | **Struktur** | **HPLC, MS Rₜ [Methode]** |
|---|---|---|
| **214** | 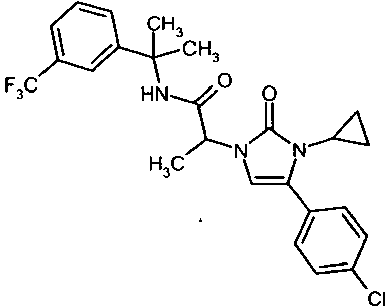 | MS [CIpos]: m/z = 509 (M+NH₄)⁺, 492 (M+H)⁺ Rₜ = 5.06 min [1] |
| **215** | 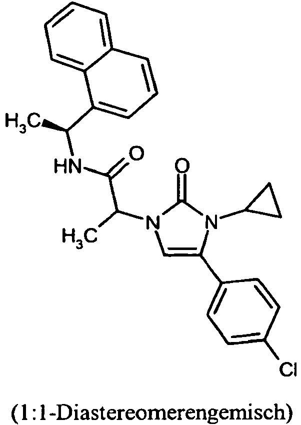 | MS [CIpos]: m/z = 477 (M+NH₄)⁺, 460 (M+H)⁺ Rₜ = 4.95 min [1] |

### Beispiel 216

### 2-[3-(4-Chlor-2-methoxyphenyl)-4-(2-fluorbenzyl)-5-oxo-4,5-dihydro-1H-1,2,4-triazol-1-yl]-N-[1-methyl-l-(3-trifluormethyl-phenyl)ethyl]-essigsäureamid

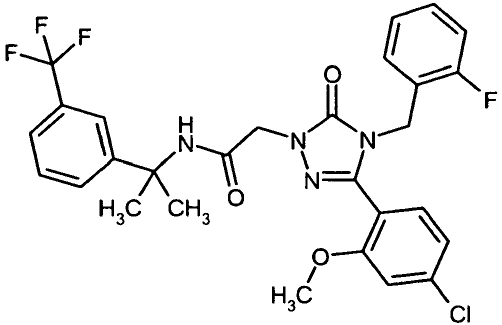

Unter Argon werden 70 mg (0.14 mmol) des Bromids aus Beispiel 141A und 35.4 mg (2-Methoxy-4-chlorphenyl)boronsäure (0.19 mmol) in 1.75 ml entgastem DMF gelöst und mit 204 µl einer entgasten 2 M Natriumcarbonat-Lösung in Wasser (0.41 mmol) versetzt. Es werden ca. 8 mg Tetrakis-(triphenylphosphin)palladium (ca. 7 µmol) hinzugefügt und die Mischung für 5 h auf 90°C erwärmt. Nach Abkühlung wird filtriert, der Feststoff mit wenig DMSO gewaschen und das gesamte Filtrat über präparative HPLC (Methode 20) aufgetrennt. Man erhält 65 mg (83% d. Th.) der Titelverbindung.
LC/MS [Methode 7]: Rₜ = 2.63 min; m/z = 577 (M+H)⁺
¹H-NMR (400 MHz, DMSO-d₆): δ = 1.60 (s, 6H), 3.68 (s, 3H), 4.49 (s, 2H), 4.71 (s, 2H), 6.96-7.08 (m, 4H), 7.16 (d, 1H), 7.19-7.28 (m, 2H), 7.48-7.57 (m, 2H), 7.62 (s, 1H), 7.67 (d, 1H), 8.51 (br. s, 1H).

Nach dem gleichen Verfahren werden aus den entsprechenden Boronsäuren (alle kommerziell erhältlich) und den genannten Edukt-Bromiden die folgenden Beispiele hergestellt. In wenigen Fällen wird anstelle der Boronsäure das entsprechende 4,4,5,5-Tetramethyl-1,2,3-borolan-Derivat (cyclischer Pinacol-Boronsäureester) verwendet.

| **Beispiel Nr.** | **Struktur** | **Edukt; Ausbeute** | **LC/MS: Rₜ [Methode], m/z** |
|---|---|---|---|
| **217** | 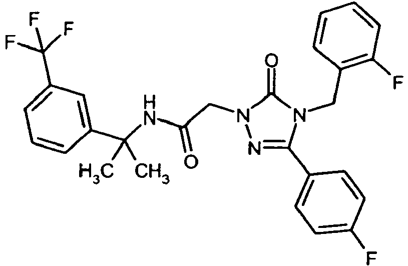 | 141A; 87% | Rₜ = 2.81 min [8] m/z = 531 (M+H)⁺ |
| **218** | 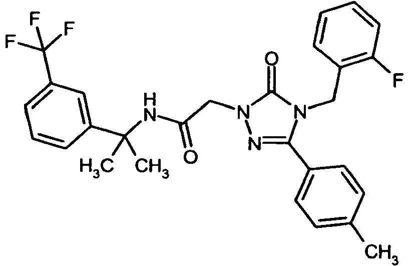 | 141A; 71% | Rₜ = 2.90 min [8] m/z = 527 (M+H)⁺ |
| **219** | 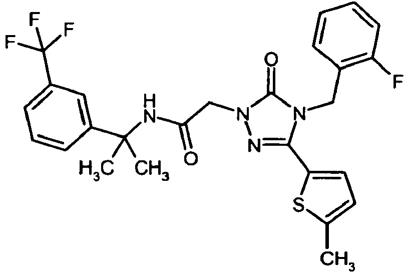 | 141A; 70% | Rₜ = 2.58 min [7] m/z = 533 (M+H)⁺ |
| **220** | 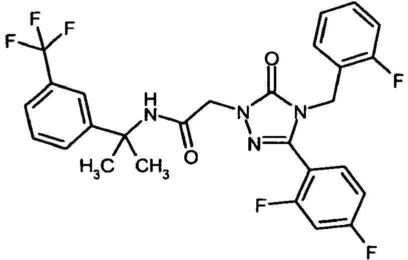 | 141A; 78% | Rₜ = 2.53 min [7] m/z = 549 (M+H)⁺ |
| **221** | 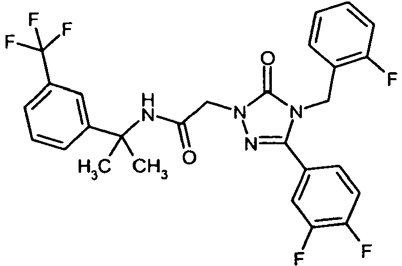 | 141A; 63% | Rₜ = 2.58 min [7] m/z = 549 (M+H)⁺ |
| **222** | 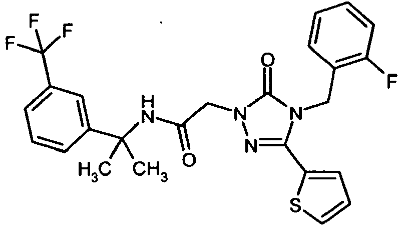 | 141A; 77% | Rₜ = 2.66 min [8] m/z = 519 (M+H)⁺ |
| **223** | 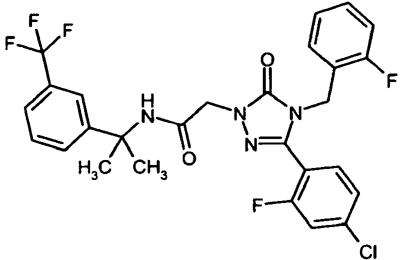 | 141A; 27% | Rₜ = 2.94 min [8] m/z = 565 (M+H)⁺ |
| **224** | 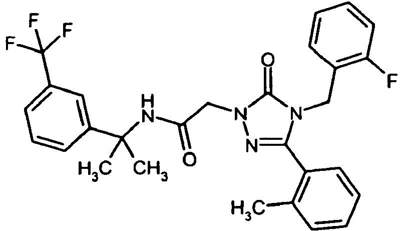 | 141A; 63% | Rₜ = 2.85 min [8] m/z = 527 (M+H)⁺ |
| **225** | 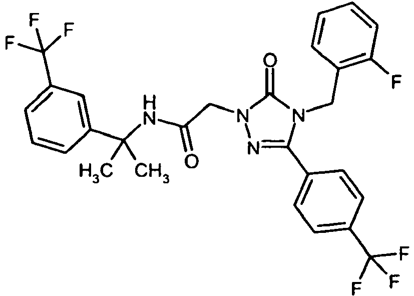 | 141A; 82% | Rₜ = 4.15 min [17] m/z = 581 (M+H)⁺ |
| **226** | 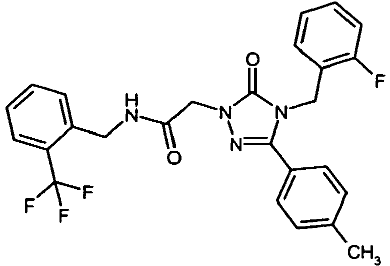 | 142A; 68% | Rₜ = 2.45 min [7] m/z = 499 (M+H)⁺ |
| **227** | 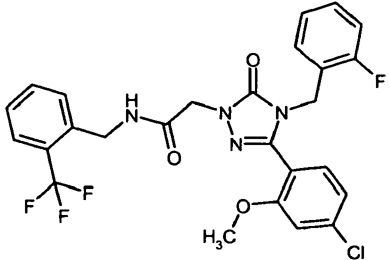 | 142A; 68% | Rₜ = 2.47 min [7] m/z = 549 (M+H)⁺ |
| **228** | 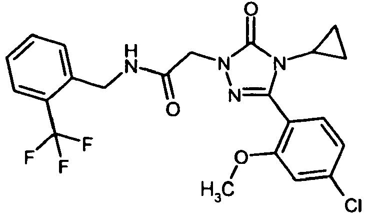 | 145A; 98% | Rₜ = 2.39 min [18] m/z = 481 (M+H)⁺ |
| **229** | 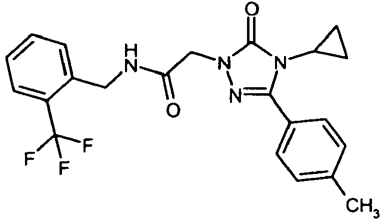 | 145A; 66% | Rₜ = 2.21 min [7] m/z = 431 (M+H)⁺ |
| **230** | 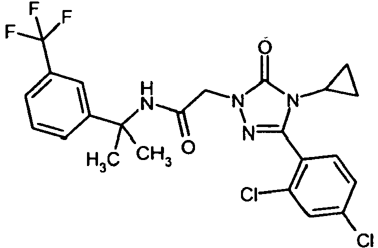 | 144A; 28% | Rₜ = 2.63 min [18] m/z = 513 (M+H)⁺ |
| **231** | 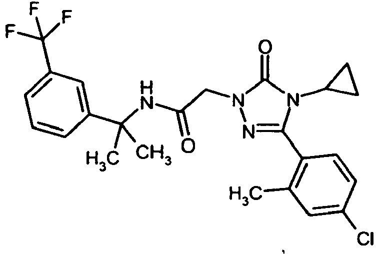 | 144A; 80% | Rₜ = 3.89 min [17] m/z = 493 (M+H)⁺ |
| **232** | 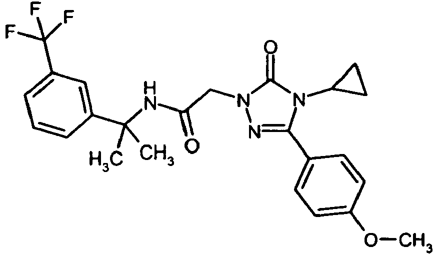 | 144A; 77% | Rₜ = 2.26 min [7] m/z = 475 (M+H)⁺ |
| **233** | 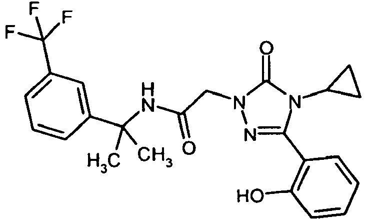 | 144A; 52% | Rₜ = 2.15 min [7] m/z = 461 (M+H)⁺ |
| **234** | 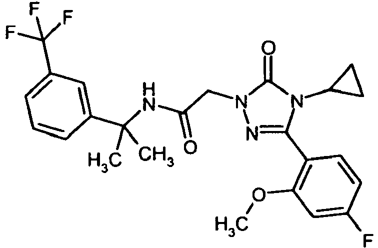 | 144A; 66% | Rₜ = 2.42 min [18] m/z = 493 (M+H)⁺ |
| **235** | 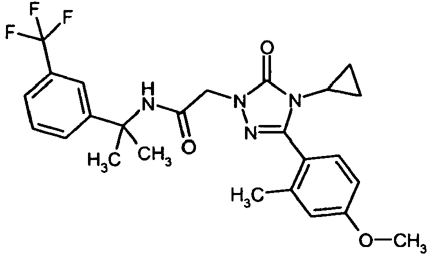 | 144A; 82% | Rₜ = 2.56 min [8] m/z = 489 (M+H)⁺ |
| **236** | 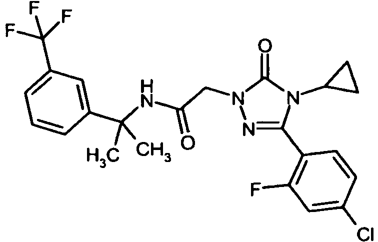 | 144A; 25% | Rₜ = 2.56 min [18] m/z = 497 (M+H)⁺ |
| **237** | 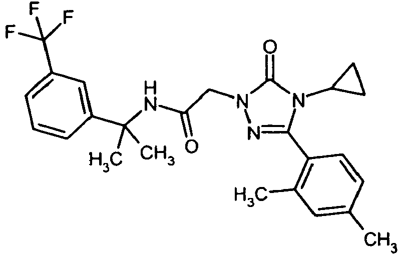 | 144A; 81% | Rₜ = 3.70 min [8] m/z = 473 (M+H)⁺ |
| **238** | 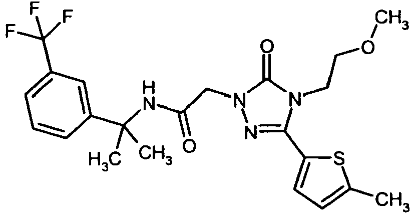 | 143A; 42% | Rₜ = 2.32 min [7] m/z = 483 (M+H)⁺ |
| **239** | 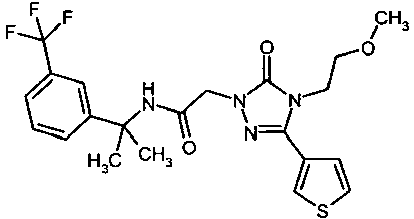 | 143A; 79% | Rₜ = 2.17 min [7] m/z = 469 (M+H)⁺ |
| **240** | 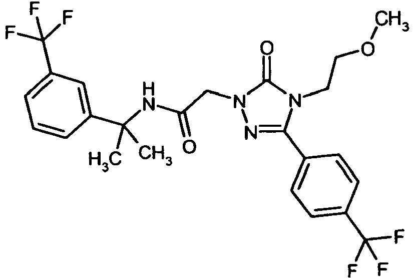 | 143A; 74% | Rₜ = 3.85 min [17] m/z = 531 (M+H)⁺ |
| **241** | 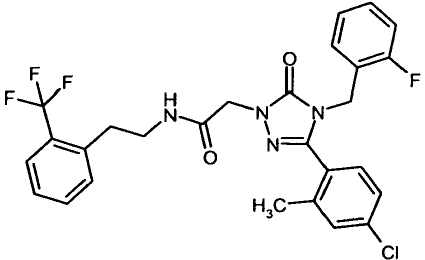 | 140A; 64% | Rₜ = 2.68 min [8] m/z = 547 (M+H)⁺ |
| **242** | 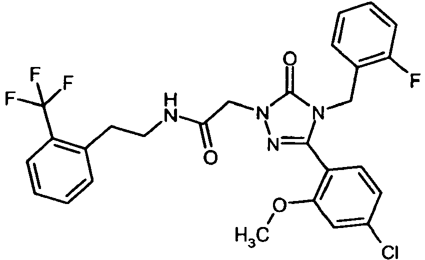 | 140A; 67% | Rₜ = 3.93 min [17] m/z = 563 (M+H)⁺ |
| **243** | 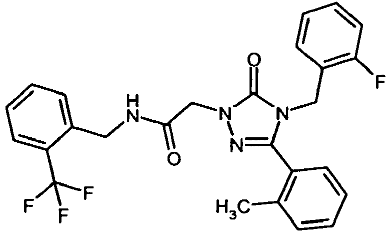 | 142A; 68% | Rₜ = 2.61 min [8] m/z = 499 (M+H)⁺ |

### Beispiel 244

### 2-[3-(4-Chlor-2-hydroxyphenyl)-4-(2-fluorbenzyl)-5-oxo-4,5-dihydro-1H-1,2,4-triazol-1-yl]-N-[1-methyl-1-(3-trifluormethyl-phenyl)ethyl]-essigsäureamid

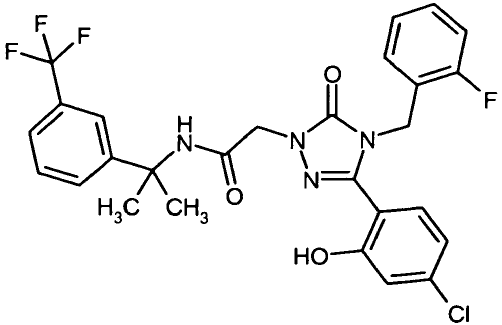

Unter Argon wird bei -20°C eine Lösung von 41 mg (71 µmol) der Verbindung aus Beispiel 216 mit 355 µl einer 1 M Lösung von Bortribromid in Dichlormethan (355 µmol) versetzt. Das Kühlbad wird entfernt und die Reaktionsmischung noch über Nacht bei RT weitergerührt. Die Reaktion wird durch Zugabe von 750 µl Methanol gestoppt. Die flüchtigen Komponenten werden am Rotationsverdampfer entfernt und der Rückstand durch präparative HPLC (Methode 20) gereinigt. Man erhält 15 mg (38% d. Th.) der Titelverbindung.
LC/MS [Methode 8]: Rₜ = 2.72 min; m/z = 563 (M+H)⁺
¹H-NMR (400 MHz, DMSO-d₆): δ = 1.60 (s, 6H), 4.49 (s, 2H), 4.71 (s, 2H), 6.86 (dd, 1H), 6.96 (d, 1H), 6.96-7.07 (m, 4H), 7.23 (m, 1H), 7.49-7.56 (m, 2H), 7.52 (s, 1H), 7.58 (d, 1H), 8.52 (s, 1H), 10.89 (br. s, 1H).

### Beispiel 245

### 2-[3-(4-Chlor-2-hydroxyphenyl)-4-(2-fluorbenzyl)-5-oxo-4,5-dihydro-1H-1,2,4-triazol-1-yl]-N-[(2-trifluormethyl-phenyl)methyl]-essigsäureamid

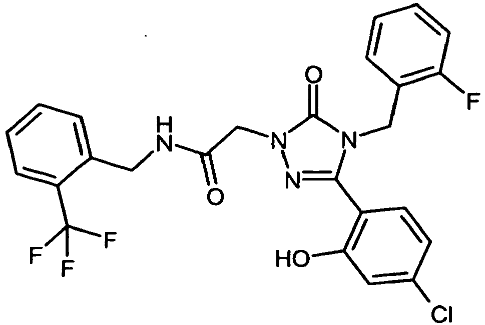

Die Titelverbindung wird ausgehend von Beispiel 227 nach dem gleichen Verfahren, wie für das Beispiel 244 beschrieben, hergestellt.
LC/MS [Methode 7]: Rₜ = 2.33 min; m/z = 535 (M+H)⁺
¹H-NMR (400 MHz, DMSO-d₆): δ = 4.50 (d, 2H), 4.55 (s, 2H), 4.86 (s, 2H), 6.89 (dd, 1H), 6.98 (d, 1H), 7.02-7.10 (m, 3H), 7.12 (d, 1H), 7.25 (m, 1H), 7.49 (t, 1H), 7.59 (d, 1H), 7.65 (t, 1H), 7.72 (d, 1H), 8.54 (t, 1H), 10.90 (br. s, 1H).

### Beispiel 246

### 2-[3-(5-Chlorthiophen-2-yl)-4-(2-fluorbenzyl)-5-oxo-4,5-dihydro-1H-1,2,4-triazol-1-yl]-N-[1-methyl-1-(3-trifluormethyl-phenyl)ethyl]-essigsäureamid

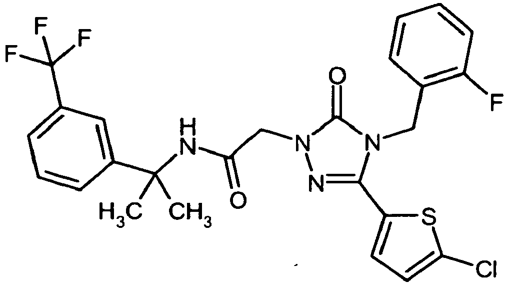

Die Carbonsäure aus Beispiel 154A (150 mg, 0.41 mmol) und 1-Methyl-1-(3-trifluormethylphenyl)ethylamin (Beispiel 1A, 91.2 mg, 0.45 mmol) werden in 4 ml DMF vorgelegt und bei RT mit 66.1 mg (0.49 mmol) HOBt versetzt. Nach 10 min werden 101.6 mg (0.53 mmol) EDC hinzugefügt und die Mischung über Nacht bei RT gerührt. Sie wird dann mit Wasser und Essigsäureethylester versetzt. Die Phasen werden getrennt, und die wässrige Phase wird mit Essigsäureethylester extrahiert. Die vereinigten organischen Phasen werden mit Wasser, dann mit ges. Kochsalz-Lösung gewaschen, durch Filtration über eine Extrelut-Kartusche getrocknet und am Rotationsverdampfer von den flüchtigen Komponenten befreit. Der Rückstand wird durch Chromatographie an Kieselgel (Biotage-Kartusche 25M, Eluent: Cyclohexan/Ethylacetat 1:1) gereinigt. Man erhält so 192 mg (85% d. Th.) der Titelverbindung.
LC/MS [Methode 8]: Rₜ = 2.92 min; m/z = 553 (M+H)⁺
¹H-NMR (400 MHz, DMSO-d₆): δ = 1.72 (s, 6H), 4.53 (s, 2H), 5.15 (s, 2H), 6.63 (s, 1H), 6.86 (d, 1H), 6.94 (d, 1H), 7.04-7.11 (m, 3H), 7.30 (m, 1H), 7.43 (t, 1H), 7.49 (d, 1H), 7.58 (d, 1H), 7.59 (s, 1H).

Auf analoge Weise werden die Verbindungen in der folgenden Tabelle hergestellt. Die hierbei eingesetzten Amine sind kommerziell erhältlich.

| **Beispiel Nr.** | **Struktur** | **Edukt; Ausbeute** | **LC/MS: Rₜ [Methode], m/z oder Rₜ-wert** |
|---|---|---|---|
| **247** | 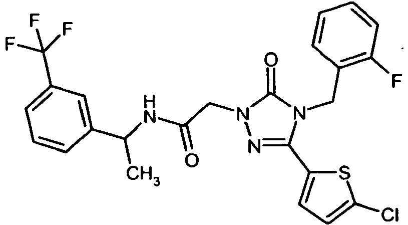 | 154A; 86% | Rₜ = 2.86 min [8] m/z = 539 (M+H)⁺ |
| **248** | 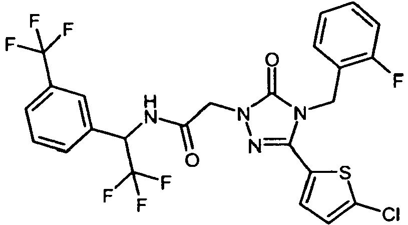 | 154A; 87% | Rₜ = 3.00 min [8] m/z = 593 (M+H)⁺ |
| **249** | 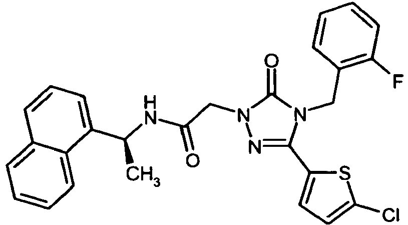 | 154A; 56% | Rₜ = 2.67 min [7] m/z = 521 (M+H)⁺ |
| **250** | 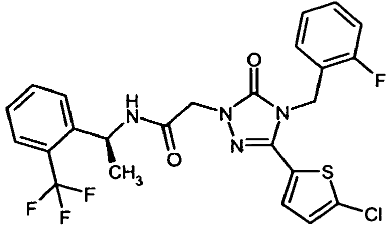 | 154A; 98% | R_{f}=0.17 (Cyclohexan/EE 1:1) MS [DCI]: m/z = 556 (M+NH₄)⁺ |

| **Beispiel Nr.** | **Struktur** | **Edukt; Ausbeute** | **LC/MS: Rₜ [Methode], m/z oder R_{f}-Wert** |
|---|---|---|---|
| **251** | 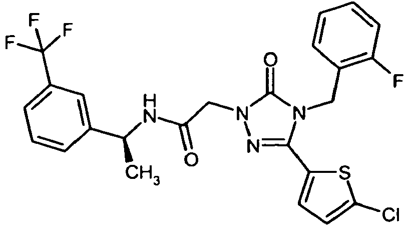 | 154A; 96% | R_{f}= 0.18 (Cyclohexan/EE 1:1) MS [ESIpos]: m/z = 539 (M+H)⁺ |
| **252** | 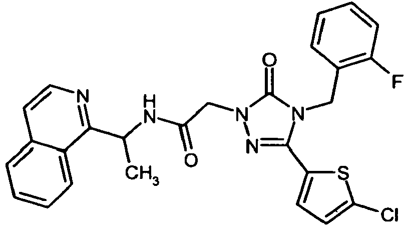 | 154A; 94% | R_{f}= 0.09 (Cyclohexan/EE 1:1) MS [ESIpos]: m/z = 522 (M+H)⁺ |

### Beispiel 253

### 2-[4-(3-Fluorbenzyl)-3-(2-hydroxyphenyl)-5-oxo-4,5-dihydro-1H-1,2,4-triazol-1-yl]-N-[1-methyl-1-(3-trifluormethyl-phenyl)ethyl]-essigsäureamid

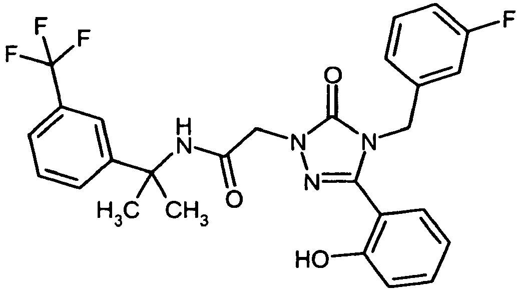

Die Carbonsäure aus Beispiel 150A (50 mg, 0.15 mmol) und HOBt (35.4 mg, 0.26 mmol) werden in 1 ml DMF vorgelegt und bei RT mit 50.3 mg (0.26 mmol) EDC versetzt. Nach 20 min wird 1-Methyl-1-(3-trifluormethyl-phenyl)ethylamin (Beispiel 1A, 44.4 mg, 0.22 mmol) hinzugefügt und die Mischung über Nacht bei RT gerührt. Das Reaktionsgemisch wird danach direkt durch präparative HPLC (Methode 20) aufgetrennt. Man erhält 24 mg (31% d. Th.) der Titelverbindung.
LC/MS [Methode 17]: Rₜ = 3.68 min; m/z = 529 (M+H)⁺
¹H-NMR (400 MHz, DMSO-d₆): δ = 1.59 (s, 6H), 4.48 (s, 2H), 4.78 (s, 2H), 6.72 (d, 1H), 6.76 (d, 1 H), 6.82 (t, 1 H), 6.94-7.03 (m, 2H), 7.08 (dd, 1 H), 7.23 (dt, 1 H), 7.33 (dt, 1 H), 7.48-7.56 (m, 2H), 7.63 (s, 1 H), 7.67 (d, 1 H), 8.53 (s, 1 H), 10.33 (s, 1 H).

### Aufarbeitungsalternativen:

Am Ende der Umsetzung kann das Reaktionsgemisch mit 1 N Salzsäure (2-8 ml pro mmol eingesetzter Carbonsäure) versetzt werden, bevor die Lösung dann direkt durch präparative HPLC (Methode 20) aufgetrennt wird. Für größere Mengen (>0.4 mmol eingesetzter Carbonsäure) wird eine extraktive Aufarbeitung, wie unter Beispiel 246 beschrieben, vorgenommen, bevor das Rohprodukt durch präparative HPLC gereinigt wird.

Analog zu Beispiel 253 werden die Verbindungen in der folgenden Tabelle hergestellt. Sofern nichts anderes angegeben, sind die Amin-Edukte kommerziell erhältlich. Wenn das Amin-Edukt als Salz (Hydrochlorid oder Trifluoracetat) eingesetzt wird, wird der Reaktionsmischung 1 eq. N,N-Diisopropylethylamin hinzugefügt.

| **Beispiel Nr.** | **Struktur** | **Edukt(e); Ausbeute** | **LC/MS: Rₜ [Methode], m/z** |
|---|---|---|---|
| **254** | 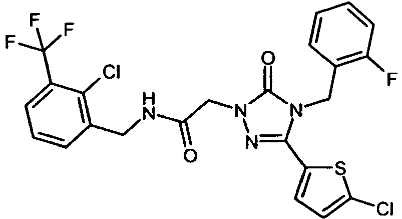 | 154A + 170A; 39% | Rₜ= 2.66 min [7] m/z = 559 (M+H)⁺ |
| **255** | 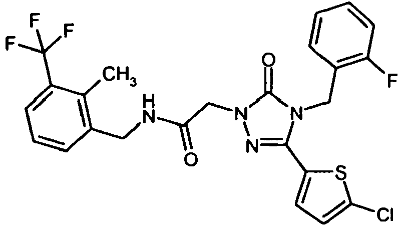 | 154A + 171A; 75% | Rₜ= 2.65 min [7] m/z = 539 (M+H)⁺ |
| **256** | 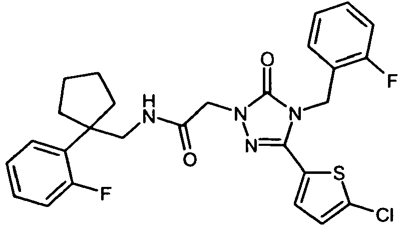 | 154A; 96% | Rₜ= 2.80 min [7] m/z = 543 (M+H)⁺ |
| **257** | 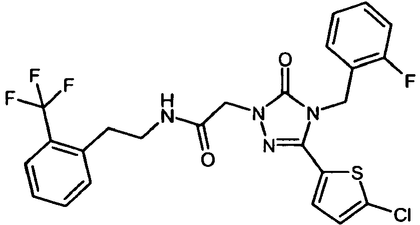 | 154A; 91% | Rₜ = 2.60 min [7] m/z = 539 (M+H)⁺ |
| **258** | 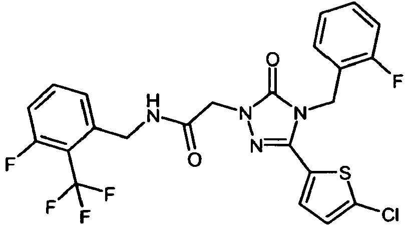 | 154A + 160A; 98% | Rₜ =2.56 min [7] m/z = 534 (M+H)⁺ |
| **259** | 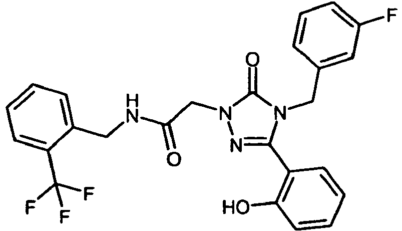 | 150A; 66% | Rₜ= 3.31 min [19] m/z = 501 (M+H)⁺ |
| **260** | 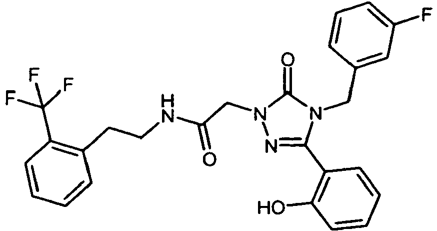 | 150A; 61% | Rₜ= 3.37 min [19] m/z = 515 (M+H)⁺ |
| **261** | 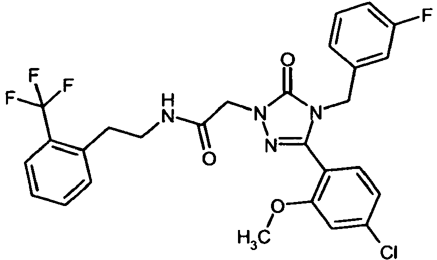 | 148A; 81% | Rₜ= 3.76 min [19] m/z = 563 (M+H)⁺ |
| **262** | 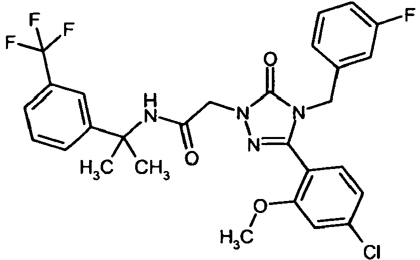 | 148A + 1A; 83% | Rₜ = 4.06 min [17] m/z = 577 (M+H)⁺ |

### Beispiel 263

### 2-[3-(4-Chlorphenyl)-4-(2-fluorbenzyl)-5-oxo-4,5-dihydro-1H-1,2,4-triazol-1-yl]-N-[1-methyl-1-(3-trifluormethyl-phenyl)ethyl]-essigsäureamid

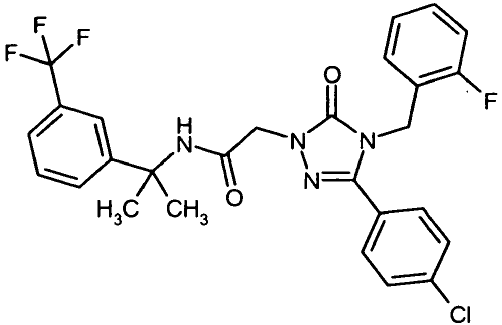

100 mg (0.36 mmol) der Verbindung aus Beispiel 133A, 108.6 mg (0.36 mmol) der Verbindung aus Beispiel 40A und 98.8 mg (0.72 mmol) Kaliumcarbonat werden in 2.7 ml Acetonitril über Nacht bei Rückflusstemperatur gerührt. Nach dem Abkühlen wird die Mischung mit Wasser verdünnt und dreimal mit Ethylacetat extrahiert. Die vereinigten organischen Phasen werden über Natriumsulfat getrocknet und am Rotationsverdampfer von den flüchtigen Komponenten befreit. Der Rückstand wird im Hochvakuum getrocknet. Man erhält 192 mg (98% d. Th.) der Titelverbindung.
LC/MS [Methode 7]: Rₜ = 2.70 min; m/z = 547 (M+H)⁺
¹H-NMR (400 MHz, DMSO-d₆): δ = 1.60 (s, 6H), 4.52 (s, 2H), 5.00 (s, 2H), 6.98-7.16 (m, 3H), 7.25-7.32 (m, 1H), 7.48-7.56 (m, 6H), 7.62 (s, 1H), 7.67 (d, 1H), 8.58 (s, 1H).

### Beispiel 264

### 2-[3-(4-Chlorphenyl)-4-(2-fluorbenzyl)-5-oxo-4,5-dihydro-1H-1,2,4-triazol-1-yl]-N-[2-(2-trifluormethyl-phenyl)ethyl]-essigsäureamid

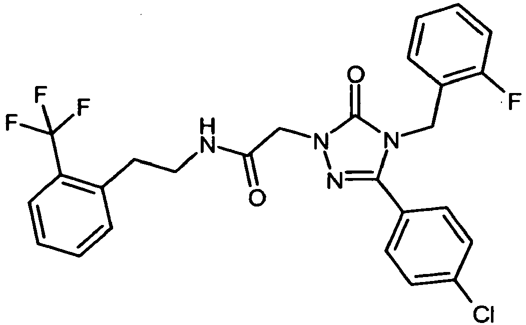

Die Carbonsäure aus Beispiel 156A (60 mg, 0.14 mmol) und HOBt (27.0 mg, 0.20 mmol) werden in 0.86 ml DMF vorgelegt und bei RT mit 38.2 mg (0.20 mmol) EDC versetzt. Nach 20 min werden 27 mg 2-(2-Trifluormethyl-phenyl)ethylamin (0.14 mmol) hinzugefügt und die Mischung über Nacht bei RT gerührt. Das Gemisch wird dann direkt durch präparative HPLC (Methode 20) aufgetrennt. Man erhält 69 mg (91% d. Th.) der Titelverbindung.
LC/MS [Methode 7]: Rₜ = 2.57 min; m/z = 533 (M+H)⁺
¹(400 MHz, DMSO-d₆): δ = 2.92 (t, 2H), 3.35 (q, 2H), 4.44 (s, 2H), 5.03 (s, 2H), 7.06-7.19 (m, 3H), 7.28-7.35 (m, 1H), 7.43 (t, 1H), 7.49 (d, 1H), 7.60 (t, 1H), 7.68 (d, 1H), 8.32 (t, 1H).

Auf analoge Weise werden die Verbindungen in der folgenden Tabelle hergestellt. Sofern nichts anderes angegeben, sind die Amin-Edukte kommerziell erhältlich. Wenn das Amin-Edukt als Salz (Hydrochlorid oder Trifluoracetat) eingesetzt wird, wird der Reaktionsmischung 1 eq. N,N-Diisopropylethylamin hinzugefügt.

| **Beispiel Nr.** | **Struktur** | **Edukt(e); Ausbeute** | **LC/MS: Rₜ [Methode], m/z** |
|---|---|---|---|
| **265** | 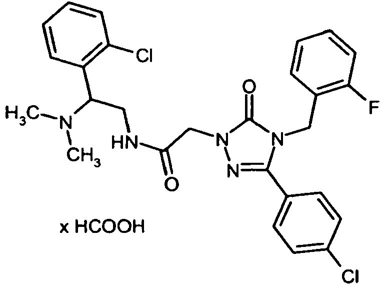 | 156A; 60% | Rₜ = 1.63 min [8] m/z = 542 (M+H)⁺ |
| **266** | 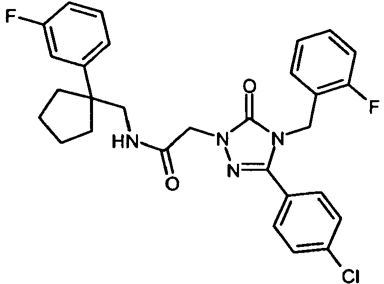 | 156A; 85% | Rₜ = 2.93 min [8] m/z = 537 (M+H)⁺ |
| **267** | 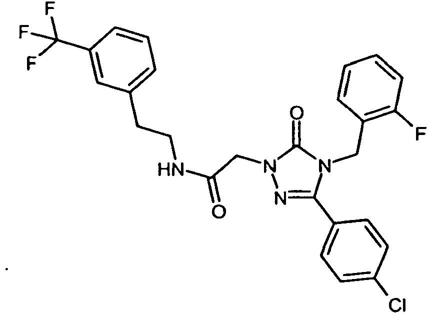 | 156A; 79% | Rₜ = 3.79 min [17] m/z = 533 (M+H)⁺ |
| **268** | 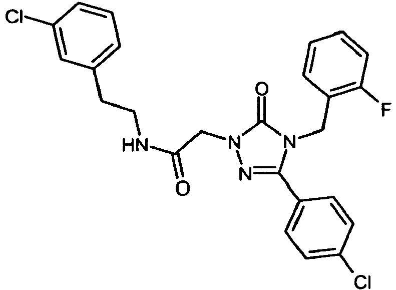 | 156A; 81% | Rₜ = 3.90 min [17] m/z = 499 (M+H)⁺ |
| **269** | 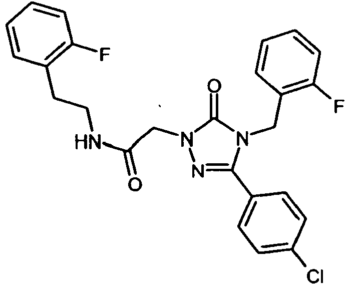 | 156A; 80% | Rₜ= 3.76 min [17] m/z = 483 (M+H)⁺ |
| **270** | 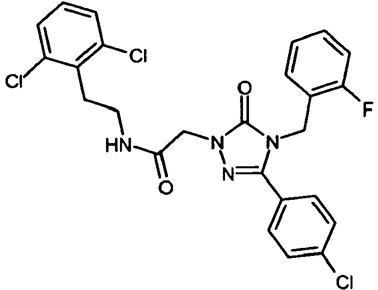 | 156A; 66% | Rₜ= 3.98 min [17] m/z = 533 (M+H)⁺ |
| **271** | 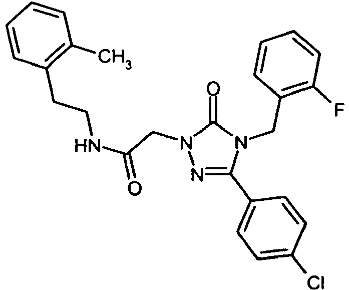 | 156A; 79% | Rₜ= 3.68 min [17] m/z = 479 (M+H)⁺ |
| **272** | 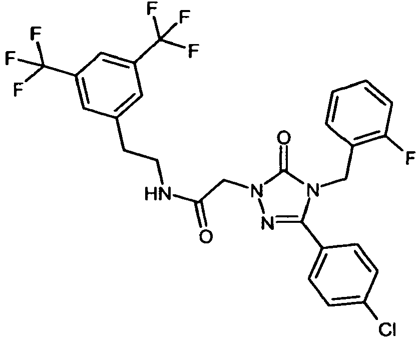 | 156A; 90% | Rₜ = 4.02 min [17] m/z = 601 (M+H)⁺ |
| **273** | 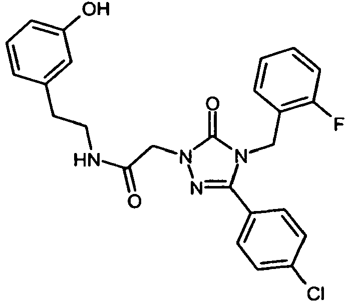 | 156A; 97% | Rₜ = 2.34 min [8] m/z = 481 (M+H)⁺ |
| **274** | 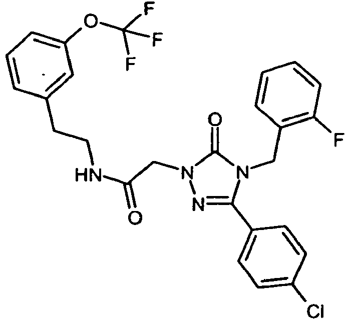 | 156A; 72% | Rₜ = 2.81 min [8] m/z = 549 (M+H)⁺ |
| **275** | 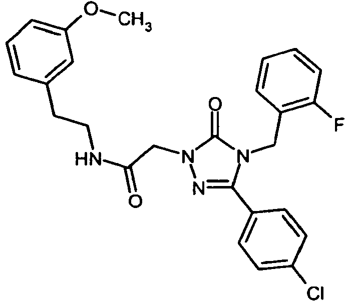 | 156A; 93% | Rₜ = 3.56 min [19] m/z = 495 (M+H)⁺ |
| **276** | 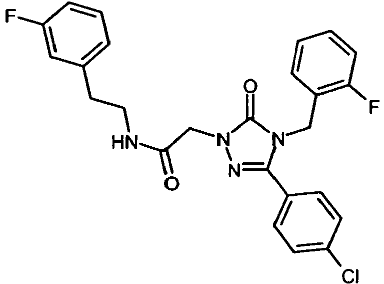 | 156A; 83% | Rₜ = 3.60 min [19] m/z = 483 (M+H)⁺ |
| **277** | 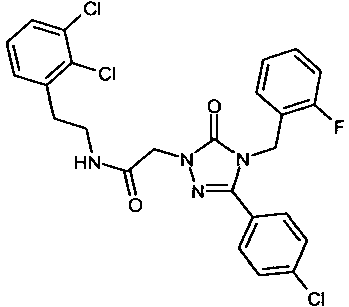 | 156A; 93% | Rₜ = 4.04 min [17] m/z = 533 (M+H)⁺ |
| **278** | 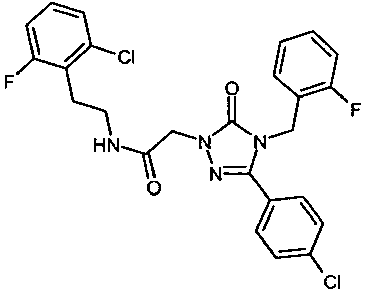 | 156A; 100% | Rₜ = 3.70 min [19] m/z = 517 (M+H)⁺ |
| **279** | 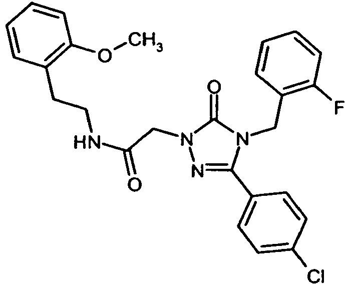 | 156A; 97% | Rₜ = 3.81 min [17] m/z = 495 (M+H)⁺ |
| **280** | 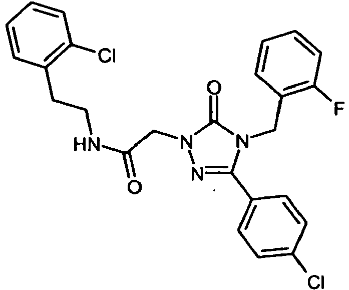 | 156A; 94% | Rₜ = 2.77 min [8] m/z = 499 (M+H)⁺ |
| **281** | 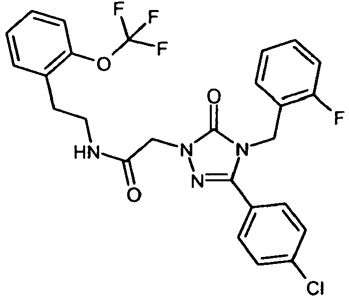 | 156A; 100% | Rₜ = 2.86 min [17] m/z = 549 (M+H)⁺ |
| **282** | 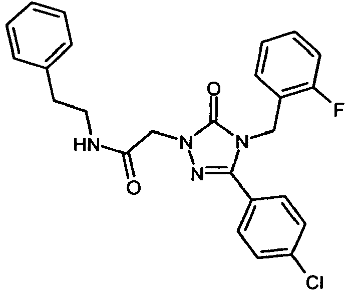 | 156A; 92% | Rₜ = 3.76 min [17] m/z = 465 (M+H)⁺ |
| **283** | 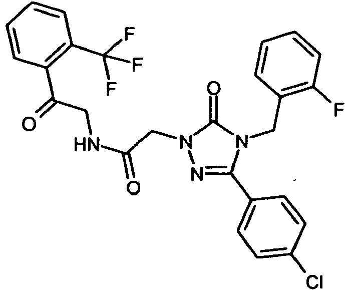 | 156A + 169A; 66% | Rₜ = 2.67 min [8] m/z = 547 (M+H)⁺ |
| **284** | 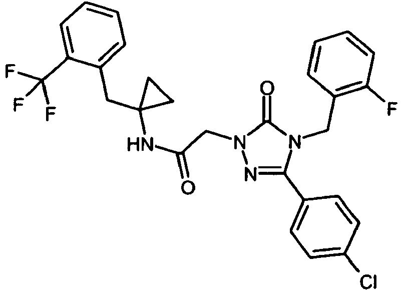 | 156A + 162A; 78% | Rₜ = 2.86 min [8] m/z = 559 (M+H)⁺ |
| **285** | 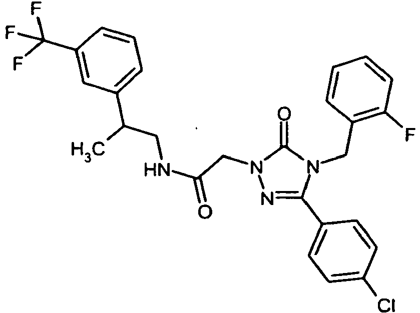 | 156A + 168A; 70% | Rₜ = 2.91 min [8] m/z = 547 (M+H)⁺ |
| **286** | 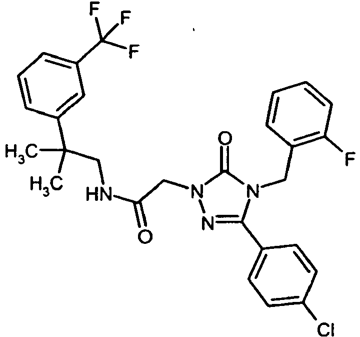 | 156A + 164A; 91% | Rₜ = 3.00 min [8] m/z = 561 (M+H)⁺ |
| **287** | 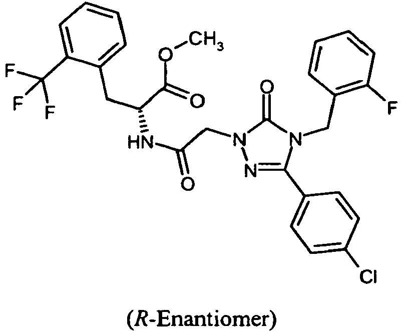 | 156A; 63% | Rₜ = 4.01 min [17] m/z = 591 (M+H)⁺ |
| **288** | 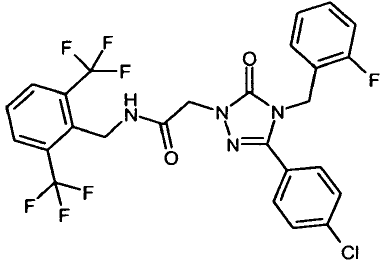 | 156A + 158A; 72% | Rₜ= 2.58 min [7] m/z = 587 (M+H)⁺ |
| **289** | 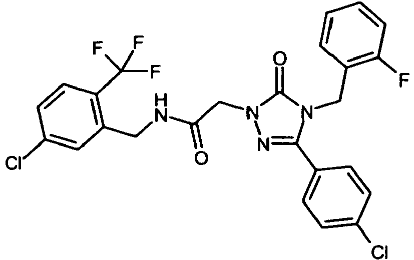 | 156A + 157A; 70% | Rₜ = 2.66 min [7] m/z = 553 (M+H)⁺ |
| **290** | 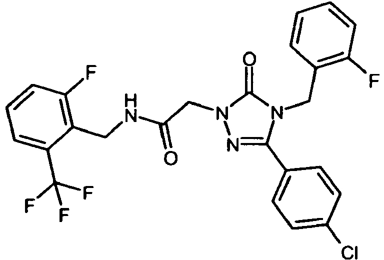 | 156A + 159A; 82% | Rₜ = 2.48 min [7] m/z = 537 (M+H)⁺ |
| **291** | 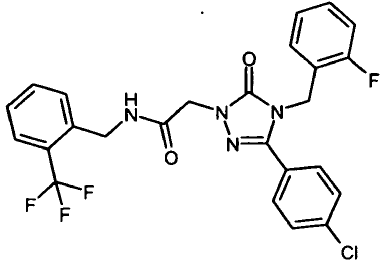 | 156A; 87% | Rₜ = 2.53 min [7] m/z = 519 (M+H)⁺ |
| **292** | 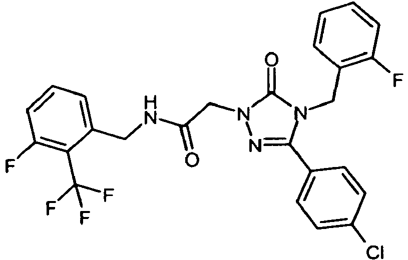 | 156A + 160A; 81% | Rₜ = 3.91 min [17] m/z = 537 (M+H)⁺ |
| **293** | 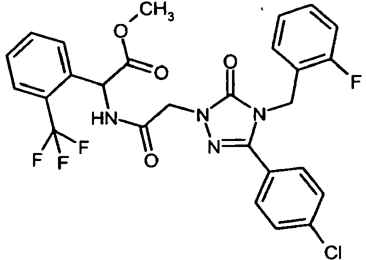 | 156A + 172A; 73% | Rₜ = 2.77 min [8] m/z = 577 (M+H)⁺ |
| **294** | 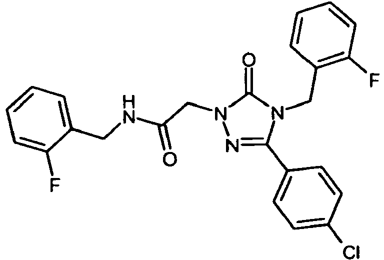 | 156A; 81% | Rₜ = 3.70 min [17] m/z = 469 (M+H)⁺ |
| **295** | 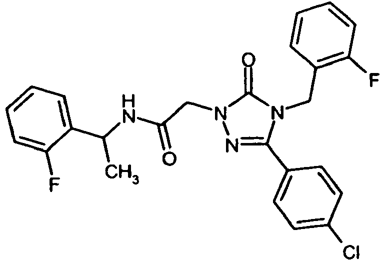 | 156A; 86% | Rₜ =2.67 min [8] m/z = 483 (M+H)⁺ |
| **296** | 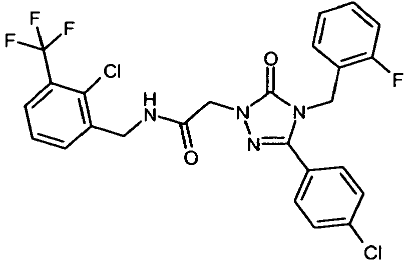 | 156A + 170A; 68% | Rₜ = 4.03 min [17] m/z = 553 (M+H)⁺ |
| **297** | 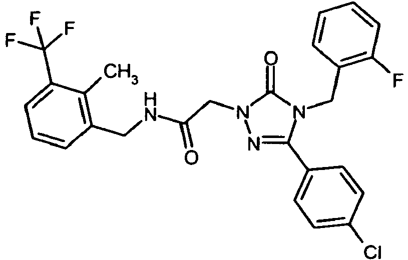 | 156A + 171A; 79% | Rₜ = 3.84 min [19] m/z = 533 (M+H)⁺ |
| **298** | 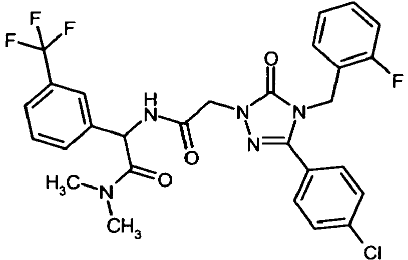 | 156A + 175A; 81% | Rₜ =3.70 min [17] m/z = 469 (M+H)⁺ |
| **299** | 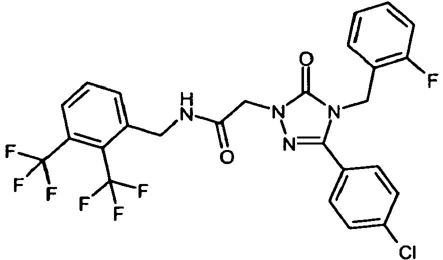 | 156A + 161A; 86% | Rₜ = 2.93 min [8] m/z = 587 (M+H)⁺ |

### Beispiel 300

### N-{[3-(4-Chlorphenyl)-4-(2-fluorbenzyl)-5-oxo-4,5-dihydro-1H-1,2,4-triazol-1-yl]acetyl}-(2-trifluormethyl)-D-Phenylalanin

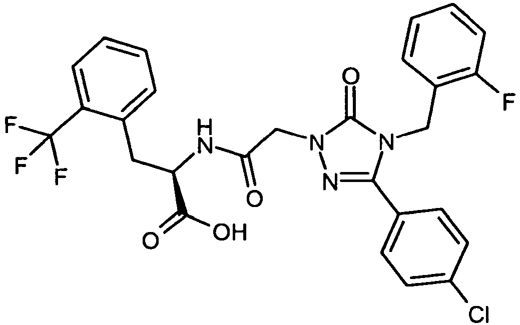

Die Verbindung aus Beispiel 287 (50 mg, 0.085 mmol) in 5 ml Methanol wird mit 1 M wässriger Lithiumhydroxid-Lösung (423 µl) versetzt und die Mischung über Nacht bei RT gerührt. Dann wird mit 1 N Salzsäure bis pH 1 angesäuert, mit 30 ml Wasser versetzt und dreimal mit Ethylacetat extrahiert. Die vereinigten organischen Phasen werden über Natriumsulfat getrocknet und am Rotationsverdampfer von den flüchtigen Komponenten befreit. Der Rückstand wird im Hochvakuum getrocknet. Man erhält 44 mg (90% d. Th.) der Titelverbindung.
LC/MS [Methode 8]: Rₜ = 2.76 min; m/z = 577 (M+H)⁺.

### Beispiel 301

### N^{α}-{[3-(4-Chlorphenyl)-4-(2-fluorbenzyl)-5-oxo-4,5-dihydro-1H-1,2,4-triazol-1-yl]acetyl}-N,N-dimethyl-2-trifluormethyl-L-Phenylalaninamid

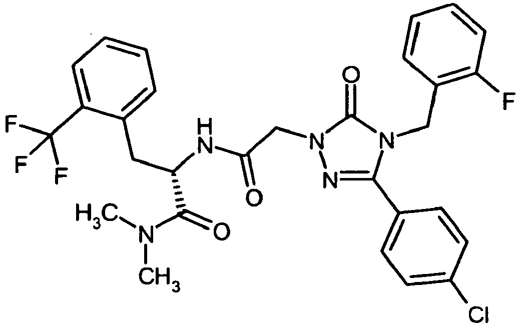

24 mg (42 µmol) *N*-{[3-(4-Chlorphenyl)-4-(2-fluorbenzyl)-5-oxo-4,5-dihydro-1*H*-1,2,4-triazol-1-yl]acetyl}-(2-trifluormethyl)-L-Phenylalanin [Herstellung analog zu Beispiel 287 ausgehend von Beispiel 156A und L-2-Trifluormethylphenylalaninmethylester, gefolgt von der Verseifung des Methylesters analog zu Beispiel 300] und 8 mg HOBt (58 µmol) werden in 1 ml DMF vorgelegt und bei RT mit 11 mg (58 µmol) EDC versetzt. Nach 20 min wird eine 2 M Lösung von Dimethylamin in THF (25 µl, 50 µmol) hinzugefügt und die Mischung über Nacht bei RT gerührt. Nach Zugabe von 1 ml 1 N Salzsäure wird das Gemisch direkt durch präparative HPLC (Methode 20) aufgetrennt. Man erhält 16 mg (64% d. Th.) der Titelverbindung.
LC/MS [Methode 19]: Rₜ = 3.68 min; m/z = 604 (M+H)⁺.

### Beispiel 302

### N-{2-Amino-2-oxo-1-[2-(trifluormethyl)phenyl]ethyl}-2-[3-(4-chlorphenyl)-4-(2-fluorbenzyl)-5-oxo-4,5-dihydro-1H-1,2,4-triazol-1-yl]-acetamid

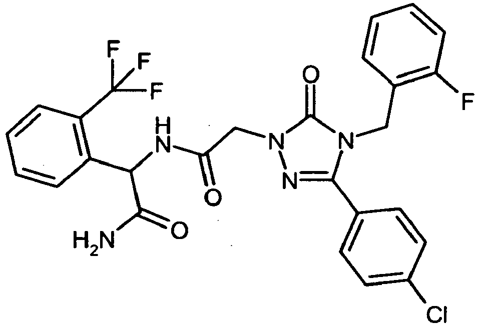

200 mg der Verbindung aus Beispiel 293 (347 µmol) werden in 36 ml Methanol mit 3.47 ml 1 N wässriger Lithiumhydroxid-Lösung für 2 h bei RT verrührt. Anschließend wird die Mischung mit 1 N Salzsäure auf pH 2 gestellt. Das Methanol wird am Rotationsverdampfer entfernt, das verbleibende Gemisch mit Wasser verdünnt und dreimal mit Ethylacetat extrahiert. Die vereinigten organischen Phasen werden über Natriumsulfat getrocknet und am Rotationsverdampfer von den flüchtigen Komponenten befreit. Die so erhaltene Carbonsäure wird im Hochvakuum getrocknet [quantitative Ausbeute, LC/MS [Methode 17]: Rₜ = 3.67 min; m/z = 563 (M+H)⁺].

23 mg (41 µmol) der oben erhaltenen Carbonsäure werden mit 10 mg HOBt (74 µmol) in 0.9 ml DMF vorgelegt und bei RT mit 14 mg (74 µmol) EDC versetzt. Nach 20 min wird eine wässrige Ammoniak-Lösung (32%-ig, 1.09 g, 20 mmol) hinzugefügt und die Mischung über Nacht bei RT gerührt. Nach Zugabe von 1 ml 1 N Salzsäure wird das Gemisch direkt durch präparative HPLC (Methode 20) aufgetrennt. Man erhält 13 mg (57% d. Th.) der Titelverbindung.
LC/MS [Methode 22]: Rₜ = 1.94 min; m/z = 562 (M+H)⁺
¹H-NMR (400 MHz, DMSO-d₆): δ = 4.50 (d, 1H), 4.68 (d, 1H), 5.02 (s, 2H), 5.66 (d, 1H), 7.01-7.18 (m, 3H), 7.27-7.34 (m, 1H), 7.44 (s, 1H), 7.52 (s, 4H), 7.54 (t, 1H), 7.64 (d, 1H), 7.65 (s, 1H), 7.69-7.75 (m, 2H), 9.04 (d, 1H).

### Beispiel 303

### 2-[3-(4-Chlorphenyl)-4-(cyclopropyl)-5-oxo-4,5-dihydro-1H-1,2,4-triazol-1-yl]-N-[2-(2-trifluormethyl-phenyl)ethyl]-essigsäureamid

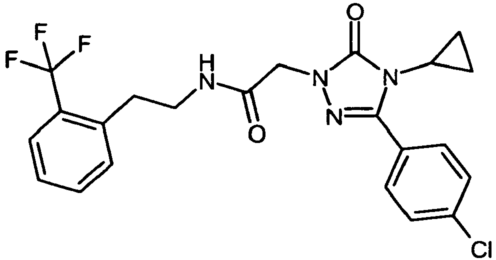

Die Carbonsäure aus Beispiel 88A (50 mg, 0.14 mmol) und HOBt (35 mg, 0.26 mmol) werden in 3 ml DMF vorgelegt und bei RT mit 49 mg (0.26 mmol) EDC versetzt. Nach 20 min werden 41 mg 2-(2-Trifluormethyl-phenyl)ethylamin (0.21 mmol) hinzugefügt und die Mischung über Nacht bei RT gerührt. Nach Zugabe von 1 ml 1 N Salzsäure wird das Gemisch direkt durch präparative HPLC (Methode 20) aufgetrennt. Man erhält 51 mg (77% d. Th.) der Titelverbindung.
LC/MS [Methode 19]: Rₜ = 3.50 min; m/z = 465 (M+H)⁺
¹H-NMR (400 MHz, DMSO-d₆): δ = 0.56-0.63 (m, 2H), 0.87-0.94 (m, 2H), 2.89 (t, 2H), 3.17 (m, 1H), 3.30-3.37 (m, 2H), 4.33 (s, 2H), 7.43 (t, 1H), 7.47 (d, 1H), 7.57-7.64 (m, 3H), 7.68 (d, 1H), 7.81 (d, 2H), 8.25 (t, 1H).

Auf analoge Weise werden die Verbindungen in der folgenden Tabelle hergestellt. Sofern nichts anderes angegeben, sind die Amin-Edukte kommerziell erhältlich. Wenn das Amin-Edukt als Salz (Hydrochlorid oder Trifluoracetat) eingesetzt wird, wird der Reaktionsmischung 1 eq. *N,N*-Diisopropylethylamin hinzugefügt.

| **Beispiel Nr.** | **Struktur** | **Edukt(e); Ausbeute** | **LC/MS: Rₜ [Methode], m/z** |
|---|---|---|---|
| **304** | 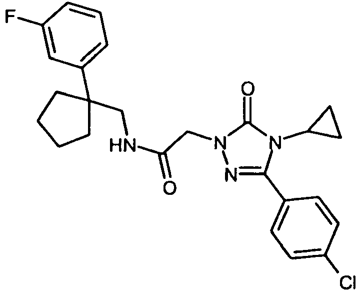 | 88A; 98% | Rₜ = 2.75 min [8] m/z = 469 (M+H)⁺ |
| **305** | 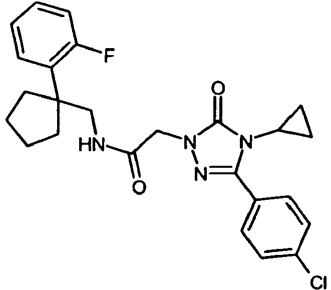 | 88A; 91% | Rₜ = 2.74 min [8] m/z = 469 (M+H)⁺ |
| **306** | 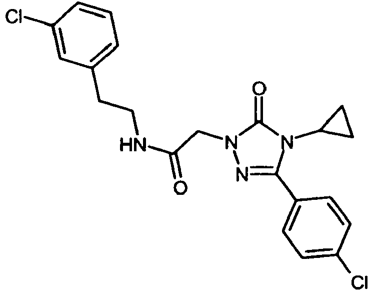 | 88A; 89% | Rₜ = 2.47 min [8] m/z = 431 (M+H)⁺ |
| **307** | 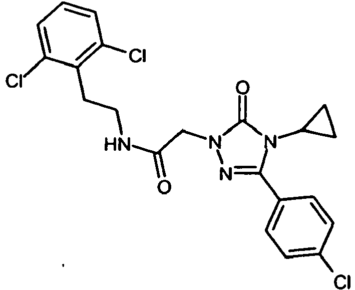 | 88A; 71% | Rₜ = 2.56 min [8] m/z = 467 (M+H)⁺ |
| **308** | 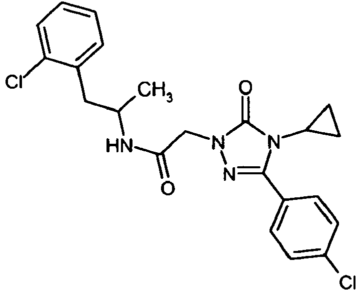 | 88A; 97% | Rₜ = 3.62 min [17] m/z = 445 (M+H)⁺ |
| **309** | 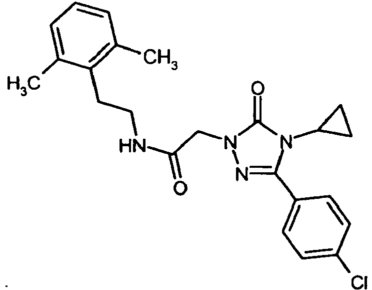 | 88A; 74% | Rₜ =3.51 min[19] m/z = 425 (M+H)⁺ |
| **310** | 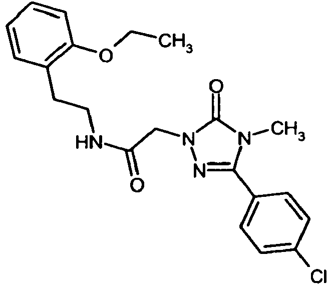 | 88A; 78% | Rₜ = 2.53 min [8] m/z = 441 (M+H)⁺ |
| **311** | 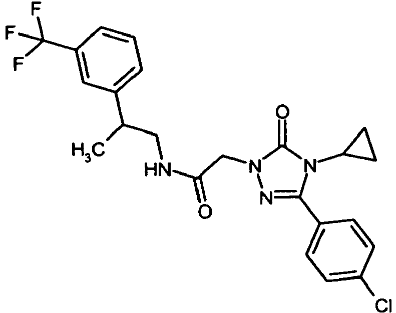 | 88A + 168A; 74% | Rₜ = 3.76 min [17] m/z = 479 (M+H)⁺ |
| **312** | 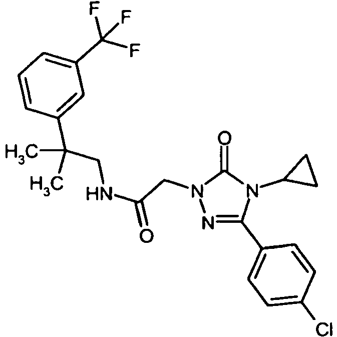 | 88A+ 164A; 85% | Rₜ = 3.88 min [17] m/z = 493 (M+H)⁺ |
| **313** | 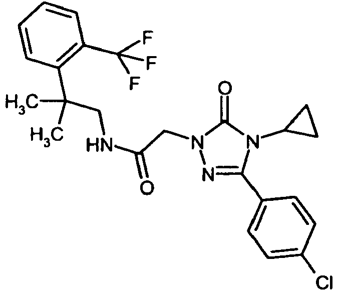 | 88A + 166A; 79% | Rₜ = 3.86 min [17] m/z = 493 (M+H)⁺ |
| **314** | 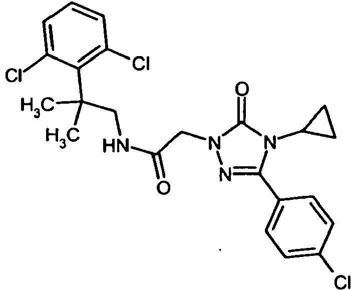 | 88A + 191 A; 74% | Rₜ = 3.76 min [19] m/z = 493 (M+H)⁺ |
| **315** | 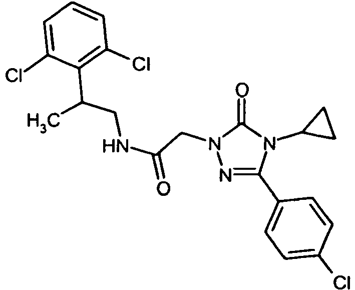 | 88A + 192A; 83% | Rₜ = 3.60 min [19] m/z = 479 (M+H)⁺ |
| **316** | 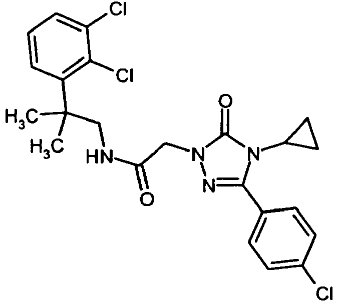 | 88A + 190A; 95% | Rₜ= 2.42 min [23] m/z = 493 (M+H)⁺ |
| **317** | 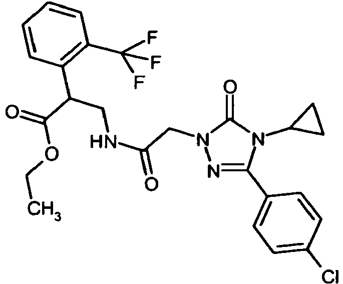 | 88A + 194A; 79% | Rₜ = 3.63 min [8] m/z = 537 (M+H)⁺ |
| **318** | 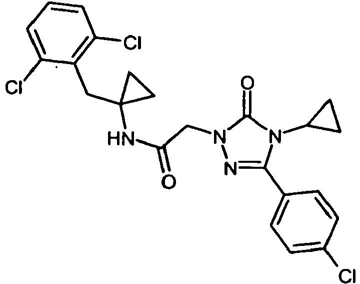 | 88A+ 198A; 64% | Rₜ = 3.72 min [17] m/z = 491 (M+H)⁺ |
| **319** | 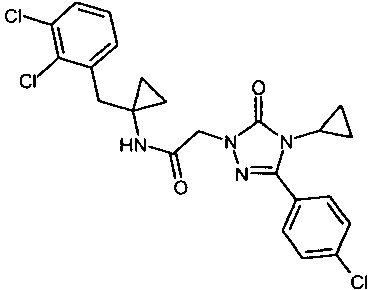 | 88A+ 197A; 90% | Rₜ = 2.29 min [23] m/z = 491 (M+H)⁺ |
| **320** | 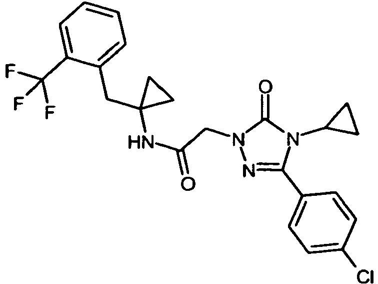 | 88A + 162A; 68% | Rₜ = 2.60 min [8] m/z = 491 (M+H)⁺ |
| **321** | 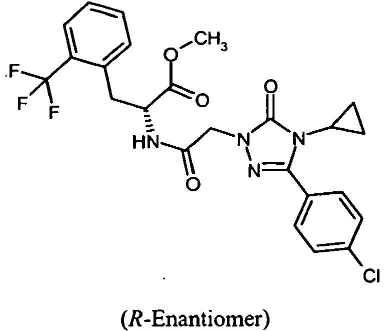 | 88A; 69% | Rₜ= 3.50 min [19] m/z = 523(M+H)⁺ |
| **322** | 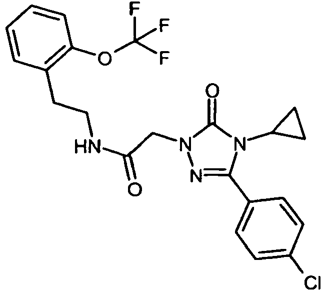 | 88A; 80% | Rₜ = 2.59 min [8] m/z = 481 (M+H)⁺ |
| **323** | 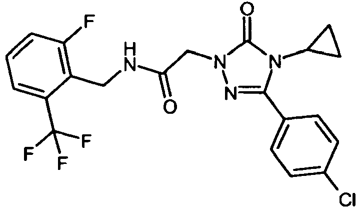 | 88A + 159A; 85% | Rₜ = 3.37 min [19] m/z = 469 (M+H)⁺ |
| **324** | 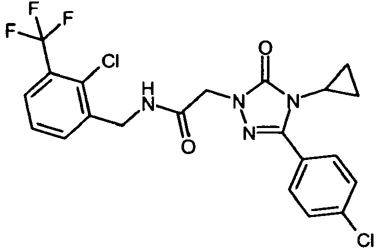 | 88A + 170A; 47% | Rₜ = 3.70 min [17] m/z = 485 (M+H)⁺ |
| **325** | 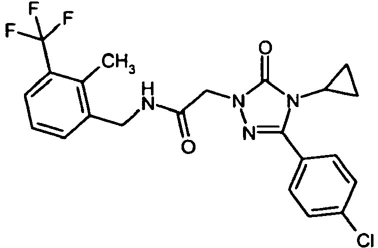 | 88A + 171A; 72% | Rₜ= 3.69 min [17] m/z = 465 (M+H)⁺ |
| **326** | 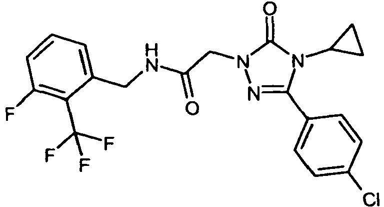 | 88A+ 160A; 82% | Rₜ = 3.58 min [17] m/z = 469 (M+H)⁺ |
| **327** | 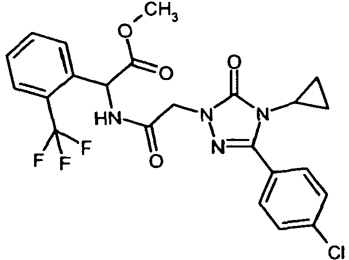 | 88A + 172A; 81% | Rₜ = 3.58 min [17] m/z = 509 (M+H)⁺ |
| **328** | 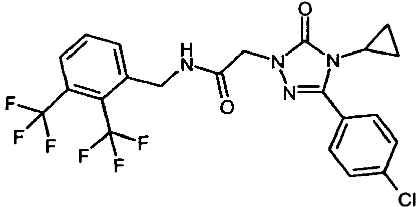 | 88A + 161A; 88% | Rₜ = 3.79 min [17] m/z = 519 (M+H)⁺ |
| **329** | 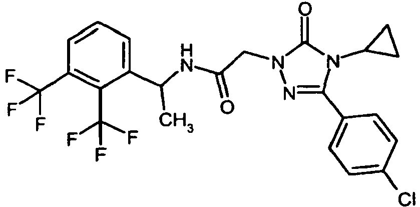 | 88A + 200A; 88% | Rₜ = 2.74 min [8] m/z = 533 (M+H)⁺ |
| **330** | 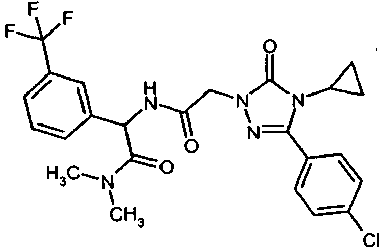 | 88A + 175A; 84% | Rₜ= 3.37 min [19] m/z = 522 (M+H)⁺ |
| **331** | 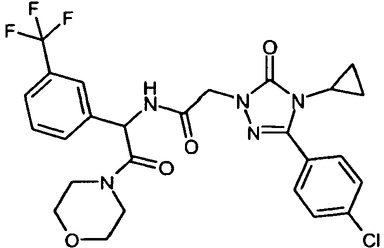 | 88A + 177A; 73% | Rₜ = 2.47 min [8] m/z = 564 (M+H)⁺ |
| **332** | 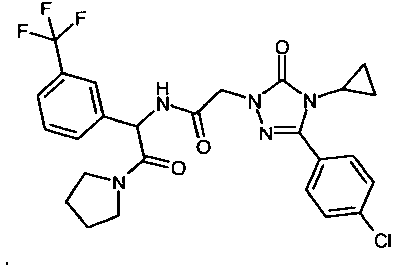 | 88A + 178A; 79% | Rₜ = 2.19 min [23] m/z = 548 (M+H)⁺ |
| **333** | 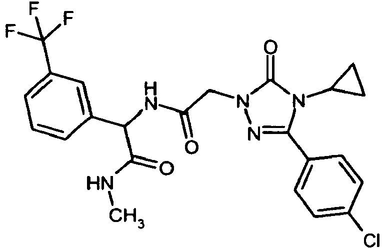 | 88A + 179A; 55% | Rₜ = 1.99 min [23] m/z = 508 (M+H)⁺ |
| **334** | 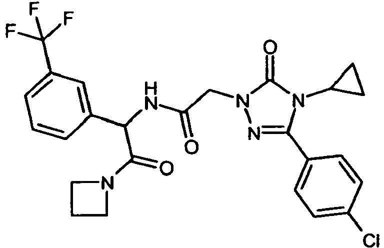 | 88A+ 180A; 91% | Rₜ =2.08 min [23] m/z = 534 (M+H)⁺ |
| **335** | 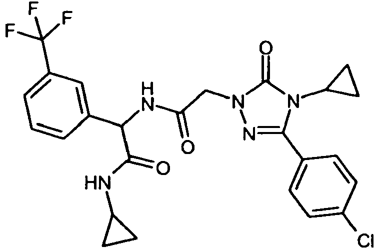 | 88A + 181A; 91% | Rₜ = 2.54 min [8] m/z = 534 (M+H)⁺ |
| **336** | 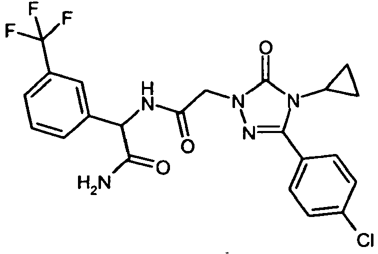 | 88A + 184A; 81% | Rₜ = 1.92 min [22] m/z = 494 (M+H)⁺ |
| **337** | 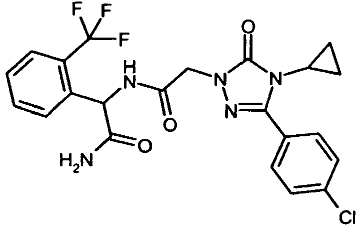 | 88A + 185A; 29% | Rₜ = 1.66 min [22] m/z = 494 (M+H)⁺ |
| **338** | 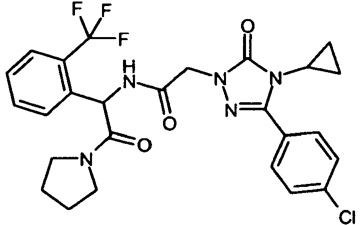 | 88A + 182A; 64% | Rₜ = 1.93 min [23] m/z = 548 (M+H)⁺ |
| **339** | 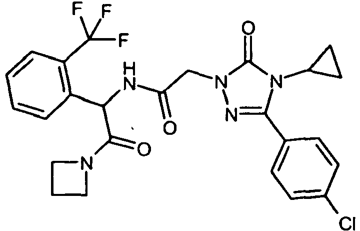 | 88A + 186A; 74% | Rₜ = 1.85 min [23] m/z = 534 (M+H)⁺ |
| **340** | 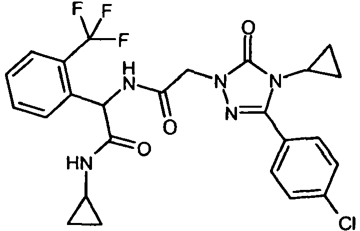 | 88A + 183A; 75% | Rₜ = 1.86 min [23] m/z = 534 (M+H)⁺ |
| **341** | 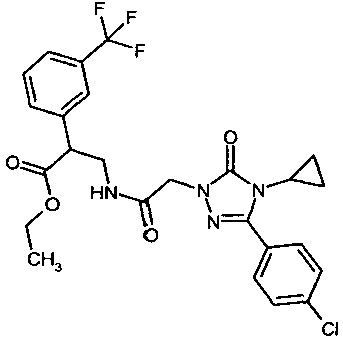 | 88A + 196A; 93% | Rₜ =2.70 min [8] m/z = 537 (M+H)⁺ |

### Beispiel 342

### 2-[3-(4-Chlorphenyl)-4-(cyclopropyl)-5-oxo-4,5-dihydro-1H-1,2,4-triazol-1-yl]-N-[2-(2-chlorphenyl)-3-hydroxypropyl]-essigsäureamid

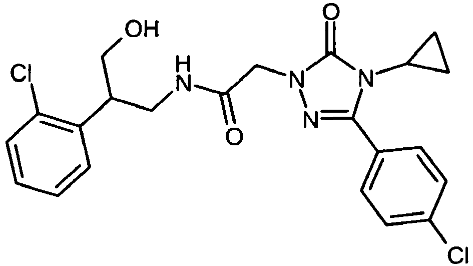

Nach der unter Beispiel 303 beschriebenen Methode erhält man aus der Carbonsäure aus Beispiel 88A und 2-(2-Chlorphenyl)-3-hydroxypropylamin [Herstellung siehe Arch. Pharm. 301, 750 (1968)] die Titelverbindung in 83% Ausbeute.
LC/MS [Methode 19]: Rₜ = 2.99 min; m/z = 461 (M+H)⁺.

### Beispiel 343

### 2-({[3-(4-Chlorphenyl)-4-(2-methoxyethyl)-5-oxo-4,5-dihydro-1H-1,2,4-triazol-1-yl]acetyl}-amino)-N-cyclopropyl-2-[3-(trifluormethyl)phenyl]-acetamid

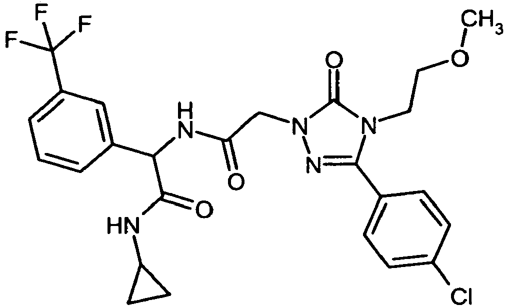

Die Carbonsäure aus Beispiel 90A (25 mg, 81 µmol) und HOBt (20 mg, 145 µmol) werden in 1.7 ml DMF vorgelegt und bei RT mit 28 mg (145 µmol) EDC versetzt. Nach 20 min werden 36 mg (97 µmol) des Amin-Trifluoracetats aus Beispiel 181A und 28 µl (161 µmol) *N,N*-Diisopropylethylamin hinzugefügt und die Mischung über Nacht bei RT gerührt. Nach Zugabe von 1 ml 1 N Salzsäure wird das Gemisch direkt durch präparative HPLC (Methode 20) aufgetrennt. Man erhält 30 mg (67% d. Th.) der Titelverbindung.
LC/MS [Methode 23]: Rₜ = 2.09 min; m/z = 552 (M+H)^{+ 1}H-NMR (400 MHz, DMSO-d₆): δ = 0.27-0.35 (m, 1H), 0.39-0.47 (m, 1H), 0.56-0.70 (m, 2H), 2.58-2.66 (m, 1H), 3.11 (s, 3H), 3.46 (t, 2H), 3.87 (t, 2H), 4.57 (m [AB], 2H), 5.48 (d, 1H), 7.57-7.63 (m, 3H), 7.65-7.73 (m, 4H), 7.76 (s, 1H), 8.53 (d, 1H), 9.05 (d, 1H).

Auf analoge Weise werden die Verbindungen in der folgenden Tabelle hergestellt. Sofern nichts anderes angegeben, sind die Amin-Edukte kommerziell erhältlich.

| **Beispiel Nr.** | **Struktur** | **Edukt(e); Ausbeute** | **LC/MS: Rₜ [Methode], m/z** |
|---|---|---|---|
| **344** | 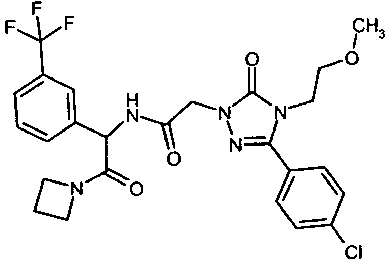 | 90A + 180A; 86% | Rₜ = 2.07 min [23] m/z = 552 (M+H)⁺ |
| **345** | 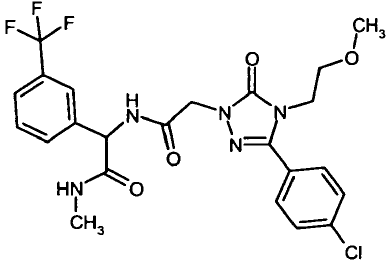 | 90A + 179A; 37% | Rₜ = 1.98 min [23] m/z = 526 (M+H)⁺ |
| **346** | 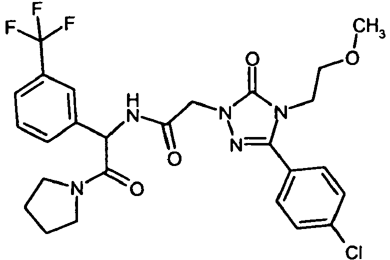 | 90A + 178A; 52% | Rₜ = 3.46 min [19] m/z = 566 (M+H)⁺ |
| **347** | 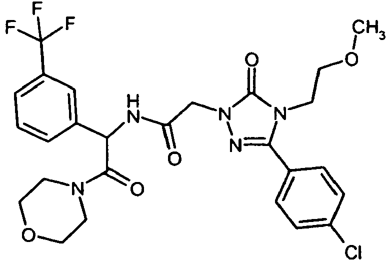 | 90A + 177A; 60% | Rₜ = 2.46 min [8] m/z = 582 (M+H)⁺ |
| **348** | 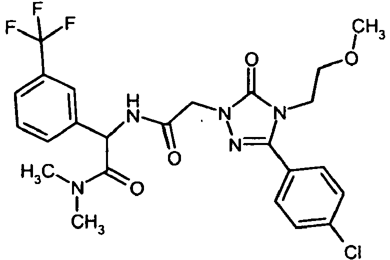 | 90A + 175A; 99% | Rₜ = 2.43 min [8] m/z = 540 (M+H)⁺ |
| **349** | 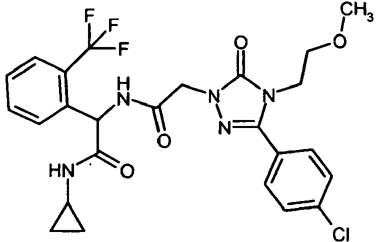 | 90A + 183A; 72% | Rₜ = 1.99 min [23] m/z = 552 (M+H)⁺ |
| **350** | 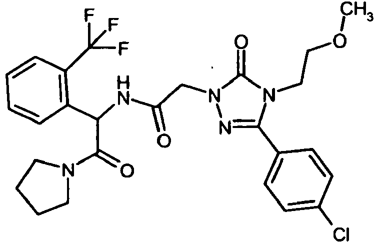 | 90A + 182A; 72% | Rₜ = 1.91 min [22] m/z = 566 (M+H)⁺ |
| **351** | 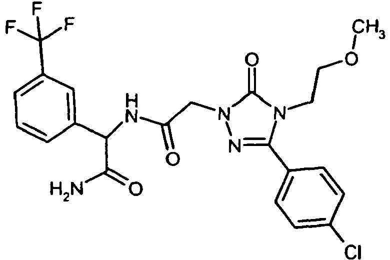 | 90A + 184A; 85% | Rₜ= 1.73 min [22] m/z = 512 (M+H)⁺ |
| **352** | 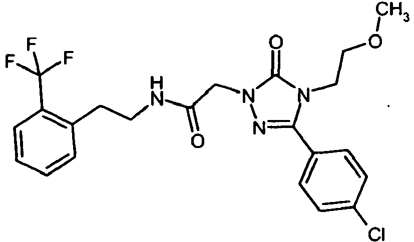 | 90A; 70% | Rₜ= 2.30min [7] m/z = 483 (M+H)⁺ |
| **353** | 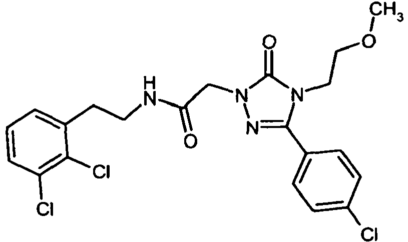 | 90A; 86% | Rₜ = 3.48 min [19] m/z = 483 (M+H)⁺ |

### Beispiel 354

### 2-[3-(4-Chlorphenyl)-4-(2-methoxyethyl)-5-oxo-4,5-dihydro-1H-1,2,4-triazol-1-yl]-N-[2,2-diethoxy-2-(pyridin-2-yl)ethyl)]-acetamid-Hydrochlorid

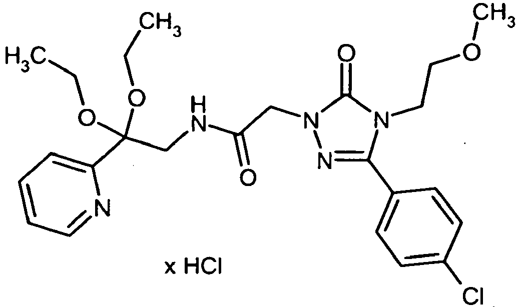

Aus der Carbonsäure aus Beispiel 90A (50 mg, 160 µmol) und 2,2-Diethoxy-2-(pyridyl-2-yl)ethylamin-Dihydrochlorid (68 mg, 240 µmol; Herstellung siehe Synthesis, 1980 (4), 329) werden nach der unter Beispiel 343 beschriebenen Methode 80 mg (92% d. Th.) der Titelverbindung erhalten.
LC/MS [Methode 8]: Rₜ = 2.09 min; m/z = 504 (M+H)⁺.

### Beispiel 355

### 2-[3-(4-Chlorphenyl)-4-(cyclopropyl)-5-oxo-4,5-dihydro-1H-1,2,4-triazol-1-yl]-N-{(1RS,2RS)-[2-(3-trifluormethyl-phenyl)cyclopropyl]}-essigsäureamid

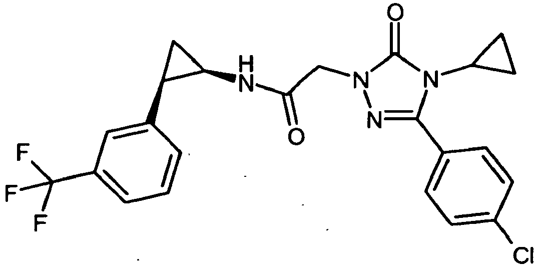

Die Carbonsäure aus Beispiel 88A (28 mg, 96 µmol) und HOBt (19 mg, 0.14 mmol) werden in 1 ml DMF vorgelegt und bei RT mit 28 mg (0.14 mmol) EDC versetzt. Nach 20 min werden 25 mg (0.11 mmol) der Verbindung aus Beispiel 203A und 23 µl *N,N*-Diisopropylethylamin (134 µmol) hinzugefügt und die Mischung 5 h bei RT gerührt. Das Gemisch wird danach direkt durch präparative HPLC (Methode 20) aufgetrennt. Man erhält 39 mg (86% d. Th.) der Titelverbindung.
LC/MS [Methode 8]: Rₜ = 2.57 min; m/z = 477 (M+H)⁺
¹H-NMR (400 MHz, DMSO-d₆): δ = 0.50-0.60 (m, 2H), 0.83-0.92 (m, 2H), 1.14 (m, 1H), 1.35 (m, 1H), 2.31 (m, 1H), 3.01 (m, 1H), 3.14 (m, 1H), 4.01 (d, 1H), 4.19 (d, 1H), 7.38-7.52 (m, 4H), 7.59 (d, 2H), 7.73 (d, 2H), 8.20 (d, 1H).

### Beispiel 356

### 2-[3-(4-Chlorphenyl)-4-(cyclopropyl)-5-oxo-4,5-dihydro-1H-1,2,4-triazol-1-yl]-N-{2,2-difluor-2-[3-(trifluormethyl)phenyl]ethyl}-essigsäureamid

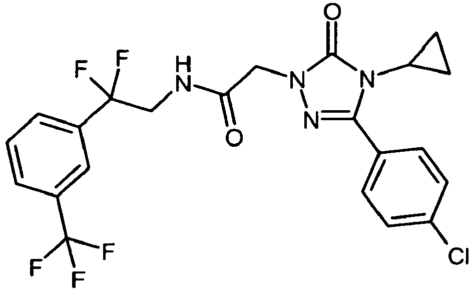

Nach der unter Beispiel 355 beschriebenen Methode erhält man ausgehend von Beispiel 88A und Beispiel 205A die Titelverbindung in 68% Ausbeute.
LC/MS [Methode 23]: Rₜ = 2.30 min; m/z = 501 (M+H)⁺
¹H-NMR (400 MHz, DMSO-d₆): δ = 0.54-0.60 (m, 2H), 0.86-0.93 (m, 2H), 3.17 (m, 1H), 3.94 (dt, 2H), 4.36 (s, 2H), 7.59 (d, 2H), 7.71-7.81 (m, 3H), 7.82-7.88 (m, 2H), 7.91 (d, 1H), 8.59 (t, 1H).

### Beispiel 357

### 2-[3-(4-Chlorphenyl)-5-oxo-4-(3,3,3-trifluor-2-hydroxypropyl)-4,5-dihydro-1H-1,2,4-triazol-1-yl]-N-{(1RS,2RS)-[2-(3-trifluormethyl-phenyl)cyclopropyl]}-essigsäureamid

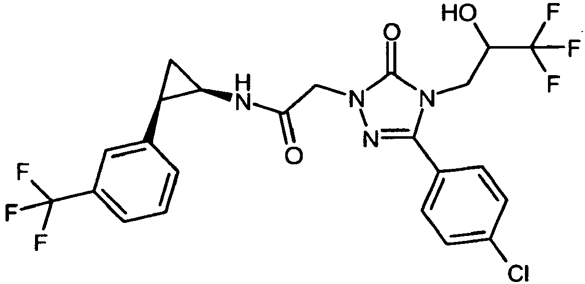

Die Carbonsäure aus Beispiel 229A (Enantiomer 1; 35 mg, 96 µmol) und HOBt (19 mg, 0.14 mmol) werden in 1 ml DMF vorgelegt und bei RT mit 28 mg (0.14 mmol) EDC versetzt. Nach 20 min werden 25 mg (0.11 mmol) der Verbindung aus Beispiel 203A und 23 µl (0.13 mmol) *N,N-*Diisopropylethylamin hinzugefügt und die Mischung über Nacht bei RT gerührt. Das Gemisch wird danach direkt durch präparative HPLC (Methode 20) aufgetrennt. Man erhält 46 mg (88% d. Th.) der Titelverbindung.
LC/MS [Methode 8]: Rₜ = 2.67 min; m/z = 549 (M+H)⁺
¹H-NMR (400 MHz, DMSO-d₆): δ = 1.15 (q, 1H), 1.37 (dt, 1H), 2.33 (q, 1H), 3.03 (m, 1H), 3.80 (dd, 1H), 3.93 (br. d, 1 H), 4.06 (dd, 1H), 4.25 (m, 1H), 4.26 (dd, 1H), 6.89 (d, 1H), 7.40-7.52 (m, 4H), 7.60-7.65 (m, 2H), 7.66-7.72 (m, 2H), 8.25 (dd, 1H).

Auf analoge Weise werden die Verbindungen in der folgenden Tabelle hergestellt. Sofern nichts anderes angegeben, sind die Amin-Edukte kommerziell erhältlich.

| **Beispiel Nr.** | **Struktur** | **Edukt; Ausbeute** | **LC/MS: Rₜ [Methode], m/z** |
|---|---|---|---|
| **358** | 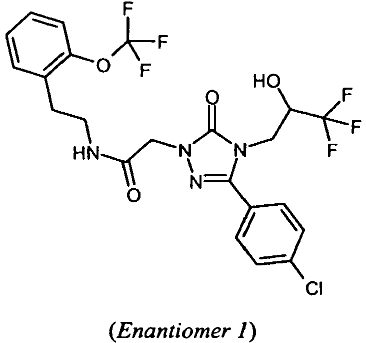 | 229A; 85% | Rₜ= 3.70 min [19] m/z = 553 (M+H)⁺ |
| **359** | 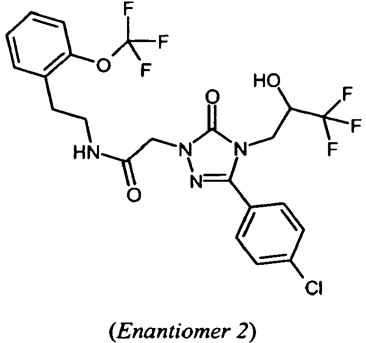 | 230A; 83% | Rₜ= 3.70 min [19] m/z = 553 (M+H)⁺ |
| **360** | 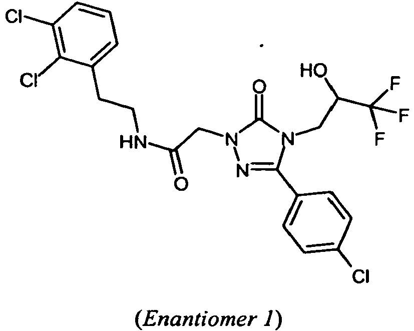 | 229A; 84% | Rₜ = 3.69 min [19] m/z = 537 (M+H)⁺ |
| **361** | 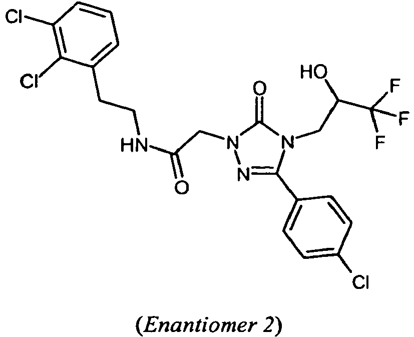 | 230A; 82% | Rₜ = 3.70 min [19] m/z = 537 (M+H)⁺ |
| **362** | 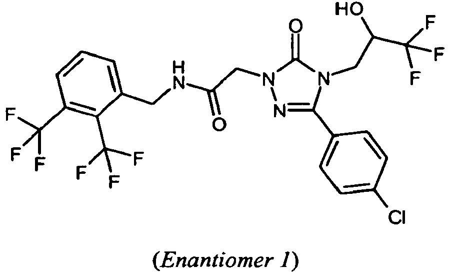 | 229A; 80% | Rₜ = 3.79 min [19] m/z = 591 (M+H)⁺ |
| **363** | 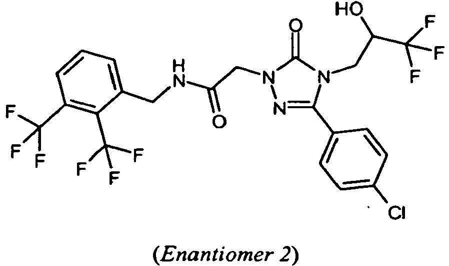 | 230A; 7.4% | Rₜ= 3.78 min [19] m/z = 591 (M+H)⁺ |
| **364** | 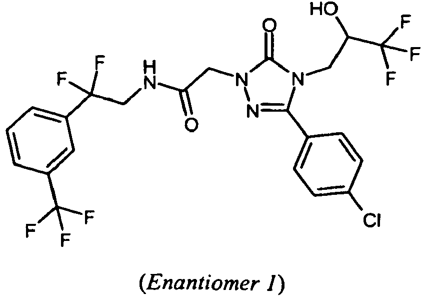 | 229A; 80% | Rₜ= 2.39 min [23] m/z = 573 (M+H)⁺ |

### Beispiel 365

### 4-{3-(4-Chlorphenyl)-1-[2-({2-methyl-2-[2-(trifluormethyl)phenyl]propyl}amino)-2-oxoethyl]-5-oxo-1,5-dihydro-4H-1,2,4-triazol-4-yl}-butansäure-tert.-butylester

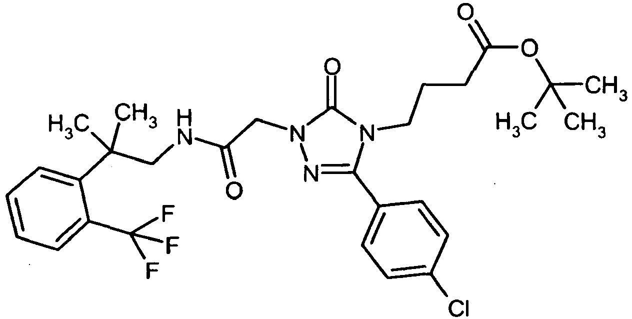

Die Carbonsäure aus Beispiel 224A (20 mg, 51 µmol), HOBt (13 mg, 91 µmol) und *N,N*-Diisopropylethylamin (13 µl, 76 µmol) werden in 0.75 ml DMF vorgelegt und bei RT mit 17.4 mg (91 µmol) EDC versetzt. Nach 20 min werden 17 mg (66 µmol) der Verbindung aus Beispiel 166A hinzugefügt und die Mischung über Nacht bei RT gerührt. Das Gemisch wird danach direkt durch präparative HPLC (Methode 20) aufgetrennt. Man erhält 24 mg (80% d. Th.) der Titelverbindung.
LC/MS [Methode 22]: Rₜ = 2.54 min; m/z = 595 (M+H)⁺
¹H-NMR (400 MHz, DMSO-d₆): δ = 1.35 (s, 9H), 1.39 (s, 6H), 1.69 (quin, 2H), 2.14 (t, 2H), 3.45 (d, 2H), 3.75 (t, 2H), 4.45 (s, 2H), 7.45 (t, 1H), 7.56-7.69 (m, 6H), 7.75 (d, 1H), 7.91 (t, 1H).

### Beispiel 366

### 4-{3-(4-Chlorphenyl)-1-[2-({2-(dimethylamino)-2-oxo-1-[3-(trifluormethyl)phenyl]ethyl}amino)-2-oxoethyl]-5-oxo-1,5-dihydro-4H-1,2,4-triazol-4-yl}-butansäure-tert.-butylester

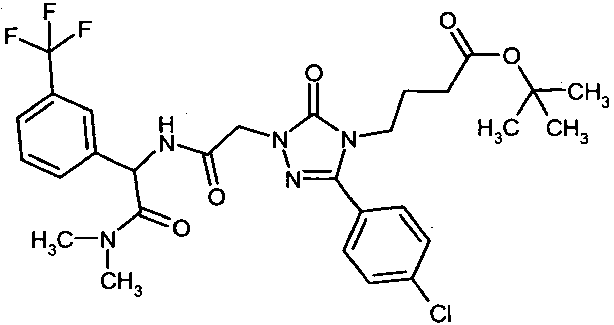

Analog zu Beispiel 365 wird ausgehend von Beispiel 224A und Beispiel 175A (als Trifluoracetatsalz) die Titelverbindung erhalten.
LC/MS [Methode 22]: Rₜ = 2.32 min; m/z = 624 (M+H)⁺
¹H-NMR (400 MHz, DMSO-d₆): δ = 1.32 (s, 9H), 1.69 (quin, 2H), 2.14 (t, 2H), 2.85 (s, 3H), 2.99 (s, 3H), 3.75 (t, 2H), 4.50 (m, 2H), 5.99 (d, 1H), 7.58-7.72 (m, 7H), 7.77 (s, 1H), 8.99 (d, 1H).

### Beispiel 367

### 4-{3-(4-Chlorphenyl)-1-[2-({2-methyl-2-[2-(trifluormethyl)phenyl]propyl}amino)-2-oxoethyl]-5-oxo-1,5-dihydro-4H-1,2,4-triazol-4-yl}-butansäure

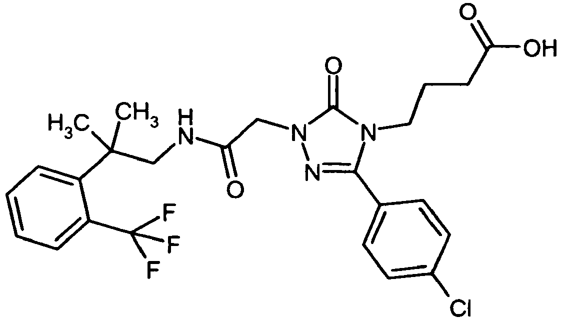

Die Verbindung aus Beispiel 365 (22 mg, 37 µmol) wird über Nacht bei RT mit 1 ml einer 4 M Chlorwasserstoff-Lösung in Dioxan verrührt. Das Lösungsmittel wird danach am Rotationsverdampfer entfernt. Der Rückstand wird in DMSO aufgenommen und durch präparative HPLC (Methode 20) gereinigt. Man erhält 14 mg (70% d. Th.) der Titelverbindung.
LC/MS [Methode 22]: Rₜ = 1.98 min, m/z = 539 (M+H)⁺
¹H-NMR (400 MHz, DMSO-d₆): δ = 1.39 (s, 6H), 1.69 (quin, 2H), 2.18 (t, 2H), 3.45 (d, 2H), 3.74 (t, 2H), 4.35 (s, 2H), 7.44 (t, 1H), 7.56-7.63 (m, 3H), 7.63-7.68 (m, 3H), 7.77 (d, 1H), 7.91 (t, 1H), 12.12 (br. s, 1 H).

### Beispiel 368

### 4-{3-(4-Chlorphenyl)-1-[2-({2-(dimethylamino)-2-oxo-1-[3-(trifluormethyl)phenyl]ethyl}amino)-2-oxoethyl]-5-oxo-1,5-dihydro-4H-1,2,4-triazol-4-yl}-butansäure

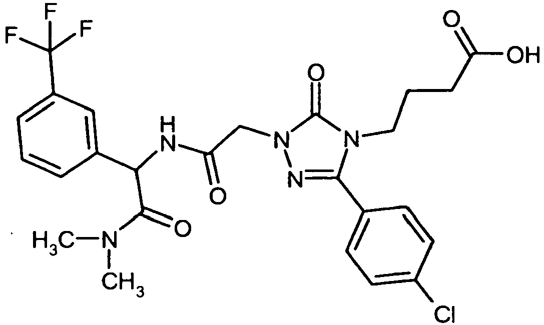

Die Titelverbindung wird analog zu Beispiel 367 ausgehend von Beispiel 366 erhalten.
LC/MS [Methode 22]: Rₜ = 1.80 min; m/z = 568 (M+H)⁺
¹H-NMR (400 MHz, DMSO-d₆): δ = 1.69 (quin, 2H), 2.17 (t, 2H), 2.85 (s, 3H), 2.99 (s, 3H), 3.75 (t, 2H), 4.50 (m, 2H), 5.99 (d, 1H), 7.57-7.73 (m, 7H), 7.76 (s, 1H), 8.99 (d, 1H), 12.11 (br. s, 1H).

### Beispiel 369

### 2-[3-(4-Chlorphenyl)-4-(2-fluorbenzyl)-5-oxo-4,5-dihydro-1H-1,2,4-triazol-1-yl]-N-[1-(isochinolin-1-yl)ethyl]-essigsäureamid

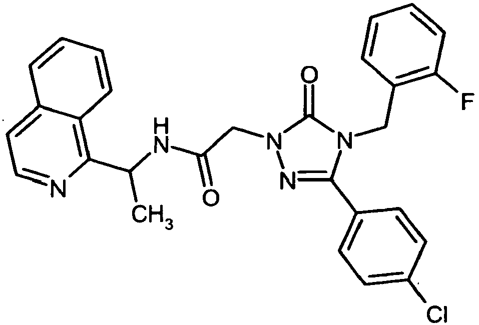

Die Titelverbindung wird analog zu Beispiel 264 aus der Carbonsäure aus Beispiel 156A (40 mg, 0.11 mmol) und 26.7 mg (0.16 mmol) 1-Isochinolin-1-ylethylamin [Herstellung siehe Chem. Ber. 108, 3771-3778 (1975)] hergestellt. Ausbeute: 70% d. Th.
LC/MS [Methode 17]: Rₜ = 3.47 min; m/z = 516 (M+H)⁺.

### Beispiel 370

### 2-[3-(4-Chlorphenyl)-4-(cyclopropyl)-5-oxo-4,5-dihydro-1H-1,2,4-triazol-1-yl]-N-{3-dimethyl-amino-3-oxo-2-[2-(trifluormethyl)phenyl]propyl}-essigsäureamid

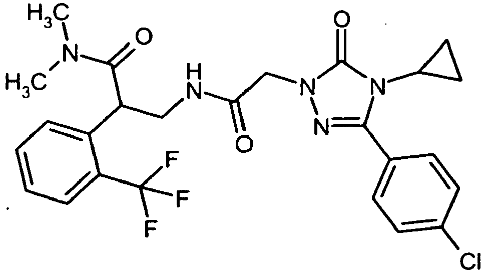

Die Verbindung aus Beispiel 317 (55 mg, 102 µmol) wird analog zu Beispiel 300 zur entsprechenden Carbonsäure (52 mg) verseift. 30 mg dieser Säure (59 µmol) werden dann analog zu Beispiel 301 mit Dimethylamin zur Titelverbindung (31 mg, 98% d. Th.) umgesetzt.
LC/MS [Methode 22]: Rₜ = 1.93 min; m/z = 536 (M+H)⁺.

### Beispiel 371

### 2-[3-(4-Chlorphenyl)-4-allyl-5-oxo-4,5-dihydro-1H-1,2,4-triazol-1-yl]-N-{1-methyl-1-[3-(trifluormethyl)phenyl]ethyl}-essigsäureamid

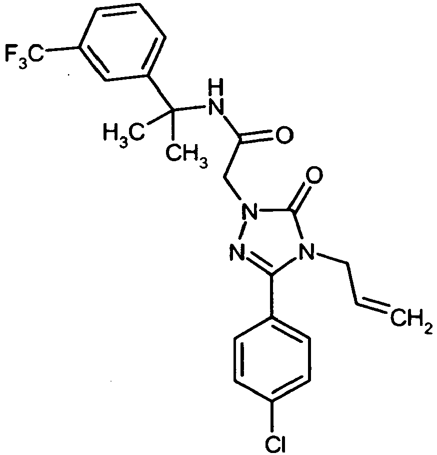

1.98 g (6.74 mmol) [3-(4-Chlorphenyl)-4-allyl-5-oxo-4,5-dihydro-1*H*-1,2,4-triazol-1-yl]-essigsäure aus Beispiel 218A und 1.51 g (7.42 mmol) 1-Methyl-1-[(3-trifluormethyl)phenyl]ethylamin aus Beispiel 1 A werden in 50 ml DMF vorgelegt und mit 1.09 g (8.09 mmol) HOBt versetzt. Nach 10 min Rühren werden 1.68 g (8.76 mmol) EDC-Hydrochlorid zugegeben und das Gemisch über Nacht bei RT gerührt. Zur Aufarbeitung wird das Reaktionsgemisch mit 500 ml Wasser verrührt. Anschließend wird der ausgefallene Niederschlag abgesaugt, mit Wasser gewaschen und im Hochvakuum getrocknet. Man erhält so 2.41 g (75% d. Th.) der Zielverbindung.
LC/MS [Methode 7]: Rₜ = 2.49 min; MS [ESIpos]: m/z = 479 (M+H)⁺
¹H-NMR (400 MHz, DMSO-d₆): δ=1.60 (s, 6H), 4.30-4.38 (m, 4H), 4.50 (s, 2H), 4.92 (d, 1H), 5.11 (d, 1 H), 5.82 (m, 1 H), 7.50-7.70 (m, 8H), 8.55 (s, 1 H).

Auf analoge Weise werden die folgenden Verbindungen hergestellt:

| **Beispiel Nr.** | **Struktur** | **LC/MS oder HPLC, MS Rₜ [Methode]** |
|---|---|---|
| **372** | 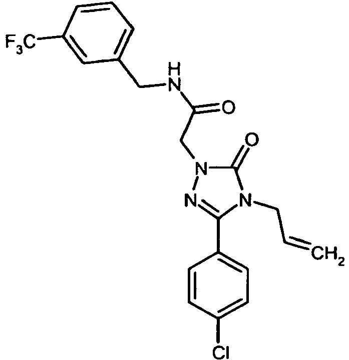 | LC/MS: Rₜ = 2.55 min [8] [ESIpos]: m/z =451 (M+H)⁺ |
| **373** | 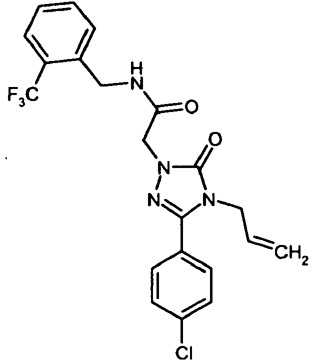 | LC/MS: Rₜ = 2.60 min [8] [ESIpos]: m/z = 451 (M+H)⁺ |
| **374** | 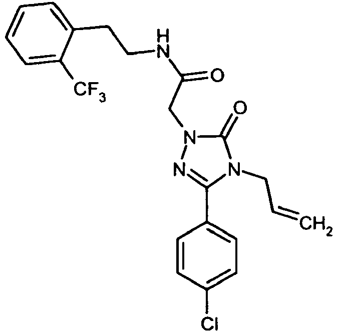 | LC/MS: Rₜ = 3.72 min [17] [ESIpos]: m/z = 465 (M+H)⁺ |
| **375** | 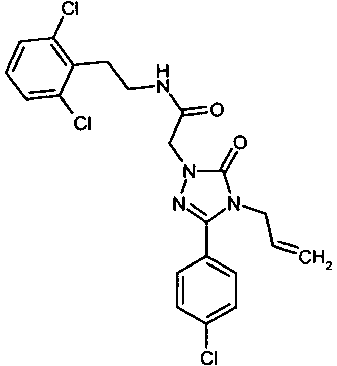 | LC/MS: Rₜ = 3.72 min [17] [ESIpos]: m/z = 465 (M+H)⁺ |
| **376** | 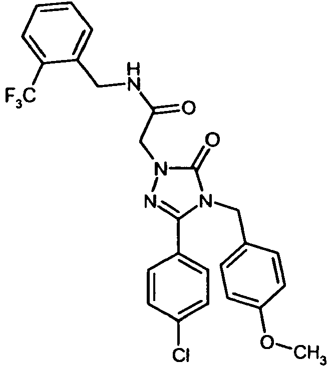 | LC/MS: Rₜ = 2.51 min [7] [ESIpos]: m/z = 531 (M+H)⁺ |
| **377** | 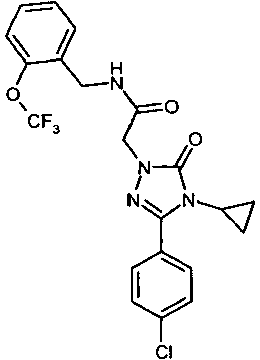 | [ESIpos]: m/z = 467 (M+H)⁺ |

### Beispiel 378

### 2-[3-(4-Chlorphenyl)-4-(2-methoxyethyl)-5-oxo-4,5-dihydro-1H-1,2,4-triazol-1-yl]-N-1-methyl-1-[3-(trifluormethyl)phenyl]ethyl-acetamid

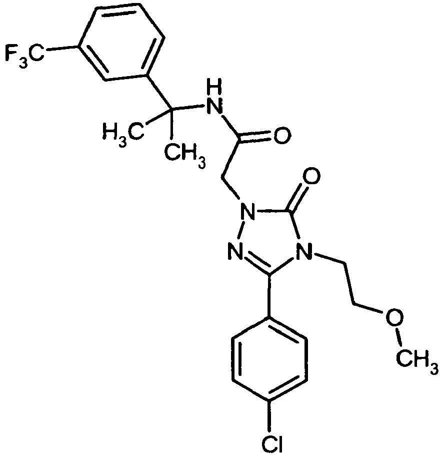

2.90 g (9.30 mmol) [3-(4-Chlorphenyl)-4-(2-methoxyethyl)-5-oxo-4,5-dihydro-1*H*-1,2,4-triazol-1-yl]-essigsäure aus Beispiel 90A und 2.08 g (10.23 mmol) 1-Methyl-1-[(3-trifluormethyl)phenyl]-ethylamin aus Beispiel 1A werden in 60 ml DMF vorgelegt und mit 1.51 g (11.2 mmol) HOBt versetzt. Nach 10 min Rühren werden 2.32 g (12.1 mmol) EDC-Hydrochlorid zugegeben und das Gemisch über Nacht bei RT gerührt. Zur Aufarbeitung wird das Reaktionsgemisch mit 500 ml Wasser verrührt. Anschließend wird der ausgefallene Niederschlag abgesaugt, mit Wasser gewaschen und im Hochvakuum getrocknet. Man erhält so 3.27 g (71% d. Th.) der Zielverbindung.
LC/MS [Methode 19]: Rₜ = 3.60 min; MS [ESIpos]: m/z = 497 (M+H)⁺
¹H-NMR (400 MHz, DMSO-d₆): δ = 1.60 (s, 6H), 3.10 (s, 3H), 3.45 (t, 2H), 3.84 (t, 2H), 4.47 (s, 2H), 7.48-7.72 (m, 8H), 8.56 (s, 1H).

Auf analoge Weise werden die folgenden Verbindungen hergestellt:

| **Beispiel Nr.** | **Struktur** | **LC/MS oder HPLC, MS Rₜ [Methode]** |
|---|---|---|
| **379** | 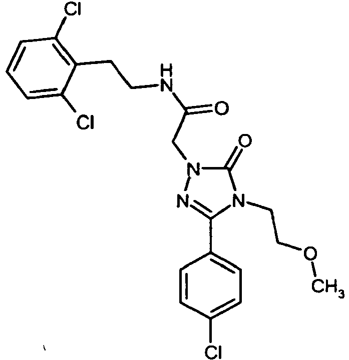 | LC/MS: Rₜ = 2.56 min [8] [ESIpos]: m/z = 483/485 (M+H)⁺ |
| **380** | 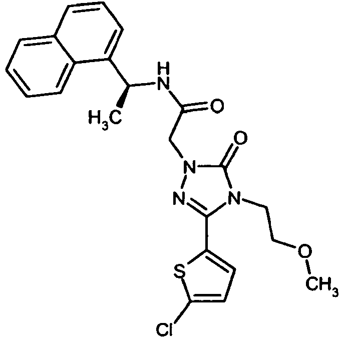 | LC/MS: Rₜ = 2.36 min [7] [ESIpos]: m/z = 471 (M+H)⁺ |
| **381** | 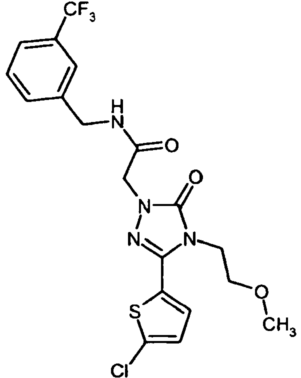 | LC/MS: Rₜ = 2.51 min [8] [ESIpos]: m/z = 475 (M+H)⁺ |
| **382** | 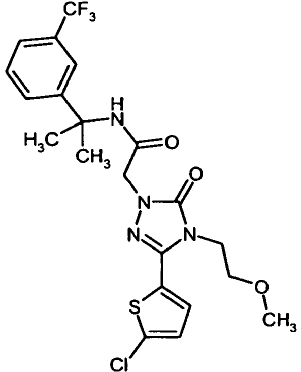 | LC/MS: Rₜ = 3.02 min [8] [ESIpos]: m/z = 503 (M+H)⁺ |
| **383** | 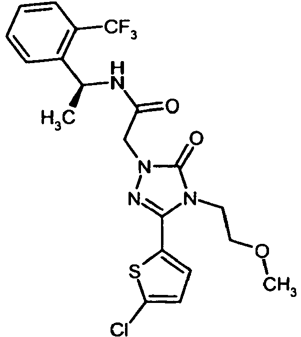 | LC/MS: Rₜ = 3.66 min [17] [ESIpos]: m/z = 489 (M+H)⁺ |
| **384** | 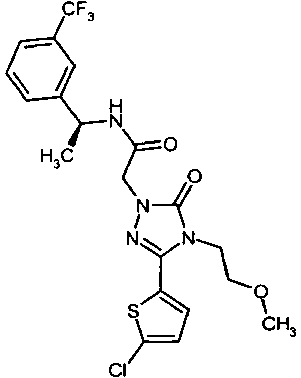 | LC/MS: Rₜ = 3.66 min [8] [ESIpos]: m/z = 489 (M+H)⁺ |
| **385** | 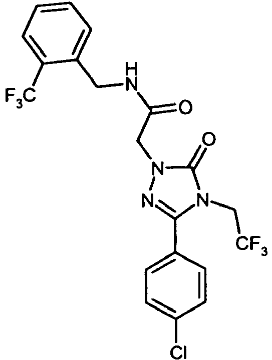 | LC/MS: Rₜ = 2.59 min [8] [ESIpos]: m/z = 493 (M+H)⁺ |
| **386** | 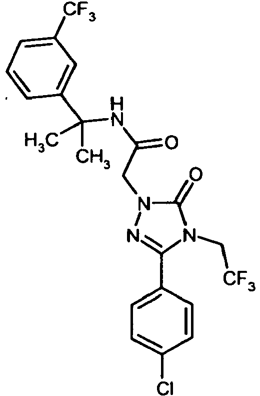 | LC/MS: Rₜ = 3.95 min [17] [ESIpos]: m/z = 521 (M+H)⁺ |
| **387** | 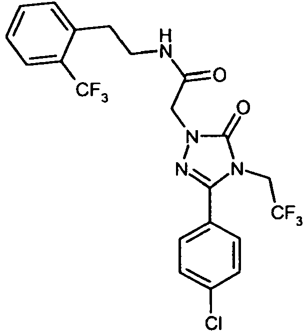 | LC/MS: Rₜ = 3.82 min [17] [ESIpos]: m/z = 507 (M+H)⁺ |
| **388** | 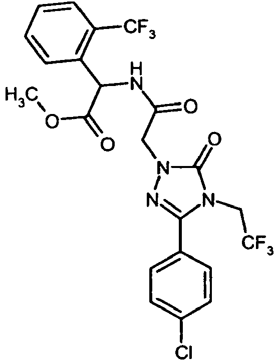 | LC/MS: Rₜ = 3.60 min [19] [ESIpos] : m/z = 551 (M+H)⁺ |
| **389** | 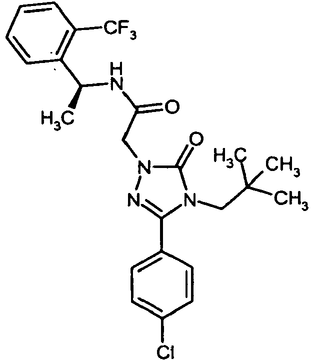 | LC/MS: Rₜ =4.02 min [17] [ESIpos]: m/z = 495 (M+H)⁺ |
| **390** | 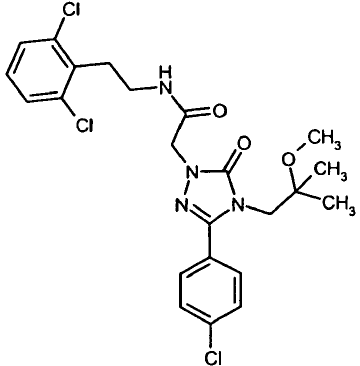 | LC/MS: Rₜ = 3.78 min [17] [ESIpos]: m/z = 511/513 (M+H)⁺ |
| **391** | 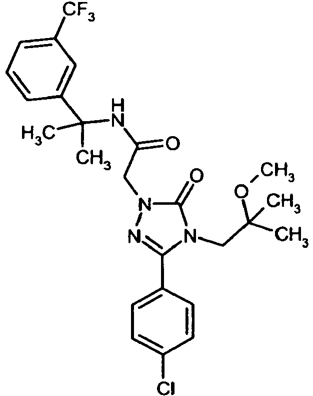 | LC/MS: Rₜ = 3.91 min [17] [ESIpos]: m/z = 525 (M+H)⁺ |
| **392** | 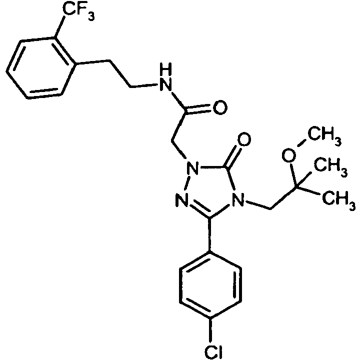 | LC/MS: Rₜ = 3.77 min [17] [ESIpos]: m/z = 511 (M+H)⁺ |
| **393** | 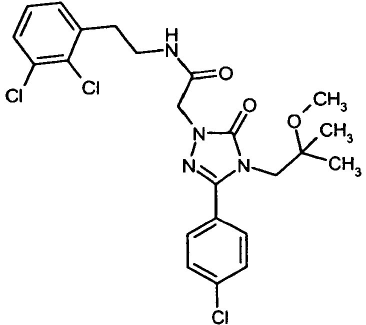 | LC/MS: Rₜ = 3.64 min [19] [ESIpos]: m/z = 511/513 (M+H)⁺ |
| **394** | 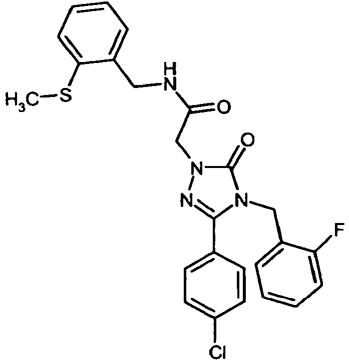 | LC/MS: Rₜ = 2.16 min [22] [ESIpos]: m/z = 497 (M+H)⁺ |
| **395** | 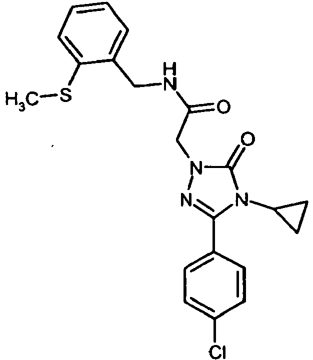 | MS [DCI]: m/z = 429 (M+H)⁺, 446 (M+NH₄)⁺ |

### Beispiel 396

### 2-[3-(4-Chlorphenyl)-4-(2-hydroxyethyl)-5-oxo-4,5-dihydro-1H-1,2,4-triazol-1-yl]-N-1-methyl-1-[3-(trifluormethyl)phenyl]ethyl-acetamid

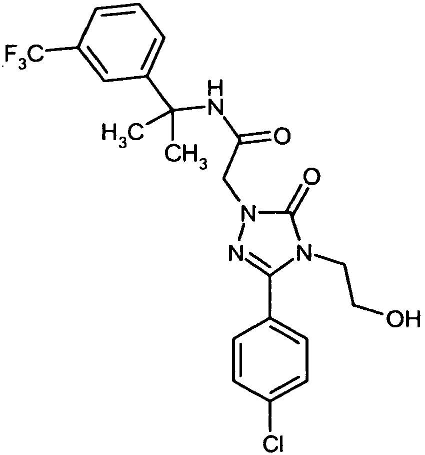

2.95 g (5.94 mmol) 2-[3-(4-Chlorphenyl)-4-(2-methoxyethyl)-5-oxo-4,5-dihydro-1H-1,2,4-triazol-1-yl]-N-1-methyl-1-[3-(trifluormethyl)phenyl]ethyl-acetamid aus Beispiel 378 werden in 40 ml Trichlormethan gelöst und bei RT mit 6.76 ml (47.5 mmol) Iodtrimethylsilan versetzt. Es wird 1 h bei RT gerührt. Anschließend wird unter Eiskühlung eine Mischung aus 40 ml Methanol und 5.99 g (47.5 mmol) Natriumsulfit unter kräftigem Rühren hinzugefügt. Die Reaktionslösung wird mit 100 ml Wasser verdünnt und zweimal mit je 50 ml Essigsäureethylester extrahiert. Die vereinigten organischen Phasen werden über Natriumsulfat getrocknet, filtriert und im Vakuum eingeengt. Das Rohprodukt wird in 50 ml Isopropanol gelöst. Nach Zugabe von ca. 50 ml Wasser fällt das gewünschte Produkt aus. Es wird eine Stunde bei RT gerührt. Anschließend werden die Kristalle abgesaugt und mit wenig Wasser und wenig Cyclohexan gewaschen. Nach Trocknen im Hochvakuum erhält man so 2.56 g (89% d. Th.) der Zielverbindung.
LC/MS [Methode 17]: Rₜ = 3.45 min; MS [ESIpos]: m/z = 483 (M+H)⁺
¹H-NMR (400 MHz, DMSO-d₆): δ = 1.60 (s, 6H), 3.54 (q, 2H), 3.75 (t, 2H), 4.47 (s, 2H), 5.00 (t, 1H), 7.48-7.78 (m, 8H), 8.55 (s, 1H).

Auf analoge Weise werden die folgenden Verbindungen hergestellt:

| **Beispiel Nr.** | **Struktur** | **LC/MS oder HPLC, MS Rₜ [Methode]** |
|---|---|---|
| **397** | 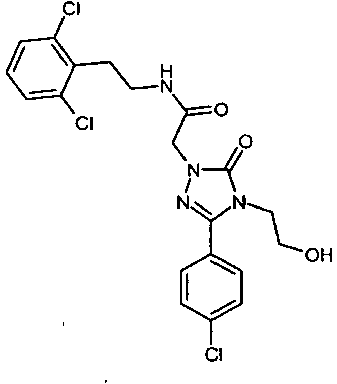 | LC/MS: Rₜ = 3.12 min [19] [ESIpos]: m/z = 469/471 (M+H)⁺ |
| **398** | 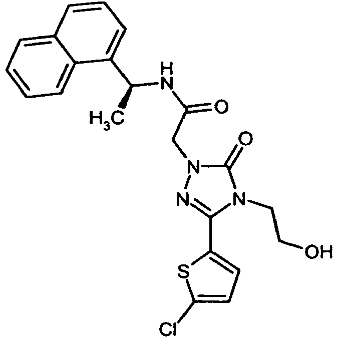 | LC/MS: Rₜ = 2.09 min [7] [ESIpos]: m/z = 457 (M+H)⁺ |
| **399** | 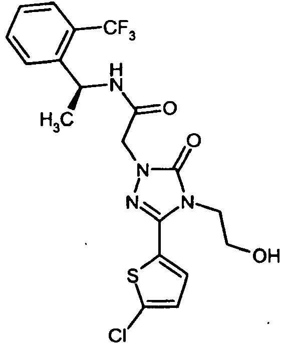 | LC/MS: Rₜ = 2.07 min [7] [ESIpos]: m/z = 475 (M+H)⁺ |
| **400** | 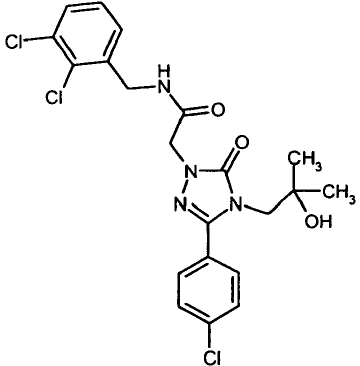 | LC/MS: Rₜ = 3.52 min [17] [ESIpos]: m/z = 497/499 (M+H)⁺ |

### Beispiel 401

### 2-[3-(4-Chlorphenyl)-4-(2-oxoethyl)-5-oxo-4,5-dihydro-1H-1,2,4-triazol-1-yl]-N-1-methyl-1-[3-(trifluormethyl)phenyl]ethyl-acetamid

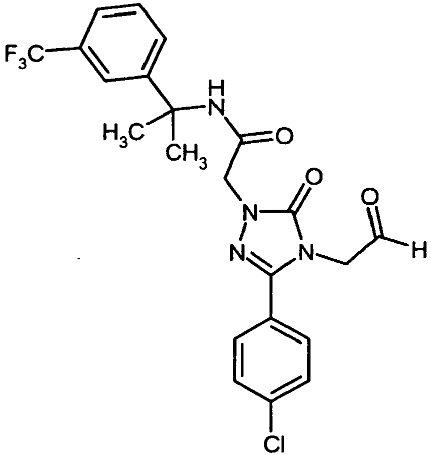

0.5 g (1.04 mmol) 2-[3-(4-Chlorphenyl)-4-(2-hydroxyethyl)-5-oxo-4,5-dihydro-1*H*-1,2,4-triazol-1-yl]-N-1-methyl-1-[3-(trifluormethyl)phenyl]ethyl-acetamid aus Beispiel 396 werden in 30 ml Dichlormethan gelöst und bei 0°C mit 0.57 g (1.35 mmol) 1,1,1-Tris(acetoxy)-1,1-dihydro-1,2-benziodoxol-3-(1*H*)-on (Dess-Martin-Periodinan) versetzt. Es wird 18 h bei RT gerührt. Danach werden weitere 0.38 g (0.90 mmol) Dess-Martin-Periodinan hinzugefügt. Nach 3 h wird die Suspension direkt mittels Flash-Chromatographie an Kieselgel (Eluent: Dichlormethan/Essigsäureethylester/Methanol 100: 100: 1) gereinigt. Das so erhaltene Produkt wird in 50 ml Essigsäureethylester aufgenommen und einmal mit 50 ml 0.05 N Salzsäure gewaschen. Die organische Phase wird über Natriumsulfat getrocknet, filtriert und im Vakuum eingeengt. Man erhält so 0.32 g (64% d. Th.) der Zielverbindung.
LC/MS [Methode 19]: Rₜ = 3.21 min; MS [ESIpos]: m/z = 481 (M+H)⁺
¹H-NMR (400 MHz, CDCl₃): δ = 1.71 (s, 6H), 4.52 (s, 2H), 4.49 (s, 2H), 6.55 (s, 1H), 7.44-7.70 (m, 8H), 9.61 (s, 1H).

Auf analoge Weise wird die folgende Verbindung hergestellt:

| **Beispiel Nr.** | **Struktur** | **LC/MS oder HPLC, MS Rₜ [Methode]** |
|---|---|---|
| **402** | 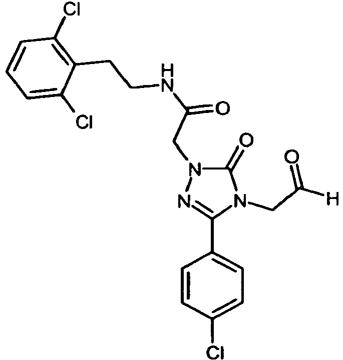 | LC/MS: Rₜ = 2.20 min [8] [ESIpos]: m/z = 467/469 (M+H)⁺ |

### Beispiel 403

### 2-[3-(4-Chlorphenyl)-4-(3-methoxybenzyl)-5-oxo-4,5-dihydro-1H-1,2,4-triazol-1-yl]-N-1-methyl-1 - [3-(trifluormethyl)phenyl]ethyl-acetamid

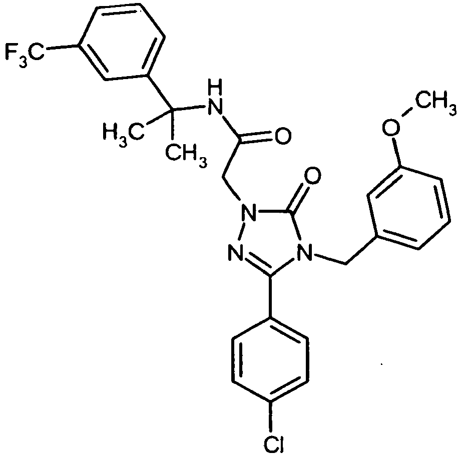

75 mg (0.17 mmol) 2-[3-(4-Chlorphenyl)-5-oxo-4,5-dihydro-1*H*-1,2,4-triazol-1-yl]-*N*-{1-methyl-1-[3-(trifluormethyl)phenyl]ethyl}-essigsäureamid aus Beispiel 241A werden mit 84 mg (0.26 mmol) Cäsiumcarbonat in 2.5 ml Aceton suspendiert und mit 45 mg (0.22 mmol) 3-Methoxybenzylchlorid versetzt. Es wird 6 h bei 50°C und danach 18 h bei RT gerührt. Die Suspension wird mit 1 ml Wasser versetzt und dreimal mit Essigsäureethylester extrahiert. Die vereinigten organischen Phasen werden über Natriumsulfat getrocknet und filtriert. Nach Einengen der organischen Phase wird das Rohprodukt durch präparative HPLC [Methode 10] gereinigt. Man erhält so 55.6 mg (58% d. Th.) der Zielverbindung.
LC/MS [Methode 18]: Rₜ = 2.78 min; MS [ESIpos]: m/z = 559 (M+H)⁺
¹H-NMR (400 MHz, DMSO-d₆): δ = 1.60 (s, 6H), 3.60 (s, 3H), 4.54 (s, 2H), 4.95 (s, 2H), 6.59-6.64 (m, 2H), 6.75-6.80 (m, 1H), 7.18 (t, 1H), 7.46-7.72 (m, 8H), 8.58 (s, 1H).

Auf analoge Weise werden die folgenden Verbindungen hergestellt:

| **Beispiel Nr.** | **Struktur** | **LC/MS oder HPLC, MS Rₜ [Methode]** |
|---|---|---|
| **404** | 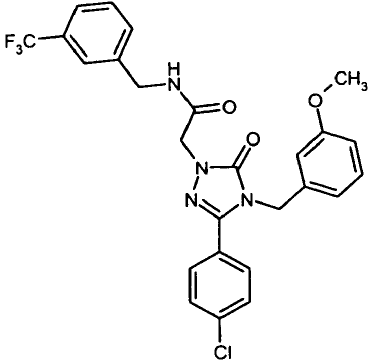 | LC/MS: Rₜ = 2.66 min [18] [ESIpos]: m/z = 531 (M+H)⁺ |
| **405** | 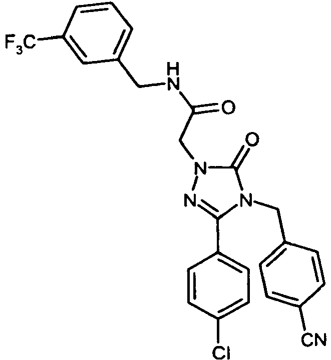 | LC/MS: Rₜ = 2.43 min [7] [ESIpos]: m/z = 526 (M+H)⁺ |
| **406** | 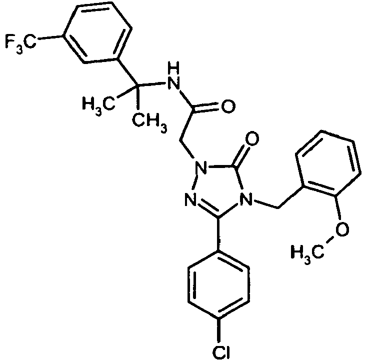 | LC/MS: Rₜ = 2.73 min [7] [ESIpos]: m/z = 559 (M+H)⁺ |
| **407** | 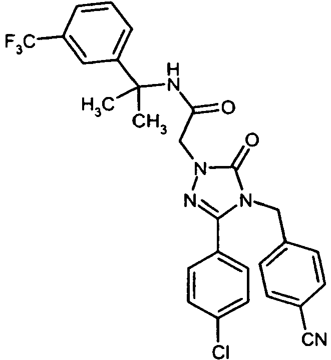 | LC/MS: Rₜ = 2.67 min [18] [ESIpos]: m/z = 554 (M+H)⁺ |
| **408** | 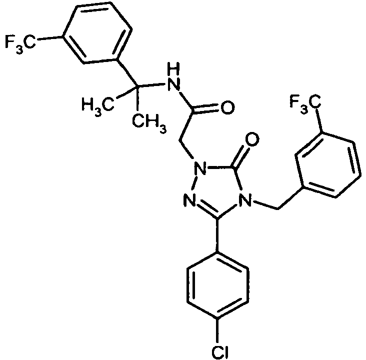 | LC/MS: Rₜ=2.91 min [18] [ESIpos]: m/z = 597 (M+H)⁺ |
| **409** | 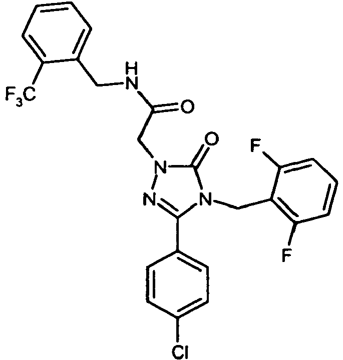 | LC/MS: Rₜ = 2.47 min [7] [ESIpos]: m/z = 537 (M+H)⁺ |
| **410** | 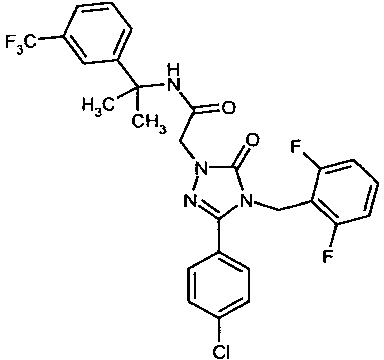 | LC/MS: Rₜ = 2.63 min [7] [ESIpos]: m/z = 565 (M+H)⁺ |
| **411** | 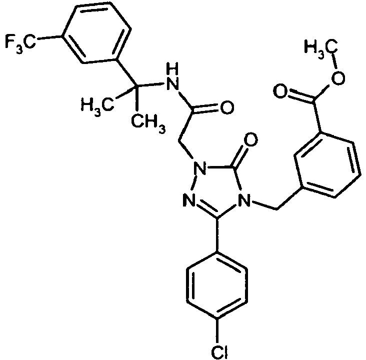 | LC/MS: Rₜ = 3.86 min [19] [ESIpos]: m/z = 587 (M+H)⁺ |
| **412** | 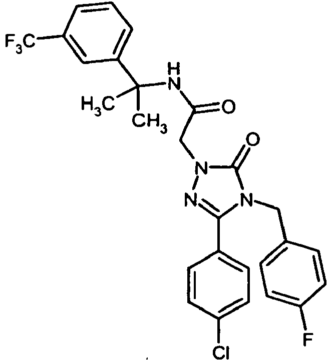 | LC/MS: Rₜ = 2.91 min [8] [ESIpos]: m/z = 547 (M+H)⁺ |
| **413** | 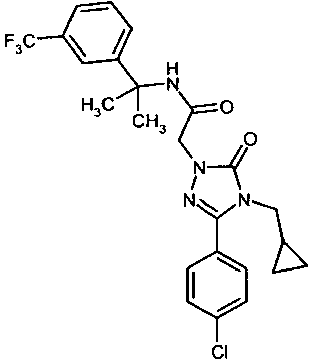 | LC/MS: Rₜ = 2.55 min [7] [ESIpos]: m/z = 493 (M+H)⁺ |
| **414** | 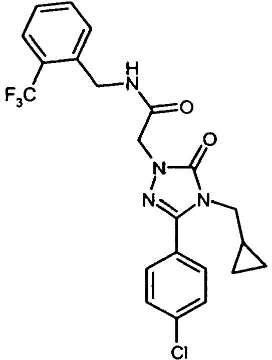 | LC/MS: Rₜ = 3.58 min [19] [ESIpos]: m/z = 465 (M+H)⁺ |
| **415** | 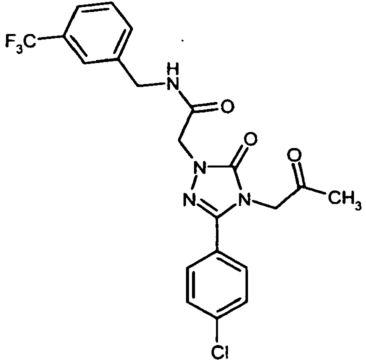 | LC/MS: Rₜ = 2.45 min [8] [ESIpos]: m/z = 467 (M+H)⁺ |
| **416** | 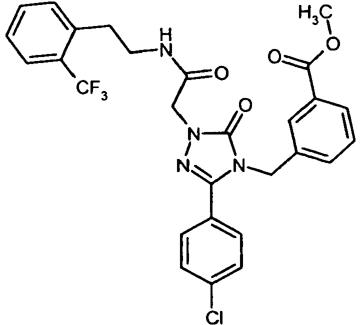 | LC/MS: Rₜ = 3.73 min [19] [ESIpos]: m/z = 573 (M+H)⁺ |
| **417** | 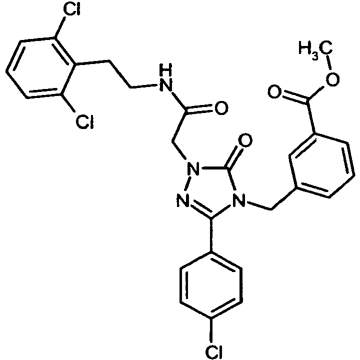 | LC/MS: Rₜ = 3.94 min [19] [ESIpos]: m/z = 573/575 (M+H)⁺ |
| **418** | 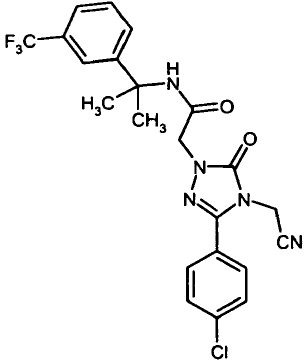 | LC/MS: Rₜ = 2.61 min [8] [ESIpos]: m/z = 478 (M+H)⁺ |
| **419** | 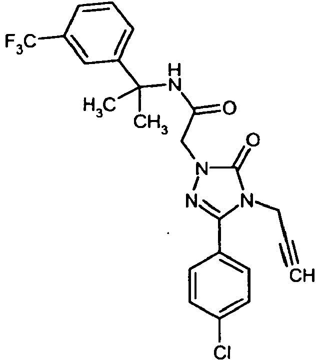 | LC/MS: Rₜ = 3.80 min [17] [ESIpos]: m/z = 477 (M+H)⁺ |
| **420** | 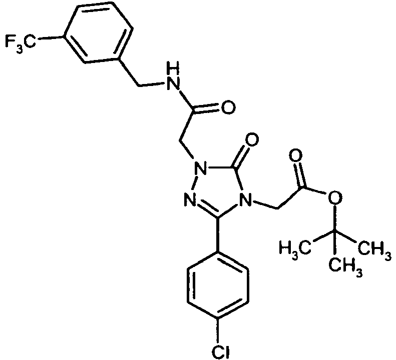 | LC/MS: Rₜ = 2.57 min [7] [ESIpos]: m/z = 525 (M+H)⁺ |
| **421** | 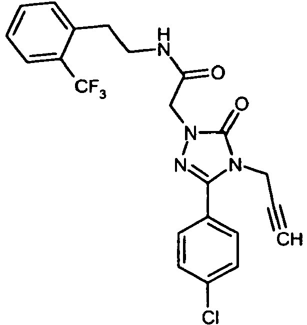 | LC/MS: Rₜ = 3.66 min [17] [ESIpos]: m/z = 463 (M+H)⁺ |
| **422** | 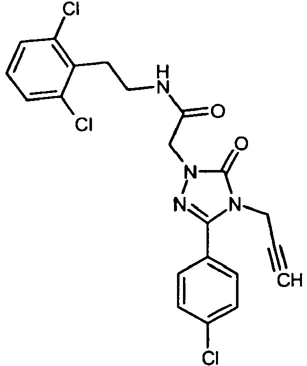 | LC/MS: Rₜ = 3.67 min [17] [ESIpos]: m/z = 463/465 (M+H)⁺ |

### Beispiel 423

### 2-[3-(4-Chlorphenyl)-4-(2-methylprop-2-en-1-yl)-5-oxo-4,5-dihydro-1H-1,2,4-triazol-1-yl]-N-1-methyl-1-[3-(trifluormethyl)phenyl]ethyl-acetamid

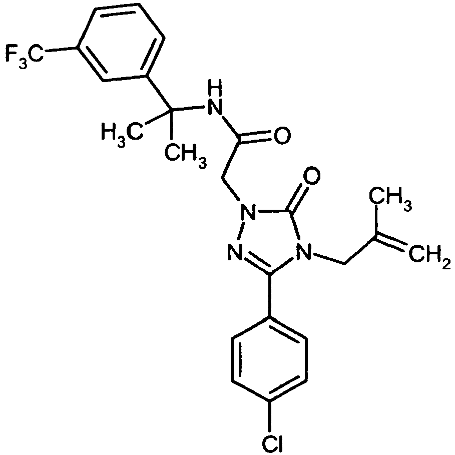

75 mg (0.17 mmol) 2-[3-(4-Chlorphenyl)-5-oxo-4,5-dihydro-1*H*-1,2,4-triazol-1-yl]-*N*-{1-methyl-1-[3-(trifluormethyl)phenyl]ethyl}-essigsäureamid aus Beispiel 241A werden mit 84 mg (0.26 mmol) Cäsiumcarbonat in einem Gemisch aus 2.5 ml Aceton und 0.5 ml DMF suspendiert und mit 185 mg (1.12 mmol) 1-Brom-2-fluor-2-methylpropan versetzt. Es wird 18 h bei 50°C gerührt. Die Suspension wird danach mit 1 ml Wasser versetzt und dreimal mit Essigsäureethylester extrahiert. Die vereinigten organischen Phasen werden über Natriumsulfat getrocknet und filtriert. Nach Einengen wird das Rohprodukt durch präparative HPLC [Methode 10] gereinigt. Man erhält 37.3 mg (44% d. Th.) der Zielverbindung.
LC/MS [Methode 7]: Rₜ = 2.56 min; MS [ESIpos]: m/z = 493 (M+H)⁺
¹H-NMR (400 MHz, DMSO-d₆): δ = 1.60 (s, 6H), 1.62 (s, 3H), 3.60 (s, 3H), 4.27 (s, 2H), 4.46 (s, 1H), 4.52 (s, 2H), 4.80 (s, 1H), 7.48-7.70 (m, 8H), 8.54 (s, 1H).

Auf analoge Weise wird die folgende Verbindung hergestellt:

| **Beispiel Nr.** | **Struktur** | **LC/MS oder HPLC, MS Rₜ [Methode]** |
|---|---|---|
| **424** | 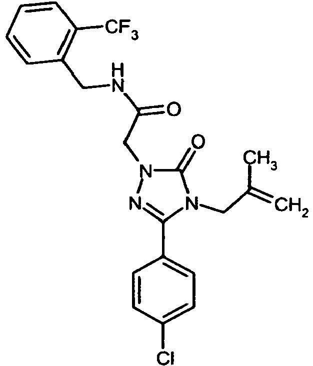 | LC/MS: Rₜ = 3.77 min [17] [ESIpos]: m/z = 465 (M+H)⁺ |

### Beispiel 425

### Ethyl (2Z)-3-3-(4-chlorphenyl)-1-[2-(1-methyl-1-[3-(trifluormethyl)phenyl]ethylamino)-2-oxo-ethyl]-5-oxo-1,5-dihydro-4H-1,2,4-triazol-4-yl-acrylat

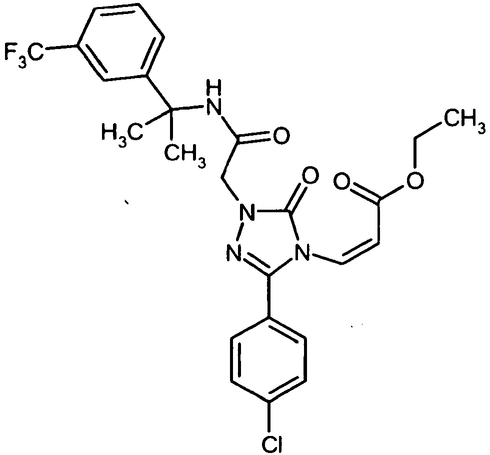

und

### Beispiel 426

### Ethyl (2E)-3-3-(4-chlorphenyl)-1-[2-(1-methyl-1-[3-(trifluormethyl)phenyl]ethylamino)-2-oxo-ethyl]-5-oxo-1,5-dihydro-4H-1,2,4-triazol-4-yl-acrylat

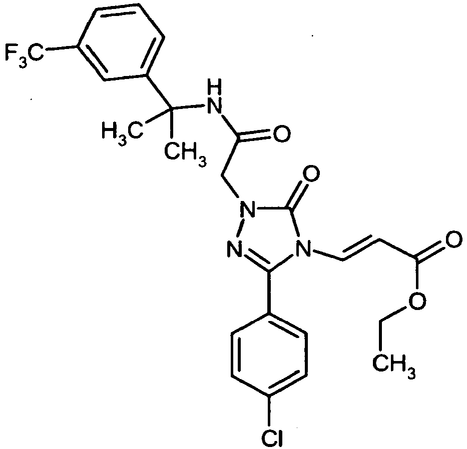

75 mg (0.17 mmol) 2-[3-(4-Chlorphenyl)-5-oxo-4,5-dihydro-1*H*-1,2,4-triazol-1-yl]-*N*-(1-methyl-1-[3-(trifluormethyl)phenyl]ethyl}-essigsäureamid aus Beispiel 241A werden mit 84 mg (0.26 mmol) Cäsiumcarbonat in 2.5 ml Aceton suspendiert und mit 40 mg (0.22 mmol) Ethyl cis-3-bromacrylat versetzt. Es wird 18 h bei 50°C gerührt. Die Suspension wird danach mit 1 ml Wasser versetzt und dreimal mit Essigsäureethylester extrahiert. Die vereinigten organischen Phasen werden über Natriumsulfat getrocknet und filtriert. Nach Einengen wird das Rohprodukt durch präparative HPLC [Methode 10] gereinigt. Es werden in getrennter Form das Z- und das E-Isomer der Titelverbindung erhalten.

### Z-Isomer (Beispiel 425):

Ausbeute: 32.1 mg (35% d. Th.)
LC/MS [Methode 8]: Rₜ = 2.79 min; MS [ESIpos]: m/z = 537 (M+H)⁺
¹H-NMR (400 MHz, DMSO-d₆): δ = 1.01 (t, 3H), 1.60 (s, 6H), 3.90 (q, 2H), 4.50 (s, 2H), 6.09 (d, 1H), 6.90 (d, 1H), 7.48-7.70 (m, 8H), 8.56 (s, 1H).

### E-Isomer (Beispiel 426):

### Ausbeute: 43.4 mg (49% d. Th.)

LC/MS [Methode 8]: Rₜ = 2.91 min; MS [ESIpos]: m/z = 537 (M+H)⁺
¹H-NMR (400 MHz, DMSO-d₆): δ = 1.20 (t, 3H), 1.60 (s, 6H), 4.14 (q, 2H), 4.52 (s, 2H), 6.72 (d, 1H), 7.41 (d, 1H), 7.48-7.70 (m, 8H), 8.60 (s, 1H).

### Beispiel 427

### Methyl 4-(3-(4-chlorphenyl)-1-[2-(1-methyl-1-[3-(trifluormethyl)phenyl]ethylamino)-2-oxoethyl]-5-oxo-1,5-dihydro-4H-1,2,4-triazol-4-ylmethyl)-benzoat

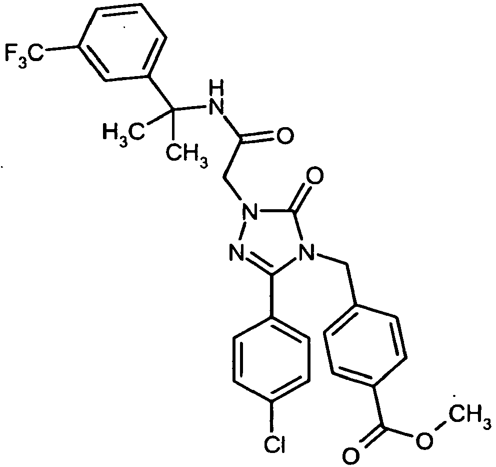

100 mg (0.23 mmol) 2-[3-(4-Chlorphenyl)-5-oxo-4,5-dihydro-1*H*-1,2,4-triazol-1-yl]-*N*-{1-methyl-1-[3-(trifluormethyl)phenyl]ethyl}-essigsäureamid aus Beispiel 241A werden mit 111 mg (0.34 mmol) Cäsiumcarbonat sowie 34 mg (0.23 mmol) Natriumiodid in 2.5 ml Aceton suspendiert und mit 55 mg (0.30 mmol) Methyl 4-(chlormethyl)-benzoat versetzt. Es wird 4 h bei 50°C gerührt. Die Suspension wird dann mit 1.5 ml Wasser versetzt und viermal mit je 2 ml Essigsäureethylester extrahiert. Die vereinigten organischen Phasen werden über Natriumsulfat getrocknet und filtriert. Nach Einengen wird das Rohprodukt durch präparative HPLC [Methode 10] gereinigt. Man erhält so 56.6 mg (42% d. Th.) der Zielverbindung.
LC/MS [Methode 19]: Rₜ = 3.84 min; MS [ESIpos]: m/z = 587 (M+H)⁺
¹H-NMR (400 MHz, DMSO-d₆): δ = 1.60 (s, 6H), 3.81 (s, 3H), 4.54 (s, 2H), 5.07 (s, 2H), 7.21 (d, 2H), 7.43-7.71 (m, 8H), 7.86 (d, 2H), 8.60 (s, 1H).

Auf analoge Weise werden die folgenden Verbindungen hergestellt:

| **Beispiel Nr.** | **Struktur** | **LC/MS oder HPLC, MS Rₜ [Methode]** |
|---|---|---|
| **428** | 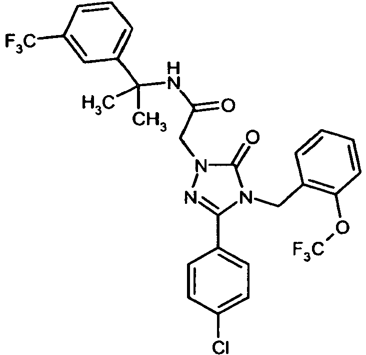 | LC/MS: Rₜ = 4.14 min [19] [ESIpos]: m/z = 613 (M+H)⁺ |
| **429** | 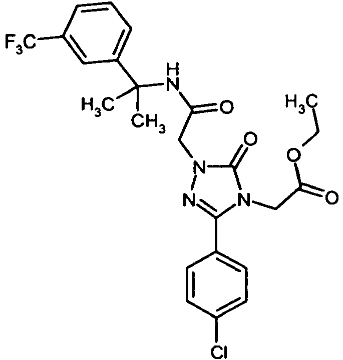 | LC/MS: Rₜ = 3.72 min [19] [ESIpos]: m/z = 525 (M+H)⁺ |

### Beispiel 430

### 2-[4-(4-tert.-Butylbenzyl)-3-(4-chlorphenyl)-5-oxo-4,5-dihydro-1H-1,2,4-triazo1-1-yl]-N-1-methyl-1-[3-(trifluormethyl)phenyl]ethyl-acetamid

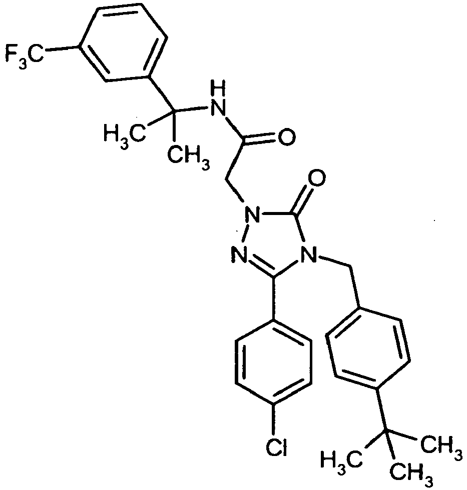

75 mg (0.17 mmol) 2-[3-(4-Chlorphenyl)-5-oxo-4,5-dihydro-1*H*-1,2,4-triazol-1-yl]-*N*-{1-methyl-1-[3-(trifluormethyl)phenyl]ethyl}-essigsäureamid aus Beispiel 241A werden mit 84 mg (0.26 mmol) Cäsiumcarbonat sowie 25 mg (0.17 mmol) Natriumiodid in 2.5 ml DMF suspendiert und mit 50 mg (0.22 mmol) 1-(Brommethyl)-4-*tert.*-butylbenzol versetzt. Es wird 1 h bei RT gerührt. Die Suspension wird nach Filtration durch einen Milipore-Filter direkt durch präparative HPLC [Methode 9] gereinigt. Man erhält so 44 mg (44% d. Th.) der Zielverbindung.
LC/MS [Methode 7]: Rₜ = 3.09 min; MS [ESIpos]: m/z = 585 (M+H)⁺
¹H-NMR (400 MHz, DMSO-d₆): δ = 1.21 (s, 9H), 1.60 (s, 6H), 4.52 (s, 2H), 4.94 (s, 2H), 6.99 (d, 2H), 7.29 (d, 2H), 7.50-7.70 (m, 8H), 8.59 (s, 1H).

Auf analoge Weise werden die folgenden Verbindungen hergestellt:

| **Beispiel Nr.** | **Struktur** | **LC/MS oder HPLC, MS Rₜ [Methode]** |
|---|---|---|
| **431** | 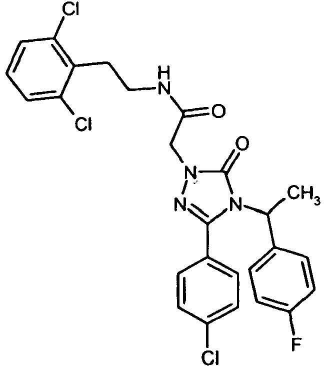 | LC/MS: Rₜ = 3.97 min [19] [ESIpos]: m/z = 547/549 (M+H)⁺ |
| **432** | 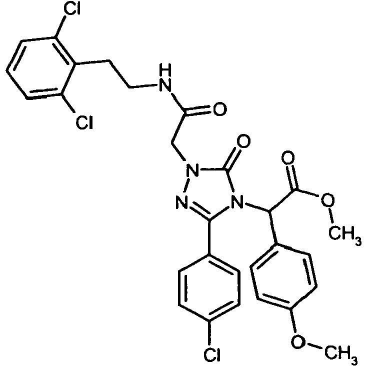 | LC/MS: Rₜ = 3.97 min [17] [ESIpos]: m/z = 603/605 (M+H)⁺ |

### Beispiel 433

### 2-[3-(4-Chlorphenyl)-4-(3-fluorbenzyl)-5-oxo-4,5-dihydro-1H-1,2,4-triazol-1-yl]-N-1-methyl-1-[3-(trifluormethyl)phenyl]ethyl-acetamid

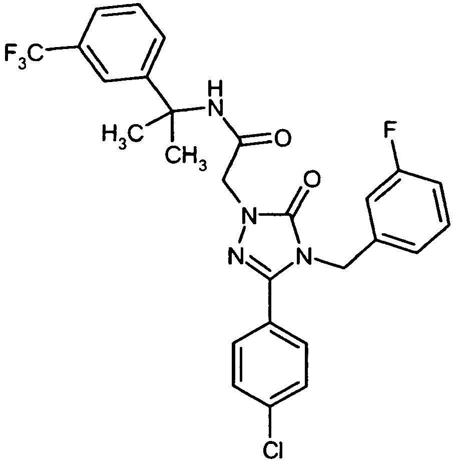

75 mg (0.17 mmol) 2-[3-(4-Chlorphenyl)-5-oxo-4,5-dihydro-1*H*-1,2,4-triazol-1-yl]-*N*-{1-methyl-1-[3-(trifluormethyl)phenyl]ethyl}-essigsäureamid aus Beispiel 241A werden in einem Gemisch aus 0.6 ml DMF und 1.1 ml 1,2-Dimethoxyethan (DME) gelöst. Es wird auf 0°C gekühlt und mit 9 mg (0.22 mmol) Natriumhydrid versetzt. Nach 10 min werden 45 mg (0.51 mmol) Lithiumbromid zugegeben und 15 min bei RT nachgerührt. Anschließend werden 42 mg (0.22 mmol) 3-Fluorbenzylbromid, gelöst in 0.3 ml DME, zugesetzt und die Mischung 5 h bei 75°C gerührt. Das Reaktionsgemisch wird danach mit Essigsäureethylester verdünnt, über eine Kieselgel/Extrelut-Kartusche filtriert, mit Essigsäureethylester nachgewaschen und im Vakuum eingeengt. Das Rohprodukt wird in Acetonitril aufgenommen und durch präparative HPLC [Methode 10] gereinigt. Man erhält so 68 mg (72% d. Th.) der Zielverbindung.
LC/MS [Methode 8]: Rₜ = 2.91 min; MS [ESIpos]: m/z = 547 (M+H)⁺
¹H-NMR (400 MHz, DMSO-d₆): δ = 1.60 (s, 6H), 4.54 (s, 2H), 4.99 (s, 2H), 6.85-6.95 (m, 2H), 7.02-7.11 (m, 1H), 7.28-7.36 (m, 1H), 7.45-7.72 (m, 8H), 8.60 (s, 1H).

Auf analoge Weise werden die folgenden Verbindungen hergestellt:

| **Beispiel Nr.** | **Struktur** | **LC/MS oder HPLC, MS Rₜ [Methode]** |
|---|---|---|
| **434** | 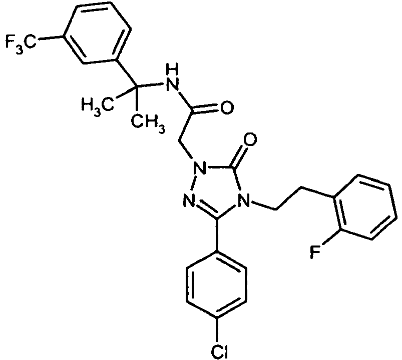 | LC/MS: Rₜ = 4.15 min [17] [ESIpos]: m/z = 561 (M+H)⁺ |
| **435** | 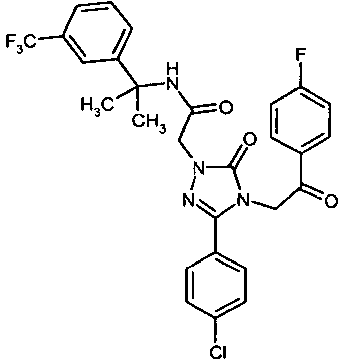 | LC/MS: Rₜ = 3.90 min [19] [ESIpos]: m/z = 575 (M+H)⁺ |
| **436** | 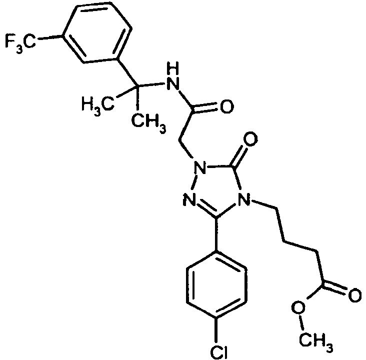 | LC/MS: Rₜ = 2.66 min [8] [ESIpos] : m/z = 539 (M+H)⁺ |
| **437** | 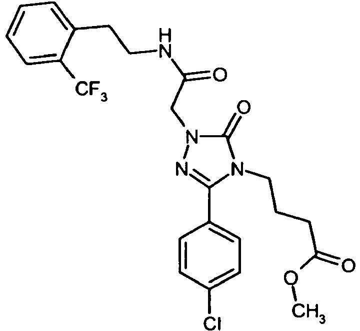 | LC/MS: Rₜ = 3.65 min [17] [ESIpos]: m/z = 525 (M+H)⁺ |
| **438** | 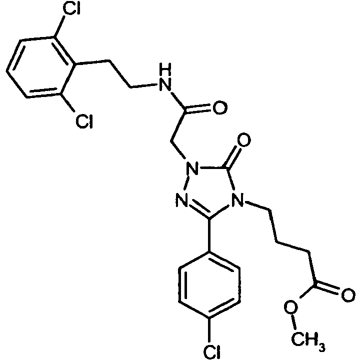 | LC/MS: Rₜ = 3.66 min [17] [ESIpos]: m/z = 525/527 (M+H)⁺ |
| **439** | 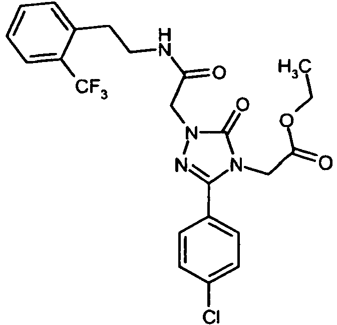 | LC/MS: Rₜ = 2.63 min [8] [ESIpos]: m/z = 511 (M+H)⁺ |
| **440** | 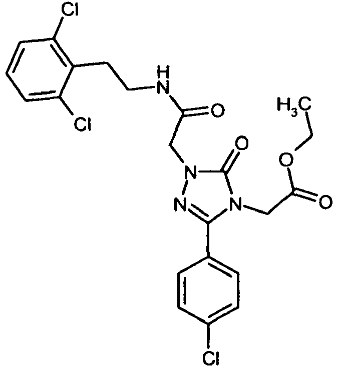 | LC/MS: Rₜ = 2.65 min [8] [ESIpos]: m/z = 511/513 (M+H)⁺ |

### Beispiel 441

### 2-[4-(2-Chlorethyl)-3-(4-chlorphenyl)-5-oxo-4,5-dihydro-1H-1,2,4-triazol-1-yl]-N-[2-(trifluormethyl)benzyl]-acetamid

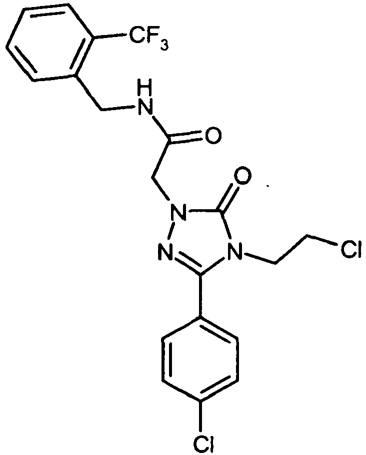

500 mg (1.01 mmol) 2-[3-(4-Chlorphenyl)-5-oxo-4,5-dihydro-1*H*-1,2,4-triazol-1-yl]-*N*-[2-(trifluormethyl)benzyl]-acetamid aus Beispiel 243A werden mit 230 mg (1.01 mmol) Benzyltriethylammoniumchlorid in 10 ml Toluol gelöst. Anschließend wird eine Lösung von 658 mg (2.02 mmol) Cäsiumcarbonat, gelöst in 1.0 ml Wasser, zugegeben und die Mischung 30 min bei RT kräftig gerührt. Nach Zugabe von 1.92 g (10.13 mmol) 1-Iod-2-chlorethan wird unter starkem Rühren 7 h auf 80°C erhitzt. Die Suspension wird danach mit Essigsäureethylester verdünnt und je einmal mit Wasser, 10%-iger Natriumthiosulfat- und gesättigter Ammoniumchlorid-Lösung gewaschen. Die organische Phase wird über Natriumsulfat getrocknet, filtriert und im Vakuum eingeengt. Reinigung des Rohprodukts erfolgt durch präparative HPLC [Methode 9]. Man erhält so 195 mg (41% d. Th.) der Zielverbindung.
LC/MS [Methode 7]: Rₜ = 2.31 min; MS [ESIpos]: m/z = 473/475 (M+H)⁺
¹H-NMR (400 MHz, DMSO-d₆): δ = 3.79 (t, 2H), 4.10 (t, 3H), 4.50 (d, 2H), 4.56 (d, 2H), 4.99 (s, 2H), 6.85-6.95 (m, 2H), 7.50 (t, 1H), 7.52-7.73 (m, 7H), 8.70 (t, 1H).

Auf analoge Weise wird die folgende Verbindung hergestellt:

| **Beispiel Nr.** | **Struktur** | **LC/MS oder HPLC, MS Rₜ [Methode]** |
|---|---|---|
| **442** | 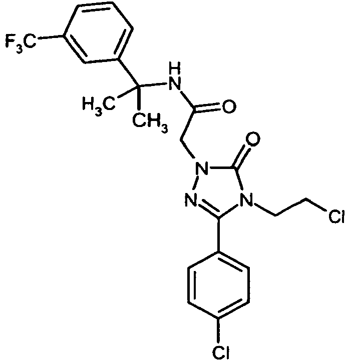 | LC/MS: Rₜ = 2.64 min [8] [ESIpos]: m/z = 501/503 (M+H)⁺ |

### Beispiel 443

### 2-[3-(4-Chlorphenyl)-5-oxo-4-(2-oxobutyl)-4,5-dihydro-1H-1,2,4-triazol-1-yl]-N-1-methyl-1-[3-(trifluormethyl)phenyl]ethyl-acetamid

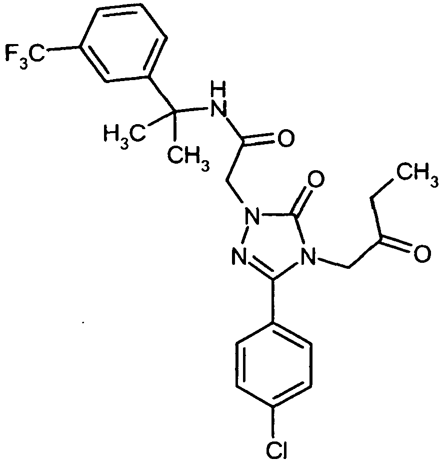

94 mg (0.21 mmol) 2-[3-(4-Chlorphenyl)-5-oxo-4,5-dihydro-1*H*-1,2,4-triazol-1-yl]-*N*-{1-methyl-1-[3-(trifluormethyl)phenyl]ethyl}-essigsäureamid aus Beispiel 241A werden mit 97.7 mg (0.30 mmol) Cäsiumcarbonat in 2.5 ml DMF suspendiert und mit 50 mg (0.30 mmol) 1-Brom-2-butanon (90%-ig) versetzt. Es wird 3 h bei 75°C gerührt. Die Suspension wird danach mit 10 ml Essigsäureethylester verdünnt und zweimal mit je 5 ml Wasser sowie einmal mit 5 ml gesättigter Natriumchlorid-Lösung gewaschen. Die organische Phase wird über Natriumsulfat getrocknet und filtriert. Nach Einengen wird das Rohprodukt mittels präparativer Dünnschichtchromatographie an Kieselgel (Eluent: Dichlormethan/Methanol 10:1) gereinigt. Man erhält so 64.8 mg (59% d. Th.) der Zielverbindung.
LC/MS [Methode 19]: Rₜ = 3.66 min; MS [ESIpos]: m/z = 509 (M+H)⁺
¹H-NMR (400 MHz, DMSO-d₆): δ = 0.88 (t, 3H), 1.60 (s, 6H), 2.50 (q, 2H), 4.49 (s, 2H), 4.77 (s, 2H), 6.59-6.64 (m, 2H), 6.75-6.80 (m, 1H), 7.18 (t, 1H), 7.46-7.72 (m, 8H), 8.55 (s, 1H).

### Beispiel 444

### 2-[3-(4-Chlorphenyl)-5-oxo-4-(3,3,3-trifluor-2-hydroxypropyl)-4,5-dihydro-1H-1,2,4-triazol-1-yl]-N-[2-(trifluormethyl)benzyl]-acetamid

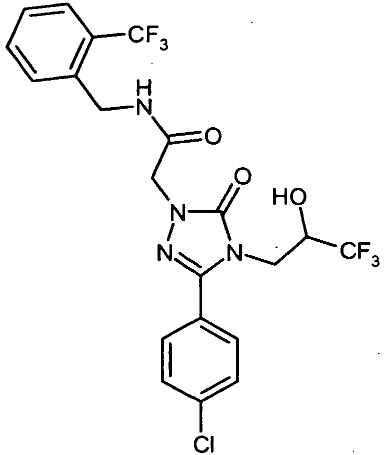

100 mg (0.24 mmol) 2-[3-(4-Chlorphenyl)-5-oxo-4,5-dihydro-1*H*-1,2,4-triazol-1-yl]-*N*-[2-(trifluormethyl)benzyl]-acetamid aus Beispiel 243A werden mit 119 mg (0.37 mmol) Cäsiumcarbonat in 0.6 ml DMSO vorgelegt und mit 137 mg (1.22 mmol) 1,1,1-Trifluor-2,3-epoxypropan versetzt. Es wird 3 h bei 120°C gerührt. Anschließend werden erneut 137 mg (1.22 mmol) 1,1,1-Trifluor-2,3-epoxypropan hinzugefügt und eine weitere Stunde bei 120°C gerührt. Die Suspension wird danach auf RT abgekühlt, mit Essigsäureethylester verdünnt und dreimal mit gesättigter Ammoniumchlorid-Lösung gewaschen. Die organische Phase wird über Natriumsulfat getrocknet, filtriert und im Vakuum eingeengt. Das Rohprodukt wird durch präparative HPLC [Methode 10] gereinigt. Man erhält so 30.6 mg (24% d. Th.) der Zielverbindung.
LC/MS [Methode 17]: Rₜ = 3.69 min; MS [ESIpos]: m/z = 523 (M+H)⁺
¹H-NMR (400 MHz, DMSO-d₆): δ = 3.83 (dd, 1H), 3.98 (dd, 1H), 4.30 (m, 1H), 4.47-4.60 (m, 4H), 6.90 (d, 1H), 7.46-7.81 (m, 8H), 8.70 (t, 1H).

Auf analoge Weise werden die folgenden Verbindungen hergestellt:

| **Beispiel Nr.** | **Struktur** | **LC/MS oder HPLC, MS Rₜ (Methode)** |
|---|---|---|
| **445** | 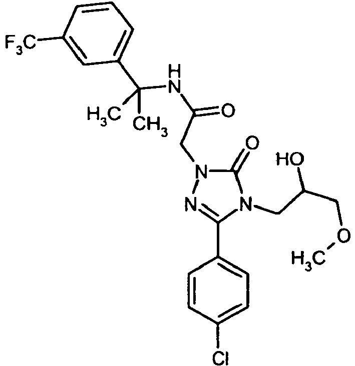 | LC/MS: Rₜ = 3.54 min [17] [ESIpos]: m/z = 527 (M+H)⁺ |
| **446** | 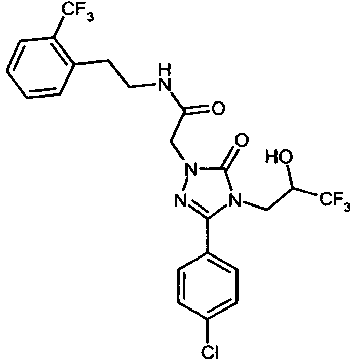 | LC/MS: Rₜ = 3.78 min [17] [ESIpos]: m/z = 537 (M+H)⁺ |
| **447** | 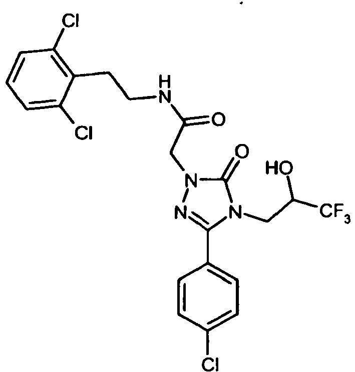 | LC/MS: Rₜ = 2.67 min [8] [ESIpos]: m/z = 537/539 (M+H)⁺ |

Durch präparative HPLC an chiraler Phase [Daicel Chiralcel OD-H, 5 µm, 250 mm x 20 mm; Eluent: Isohexan/Isopropanol 3:1; Fluss: 15 ml/min; Temperatur: 30°C; UV-Detektion: 220 nm] wird das Racemat aus Beispiel 447 in die Enantiomere getrennt (siehe Beispiele 448 und 449). Der spezifische Drehwert α_{D} für die Enantiomere wird wie folgt bestimmt [Perkin-Elmer Polarimeter 341; Wellenlänge: 589 nm; Solvens: Methanol; Schichtdicke: 100 mm]:

| **Beispiel Nr.** | **Struktur** | **Drehwert α_{D}** |
|---|---|---|
| **448** | 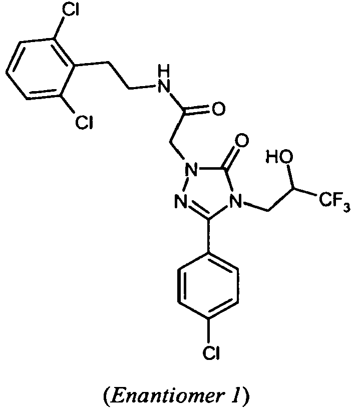 | -8.0° (c = 0.16 mg/100 ml; 20.5°C) |
| **449** | 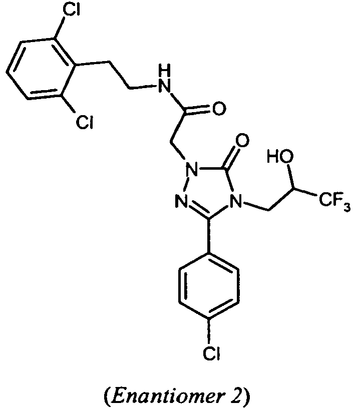 | +6.1 ° (c = 0.315 mg/100 ml; 20.3°C) |

### Beispiel 450

### 2-[3-(4-Chlorphenyl)-5-oxo-4-(3,3,3-trifluor-2-hydroxypropyl)-4,5-dihydro-1H-1,2,4-triazol-1-yl]-N-{1-methyl-1-[3-(trifluormethyl)phenyl]ethyl}-acetamid (Racemat)

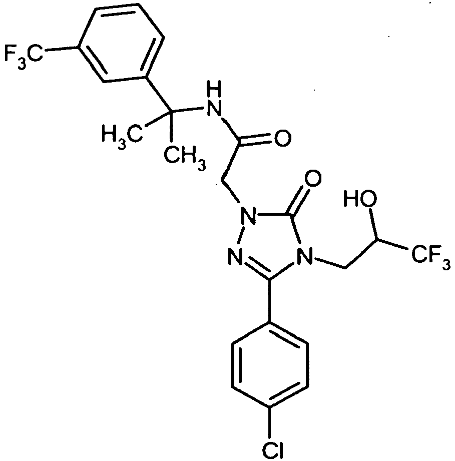

3.61 g (8.23 mmol) 2-[3-(4-Chlorphenyl)-5-oxo-4,5-dihydro-1*H*-1,2,4-triazol-1-yl]-*N*-{1-methyl-1-[3-(trifluormethyl)phenyl]ethyl}-essigsäureamid aus Beispiel 241A werden mit 3.75 g (11.52 mmol) Cäsiumcarbonat in 20 ml DMF gelöst und mit 2.22 g (11.52 mmol) 1,1,1-Trifluor-2,3-epoxypropan versetzt. Es wird 2.5 h bei 75°C gerührt. Die Suspension wird danach mit 30 ml Essigsäureethylester verdünnt und zweimal mit je 20 ml Wasser gewaschen. Die organische Phase wird über Natriumsulfat getrocknet und filtriert. Nach Einengen wird das Rohprodukt mittels Flash-Chromatographie an Kieselgel (Eluent: Cyclohexan/Essigsäureethylester zunächst 5:1, dann 1:1) gereinigt. Man erhält so 3.14 g (69% d. Th.) der Zielverbindung.
LC/MS [Methode 8]: Rₜ = 2.75 min; MS [ESIpos]: m/z = 551 (M+H)⁺
¹H-NMR (400 MHz, CDCl₃): δ = 1.72 (s, 6H), 3.99 (dd, 1H), 4.06 (dd, 1H), 4.41-4.58 (m, 3H), 4.85 (m, 1H), 6.45 (s, 1H), 7.40-7.63 (m, 8H).

Durch präparative HPLC an chiraler Phase [Daicel Chiralpak AD-H, 5 µm, 250 mm x 20 mm; Eluent: Isohexan/Isopropanol 85:15; Fluss: 15 ml/min; Temperatur: 40°C; UV-Detektion: 220 nm] wird das Racemat aus Beispiel 450 in die Enantiomere getrennt (siehe Beispiele 451 und 452):

### Beispiel 451

### 2-[3-(4-Chlorphenyl)-5-oxo-4-(3,3,3-trifluor-2-hydroxypropyl)-4,5-dihydro-1H-1,2,4-triazol-1-yl]-N-{1-methyl-1-[3-(trifluormethyl)phenyl]ethyl}-acetamid (Enantiomer 1)

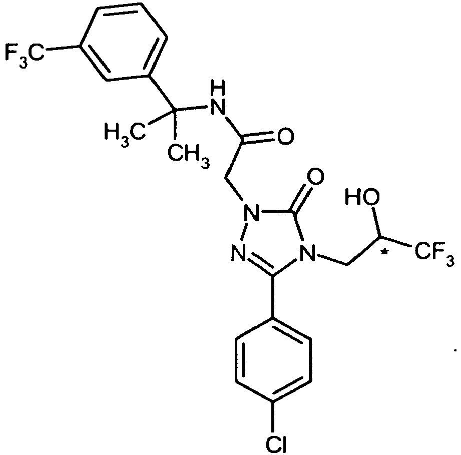

Ausbeute: 1.31 g (29% d.Th.)
Rₜ = 4.02 min [Daicel Chiralpak AD-H, 5 µm, 250 mm x 4.6 mm; Eluent: Isohexan/Isopropanol 85:15; Fluss: 1.0 ml/min; Temperatur: 40°C; UV-Detektion: 220 nm].

### Beispiel 452

### 2-[3-(4-Chlorphenyl)-5-oxo-4-(3,3,3-trifluor-2-hydroxypropyl)-4,5-dihydro-1H-1,2,4-triazol-1-yl]-N-{1-methyl-1-[3-(trifluormethyl)phenyl]ethyl}-acetamid (Enantiomer 2)

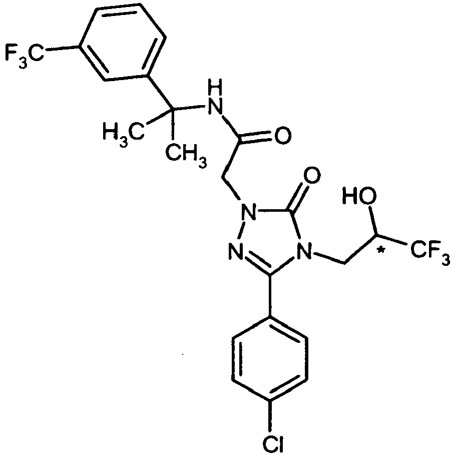

Ausbeute: 1.20 g (26% d.Th.)
Rₜ = 4.71 min [Daicel Chiralpak AD-H, 5 µm, 250 mm x 4.6 mm; Eluent: Isohexan/Isopropanol 85:15; Fluss: 1.0 ml/min; Temperatur: 40°C; UV-Detektion: 220 nm].

### Beispiel 453

### 2-[3-(4-Chlorphenyl)-4-(3-hydroxypropyl)-5-oxo-4,5-dihydro-1H-1,2,4-triazol-1-yl]-N-[3-(trifluormethyl)benzyl]-acetamid

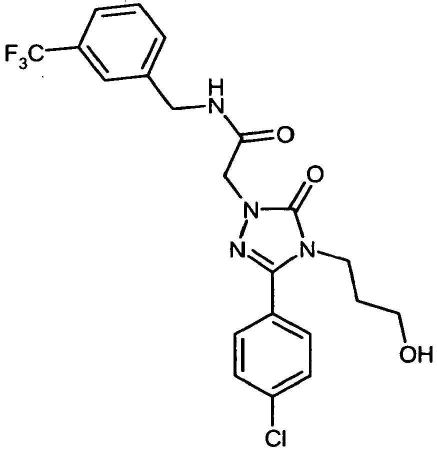

und

### Beispiel 454

### 2-[3-(4-Chlorphenyl)-4-(2-hydroxypropyl)-5-oxo-4,5-dihydro-1H-1,2,4-triazol-1-yl]-N-[3-(trifluormethyl)benzyl]-acetamid

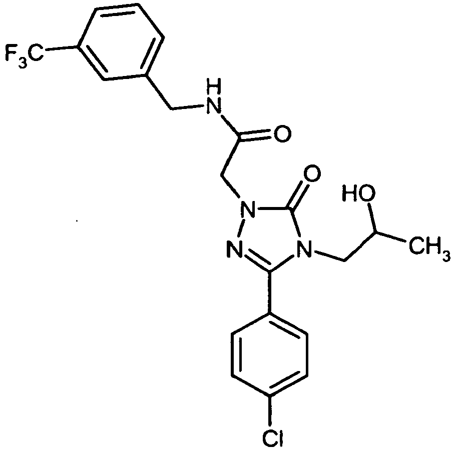

100 mg (0.22 mmol) 2-[4-Allyl-3-(4-Chlorphenyl)-5-oxo-4,5-dihydro-1*H*-1,2,4-triazol-1-yl]-*N*-[3-(trifluormethyl)benzyl]-acetamid aus Beispiel 372 werden in 5 ml THF gelöst und bei 0°C mit 0.67 ml einer 1 M Lösung von Boran-THF-Komplex in THF versetzt. Es wird 18 h bei RT gerührt. Die Reaktionslösung wird danach wieder auf 0°C abgekühlt, und es werden 4.5 ml einer 10%-igen Natronlauge sowie 4.5 ml einer 30%-igen Wasserstoffperoxid-Lösung zugegeben. Nach 3 h Rühren wird die Mischung auf 10 ml Wasser gegeben und dreimal mit je 15 ml Essigsäureethylester extrahiert. Die vereinigten organischen Phasen werden mit 5 ml 10%-iger Natriumthiosulfat-Lösung und 5 ml gesättigter Natriumchlorid-Lösung gewaschen, über Natriumsulfat getrocknet, filtriert und im Vakuum eingeengt. Das Rohprodukt wird durch präparative HPLC [Methode 10] gereinigt. Es werden in getrennter Form die beiden isomeren Alkohole erhalten.

### Beispiel 453:

Ausbeute: 20 mg (19% d. Th.)
LC/MS [Methode 18]: Rₜ = 2.18 min; MS [ESIpos]: m/z = 469 (M+H)⁺
¹H-NMR (400 MHz, DMSO-d₆): δ = 1.68 (quin, 2H), 3.27-3.40 (m, 2H), 3.80 (t, 2H), 4.41 (d, 2H), 4.45-4.56 (m, 3H), 7.52-7.73 (m, 8H), 8.68 (t, 1H).

### Beispiel 454:

Ausbeute: 13 mg (13% d. Th.)
LC/MS [Methode 18]: Rₜ = 2.18 min; MS [ESIpos]: m/z = 469 (M+H)⁺
¹H-NMR (400 MHz, DMSO-d₆): δ = 1.0 (d, 3H), 3.52-3.68 (m, 2H), 3.86 (m, 1H), 4.36-4.56 (m, 4H), 5.04 (d, 1H), 7.51-7.67 (m, 6H), 7.80 (d, 2H), 8.68 (t, 1H).

Auf analoge Weise werden die folgenden Verbindungen hergestellt:

| **Beispiel Nr.** | **Struktur** | **LC/MS oder HPLC, MS Rₜ [Methode]** |
|---|---|---|
| **455** | 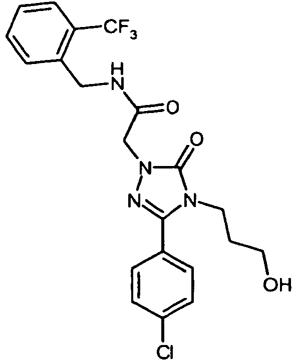 | LC/MS: Rₜ = 2.28 min [8] [ESIpos]: m/z = 469 (M+H)⁺ |
| **456** | 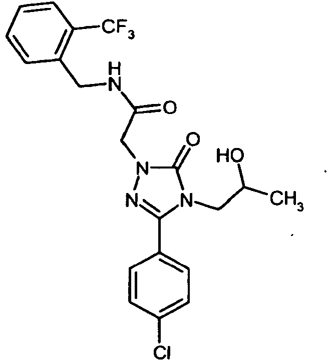 | LC/MS: Rₜ = 2.33 min [8] [ESIpos]: m/z = 469 (M+H)⁺ |

### Beispiel 457

### 2-{3-(4-Chlorphenyl)-5-oxo-4-[(1E)-prop-1-en-1-yl]-4,5-dihydro-1H-1,2,4-thazol-1-yl}-N-[3-(trifluormethyl)benzyl]-acetamid

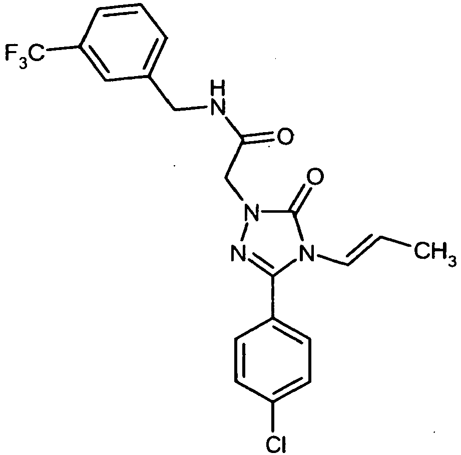

22 mg (0.047 mmol) 2-[3-(4-Chlorphenyl)-4-(2-hydroxypropyl)-5-oxo-4,5-dihydro-1*H*-1,2,4-triazol-1-yl]-*N*-[3-(trifluormethyl)benzyl]-acetamid aus Beispiel 454 werden in 1 ml Pyridin gelöst und bei RT mit 9.6 mg (0.084 mmol) Methansulfonsäurechlorid versetzt. Es wird 2 h bei RT gerührt. Anschließend wird mit 5 ml Essigsäureethylester verdünnt und dreimal mit je 5 ml 1 N Salzsäure gewaschen. Die organische Phase wird über Natriumsulfat getrocknet, filtriert und im Vakuum eingeengt. Der Rückstand wird in 1 ml trockenem Methanol gelöst, mit 20 mg (0.94 mmol) Natriummethanolat versetzt und 18 h bei RT gerührt. Zur Vervollständigung der Reaktion werden weitere 81 mg (0.38 mmol) Natriummethanolat zugegeben und bei RT nochmals für 48 h gerührt. Das Gemisch wird mit 0.5 ml 1N Salzsäure neutralisiert und direkt durch präparative HPLC [Methode 10] gereinigt. Man erhält so 4.8 mg (23% d. Th.) der Zielverbindung.
LC/MS [Methode 8]: Rₜ = 2.66 min; MS [ESIpos]: m/z = 451 (M+H)⁺
¹H-NMR (400 MHz, CDCl₃): δ = 1.80 (d, 3H), 4.55 (d, 2H), 4.62 (s, 2H), 6.19-6.34 (m, 3H), 6.67 (m, 1H), 7.43-7.59 (m, 8H).

### Beispiel 458

### 2-{3-(4-Chlorphenyl)-4-[2-(dimethylamino)ethyl]-5-oxo-4,5-dihydro-1H-1,2,4-triazol-1-yl}-N-{1-methyl-1-[3-(trifluormethyl)phenyl]ethyl}-acetamid

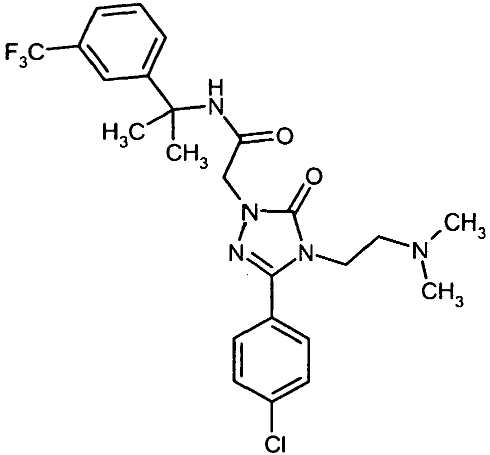

und

### Beispiel 459

### 2-[3-(4-Chlorphenyl)-5-oxo-4-vinyl-4,5-dihydro-1H-1,2,4-triazol-1-yl]-N-{1-methyl-1-[3-(trifluormethyl)phenyl]ethyl}-acetamid

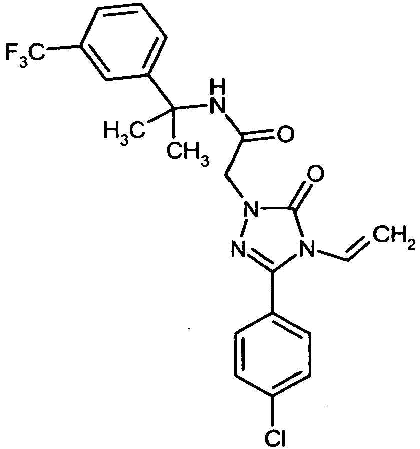

35 mg (0.07 mmol) 2-[4-(2-Chlorethyl)-3-(4-chlorphenyl)-5-oxo-4,5-dihydro-1*H*-1,2,4-triazol-l-yl]-*N*-{1-methyl-1-[3-(trifluormethyl)phenyl]ethyl}-acetamid aus Beispiel 442 werden in 0.7 ml DMF gelöst und bei RT mit 127 mg (1.56 mmol) Dimethylamin-Hydrochlorid, 10.5 mg (0.07 mmol) Natriumiodid sowie 106 mg (0.77 mmol) Kaliumcarbonat versetzt. Es wird 24 h bei 100°C im geschlossenen Gefäß gerührt. Nach Abkühlen wird mit 5 ml Wasser verdünnt und zweimal mit je 5 ml Essigsäureethylester extrahiert. Die vereinigten organischen Phasen werden über Natriumsulfat getrocknet, filtriert und im Vakuum eingeengt. Reinigung durch Flash-Chromatographie an Kieselgel (Eluent: Cyclohexan/Essigsäureethylester 1:1) liefert zunächst das 4-Vinyl-Derivat (Beispiel 459). Durch weitere Elution mit Dichlormethan/7 N methanolische Ammoniak-Lösung (10:1) wird das 4-[2-(Dimethylamino)ethyl]-Derivat (Beispiel 458) erhalten, welches durch präparative Dickschicht-Chromatographie (Eluent: Dichlormethan/Essigsäureethylester/7 N methanolische Ammoniak-Lösung 10:10:0.5) weiter gereinigt wird.

### Beispiel 458:

Ausbeute: 7.6 mg (21 % d. Th.)
LC/MS [Methode 8]: Rₜ = 1.60 min; MS [ESIpos]: m/z = 510 (M+H)⁺
¹H-NMR (400 MHz, DMSO-d₆): δ = 1.60 (s, 6H), 1.98 (s, 6H), 2.29 (t, 2H), 3.79 (t, 2H), 4.46 (s, 2H), 7.48-7.70 (m, 8H), 8.54 (s, 1H).

### Beispiel 459:

Ausbeute: 6.8 mg (21 % d. Th.)
LC/MS [Methode 8]: Rₜ = 2.62 min; MS [ESIpos]: m/z = 465 (M+H)⁺
¹H-NMR (400 MHz, DMSO-d₆): δ = 1.60 (s, 6H), 4.50 (s, 2H), 5.10 (d, 1H), 5.74 (d, 1H), 6.61 (dd, 1H), 7.49-7.70 (m, 8H), 8.60 (s, 1H).

Auf analoge Weise werden die folgenden Verbindungen hergestellt:

| **Beispiel Nr.** | **Struktur** | **LC/MS oder HPLC, MS Rₜ [Methode]** |
|---|---|---|
| **460** | 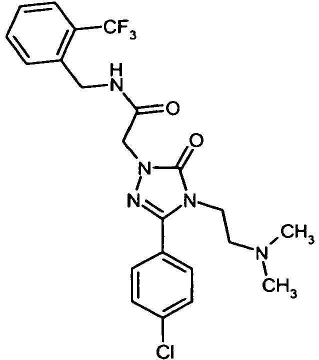 | LC/MS: Rₜ=2.14min[19] [ESIpos]: m/z = 482 (M+H)⁺ |
| **461** | 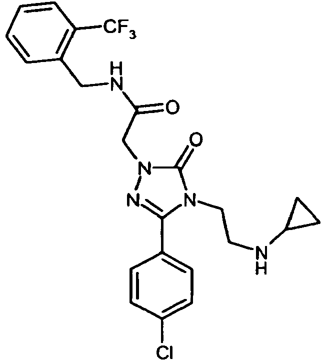 | LC/MS: Rₜ = 1.59 min [8] [ESIpos]: m/z = 494 (M+H)⁺ |

### Beispiel 462

### 2-[3-(4-Chlorphenyl)-4-(2-morpholin-4-ylethyl)-5-oxo-4,5-dihydro-1H-1,2,4-triazol-1-yl]-N-{1-methyl-1-[3-(trifluormethyl)phenyl]ethyl}-acetamid

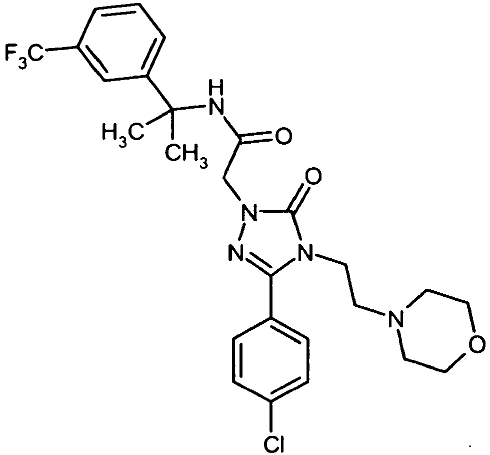

0.10 g (0.21 mmol) 2-[3-(4-Chlorphenyl)-4-(2-oxoethyl)-5-oxo-4,5-dihydro-1*H*-1,2,4-triazol-1-yl]-*N*-{1-methyl-1-[3-(trifluormethyl)phenyl]ethyl}-acetamid aus Beispiel 401 werden in 30 ml Dichlormethan und 0.4 ml DMF gelöst und mit 22 mg (0.2 mmol) Morpholin 1 h lang bei RT gerührt. Anschließend werden 66 mg (0.31 mmol) Natrium-triacetoxyborhydrid hinzugefügt und die Mischung 18 h bei RT gerührt. Das Reaktionsgemisch wird mit 10 ml gesättigter Natriumhydrogencarbonat-Lösung versetzt und dreimal mit je 10 ml Essigsäureethylester extrahiert. Die vereinigten organischen Phasen werden über Natriumsulfat getrocknet, filtriert und im Vakuum eingeengt. Das Rohprodukt wird durch präparative HPLC [Methode 10] gereinigt. Man erhält 11.0 mg (10% d. Th.) der Zielverbindung.
LC/MS [Methode 17]: Rₜ = 2.78 min; MS [ESIpos]: m/z = 552 (M+H)⁺
¹H-NMR (400 MHz, DMSO-d₆): δ = 1.59 (s, 6H), 2.16 (m, 4H), 2.34 (t, 2H), 3.31 (m, 4H), 3.80 (t, 2H), 4.46 (s, 2H), 7.48-7.72 (m, 8H), 8.54 (s, 1H).

Auf analoge Weise werden die folgenden Verbindungen hergestellt:

| **Beispiel Nr.** | **Struktur** | **LC/MS oder HPLC, MS Rₜ [Methode]** |
|---|---|---|
| **463** | 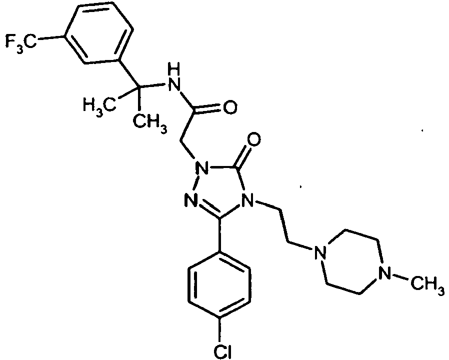 | LC/MS: Rₜ = 2.65 min [19] [ESIpos]: m/z = 565 (M+H)⁺ |
| **464** | 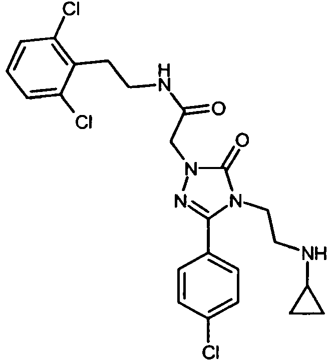 | LC/MS: Rₜ = 2.43 min [19] [ESIpos]: m/z = 508 (M+H)⁺ |
| **465** | 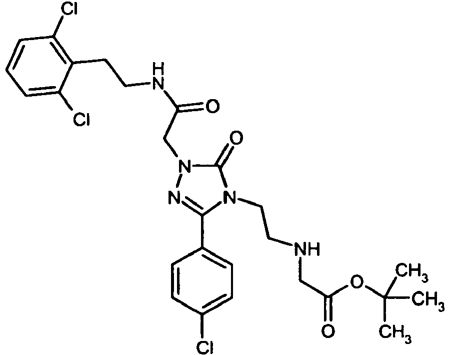 | LC/MS: Rₜ = 2.79 min [19] [ESIpos]: m/z = 582/584 (M+H)⁺ |

### Beispiel 466

### [3-(4-Chlorphenyl)-5-oxo-1-(2-oxo-2-[3-(trifluormethyl)benzyl]aminoethyl)-1,5-dihydro-4H-1,2,4-triazol-4-yl]-essigsäure

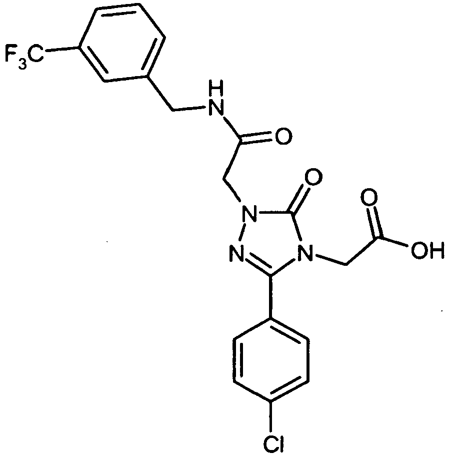

119 mg (0.23 mmol) tert.-Butyl [3-(4-chlorphenyl)-5-oxo-1-(2-oxo-2-{[3-(trifluormethyl)benzyl]-amino}ethyl)-1,5-dihydro-4*H*-1,2,4-triazol-4-yl]-acetat aus Beispiel 420 werden in 9 ml Dichlormethan gelöst und mit 3 ml Trifluoressigsäure versetzt. Bei RT wird 24 h gerührt. Das Reaktionsgemisch wird dann mit 10 ml Toluol versetzt und unter vermindertem Druck eingeengt. Man fügt weitere 10 ml Toluol hinzu und evaporiert erneut. Dieser Vorgang wird noch einmal wiederholt. Das Rohprodukt wird anschließend im Hochvakuum von Lösungsmittelresten befreit. Man erhält so 110 mg (quantitativ) der Zielverbindung.
LC/MS [Methode 7]: Rₜ = 2.00 min; MS [ESIpos]: m/z = 469 (M+H)⁺
¹H-NMR (500 MHz, DMSO-d₆): δ = 4.41 (s, 2H), 4.52 (2s, 4H), 7.52-7.66 (m, 8H), 8.76 (t, 1H), 13.30 (br. s, 1H).

### Beispiel 467

### 3-(4-Chlorphenyl)-1-[2-(1-methyl-1-[3-(trifluormethyl)phenyl]ethylamino)-2-oxoethyl]-5-oxo-1,5-dihydro-4H-1,2,4-triazol-4-yl-essigsäure

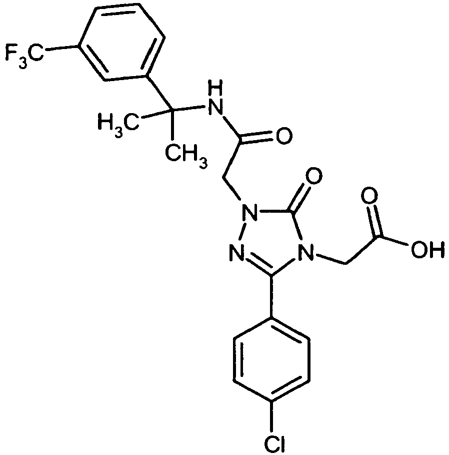

237 mg (0.45 mmol) Ethyl {3-(4-chlorphenyl)-1-[2-({1-methyl-1-[3-(trifluormethyl)phenyl]ethyl}-amino)-2-oxoethyl]-5-oxo-1,5-dihydro-4*H*-1,2,4-triazol-4-yl}-acetat aus Beispiel 429 werden in 3 ml Methanol gelöst und mit 0.9 ml 1 N wässriger Lithiumhydroxid-Lösung versetzt. Bei RT wird 24 h lang gerührt. Das Reaktionsgemisch wird danach unter vermindertem Druck eingeengt. Man fügt 5 ml Wasser hinzu, säuert mit 1 ml 1 N Salzsäure an und extrahiert zweimal mit je 10 ml Essigsäureethylester. Die vereinigten organischen Phasen werden über Natriumsulfat getrocknet, filtriert und im Vakuum eingeengt. Das verbleibende Produkt wird im Hochvakuum von Lösungsmittelresten befreit. Man erhält so 220 mg (97% d. Th.) der Zielverbindung.
LC/MS [Methode 17]: Rₜ = 3.49 min; MS [ESIpos]: m/z = 497 (M+H)⁺
¹H-NMR (400 MHz, DMSO-d₆): δ = 1.60 (s, 6H), 4.50 (2s, 4H), 7.48-7.70 (m, 8H), 8.57 (s, 1H), 13.30 (br. s, 1H).

Auf analoge Weise werden die folgenden Verbindungen hergestellt:

| **Beispiel Nr.** | **Struktur** | **LC/MS oder HPLC, MS Rₜ [Methode]** |
|---|---|---|
| **468** | 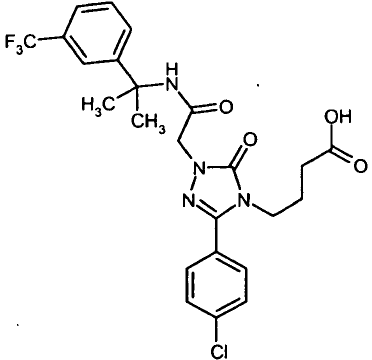 | LC/MS: Rₜ = 3.35 min [19] [ESIpos]: m/z = 525 (M+H)⁺ |
| **469** | 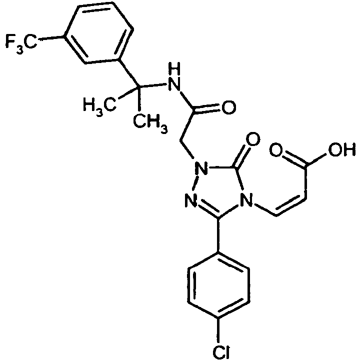 | LC/MS: Rₜ =3.53 min [17] [ESIpos]: m/z = 509 (M+H)⁺ |
| **470** | 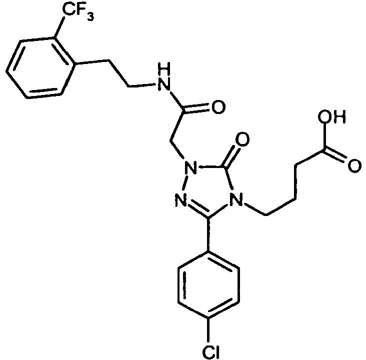 | LC/MS: Rₜ = 3.37 min [17] [ESIpos]: m/z = 511 (M+H)⁺ |

### Beispiel 471

### 3-(3-(4-Chlorphenyl)-1-[2-(1-methy-1-[3-(trifluormethyl)phenyl]ethylamino)-2-oxoethyl]-5-oxo-1,5-dihydro-4H-1,2,4-triazol-4-ylmethyl)-benzoesäure

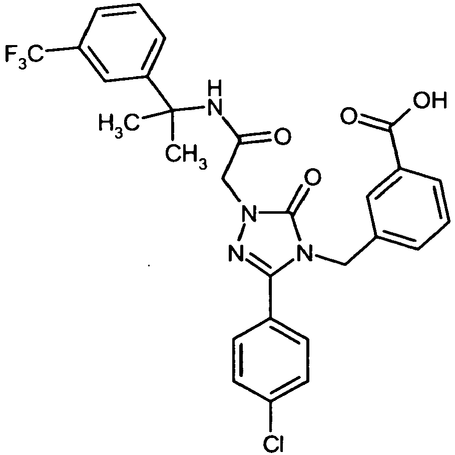

180 mg (0.31 mmol) Methyl 3-(3-(4-chlorphenyl)-1-[2-(1-methyl-1-[3-(trifluormethyl)phenyl]-ethylamino)-2-oxoethyl]-5-oxo-1,5-dihydro-4H-1,2,4-triazol-4-ylmethyl)-benzoat (Beispiel 411) werden in 3 ml Methanol und 3 ml THF gelöst, mit 0.3 ml 2 N Natronlauge versetzt und 1 h bei 70°C gerührt. Das Reaktionsgemisch wird dann in ca. 10 ml Wasser eingetragen, mit 1 N Salzsäure auf pH 4 gebracht und 2 h bei RT gerührt. Der ausgefallene Niederschlag wird abgesaugt, mit Wasser gewaschen und im Hochvakuum von Lösungsmittelresten befreit. Man erhält 154 mg (88% d. Th.) der Zielverbindung.
LC/MS [Methode 17]: Rₜ = 3.69 min; MS [ESIpos]: m/z = 573 (M+H)⁺
¹H-NMR (400 MHz, DMSO-d₆): δ = 1.60 (s, 6H), 4.52 (s, 2H), 5.05 (s, 2H), 7.28 (d, 1H), 7.40 (t, 1H), 7.47-7.72 (m, 6H), 7.80 (d, 2H), 8.60 (s, 1H), 13.02 (br. s, 1H).

Auf analoge Weise werden die folgenden Verbindungen hergestellt:

| **Beispiel Nr.** | **Struktur** | **LC/MS oder HPLC, MS Rₜ [Methode]** |
|---|---|---|
| **472** | 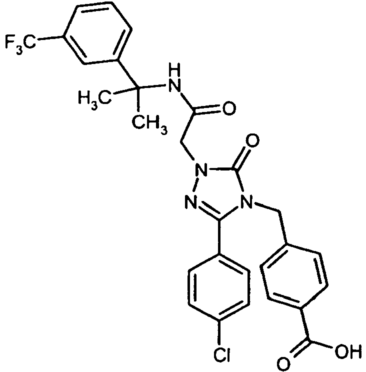 | LC/MS: Rₜ = 3.68 min [17] [ESIpos]: m/z = 573 (M+H)⁺ |
| **473** | 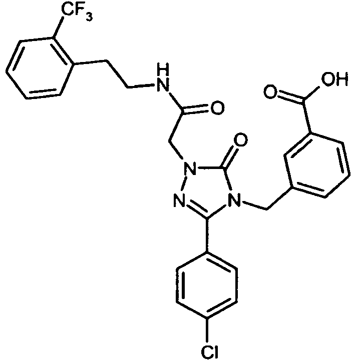 | LC/MS: Rₜ = 2.51 min [8] [ESIpos]: m/z = 559 (M+H)⁺ |
| **474** | 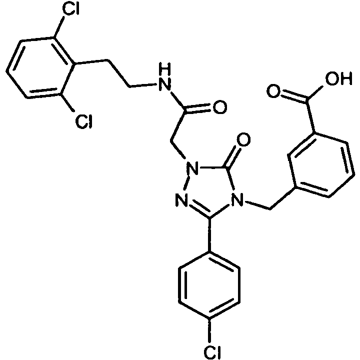 | LC/MS: Rₜ = 3.58 min [17] [ESIpos]: m/z = 559/561 (M+H)⁺ |

### Beispiel 475

### 3-(3-(4-Chlorphenyl)-1-[2-(1-methyl-1-[3-(trifluormethyl)phenyl]ethylamino)-2-oxoethyl]-5-oxo-1,5-dihydro-4H-1,2,4-triazol-4-ylmethyl)-N,N-dimethylbenzamid

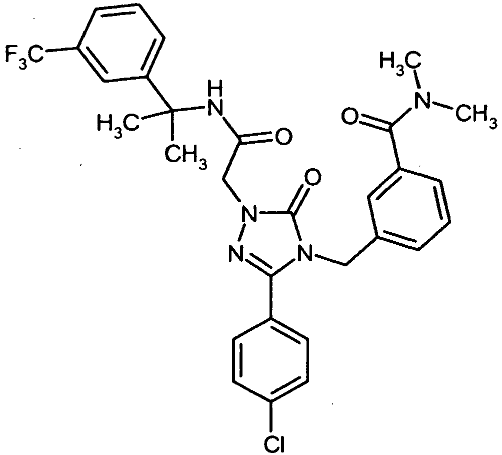

30 mg (0.052 mmol) 3-(3-(4-Chlorphenyl)-1-[2-(1-methyl-1-[3-(trifluormethyl)phenyl]ethylamino)-2-oxoethyl]-5-oxo-1,5-dihydro-4*H*-1,2,4-triazol-4-ylmethyl)-benzoesäure (Beispiel 471) werden in 0.5 ml DMF vorgelegt und mit 9.2 mg (0.068 mmol) HOBt sowie 13.0 mg (0.068 mmol) EDC-Hydrochlorid versetzt. Nach 10 min Rühren werden 6.0 mg (0.073 mmol) Dimethylamin-Hydrochlorid sowie 10.2 mg (0.079 mmol) *N*,*N*-Diisopropylethylamin zugegeben und das Gemisch über Nacht bei RT gerührt. Das Rohprodukt wird ohne weitere Aufarbeitung direkt durch präparative HPLC [Methode 10] gereinigt. Man erhält so 29.8 mg (95% d. Th.) der Zielverbindung.
LC/MS [Methode 8]: Rₜ = 2.59 min; MS [ESIpos]: m/z = 600 (M+H)⁺
¹H-NMR (400 MHz, DMSO-d₆): δ = 1.60 (s, 6H), 2.66 (s, 3H), 2.85 (s, 3H), 4.55 (s, 2H), 5.02 (s, 2H), 7.04 (s, 1H), 7.12 (d, 1H), 7.23 (d, 1H), 7.33 (t, 1H), 7.45-7.75 (m, 8H), 8.60 (s, 1H).

Auf analoge Weise werden die folgenden Verbindungen hergestellt:

| **Beispiel Nr.** | **Struktur** | **LC/MS oder HPLC, MS Rₜ [Methode]** |
|---|---|---|
| **476** | 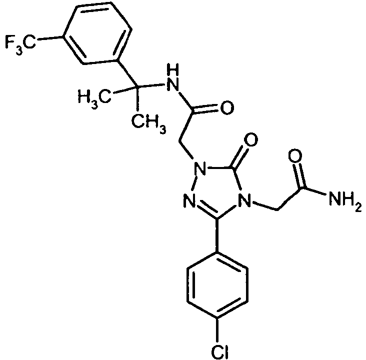 | LC/MS: Rₜ = 2.31 min [8] [ESIpos]: m/z = 496 (M+H)⁺ |
| **477** | 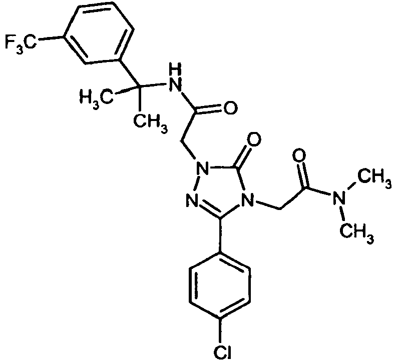 | LC/MS: Rₜ = 2.47 min [8] [ESIpos] : m/z = 524 (M+H)⁺ |
| **478** | 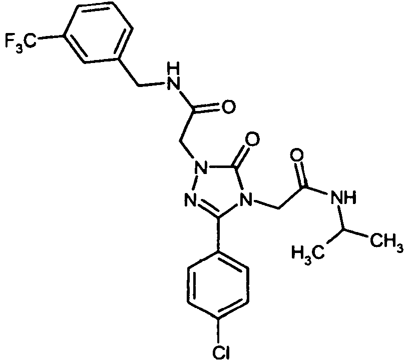 | LC/MS: Rₜ = 2.44 min [8] [ESIpos]: m/z = 510 (M+H)⁺ |

### Beispiel 479

### 2-{3-(4-Chlorphenyl)-4-[3-(hydroxymethyl)benzyl]-5-oxo-4,5-dihydro-1H-1,2,4-triazol-1-y}-N-{1-methyl-1-[3-(trifluormethyl)phenyl]ethyl}-acetamid

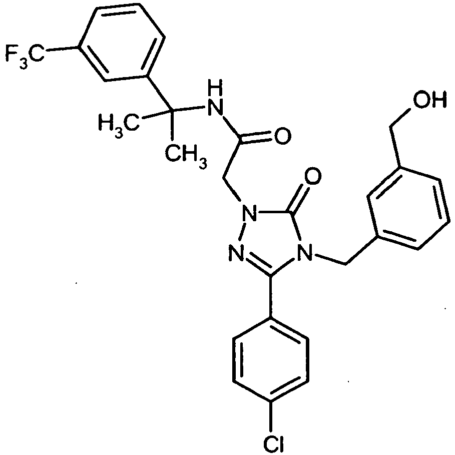

30 mg (0.052 mmol) 3-(3-(4-Chlorphenyl)-1-[2-(1-methyl-1-[3-(trifluormethyl)phenyl]ethylamino)-2-oxoethyl]-5-oxo-1,5-dihydro-4*H*-1,2,4-triazol-4-ylmethyl)-benzoesäure (Beispiel 471) werden in 2 ml THF gelöst und mit 24 mg (0.24 mmol) Triethylamin sowie 33 mg (0.24 mmol) Isobutylchlorformiat versetzt. Es wird 1 h bei RT gerührt. Anschließend wird eine Lösung von 24 mg (0.63 mmol) Natriumborhydrid in 0.05 ml Wasser langsam zugegeben. Nach 1 h wird das Gemisch mit 0.06 ml (1.05 mmol) Essigsäure versetzt und anschließend im Vakuum eingeengt. Der Rückstand wird in 10 ml Essigsäureethylester aufgenommen und mit 10 ml Wasser gewaschen. Die wässrige Phase wird einmal mit 10 ml Essigsäureethylester rückextrahiert. Die vereinigten organischen Phasen werden über Natriumsulfat getrocknet, filtriert und im Vakuum eingeengt. Das Rohprodukt wird durch präparative HPLC [Methode 10] gereinigt. Man erhält so 23 mg (79% d. Th.) der Zielverbindung.
LC/MS [Methode 19]: Rₜ = 3.53 min; MS [ESIpos]: m/z = 559 (M+H)⁺
¹H-NMR (400 MHz, DMSO-d₆): δ = 1.60 (s, 6H), 4.40 (d, 2H), 4.52 (s, 2H), 4.96 (s, 2H), 5.15 (t, 1H), 6.89 (d, 1H), 7.04 (s, 1H), 7.14-7.26 (m, 2H), 7.47-7.59 (m, 6H), 7.65 (s, 1H), 7.69 (d, 1H), 8.60 (s, 1H).

### Beispiel 480

### 2-[3-(4-Chlorphenyl)-4-(2-fluorethyl)-5-oxo-4,5-dihydro-1H-1,2,4-triazol-1-yl]-N-{1-methyl-1-[3-(trifluormethyl)phenyl]ethyl}-acetamid

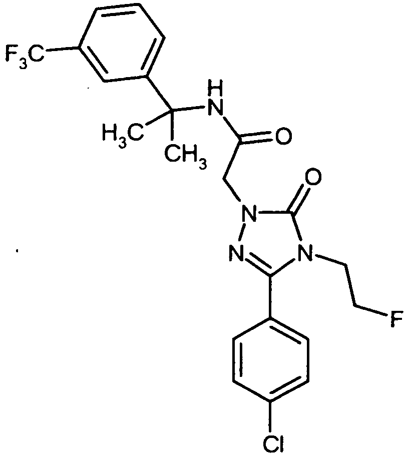

50 mg (0.10 mmol) 2-[3-(4-Chlorphenyl)-4-(2-hydroxyethyl)-5-oxo-4,5-dihydro-1*H*-1,2,4-triazol-1-yl]-*N*-{1-methyl-1-[3-(trifluormethyl)phenyl]ethyl}-acetamid aus Beispiel 396 werden in 1.5 ml Dichlormethan gelöst und bei -10°C mit 20.5 µl (0.155 mmol) Diethylaminoschwefeltrifluorid versetzt. Es wird innerhalb 1 h auf RT erwärmt. Anschließend wird die Reaktionslösung mit 5 ml Wasser versetzt und zweimal mit je 5 ml Essigsäureethylester extrahiert. Die vereinigten organischen Phasen werden über Natriumsulfat getrocknet, filtriert und im Vakuum eingeengt. Das Rohprodukt wird durch präparative HPLC [Methode 10] gereinigt. Man erhält so 37 mg (73% d. Th.) der Zielverbindung.
LC/MS [Methode 8]: Rₜ = 2.63 min; MS [ESIpos]: m/z = 485 (M+H)⁺
¹H-NMR (400 MHz, DMSO-d₆): δ = 1.60 (s, 6H), 4.00 (t, 1H), 4.07 (t, 1H), 4.43 (s, 2H), 4.44 (t, 1H), 4.62 (t, 1H), 7.48-7.70 (m, 8H), 8.59 (s, 1H).

Auf analoge Weise wird die folgende Verbindung hergestellt:

| **Beispiel Nr.** | **Struktur** | **LC/MS oder HPLC, MS Rt [Methode]** |
|---|---|---|
| **481** | 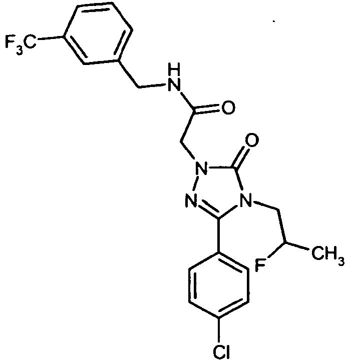 | LC/MS: Rₜ = 3.68 min [17] [ESIpos]: m/z = 471 (M+H)⁺ |

### Beispiel 482

### 2-[3-(4-Chlorphenyl)-4-(2-fluorbenzyl)-5-oxo-4,5-dihydro-1H-1,2,4-triazol-1-yl]-N-[2-(methylsulfonyl)benzyl]-acetamid

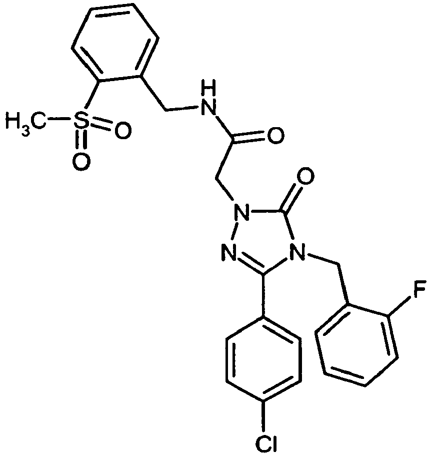

55 mg (0.11 mmol) 2-[3-(4-Chlorphenyl)-4-(2-fluorbenzyl)-5-oxo-4,5-dihydro-1*H*-1,2,4-triazol-1-yl]-*N*-[2-(methylthio)benzyl]-acetamid aus Beispiel 394 werden in 3 ml Trichlormethan gelöst und bei RT mit 82 mg (0.33 mmol) *meta*-Chlorperbenzoesäure versetzt. Nach 1 h wird die Reaktionslösung mit 10 ml Dichlormethan verdünnt und mit 5 ml gesättigter Natriumhydrogencarbonat-Lösung gewaschen. Die wässrige Phase wird einmal mit 10 ml Dichlormethan extrahiert. Die vereinigten organischen Phasen werden über Natriumsulfat getrocknet, filtriert und im Vakuum eingeengt. Das Rohprodukt wird durch präparative Dickschicht-Chromatographie (Eluent: Dichlormethan/Methanol 20:1) gereinigt. Man erhält so 18 mg (30% d. Th.) der Zielverbindung.
LC/MS [Methode 23]: Rₜ = 2.08 min; MS [ESIpos]: m/z = 529 (M+H)⁺
1H-NMR (400 MHz, DMSO-d6): δ = 3.32 (s, 3H), 4.60 (s, 2H), 4.77 (d, 2H), 5.04 (s, 2H), 7.03-7.20 (m, 3H), 7.25-7.49 (m, 1H), 7.48-7.72 (m, 7H), 7.93 (d, 1H), 8.80 (t, 1H).

### Beispiel 483

### 2-[3-(4-Chlorphenyl)-5-oxo-4-(2-hydroxybutyl)-4,5-dihydro-1H-1,2,4-triazol-1-yl]-N-(1-methyl-1-[3-(trifluormethyl)phenyl]ethyl}-acetamid (Racemat)

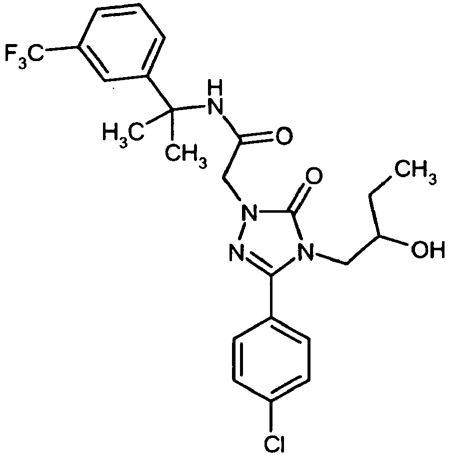

62 mg (0.12 mmol) 2-[3-(4-Chlorphenyl)-5-oxo-4-(2-oxobutyl)-4,5-dihydro-1*H*-1,2,4-triazol-1-yl]-*N*-(1-methyl-1-[3-(trifluormethyl)phenyl]ethyl)-acetamid aus Beispiel 443 werden in 2 ml Methanol gelöst und bei RT mit 4.7 mg (0.12 mmol) Natriumborhydrid versetzt. Es wird 1 h bei RT gerührt. Danach wird mit gesättigter Ammoniumchlorid-Lösung versetzt und mit 10 ml Essigsäureethylester extrahiert. Die organische Phase wird über Natriumsulfat getrocknet, filtriert und eingeengt. Man erhält so 57 mg (92% d. Th.) der Zielverbindung.
LC/MS [Methode 23]: Rₜ = 2.26 min; MS [ESIpos]: m/z = 511 (M+H)⁺.

Durch präparative HPLC an chiraler Phase [Sepaserve Sepapak-2, 5 µm, 250 mm x 20 mm; Eluent: Isohexan/Ethanol 70:30; Fluss: 15 ml/min; Temperatur: 35°C; UV-Detektion: 220 nm] wird das Racemat aus Beispiel 483 in die Enantiomere getrennt (siehe Beispiele 484 und 485):

### Beispiel 484

### 2-[3-(4-Chlorphenyl)-5-oxo-4-(2-hydroxybutyt)-4,5-dihydro-1H-1,2,4-triazol-1-yl]-N-{1-methyl-1-[3-(trifluormethyl)phenyl]ethyl}-acetamid (Enantiomer 1)

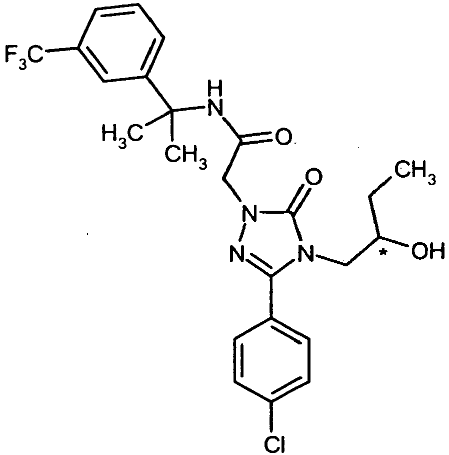

Ausbeute: 19 mg (31% d. Th.)
Rₜ = 5.55 min [Sepaserve Sepapak-2, 5 µm, 250 mm x 4.6 mm; Eluent: Isohexan/Ethanol 70:30; Fluss: 1 ml/min; Temperatur: 35°C; UV-Detektion: 220 nm]
¹H-NMR (400 MHz, CDCl₃): δ = 0.95 (t, 3H), 1.40-1.52 (m, 2H), 1.72 (s, 6H), 1.90 (br. s, 1H), 3.67-3.87 (m, 2H), 3.93 (m, 1H), 4.50 (s, 2H), 6.63 (s, 1H), 7.35-7.65 (m, 8H).

### Beispiel 485

### 2-[3-(4-Chlorphenyl)-5-oxo-4-(2-hydroxybutyl)-4,5-dihydro-1H-1,2,4-triazol-1-yl]-N-{1-methyl-1-[3-(trifluormethyl)phenyl]ethyl}-acetamid (Enantiomer 2)

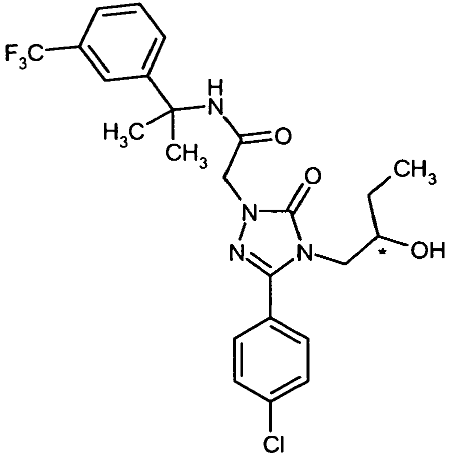

Ausbeute: 21 mg (33% d. Th.)
Rₜ = 7.44 min [Sepaserve Sepapak-2, 5 µm, 250 mm x 4.6 mm; Eluent: Isohexan/Ethanol 70:30; Fluss: 1 ml/min; Temperatur: 35°C; UV-Detektion: 220 nm].

### Beispiel 486

### 2-[3-(4-Chlorphenyl)-4-(2-cyclopropyl-2-hydroxyethyl)-5-oxo-4,5-dihydro-1H-1,2,4-triazol-1-yl]-N-{1-methyl-1-[3-(trifluormethyl)phenyl]ethyl}-acetamid

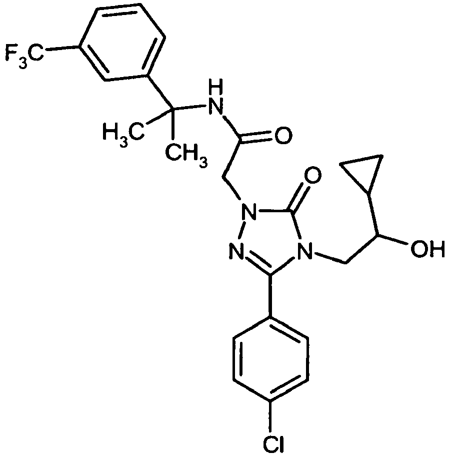

103 mg (0.21 mmol) 2-[3-(4-Chlorphenyl)-4-(2-oxoethyl)-5-oxo-4,5-dihydro-1*H*-1,2,4-triazol-1-yl]-*N*-{1-methyl-1-[3-(trifluormethyl)phenyl]ethyl}-acetamid aus Beispiel 401 werden in 1 ml THF gelöst und bei -78°C mit 1.1 ml (0.54 mmol) Cyclopropylmagnesiumbromid (0.5 M Lösung in THF) versetzt. Es wird 3 h bei RT und anschließend weitere 2 h bei 50°C gerührt. Zur Aufarbeitung wird mit gesättigter Ammoniumchlorid-Lösung versetzt und zweimal mit je 10 ml Essigsäureethylester extrahiert. Die vereinigten organischen Phasen werden über Natriumsulfat getrocknet, filtriert und im Vakuum eingeengt. Das Rohprodukt wird durch präparative HPLC [Methode 10] gereinigt. Man erhält so 12 mg (11% d. Th.) der Zielverbindung.
LC/MS [Methode 8]: Rₜ = 2.61 min; MS [ESIpos]: m/z = 523 (M+H)⁺
¹H-NMR (400 MHz, CDCl₃): δ = 0.22 (m, 1H), 0.33 (m, 1H), 0.50 (m, 2H), 0.71 (m, 1H), 1.72 (s, 6H), 2.68 (d, 1H), 3.34 (m, 1H), 3.91 (dd, 1H), 3.99 (dd, 1H), 4.50 (s, 2H), 6.62 (s, 1H), 7.37-7.65 (m, 8H).

### Analog wird hergestellt:

| **Beispiel Nr.** | **Struktur** | **LC/MS oder HPLC, MS Rₜ [Methode]** |
|---|---|---|
| **487** | 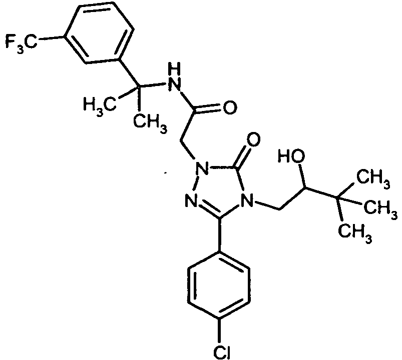 | LC/MS: Rₜ = 4.02 min [17] [ESIpos]: m/z = 539 (M+H)⁺ |

### Beispiel 488

### 2-{3-(4-Chlorphenyl)-4-[(1-hydroxycyclopropyl)methyl]-5-oxo-4,5-dihydro-1H-1,2,4-triazol-1-yl}-N-{1-methyl-1-[3-(trifluormethyl)phenyl]ethyl}-acetamid

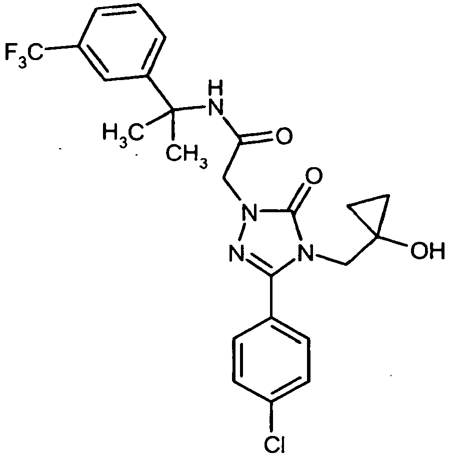

72 mg (0.14 mmol) 2-[3-(4-Chlorphenyl)-4-(2-oxoethyl)-5-oxo-4,5-dihydro-1*H*-1,2,4-triazol-1-yl]-*N*-{1-methyl-1-[3-(trifluormethyl)phenyl]ethyl}-acetamid aus Beispiel 401 werden zusammen mit 4 mg (0.014 mmol) Titan(IV)isopropylat in 0.45 ml Diethylether und 0.3 ml THF gelöst und bei RT innerhalb von 1 h mit 108 µl (0.32 mmol) Ethylmagnesiumbromid (3 M Lösung in Diethylether), verdünnt mit 0.4 ml Diethylether, versetzt. Es wird 10 min bei RT nachgerührt. Zur Aufarbeitung wird das Gemisch in 10 ml eiskalte 10%-ige Schwefelsäure gegeben und zweimal mit je 10 ml Essigsäureethylester extrahiert. Die vereinigten organischen Phasen werden über Natriumsulfat getrocknet, filtriert und im Vakuum eingeengt. Das Rohprodukt wird durch präparative HPLC [Methode 10] gereinigt. Man erhält so 5 mg (7% d. Th.) der Zielverbindung.
LC/MS [Methode 8]: Rₜ = 2.54 min; MS [ESIpos]: m/z = 509 (M+H)⁺
¹H-NMR (400 MHz, CDCl₃): δ = 0.51 (m, 2H), 0.81 (m, 2H), 1.72 (s, 6H), 3.82 (br. s, 1H), 3.95 (d, 2H), 4.52 (s, 2H), 6.62 (s, 1H), 7.37-7.62 (m, 8H).

### Beispiel 489

### 2-[3-(4-Chlorphenyl)-5-oxo-4-(3,3,3-trifluor-2-oxopropyl)-4,5-dihydro-1H-1,2,4-triazol-1-yl]-N-{1-methyl-1-[3-(trifluonnethyl)phenyl]ethyl}-acetamid

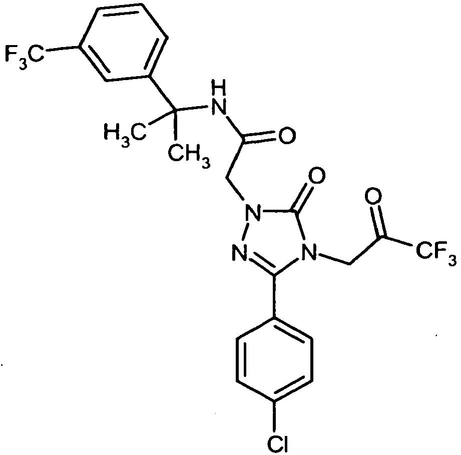

1.2 g (2.18 mmol) 2-[3-(4-Chlorphenyl)-5-oxo-4-(3,3,3-trifluor-2-hydroxypropyl)-4,5-dihydro-1*H-*1,2,4-triazol-1-yl]-*N*-{1-methyl-1-[3-(trifluormethyl)phenyl]ethyl}-acetamid aus Beispiel 452 werden in 30 ml Dichlormethan gelöst und bei 0°C mit 1.2 g (2.83 mmol) 1,1,1-Tris(acetoxy)-1,1-dihydro-1,2-benziodoxol-3-(1*H*)-on (Dess-Martin-Periodinan) versetzt. Es wird 3 h bei RT gerührt. Die Reaktionslösung wird danach mit 30 ml Essigsäureethylester verdünnt und dreimal mit je 15 ml 1 N Natronlauge gewaschen. Die organische Phase wird über Natriumsulfat getrocknet, filtriert und im Vakuum eingeengt. Das Rohprodukt wird durch Flash-Chromatographie an Kieselgel (Eluent: Cyclohexan/Essigsäureethylester zunächst 4:1, dann 1:1) gereinigt. Man erhält 0.90 g (75% d. Th.) der Zielverbindung.
LC/MS [Methode 22]: Rₜ = 2.25 min; MS [ESIpos]: m/z = 549 (M+H)⁺
¹H-NMR (400 MHz, DMSO-d₆): δ = 1.60 (s, 6H), 4.08 (s, 2H), 4.48 (s, 2H), 7.44-7.71 (m, 8H), 8.54 (s, 1H).

Weitere Ausführungsbeispiele werden parallelsynthetisch wie folgt hergestellt:

0.10 mmol der entsprechenden Amin-Komponente werden in 0.2 ml DMSO vorgelegt und mit 0.10 mmol der Triazolylessigsäure aus Beispiel 90A, gelöst in 0.2 ml DMSO, versetzt. Anschließend wird mit 25.8 mg (0.2 mmol) *N*,*N*-Diisopropylethylamin und 41.7 mg (0.130 mmol) TBTU versetzt und die Mischung über Nacht bei RT geschüttelt. Die Reaktionslösung wird danach filtriert und das Filtrat mittels präparativer LC/MS [Methode 24] aufgereinigt. Auf diese Weise werden erhalten:

| **Beispiel Nr.** | **Struktur** | **LC/MS oder HPLC, MS Rₜ [Methode]** |
|---|---|---|
| **490** | 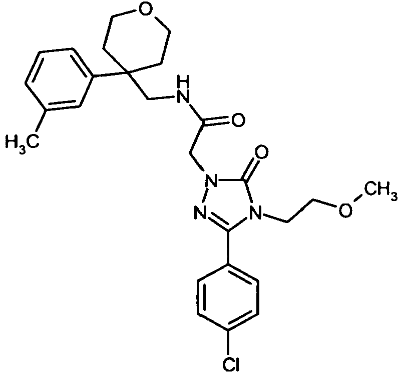 | LC/MS: Rₜ = 2.21 min [24] [ESIpos]: m/z = 500 (M+H)⁺ |
| **491** | 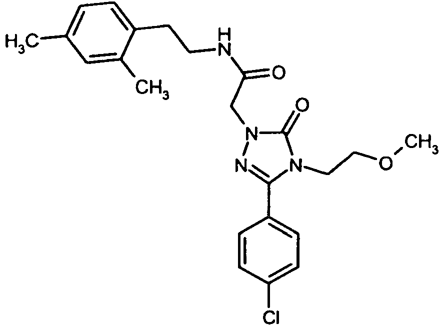 | LC/MS: Rₜ = 2.28 min [24] [ESIpos]: m/z = 443 (M+H)⁺ |
| **492** | 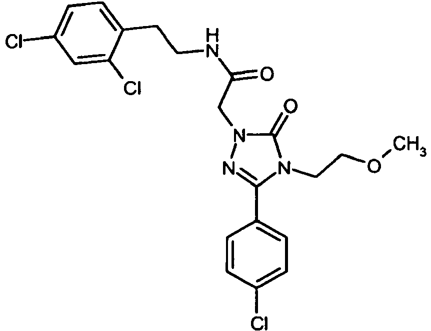 | LC/MS: Rₜ = 2.31 min [24] [ESIpos]: m/z = 484 (M+H)⁺ |
| **493** | 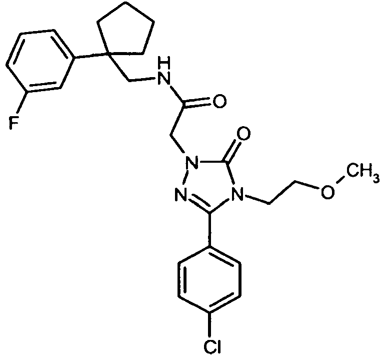 | LC/MS: Rₜ = 2.36 min [24] [ESIpos]: m/z = 487 (M+H)⁺ |
| **494** | 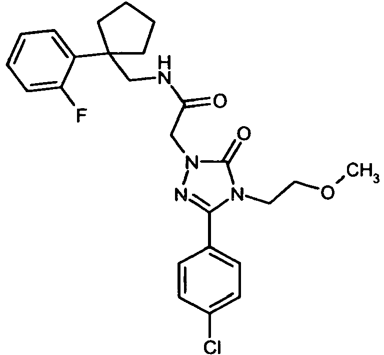 | LC/MS: Rₜ = 2.36 min [24] [ESIpos]: m/z = 487 (M+H)⁺ |
| **495** | 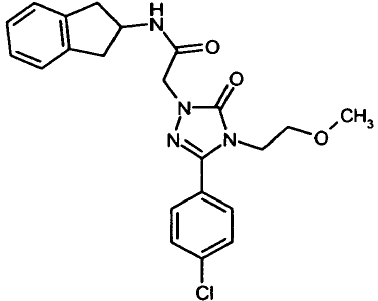 | LC/MS: Rₜ = 2.01 min [24] [ESIpos]: m/z = 427 (M+H)⁺ |

### Beispiel 496

### 2-[3-(3-Chlor-4-methyl-2-thienyl)-4-isobutyl-5-oxo-4,5-dihydro-1H-1,2,4-triazo1-1-yl]-N-[3-(trifluormethyl)phenylmethyl]-essigsäureamid

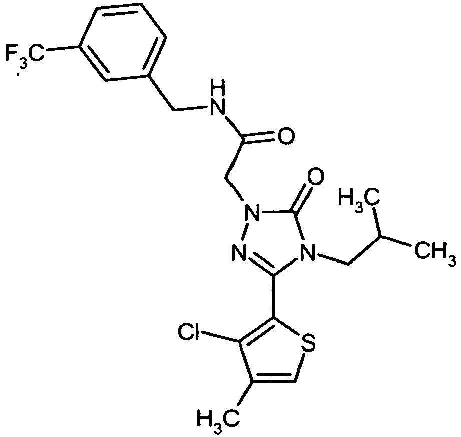

40.0 mg (0.121 mmol) 2-[3-(3-Chlor-4-methyl-2-thienyl)-4-isobutyl-5-oxo-4,5-dihydro-1*H*-1,2,4-triazol-1-yl]-essigsäure aus Beispiel 249A und 23.4 mg (0.133 mmol) 3-Trifluormethylbenzylamin werden in 1.5 ml DMF vorgelegt und mit 19.7 mg (0.146 mmol) HOBt versetzt. Man gibt 30.2 mg (0.158 mmol) EDC-Hydrochlorid hinzu und rührt das Gemisch über Nacht bei RT. Zur Aufarbeitung wird das Reaktionsgemisch mit etwa 15 ml Wasser verrührt und der entstandene Niederschlag abfiltriert, mit Wasser gewaschen und im Vakuum getrocknet. Man erhält so 43 mg (73% d. Th.) der Zielverbindung.
LC/MS [Methode 7]: Rₜ = 2.52 min;
¹H-NMR (400 MHz, DMSO-d₆): δ = 0.73 (d, 6H), 1.73-1.85 (m, 1H), 2.23 (s, 3H), 3.45 (d, 2H), 4.41 (d, 2H), 4.52 (s, 2H), 7.52-7.65 (m, 4H), 7.72 (s, 1H), 8.73 (t, 1H).

### Beispiel 497

### 2-[3-(3-Chlor-4-methyl-2-thienyl)-4-isobutyl-5-oxo-4,5-dihydro-1H-1,2,4-triazo1-1-yl]-N-[2-(trifluormethyl)phenylmethyl]-essigsäureamid

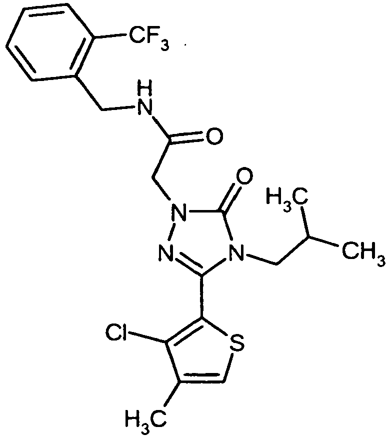

40.0 mg (0.121 mmol) 2-[3-(3-Chlor-4-methyl-2-thienyl)-4-isobutyl-5-oxo-4,5-dihydro-1*H*-1,2,4-triazol-1-yl]-essigsäure aus Beispiel 249A und 23.4 mg (0.133 mmol) 2-Trifluormethylbenzylamin werden in 1.5 ml DMF vorgelegt und mit 19.7 mg (0.146 mmol) HOBt versetzt. Man gibt 30.2 mg (0.158 mmol) EDC-Hydrochlorid hinzu und rührt das Gemisch über Nacht bei RT. Zur Aufarbeitung wird das Reaktionsgemisch mit etwa 15 ml Wasser verrührt, mit Natriumchlorid gesättigt und mit Essigsäureethylester extrahiert. Die organische Phase wird abgetrennt und eingeengt, der Rückstand in Methanol gelöst und durch präparative HPLC [Methode 12] gereinigt. Man erhält so 26 mg (44% d. Th.) der Zielverbindung.
LC/MS [Methode 7]: Rₜ = 2.52 min;
¹H-NMR (400 MHz, DMSO-d₆): δ = 0.73 (d, 6H), 1.72-1.87 (m, 1H), 2.23 (s, 3H), 3.45 (d, 2H), 4.49 (d, 2H), 4.57 (s, 2H), 7.49 (t, 1H), 7.56 (d, 1H), 7.65 (t, 1H), 7.69-7.75 (m, 2H), 8.73 (t, 1H).

### Beispiel 498

### [3-(4-Chlorphenyl)-5-oxo-1-(2-oxo-2-{[3-(trifluonnethyl)phenylmethyl]amino}ethyl)-1,5-dihydro-4H-1,2,4-triazol-4-yl]-essigsäure-tert.-butylester

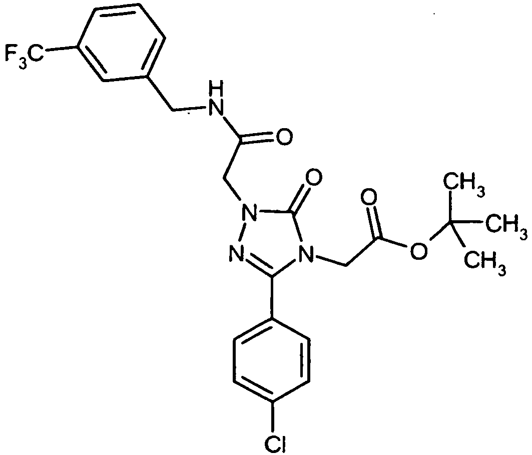

Zu 150.0 mg (0.365 mmol) 2-[3-(4-Chlorphenyl)-5-oxo-4,5-dihydro-1*H*-1,2,4-triazol-1-yl]-*N*-[3-(trifluormethyl)phenylmethyl]-essigsäureamid aus Beispiel 242A und 178.5 mg (0.548 mmol) Cäsiumcarbonat in 5.0 ml Aceton gibt man 92.6 mg (0.475 mmol) Bromessigsäure-tert.-butylester und erhitzt für 5 h unter Rückfluss. Zur Aufarbeitung wird das Reaktionsgemisch nach dem Abkühlen eingeengt, der Rückstand zwischen Wasser und Essigsäureethylester verteilt, die organische Phase abgetrennt und die wässrige Phase noch mehrfach mit Essigsäureethylester extrahiert. Die vereinigten organischen Phasen werden eingeengt und der Rückstand durch präparative HPLC [Methode 12] gereinigt. Man erhält so 139 mg (73% d. Th.) der Zielverbindung.
LC/MS [Methode 7]: Rₜ = 2.56 min;
¹H-NMR (400 MHz, DMSO-d₆): δ = 1.28 (s, 9H), 4.41 (d, 2H), 4.54 (d, 4H), 7.53-7.65 (m, 8H), 8.75 (t, 1H).

### Beispiel 499

### 2-[4-(4-Chlorphenyl)-2-oxo-3-(3,3,3-trifluor-2-hydroxypropyl)-2,3-dihydro-1H-imidazol-1-yl]-N-{1-methyl-1-[3-(trifluormethyl)phenyl]ethyl}-acetamid (Racemat)

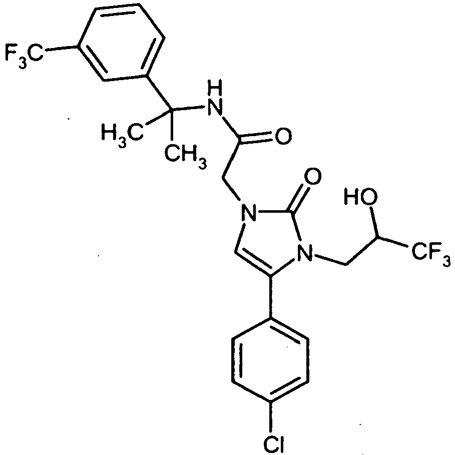

174 mg (0.397 mmol) 2-[4-(4-Chlorphenyl)-2-oxo-2,3-dihydro-1*H*-imidazol-1-yl]-*N*-{1-methyl-1-[3-(trifluormethyl)phenyl]ethyl}-acetamid aus Beispiel 234A, 12.8 mg (0.04 mmol) Tetra-*n*-butylammoniumbromid sowie 37.5 mg (0.199 mmol) Kaliumcarbonat werden in 0.45 ml DMF vorgelegt und mit 49.0 mg (0.437 mmol) 1,1,1-Trifluor-2,3-epoxypropan versetzt. Es wird 1 h bei 130°C gerührt. Die Suspension wird mit 5 ml Essigsäureethylester verdünnt und zweimal mit je 5 ml Wasser gewaschen. Die organische Phase wird über Natriumsulfat getrocknet und filtriert. Nach Einengen wird das Rohprodukt durch präparative HPLC [Methode 10] gereinigt. Man erhält 48 mg (22% d. Th.) der Zielverbindung.
LC/MS [Methode 19]: Rₜ = 3.76 min; MS [ESIpos]: m/z = 550 (M+H)⁺
¹H-NMR (400 MHz, DMSO-d₆): δ = 1.60 (s, 6H), 3.73 (dd, 1H), 3.87 (dd, 1H), 4.23 (m, 1H), 4.31 (s, 2H), 6.15 (s, 1H), 6.20 (d, 1H), 7.45-7.70 (m, 8H), 8.56 (s, 1H).

Durch präparative HPLC an chiraler Phase [Daicel Chiralpak AD-H, 5 µm, 250 mm x 20 mm; Eluent: Isohexan/Isopropanol 85:15; Fluss: 15 ml/min; Temperatur: 40°C; UV-Detektion: 220 nm] wird das Racemat aus Beispiel 499 in die Enantiomere getrennt (siehe Beispiele 500 und 501):

### Beispiel 500

### 2-[4-(4-Chlorphenyl)-2-oxo-3-(3,3,3-trifluor-2-hydroxypropyl)-2,3-dihydro-1H-imidazol-1-yl]-N-{1-methyl-1-[3-(trifluormethyl)phenyl]ethyl}-acetamid (Enantiomer 1)

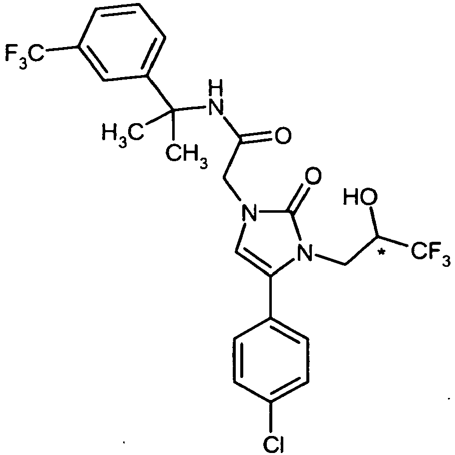

Rₜ = 7.23 min [Daicel Chiralpak AD-H, 5 µm, 250 mm x 4.6 mm; Eluent: Isohexan/Isopropanol 85:15; Fluss: 1.0 ml/min; Temperatur: 40°C; UV-Detektion: 220 nm].

### Beispiel 501

### 2-[4-(4-Chlorphenyl)-2-oxo-3-(3,3,3-trifluor-2-hydroxypropyl)-2,3-dihydro-1H-imidazol-1-yl]-N-{1-methyl-1-[3-(trifluormethyl)phenyl]ethyl}-acetamid (Enantiomer 2)

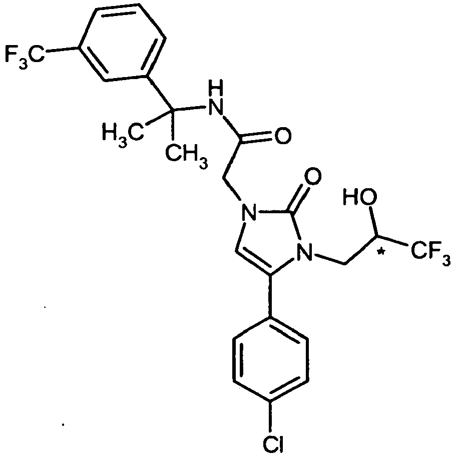

Rₜ = 5.43 min [Daicel Chiralpak AD-H, 5 µm, 250 mm x 4.6 mm; Eluent: Isohexan/Isopropanol 85:15; Fluss: 1.0 ml/min; Temperatur: 40°C; UV-Detektion: 220 nm].

### Beispiel 502

### 2-[4-(5-Chlor-2-thienyl)-3-(2-fluorbenzyl)-2-oxo-2,3-dihydro-1H-imidazol-1-yl]-N-{1-methyl-1-[3-(trifluormethyl)phenyl]ethyl}-acetamid

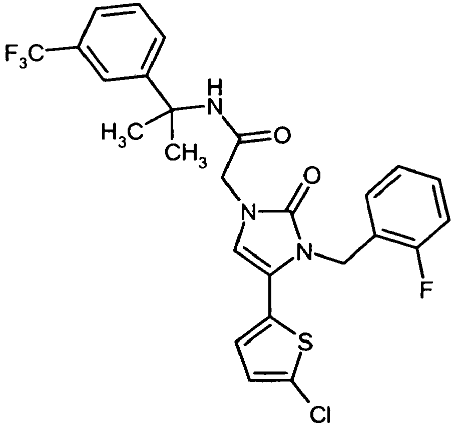

45 mg (0.123 mmol) [4-(5-Chlor-2-thienyl)-3-(2-fluorbenzyl)-2-oxo-2,3-dihydro-1H-imidazol-1-yl]-essigsäure aus Beispiel 238A, 20 mg (0.147 mmol) HOBt sowie 31 mg (0.159 mmol) EDC-Hydrochlorid werden in 1.5 ml DMF vorgelegt und 10 min gerührt. Anschließend werden 30 mg (0.147 mmol) 1-Methyl-1-[(3-trifluormethyl)phenyl]ethylamin aus Beispiel 1 A zugegeben und das Gemisch über Nacht bei RT gerührt. Zur Aufarbeitung wird das Reaktionsgemisch mit 2 ml Wasser verrührt und zweimal mit je 5 ml Essigsäureethylester extrahiert. Die vereinigten organischen Phasen werden über Natriumsulfat getrocknet, filtriert und im Vakuum eingeengt. Das Rohprodukt wird durch präparative HPLC [Methode 10] gereinigt. Man erhält so 50 mg (74% d. Th.) der Zielverbindung.
LC/MS [Methode 8]: Rₜ = 3.00 min; MS [ESIpos]: m/z = 552 (M+H)⁺
¹H-NMR (400 MHz, DMSO-d₆): δ = 1.60 (s, 6H), 4.36 (s, 2H), 4.93 (s, 2H), 6.82 (d, 1H), 6.84 (s, 1H), 6.88 (t, 1H), 7.05 (d, 1H), 7.07 (t, 1H), 7.16 (t, 1H), 7.30 (m, 1H), 7.48-7.58 (m, 2H), 7.63 (s, 1H), 7.67 (d, 1H), 8.56 (s, 1H).

Auf analoge Weise werden die folgenden Verbindungen hergestellt:

| **Beispiel Nr.** | **Struktur** | **LC/MS oder HPLC, MS Rₜ [Methode]** |
|---|---|---|
| **503** | 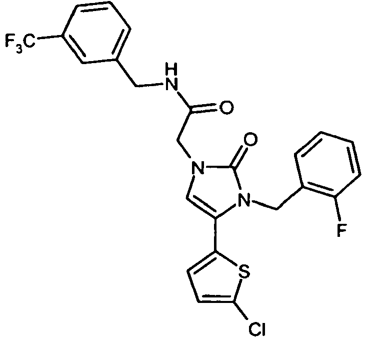 | LC/MS: Rₜ = 3.97 min [17] [ESIpos]: m/z = 524 (M+H)⁺ |
| **504** | 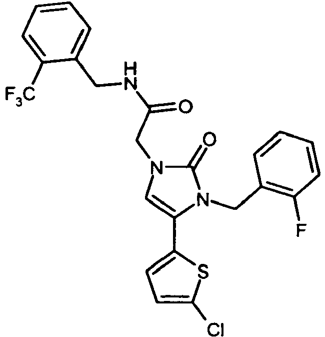 | LC/MS: Rₜ = 3.96 min [17] [ESIpos]: m/z = 524 (M+H)⁺ |
| **505** | 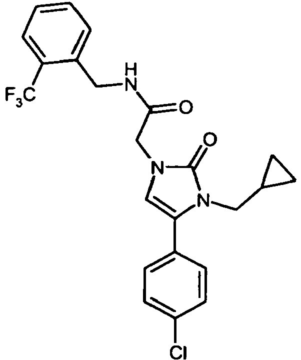 | LC/MS: Rₜ = 2.63 min [8] [ESIpos]: m/z = 464 (M+H)⁺ |

| **Beispiel Nr.** | **Struktur** | **LC/MS oder HPLC, MS Rₜ (Methode)** |
|---|---|---|
| **506** | 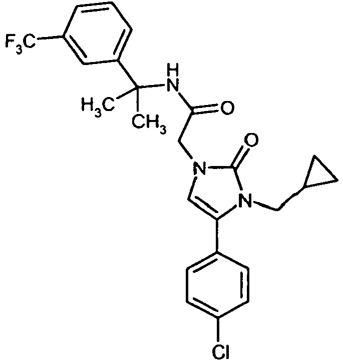 | LC/MS: Rₜ = 2.80 min [8] [ESIpos]: m/z = 492 (M+H)⁺ |
| **507** | 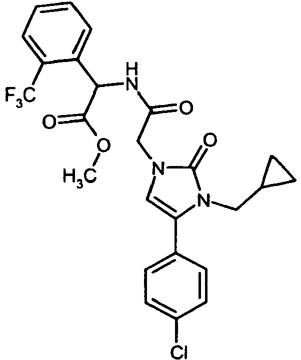 | LC/MS: Rₜ = 2.66 min [8] [ESIpos]: m/z = 522 (M+H)⁺ |
| **508** | 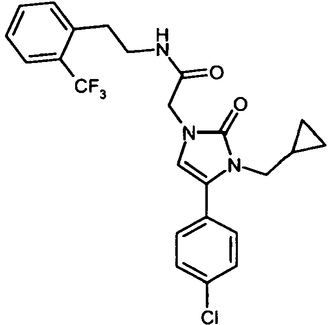 | LC/MS: Rₜ = 2.68 min [8] [ESIpos]: m/z = 478 (M+H)⁺ |

### Beispiel 509

### 2-[3-(4-Chlorphenyl)-5-oxo-4-(3,3,3-trifluor-2-hydroxypropyl)-4,5-dihydro-1H-1,2,4-triazol-1-yl]-N-{2-amino-2-oxo-1-[3-(trifluormethyl)phenyl]ethyl}-essigsäureamid

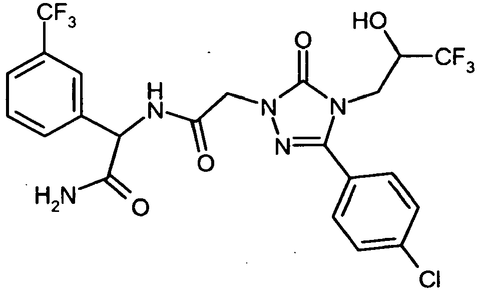

Die Carbonsäure aus Beispiel 229A (Enantiomer 1; 23 mg, 63 µmol) und HOBt (13 mg, 94 µmol) werden in 0.91 ml DMF vorgelegt und bei RT mit 18 mg (94 µmol) EDC versetzt. Nach 20 min werden 25 mg (0.11 mmol) der Verbindung aus Beispiel 184A sowie 22 µl (0.13 mmol) *NN*-Diisopropylethylamin hinzugefügt und die Mischung über Nacht bei RT gerührt. Danach wird 1 ml 1 N Salzsäure zugesetzt und die Mischung direkt durch präparative HPLC (Methode 20) aufgetrennt. Man erhält 26 mg (73% d. Th.) der Titelverbindung.
LC/MS [Methode 23]: Rₜ = 2.06 min; m/z = 566 (M+H)⁺
¹H-NMR (400 MHz, DMSO-d₆): δ = 3.82 (dd, 1H), 3.96 (br. d, 1H), 4.26 (m, 1H), 4.50-4.70 (m, 2H [ABM-System]), 5.51 (d, 1H), 6.89 (t, 1H), 7.33 (s, 1H), 7.57-7.65 (m, 3H), 7.68 (d, 1H), 7.70-7.77 (m, 3H), 7.81 (s, 1H), 7.88 (s, 1H), 9.99 (d, 1H).

### Beispiel 510

### 2-([3-(4-Chlorphenyl)-5-oxo-4-(3,3,3-trifluor-2-hydroxypropyl)-4,5-dihydro-1H-1,2,4-triazol-1-yl]-acetylamino)-N-cyclopropyl-2-[3-(trifluormethyl)phenyl]-essigsäureamid

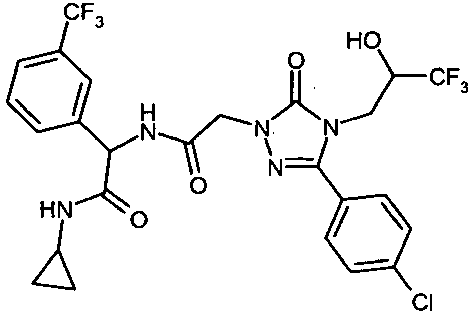

Analog zur Herstellung von Beispiel 509 werden aus 25 mg (69 µmol) der Carbonsäure aus Beispiel 229A und 31 mg (82 µmol) der Verbindung aus Beispiel 181A 27 mg (65% d. Th.) der Titelverbindung erhalten.

### LC/MS [Methode 23]: Rₜ = 2.24 min; m/z = 606 (M+H)⁺

¹H-NMR (400 MHz, DMSO-d₆): δ = 0.25-0.35 (m, 1H), 0.39-0.48 (m, 1H), 0.55-0.71 (m, 2H), 2.58-2.69 (m, 1H), 3.82 (dd, 1H), 3.96 (br. d, 1H), 4.26 (m, 1H), 4.53-4.65 (m, 2H [ABM-System]), 5.48 (d, 1H), 6.89 (t, 1H), 7.57-7.79 (m, 8H), 8.53 (d, 1H), 9.06 (d, 1H).

### Beispiel 511

### 2-[3-(4-Chlorphenyl)-5-oxo-4-(3,3,3-trifluor-2-hydroxypropyl)-4,5-dihydro-1H-1,2,4-triazol-1-yl]-N-{2-morpholin-4-yl-2-oxo-1-[3-(trifluormethyl)phenyl]ethyl)-essigsäureamid

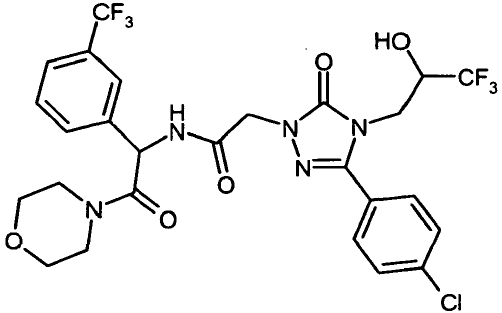

Analog zur Herstellung von Beispiel 509 werden aus 21 mg (58 µmol) der Carbonsäure aus Beispiel 229A und 28 mg (70 µmol) der Verbindung aus Beispiel 177A 36 mg (97% d. Th.) der Titelverbindung erhalten.
LC/MS [Methode 8]: Rₜ = 2.57 min; m/z = 636 (M+H)⁺
¹H-NMR (400 MHz, DMSO-d₆): δ = 3.18-3.39 (m, 2H), 3.40-3.66 (m, 6H), 3.83 (dd, 1H), 3.97 (br. d, 1H), 4.26 (m, 1H), 4.49-4.59 (m, 2H [ABM-System]), 6.03 (d, 1H), 6.90 (dd, 1H), 7.59-7.79 (m, 8H), 8.53 (d, 1H), 9.07 (d, 1H).

### Beispiel 512

### 2-[3-(4-Chlorphenyl)-5-oxo-4-(3,3,3-trifluor-2-hydroxypropyl)-4,5-dihydro-1H-1,2,4-triazol-1-yl]-N-({3-[3-(trifluormethyl)phenyl]oxetan-3-yl}methyl)-acetamid

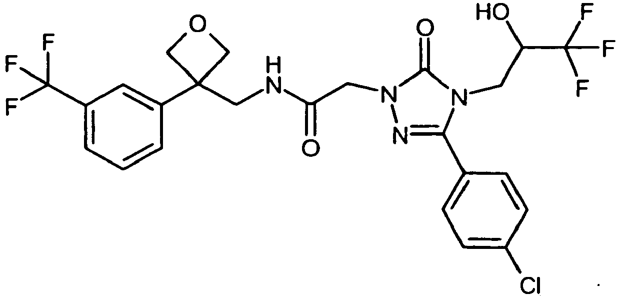

24.8 mg (68 µmol) der Carbonsäure aus Beispiel 229A (Enantiomer 1) in 710 µl DMF werden nacheinander mit 14 mg (102 µmol) HOBt, 20 mg (102 µmol) EDC, 20 mg (75 µmol) der Verbindung aus Beispiel 252A sowie 17 µl *N*,*N*-Diisopropylethylamin (95 µmol) versetzt. Die Reaktionsmischung wird über Nacht bei RT gerührt und dann direkt über präparative HPLC (Methode 20) aufgetrennt. Die produkthaltigen Fraktionen werden vereinigt und am Rotationsverdampfer eingeengt. Der Rückstand enthält die Titelverbindung sowie Nebenprodukte und wird durch Kieselgel-Chromatographie (Eluent: Cyclohexan/Essigsäureethylester 7:1) weiter gereinigt. Man erhält so 9 mg (23% d. Th) der Titelverbindung.
LC/MS [Methode 22]: Rₜ = 2.07 min; m/z = 579 [M+H]⁺
¹H-NMR (400 MHz, DMSO-d₆): δ = 3.82 (dd, 1H), 3.95 (dd, 1H), 4.26 (m, 1H), 4.36 (m, 2H [AB-System]), 4.75 (m, 2H), 4.83 (d, 2H), 4.89 (d, 2H), 6.90 (d, 1H), 7.46-7.76 (m, 8H), 8.29 (t, 1H).

### B. Bewertung der pharmakolopischen Wirksamkeit

Die pharmakologische Wirkung der erfindungsgemäßen Verbindungen kann in folgenden Assays gezeigt werden:

### Abkürzungen:

- EDTA: Ethylendiamintetraessigsäure
- DMEM: Dulbecco's Modified Eagle Medium
- FCS: Fötales Kälberserum
- HEPES: 4-(2-Hydroxyethyl)-1-piperazinethansulfonsäure
- SmGM: Smooth Muscle Cell Growth Media
- Tris-HCl: 2-Amino-2-(hydroxymethyl)-1,3-propandiol-Hydrochlorid
- UtSMC: Uterine Smooth Muscle Cells

### B-1. Zellulärer in vitro-Test zur Bestimmung der Vasopressin-Rezeptor-Aktivität

Die Identifizierung von Agonisten und Antagonisten der V1a- und V2-Vasopressin-Rezeptoren von Mensch und Ratte sowie die Quantifizierung der Wirksamkeit der erfindungsgemäßen Verbindungen erfolgt mit Hilfe von rekombinanten Zelllinien. Diese Zellen leiten sich ursprünglich von einer Ovarepithelzelle des Hamsters (Chinese Hamster Ovary, CHO K1, ATCC: American Type Culture Collection, Manassas, VA 20108, USA) ab. Die Testzelllinien exprimieren konstitutiv eine modifizierte Form des Calcium-sensitiven Photoproteins Aequorin, das nach Rekonstitution mit dem Co-Faktor Coelenterazin bei Erhöhung der freien Calcium-Konzentrationen Licht emittiert [Rizzuto R, Simpson AW, Brini M, Pozzan T, Nature 358, 325-327 (1992)]. Zusätzlich sind die Zellen stabil transfiziert mit den V1a- oder V2-Rezeptoren des Menschen oder der Ratte. Im Falle der Gs-koppelnden V2-Rezeptoren sind die Zellen mit einem weiteren Gen, das für das promiskuitive G_{α16}-Protein kodiert [Amatruda TT, Steele DA, Slepak VZ, Simon MI, Proceedings in the National Academy of Science USA 88, 5587-5591 (1991)], stabil transfiziert. Die resultierenden Vasopressin-Rezeptor-Testzellen reagieren auf Stimulation der rekombinant exprimierten Vasopressin-Rezeptoren mit einer intrazellulären Freisetzung von Calcium-Ionen, die durch die resultierende Aequorin-Lumineszens mit einem geeignetem Luminometer quantifiziert werden kann [Milligan G, Marshall F, Rees S, Trends in Pharmacological Sciences 17, 235-237 (1996)].

### Testablauf:

Die Zellen werden am Tag vor dem Test in Kulturmedium (DMEM, 10% FCS, 2 mM Glutamin, 10 mM HEPES) in 384-Loch-Mikrotiterplatten ausplattiert und in einem Zellinkubator (96% Luftfeuchtigkeit, 5% v/v CO₂, 37°C) gehalten. Am Testtag wird das Kulturmedium durch eine Tyrodelösung (140 mM NaCl, 5 mM KCl, 1 mM MgCl₂, 2 mM CaCl₂, 20 mM Glucose, 20 mM HEPES), das zusätzlich den Co-Faktor Coelenterazin (50 µM) enthält, ausgetauscht und die Mikrotiterplatte anschließend für weitere 3-4 Stunden inkubiert. Die Testsubstanzen werden in verschiedenen Konzentrationen für 10 bis 20 Minuten in den Vertiefungen der Mikrotiterplatte vorgelegt, ehe der Agonist [Arg⁸]-Vasopressin hinzugegeben und das resultierende Lichtsignal sofort im Luminometer gemessen wird. Die Berechnung der IC₅₀-Werte erfolgt mit Hilfe des Computerprogramms GraphPad PRISM (Version 3.02).

In der folgenden Tabelle sind repräsentative IC₅₀-Werte für die erfindungsgemäßen Verbindungen an der mit dem humanen V1a- bzw. V2-Rezeptor transfizierten Zelllinie aufgeführt:

**Tabelle**

| **Beispiel Nr.** | **IC₅₀ hV1a [µM]** | **IC₅₀ hV2 [µM]** |
|---|---|---|
| 25 | 6.3 | 0.18 |
| 28 | 0.030 | 0.18 |
| 29 | 0.17 | 0.009 |
| 59 | 1.1 | 0.009 |
| 63 | 0.038 | >10 |
| 75 | 0.39 | 0.026 |
| 84 | 0.32 | 0.008 |
| 101 | 0.094 | 1.1 |
| 143 | 0.96 | 0.006 |
| 145 | 0.095 | 0.007 |
| 151 | 3.4 | 0.93 |
| 153 | 0.042 | 0.012 |
| 159 | 0.44 | 0.42 |
| 207 | 0.23 | 0.063 |
| 209 | 0.14 | 0.26 |
| 214 | 6.3 | 0.18 |
| 216 | 0.073 | 0.006 |
| 219 | 0.090 | 0.009 |
| 221 | 0.165 | 0.030 |
| 227 | 0.008 | 0.037 |
| 230 | 0.783 | 0.083 |
| 241 | 0.076 | 0.271 |
| 251 | 0.037 | 0.013 |
| 252 | 0.018 | 0.447 |
| 260 | 0.030 | 0.154 |
| 262 | 0.157 | 0.005 |
| 265 | 0.028 | 0.749 |
| 284 | 0.009 | 0.191 |
| 288 | 0.761 | 0.666 |
| 296 | 0.054 | 0.009 |
| 305 | 0.093 | 0.784 |
| 313 | 0.003 | 1.025 |
| 320 | 0.024 | 1.517 |
| 321 | 0.050 | 0.51 |
| 325 | 0.107 | 0.028 |
| 340 | 0.039 | 1.149 |
| 348 | 0.004 | 0.217 |
| 357 | 0.338 | 0.005 |
| 359 | 0.019 | 0.009 |
| 364 | 0.022 | 0.008 |
| 386 | 0.611 | 0.023 |
| 394 | 0.019 | 0.019 |
| 415 | 0.870 | 0.026 |
| 421 | 0.032 | 0.088 |
| 423 | 0.157 | 0.015 |
| 433 | 0.199 | 0.009 |
| 439 | 0.058 | 0.077 |
| 443 | 0.093 | 0.023 |
| 451 | 0.012 | 0.002 |
| 457 | 0.043 | 0.029 |
| 462 | 0.373 | 0.046 |
| 467 | 0.439 | 0.026 |
| 471 | 0.039 | 0.002 |
| 475 | 0.328 | 0.020 |
| 476 | 1.229 | 0.094 |
| 482 | 0.009 | 0.25 |
| 485 | 0.012 | 0.002 |
| 489 | 0.035 | 0.002 |
| 494 | 0.049 | 0.257 |
| 496 | 0.166 | 0.576 |
| 501 | 0.012 | 0.003 |
| 503 | 0.025 | 0.221 |
| 508 | 0.005 | 0.336 |
| 509 | 0.008 | 0.004 |
| 510 | 0.004 | 0.007 |
| 511 | 0.004 | 0.011 |

### B-2. Bindungsstudien an Membranpräparationen humaner Glattmuskelzellen des Uterus zur Bestimmung der Oxytozin-Rezeptoraffinität

Humane Glattmuskelzellen des Uterus (UtSMC; Fa. Cambrex Bio Science, Walkersville, USA) werden in SmGM-2-Medium (Fa. Cambrex Bio Science) kultiviert. Nach Erreichen von 80% Konfluenz werden die Zellen in 10 ml eiskaltem Homogenisierungspuffer (10 mM Tris-HCl, 5 mM EDTA, pH 7.4) pro 175 cm²-Zellkulturflasche suspendiert und mittels eines Ultra-Turrax-Gerätes homogenisiert. Die Homogenate werden 10 Minuten lang bei 1000 g und 4°C zentrifugiert. Der Überstand wird abgenommen und 20 Minuten lang bei 35000 g und 4°C zentrifugiert. Das Membransediment mit den Oxytozin-Rezeptoren wird in 10 ml Bindungspuffer (50 mM Tris-HCl, 10 mM MgCl₂, pH 7.4) aufgenommen und bei -80°C gelagert. Für den Bindungsversuch werden 100 µg der Membranpräparation mit dem Radioliganden [³H]-Oxytozin (0.5 nM) gemischt und mit zunehmenden Konzentrationen der Testverbindungen in Bindungspuffer mit 0.1 % Rinderserumalbumin bei Raumtemperatur für 60 Minuten inkubiert. Die Inkubation wird durch 10-minütige Zentrifugation bei 10000 g und anschließendes Waschen mit 0.1% Rinderserumalbumin in Bindungspuffer bei 4°C abgestoppt. Es wird nochmals 10 Minuten lang bei 10000 g und 4°C zentrifugiert. Das Sediment wird in 0.1 ml 1 N Natronlauge resuspendiert und in Szintillationsröhrchen überführt. Nach Zugabe von 4 ml Ultima Gold-Szintillator wird die an den Membranen gebundene Radioaktivität mittels eines LS6000 IC-Szintillationszählers (Fa. BeckmanCoulter) quantifiziert. Die Radioaktivität in Gegenwart von 1 µM Oxytozin wird als nicht-spezifische Bindung definiert. Die Berechnung der IC₅₀-Werte erfolgt mit Hilfe des Computerprogramms GraphPad PRISM (Version 3.02).

### B-3. In vivo-Test zum Nachweis der kardiovaskulären Wirkung: Blutdruckmessung an narkotisierten Ratten

Bei männlichen Wistar-Ratten (350-450 g Körpergewicht) werden in Isofluran-Narkose (2% Isofluran, 33% Sauerstoff und 65% Stickoxydul) Polyethylenschläuche (PE-50; Intramedic^{®}), die mit Heparin-haltiger (500 I.E./ml) isotoner Natriumchlorid-Lösung vorgefüllt sind, in die Arteria femoralis und die Vena femoralis eingeführt und anschließend eingebunden. Über den venösen Zugang werden mit Hilfe einer Spritze die Testsubstanzen appliziert. Der arterielle Katheder wird an einen Druckwandler angeschlossen, der seine Signale in einen mit einer geeigneten Registrierungssoftware ausgestatteten Messcomputer einspeist. Anhand der kontinuierlich registrierten Druckkurve werden systolischer und diastolischer Blutdruck und hieraus berechnet der arterielle Mitteldruck sowie die Herzfrequenz bestimmt. In einer weiteren Variante des Versuchsaufbaus wird zusätzlich der Bauchraum eröffnet, die Harnblase dargestellt und über einen kleinen Schnitt ein Plastikschlauch in die Harnblase eingeführt und durch Naht fixiert, über den kontinuierlich der Urin gesammelt wird.

In einem typischen Experiment wird dem Versuchstier eine Bolusinjektion mit einer definierten Menge Arg-Vasopressin in isotoner Natriumchlorid-Lösung verabreicht und, nachdem der Blutdruck wieder Ausgangswerte erreicht hat, die zu testende Substanz als Bolus in einem geeigneten Lösungsmittel appliziert. Hiernach wird in definierten Abständen erneut die gleiche Menge Arg-Vasopressin wie zu Anfang verabreicht. Anhand der Blutdruckwerte wird ermittelt, inwieweit und wie dauerhaft die Testsubstanz der blutdrucksteigernden Wirkung des Arg-Vasopressins entgegenwirkt. Kontrolltiere erhalten nur Lösungsmittel statt der Testsubstanz.

Die erfindungsgemäßen Verbindungen bewirken nach intravenöser Applikation im Vergleich zu den Lösungsmittelkontrollen eine Hemmung des durch Arg-Vasopressin verursachten Blutdruckanstiegs.

### B-4. In vivo-Test zum Nachweis der kardiovaskulären Wirkung: Diurese-Untersuchungen an wachen Ratten in Stoffwechselkäfigen

Wistar-Ratten (300-450 g Körpergwicht) werden mit freiem Zugang zu Futter (Altromin) und Trinkwasser gehalten. Während des Versuchs werden die Tiere für 4 bis 6 Stunden einzeln in für Ratten dieser Gewichtsklasse geeigneten Stoffwechselkäfigen (Fa. Tecniplast Deutschland GmbH, D-82383 Hohenpeißenberg) mit freiem Zugang zu Trinkwasser gehalten. Am Versuchsbeginn wird den Tieren die zu prüfende Substanz in einem Volumen von 3 ml/kg Körpergewicht eines geeigneten Lösungsmittels mit Hilfe einer Schlundsonde in den Magen verabreicht. Als Kontrolle dienende Tiere erhalten nur Lösungsmittel. Kontrollen und Substanztestungen werden am selben Tag parallel durchgeführt. Kontrollgruppen und Substanzdosisgruppen bestehen aus jeweils 3 bis 6 Tieren. Während des Versuchs wird der von den Tieren ausgeschiedene Urin kontinuierlich in einem Auffangbehälter am Käfigboden gesammelt. Für jedes Tier wird gesondert das Urinvolumen pro Zeiteinheit bestimmt und die Konzentration der im Urin ausgeschiedenen Natrium-bzw. Kalium-Ionen mittels flammenphotometrischer Standardmethoden gemessen. Um eine ausreichende Urinmenge zu erhalten, wird den Tieren zu Versuchsbeginn per Schlundsonde eine definierte Menge Wasser zugeführt (typischerweise 10 ml pro kg Körpergewicht). Vor Versuchsbeginn und nach Versuchsende wird das Körpergewicht der einzelnen Tiere bestimmt.

Die erfindungsgemäßen Verbindungen bewirken nach oraler Applikation im Vergleich zu Lösungsmittel-Kontrollapplikationen eine gesteigerte Ausscheidung von Urin, die im wesentlichen auf einer gesteigerten Wasserausscheidung (Aquarese) beruht.

### C. Ausführunesbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 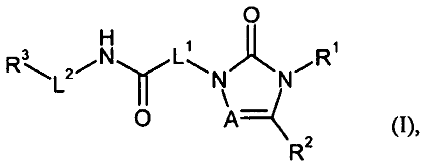 in welcher
A für N oder C-R⁴ steht, worin
R⁴ Wasserstoff oder (C₁-C₄)-Alkyl bedeutet,
R¹ für (C₁-C₆)-Alkyl, (C₂-C₆)-Alkenyl oder (C₂-C₆)-Alkinyl steht, welche jeweils einbis dreifach, gleich oder verschieden, mit Resten ausgewählt aus der Reihe Halogen, Cyano, Oxo, Trifluormethyl, (C₃-C₇)-Cycloalkyl, Phenyl, -OR¹⁰, -NR¹¹R¹², -C(=O)-OR¹³ und -C(=O)-NR¹⁴R¹⁵ substituiert sein können, worin
(i) (C₃-C₇)-Cycloalkyl bis zu zweifach, gleich oder verschieden, mit (C₁-C₄)-Alkyl, Oxo, Hydroxy, (C₁-C₄)-Alkoxy und/oder Amino substituiert sein kann,
(ii) Phenyl bis zu dreifach, gleich oder verschieden, mit Resten ausgewählt aus der Reihe Halogen, Cyano, Nitro, (C₁-C₄)-Alkyl, Trifluormethyl, Hydroxy, Hydroxymethyl, (C₁-C₄)-Alkoxy, Trifluormethoxy, (C₁-C₄)-Alkoxymethyl, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl substituiert sein kann,
(*iii*) R¹⁰, R¹¹, R¹², R¹³, R¹⁴ und R¹⁵ unabhängig voneinander bei jedem einzelnen Auftreten Wasserstoff, (C₁-C₆)-Alkyl oder (C₃-C₇)-Cycloalkyl bedeuten, wobei
(C₁-C₆)-Alkyl seinerseits bis zu zweifach, gleich oder verschieden, mit Amino, Hydroxy, (C₁-C₄)-Alkoxy, Hydroxycarbonyl und/oder (C₁-C₄)-Alkoxycarbonyl substituiert sein kann
und
(C₃-C₇)-Cycloalkyl seinerseits bis zu zweifach, gleich oder verschieden, mit (C₁-C₄)-Alkyl, Oxo, Hydroxy, (C₁-C₄)-Alkoxy und/oder Amino substituiert sein kann,
und/oder
(*iv*) R¹¹ und R¹² sowie R¹⁴ und R¹⁵ jeweils paarweise zusammen mit dem Stickstoffatom, an das sie gebunden sind, einen 4- bis 7-gliedrigen Heterocyclus bilden, welcher ein weiteres Heteroatom aus der Reihe N, O und S enthalten und bis zu zweifach, gleich oder verschieden, mit (C₁-C₄)-Alkyl, Oxo, Hydroxy, (C₁-C₄)-Alkoxy und/oder Amino substituiert sein kann,
oder
R¹ für (C₃-C₇)-Cycloalkyl steht, welches bis zu zweifach, gleich oder verschieden, mit (C₁-C₄)-Alkyl, (C₁-C₄)-Alkoxy, Hydroxy, Amino und/oder Oxo substituiert sein kann,
R² für Phenyl, Naphthyl, Thienyl, Benzothienyl, Furyl oder Benzofuryl steht, welche jeweils ein- bis dreifach, gleich oder verschieden, mit Resten ausgewählt aus der Reihe Halogen, Cyano, Nitro, (C₁-C₄)-Alkyl, Trifluormethyl, Hydroxy, (C₁-C₄)-Alkoxy, Trifluormethoxy und Phenyl substituiert sein können,
wobei der letztgenannte Phenyl-Rest seinerseits bis zu zweifach, gleich oder verschieden, mit Resten ausgewählt aus der Reihe Halogen, Cyano, Nitro, (C₁-C₄)-Alkyl, Trifluormethyl, Hydroxy, (C₁-C₄)-Alkoxy, Trifluormethoxy, Hydroxy-(C₁-C₄)-alkyl und (C₁-C₄)-Alkylthio substituiert sein kann,
L¹ für eine Gruppe der Formel -(CR^{5A}R^{5B})ₘ- steht, worin
m die Zahl 1, 2 oder 3 bedeutet
und
R^{5A} und R^{5B} unabhängig voneinander Wasserstoff oder (C₁-C₄)-Alkyl bedeuten
oder
zwei an demselben Kohlenstoffatom gebundene Reste R^{5A} und R^{5B} miteinander verknüpft sind und zusammen eine -(CH₂)ₙ-Brücke bilden, worin
n die Zahl 2, 3, 4 oder 5 bedeutet,
oder im Falle, dass m für die Zahl 2 oder 3 steht,
zwei an benachbarten (1,2- oder 2,3-) oder nicht benachbarten (1,3-) Kohlenstoffatomen gebundene Reste R^{5A} und/oder R^{5B} miteinander verknüpft sind und zusammen eine -(CH₂)ₚ-Brücke bilden, worin
p die Zahl 1, 2, 3 oder 4 bedeutet,
wobei im Falle, dass die Gruppe -CR^{5A}R^{5B}- mehrfach auftritt, die einzelnen Bedeutungen von R^{5A} und R^{5B} jeweils gleich oder verschieden sein können,
oder
L¹ für eine Gruppe der Formel 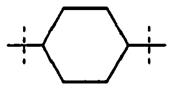 steht,
L² für eine Gruppe der Formel *-CR^{6A}R^{6B}-(CR^{7A}R^{7B})_{q}- oder
*-CR^{6A}R^{6B}-CR^{7A}R^{7B}-O- steht, worin
* die Verknüpfungsstelle mit dem N-Atom der Amid-Gruppierung bedeutet,
q die Zahl 0, 1 oder 2 bedeutet,
R^{6A} Wasserstoff oder (C₁-C₄)-Alkyl bedeutet,
R^{6B} Wasserstoff, (C₁-C₄)-Alkyl, Trifluormethyl, (C₃-C₆)-Cycloalkyl oder Phenyl, das bis zu zweifach, gleich oder verschieden, mit Halogen, (C₁-C₄)-Alkyl und/oder Trifluormethyl substituiert sein kann, oder einen Rest der Formel -C(=O)-OR¹⁶ oder -C(=O)-NR¹⁷R¹⁸ bedeutet, worin
R¹⁶, R¹⁷ und R¹⁸ unabhängig voneinander Wasserstoff, (C₁-C₄)-Alkyl oder (C₃-C₆)-Cycloalkyl darstellen
oder
R¹⁷ und R¹⁸ zusammen mit dem Stickstoffatom, an das sie gebunden sind, einen 4- bis 6-gliedrigen Heterocyclus bilden, welcher ein weiteres Heteroatom aus der Reihe N, O und S enthalten und bis zu zweifach, gleich oder verschieden, mit (C₁-C₄)-Alkyl, Hydroxy und/ oder (C₁-C₄)-Alkoxy substituiert sein kann,
oder
R^{6A} und R^{6B} miteinander verknüpft sind und zusammen eine -(CH₂)ᵣ-Brücke bilden, worin
r die Zahl 2, 3, 4 oder 5 bedeutet
und eine CH₂-Gruppe der Brücke gegen -O-, -S- oder >N-R¹⁹, worin
R¹⁹ Wasserstoff oder (C₁-C₄)-Alkyl darstellt,
ausgetauscht sein kann,
R^{7A} Wasserstoff, Fluor, (C₁-C₄)-Alkyl oder (C₁-C₄)-Alkoxy bedeutet,
R^{7B} Wasserstoff, Fluor, (C₁-C₄)-Alkyl, Hydroxy-(C₁-C₄)-alkyl oder einen Rest der Formel -OR²⁰, -NR²¹R²², -C(=O)-OR²³ oder -C(=O)-NR²⁴R²⁵ bedeutet, worin
R²⁰, R²¹, R²², R²³, R²⁴ und R²⁵ unabhängig voneinander Wasserstoff, (C₁-C₄)-Alkyl oder (C₃-C₆)-Cycloalkyl darstellen
oder
R²¹ und R²² sowie R²⁴ und R²⁵ jeweils paarweise zusammen mit dem Stickstoffatom, an das sie gebunden sind, einen 4- bis 6-gliedrigen Heterocyclus bilden, welcher ein weiteres Heteroatom aus der Reihe N, O und S enthalten und bis zu zweifach, gleich oder verschieden, mit (C₁-C₄)-Alkyl, Hydroxy und/oder (C₁-C₄)-Alkoxy substituiert sein kann,
oder
R^{7A} und R^{7B} zusammen eine Oxo-Gruppe bilden
oder
R^{7A} und R^{7B} miteinander verknüpft sind und zusammen eine -(CH₂)ₛ-Brücke bilden, worin
s die Zahl 2, 3, 4 oder 5 bedeutet
und eine CH₂-Gruppe der Brücke gegen -O-, -S- oder >N-R²⁶, worin
R²⁶ Wasserstoff oder (C₁-C₄)-Alkyl darstellt,
ausgetauscht sein kann,
wobei im Falle, dass die Gruppe -CR^{7A}R^{7B}- mehrfach auftritt, die einzelnen Bedeutungen von R^{7A} und R^{7B} jeweils gleich oder verschieden sein können,
oder
L² für eine Gruppe der Formel 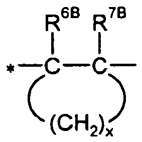 steht, worin
* die Verknüpfungsstelle mit dem N-Atom der Amid-Gruppierung bedeutet,
x die Zahl 1, 2, 3 oder 4 bedeutet,
wobei eine CH₂-Gruppe des Rings gegen -O-, -S- oder >N-R²⁷, worin
R²⁷ Wasserstoff oder (C₁-C₄)-Alkyl darstellt,
ausgetauscht sein kann,
und
R^{6B} und R^{7B} jeweils die oben angegebenen Bedeutungen haben,
R³ für Phenyl, Naphthyl oder 5- bis 10-gliedriges Heteroaryl mit bis zu drei Heteroatomen aus der Reihe N, O und/oder S steht, welche jeweils ein- bis dreifach, gleich oder verschieden, mit Resten ausgewählt aus der Reihe Halogen, Cyano, Nitro, (C₁-C₄)-Alkyl, Trifluormethyl, Hydroxy, (C₁-C₄)-Alkoxy, Trifluormethoxy, (C₁-C₄)-Alkylthio, (C₁-C₄)-Alkylsulfinyl, (C₁-C₄)-Alkylsulfonyl, Di-(C₁-C₄)-alkylamino und Phenyl substituiert sein können,
wobei der letztgenannte Phenyl-Rest seinerseits bis zu zweifach, gleich oder verschieden, mit Halogen, Cyano, Nitro, (C₁-C₄)-Alkyl, (C₁-C₄)-Alkoxy, Trifluormethyl und/oder Trifluormethoxy substituiert sein kann,
oder die Gruppierung
L²-R³ zusammen eine Gruppe der Formel 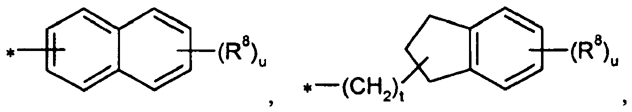 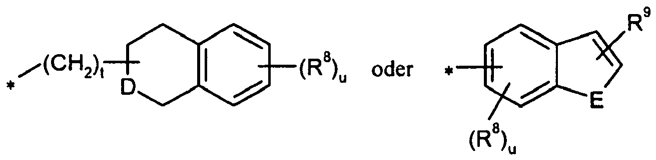 bildet, worin
* die Verknüpfungsstelle mit dem N-Atom der Amid-Gruppierung bedeutet,
D CH₂ oder O bedeutet,
E NH, N-CH₃, O oder S bedeutet,
t die Zahl 0 oder 1 bedeutet,
R⁸ einen Substituenten ausgewählt aus der Reihe Halogen, Cyano, Nitro, (C₁-C₄)-Alkyl, (C₁-C₄)-Alkoxy, Trifluormethyl und Trifluormethoxy bedeutet,
u die Zahl 0, 1 oder 2 bedeutet,
wobei im Falle, dass der Substituent R⁸ mehrfach auftritt, seine Bedeutungen gleich oder verschieden sein können,
und
R⁹ Wasserstoff oder (C₁-C₄)-Alkyl bedeutet,
sowie ihre Salze, Solvate und Solvate der Salze.

2. Verbindung der Formel (I) nach Anspruch 1, in welcher
A für N oder C-R⁴ steht, worin
R⁴ Wasserstoff oder (C₁-C₄)-Alkyl bedeutet,
R¹ für (C₁-C₆)-Alkyl, das mit Hydroxy, (C₁-C₆)-Alkoxy, (C₃-C₇)-Cycloalkyl oder Phenyl substituiert sein kann, oder für (C₃-C₇)-Cycloalkyl steht,
wobei die genannten Cycloalkyl-Reste ihrerseits bis zu zweifach, gleich oder verschieden, mit (C₁-C₄)-Alkyl, (C₁-C₄)-Alkoxy, Hydroxy, Amino und/oder Oxo
und
der Phenyl-Rest bis zu dreifach, gleich oder verschieden, mit Halogen, Cyano, Nitro, (C₁-C₄)-Alkyl, (C₁-C₄)-Alkoxy, Trifluormethyl und/oder Trifluormethoxy substituiert sein können,
R² für Phenyl, Naphthyl, Thienyl, Benzothienyl, Furyl oder Benzofuryl steht, welche jeweils bis zu dreifach, gleich oder verschieden, mit Halogen, Cyano, Nitro, (C₁-C₄)-Alkyl, (C₁-C₄)-Alkoxy, Trifluormethyl, Trifluormethoxy und/oder Phenyl substituiert sein können,
wobei der letztgenannte Phenyl-Rest seinerseits bis zu zweifach, gleich oder verschieden, mit Halogen, Cyano, Nitro, (C₁-C₄)-Alkyl, (C₁-C₄)-Alkoxy, Trifluormethyl und/oder Trifluormethoxy substituiert sein kann,
L¹ für eine Gruppe der Formel -(CR^{5A}R^{5B})ₘ- steht, worin
m die Zahl 1, 2 oder 3 bedeutet
und
R^{5A} und R^{5B} unabhängig voneinander Wasserstoff oder (C₁-C₄)-Alkyl bedeuten
oder
zwei an demselben Kohlenstoffatom gebundene Reste R^{5A} und R^{5B} miteinander verknüpft sind und zusammen eine -(CH₂)ₙ-Brücke bilden, worin
n die Zahl 2, 3, 4 oder 5 bedeutet,
oder im Falle, dass m für die Zahl 2 oder 3 steht,
zwei an benachbarten (1,2- oder 2,3-) oder nicht benachbarten (1,3-) Kohlenstoffatomen gebundene Reste R^{5A} und/oder R^{5B} miteinander verknüpft sind und zusammen eine -(CH₂)ₚ-Brücke bilden, worin
p die Zahl 1, 2, 3 oder 4 bedeutet,
wobei im Falle, dass die Gruppe -CR^{5A}R^{5B}- mehrfach auftritt, die einzelnen Bedeutungen von R^{5A} und R^{5B} jeweils gleich oder verschieden sein können,
oder
L¹ für eine Gruppe der Formel 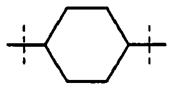 steht,
L² für eine Gruppe der Formel *-CR^{6A}R^{6B}-(CR^{7A}R^{7B})_{q}- oder
*-CR^{6A}R^{6B}-CR^{7A}R^{7B}-O- steht, worin
* die Verknüpfungsstelle mit dem N-Atom der Amid-Gruppierung bedeutet,
q die Zahl 0, 1 oder 2 bedeutet,
R^{6A} Wasserstoff oder (C₁-C₄)-Alkyl bedeutet,
R^{6B} Wasserstoff, (C₁-C₄)-Alkyl, Trifluormethyl, (C₃-C₆)-Cycloalkyl oder Phenyl, das bis zu zweifach, gleich oder verschieden, mit Halogen, (C₁-C₄)-Alkyl und/oder Trifluormethyl substituiert sein kann, bedeutet
oder
R^{6A} und R^{6B} miteinander verknüpft sind und zusammen eine -(CH₂)ᵣ-Brücke bilden, worin
r die Zahl 2, 3, 4 oder 5 bedeutet,
und
R^{7A} und R^{7B} unabhängig voneinander Wasserstoff oder (C₁-C₄)-Alkyl bedeuten,
wobei im Falle, dass die Gruppe -CR^{7A}R^{7B}- mehrfach auftritt, die einzelnen Bedeutungen von R^{7A} und R^{7B} jeweils gleich oder verschieden sein können,
R³ für Phenyl, Naphthyl oder 5- bis 10-gliedriges Heteroaryl mit bis zu drei Heteroatomen aus der Reihe N, O und/oder S steht, welche jeweils bis zu dreifach, gleich oder verschieden, mit Halogen, Cyano, Nitro, (C₁-C₄)-Alkyl, (C₁-C₄)-Alkoxy, Trifluormethyl, Trifluormethoxy, Di-(C₁-C₄)-alkylamino und/oder Phenyl substituiert sein können,
wobei der letztgenannte Phenyl-Rest seinerseits bis zu zweifach, gleich oder verschieden, mit Halogen, Cyano, Nitro, (C₁-C₄)-Alkyl, (C₁-C₄)-Alkoxy, Trifluormethyl und/oder Trifluormethoxy substituiert sein kann,
oder die Gruppierung
L²-R³ zusammen eine Gruppe der Formel 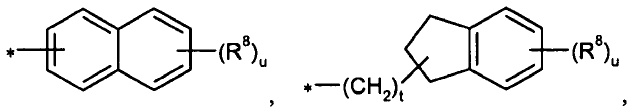 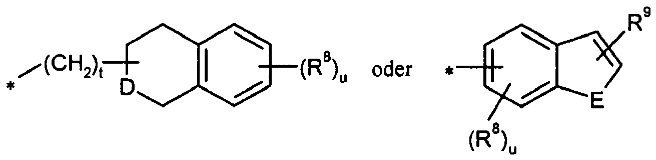 bildet, worin
* die Verknüpfungsstelle mit dem N-Atom der Amid-Gruppierung bedeutet,
D CH₂ oder O bedeutet,
E NH, N-CH₃, O oder S bedeutet,
t die Zahl 0 oder 1 bedeutet,
R⁸ einen Substituenten ausgewählt aus der Reihe Halogen, Cyano, Nitro, (C₁-C₄)-Alkyl, (C₁-C₄)-Alkoxy, Trifluormethyl und Trifluormethoxy bedeutet,
u die Zahl 0, 1 oder 2 bedeutet,
wobei im Falle, dass der Substituent R⁸ mehrfach auftritt, seine Bedeutungen gleich oder verschieden sein können,
und
R⁹ Wasserstoff oder (C₁-C₄)-Alkyl bedeutet,
sowie ihre Salze, Solvate und Solvate der Salze.

3. Verbindung der Formel (I) nach Anspruch 1, in welcher
A für N oder C-R⁴ steht, worin
R⁴ Wasserstoff oder (C₁-C₄)-Alkyl bedeutet,
R¹ für (C₁-C₆)-Alkyl steht, welches ein- bis dreifach, gleich oder verschieden, mit Resten ausgewählt aus der Reihe Fluor, Chlor, Cyano, Oxo, Trifluormethyl, (C₃-C₆)-Cycloalkyl, Phenyl, -OR¹⁰, -NR¹¹R¹², -C(=O)-OR¹³ und -C(=O)-NR¹⁴R¹⁵ substituiert sein kann, worin
(*i*) (C₃-C₆)-Cycloalkyl bis zu zweifach, gleich oder verschieden, mit (C₁-C₄)-Alkyl, Oxo, Hydroxy, (C₁-C₄)-Alkoxy und/oder Amino substituiert sein kann,
(*ii*) Phenyl bis zu dreifach, gleich oder verschieden, mit Resten ausgewählt aus der Reihe Fluor, Chlor, Cyano, (C₁-C₄)-Alkyl, Trifluormethyl, Hydroxy, Hydroxymethyl, (C₁-C₄)-Alkoxy, Trifluormethoxy, (C₁-C₄)-Alkoxymethyl, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl substituiert sein kann,
(*iii*) R¹⁰, R¹¹, R¹², R¹³, R¹⁴ und R¹⁵ unabhängig voneinander bei jedem einzelnen Auftreten Wasserstoff, (C₁-C₄)-Alkyl oder (C₃-C₆)-Cycloalkyl bedeuten, wobei
(C₁-C₄)-Alkyl seinerseits bis zu zweifach, gleich oder verschieden, mit Amino, Hydroxy, (C₁-C₄)-Alkoxy, Hydroxycarbonyl und/oder (C₁-C₄)-Alkoxycarbonyl substituiert sein kann
und
(C₃-C₆)-Cycloalkyl seinerseits bis zu zweifach, gleich oder verschieden, mit (C₁-C₄)-Alkyl, Oxo, Hydroxy, (C₁-C₄)-Alkoxy und/oder Amino substituiert sein kann,
und/oder
(iv) R¹¹ und R¹² sowie R¹⁴ und R¹⁵ jeweils paarweise zusammen mit dem Stickstoffatom, an das sie gebunden sind, einen 4- bis 6-gliedrigen Heterocyclus bilden, welcher ein weiteres Heteroatom aus der Reihe N und O enthalten und bis zu zweifach, gleich oder verschieden, mit (C₁-C₄)-Alkyl, Oxo, Hydroxy, (C₁-C₄)-Alkoxy und/oder Amino substituiert sein kann,
oder
R¹ für (C₂-C₆)-Alkenyl, welches mit Hydroxy, (C₁-C₄)-Alkoxy, Hydroxycarbonyl oder (C₁-C₄)-Alkoxycarbonyl substituiert sein kann,
oder
für (C₃-C₆)-Cycloalkyl, welches bis zu zweifach, gleich oder verschieden, mit (C₁-C₄)-Alkyl, (C₁-C₄)-Alkoxy, Hydroxy, Amino und/oder Oxo substituiert sein kann,
steht,
R² für Phenyl oder Thienyl steht, welche ein- bis dreifach, gleich oder verschieden, mit Resten ausgewählt aus der Reihe Fluor, Chlor, Cyano, (C₁-C₄)-Alkyl, Trifluormethyl, Hydroxy, (C₁-C₄)-Alkoxy, Trifluormethoxy und Phenyl substituiert sein können,
wobei der letztgenannte Phenyl-Rest seinerseits bis zu zweifach, gleich oder verschieden, mit Resten ausgewählt aus der Reihe Fluor, Chlor, Cyano, (C₁-C₄)-Alkyl, Trifluormethyl, (C₁-C₄)-Alkoxy und Trifluormethoxy substituiert sein kann,
L¹ für eine Gruppe der Formel -CR^{5A}R^{5B}- steht, worin
R^{5A} und R^{5B} unabhängig voneinander Wasserstoff oder (C₁-C₄)-Alkyl bedeuten,
L² für eine Gruppe der Formel *-CR^{6A}R^{6B}-(CR^{7A}R^{7B})_{q}- steht, worin
* die Verknüpfungsstelle mit dem N-Atom der Amid-Gruppierung bedeutet,
q die Zahl 0 oder 1 bedeutet,
R^{6A} Wasserstoff oder (C₁-C₄)-Alkyl bedeutet,
R^{6B} Wasserstoff, (C₁-C₄)-Alkyl, Trifluormethyl, (C₃-C₆)-Cycloalkyl oder Phenyl, das bis zu zweifach, gleich oder verschieden, mit Fluor, Chlor, (C₁-C₄)-Alkyl und/oder Trifluormethyl substituiert sein kann, oder einen Rest der Formel -C(=O)-OR¹⁶ oder -C(=O)-NR¹⁷R¹⁸ bedeutet, worin
R¹⁶, R¹⁷ und R¹⁸ unabhängig voneinander Wasserstoff, (C₁-C₄)-Alkyl oder (C₃-C₆)-Cycloalkyl darstellen
oder
R¹⁷ und R¹⁸ zusammen mit dem Stickstoffatom, an das sie gebunden sind, einen 4- bis 6-gliedrigen Heterocyclus bilden, welcher ein weiteres Heteroatom aus der Reihe N und O enthalten und bis zu zweifach, gleich oder verschieden, mit (C₁-C₄)-Alkyl, Hydroxy und/oder (C₁-C₄)-Alkoxy substituiert sein kann,
oder
R^{6A} und R^{6B} miteinander verknüpft sind und zusammen eine -(CH₂)ᵣ-Brücke bilden, worin
r die Zahl 2, 3, 4 oder 5 bedeutet
und eine CH₂-Gruppe der Brücke gegen -O- ausgetauscht sein kann,
R^{7A} Wasserstoff, Fluor oder (C₁-C₄)-Alkyl bedeutet,
R^{7B} Wasserstoff, Fluor, (C₁-C₄)-Alkyl, Hydroxy-(C₁-C₄)-alkyl oder einen Rest der Formel -OR²⁰, -NR²¹R²², -C(=O)-OR²³ oder -C(=O)-NR²⁴ R²⁵ bedeutet, worin
R²⁰ R²¹ R²², R²³ R²⁴ und R²⁵ unabhängig voneinander Wasserstoff, (C₁-C₄)-Alkyl oder (C₃-C₆)-Cycloalkyl darstellen
oder
R²¹ und R²² sowie R²⁴ und R²⁵ jeweils paarweise zusammen mit dem Stickstoffatom, an das sie gebunden sind, einen 4- bis 6-gliedrigen Heterocyclus bilden, welcher ein weiteres Heteroatom aus der Reihe N und O enthalten und bis zu zweifach, gleich oder verschieden, mit (C₁-C₄)-Alkyl, Hydroxy und/oder (C₁-C₄)-Alkoxy substituiert sein kann,
oder
R^{7A} und R^{7B} zusammen eine Oxo-Gruppe bilden
oder
R^{7A} und R^{7B} miteinander verknüpft sind und zusammen eine -(CH₂)ₛ-Brücke bilden, worin
s die Zahl 2, 3, 4 oder 5 bedeutet
und eine CH₂-Gruppe der Brücke gegen -O- ausgetauscht sein kann,
oder
L² für eine Gruppe der Formel 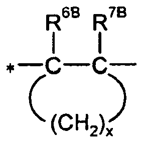 steht, worin
* die Verknüpfungsstelle mit dem N-Atom der Amid-Gruppierung bedeutet,
x die Zahl 1, 2, 3 oder 4 bedeutet,
wobei eine CH₂-Gruppe des Rings gegen -O- ausgetauscht sein kann,
und
R^{6B} und R^{7B} jeweils die oben angegebenen Bedeutungen haben,
und
R³ für Phenyl, Naphthyl oder 5- bis 10-gliedriges Heteroaryl mit bis zu zwei Heteroatomen aus der Reihe N, O und/oder S steht, welche jeweils ein- bis dreifach, gleich oder verschieden, mit Resten ausgewählt aus der Reihe Fluor, Chlor, Cyano, (C₁-C₄)-Alkyl, Trifluormethyl, Hydroxy, (C₁-C₄)-Alkoxy, Trifluormethoxy, (C₁-C₄)-Alkylthio, (C₁-C₄)-Alkylsulfonyl, Di-(C₁-C₄)-alkylamino und Phenyl substituiert sein können,
wobei der letztgenannte Phenyl-Rest seinerseits bis zu zweifach, gleich oder verschieden, mit Fluor, Chlor, Cyano, (C₁-C₄)-Alkyl, (C₁-C₄)-Alkoxy, Trifluormethyl und/oder Trifluormethoxy substituiert sein kann,
sowie ihre Salze, Solvate und Solvate der Salze.

4. Verbindung der Formel (I) nach Anspruch 1 oder 2, in welcher
A für N oder C-R⁴ steht, worin
R⁴ Wasserstoff oder (C₁-C₄)-Alkyl bedeutet,
R¹ für (C₁-C₆)-Alkyl, das mit Hydroxy, (C₁-C₄)-Alkoxy, (C₃-C₆)-Cycloalkyl oder Phenyl substituiert sein kann, oder für (C₃-C₆)-Cycloalkyl steht,
wobei die genannten Cycloalkyl-Reste ihrerseits bis zu zweifach, gleich oder verschieden, mit (C₁-C₄)-Alkyl, (C₁-C₄)-Alkoxy, Hydroxy und/oder Amino
und
der Phenyl-Rest bis zu zweifach, gleich oder verschieden, mit Halogen, Cyano, Nitro, (C₁-C₄)-Alkyl, (C₁-C₄)-Alkoxy, Trifluormethyl und/oder Trifluormethoxy substituiert sein können,
R² für Phenyl, Naphthyl, Thienyl oder Benzothienyl steht, welche jeweils bis zu dreifach, gleich oder verschieden, mit Halogen, Cyano, Nitro, (C₁-C₄)-Alkyl, (C₁-C₄)-Alkoxy, Trifluormethyl, Trifluormethoxy und/oder Phenyl substituiert sein können,
wobei der letztgenannte Phenyl-Rest seinerseits bis zu zweifach, gleich oder verschieden, mit Halogen, Cyano, Nitro, (C₁-C₄)-Alkyl, (C₁-C₄)-Alkoxy, Trifluormethyl und/oder Trifluormethoxy substituiert sein kann,
L¹ für eine Gruppe der Formel -(CR^{5A}R^{5B})ₘ- steht, worin
m die Zahl 1, 2 oder 3 bedeutet
und
R^{5A} und R^{5B} unabhängig voneinander Wasserstoff oder Methyl bedeuten
oder
zwei an demselben Kohlenstoffatom gebundene Reste R^{5A} und R^{5B} miteinander verknüpft sind und zusammen eine -(CH₂)ₙ-Brücke bilden, worin
n die Zahl 2, 3, 4 oder 5 bedeutet,
oder im Falle, dass m für die Zahl 2 oder 3 steht,
zwei an benachbarten (1,2- oder 2,3-) oder nicht benachbarten (1,3-) Kohlenstoffatomen gebundene Reste R^{5A} und/oder R^{5B} miteinander verknüpft sind und zusammen eine -(CH₂)ₚ-Brücke bilden, worin
p die Zahl 1, 2, 3 oder 4 bedeutet,
wobei im Falle, dass die Gruppe -CR^{5A}R^{5B}- mehrfach auftritt, die einzelnen Bedeutungen von R^{5A} und R^{5B} jeweils gleich oder verschieden sein können,
oder
L¹ für eine Gruppe der Formel 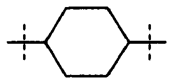 steht,
L² für eine Gruppe der Formel *-CR^{6A}R^{6B}-(CH₂)_{q} steht, worin
* die Verknüpfungsstelle mit dem N-Atom der Amid-Gruppierung bedeutet,
q die Zahl 0 oder 1 bedeutet,
R^{6A} Wasserstoff oder Methyl bedeutet,
R^{6B} Wasserstoff, (C₁-C₄)-Alkyl, Trifluormethyl oder (C₃-C₆)-Cycloalkyl bedeutet
oder
R^{6A} und R^{6B} miteinander verknüpft sind und zusammen eine -(CH₂)ᵣ-Brücke bilden, worin
r die Zahl 2, 3, 4 oder 5 bedeutet,
und
R³ für Phenyl, Naphthyl oder 5- bis 10-gliedriges Heteroaryl mit bis zu zwei Heteroatomen aus der Reihe N, O und/oder S steht, welche jeweils bis zu dreifach, gleich oder verschieden, mit Halogen, Cyano, Nitro, (C₁-C₄)-Alkyl, (C₁-C₄)-Alkoxy, Trifluormethyl, Trifluormethoxy und/oder Phenyl substituiert sein können,
wobei der letztgenannte Phenyl-Rest seinerseits bis zu zweifach, gleich oder verschieden, mit Halogen, Cyano, Nitro, (C₁-C₄)-Alkyl, (C₁-C₄)-Alkoxy, Trifluormethyl und/oder Trifluormethoxy substituiert sein kann,
sowie ihre Salze, Solvate und Solvate der Salze.

5. Verbindung der Formel (I) nach Anspruch 1 oder 3, in welcher
A für N oder C-R⁴ steht, worin
R⁴ Wasserstoff oder Methyl bedeutet,
R¹ für (C₁-C₆)-Alkyl steht, welches ein- bis zweifach, gleich oder verschieden, mit Resten ausgewählt aus der Reihe Fluor, Oxo, Trifluormethyl, (C₃-C₆)-Cycloalkyl, Phenyl, -OR¹⁰, -C(=O)-OR¹³ und -C(=O)-NR¹⁴R¹⁵ substituiert sein kann, worin
(*i*) (C₃-C₆)-Cycloalkyl bis zu zweifach, gleich oder verschieden, mit (C₁-C₄)-Alkyl, Hydroxy und/oder (C₁-C₄)-Alkoxy substituiert sein kann,
(*ii*) Phenyl bis zu zweifach, gleich oder verschieden, mit Resten ausgewählt aus der Reihe Fluor, Chlor, Cyano, (C₁-C₄)-Alkyl, Trifluormethyl, Hydroxymethyl, (C₁-C₄)-Alkoxy, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl substituiert sein kann,
und
(*iii*) R¹⁰, R¹³, R¹⁴ und R¹⁵ unabhängig voneinander bei jedem einzelnen Auftreten Wasserstoff, (C₁-C₄)-Alkyl oder (C₃-C₆)-Cycloalkyl bedeuten, wobei
(C₁-C₄)-Alkyl seinerseits bis zu zweifach, gleich oder verschieden, mit Hydroxy, (C₁-C₄)-Alkoxy, Hydroxycarbonyl und/oder (C₁-C₄)-Alkoxycarbonyl substituiert sein kann
und
(C₃-C₆)-Cycloalkyl seinerseits bis zu zweifach, gleich oder verschieden, mit (C₁-C₄)-Alkyl, Hydroxy und/oder (C₁-C₄)-Alkoxy substituiert sein kann,
oder
R¹ für (C₂-C₆)-Alkenyl, welches mit Hydroxycarbonyl oder (C₁-C₄)-Alkoxycarbonyl substituiert sein kann,
oder
für (C₃-C₆)-Cycloalkyl, welches bis zu zweifach, gleich oder verschieden, mit (C₁-C₄)-Alkyl, (C₁-C₄)-Alkoxy und/oder Hydroxy substituiert sein kann,
steht,
R² für Phenyl oder Thienyl steht, welche ein- bis zweifach, gleich oder verschieden, mit Resten ausgewählt aus der Reihe Fluor, Chlor, (C₁-C₄)-Alkyl, Trifluormethyl, (C₁-C₄)-Alkoxy und Trifluormethoxy substituiert sein können,
L¹ für eine Gruppe der Formel -CR^{5A}R^{5B}- steht, worin
R^{5A} und R^{5B} unabhängig voneinander Wasserstoff oder Methyl bedeuten,
L² für eine Gruppe der Formel *-CR^{6A}R^{6B}-(CR^{7A}R^{7B})_{q}- steht, worin
* die Verknüpfungsstelle mit dem N-Atom der Amid-Gruppierung bedeutet,
q die Zahl 0 oder 1 bedeutet,
R^{6A} Wasserstoff oder Methyl bedeutet,
R^{6B} Wasserstoff, Methyl, Trifluormethyl oder einen Rest der Formel -C(=O)-OR¹⁶ oder -C(=O)-NR¹⁷R¹⁸ bedeutet, worin
R¹⁶, R¹⁷ und R¹⁸ unabhängig voneinander Wasserstoff, (C₁-C₄)-Alkyl oder (C₃-C₆)-Cycloalkyl darstellen
oder
R¹⁷ und R¹⁸ zusammen mit dem Stickstoffatom, an das sie gebunden sind, einen 4- bis 6-gliedrigen Heterocyclus bilden, welcher ein O-Atom als weiteres Heteroatom enthalten kann,
oder
R^{6A} und R^{6B} miteinander verknüpft sind und zusammen eine -(CH₂)ᵣ-Brücke bilden, worin
r die Zahl 2, 3, 4 oder 5 bedeutet
und eine CH₂-Gruppe der Brücke gegen -O- ausgetauscht sein kann,
R^{7A} Wasserstoff, Fluor oder Methyl bedeutet,
R^{7B} Wasserstoff, Fluor, Methyl oder einen Rest der Formel -C(=O)-OR²³ oder -C(=O)-NR²⁴R²⁵ bedeutet, worin
R²³, R²⁴ und R²⁵ unabhängig voneinander Wasserstoff, (C₁-C₄)-Alkyl oder (C₃-C₆)-Cycloalkyl darstellen
oder
R²⁴ und R²⁵ zusammen mit dem Stickstoffatom, an das sie gebunden sind,
einen 4- bis 6-gliedrigen Heterocyclus bilden, welcher ein O-Atom als weiteres Heteroatom enthalten kann,
oder
R^{7A} und R^{7B} zusammen eine Oxo-Gruppe bilden
oder
R^{7A} und R^{7B} miteinander verknüpft sind und zusammen eine -(CH₂)ₛ-Brücke bilden, worin
s die Zahl 2, 3, 4 oder 5 bedeutet
und eine CH₂-Gruppe der Brücke gegen -O- ausgetauscht sein kann,
oder
L² für eine Gruppe der Formel 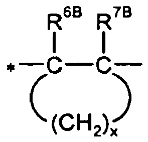 steht, worin
* die Verknüpfungsstelle mit dem N-Atom der Amid-Gruppierung bedeutet,
x die Zahl 1, 2, 3 oder 4 bedeutet,
wobei eine CH₂-Gruppe des Rings gegen -O- ausgetauscht sein kann,
und
R^{6B} und R^{7B} jeweils die oben angegebenen Bedeutungen haben, und
R³ für Phenyl, Naphthyl, Pyridyl, Chinolinyl oder Isochinolinyl steht, welche jeweils ein- bis zweifach, gleich oder verschieden, mit Resten ausgewählt aus der Reihe Fluor, Chlor, Cyano, (C₁-C₄)-Alkyl, Trifluormethyl, (C₁-C₄)-Alkoxy, Trifluormethoxy, (C₁-C₄)-Alkylthio und Phenyl substituiert sein können,
wobei der letztgenannte Phenyl-Rest seinerseits bis zu zweifach, gleich oder verschieden, mit Fluor, Chlor, Cyano, (C₁-C₄)-Alkyl, (C₁-C₄)-Alkoxy, Trifluormethyl und/oder Trifluormethoxy substituiert sein kann,
sowie ihre Salze, Solvate und Solvate der Salze.

6. Verbindung der Formel (I) nach einem der Ansprüche 1 bis 5, in welcher
A für N oder CH steht,
R¹ für (C₁-C₆)-Alkyl steht, welches ein- bis zweifach, gleich oder verschieden, mit Resten ausgewählt aus der Reihe Fluor, Oxo, Hydroxy, (C₁-C₄)-Alkoxy, Trifluormethyl, (C₃-C₆)-Cycloalkyl und Phenyl substituiert sein kann,
wobei Phenyl seinerseits bis zu zweifach, gleich oder verschieden, mit Resten ausgewählt aus der Reihe Fluor, Chlor, Cyano, (C₁-C₄)-Alkyl, Trifluormethyl, Hydroxymethyl, (C₁-C₄)-Alkoxy, Trifluormethoxy, Hydroxycarbonyl, Aminocarbonyl und Di-(C₁-C₄)-alkylaminocarbonyl substituiert sein kann,
oder
R¹ für (C₂-C₄)-Alkenyl oder (C₃-C₆)-Cycloalkyl steht,
R² für Phenyl oder Thienyl steht, welche ein- bis zweifach, gleich oder verschieden, mit Resten ausgewählt aus der Reihe Fluor, Chlor, Brom, (C₁-C₄)-Alkyl und (C₁-C₄)-Alkoxy substituiert sein können,
L¹ für -CH₂-, -CH(CH₃)- oder -CH₂CH₂- steht,
L² für eine Gruppe der Formel *-CR^{6A}R^{6B}-(CR^{7A}R^{7B})_{q}- steht, worin
* die Verknüpfungsstelle mit dem N-Atom der Amid-Gruppierung bedeutet,
q die Zahl 0 oder 1 bedeutet,
R^{6A} Wasserstoff oder Methyl bedeutet,
R^{6B} Wasserstoff, Methyl, Trifluormethyl oder einen Rest der Formel -C(=O)-NR¹⁷R¹⁸ bedeutet, worin
R¹⁷ und R¹⁸ unabhängig voneinander Wasserstoff, (C₁-C₄)-Alkyl oder (C₃-C₆)-Cycloalkyl darstellen
oder
R¹⁷ und R¹⁸ zusammen mit dem Stickstoffatom, an das sie gebunden sind, einen 4- bis 6-gliedrigen Heterocyclus bilden, welcher ein O-Atom als weiteres Heteroatom enthalten kann,
oder
R^{6A} und R^{6B} miteinander verknüpft sind und zusammen eine -(CH₂)ᵣ-Brücke bilden, worin
r die Zahl 2, 3, 4 oder 5 bedeutet
und eine CH₂-Gruppe der Brücke gegen -O- ausgetauscht sein kann,
R^{7A} Wasserstoff, Fluor oder Methyl bedeutet,
R^{7B} Wasserstoff, Fluor, Methyl oder einen Rest der Formel -C(=O)-OR²³ oder -C(=O)-NR²⁴R²⁵ bedeutet, worin
R²³, R²⁴ und R²⁵ unabhängig voneinander Wasserstoff oder (C₁-C₄)-Alkyl darstellen
oder
R²⁴ und R²⁵ zusammen mit dem Stickstoffatom, an das sie gebunden sind, einen 4- bis 6-gliedrigen Heterocyclus bilden, welcher ein O-Atom als weiteres Heteroatom enthalten kann,
oder
R^{7A} und R^{7B} zusammen eine Oxo-Gruppe bilden
oder
R^{7A} und R^{7B} miteinander verknüpft sind und zusammen eine -(CH₂)ₛ-Brücke bilden, worin
s die Zahl 2, 3, 4 oder 5 bedeutet
und eine CH₂-Gruppe der Brücke gegen -O- ausgetauscht sein kann,
oder
L² für eine Gruppe der Formel 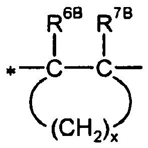 steht, worin
* die Verknüpfungsstelle mit dem N-Atom der Amid-Gruppierung bedeutet,
x die Zahl 1, 2, 3 oder 4 bedeutet
und
R^{6B} und R^{7B} jeweils die oben angegebenen Bedeutungen haben,
und
R³ für Phenyl oder Pyridyl, welche ein- bis zweifach, gleich oder verschieden, mit Resten ausgewählt aus der Reihe Fluor, Chlor, (C₁-C₄)-Alkyl, Trifluormethyl, (C₁-C₄)-Alkoxy und Trifluormethoxy substituiert sein können, oder für Naphthyl steht,
sowie ihre Salze, Solvate und Solvate der Salze.

7. Verbindung der Formel (I) nach einem der Ansprüche 1 bis 6, in welcher
A für N oder CH steht,
R¹ für (C₁-C₆)-Alkyl steht, welches ein- bis zweifach, gleich oder verschieden, mit Resten ausgewählt aus der Reihe Fluor, Oxo, Hydroxy, Methoxy, Ethoxy, Trifluormethyl, Cyclopropyl und Phenyl substituiert sein kann,
wobei Phenyl seinerseits bis zu zweifach, gleich oder verschieden, mit Resten ausgewählt aus der Reihe Fluor, Chlor, Cyano, Methyl, Hydroxymethyl, Methoxy, Hydroxycarbonyl, Aminocarbonyl und Dimethylaminocarbonyl substituiert sein kann,
oder
R¹ für Vinyl, Allyl oder Cyclopropyl steht,
R² für Phenyl oder Thienyl steht, welche ein- bis zweifach, gleich oder verschieden, mit Resten ausgewählt aus der Reihe Fluor, Chlor, Methyl und Methoxy substituiert sein können,
L¹ für -CH₂- steht,
L² für eine Gruppe der Formel *-CR^{6A}R^{6B}-(CR^{7A}R^{7B})_{q}- steht, worin
* die Verknüpfungsstelle mit dem N-Atom der Amid-Gruppierung bedeutet,
q die Zahl 0 oder 1 bedeutet,
R^{6A} Wasserstoff oder Methyl bedeutet,
R^{6B} Wasserstoff, Methyl, Trifluormethyl oder einen Rest der Formel -C(=O)-NR¹⁷R¹⁸ bedeutet, worin
R¹⁷ und R¹⁸ unabhängig voneinander Wasserstoff, Methyl, Ethyl oder Cyclopropyl darstellen
oder
R¹⁷ und R¹⁸ zusammen mit dem Stickstoffatom, an das sie gebunden sind, einen Azetidin-, Pyrrolidin-, Piperidin- oder Morpholin-Ring bilden,
oder
R^{6A} und R^{6B} miteinander verknüpft sind und zusammen mit dem Kohlenstoffatom, an das sie gebunden sind, eine Gruppe der Formel 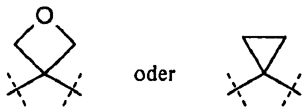 bilden,
R^{7A} Wasserstoff, Fluor oder Methyl bedeutet,
R^{7B} Wasserstoff, Fluor, Methyl oder einen Rest der Formel -C(=O)-OR²³ oder -C(=O)-NR²⁴R²⁵ bedeutet, worin
R²³, R²⁴ und R²⁵ unabhängig voneinander Wasserstoff, Methyl oder Ethyl darstellen
oder
R²⁴ und R²⁵ zusammen mit dem Stickstoffatom, an das sie gebunden sind, einen Azetidin-, Pyrrolidin-, Piperidin- oder Morpholin-Ring bilden,
oder
R^{7A} und R^{7B} zusammen eine Oxo-Gruppe bilden
oder
R^{7A} und R^{7B} miteinander verknüpft sind und zusammen eine -(CH₂)ₛ-Brücke bilden, worin
s die Zahl 2, 3, 4 oder 5 bedeutet
und eine CH₂-Gruppe der Brücke gegen -O- ausgetauscht sein kann,
oder
L² für eine Gruppe der Formel 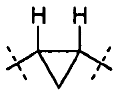 steht,
und
R³ für Phenyl, das ein- oder zweifach, gleich oder verschieden, mit Fluor, Chlor, Trifluormethyl und/oder Trifluormethoxy substituiert sein kann, oder für 1-Naphthyl steht,
sowie ihre Salze, Solvate und Solvate der Salze.

8. Verbindung der Formel (I) nach einem der Ansprüche 1 bis 6, in welcher
A für N oder CH steht,
R¹ für (C₁-C₄)-Alkyl, 2-Methoxyethyl, Cyclopropyl, Cyclohexylmethyl, Benzyl oder 1-Phenethyl steht,
wobei der Phenylring in den genannten Benzyl- und 1-Phenethyl-Resten mit Fluor, Chlor, Methyl, Trifluormethyl, Methoxy oder Trifluormethoxy substituiert sein kann,
R² für Phenyl oder Thienyl, welche jeweils ein- oder zweifach, gleich oder verschieden, mit Fluor, Chlor, Brom, Methyl und/oder Methoxy substituiert sind, steht,
L¹ für -CH₂-, -CH₂CH₂- oder -CH(CH₃)- steht,
L² für -CH₂-, -CH(CH₃)- oder -C(CH₃)₂- steht
und
R³ für Phenyl, das ein- oder zweifach, gleich oder verschieden, mit Fluor, Chlor, Trifluormethyl und/oder Trifluormethoxy substituiert ist, oder für 1-Naphthyl steht, sowie ihre Salze, Solvate und Solvate der Salze.

9. Verfahren zur Herstellung von Verbindungen der Formel (I), wie in den Ansprüchen 1 bis 8 definiert, **dadurch gekennzeichnet, dass** man
[A] eine Verbindung der Formel (II) 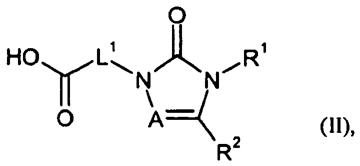 in welcher A, L¹, R¹ und R² jeweils die in den Ansprüchen 1 bis 8 angegebenen Bedeutungen haben,
in einem inerten Lösungsmittel unter Aktivierung der Carbonsäure-Funktion mit einer Verbindung der Formel (III) 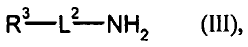 in welcher L² und R³ die in den Ansprüchen 1 bis 8 angegebenen Bedeutungen haben,
kuppelt
oder
[B] eine Verbindung der Formel (IV) 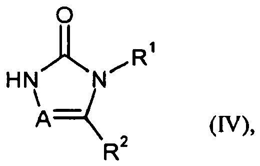 in welcher A, R¹ und R² jeweils die in den Ansprüchen 1 bis 8 angegebenen Bedeutungen haben,
in einem inerten Lösungsmittel in Gegenwart einer Base mit einer Verbindung der Formel (V) 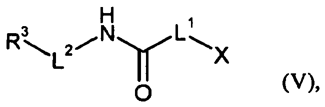 in welcher L¹ L² und R³ jeweils die in den Ansprüchen 1 bis 8 angegebenen Bedeutungen haben
und
X für eine Abgangsgruppe, wie beispielsweise Halogen, Mesylat oder
Tosylat, steht,
umsetzt
und die resultierenden Verbindungen der Formel (I) gegebenenfalls mit den entsprechenden (i) Lösungsmitteln und/oder (ii) Basen oder Säuren in ihre Solvate, Salze und/oder Solvate der Salze überführt.

10. Verbindung der Formel (I), wie in einem der Ansprüche 1 bis 8 definiert, zur Behandlung und/oder Prophylaxe von Krankheiten.

11. Verwendung einer Verbindung der Formel (I), wie in einem der Ansprüche 1 bis 8 definiert, zur Herstellung eines Arzneimittels zur Behandlung und/oder Prophylaxe von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

12. Arzneimittel enthaltend eine Verbindung der Formel (I), wie in einem der Ansprüche 1 bis 8 definiert, in Kombination mit einem inerten, nicht-toxischen, pharmazeutisch geeigneten Hilfsstoff.

13. Arzneimittel enthaltend eine Verbindung der Formel (I), wie in einem der Ansprüche 1 bis 8 definiert, in Kombination mit einem oder mehreren weiteren Wirkstoffen ausgewählt aus der Gruppe bestehend aus Diuretika, Angiotensin AII-Antagonisten, ACE-Hemmer, beta-Rezeptoren-Blocker, Mineralocorticoid-Rezeptor-Antagonisten, organische Nitrate, NO-Donatoren und positiv-inotrop wirksame Substanzen.

14. Arzneimittel nach Anspruch 12 oder 13 zur Behandlung und/oder Prophylaxe von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

## Claims

1. Compound of the formula (I) 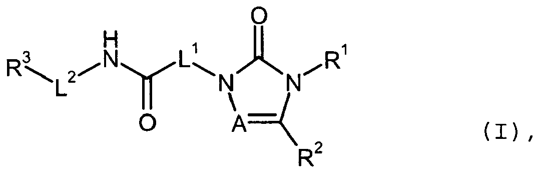 in which
A stands for N or C-R⁴, wherein
R⁴ means hydrogen or (C₁-C₄) alkyl,
R¹ stands for (C₁-C₆) alkyl, (C₂-C₆) alkenyl or (C₂-C₆) alkynyl, which can each be singly to triply, similarly or differently, substituted with residues selected from the range halogen, cyano, oxo, trifluoromethyl, (C₃-C₇) cycloalkyl, phenyl, -OR¹⁰, -NR¹¹R¹², -C(=O)-OR¹³ and -C(=O)-NR¹⁴R¹⁵, wherein
(*i*) (C₃-C₇) cycloalkyl can be up to doubly, similarly or differently, substituted with (C₁-C₄) alkyl, oxo, hydroxy, (C₁-C₄) alkoxy and/or amino,
(*ii*) phenyl can be up to triply, similarly or differently, substituted with residues selected from the range halogen, cyano, nitro, (C₁-C₄) alkyl, trifluoromethyl, hydroxy, hydroxymethyl, (C₁-C₄) alkoxy, trifluoromethoxy, (C₁-C₄) alkoxymethyl, hydroxycarbonyl, (C₁-C₄) alkoxycarbonyl, aminocarbonyl, mono-(C₁-C₄) alkylaminocarbonyl and di-(C₁-C₄) alkylaminocarbonyl,
(*iii*) R¹⁰, R¹¹, R¹², R¹³, R¹⁴ and R¹⁵ mutually independently on each single occurrence mean hydrogen, (C₁-C₆) alkyl or (C₃-C₇) cycloalkyl, wherein
(C₁-C₆) alkyl can itself be up to doubly, similarly or differently, substituted with amino, hydroxy, (C₁-C₄) alkoxy, hydroxycarbonyl and/or (C₁-C₄) alkoxycarbonyl
and
(C₃-C₇) cycloalkyl can itself be up to doubly, similarly or differently, substituted with (C₁-C₄) alkyl, oxo, hydroxy, (C₁-C₄) alkoxy and/or amino,
and/or
(iv) R¹¹ and R¹² and also R¹⁴ and R¹⁵ respectively in pairs together with the nitrogen atom to which they are bound form a 4 to 7-membered heterocycle, which can contain a further hetero atom from the range N, O and S and be up to doubly, similarly or differently, substituted with (C₁-C₄) alkyl, oxo, hydroxy, (C₁-C₄) alkoxy and/or amino,
or
R¹ stands for (C₃-C₇) cycloalkyl, which can be up to doubly, similarly or differently, substituted with (C₁-C₄) alkyl, (C₁-C₄) alkoxy, hydroxy, amino and/or oxo,
R² stands for phenyl, naphthyl, thienyl, benzothienyl, furyl or benzofuryl, which can each be singly to triply, similarly or differently, substituted with residues selected from the range halogen, cyano, nitro, (C₁-C₄) alkyl, trifluoromethyl, hydroxy, (C₁-C₄) alkoxy, trifluoromethoxy and phenyl,
wherein the last-named phenyl residue can itself be up to doubly, similarly or differently, substituted with residues selected from the range halogen, cyano, nitro, (C₁-C₄) alkyl, trifluoromethyl, hydroxy, (C₁-C₄) alkoxy, trifluoromethoxy, hydroxy- (C₁-C₄) alkyl and (C₁-C₄) alkylthio,
L¹ stands for a group of the formula -(CR^{5A}R^{5B})ₘ-, wherein
m means the number 1, 2 or 3
and
R^{5A} and R^{5B} mutually independently mean hydrogen or (C₁-C₄) alkyl or
two residues R^{5A} and R^{5B} bound to the same carbon atom are linked to one another and together form a -(CH₂)ₙ bridge, wherein
n means the number 2, 3, 4 or 5, or, in the event that m stands for the number 2 or 3,
two residues bound to adjacent (1,2- or 1,3-) or non-adjacent (1,3-) carbon atoms R^{5A} and/or R^{5B} are linked to one another and together form a -(CH₂)ₚ bridge, wherein
p means the number 1, 2, 3 or 4,
where, in the event that the group -CR^{5A}R^{5B}- occurs several times, the individual meanings of R^{5A} and R^{5B} can in each case be the same or different,
or
L¹ stands for a group of the formula 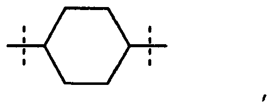
L² stands for a group of the formula *-CR^{6A}R^{6B}- (CR^{7A}R^{7B})_{q}- or *-CR^{6A}R^{6B}-CR^{7A}R^{7B}-O-, wherein
* means the binding site with the N atom of the amide group,
q means the number 0, 1 or 2,
R^{6A} means hydrogen or (C₁-C₄) alkyl,
R^{6B} means hydrogen, (C₁-C₄) alkyl, trifluoromethyl, (C₃-C₆) cycloalkyl or phenyl, which can be up to doubly, similarly or differently, substituted with halogen, (C₁-C₄) alkyl and/or trifluoromethyl, or a residue of the formula -C(=O)-OR¹⁶ or -C(=O)-NR¹⁷R¹⁸, wherein
R¹⁶, R¹⁷ and R¹⁸ mutually independently represent hydrogen, (C₁-C₄) alkyl or (C₃-C₆) cycloalkyl
or
R¹⁷ and R¹⁸ together with the nitrogen atom to which they are bound form a 4 to 6-membered heterocycle, which can contain a further hetero atom from the range N, O and S and be up. to doubly, similarly or differently, substituted with (C₁-C₄) alkyl, hydroxy and/ or (C₁-C₄) alkoxy,
or
R^{6A} and R^{6B} are linked to one another and together form a -(CH₂)ᵣ bridge, wherein
r means the number 2, 3, 4 or 5
and one CH₂ group of the bridge can be exchanged for -O-, -S- or >N-R¹⁹, wherein
R¹⁹ represents hydrogen or (C₁-C₄) alkyl,
R^{7A} means hydrogen, fluorine, (C₁-C₄) alkyl or (C₁-C₄) alkoxy,
R^{7B} means hydrogen, fluorine, (C₁-C₄) alkyl, hydroxy-(C₁-C₄) alkyl or a residue of the formula -OR²⁰, -NR²¹R²², -C(=O)-OR²³ or - C (=O) -NR²⁴R²⁵, wherein
R²⁰, R²¹, R²², R²³, R²⁴ and R²⁵ mutually independently represent hydrogen, (C₁-C₄) alkyl or (C₃-C₆) cycloalkyl
or
R²¹ and R²² and also R²⁴ and R²⁵ respectively in pairs together with the nitrogen atom to which they are bound form a 4 to 6-membered heterocycle, which can contain a further hetero atom from the range N, O and S and be up to doubly, similarly or differently, substituted with (C₁-C₄) alkyl, hydroxy and/or (C₁-C₄) alkoxy,
or
R^{7A} and R^{7B} together form an oxo group
or
R^{7A} and R^{7B} are linked to one another and together form a -(CH₂)ₛ bridge, wherein
s means the number 2, 3, 4 or 5
and one CH₂ group of the bridge can be exchanged for -O-, -S- or >N-R²⁶, wherein
R²⁶ represents hydrogen or (C₁-C₄) alkyl,
where, in the event that the group -CR^{7A}R^{7B}- occurs several times, the individual meanings of R^{7A} and R^{7B} can in each case be the same or different,
or
L² stands for a group of the formula 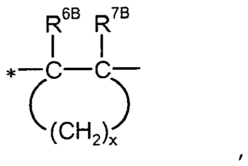 wherein
* means the binding site with the N atom of the amide group,
x means the number 1, 2, 3 or 4,
where one CH₂ group of the ring can be exchanged for -O-, -S- or >N-R²⁷, wherein
R²⁷ represents hydrogen or (C₁-C₄) alkyl,
and
R^{6B} and R^{7B} each have the aforesaid meanings,
R³ stands for phenyl, naphthyl or 5 to 10-membered heteroaryl with up to three hetero atoms from the range N, O and/or S, which can each be singly to triply, similarly or differently, substituted with residues selected from the range halogen, cyano, nitro, (C₁-C₄) alkyl, trifluoromethyl, hydroxy, (C₁-C₄) alkoxy, trifluoromethoxy, (C₁-C₄) alkylthio, (C₁-C₄) alkylsulfinyl, (C₁-C₄) alkylsulphonyl, di-(C₁-C₄) alkylamino and phenyl,
wherein the last-named phenyl residue can itself be up to doubly, similarly or differently, substituted with halogen, cyano, nitro, (C₁-C₄) alkyl, (C₁-C₄) alkoxy, trifluoromethyl and/or trifluoromethoxy,
or the grouping
L²-R³ together forms a group of the formula 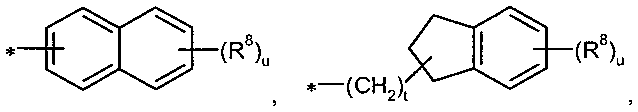 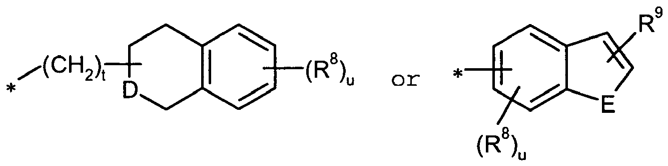 wherein
* means the binding site with the N atom of the amide group,
D means CH₂ or 0,
E means NH, N-CH₃, O or S,
t means the number 0 or 1,
R⁸ means a substituent selected from the range halogen, cyano, nitro, (C₁-C₄) alkyl, (C₁-C₄) alkoxy, trifluoromethyl and trifluoromethoxy,
u means the number 0, 1 or 2,
where, in the event that the substituent R⁸ occurs several times, its meanings can be the same or different,
and
R⁹ means hydrogen or (C₁-C₄) alkyl,
and salts, solvates and solvates of the salts thereof.

2. Compound of the formula (I) according to Claim 1, in which
A stands for N or C-R⁴, wherein
R⁴ means hydrogen or (C₁-C4) alkyl,
R¹ stands for (C₁-C₆) alkyl, which can be substituted with hydroxy, (C₁-C₆) alkoxy, (C₃-C₇) cycloalkyl or phenyl, or for (C₃-C₇) cycloalkyl,
wherein the said cycloalkyl residues can themselves be up to doubly, similarly or differently, substituted with (C₁-C₄) alkyl, (C₁-C₄) alkoxy, hydroxy, amino and/or oxo
and
the phenyl residue up to triply, similarly or differently, with halogen, cyano, nitro, (C₁-C₄) alkyl, (C₁-C₄) alkoxy, trifluoromethyl and/or trifluoromethoxy,
R² stands for phenyl, naphthyl, thienyl, benzothienyl, furyl or benzofuryl, which can each be substituted up to triply, similarly or differently, with halogen, cyano, nitro, (C₁-C₄) alkyl, (C₁-C₄) alkoxy, trifluoromethyl, trifluoromethoxy and/or phenyl,
wherein the last-named phenyl residue can itself be up to doubly, similarly or differently, substituted with halogen, cyano, nitro, (C₁-C₄) alkyl, (C₁-C₄) alkoxy, trifluoromethyl and/or trifluoromethoxy,
L¹ stands for a group of the formula -(CR^{5A}R^{5B})ₘ-, wherein
m means the number 1, 2 or 3
and
R^{5A} and R^{5B} mutually independently mean hydrogen or (C₁-C₄) alkyl
or
two residues R^{5A} and R^{5B} bound to the same carbon atom are linked to one another and together form a - (CH₂)ₙ bridge, wherein
n means the number 2, 3, 4 or 5,
or, in the event that m stands for the number 2 or 3,
two residues bound to adjacent (1,2- or 2,3-) or non-adjacent (1,3-) carbon atoms R^{5A} and/or R^{5B} are linked to one another and together form a -(CH₂)ₚ bridge, wherein
p means the number 1, 2, 3 or 4,
where, in the event that the group -CR^{5A}R^{5B}- occurs several times, the individual meanings of R^{5A} and R^{5B} can in each case be the same or different,
or
L¹ stands for a group of the formula 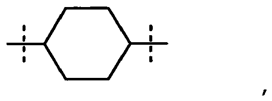
L² stands for a group of the formula *-CR^{6A}R^{6B}- (CR^{7A}R^{7B})_{q} or *-CR^{6A}R^{6B}-CR^{7A}R^{7B}-O-, wherein
* means the binding site with the N atom of the amide group,
q means the number 0, 1 or 2,
R^{6A} means hydrogen or (C₁-C₄) alkyl,
R^{6B} means hydrogen, (C₁-C₄) alkyl, trifluoromethyl, (C₃-C₆) cycloalkyl or phenyl, which can be up to doubly, similarly or differently, substituted with halogen, (C₁-C₄) alkyl and/or trifluoromethyl
or
R^{6A} and R^{6B} are linked to one another and together form a -(CH₂)ᵣ bridge, wherein
r means the number 2, 3, 4 or 5,
and
R^{7A} and R^{7B} mutually independently mean hydrogen or (C₁-C₄) alkyl,
where, in the event that the group -CR^{7A}R^{7B}- occurs several times, the individual meanings of R^{7A} and R^{7B} can in each case be the same or different,
R³ stands for phenyl, naphthyl or 5 to 10-membered heteroaryl with up to three hetero atoms from the range N, O and/or S, which can each be substituted up to triply, similarly or differently, with halogen, cyano, nitro, (C₁-C₄) alkyl, (C₁-C₄) alkoxy, trifluoromethyl, trifluoromethoxy, di-(C₁-C₄) alkylamino and/or phenyl,
wherein the last-named phenyl residue can itself be up to doubly, similarly or differently, substituted with halogen, cyano, nitro, (C₁-C₄) alkyl, (C₁-C₄) alkoxy, trifluoromethyl and/or trifluoromethoxy,
or the grouping
L²-R³ together forms a group of the formula 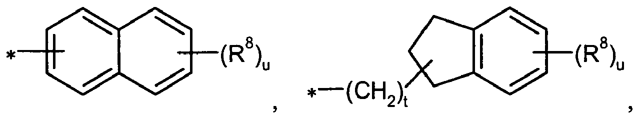 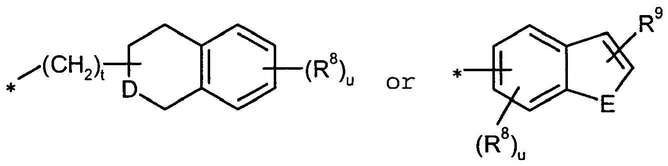 wherein
* means the binding site with the N atom of the amide group,
D means CH₂ or O,
E means NH, N-CH₃, O or S,
t means the number 0 or 1,
R⁸ means a substituent selected from the range halogen, cyano, nitro, (C₁-C₄) alkyl, (C₁-C₄) alkoxy, trifluoromethyl and trifluoromethoxy,
u means the number 0, 1 or 2,
where, in the event that the substituent R⁸ occurs several times, its meanings can be the same or different,
and
R⁹ means hydrogen or (C₁-C₄) alkyl,
and salts, solvates and solvates of the salts thereof.

3. Compound of the formula (I) according to Claim 1, in which
A stands for N or C-R⁴, wherein
R⁴ means hydrogen or (C₁-C₄) alkyl,
R¹ stands for (C₁-C₆) alkyl, which can be singly to triply, similarly or differently, substituted with residues selected from the range fluorine, chlorine, cyano, oxo, trifluoromethyl, (C₃-C₆) cycloalkyl, phenyl, - OR¹⁰, -NR¹¹R¹², -C(=O) -OR¹³ and -C (=O) -NR¹⁴R¹⁵, wherein
(*i*) (C₃-C₆) cycloalkyl can be up to doubly, similarly or differently, substituted with (C₁-C₄) alkyl, oxo, hydroxy, (C₁-C₄) alkoxy and/or amino,
(*ii*) phenyl can be up to triply, similarly or differently, substituted with residues selected from the range fluorine, chlorine, cyano, (C₁-C₄) alkyl, trifluoromethyl, hydroxy, hydroxymethyl, (C₁-C₄) alkoxy, trifluoromethoxy, (C₁-C₄) alkoxymethyl, hydroxycarbonyl, (C₁-C₄) alkoxy-carbonyl, aminocarbonyl, mono-(C₁-C₄) alkylaminocarbonyl and di-(C₁-C₄) alkylaminocarbonyl,
(*iii*) R¹⁰, R¹¹, R¹², R¹³, R¹⁴ and R¹⁵ mutually independently on each single occurrence mean hydrogen, (C₁-C₄) alkyl or (C₃-C₆) cycloalkyl, wherein
(C₁-C₄) alkyl can itself be up to doubly, similarly or differently, substituted with amino, hydroxy, (C₁-C₄) alkoxy, hydroxycarbonyl and/or (C₁-C₄) alkoxy-carbonyl
and
(C₃-C₆) cycloalkyl can itself be up to doubly, similarly or differently, substituted with (C₁-C₄) alkyl, oxo, hydroxy, (C₁-C₄) alkoxy and/or amino,
and/or
(*iv*) R¹¹ and R¹² and also R¹⁴ and R¹⁵ respectively in pairs together with the nitrogen atom to which they are bound form a 4 to 6-membered heterocycle, which can contain a further hetero atom from the range N and O and be up to doubly, similarly or differently, substituted with (C₁-C₄) alkyl, oxo, hydroxy, (C₁-C₄) alkoxy and/or amino,
or
R¹ stands for (C₂-C₆) alkenyl, which can be substituted with hydroxy, (C₁-C₄) alkoxy, hydroxycarbonyl or (C₁-C₄) alkoxycarbonyl,
or
for (C₃-C₆) cycloalkyl, which can be up to doubly, similarly or differently, substituted with (C₁-C₄) alkyl, (C₁-C₄) alkoxy, hydroxy, amino and/or oxo,
R² stands for phenyl or thienyl, which can be singly to triply, similarly or differently, substituted with residues selected from the range fluorine, chlorine, cyano, (C₁-C₄) alkyl, trifluoromethyl, hydroxy, (C₁-C₄) alkoxy, trifluoromethoxy and phenyl,
wherein the last-named phenyl residue can itself be up to doubly, similarly or differently, substituted with residues selected from the range fluorine, chlorine, cyano, (C₁-C₄) alkyl, trifluoromethyl, (C₁-C₄) alkoxy and trifluoromethoxy,
L¹ stands for a group of the formula -CR^{5A}R^{5B}-, wherein
R^{5A} and R^{5B} mutually independently mean hydrogen or (C₁-C₄) alkyl,
L² stands for a group of the formula *-CR^{6A}R^{6B}-(CR^{7A}R^{7B})_{q}-, wherein
* means the binding site with the N atom of the amide group,
q means the number 0 or 1,
R^{6A} means hydrogen or (C₁-C₄) alkyl,
R^{6B} means hydrogen, (C₁-C₄) alkyl, trifluoromethyl, (C₃-C₆) cycloalkyl or phenyl, which can be up to doubly, similarly or differently, substituted with fluorine, chlorine, (C₁-C₄) alkyl and/or trifluoromethyl, or a residue of the formula -C(=O)-OR¹⁶ or -C(=O)-NR¹⁷R¹⁸, wherein
R¹⁶, R¹⁷ and R¹⁸ mutually independently represent hydrogen, (C₁-C₄) alkyl or (C₃-C₆) cycloalkyl
or
R¹⁷ and R¹⁸ together with the nitrogen atom to which they are bound form a 4 to 6-membered heterocycle, which can contain a further hetero atom from the range N and O and be up to doubly, similarly or differently, substituted with (C₁-C₄) alkyl, hydroxy and/or (C₁-C₄) alkoxy,
or
R^{6A} and R^{6B} are linked to one another and together form a -(CH₂)ᵣ bridge, wherein
r means the number 2, 3, 4 or 5
and one CH₂ group of the bridge can be exchanged for -O-,
R^{7A} means hydrogen, fluorine or (C₁-C₄),
R^{7B} means hydrogen, fluorine, (C₁-C₄) alkyl, hydroxy-(C₁-C₄) alkyl or a residue of the formula -OR²⁰, -NR²¹R²², -C(=O)-OR²³ or - C (=O) -NR²⁴R²⁵, wherein
R²⁰, R²¹, R²², R²³, R²⁴ and R²⁵ mutually independently represent hydrogen, (C₁-C₄) alkyl or (C₃-C₆) cycloalkyl
or
R²¹ and R²² and also R²⁴ and R²⁵ respectively in pairs together with the nitrogen atom to which they are bound form a 4 to 6-membered heterocycle, which can contain a further hetero atom from the range N and O and be up to doubly, similarly or differently, substituted with (C₁-C4) alkyl, hydroxy and/or (C₁-C₄) alkoxy,
or
R^{7A} and R^{7B} together form an oxo group
or
R^{7A} and R^{7B} are linked to one another and together form a -(CH₂)ₛ bridge, wherein
s means the number 2, 3, 4 or 5
and one CH₂ group of the bridge can be exchanged for -O-,
or
L² stands for a group of the formula 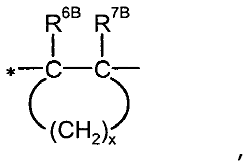 wherein
* means the binding site with the N atom of the amide group,
x means the number 1, 2, 3 or 4,
where one CH₂ group of the ring can be exchanged for -0-,
and
R^{6B} and R^{7B} each have the aforesaid meanings,
and
R³ stands for phenyl, naphthyl or 5 to 10-membered heteroaryl with up to two hetero atoms from the range N, O and/or S, which can each be singly to triply, similarly or differently, substituted with residues selected from the range fluorine, chlorine, cyano, (C₁-C₄) alkyl, trifluoromethyl, hydroxy, (C₁-C₄) alkoxy, trifluoromethoxy, (C₁-C₄) alkylthio, (C₁-C₄) alkylsulphonyl, di-(C₁-C₄) alkylamino and phenyl,
wherein the last-named phenyl residue can itself be up to doubly, similarly or differently, substituted with fluorine, chlorine, cyano, (C₁-C₄) alkyl, (C₁-C₄) alkoxy, trifluoromethyl and/or trifluoromethoxy,
and salts, solvates and solvates of the salts thereof.

4. Compound of the formula (I) according to Claim 1 or 2, in which
A stands for N or C-R⁴, wherein
R⁴ means hydrogen or (C₁-C₄) alkyl,
R¹ stands for (C₁-C₆) alkyl, which can be substituted with hydroxy, (C₁-C₄) alkoxy, (C₃-C₆) cycloalkyl or phenyl, or for (C₃-C₆) cycloalkyl,
wherein the said cycloalkyl residues can themselves be up to doubly, similarly or differently, substituted with (C₁-C₄) alkyl, (C₁-C₄) alkoxy, hydroxy and/or amino
and
the phenyl residue can be up to doubly, similarly or differently, substituted with halogen, cyano, nitro, (C₁-C₄) alkyl, (C₁-C₄) alkoxy, trifluoromethyl and/or trifluoromethoxy,
R² stands for phenyl, naphthyl, thienyl or benzothienyl, which can each be up to triply, similarly or differently, substituted with halogen, cyano, nitro, (C₁-C₄) alkyl, (C₁-C₄) alkoxy, trifluoromethyl, trifluoromethoxy and/or phenyl,
wherein the last-named phenyl residue can itself be up to doubly, similarly or differently, substituted with halogen, cyano, nitro, (C₁-C₄) alkyl, (C₁-C₄) alkoxy, trifluoromethyl and/or trifluoromethoxy,
L¹ stands for a group of the formula -(CR^{5A}R^{5B})ₘ-, wherein
m means the number 1, 2 or 3
and
R^{5A} and R^{5B} mutually independently mean hydrogen or methyl
or
two residues R^{5A} and R^{5B} bound to the same carbon atom are linked to one another and together form a -(CH₂)ₙ bridge, wherein
n means the number 2, 3, 4 or 5,
or, in the event that m stands for the number 2 or 3,
two residues bound to adjacent (1,2- or 2,3-) or non-adjacent (1,3-) carbon atoms R^{5A} and/or R^{5B} are linked to one another and together form a - (CH₂)ₚ bridge, wherein
p means the number 1, 2, 3 or 4,
where, in the event that the group -CR^{5A}R^{5B}- occurs several times, the individual meanings of R^{5A} and R^{5B} can in each case be the same or different,
or
L¹ stands for a group of the formula 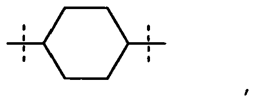
L² stands for a group of the formula *-CR⁶AR^{6B}-(CH₂)q-, wherein
* means the binding site with the N atom of the amide group,
q means the number 0 or 1,
R^{6A} means hydrogen or methyl,
R^{6B} means hydrogen, (C₁-C₄) alkyl, trifluoromethyl or (C₃-C₆) cycloalkyl
or
R^{6A} and R^{6B} are linked to one another and together form a -(CH₂)ᵣ bridge, wherein
r means the number 2, 3, 4 or 5,
and
R³ stands for phenyl, naphthyl or 5 to 10-membered heteroaryl with up to two hetero atoms from the range N, O and/or S, which can each be substituted up to triply, similarly or differently, with halogen, cyano, nitro, (C₁-C₄) alkyl, (C₁-C₄) alkoxy, trifluoromethyl, trifluoromethoxy and/or phenyl,
wherein the last-named phenyl residue can itself be up to doubly, similarly or differently, substituted with halogen, cyano, nitro, (C₁-C₄) alkyl, (C₁-C₄) alkoxy, trifluoromethyl and/or trifluoromethoxy,
and salts, solvates and solvates of the salts thereof.

5. Compound of the formula (I) according to Claim 1 or 3, in which
A stands for N or C-R⁴, wherein
R⁴ means hydrogen or methyl,
R¹ stands for (C₁-C₆) alkyl, which can be singly or doubly, similarly or differently, substituted with residues selected from the range fluorine, oxo, trifluoromethyl, (C₃-C₆) cycloalkyl, phenyl, -OR¹⁰, -C(=O)-OR¹³ and - C (=O) -NR¹⁴R¹⁵, wherein
(i) (C₃-C₆) cycloalkyl can be up to doubly, similarly or differently, substituted with (C₁-C₄) alkyl, hydroxy and/or (C₁-C₄) alkoxy,
(ii) phenyl can be up to doubly, similarly or differently, substituted with residues selected from the range fluorine, chlorine, cyano, (C₁-C₄) alkyl, trifluoromethyl, hydroxymethyl, (C₁-C₄) alkoxy, hydroxycarbonyl, (C₁-C₄) alkoxycarbonyl, aminocarbonyl, mono-(C₁-C₄) alkylaminocarbonyl and di-(C₁-C₄) alkylaminocarbonyl,
and
(iii) R¹⁰, R¹³, R¹⁴ and R¹⁵ mutually independently on each single occurrence mean hydrogen, (C₁-C₄) alkyl or (C₃-C₆) cycloalkyl, wherein
(C₁-C₄) alkyl can itself be up to doubly, similarly or differently, substituted with hydroxy, (C₁-C₄) alkoxy, hydroxycarbonyl and/or (C₁-C₄) alkoxy-carbonyl
and
(C₃-C₆) cycloalkyl can itself be up to doubly, similarly or differently, substituted with (C₁-C₄) alkyl, hydroxy and/or (C₁-C₄) alkoxy,
or
R¹ stands for (C₂-C₆) alkenyl, which can be substituted with hydroxycarbonyl or (C₁-C₄) alkoxycarbonyl,
or
for (C₃-C₆) cycloalkyl, which can be up to doubly, similarly or differently, substituted with (C₁-C₄) alkyl, (C₁-C₄) alkoxy and/or hydroxy,
R² stands for phenyl or thienyl, which can be singly to doubly, similarly or differently, substituted with residues selected from the range fluorine, chlorine, (C₁-C₄) alkyl, trifluoromethyl , (C₁-C₄) alkoxy and trifluoromethoxy,
L¹ stands for a group of the formula -CR^{5A}R^{5B-}, wherein
R^{5A} and R^{5B} mutually independently mean hydrogen or methyl,
L² stands for a group of the formula *-CR^{6A}R^{6B}-(CR^{7A}R^{7B})_{q}-, wherein
* means the binding site with the N atom of the amide group,
q means the number 0 or 1,
R^{6A} means hydrogen or methyl,
R^{6B} means hydrogen, methyl, trifluoromethyl or a residue of the formula -C (=O)-OR¹⁶ or -C (=O)-NR¹⁷R¹⁸, wherein
R¹⁶, R¹⁷ and R¹⁸ mutually independently represent hydrogen, (C₁-C₄) alkyl or (C₃-C₆) cycloalkyl
or
R¹⁷ and R¹⁸ together with the nitrogen atom to which they are bound form a 4 to 6-membered heterocycle, which can contain an O atom as a further hetero atom,
or
R^{6A} and R^{6B} are linked to one another and together form a -(CH₂)ᵣ bridge, wherein
r means the number 2, 3, 4 or 5
and one CH₂ group of the bridge can be exchanged for -O-,
R^{7A} means hydrogen, fluorine or methyl,
R^{7B} means hydrogen, fluorine, methyl or a residue of the formula -C(=O)-OR²³ or -C(=O)-NR²⁴R²⁵, wherein
R²³, R²⁴ and R²⁵ mutually independently represent hydrogen, (C₁-C₄) alkyl or (C₃-C₆) cycloalkyl
or
R²⁴ and R²⁵ together with the nitrogen atom to which they are bound form a 4 to 6-membered heterocycle, which can contain an O atom as a further hetero atom,
or
R^{7A} and R^{7B} together form an oxo group
or
R^{7A} and R^{7B} are linked to one another and together form a -(CH₂)ₛ bridge, wherein
s means the number 2, 3, 4 or 5
and one CH₂ group of the bridge can be exchanged for -O-,
or
L² stands for a group of the formula 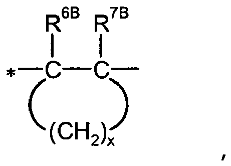 wherein
* means the binding site with the N atom of the amide group,
x means the number 1, 2, 3 or 4,
where one CH₂ group of the ring can be exchanged for -O-,
and
R^{6B} and R^{7B} each have the aforesaid meanings,
and
R³ stands for phenyl, naphthyl, pyridyl, quinolinyl or isoquinolinyl, which can each be singly to doubly, similarly or differently, substituted with residues selected from the range fluorine, chlorine, cyano, (C₁-C₄) alkyl, trifluoromethyl, (C₁-C₄) alkoxy, trifluoromethoxy, (C₁-C₄) alkylthio and phenyl,
wherein the last-named phenyl residue can itself be up to doubly, similarly or differently, substituted with fluorine, chlorine, cyano, (C₁-C₄) alkyl, (C₁-C₄) alkoxy, trifluoromethyl and/or trifluoromethoxy,
and salts, solvates and solvates of the salts thereof.

6. Compound of the formula (I) according to one of Claims 1 to 5, in which
A stands for N or CH,
R¹ stands for (C₁-C₆) alkyl, which can be singly or doubly, similarly or differently, substituted with residues selected from the range fluorine, oxo, hydroxy, (C₁-C₄) alkoxy, trifluoromethyl, (C₃-C₆) cycloalkyl and phenyl,
where phenyl can itself be up to doubly, similarly or differently, substituted with residues selected from the range fluorine, chlorine, cyano, (C₁-C₄) alkyl, trifluoromethyl, hydroxymethyl, (C₁-C₄) alkoxy, trifluoromethoxy, hydroxycarbonyl, aminocarbonyl and di-(C₁-C₄) alkylaminocarbonyl,
or
R¹ stands for (C₂-C₄) alkenyl or (C₃-C₆) cycloalkyl,
R² stands for phenyl or thienyl, which can be singly to doubly, similarly or differently, substituted with residues selected from the range fluorine, chlorine, bromine, (C₁-C₄) alkyl and (C₁-C₄) alkoxy,
L¹ stands for -CH₂-, -CH (CH₃) - or -CH₂CH₂-,
L² stands for a group of the formula *-CR^{6A}R^{6B}-(CR^{7A}R^{7B})_{q}-, wherein
* means the binding site with the N atom of the amide group,
q means the number 0 or 1,
R^{6A} means hydrogen or methyl,
R^{6B} means hydrogen, methyl, trifluoromethyl or a residue of the formula -C(=O)-NR¹⁷R¹⁸, wherein
R¹⁷ and R¹⁸ mutually independently represent hydrogen, (C₁-C₄) alkyl or (C₃-C₆) cycloalkyl
or
R¹⁷ and R¹⁸ together with the nitrogen atom to which they are bound form a 4 to 6-membered heterocycle, which can contain an O atom as a further hetero atom,
or
R^{6A} and R^{6B} are linked to one another and together form a -(CH₂)r bridge, wherein
r means the number 2, 3, 4 or 5
and one CH₂ group of the bridge can be exchanged for -O-,
R^{7A} means hydrogen, fluorine or methyl,
R^{7B} means hydrogen, fluorine, methyl or a residue of the formula -C(=O)-OR²³ or -C (=O) -NR²⁴R²⁵, wherein
R²³, R²⁴ and R²⁵ mutually independently represent hydrogen or (C₁-C₄) alkyl
or
R²⁴ and R²⁵ together with the nitrogen atom to which they are bound form a 4 to 6-membered heterocycle, which can contain an O atom as a further hetero atom,
or
R^{7A} and R^{7B} together form an oxo group
or
R^{7A} and R^{7B} are linked to one another and together form a -(CH₂) ₛ bridge, wherein
s means the number 2, 3, 4 or 5
and one CH₂ group of the bridge can be exchanged for -O-,
or
L² stands for a group of the formula 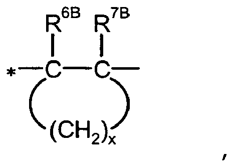 wherein
* means the binding site with the N atom of the amide group,
x means the number 1, 2, 3 or 4
and
R^{6B} and R^{7B} each have the aforesaid meanings,
and
R³ stands for phenyl or pyridyl, which can be singly to doubly, similarly or differently, substituted with residues selected from the range fluorine, chlorine, (C₁-C₄) alkyl, trifluoromethyl, (C₁-C₄) alkoxy and trifluoromethoxy, or for naphthyl,
and salts, solvates and solvates of the salts thereof.

7. Compound of the formula (I) according to one of Claims 1 to 6, in which
A stands for N or CH,
R¹ stands for (C₁-C₆) alkyl, which can be singly or doubly, similarly or differently,
substituted with residues selected from the range fluorine, oxo, hydroxy, methoxy, ethoxy, trifluoromethyl, cyclopropyl and phenyl,
where phenyl can itself be up to doubly, similarly or differently, substituted with residues selected from the range fluorine, chlorine, cyano, methyl, hydroxymethyl, methoxy, hydroxycarbonyl, aminocarbonyl and dimethylaminocarbonyl,
or
R¹ stands for vinyl, allyl or cyclopropyl,
R² stands for phenyl or thienyl, which can be singly to doubly, similarly or differently, substituted with residues selected from the range fluorine, chlorine, methyl and methoxy
L¹ stands for -CH₂-,
L² stands for a group of the formula *-CR^{6A}R^{6B}-(CR^{7A}R^{7B}) _{q}-, wherein
* means the binding site with the N atom of the amide group,
q means the number 0 or 1,
R^{6A} means hydrogen or methyl,
R^{6B} means hydrogen, methyl, trifluoromethyl or a residue of the formula -C(=O)-NR¹⁷R¹⁸, wherein
R¹⁷ and R¹⁸ mutually independently represent hydrogen, methyl, ethyl or cyclopropyl
or
R¹⁷ and R¹⁸ together with the nitrogen atom to which they are bound form an azetidine, pyrrolidine, piperidine or morpholine ring,
or
R^{6A} and R^{6B} are linked together and together with the carbon atom to which they are bound form a group of the formula 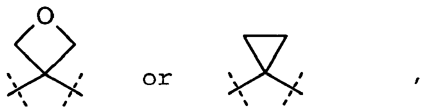
R^{7A} means hydrogen, fluorine or methyl,
R^{7B} means hydrogen, fluorine, methyl or a residue of the formula -C (=O) -OR²³ or -C (=O) -NR²⁴R²⁵ , wherein
R²³, R²⁴ and R²⁵ mutually independently represent hydrogen, methyl or ethyl
or
R²⁴ and R²⁵ together with the nitrogen atom to which they are bound form an azetidine, pyrrolidine, piperidine or morpholine ring,
or
R^{7A} and R^{7B} together form an oxo group
or
R^{7A} and R^{7B} are linked to one another and together form a -(CH₂)ₛ bridge, wherein
s means the number 2, 3, 4 or 5 and one CH₂ group of the bridge can be exchanged for -O-,
or
L² stands for a group of the formula 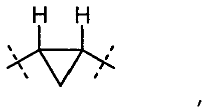 and
R³ stands for phenyl, which can be singly or doubly, similarly or differently, substituted with fluorine, chlorine, trifluoromethyl and/or trifluoromethoxy, or for 1-naphthyl,
and salts, solvates and solvates of the salts thereof.

8. Compound of the formula (I) according to one of Claims 1 to 6, in which
A stands for N or CH,
R¹ stands for (C₁-C₄) alkyl, 2-methoxyethyl, cyclopropyl, cyclohexylmethyl, benzyl or 1-phenethyl,
where the phenyl ring in the said benzyl- and 1-phenethyl residues can be substituted with fluorine, chlorine, methyl, trifluoromethyl, methoxy or trifluoromethoxy,
R² stands for phenyl or thienyl, which are each singly or doubly, similarly or differently, substituted with fluorine, chlorine, bromine, methyl and/or methoxy,
L¹ stands for -CH₂-, -CH₂CH₂- or -CH (CH₃)-,
L² stands for -CH₂-, -CH (CH₃)- or -C(CH₃)₂-
and
R³ stands for phenyl, which is singly or doubly, similarly or differently, substituted with fluorine, chlorine, trifluoromethyl and/or trifluoromethoxy, or for 1-naphthyl,
and salts, solvates and solvates of the salts thereof.

9. Process for the production of compounds of the formula (I), as defined in Claims 1 to 8, **characterized in that**
[A] a compound of the formula (II) 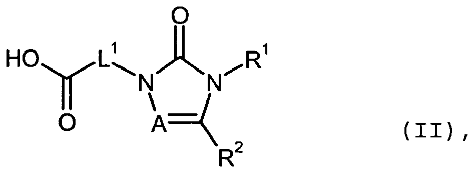 in which A, L¹, R¹ and R² each have the meanings stated in Claims 1 to 8,
is coupled with a compound of the formula (III) 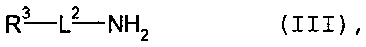 in which L² and R³ have the meanings stated in Claims 1 to 8, in an inert solvent with activation of the carboxylic acid function
or
[B] a compound of the formula (IV) 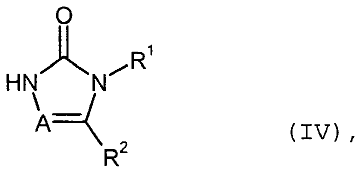 in which A, R¹ and R² each have the meanings stated in claims 1 to 8,
is reacted with a compound of the formula (V) 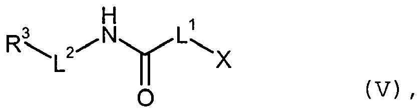 in which L¹, L² and R³ each have the meanings stated in Claims 1 to 8
and
X stands for a leaving group, such as for example halogen, mesylate or tosylate,
in an inert solvent in the presence of a base
and the resulting compounds of the formula (I) are optionally converted into their solvates, salts and/or solvates of the salts with the appropriate (i) solvents and/or (ii) bases or acids.

10. Compound of the formula (I), as defined in one of Claims 1 to 8, for the treatment and/or prophylaxis of diseases.

11. Use of a compound of the formula (I), as defined in one of Claims 1 to 8, for the production of a medicament for the treatment and/or prophylaxis of acute and chronic cardiac insufficiency, hypervolaemic and euvolaemic hyponatraemia, liver cirrhosis, ascites, oedema and syndrome of inappropriate ADH secretion (SIADH).

12. Medicament containing a compound of the formula (I), as defined in one of Claims 1 to 8, in combination with an inert, non-toxic, pharmaceutically suitable additive.

13. Medicament containing a compound of the formula (I), as defined in one of Claims 1 to 8, in combination with one or more further active substances selected from the group consisting of diuretics, angiotensin AII antagonists, ACE inhibitors, beta receptor blockers, mineralocorticoid receptor antagonists, organic nitrates, NO donors and substances with positive inotropic action.

14. Medicament according to Claim 12 or 13 for the treatment and/or prophylaxis of acute and chronic cardiac insufficiency, hypervolaemic and euvolaemic hyponatraemia, liver cirrhosis, ascites, oedema and syndrome of inappropriate ADH secretion (SIADH).

## Revendications

1. Composé de formule (I) 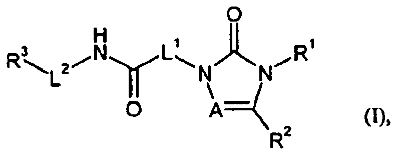 dans laquelle
A représente N ou C-R⁴, où R⁴ signifie hydrogène ou (C₁-C₄) -alkyle,
R¹ représente (C₁-C₆) -alkyle, (C₂-C₆)-alcényle ou (C₂-C₆)-alcynyle, qui peuvent à chaque fois être monosubstitués à trisubstitués, de manière identique ou différente, par des radicaux choisis dans la série halogène, cyano, oxo, trifluorométhyle, (C₃-C₇)-cycloalkyle, phényle, -OR¹⁰, -NR¹¹R¹², -C(=O)-OR¹³ et -C(=O)-NR¹⁴R¹⁵, où
(i) (C₃-C₇)-cycloalkyle peut être monosubstitué jusqu'à disubstitué, de manière identique ou différente, par (C₁-C₄)-alkyle, oxo, hydroxy, (C₁-C₄)-alcoxy et/ou amino,
(ii) phényle peut être jusqu'à trisubstitué, de manière identique ou différente, par des radicaux choisis dans la série halogène, cyano, nitro, (C₁-C₄) -alkyle, trifluorométhyle, hydroxy, hydroxyméthyle, (C₁-C₄ -alcoxy, trifluorométhoxy, (C₁-C₄)-alcoxyméthyle, hydroxycarbonyle, (C₁-C₄)-alcoxycarbonyle, aminocarbonyle, mono-(C₁-C₄)-alkylaminocarbonyle et di-(C₁-C₄)-alkylaminocarbonyle,
(iii) R¹⁰, R¹¹, R¹², R¹³, R¹⁴ et R¹⁵ signifient, indépendamment l'un de l'autre, à chaque occurrence, hydrogène, (C₁-C₆)-alkyle ou (C₃-C₇)-cycloalkyle, où
(C₁-C₆)-alkyle peut à son tour être jusqu'à disubstitué, de manière identique ou différente, par amino, hydroxy, (C₁-C₄)-alcoxy, hydroxycarbonyle et/ou (C₁-C₄)-alcoxycarbonyle et (C₃-C₇)-cycloalkyle peut à son tour être jusqu'à disubstitué, de manière identique ou différente, par (C₁-C₄) - alkyle, oxo, hydroxy, (C₁-C₄)-alcoxy et/ou amino, et/ou
(iv) R¹¹ et R¹² ainsi que R¹⁴ et R¹⁵, forment à chaque fois par paire, ensemble avec l'atome d'azote auquel ils sont liés, un hétérocycle de 4 à 7 chaînons, qui peut contenir un autre hétéroatome de la série N, O et S et qui peut être jusqu'à disubstitué, de manière identique ou différente, par (C₁-C₄)-alkyle, oxo, hydroxy, (C₁-C₄)-alcoxy et/ou amino,
ou
R¹ représente (C₃-C₇) -cycloalkyle, qui peut être jusqu'à disubstitué, de manière identique ou différente, par (C₁-C₄)-alkyle, (C₁-C₄-alcoxy, hydroxy, amino et/ou oxo,
R² représente phényle, naphtyle, thiényle, benzothiényle, furyle ou benzofuryle, qui peuvent à chaque fois être monosubstitués à trisubstitués, de manière identique ou différente, par des radicaux choisis dans la série halogène, cyano, nitro, (C₁-C₄)-alkyle, trifluorométhyle, hydroxy, (C₁-C₄)-alcoxy, trifluorométhoxy et phényle,
où le dernier radical phényle mentionné peut à son tour être jusqu'à disubstitué, de manière identique ou différente, par des radicaux choisis dans la série halogène, cyano, nitro, (C₁-C₄)-alkyle, trifluorométhyle, hydroxy, (C₁-C₄)-alcoxy, trifluorométhoxy, hydroxy-(C₁-C₄)-alkyle et (C₁-C₄)-alkylthio,
L¹ représente un groupe de formule -(CR^{5A}R^{5B})ₘ-, où
m vaut le nombre 1, 2 ou 3, et
R^{5A} et R^{5B} signifient, indépendamment l'un de l'autre, hydrogène ou (C₁-C₄)-alkyle, ou deux radicaux R^{5A} et R^{5B} liés au même atome de carbone sont liés l'un à l'autre et forment ensemble un pont -(CH₂)ₙ-, où
n vaut le nombre 2, 3, 4 ou 5,
ou dans le cas où m vaut 2 ou 3,
deux radicaux R^{5A} et/ou R^{5B} liés à des atomes de carbone adjacents (1,2- ou 2,3-) ou non adjacents (1,3-) sont liés l'un à l'autre et forment ensemble un pont -(CH₂)p-, où
p vaut le nombre 1, 2, 3 ou 4, où, dans le cas où le groupe -CR^{5A}R^{5B}- existe plusieurs fois, les différentes significations de R^{5A} et R^{5B} peuvent à chaque fois être identiques ou différentes,
ou
L¹ représente un groupe de formule 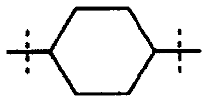
L² représente un groupe de formule *-CR^{6A}R^{6B}-(CR^{7A}R^{7B})_{q}-ou *-CR^{6A}R^{6B}-CR^{7A}R^{7B}-O-, où
* signifie le site de liaison avec l'atome N du groupement amide,
q vaut le nombre 0, 1 ou 2,
R^{6A} signifie hydrogène ou (C₁-C₄) -alkyle,
R^{6B} signifie hydrogène, (C₁-C₄) -alkyle, trifluorométhyle, (C₃-C₆)-cycloalkyle ou phényle, qui peut être jusqu'à disubstitué, de manière identique ou différente, par halogène, (C₁-C₄)-alkyle et/ou trifluorométhyle, ou signifie un radical de formule -C(=O) -OR¹⁶ ou -C(=O)-NR¹⁷R¹⁸, où
R¹⁶, R¹⁷ et R¹⁸ représentent, indépendamment l'un de l'autre, hydrogène, (C₁-C₄)-alkyle ou (C₃-C₆) -cycloalkyle ou
R¹⁷ et R¹⁸ forment ensemble avec l'atome d'azote auquel ils sont liés, un hétérocycle de 4 à 6 chaînons, qui peut contenir un autre hétéroatome de la série N, O et S et qui peut être jusqu'à disubstitué, de manière identique ou différente, par (C₁-C₄)-alkyle, hydroxy et/ou (C₁-C₄)-alcoxy, ou
R^{6A} et R^{6B} sont liés l'un à l'autre et forment, ensemble, un pont -(CH₂)ᵣ-, où
r vaut le nombre 2, 3, 4 ou 5,
et un groupe CH₂ du pont peut être remplacé par -O-, -S- ou >N-R¹⁹, où
R¹⁹ représente hydrogène ou (C₁-C₄) -alkyle,
R^{7A} signifie hydrogène, fluor, (C₁-C₄)-alkyle ou (C₁-C₄)-alcoxy,
R^{7B} signifie hydrogène, fluor, (C₁-C₄) -alkyle, hydroxy- (C₁-C₄) -alkyle ou un radical de formule -OR²⁰, -NR²¹R²², -C(=O)-OR²³ ou -C(=O)-NR²⁴R²⁵, où
R²⁰, R²¹, R²² R²³, R²⁴ et R²⁵ représentent, indépendamment l'un de l'autre, hydrogène, (C₁-C₄) -alkyle ou (C₃-C₆)-cycloalkyle ou
R²¹ et R²² ainsi que R²⁴ et R²⁵, forment à chaque fois par paire, ensemble avec l'atome d'azote auquel ils sont liés, un hétérocycle de 4 à 6 chaînons, qui peut contenir un autre hétéroatome de la série N, O et S et qui peut être jusqu'à disubstitué, de manière identique ou différente, par (C₁-C₄)-alkyle, hydroxy et/ou (C₁-C₄)-alcoxy, ou
R^{7A} et R^{7B} forment ensemble un groupe oxo ou
R^{7A} et R^{7B} sont liés l'un à l'autre et forment, ensemble, un pont -(CH₂)ₛ-, où
s vaut le nombre 2, 3, 4 ou 5,
et un groupe CH₂ du pont peut être remplacé par -O-, -S- ou >N-R²⁶, où
R²⁶ représente hydrogène ou (C₁-C₄)-alkyle,
où, dans le cas où le groupe -CR^{7A}R^{7B}- existe plusieurs fois, les différentes significations de R^{7A} et R^{7B} peuvent à chaque fois être identiques ou différentes,
ou
L² représente un groupe de formule 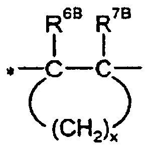 où
* signifie le site de liaison avec l'atome N du groupement amide,
x vaut le nombre 1, 2, 3 ou 4, un groupe CH₂ du cycle pouvant être remplacé par -O-, -S- ou >N-R²⁷, où
R²⁷ représente hydrogène ou (C₁-C₄)-alkyle, et
R^{6B} et R^{7B} présentent à chaque fois les significations indiquées ci-dessus,
R³ représente phényle, naphtyle ou hétéroaryle de 5 à 10 chaînons, comprenant jusqu'à trois hétéroatomes de la série N, O et/ou S, qui peuvent être à chaque fois monosubstitués à trisubstitués, de manière identique ou différente, par des radicaux choisis dans la série halogène, cyano, nitro, (C₁-C₄)-alkyle, trifluorométhyle, hydroxy, (C₁-C₄ - alcoxy, trifluorométhoxy, (C₁-C₄) -alkylthio, (C₁-C₄)-alkylsulfinyle, (C₁-C₄) -alkylsulfonyle, di-(C₁-C₄)-alkylamino et phényle,
où le dernier radical phényle mentionné peut à son tour être jusqu'à disubstitué, de manière identique ou différente, par halogène, cyano, nitro, (C₁-C₄) -alkyle, (C₁-C₄) -alcoxy, trifluorométhyle et/ou trifluorométhoxy,
ou le groupement
L²-R³ forme, dans sa totalité, un groupe de formule 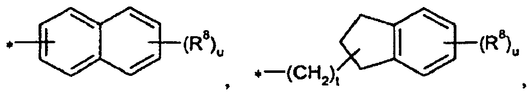 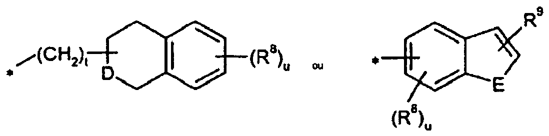 où
* signifie le site de liaison avec l'atome N du groupement amide,
D signifie CH₂ ou O,
E signifie NH, N-CH₃, O ou S,
t vaut le nombre 0 ou 1,
R⁸ signifie un substituant choisi dans la série halogène, cyano, nitro, (C₁-C₄) -alkyle, (C₁-C₄)-alcoxy, trifluorométhyle et trifluorométhoxy,
u vaut le nombre 0, 1 ou 2,
où, dans le cas où le substituant R⁸ existe plusieurs fois, ses significations peuvent être identiques ou différentes, et
R⁹ signifie hydrogène ou (C₁-C₄)-alkyle,
ainsi que ses sels, solvates et les solvates des sels.

2. Composé de formule (I) selon la revendication 1, dans laquelle
A représente N ou C-R⁴, où
R⁴ signifie hydrogène ou (C₁-C₄) -alkyle,
R¹ représente (C₁-C₆)-alkyle, qui peut être substitué par hydroxy, (C₁-C₆)-alcoxy, (C₃-C₇)-cycloalkyle ou phényle, ou représente (C₃-C₇)-cycloalkyle, les radicaux cycloalkyle mentionnés pouvant à leur tour être jusqu'à disubstitués, de manière identique ou différente, par (C₁-C₄) -alkyle, (C₁-C₄)-alcoxy, hydroxy, amino et/ou oxo et
le radical phényle pouvant être jusqu'à trisubstitué, de manière identique ou différente, par halogène, cyano, nitro, (C₁-C₄) -alkyle, (C₁-C₄) - alcoxy, trifluorométhyle et/ou trifluorométhoxy,
R² représente phényle, naphtyle, thiényle, benzothiényle, furyle ou benzofuryle, qui peuvent à chaque fois être jusqu'à trisubstitués, de manière identique ou différente, par halogène, cyano, nitro, (C₁-C₄) -alkyle, (C₁-C₄) -alcoxy, trifluorométhyle, trifluorométhoxy et/ou phényle, où le dernier radical phényle mentionné peut à son tour être jusqu'à disubstitué, de manière identique ou différente, par halogène, cyano, nitro, (C₁-C₄) -alkyle, (C₁-C₄) -alcoxy, trifluorométhyle et/ou trifluorométhoxy,
L¹ représente un groupe de formule -(CR^{5A}R^{5B})ₘ-, où
m vaut le nombre 1, 2 ou 3, et
R^{5A} et R^{5B} signifient, indépendamment l'un de l'autre, hydrogène ou (C₁-C₄)-alkyle, ou deux radicaux R^{5A} et R^{5B} liés au même atome de carbone sont liés l'un à l'autre et forment ensemble un pont -(CH₂)ₙ-, où
n vaut le nombre 2, 3, 4 ou 5,
ou dans le cas où m vaut 2 ou 3,
deux radicaux R^{5A} et/ou R^{5B} liés à des atomes de carbone adjacents (1,2- ou 2,3-) ou non adjacents (1,3-) sont liés l'un à l'autre et forment ensemble un pont -(CH₂)ₚ-, où
p vaut le nombre 1, 2, 3 ou 4, où, dans le cas où le groupe -CR^{5A}R^{5B}- existe plusieurs fois, les différentes significations de R^{5A} et R^{5B} peuvent à chaque fois être identiques ou différentes, ou
L¹ représente un groupe de formule 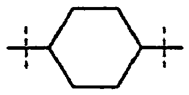
L² représente un groupe de formule *-CR^{6A}R^{6B}-(CR^{7A}R^{7B})_{q}-ou *-CR^{6A}R^{6B}-CR^{7A}R^{7B}-O- où
* signifie le site de liaison avec l'atome N du groupement amide,
q vaut le nombre 0, 1 ou 2,
R^{6A} signifie hydrogène ou (C₁-C₄) -alkyle,
R^{6B} signifie hydrogène, (C₁-C₄)-alkyle, trifluorométhyle, (C₃-C₆)-cycloalkyle ou phényle, qui peut être jusqu'à disubstitué, de manière identique ou différente, par halogène, (C₁-C₄)-alkyle et/ou trifluorométhyle, ou
R^{6A} et R^{6B} sont liés l'un à l'autre et forment, ensemble, un pont -(CH₂)ᵣ-, où
r vaut le nombre 2, 3, 4 ou 5, et
R^{7A} et R^{7B} signifient, indépendamment l'un de l'autre, hydrogène ou (C₁-C₄)-alkyle, où, dans le cas où le groupe -CR^{7A}R^{7B}- existe plusieurs fois, les différentes significations de R^{7A} et R^{7B} peuvent à chaque fois être identiques ou différentes,
R³ représente phényle, naphtyle ou hétéroaryle de 5 à 10 chaînons, comprenant jusqu'à trois hétéroatomes de la série N, O et/ou S, qui peuvent être à chaque fois jusqu'à trisubstitués, de manière identique ou différente, par halogène, cyano, nitro, (C₁-C₄) -alkyle, (C₁-C₄) -alcoxy, trifluorométhyle, trifluorométhoxy, di-(C₁-C₄)-alkylamino et/ou phényle,
où le dernier radical phényle mentionné peut à son tour être jusqu'à disubstitué, de manière identique ou différente, par halogène, cyano, nitro, (C₁-C₄) -alkyle, (C₁-C₄) -alcoxy, trifluorométhyle et/ou trifluorométhoxy,
ou le groupement
L²-R³ forme, dans sa totalité, un groupe de formule 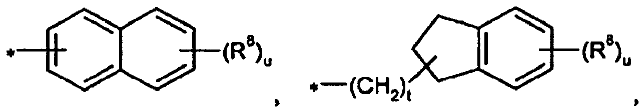 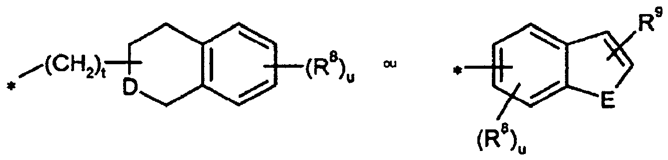 où
* signifie le site de liaison avec l'atome N du groupement amide,
D signifie CH₂ ou O,
E signifie NH, N-CH₃, O ou S,
t vaut le nombre 0 ou 1,
R⁸ signifie un substituant choisi dans la série halogène, cyano, nitro, (C₁-C₄) -alkyle, (C₁-C₄)-alcoxy, trifluorométhyle et trifluorométhoxy,
u vaut le nombre 0, 1 ou 2,
où, dans le cas où le substituant R⁸ existe plusieurs fois, ses significations peuvent être identiques ou différentes, et
R⁹ signifie hydrogène ou (C₁-C₄) -alkyle,
ainsi que ses sels, solvates et les solvates des sels.

3. Composé de formule (I) selon la revendication 1, dans laquelle
A représente N ou C-R⁴, où
R⁴ signifie hydrogène ou (C₁-C₄) -alkyle,
R¹ représente (C₁-C₆)-alkyle, qui peut être monosubstitué à trisubstitué, de manière identique ou différente, par des radicaux choisis dans la série fluor, chlore, cyano, oxo, trifluorométhyle, (C₃-C₆)-cycloalkyle, phényle, -OR¹⁰, -NR¹¹R¹², -C(=O)-OR¹³ et -C(=O)-NR¹⁴R¹⁵, où
(i) (C₃-C₆)-cycloalkyle peut être monosubstitué jusqu'à disubstitué, de manière identique ou différente, par (C₁-C₄)-alkyle, oxo, hydroxy, (C₁-C₄)-alcoxy et/ou amino,
(ii) phényle peut être jusqu'à trisubstitué, de manière identique ou différente, par des radicaux choisis dans la série fluor, chlore, cyano, (C₁-C₄)-alkyle, trifluorométhyle, hydroxy, hydroxyméthyle, (C₁-C₄)-alcoxy, trifluorométhoxy, (C₁-C₄)-alcoxyméthyle, hydroxycarbonyle, (C₁-C₄)-alcoxycarbonyle, aminocarbonyle, mono- (C₁-C₄)-alkylaminocarbonyle et di-(C₁-C₄)-alkylaminocarbonyle,
(iii) R¹⁰, R¹¹, R¹², R¹³, R¹⁴ et R¹⁵ signifient, indépendamment l'un de l'autre, à chaque occurrence, hydrogène, (C₁-C₄)-alkyle ou (C₃-C₆)-cycloalkyle, où
(C₁-C₄)-alkyle peut à son tour être jusqu'à disubstitué, de manière identique ou différente, par amino, hydroxy, (C₁-C₄)-alcoxy, hydroxycarbonyle et/ou (C₁-C₄)-alcoxycarbonyle et
(C₃-C₆)-cycloalkyle peut à son tour être jusqu'à disubstitué, de manière identique ou différente, par (C₁-C₄)-alkyle, oxo, hydroxy, (C₁-C₄)-alcoxy ou amino, et/ou
(iv) R¹¹ et R¹² ainsi que R¹⁴ et R¹⁵, forment à chaque fois par paire, ensemble avec l'atome d'azote auquel ils sont liés, un hétérocycle de 4 à 6 chaînons, qui peut contenir un autre hétéroatome de la série N et O et qui peut être jusqu'à disubstitué, de manière identique ou différente, par (C₁-C₄)-alkyle, oxo, hydroxy, (C₁-C₄)-alcoxy et/ou amino, ou
R¹ représente (C₂-C₆)-alcényle, qui peut être substitué par hydroxy, (C₁-C₄)-alcoxy, hydroxycarbonyle ou (C₁-C₄)-alcoxycarbonyle, ou représente (C₃-C₆)-cycloalkyle, qui peut être jusqu'à disubstitué, de manière identique ou différente, par (C₁-C₄) -alkyle, (C₁-C₄) -alcoxy, hydroxy, amino et/ou oxo,
R² représente phényle ou thiényle, qui peuvent être monosubstitués à trisubstitués, de manière identique ou différente, par des radicaux choisis dans la série fluor, chlore, cyano, (C₁-C₄)-alkyle, trifluorométhyle, hydroxy, (C₁-C₄)-alcoxy, trifluorométhoxy et phényle,
où le dernier radical phényle mentionné peut à son tour être jusqu'à disubstitué, de manière identique ou différente, par des radicaux choisis dans la série fluor, chlore, cyano, (C₁-C₄)-alkyle, trifluorométhyle, (C₁-C₄)-alcoxy et trifluorométhoxy,
L¹ représente un groupe de formule -CR^{5A}R^{5B}-, où
R^{5A} et R^{5B} signifient, indépendamment l'un de l'autre, hydrogène ou (C₁-C₄)-alkyle,
L² représente un groupe de formule *-CR^{6A}R^{6B}-(CR^{7A}R^{7B}) _{q}-, où
* signifie le site de liaison avec l'atome N du groupement amide,
q vaut le nombre 0 ou 1,
R^{6A} signifie hydrogène ou (C₁-C₄) -alkyle,
R^{6B} signifie hydrogène, (C₁-C₄) -alkyle, trifluorométhyle, (C₃-C₆) -cycloalkyle ou phényle, qui peut être jusqu'à disubstitué, de manière identique ou différente, par fluor, chlore, (C₁-C₄) -alkyle et/ou trifluorométhyle, ou un radical de formule -C(=O)-OR¹⁶ ou -C (=O)-NR¹⁷R¹⁸, où R¹⁶, R¹⁷ et R¹⁸ représentent, indépendamment l'un de l'autre, hydrogène, (C₁-C₄)-alkyle ou (C₃-C₆)-cycloalkyle ou
R¹⁷ et R¹⁸ forment ensemble avec l'atome d'azote auquel ils sont liés, un hétérocycle de 4 à 6 chaînons, qui peut contenir un autre hétéroatome de la série N et O et qui peut être jusqu'à disubstitué, de manière identique ou différente, par (C₁-C₄) -alkyle, hydroxy et /ou (C₁-C₄) -alcoxy, ou
R^{6A} et R^{6B} sont liés l'un à l'autre et forment, ensemble, un pont - (CH₂) ᵣ-, où
r vaut le nombre 2, 3, 4 ou 5,
et un groupe CH₂ du pont peut être remplacé par -O-,
R^{7A} signifie hydrogène, fluor ou (C₁-C₄) -alkyle,
R^{7B} signifie hydrogène, fluor, (C₁-C₄)-alkyle, hydroxy- (C₁-C₄) -alkyle ou un radical de formule -OR²⁰, -NR²¹R²², -C(=O) -OR²³ ou -C(=O)-NR²⁴R²⁵, où
R²⁰, R²¹, R²², R²³, R²⁴ et R²⁵ représentent, indépendamment l'un de l'autre, hydrogène, (C₁-C₄) -alkyle ou (C₃-C₆)-cycloalkyle ou
R²¹ et R²² ainsi que R²⁴ et R²⁵, forment à chaque fois par paire, ensemble avec l'atome d'azote auquel ils sont liés, un hétérocycle de 4 à 6 chaînons, qui peut contenir un autre hétéroatome de la série N et O et qui peut être jusqu'à disubstitué, de manière identique ou différente, par (C₁-C₄) -alkyle, hydroxy et/ou (C₁-C₄) -alcoxy, ou
R^{7A} et R^{7B} forment ensemble un groupe oxo ou
R^{7A} et R^{7B} sont liés l'un à l'autre et forment, ensemble, un pont -(CH₂)s-, où
s vaut le nombre 2, 3, 4 ou 5,
et un groupe CH₂ du pont peut être remplacé par -O-,
ou
L² représente un groupe de formule 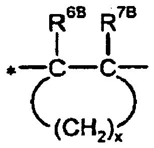 où
* signifie le site de liaison avec l'atome N du groupement amide,
x vaut le nombre 1, 2, 3 ou 4,
un groupe CH₂ du cycle pouvant être remplacé par -O-, et
R^{6B} et R^{7B} présentent à chaque fois les significations indiquées ci-dessus, et
R³ représente phényle, naphtyle ou hétéroaryle de 5 à 10 chaînons, comprenant jusqu'à deux hétéroatomes de la série N, O et/ou S, qui peuvent être à chaque fois monosubstitués à trisubstitués, de manière identique ou différente, par des radicaux choisis dans la série fluor, chlore, cyano, (C₁-C₄)-alkyle, trifluorométhyle, hydroxy, (C₁-C₄)-alcoxy, trifluorométhoxy, (C₁-C₄)-alkylthio, (C₁-C₄) -alkylsulfonyle, di- (C₁-C₄) -alkylamino et phényle,
où le dernier radical phényle mentionné peut à son tour être jusqu'à disubstitué, de manière identique ou différente, par fluor, chlore, cyano, (C₁-C₄) -alkyle, (C₁-C₄) -alcoxy, trifluorométhyle et/ou trifluorométhoxy,
ainsi que ses sels, solvates et les solvates des sels.

4. Composé de formule (I) selon la revendication 1 ou 2, dans laquelle
A représente N ou C-R⁴, où
R⁴ signifie hydrogène ou (C₁-C₄) -alkyle,
R¹ représente (C₁-C₆)-alkyle, qui peut être substitué par hydroxy, (C₁-C₄) -alcoxy, (C₃-C₆ -cycloalkyle ou phényle, ou représente (C₃-C₆)-cycloalkyle,
les radicaux cycloalkyle mentionnés pouvant à leur tour être jusqu'à disubstitués, de manière identique ou différente, par (C₁-C₄) -alkyle, (C₁-C₄)-alcoxy, hydroxy et/ou amino et
le radical phényle pouvant être jusqu'à disubstitué, de manière identique ou différente, par halogène, cyano, nitro, (C₁-C₄) -alkyle, (C₁-C₄) - alcoxy, trifluorométhyle et/ou trifluorométhoxy,
R² représente phényle, naphtyle, thiényle ou benzothiényle, qui peuvent à chaque fois être jusqu'à trisubstitués, de manière identique ou différente, par halogène, cyano, nitro, (C₁-C₄)-alkyle, (C₁-C₄)-alcoxy, trifluorométhyle, trifluorométhoxy et/ou phényle,
où le dernier radical phényle mentionné peut à son tour être jusqu'à disubstitué, de manière identique ou différente, par halogène, cyano, nitro, (C₁-C₄) -alkyle, (C₁-C₄) -alcoxy, trifluorométhyle et/ou trifluorométhoxy,
L¹ représente un groupe de formule - (CR^{5A}R^{5B}) ₘ-, où
m vaut le nombre 1, 2 ou 3, et
R^{5A} et R^{5B} signifient, indépendamment l'un de l'autre, hydrogène ou méthyle ou deux radicaux R^{5A} et R^{5B} liés au même atome de carbone sont liés l'un à l'autre et forment ensemble un pont -(CH₂)ₙ-, où
n vaut le nombre 2, 3, 4 ou 5,
ou dans le cas où m vaut 2 ou 3,
deux radicaux R^{5A} et/ou R^{5B} liés à des atomes de carbone adjacents (1,2- ou 2,3-) ou non adjacents (1,3-) sont liés l'un à l'autre et forment ensemble un pont -(CH₂)ₚ-, où
p vaut le nombre 1, 2, 3 ou 4,
où, dans le cas où le groupe -CR^{5A}R^{5B}- existe plusieurs fois, les différentes significations de R^{5A} et R^{5B} peuvent à chaque fois être identiques ou différentes,
ou
L¹ représente un groupe de formule 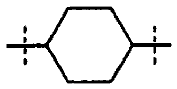
L² représente un groupe de formule *-CR^{6A}R^{6B}-(CH₂)_{q}-, où
* signifie le site de liaison avec l'atome N du groupement amide,
q vaut le nombre 0 ou 1,
R^{6A} signifie hydrogène ou méthyle,
R^{6B} signifie hydrogène, (C₁-C₄) -alkyle, trifluorométhyle ou (C₃-C₆)-cycloalkyle, ou
R^{6A} et R^{6B} sont liés l'un à l'autre et forment, ensemble, un pont -(CH₂)ᵣ-, où
r vaut le nombre 2, 3, 4 ou 5,
et
R³ représente phényle, naphtyle ou hétéroaryle de 5 à 10 chaînons, comprenant jusqu'à deux hétéroatomes de la série N, O et/ou S, qui peuvent être à chaque fois jusqu'à trisubstitués, de manière identique ou différente, par halogène, cyano, nitro, (C₁-C₄) -alkyle, (C₁-C₄) -alcoxy, trifluorométhyle, trifluorométhoxy et/ou phényle, où le dernier radical phényle mentionné peut à son tour être jusqu'à disubstitué, de manière identique ou différente, par halogène, cyano, nitro, (C₁-C₄)-alkyle, (C₁-C₄) -alcoxy, trifluorométhyle et/ou trifluorométhoxy,
ainsi que ses sels, solvates et les solvates des sels.

5. Composé de formule (I) selon la revendication 1 ou 3, dans laquelle
A représente N ou C-R⁴, où
R⁴ signifie hydrogène ou méthyle,
R¹ représente (C₁-C₆)-alkyle, qui peut être monosubstitué à disubstitué, de manière identique ou différente, par des radicaux choisis dans la série fluor, oxo, trifluorométhyle, (C₃-C₆)-cycloalkyle, phényle, -OR¹⁰, -C (=O) -OR¹³ et -C (=O) - NR¹⁴R¹⁵, où
(i) (C₃-C₆)-cycloalkyle peut être jusqu'à disubstitué, de manière identique ou différente, par (C₁-C₄)-alkyle, hydroxy et/ou (C₁-C₄) -alcoxy,
(ii) phényle peut être jusqu'à disubstitué, de manière identique ou différente, par des radicaux choisis dans la série fluor, chlore, cyano, (C₁-C₄)-alkyle, trifluorométhyle, hydroxyméthyle, (C₁-C₄) -alcoxy, hydroxycarbonyle, (C₁-C₄)-alcoxycarbonyle, aminocarbonyle, mono- (C₁-C₄) - alkylaminocarbonyle et di-(C₁-C₄)-alkylaminocarbonyle, et
(iii) R¹⁰, R¹³, R¹⁴ et R¹⁵ signifient, indépendamment l'un de l'autre, à chaque occurrence, hydrogène, (C₁-C₄) -alkyle ou (C₃-C₆)-cycloalkyle, où
(C₁-C₄)-alkyle peut à son tour être jusqu'à disubstitué, de manière identique ou différente, par hydroxy, (C₁-C₄)-alcoxy, hydroxycarbonyle et/ou (C₁-C₄)-alcoxycarbonyle et
(C₃-C₆)-cycloalkyle peut à son tour être jusqu'à disubstitué, de manière identique ou différente, par (C₁-C₄)-alkyle, hydroxy et/ou (C₁-C₄) -alcoxy,
ou
R¹ représente (C₂-C₆)-alcényle, qui peut être substitué par hydroxycarbonyle ou (C₁-C₄)-alcoxycarbonyle, ou
représente (C₃-C₆)-cycloalkyle, qui peut être jusqu'à disubstitué, de manière identique ou différente, par (C₁-C₄)-alkyle, (C₁-C₄)-alcoxy et/ou hydroxy,
R² représente phényle ou thiényle, qui peuvent être monosubstitués à disubstitués, de manière identique ou différente, par des radicaux choisis dans la série fluor, chlore, (C₁-C₄)-alkyle, trifluorométhyle, (C₁-C₄)-alcoxy et trifluorométhoxy,
L¹ représente un groupe de formule -CR^{5A}R^{5B}-, où
R^{5A} et R^{5B} signifient, indépendamment l'un de l'autre hydrogène ou méthyle,
L² représente un groupe de formule *-CR^{6A}R^{6B}-(CR^{7A}R^{7B}) q-, où
* signifie le site de liaison avec l'atome N du groupement amide,
q vaut le nombre 0 ou 1,
R^{6A} signifie hydrogène ou méthyle,
R^{6B} signifie hydrogène, méthyle, trifluorométhyle ou un radical de formule -C(=O)-OR¹⁶ ou -C(=O)-NR¹⁷R¹⁸, où
R¹⁶, R¹⁷ et R¹⁸ représentent, indépendamment l'un de l'autre, hydrogène, (C₁-C₄)-alkyle ou (C₃-C₆)-cycloalkyle ou
R¹⁷ et R¹⁸ forment ensemble, avec l'atome d'azote auquel ils sont liés, un hétérocycle de 4 à 6 chaînons qui peut contenir un atome O comme autre hétéroatome, ou
R^{6A} et R^{6B} sont liés l'un à l'autre et forment, ensemble, un pont -(CH₂) ᵣ-, où
r vaut le nombre 2, 3, 4 ou 5,
et un groupe CH₂ du pont peut être remplacé par -O-,
R^{7A} signifie hydrogène, fluor ou méthyle,
R^{7B} signifie hydrogène, fluor, méthyle ou un radical de formule -C(=O)-OR²³ ou -C(=O)-NR²⁴R²⁵, où
R²³ , R²⁴ et R²⁵ représentent, indépendamment l'un de l'autre, hydrogène, (C₁-C₄)-alkyle ou (C₃-C₆)-cycloalkyle ou
R²⁴ et R²⁵ forment ensemble, avec l'atome d'azote auquel ils sont liés, un hétérocycle de 4 à 6 chaînons qui peut contenir un atome O comme autre hétéroatome, ou
R^{7A} et R^{7B} forment ensemble un groupe oxo ou
R^{7A} et R^{7B} sont liés l'un à l'autre et forment, ensemble, un pont -(CH₂)ₛ-, où
s vaut le nombre 2, 3, 4 ou 5,
et un groupe CH₂ du pont peut être remplacé par -O-,
ou
L² représente un groupe de formule 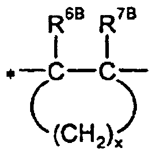 où
* signifie le site de liaison avec l'atome N du groupement amide,
x vaut le nombre 1, 2, 3 ou 4, un groupe CH₂ du cycle pouvant être remplacé par -O-, et
R^{6B} et R^{7B} présentent à chaque fois les significations indiquées ci-dessus, et
R³ représente phényle, naphtyle, pyridyle, quinoléinyle ou isoquinoléinyle, qui peuvent à chaque fois être monosubstitués à disubstitués, de manière identique ou différente, par des radicaux choisis dans la série fluor, chlore, cyano, (C₁-C₄) -alkyle, trifluorométhyle, (C₁-C₄)-alcoxy, trifluorométhoxy, (C₁-C₄)-alkylthio et phényle,
où le dernier radical phényle mentionné peut à son tour être jusqu'à disubstitué, de manière identique ou différente, par fluor, chlore, cyano, (C₁-C₄) -alkyle, (C₁-C₄) -alcoxy, trifluorométhyle et/ou trifluorométhoxy,
ainsi que ses sels, solvates et les solvates des sels.

6. Composé de formule (I) selon l'une quelconque des revendications 1 à 5, dans laquelle
A représente N ou CH,
R¹ représente (C₁-C₆)-alkyle, qui peut être monosubstitué à disubstitué, de manière identique ou différente, par des radicaux choisis dans la série fluor, oxo, hydroxy, (C₁-C₄)-alcoxy, trifluorométhyle, (C₃-C₆)-cycloalkyle et phényle, phényle pouvant à son tour être jusqu'à disubstitué, de manière identique ou différente, par des radicaux choisis dans la série fluor, chlore, cyano, (C₁-C₄)-alkyle, trifluorométhyle, hydroxyméthyle, (C₁-C₄)-alcoxy, trifluorométhoxy, hydroxycarbonyle, aminocarbonyle, et di-(C₁-C₄)-alkylaminocarbonyle, ou
R¹ représente (C₂-C₄)-alcényle ou (C₃-C₆-cycloalkyle,
R² représente phényle ou thiényle, qui peuvent être monosubstitués à disubstitués, de manière identique ou différente, par des radicaux choisis dans la série fluor, chlore, brome, (C₁-C₄) -alkyle et (C₁-C₄)-alcoxy,
L¹ représente -CH₂-, -CH (CH₃) - ou -CH₂CH₂-,
L² représente un groupe de formule *-CR^{6A}R^{6B}-(CR^{7A}R^{7B}) q-, où
* signifie le site de liaison avec l'atome N du groupement amide,
q vaut le nombre 0 ou 1,
R^{6A} signifie hydrogène ou méthyle,
R^{6B} signifie hydrogène, méthyle, trifluorométhyle ou un radical de formule -C(=O)-NR¹⁷R¹⁸, où
R¹⁷ et R¹⁸ représentent, indépendamment l'un de l'autre, hydrogène, (C₁-C₄)-alkyle ou (C₃-C₆) -cycloalkyle ou
R¹⁷ et R¹⁸ forment ensemble, avec l'atome d'azote auquel ils sont liés, un hétérocycle de 4 à 6 chaînons qui peut contenir un atome O comme autre hétéroatome, ou
R^{6A} et R^{6B} sont liés l'un à l'autre et forment, ensemble, un pont -(CH₂)ᵣ-, où
r vaut le nombre 2, 3, 4 ou 5,
et un groupe CH₂ du pont peut être remplacé par -O-,
R^{7A} signifie hydrogène, fluor ou méthyle,
R^{7B} signifie hydrogène, fluor, méthyle ou un radical de formule -C(=O)-OR²³ ou -C(=O)-NR²⁴R²⁵, où
R²³, R²⁴ et R²⁵ signifient, indépendamment l'un de l'autre, hydrogène ou (C₁-C₄)-alkyle, ou
R²⁴ et R²⁵ forment ensemble, avec l'atome d'azote auquel ils sont liés, un hétérocycle de 4 à 6 chaînons qui peut contenir un atome O comme autre hétéroatome, ou
R^{7A} et R^{7B} forment ensemble un groupe oxo ou
R^{7A} et R^{7B} sont liés l'un à l'autre et forment, ensemble, un pont -(CH₂)ₛ-, où
s vaut le nombre 2, 3, 4 ou 5,
et un groupe CH₂ du pont peut être remplacé par -O-,
ou
L² représente un groupe de formule 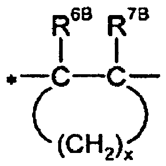 où
* signifie le site de liaison avec l'atome de N du groupement amide,
x vaut le nombre 1, 2, 3 ou 4, et
R^{6B} et R^{7B} présentent à chaque fois les significations indiquées ci-dessus, et
R³ représente phényle ou pyridyle, qui peuvent être monosubstitués à disubstitués, de manière identique ou différente, par des radicaux choisis dans la série fluor, chlore, (C₁-C₄) -alkyle, trifluorométhyle, (C₁-C₄)-alcoxy et trifluorométhoxy ou représente naphtyle,
ainsi que ses sels, solvates et les solvates des sels.

7. Composé de formule (I) selon l'une quelconque des revendications 1 à 6, dans laquelle
A représente N ou CH,
R¹ représente (C₁-C₆)-alkyle, qui peut être monosubstitué à disubstitué, de manière identique ou différente, par des radicaux choisis dans la série fluor, oxo, hydroxy, méthoxy, éthoxyle, trifluorométhyle, cyclopropyle et phényle,
phényle pouvant à son tour être jusqu'à disubstitué, de manière identique ou différente, par des radicaux choisis dans la série fluor, chlore, cyano, méthyle, hydroxyméthyle, méthoxy, hydroxycarbonyle, aminocarbonyle, et diméthylaminocarbonyle, ou
R¹ représente vinyle, allyle ou cyclopropyle,
R² représente phényle ou thiényle, qui peuvent être monosubstitués à disubstitués, de manière identique ou différente, par des radicaux choisis dans la série fluor, chlore, méthyle et méthoxy,
L¹ représente -CH₂-,
L² représente un groupe de formule *-CR^{6A}R^{6B}-(CR^{7A}R^{7B})_{q}-, où
* signifie le site de liaison avec l'atome de N du groupement amide,
q vaut le nombre 0 ou 1,
R^{6A} signifie hydrogène ou méthyle,
R^{6B} signifie hydrogène, méthyle, trifluorométhyle ou un radical de formule -C(=O)-NR¹⁷R¹⁸, où
R¹⁷ et R¹⁸ représentent, indépendamment l'un de l'autre hydrogène, méthyle, éthyle ou cyclopropyle ou
R¹⁷ et R¹⁸ forment ensemble, avec l'atome d'azote auquel ils sont liés, un cycle azétidine, pyrrolidine, pipéridine ou morpholine, ou
R^{6A} et R^{6B} sont liés l'un à l'autre et forment ensemble avec l'atome de carbone, auquel ils sont liés, un groupe de formule 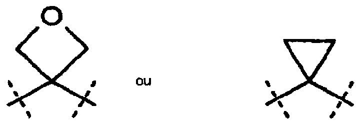
R^{7A} signifie hydrogène, fluor ou méthyle,
R^{7B} signifie hydrogène, fluor, méthyle ou un radical de formule -C(=O)-OR²³ ou -C(=O)-NR²⁴R²⁵, où
R²³, R²⁴ et R²⁵ représentent, indépendamment l'un de l'autre hydrogène, méthyle ou éthyle ou
R²⁴ et R²⁵ forment ensemble, avec l'atome d'azote auquel ils sont liés, un cycle azétidine, pyrrolidine, pipéridine ou morpholine, ou
R^{7A} et R^{7B} forment ensemble un groupe oxo ou
R^{7A} et R^{7B} sont liés l'un à l'autre et forment, ensemble, un pont -(CH₂)ₛ-, où
s vaut le nombre 2, 3, 4 ou 5,
et un groupe CH₂ du pont peut être remplacé par -O-, ou
L² représente un groupe de formule 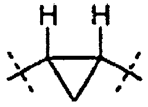 et
R³ représente phényle, qui peut être monosubstitué ou disubstitué, de manière identique ou différente, par fluor, chlore, trifluorométhyle et/ou trifluorométhoxy, ou représente 1-naphtyle,
ainsi que ses sels, solvates et les solvates des sels.

8. Composé de formule (I) selon l'une quelconque des revendications 1 à 6, dans laquelle
A représente N ou CH,
R¹ représente (C₁-C₄)-alkyle, 2-méthoxyéthyle, cyclopropyle, cyclohexylméthyle, benzyle ou 1-phénéthyle,
le cycle phényle dans les radicaux benzyle et 1-phénéthyle mentionnés pouvant être substitués par fluor, chlore, méthyle, trifluorométhyle, méthoxy ou trifluorométhoxy,
R² représente phényle ou thiényle, qui sont à chaque fois monosubstitués ou disubstitués, de manière identique ou différente, par fluor, chlore, brome, méthyle et/ou méthoxy,
L¹ représente -CH₂-, -CH₂CH₂- ou -CH (CH₃) -,
L² représente -CH₂- , -CH (CH₃) - ou -C(CH₃)₂- et
R³ représente phényle, qui peut être monosubstitué ou disubstitué, de manière identique ou différente, par fluor, chlore, trifluorométhyle et/ou trifluorométhoxy, ou représente 1-naphtyle,
ainsi que ses sels, solvates et les solvates des sels.

9. Procédé pour la préparation de composés de formule (I) tels que définis dans les revendications 1 à 8, **caractérisé en ce qu'**on
[A] couple un composé de formule (II) 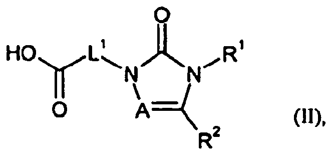 dans laquelle A, L¹, R¹ et R² présentent à chaque fois les significations indiquées dans les revendications 1 à 8,
dans un solvant inerte, en activant la fonction acide carboxylique, avec un composé de formule (III)
R³-L²-NH₂ (III),
dans laquelle L² et R³ présentent les significations indiquées dans les revendications 1 à 8,
ou
[B] transforme un composé de formule (IV) 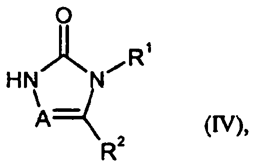 dans laquelle A, R¹ et R² présentent à chaque fois les significations indiquées dans les revendications 1 à 8,
dans un solvant inerte en présence d'une base avec un composé de formule (V) 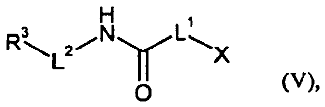 dans laquelle L¹, L² et R³ présentent à chaque fois les significations indiquées dans les revendications 1 à 8 et
X représente un groupe partant tel que par exemple halogène, mésylate ou tosylate,
et on transforme les composés obtenus de formule (I) le cas échéant avec (i) les solvants et/ou (ii) les bases ou acides correspondants en leurs solvates, sels et/ou solvates des sels.

10. Composé de formule (I), tel que défini dans l'une quelconque des revendications 1 à 8, destiné au traitement et/ou à la prophylaxie de maladies.

11. Utilisation d'un composé de formule (I), tel que défini dans l'une quelconque des revendications 1 à 8, pour la préparation d'un médicament destiné au traitement et/ou à la prophylaxie de l'insuffisance cardiaque aiguë et chronique, l'hyponatrémie hypervolémique et euvolémique, d'une cirrhose du foie, des ascites, des oedèmes et du syndrome d'une sécrétion inadéquate d'ADH (SIADH).

12. Médicament contenant un composé de formule (I), tel que défini dans l'une quelconque des revendications 1 à 8, en combinaison avec un adjuvant inerte, non toxique, pharmaceutiquement approprié.

13. Médicament contenant un composé de formule (I), tel que défini dans l'une quelconque des revendications 1 à 8, en combinaison avec une ou plusieurs autres substances actives choisies dans le groupe constitué par les diurétiques, les antagonistes de l'angiotensine AII, les inhibiteurs d'ACE, les bloquants des récepteurs bêta, les antagonistes des récepteurs des minéralocorticoïdes, les nitrates organiques, les donneurs de NO et les substances à activité inotrope positive.

14. Médicament selon la revendication 12 ou 13 pour le traitement et/ou la prophylaxie de l'insuffisance cardiaque aiguë et chronique, de l'hyponatrémie hypervolémique et euvolémique, de la cirrhose du foie, des ascites, des oedèmes et du syndrome d'une sécrétion inadéquate d'ADH (SIADH).
